(19) Europäisches Patentamt / European Patent Office / Office européen des brevets

(11) **EP 4 786 462 A1**

(12) **EUROPEAN PATENT APPLICATION**
published in accordance with Art. 153(4) EPC


(43) Date of publication:
**05.08.2026 Bulletin 2026/32**

(21) Application number: **24870670.7**

(22) Date of filing: **24.09.2024**

(51) International Patent Classification (IPC):
***C07D 401/14*** (2006.01) ***C07D 403/14*** (2006.01)
***A61K 31/506*** (2006.01) ***A61P 9/00*** (2006.01)

(52) Cooperative Patent Classification (CPC):
**A61K 31/506; A61P 9/00; C07D 401/14; C07D 403/14**

(86) International application number:
**PCT/CN2024/120513**

(87) International publication number:
**WO 2025/067126 (03.04.2025 Gazette 2025/14)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority:
**25.09.2023 CN 202311247773**
**28.09.2023 CN 202311282981**
**28.09.2023 CN 202311284021**
**28.09.2023 CN 202311281664**
**28.09.2023 CN 202311282445**
**26.10.2023 CN 202311409795**
**02.11.2023 CN 202311453050**
**02.11.2023 CN 202311458387**
**02.11.2023 CN 202311454007**
**20.11.2023 CN 202311555266**
**19.12.2023 CN 202311761849**
**19.12.2023 CN 202311762332**
**01.02.2024 CN 202410157983**
**01.02.2024 CN 202410147418**
**14.03.2024 CN 202410298905**
**24.04.2024 CN 202410517598**
**26.04.2024 CN 202410518428**
**29.05.2024 CN 202410682285**
**31.05.2024 CN 202410703062**
**07.06.2024 CN 202410740895**
**17.06.2024 CN 202410783164**
**27.06.2024 CN 202410854908**
**04.07.2024 CN 202410895133**



(71) Applicant: **Shenzhen Salubris Pharmaceuticals Co., Ltd.**
**Shenzhen, Guangdong 518017 (CN)**

(72) Inventors:
- **LAI, Yingjie**
  **Shenzhen, Guangdong 518017 (CN)**
- **LI, Zhongle**
  **Shenzhen, Guangdong 518017 (CN)**
- **XIAO, Ying**
  **Shenzhen, Guangdong 518017 (CN)**
- **ZENG, Junnan**
  **Shenzhen, Guangdong 518017 (CN)**
- **QING, Zhineng**
  **Shenzhen, Guangdong 518017 (CN)**
- **QI, Yinliang**
  **Shenzhen, Guangdong 518017 (CN)**
- **WU, Kang**
  **Shenzhen, Guangdong 518017 (CN)**
- **XIONG, Jinfeng**
  **Shenzhen, Guangdong 518017 (CN)**
- **DENG, Shenglu**
  **Shenzhen, Guangdong 518017 (CN)**
- **LIU, Hanbin**
  **Shenzhen, Guangdong 518017 (CN)**
- **TIAN, Zebing**
  **Shenzhen, Guangdong 518017 (CN)**
- **YUE, Xiaoliang**
  **Shenzhen, Guangdong 518017 (CN)**
- **XIANG, Jiehao**
  **Shenzhen, Guangdong 518017 (CN)**
- **LIU, Xiaoping**
  **Shenzhen, Guangdong 518017 (CN)**

(74) Representative: **Graf von Stosch Patentanwaltsgesellschaft mbH**
**Triftstraße 5**
**80538 München (DE)**


(54) **PYRIDONE COMPOUNDS AND PREPARATION METHOD THEREFOR, AND APPLICATION**


(57) The present application belongs to the technical field of chemical drugs, and relates to the compounds

shown in general formulae (A), (B), (C), (D), (E), (F), (G), (H) and (J), or racemates thereof, or isomers and pharmaceutically acceptable salts thereof, which act as PCSK9 inhibitors, and a method for using same to treat various specific diseases or conditions.

## Description

### TECHNICAL FIELD

The present disclosure belongs to the technical field of chemical drugs, and relates to a pyridone compound, or an isomer thereof, or a racemate thereof, or a pharmaceutically acceptable salt thereof, and preparation method therefor and application thereof. It acts as a PCSK9 inhibitor. Also related is a method of using it for treating various specific diseases or conditions.

### BACKGROUND

Proprotein convertase subtilisin/kexin type 9 (PCSK9), also known as neural apoptosis-regulated convertase 1 (NARC-1), is a prohormone-proprotein convertase belonging to the subtilisin (S8) family of serine proteases. It is expressed in cells with proliferation and differentiation capabilities, comprising hepatocytes, renal interstitial cells, ileal and colonic epithelial cells, and embryonic telencephalic neurons, etc. Studies have found that PCSK9 plays a role in the differentiation of hepatocytes and neural cells. It not only specifically acts on cholesterol biosynthesis or uptake, but also circulating PCSK9 can directly bind to the low-density lipoprotein receptor (LDLR) on the surface of hepatocytes, be internalized by hepatocytes together with LDLR, and promote the degradation of LDLR within hepatocytes, hindering the recycling of LDLR. This leads to an increase in the content of LDL cholesterol (LDL-C) in plasma, and elevated LDL-C content is closely related to human dyslipidemia and cardiovascular-related diseases.

Currently, research is ongoing regarding inhibiting the function of PCSK9 or inhibiting the production of PCSK9. For example, attempts such as inhibiting its function using monoclonal antibodies targeting PCSK9, and inhibiting the production of PCSK9 via RNA interference have been reported. However, for cardiovascular disease patients, there is a need for an effective small molecule to inhibit the function of PCSK9.

### SUMMARY

In view of the problems existing in the prior art, the present application provides a compound represented by general formula (A), (B), (C), (D), (E), (F), (G), (H), or (J), or an isomer thereof, or a racemate thereof, or a pharmaceutically acceptable salt thereof, and preparation method therefor and application thereof. It acts as a PCSK9 inhibitor. Also provided is a method of using it for treating various specific diseases or conditions.

Specifically, the present disclosure is achieved through the following technical solutions:

A compound represented by general formula (A), or an isomer thereof, or a racemate thereof, or a pharmaceutically acceptable salt thereof, wherein:

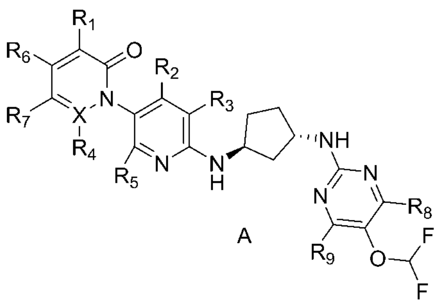

A

X is C or N, and when X is N, $R_4$ is absent;

$R_1$, $R_4$, $R_6$, and $R_7$ are selected from the group consisting of hydrogen, halogen, hydroxyl, substituted or unsubstituted alkyl, cyano, -C(O)-O-alkyl, phenylalkoxy, carboxyl, hydroxymethyl, cycloalkyl, amide, alkyl-substituted amide, Z-C(O)-, and heteroaryl, Z being selected from the group consisting of alkyl and heterocycloalkyl, and a substituent being hydroxyl;

$R_2$, $R_3$, and $R_5$ are each selected from the group consisting of hydrogen, alkyl, alkoxy, cyano, halogen, haloalkyl, amide, hydroxyl, hydroxyl-substituted alkyl, carboxyl, amino, amino-substituted alkyl, alkylcarbonyl, and $-(CH_2)_n-O-(CH_2)_n$-phenyl, and n = 1, 2, or 3;

$R_8$ and $R_9$ are each selected from the group consisting of hydrogen, alkyl, halogen, cycloalkyl, alkylthio, alkoxy, aryl, and heteroaryl; and

$R_1$, $R_2$, $R_3$, $R_4$, $R_5$, $R_6$, and $R_7$ are not all hydrogen simultaneously; or when $R_1$, $R_2$, $R_3$, $R_4$, $R_5$, $R_6$, and $R_7$ are all hydrogen simultaneously, then $R_8$ and $R_9$ are not both hydrogen simultaneously.

The present disclosure further provides a compound represented by general formula (AI), or an isomer thereof, or a racemate thereof, or a pharmaceutically acceptable salt thereof, wherein:

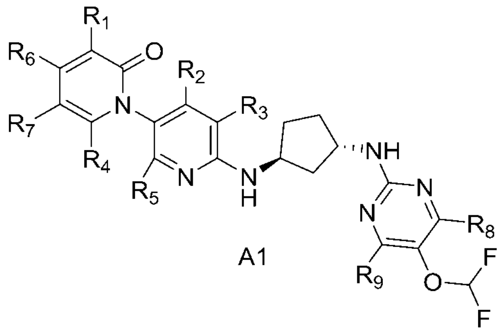

A1

$R_1$, $R_4$, $R_6$, and $R_7$ are selected from the group consisting of hydrogen, halogen, hydroxyl, substituted or unsubstituted alkyl, cyano, -C(O)-O-alkyl, phenylalkoxy, carboxyl, hydroxymethyl, cycloalkyl, amide, alkyl-substituted amide, Z-C(O)-, and heteroaryl, Z being selected from the group consisting of alkyl and heterocycloalkyl, and a substituent being hydroxyl;

$R_2$, $R_3$, and $R_5$ are each selected from the group consisting of hydrogen, alkyl, alkoxy, cyano, halogen, haloalkyl, amide, hydroxyl, hydroxyl-substituted alkyl, carboxyl, amino, amino-substituted alkyl, alkylcarbonyl, and $-(CH_2)_n-O-(CH_2)_n$-phenyl, and n = 1, 2, or 3;

$R_8$ and $R_9$ are each selected from the group consisting of hydrogen, alkyl, halogen, cycloalkyl, alkylthio, alkoxy, aryl, and heteroaryl; and

$R_1$, $R_2$, $R_3$, $R_4$, $R_5$, $R_6$, and $R_7$ are not all hydrogen simultaneously; or when $R_1$, $R_2$, $R_3$, $R_4$, $R_5$, $R_6$, and $R_7$ are all hydrogen simultaneously, then $R_8$ and $R_9$ are not both hydrogen simultaneously.

As a preferred technical solution of the present disclosure, in general formula (A) or (AI), $R_1$, $R_2$, $R_4$, $R_5$, $R_6$, and $R_7$ are all hydrogen simultaneously, and $R_3$ is selected from the group consisting of alkyl, alkoxy, cyano, halogen, haloalkyl, amide, hydroxyl, hydroxyl-substituted alkyl, carboxyl, amino, amino-substituted alkyl, alkylcarbonyl, and $-(CH_2)_n-O-(CH_2)_n$-phenyl, and n = 1, 2, or 3.

As a preferred technical solution of the present disclosure, in general formula (A) or (AI), $R_1$, $R_3$, $R_4$, $R_5$, $R_6$, and $R_7$ are all hydrogen simultaneously, and $R_2$ is selected from the group consisting of alkyl, alkoxy, cyano, halogen, haloalkyl, amide, hydroxyl, hydroxyl-substituted alkyl, carboxyl, amino, amino-substituted alkyl, alkylcarbonyl, and $-(CH_2)_n-O-(CH_2)_n$-phenyl, and n = 1, 2, or 3.

As a preferred technical solution of the present disclosure, in general formula (A) or (AI), the alkyl is selected from the group consisting of methyl, ethyl, n-propyl, isopropyl, n-butyl, isobutyl, sec-butyl, tert-butyl, n-pentyl, sec-pentyl, 1-ethylpropyl, 2-methylbutyl, tert-pentyl, 1,2-dimethylpropyl, isopentyl, neopentyl, n-hexyl, isohexyl, sec-hexyl, tert-hexyl, neohexyl, 2-methylpentyl, 1,2-dimethylbutyl, and 1-ethylbutyl; the haloalkyl refers to alkyl in which one or more hydrogens are substituted by halogen; the hydroxyl-substituted alkyl refers to alkyl in which one or more hydrogens are substituted by hydroxyl.

As a preferred technical solution of the present disclosure, in general formula (A) or (AI), the halogen is selected from the group consisting of fluorine, chlorine, bromine, and iodine.

As a preferred technical solution of the present disclosure, in general formula (A) or (AI), the cycloalkyl is selected from the group consisting of cyclopropyl, cyclobutyl, cyclopentyl, and cyclohexyl; the heterocycloalkyl refers to cycloalkyl in which one or more carbon atoms are replaced by heteroatoms, the heteroatoms being selected from the group consisting of N, O, and S.

As a preferred technical solution of the present disclosure, in general formula (A) or (AI), the alkoxy is selected from the group consisting of methoxy, ethoxy, n-propoxy, and isopropoxy.

As a preferred technical solution of the present disclosure, in general formula (A) or (AI), the aryl is phenyl; the heteroaryl is preferably selected from the group consisting of: imidazolyl, furanyl, thienyl, thiazolyl, pyrazolyl, oxazolyl, pyrrolyl, tetrazolyl, pyridyl, pyrimidinyl, thiadiazolyl, pyrazinyl,

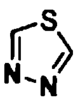 , 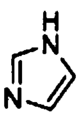 , 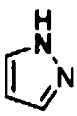 ,

etc.

As a preferred technical solution of the present disclosure, in general formula (A) or (AI), $R_2$ or $R_3$ is selected from the group consisting of F, Cl, Br, methyl, hydroxyl, hydroxymethyl, trifluoromethyl, $-CF_2H$, $-C(O)NH_2$, $-NH_2$, cyano, -COOH, methoxy, $-CH_2-O-CH_2-$phenyl,

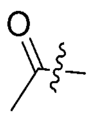, 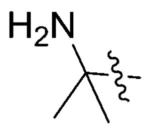, and 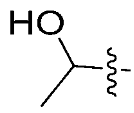.

As a preferred technical solution of the present disclosure, in general formula (A) or (AI), $R_1$ is selected from the group consisting of methyl, hydroxyl, hydroxymethyl, cyano, F, Cl, Br, $-O-CH_2-$phenyl, -COOH, $-COOCH_2CH_3$,

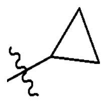, 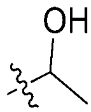,

amide, formamide, $CH_3-C(O)-$,

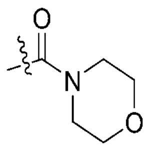, 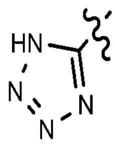, and 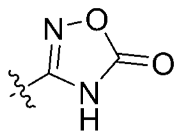.

As a preferred technical solution of the present disclosure, in general formula (A) or (AI), $R_6$ and $R_7$ are independently H or -COOH.

As a preferred technical solution of the present disclosure, in general formula (A) or (AI), $R_4$, $R_6$, and $R_7$ are independently H.

As a preferred technical solution of the present disclosure, in general formula (A) or (AI), $R_8$ and $R_9$ are each independently selected from the group consisting of hydrogen, chlorine, methyl, methoxy, methylthio, ethyl, isopropyl, phenyl, cyclopropyl, and

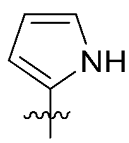.

As a preferred technical solution of the present disclosure, in general formula (A) or (AI), the compound, or an isomer thereof, or a racemate thereof, or a pharmaceutically acceptable salt thereof is selected from the group consisting of: A1, and A5 to A73.

As a preferred technical solution of the present disclosure, in general formula (A) or (AI), the isomer compound is selected from the group consisting of:

| | | | |
|---|---|---|---|
| A35A OR A35B | 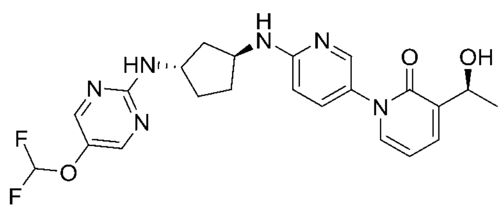 | A35B OR A35A | 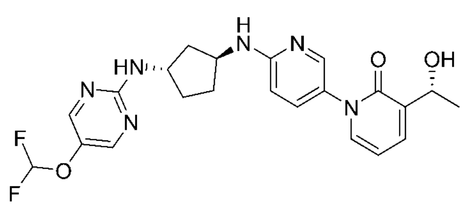 |

(continued)

| | | | |
|---|---|---|---|
| A43A OR A43B | 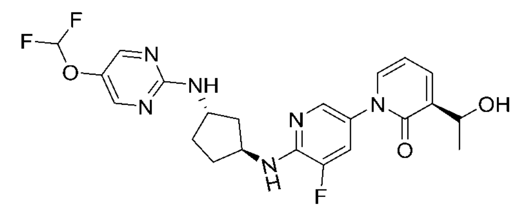 | A43B OR A43A | 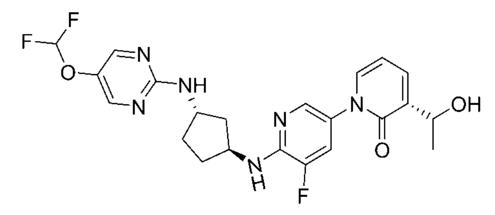 |

As a preferred technical solution of the present disclosure, in general formula (A) or (AI), the pharmaceutically acceptable salt refers to a salt prepared from the compound, or the isomer thereof, or the racemate thereof, or the pharmaceutically acceptable salt thereof, with a pharmaceutically acceptable acid or base.

As a preferred technical solution of the present disclosure, in general formula (A) or (AI), one or more hydrogen atoms in the compound, or the isomer thereof, or the racemate thereof, or the pharmaceutically acceptable salt thereof, are substituted by the isotope deuterium.

As a preferred technical solution of the present disclosure, in general formula (A) or (AI), the deuterium-substituted compound is selected from the group consisting of:

| | | | |
|---|---|---|---|
| A5-D A31D | 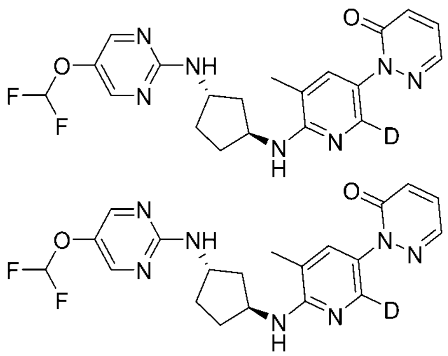 | A17-D | 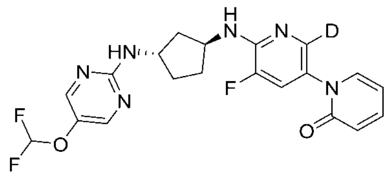 |
| A43A OR A43B-D | 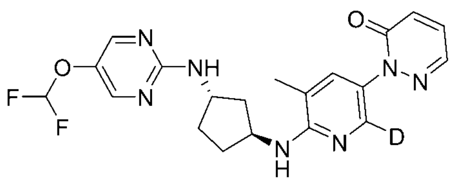 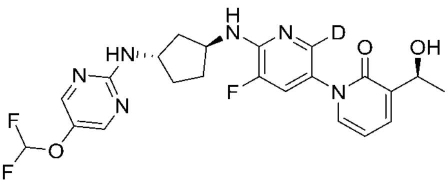 | A43B OR A43A-D | 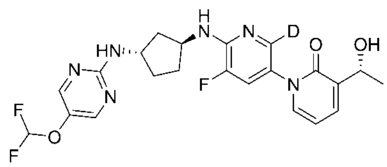 |

A compound represented by general formula (B), or an isomer thereof, or a racemate thereof, or a pharmaceutically acceptable salt thereof, wherein:

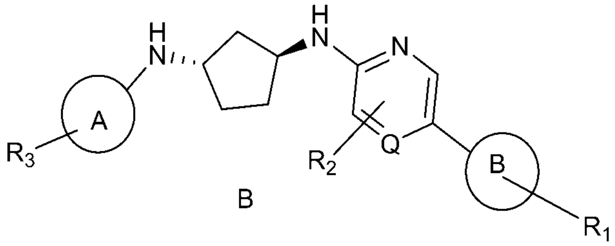

B

A is selected from the group consisting of

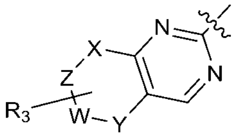 , 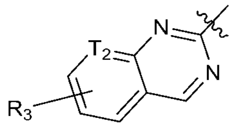 ,

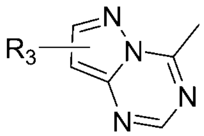 , 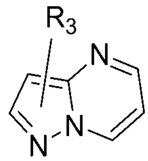 , and 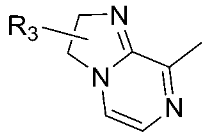 ;

B is selected from the group consisting of

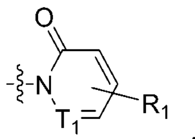 , 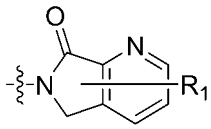 ,

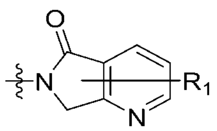 , 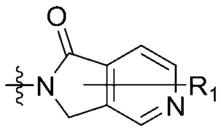 , 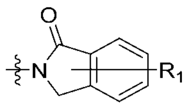 , 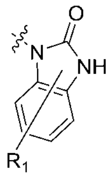 , 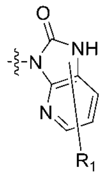 , 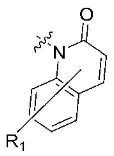 ,

and

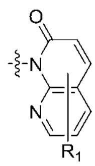 ;

Q is selected from the group consisting of N and $CR_4$, $R_4$ being selected from the group consisting of H and halogen;
$T_1$ and $T_2$ are selected from the group consisting of N and CH;
X and Y are independently selected from the group consisting of C(O), O, S, NH, and $CH_2$,
Z is absent, or Z is selected from the group consisting of C(O), O, S, NH, and $CH_2$,
W is selected from the group consisting of C(O), O, S, NH, and $CH_2$,
$R_1$ is hydrogen, or $R_1$ represents that the hydrogen on a ring is further substituted by alkyl, haloalkyl, carboxyl, alkoxy, haloalkoxy, cyano, halogen, or

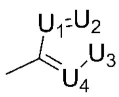 ;

$U_1$, $U_2$, and $U_4$ are independently selected from the group consisting of CH and N; $U_3$ is selected from the group consisting of $CH_2$ and NH; and a number of $R_1$ is one or more;
$R_2$ is selected from the group consisting of H, alkyl, and halogen; and a number of $R_2$ is one or more; and
$R_3$ is hydrogen, or $R_3$ represents that the hydrogen on a ring is further substituted by alkyl, halogen, oxo, substituted or unsubstituted phenyl, a substituent of the substituted phenyl is selected from the group consisting of alkyl and halogen; and a number of $R_3$ is one or more.

As a preferred technical solution of the present disclosure, in general formula (B), the alkyl being selected from the group consisting of methyl, ethyl, n-propyl, isopropyl, n-butyl, isobutyl, sec-butyl, tert-butyl, n-pentyl, sec-pentyl, 1-ethylpropyl, 2-methylbutyl, tert-pentyl, 1,2-dimethylpropyl, isopentyl, neopentyl, n-hexyl, isohexyl, sec-hexyl, tert-hexyl, neohexyl, 2-methylpentyl, 1,2-dimethylbutyl, and 1-ethylbutyl.

As a preferred technical solution of the present disclosure, in general formula (B), the alkoxy is selected from the group consisting of $C_{1-6}$ alkoxy, the $C_{1-6}$ alkoxy is selected from the group consisting of methoxy, ethoxy, n-propoxy, isopropoxy, n-butoxy, isobutoxy, sec-butoxy, tert-butoxy, n-pentoxy, sec-pentoxy, 1-ethylpropoxy, 2-methylbutoxy, tert-pentoxy, 1,2-dimethylpropoxy, isopentoxy, neopentoxy, n-hexoxy, isohexoxy, sec-hexoxy, tert-hexoxy, neohexoxy, 2-methylpentoxy, 1,2-dimethylbutoxy, and 1-ethylbutoxy.

As a preferred technical solution of the present disclosure, in general formula (B), the halogen is selected from the group consisting of fluorine, chlorine, bromine, and iodine. The haloalkyl refers to alkyl in which one or more hydrogens are substituted by halogen; the haloalkoxy refers to alkoxy in which one or more hydrogens are substituted by halogen.

As a preferred technical solution of the present disclosure, in general formula (B), Q is N.

As a preferred technical solution of the present disclosure, in general formula (B), Q is CH, and $R_2$ is F.

As a preferred technical solution of the present disclosure, in general formula (B), $T_1$ is N.

As a preferred technical solution of the present disclosure, in general formula (B), a moiety

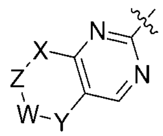

is selected from the group consisting of:

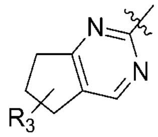, 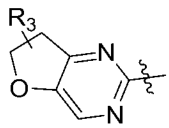, 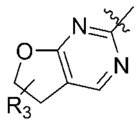,

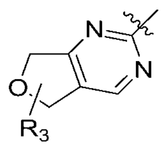, 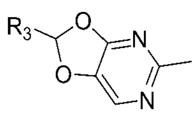, 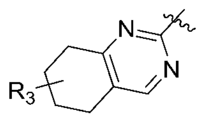, 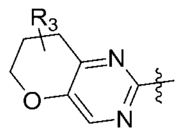, 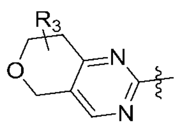, 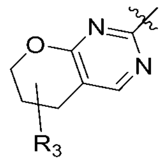,

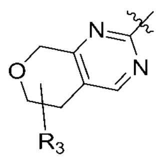, 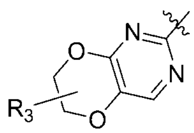, 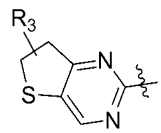, 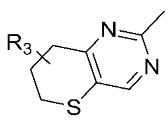, 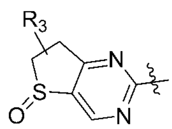, 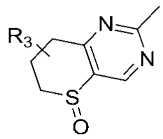,

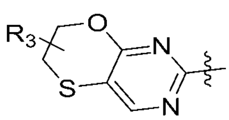, 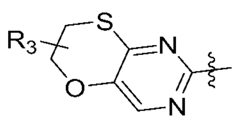, 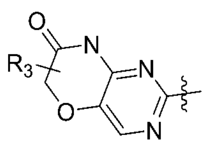, 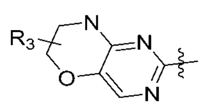, 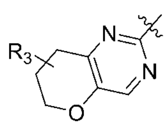,

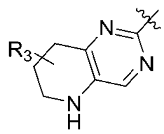, and 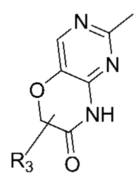,

and 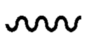 represents a connection site.

As a preferred technical solution of the present disclosure, in general formula (B), a moiety

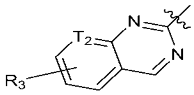

is selected from the group consisting of:

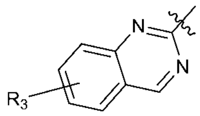 and 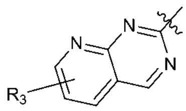,

and ∿∿ represents a connection site.

As a preferred technical solution, $R_3$ is hydrogen, methyl, F, phenyl, or

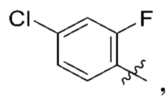,

and ∿∿ represents a connection site.

As a preferred technical solution, A is selected from the group consisting of

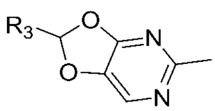 and 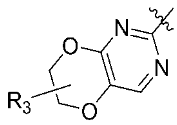;

and

B is selected from the group consisting of

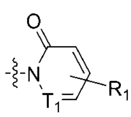, 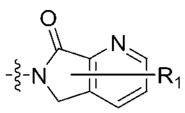, 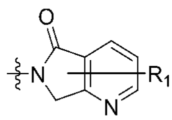,

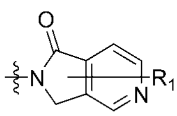, 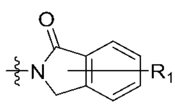, 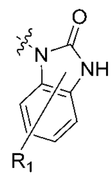, 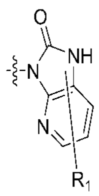, 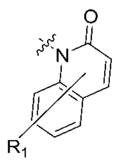, and 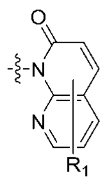.

As a preferred technical solution of the present disclosure, in general formula (B),

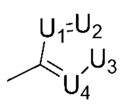

is

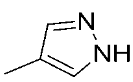.

As a preferred technical solution of the present disclosure, in general formula (B), the compound, or the isomer

thereof, or the racemate thereof, or the pharmaceutically acceptable salt thereof is selected from the group consisting of: B1 to B34.

A compound represented by general formula (C), or an isomer thereof, or a racemate thereof, or a pharmaceutically acceptable salt thereof, wherein:

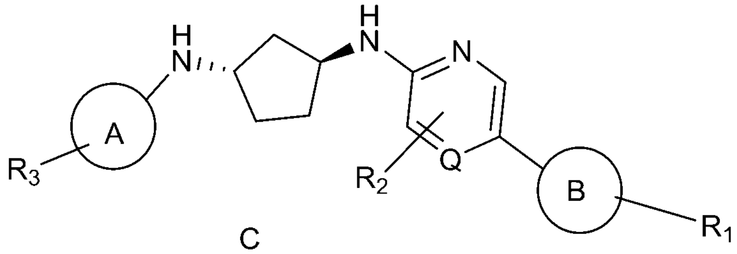

C

ring A is selected from the group consisting of:

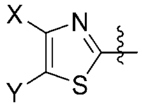, 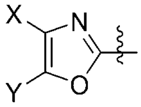, 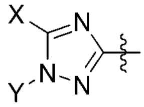,

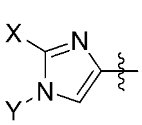, and 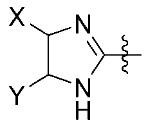,

and X and Y together form a 5- to 7-membered saturated or unsaturated ring, the 5- to 7-membered saturated or unsaturated ring comprises 0, 1, or 2 heteroatoms, the heteroatoms being selected from the group consisting of O, N, and S;

B is selected from the group consisting of

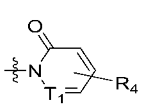, 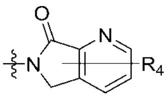, 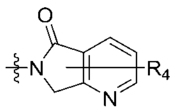,

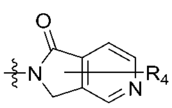, 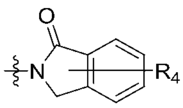, 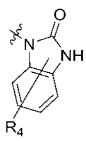, 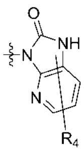, 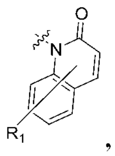, and 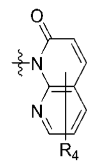;

Q is selected from the group consisting of N and $CR_{Q1}$, $R_{Q1}$ being selected from the group consisting of H and halogen;

$T_1$ is selected from the group consisting of N and CH;

$R_2$ is selected from the group consisting of H, alkyl, and halogen, and a number of $R_2$ is one or more;

$R_3$ is hydrogen, or $R_3$ represents that the hydrogen on ring A is further substituted by oxo, alkyl, halogen, alkoxy, alkylthio, haloalkyl, haloalkoxy, cycloalkyl, cycloalkylalkyl, or alkynyl, and a number of $R_3$ is one or more, or adjacent $R_3$ together form an alkoxy group

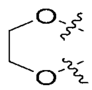;

$R_4$ is selected from the group consisting of hydrogen, halogen, hydroxyl, alkoxy, haloalkoxy, substituted or unsubstituted alkyl, cyano, -C(O)-O-alkyl, phenylalkoxy, carboxyl, hydroxymethyl, and cycloalkyl, a substituent being selected from the group consisting of hydroxyl, amide, halogen, and

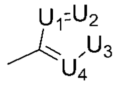 ;

$U_1$, $U_2$, and $U_4$ are independently selected from the group consisting of CH and N; $U_3$ is selected from the group consisting of $CH_2$ and NH; and a number of $R_4$ is one or more; and
when ring A is:

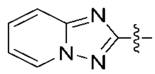 ,

ring B is not:

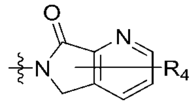 .

As a preferred technical solution of the present disclosure, in general formula (C), the alkyl is selected from the group consisting of methyl, ethyl, n-propyl, isopropyl, n-butyl, isobutyl, sec-butyl, tert-butyl, n-pentyl, sec-pentyl, 1-ethylpropyl, 2-methylbutyl, tert-pentyl, 1,2-dimethylpropyl, isopentyl, neopentyl, n-hexyl, isohexyl, sec-hexyl, tert-hexyl, neohexyl, 2-methylpentyl, 1,2-dimethylbutyl, and 1-ethylbutyl.

As a preferred technical solution of the present disclosure, in general formula (C), the alkoxy is selected from the group consisting of methoxy, ethoxy, n-propoxy, isopropoxy, n-butoxy, isobutoxy, sec-butoxy, and tert-butoxy.

As a preferred technical solution of the present disclosure, in general formula (C), the cycloalkyl is selected from the group consisting of cyclopropyl, cyclobutyl, cyclopentyl, and cyclohexyl.

As a preferred technical solution of the present disclosure, in general formula (C), the halogen is selected from the group consisting of fluorine, chlorine, bromine, and iodine.

As a preferred technical solution of the present disclosure, in general formula (C), Q is N.

As a preferred technical solution of the present disclosure, in general formula (C), Q is CH, and $R_2$ is F.

As a preferred technical solution of the present disclosure, in general formula (C), $T_1$ is N.

As a preferred technical solution of the present disclosure, in general formula (C), $R_3$ is selected from the group consisting of hydrogen, methyl, methoxy, cyclopropyl, cyclopropylmethyl, fluorine, chlorine, oxo, and $CHF_2$-O-.

As a preferred technical solution of the present disclosure, in general formula (C), $R_4$ is selected from the group consisting of hydrogen, methyl, hydroxyl, hydroxymethyl, cyano, F, Cl, Br, -O-$CH_2$-phenyl, -COOH, -$COOCH_2CH_3$,

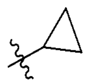 , 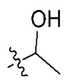 ,

amide, formamide, $CH_3$-C(O)-, ethynyl, trifluoromethyl, difluoromethoxy, and methoxy.

As a preferred technical solution of the present disclosure, in general formula (C), ring A is selected from the group consisting of:

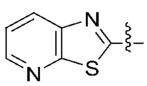 , 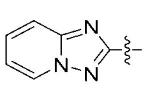 , 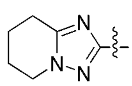 , 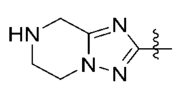 ,

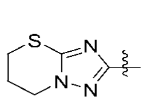 , 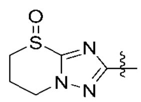 , 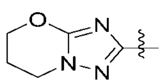 , 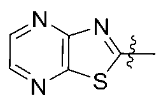 , 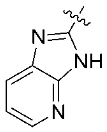 , 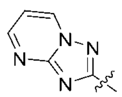 , 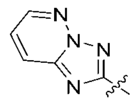 ,

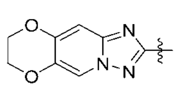 , 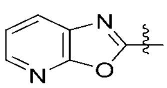 , 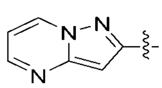 , and 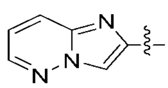 .

Furthermore, as a preferred technical solution of the present disclosure, in general formula (C), $R_3$-substituted ring A is selected from the group consisting of:

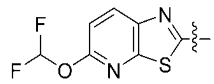, 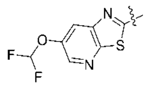,

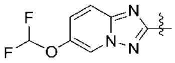, 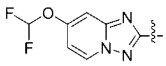, 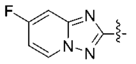, 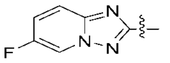, 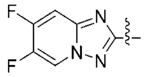, 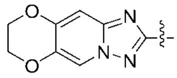,

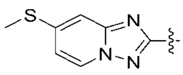, 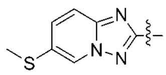, 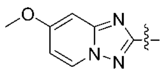, 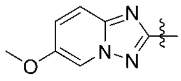, 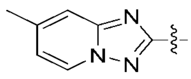, 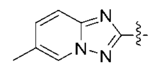,

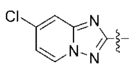, 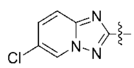, 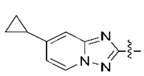, 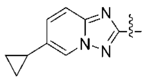, 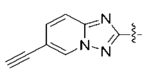, 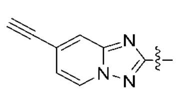, and 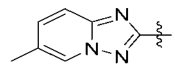.

Furthermore, as a preferred technical solution of the present disclosure, in general formula (C), the $R_4$-substituted ring B is selected from the group consisting of:

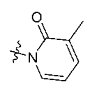, 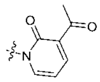, 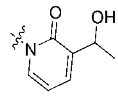,

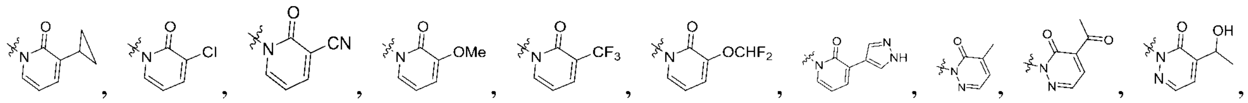,

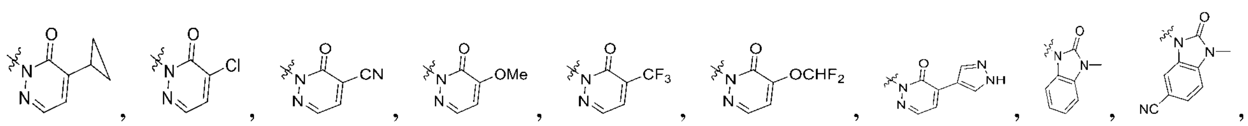,

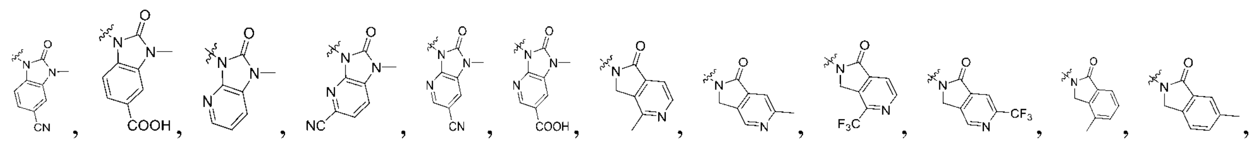,

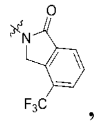, and 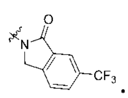.

As a preferred technical solution of the present disclosure, in general formula (C), the compound, or the isomer thereof, or the racemate thereof, or the pharmaceutically acceptable salt thereof is selected from the group consisting of: C1 to C13, and C15 to C95.

A compound represented by general formula (D), or an isomer thereof, or a racemate thereof, or a pharmaceutically acceptable salt thereof, wherein:

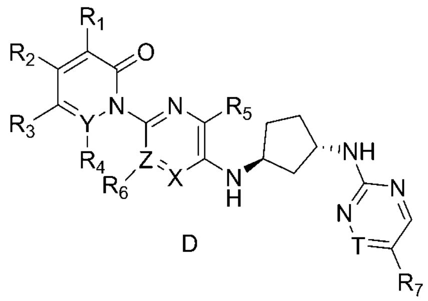

D

$R_1$, $R_2$, $R_3$, and $R_4$ are each independently selected from the group consisting of hydrogen, halogen, alkyl, cyano, haloalkyl, and cycloalkyl;
X is selected from the group consisting of CH and N, Z is selected from the group consisting of CH and N, and when Z is N, $R_6$ is absent;
Y is selected from the group consisting of CH and N, and when Y is N, $R_4$ is absent;
T is selected from the group consisting of CH, N and $CT_1$, and $T_1$ is selected from the group consisting of CN, alkyl, and haloalkyl;
$R_5$ and $R_6$ are each independently selected from the group consisting of hydrogen, halogen, and alkyl, and when X is CH, $R_5$ and $R_6$ are not both hydrogen simultaneously; or when X is CH, $R_1$, $R_2$, $R_3$, $R_4$, $R_5$, and $R_6$ are all hydrogen simultaneously; and
$R_7$ is selected from the group consisting of hydrogen, alkyl, alkoxy, cyano, alkynyl, substituted or unsubstituted cycloalkyl, alkylsulfonyl, alkylphosphonyl, substituted or unsubstituted $-N(C(O))_nX_1X_2$, substituted or unsubstituted -O-cycloalkyl, substituted or unsubstituted -O-heterocycloalkyl, boronic acid group, and halogen, and $X_1$ and $X_2$ are independently selected from the group consisting of alkyl, or $X_1$ and $X_2$ together with the N atom to which they are attached form a heterocycloalkyl, a substituent being selected from the group consisting of hydrogen, halogen, and alkyl, and n = 0 or 1; and when $R_7$ is selected from the group consisting of alkyl, alkoxy, and halogen, $R_5$ and $R_6$ are not both hydrogen simultaneously, or X is N.

As a preferred technical solution, a compound represented by general formula (D1), or an isomer thereof, or a racemate thereof, or a pharmaceutically acceptable salt thereof, wherein:

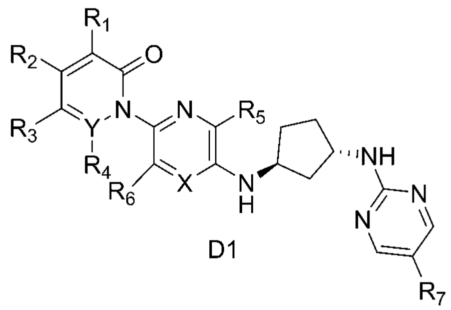

D1

$R_1$, $R_2$, $R_3$, and $R_4$ are each independently selected from the group consisting of hydrogen, halogen, alkyl, cyano, haloalkyl, and cycloalkyl;
X is selected from the group consisting of CH and N;
Y is selected from the group consisting of CH and N, and when Y is N, $R_4$ is absent;
$R_5$ and $R_6$ are each independently selected from the group consisting of hydrogen, halogen, and alkyl, and when X is selected from the group consisting of CH, $R_5$ and $R_6$ are not both hydrogen simultaneously, or when X is CH, $R_1$, $R_2$, $R_3$, $R_4$, $R_5$, and $R_6$ are all hydrogen simultaneously; and
$R_7$ is selected from the group consisting of hydrogen, alkyl, alkoxy, cyano, alkynyl, substituted or unsubstituted cycloalkyl, alkylsulfonyl, alkylphosphonyl, substituted or unsubstituted $-N(C(O))_nX_1X_2$, substituted or unsubstituted -O-cycloalkyl, substituted or unsubstituted -O-heterocycloalkyl, boronic acid group, and halogen, and $X_1$ and $X_2$ are independently selected from the group consisting of alkyl, or $X_1$ and $X_2$ together with the N atom to which they are attached form a heterocycloalkyl, the substituent being selected from the group consisting of hydrogen, halogen, and alkyl, and n = 0 or 1; and when $R_7$ is selected from the group consisting of alkyl, alkoxy, and halogen, $R_5$ and $R_6$ are not both hydrogen simultaneously, or X is N.

As a preferred technical solution of the present disclosure, in general formula (D) or (D1), the alkyl is selected from the group consisting of methyl, ethyl, n-propyl, isopropyl, n-butyl, isobutyl, sec-butyl, tert-butyl, n-pentyl, sec-pentyl, 1-ethylpropyl, 2-methylbutyl, tert-pentyl, 1,2-dimethylpropyl, isopentyl, neopentyl, n-hexyl, isohexyl, sec-hexyl, tert-hexyl, neohexyl, 2-methylpentyl, 1,2-dimethylbutyl, and 1-ethylbutyl.

As a preferred technical solution of the present disclosure, in general formula (D) or (D1), the halogen is selected from the group consisting of fluorine, chlorine, bromine, and iodine.

As a preferred technical solution of the present disclosure, in general formula (D) or (D1), the cycloalkyl is selected from the group consisting of cyclopropyl, cyclobutyl, cyclopentyl, and cyclohexyl.

As a preferred technical solution of the present disclosure, in general formula (D) or (D1), the heterocycloalkyl is cycloalkyl in which one or more carbon atoms are replaced by heteroatoms, the heteroatoms being selected from the group consisting of O, N, and S.

As a preferred technical solution of the present disclosure, in general formula (D) or (D1), the alkynyl is selected from the group consisting of $C_{2-4}$ alkynyl, for example ethynyl, propynyl, and butynyl.

As a preferred technical solution of the present disclosure, in general formula (D) or (D1), the haloalkyl is $-CF_3$.

As a preferred technical solution of the present disclosure, in general formula (D) or (D1), the $R_5$ and $R_6$ are each independently selected from the group consisting of hydrogen, fluorine, and methyl.

As a preferred technical solution of the present disclosure, in general formula (D) or (D1), the $R_7$ is selected from the group consisting of hydrogen, methyl, methoxy, ethoxy, fluorine, ethynyl, chlorine,

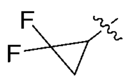 , 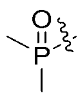 , 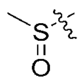 , 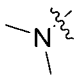 , 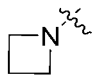 , 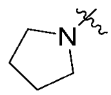 , 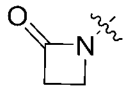 ,

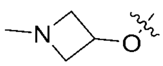 , 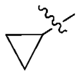 , and 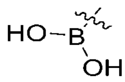 .

As a preferred technical solution of the present disclosure, in general formula (D) or (D1), the $R_1$ is selected from the group consisting of hydrogen, fluorine, chlorine, cyclopropyl, cyano, methyl, and $-CF_3$; $R_2$, $R_3$, and $R_4$ are each independently hydrogen.

As a preferred technical solution of the present disclosure, in general formula (D) or (D1), the X is N.

As a preferred technical solution of the present disclosure, in general formula (D) or (D1), the $R_6$ is selected from the group consisting of hydrogen and F.

As a preferred technical solution of the present disclosure, in general formula (D) or (D1), the compound, or the isomer thereof, or the racemate thereof, or the pharmaceutically acceptable salt thereof is selected from the group consisting of: D1 to D29.

A compound represented by general formula (E), or an isomer thereof, or a racemate thereof, or a pharmaceutically acceptable salt thereof, wherein:

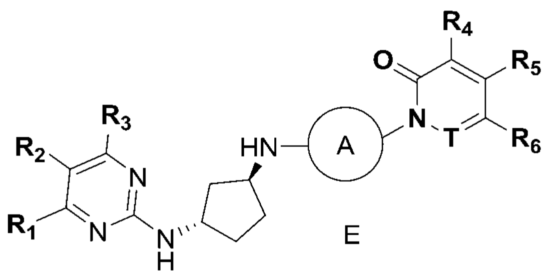

E

ring A is a benzoheterocycle, further selected from the group consisting of benzofuran, benzothiazole, 1H-indole, indole, quinoline, and a structure in which one or more -C= or -CH- in a ring of benzofuran, benzothiazole, 1H-indole, indole, or quinoline is replaced by N atom;

T is selected from the group consisting of N and $CR_7$; and

$R_1$, $R_2$, $R_3$, $R_4$, $R_5$, $R_6$, and $R_7$ are independently selected from the group consisting of hydrogen, halogen, substituted or unsubstituted alkyl, cycloalkyl, and alkoxy, and a substitution being selected from the group consisting of halogen and alkyl.

As a preferred technical solution of the present disclosure, in general formula (E), the alkyl is selected from the group consisting of methyl, ethyl, n-propyl, isopropyl, n-butyl, isobutyl, sec-butyl, tert-butyl, n-pentyl, sec-pentyl, 1-ethylpropyl, 2-methylbutyl, tert-pentyl, 1,2-dimethylpropyl, isopentyl, neopentyl, n-hexyl, isohexyl, sec-hexyl, tert-hexyl, neohexyl, 2-methylpentyl, 1,2-dimethylbutyl, and 1-ethylbutyl.

As a preferred technical solution of the present disclosure, in general formula (E), the halogen is selected from the group consisting of fluorine, chlorine, bromine, and iodine.

As a preferred technical solution of the present disclosure, in general formula (E), the alkoxy is selected from the group consisting of methoxy, ethoxy, n-propoxy, isopropoxy, n-butoxy, isobutoxy, sec-butoxy, tert-butoxy, n-pentoxy, sec-pentoxy, 1-ethylpropoxy, 2-methylbutoxy, tert-pentoxy, 1,2-dimethylpropoxy, isopentoxy, neopentoxy, n-hexoxy, isohexoxy, sec-hexoxy, tert-hexoxy, neohexoxy, 2-methylpentoxy, 1,2-dimethylbutoxy, 1-ethylbutoxy, n-heptoxy, and isoheptoxy.

As a preferred technical solution of the present disclosure, in general formula (E), the cycloalkyl is selected from the group consisting of cyclopropyl, cyclobutyl, cyclopentyl, and cyclohexyl; the heterocycloalkyl refers to cycloalkyl in which at least one carbon atom is replaced by a heteroatom, the heteroatom being selected from the group consisting of nitrogen, oxygen, and sulfur.

As a preferred technical solution of the present disclosure, in general formula (E), T is N.

As a preferred technical solution of the present disclosure, in general formula (E), ring A is selected from the group consisting of:

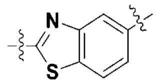, 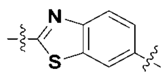, 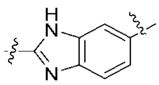, 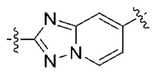,

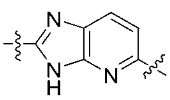, 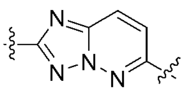, 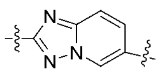, 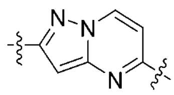, 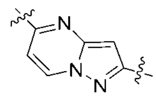, 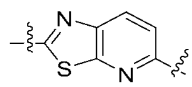,

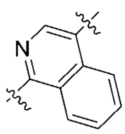, 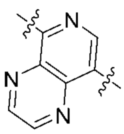, 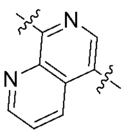, 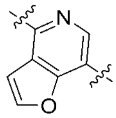, 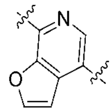, 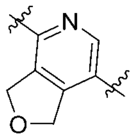, and 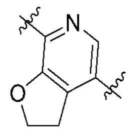.

As a preferred technical solution of the present disclosure, in general formula (E), $R_1$ and $R_3$ are hydrogen, and $R_2$ is $-OCHF_2$.

As a preferred technical solution of the present disclosure, in general formula (E), $R_4$, $R_5$, and $R_6$ are hydrogen.

As a preferred technical solution of the present disclosure, in general formula (E), the compound, or the isomer thereof, or the racemate thereof, or the pharmaceutically acceptable salt thereof is selected from the group consisting of: E1 to E18.

A compound represented by general formula (F), or an isomer thereof, or a racemate thereof, or a pharmaceutically acceptable salt thereof, wherein:

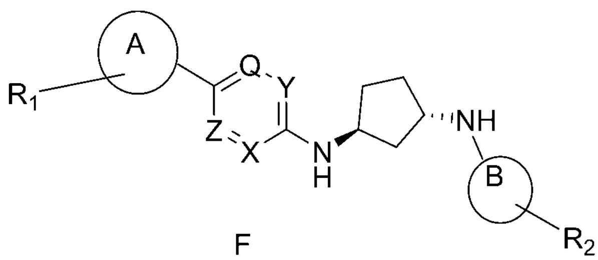

F

ring A is selected from the group consisting of:

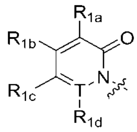, 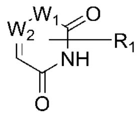,

and $R_1$-substituted or unsubstituted

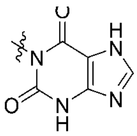, 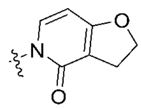, 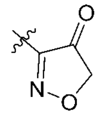, 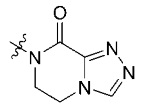, 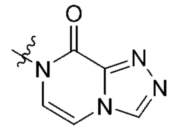, 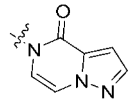,

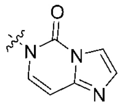, 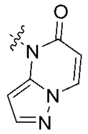, 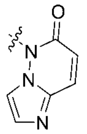, 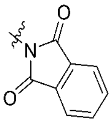, 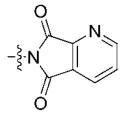, 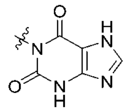, 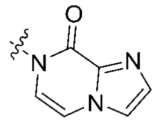, 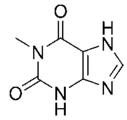,

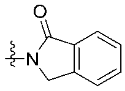, 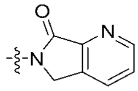, 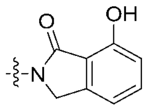, and 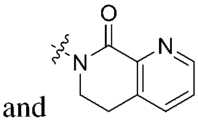;

ring B is selected from the group consisting of

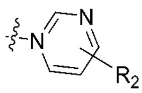, 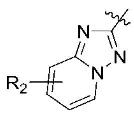, and 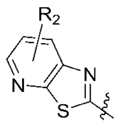;

$R_1$, $R_{1a}$, $R_{1b}$, $R_{1c}$, $R_{1d}$ are each independently selected from the group consisting of hydrogen, oxo, hydroxyl, alkynyl, alkylalkynyl, alkylalkynylalkyl, alkyl, halogen, alkoxy, alkylthio, haloalkyl, haloalkoxy, cycloalkyl, cycloalkylalkyl, cyano, and

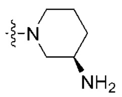,

or two adjacent substituents together form a 5- to 6-membered saturated or unsaturated heterocycle;
Q, X, Y, Z are each independently selected from the group consisting of $CR_3$ and N, $R_3$ being independently selected from the group consisting of hydrogen, oxo, alkyl, halogen, alkoxy, alkylthio, haloalkyl, haloalkoxy, cycloalkyl, cycloalkylalkyl, and cyano, and when A is

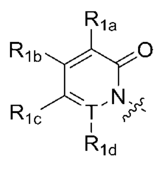,

at least two of Q, X, Y, Z are N;
T is each independently selected from the group consisting of CH and N; and
$W_1$ is selected from the group consisting of $CH_2$ and NH, and $W_2$ is each independently selected from the group consisting of CH and N.

As a preferred technical solution of the present disclosure, in general formula (F), the alkyl is selected from the group consisting of methyl, ethyl, n-propyl, isopropyl, n-butyl, isobutyl, sec-butyl, tert-butyl, n-pentyl, sec-pentyl, 1-ethylpropyl, 2-methylbutyl, tert-pentyl, 1,2-dimethylpropyl, isopentyl, neopentyl, n-hexyl, isohexyl, sec-hexyl, tert-hexyl, neohexyl, 2-methylpentyl, 1,2-dimethylbutyl, and 1-ethylbutyl.

As a preferred technical solution of the present disclosure, in general formula (F), the alkoxy is selected from the group consisting of methoxy, ethoxy, n-propoxy, isopropoxy, n-butoxy, isobutoxy, sec-butoxy, and tert-butoxy; the alkylthio refers to alkoxy in which the oxygen atom is replaced by a sulfur atom.

As a preferred technical solution of the present disclosure, in general formula (F), the halogen is selected from the group consisting of fluorine, chlorine, bromine, and iodine.

As a preferred technical solution of the present disclosure, in general formula (F), the cycloalkyl is selected from the group consisting of cyclopropyl, cyclobutyl, cyclopentyl, and cyclohexyl; and heteroatoms are selected from the group consisting of O, N, and S.

As a preferred technical solution of the present disclosure, in general formula (F), the Q is N.

As a preferred technical solution of the present disclosure, in general formula (F), $R_2$ is -O-$CHF_2$.

As a preferred technical solution of the present disclosure, in general formula (F), ring A is selected from the group consisting of

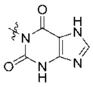, 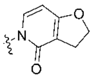, 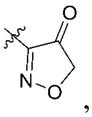, 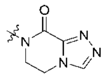, 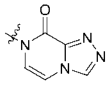, 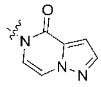,

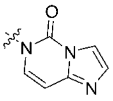, 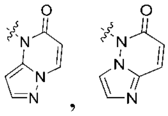, 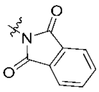, 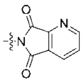, 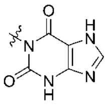, 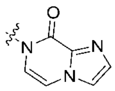, 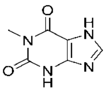, 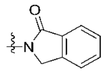, 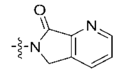,

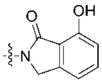, and 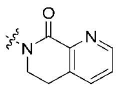;

and substituted ring A is selected from the group consisting of

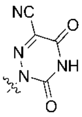,

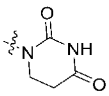, 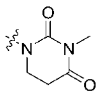, 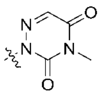, 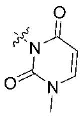, 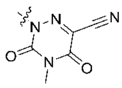, 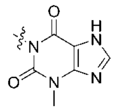, 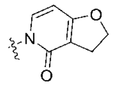, 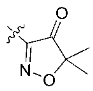, 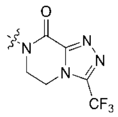,

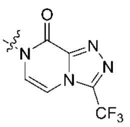, 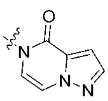, 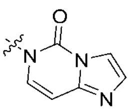, 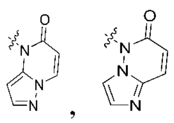, 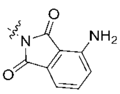, 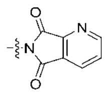, 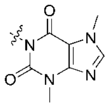, 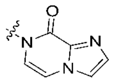,

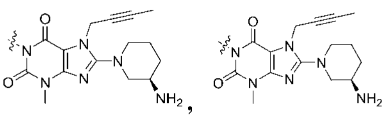, 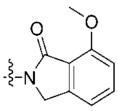, 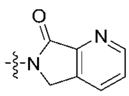, 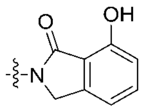, and 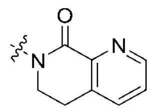;

or

ring A is selected from the group consisting of

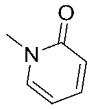 and 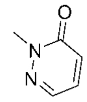,

and substituted ring A is selected from the group consisting of

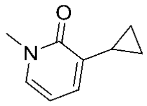 and 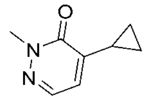.

As a preferred technical solution of the present disclosure, in general formula (F), the compound, or the isomer thereof, or the racemate thereof, or the pharmaceutically acceptable salt thereof is selected from the group consisting of: F1 to F37.

A compound represented by general formula (G), or an isomer thereof, or a racemate thereof, or a pharmaceutically acceptable salt thereof, wherein:

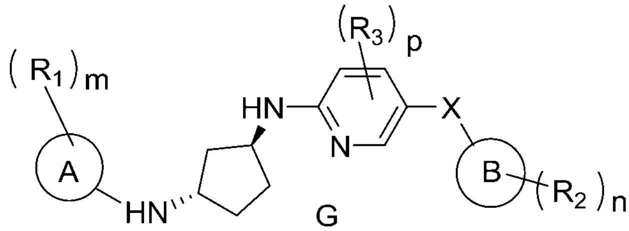

G

X is selected from the group consisting of a substituted or unsubstituted alkyl, amino, O, and S, and a substituent is selected from the group consisting of oxo, alkyl, halogen, alkoxy, hydroxyl, and alkoxycarbonyl;

ring A is $R_1$-substituted or unsubstituted 5- to 14-membered saturated or unsaturated heterocycle, and the heterocycle can be monocyclic, bicyclic, or tricyclic;

$R_1$ is independently selected from the group consisting of hydrogen, oxo, alkyl, halogen, alkoxy, alkylthio, haloalkyl, haloalkoxy, cycloalkyl, cycloalkylalkyl, and cyano, and m = 0, 1, 2, or 3;

ring B is $R_2$-substituted or unsubstituted 5- to 7-membered saturated or unsaturated heterocycle;

$R_2$ is independently selected from the group consisting of hydrogen, oxo, thio, alkyl, halogen, alkoxy, alkylthio, haloalkyl, haloalkoxy, cycloalkyl, cycloalkylalkyl, and cyano, and n = 0, 1, 2, or 3;

$R_3$ are each selected from the group consisting of hydrogen, alkyl, alkoxy, cyano, halogen, haloalkyl, amide, hydroxyl, hydroxyl-substituted alkyl, carboxyl, and amino, and p = 0, 1, 2, or 3.

As a preferred technical solution of the present disclosure, the alkyl is selected from the group consisting of methyl, ethyl, n-propyl, isopropyl, n-butyl, isobutyl, sec-butyl, tert-butyl, n-pentyl, sec-pentyl, 1-ethylpropyl, 2-methylbutyl, tert-pentyl, 1,2-dimethylpropyl, isopentyl, neopentyl, n-hexyl, isohexyl, sec-hexyl, tert-hexyl, neohexyl, 2-methylpentyl, 1,2-dimethylbutyl, and 1-ethylbutyl.

As a preferred technical solution of the present disclosure, in general formula (G), the alkoxy is selected from the group consisting of methoxy, ethoxy, n-propoxy, isopropoxy, n-butoxy, isobutoxy, sec-butoxy, and tert-butoxy.

As a preferred technical solution of the present disclosure, in general formula (G), the alkylthio is selected from the group consisting of methylthio, ethylthio, propylthio, isopropylthio, n-butylthio, isobutylthio, sec-butylthio, and tert-butylthio.

As a preferred technical solution of the present disclosure, in general formula (G), the cycloalkyl is selected from the group consisting of cyclopropyl, cyclobutyl, cyclopentyl, and cyclohexyl.

As a preferred technical solution of the present disclosure, in general formula (G), the halogen is selected from the group consisting of fluorine, chlorine, bromine, and iodine.

As a preferred technical solution of the present disclosure, ring A is selected from the group consisting of

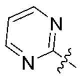, 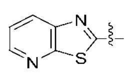, 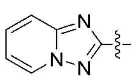, 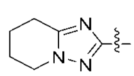, 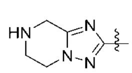, 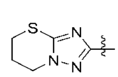, 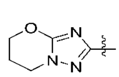,

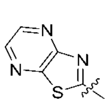, 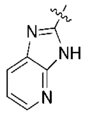, 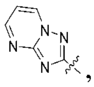, 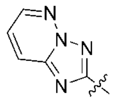, 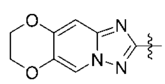, 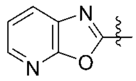, 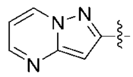, 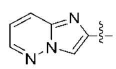, 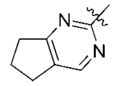, 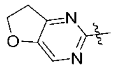, 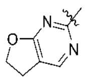, 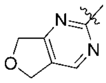, 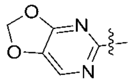, 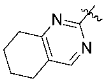, 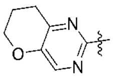, 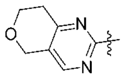, 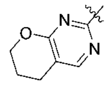, 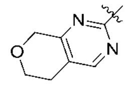, 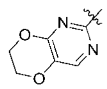, 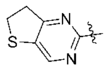, 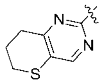, 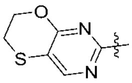, 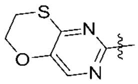, 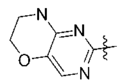, 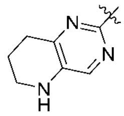, and 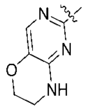.

As a preferred technical solution of the present disclosure, in general formula (G), ring B is selected from the group consisting of:

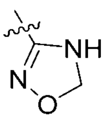, 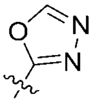, 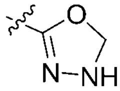, 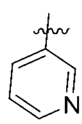, 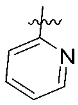, 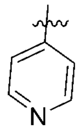, 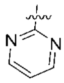, 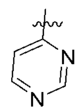, 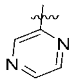, 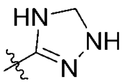, 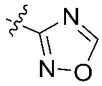, 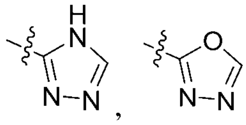, 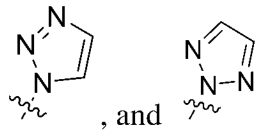, and .

As a preferred technical solution of the present disclosure, in general formula (G), the compound represented by (G1), or an isomer thereof, or a racemate thereof, or a pharmaceutically acceptable salt thereof, is selected:

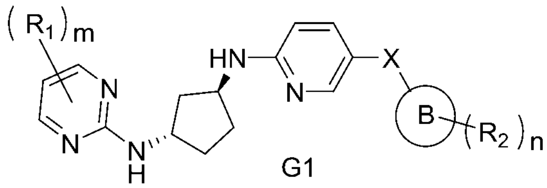

G1

.

As a preferred technical solution of the present disclosure, in general formula (G) or (G1), $R_1$ is selected from the group consisting of hydrogen, methyl, cyano, methoxy, cyclopropyl, cyclopropylmethyl, fluorine, chlorine, and $CHF_2$-O-.

As a preferred technical solution of the present disclosure, in general formula (G) or (G1), $R_2$ is selected from the group consisting of hydrogen, methyl, oxo, and thio.

As a preferred technical solution of the present disclosure, in general formula (G) or (G1), $R_3$ is selected from the group consisting of hydrogen, methyl, cyano, methoxy, cyclopropyl, cyclopropylmethyl, fluorine, and chlorine.

As a preferred technical solution of the present disclosure, in general formula (G) or (G1), X is selected from the group consisting of $-CH_2-$, -C(O)-, -CH(OH)-, $-NH_2-$, $-NH(CH_3)-$, , O, and S.

As a preferred technical solution of the present disclosure, in general formula (G) or (G1), the compound, or the isomer thereof, or the racemate thereof, or the pharmaceutically acceptable salt thereof is selected from the group consisting of: G1 to G21.

A compound represented by general formula (H), or an isomer thereof, or a racemate thereof, or a pharmaceutically acceptable salt thereof, wherein:

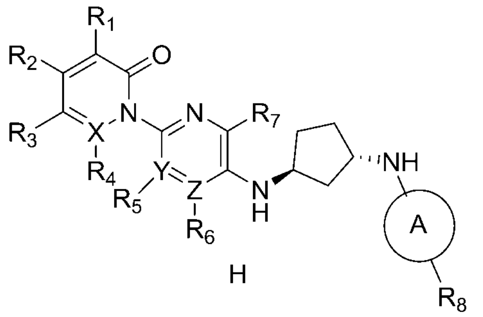

H

$R_1$, $R_2$, $R_3$, and $R_4$ are each independently selected from the group consisting of hydrogen, halogen, alkyl, cyano, haloalkyl, and cycloalkyl;
X is selected from the group consisting of C and N, and when X is N, $R_5$ is absent;
Y is selected from the group consisting of C and N, and when Y is N, $R_6$ is absent;
Z is selected from the group consisting of C and N, and when Z is N, $R_7$ is absent;
$R_5$, $R_6$, and $R_7$ are each independently selected from the group consisting of hydrogen, halogen, and alkyl;
a number of $R_8$ is one or more, which is independently selected from the group consisting of hydrogen, halogen, phenyl, substituted or unsubstituted alkyl, substituted or unsubstituted cycloalkylalkyl, and substituted or unsubstituted cycloalkyl, a substituent being selected from the group consisting of alkyl and halogen;
ring A is selected from the group consisting of

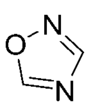 , 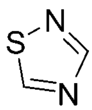 , and 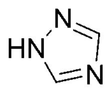 ,

and when A is

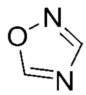 ,

and X, Y, and Z are all C, $R_1$, $R_2$, $R_3$, $R_4$, $R_5$, $R_6$, and $R_7$ are not all hydrogen simultaneously.

As a preferred technical solution of the present disclosure, in general formula (H), the alkyl is selected from the group consisting of methyl, ethyl, n-propyl, isopropyl, n-butyl, isobutyl, sec-butyl, tert-butyl, n-pentyl, sec-pentyl, 1-ethylpropyl, 2-methylbutyl, tert-pentyl, 1,2-dimethylpropyl, isopentyl, neopentyl, n-hexyl, isohexyl, sec-hexyl, tert-hexyl, neohexyl, 2-methylpentyl, 1,2-dimethylbutyl, and 1-ethylbutyl.
As a preferred technical solution of the present disclosure, in general formula (H), the halogen is selected from the group consisting of fluorine, chlorine, bromine, and iodine.
As a preferred technical solution of the present disclosure, in general formula (H), the cycloalkyl is selected from the group consisting of cyclopropyl, cyclobutyl, cyclopentyl, and cyclohexyl.
As a preferred technical solution of the present disclosure, in general formula (H), $R_1$ is selected from the group consisting of halogen and alkyl.
As a preferred technical solution of the present disclosure, in general formula (H), Y is N, or Y is C, and $R_5$ is selected from the group consisting of halogen and alkyl.
As a preferred technical solution of the present disclosure, in general formula (H), $R_8$ is selected from the group consisting of methyl, cyclopropyl, cyclobutyl,

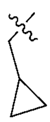 , 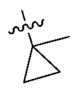 , 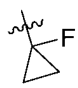 ,

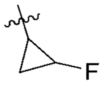, and 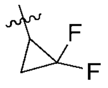.

As a preferred technical solution of the present disclosure, in general formula (H), Z is N.

As a preferred technical solution of the present disclosure, in general formula (H),

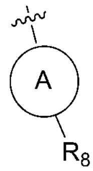

is selected from the group consisting of

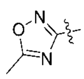, 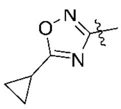, 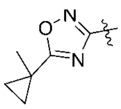, 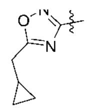, 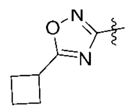,

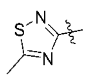, 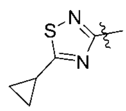, 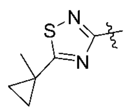, 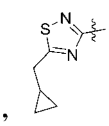, 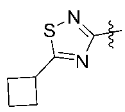, 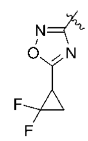, 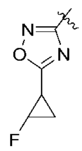, 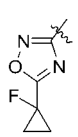, 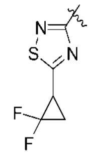, 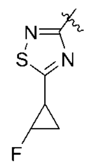,

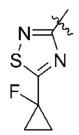, 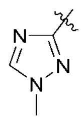, 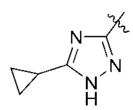, 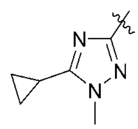, 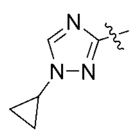, and 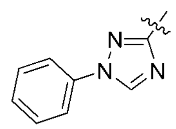.

As a preferred technical solution of the present disclosure, in general formula (H), the compound, or an isomer thereof, or a racemate thereof, or a pharmaceutically acceptable salt thereof is selected from the group consisting of: H1 to H14.

A compound represented by general formula (J), or an isomer thereof, or a racemate thereof, or a pharmaceutically acceptable salt thereof, wherein:

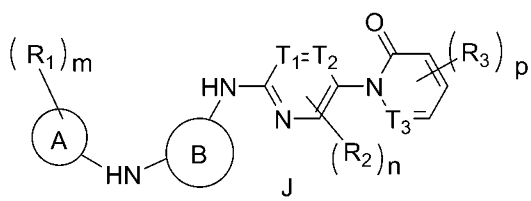

J

ring A is $R_1$-substituted or unsubstituted 5- to 14-membered saturated or unsaturated heterocycle, and the heterocycle can be monocyclic, bicyclic, or tricyclic;

$R_1$ is independently selected from the group consisting of hydrogen, oxo, alkyl, halogen, alkoxy, alkylthio, haloalkyl, haloalkoxy, cycloalkyl, cycloalkylalkyl, and cyano, and m = 0, 1, 2, or 3;

ring B is selected from the group consisting of a 5- to 8-membered saturated or unsaturated monocyclic and bicyclic ring, and is not

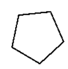;

wherein bicyclic and tricyclic rings may be spirocyclic or bridged rings;

$R_2$ is independently selected from the group consisting of hydrogen, oxo, thio, alkyl, halogen, alkoxy, alkylthio, haloalkyl, haloalkoxy, cycloalkyl, cycloalkylalkyl, and cyano, and n = 0, 1, 2, or 3;
$R_3$ is independently selected from the group consisting of hydrogen, halogen, hydroxyl, substituted or unsubstituted alkyl, cyano, -C(O)-O-alkyl, phenylalkoxy, carboxyl, hydroxymethyl, cycloalkyl, alkyl, amide, alkyl-substituted amide, Z-C(O)-, aryl, and heteroaryl, Z being selected from the group consisting of alkyl and heterocycloalkyl, a substituent is hydroxyl, and p = 0, 1, 2, or 3; and
$T_1$, $T_2$, and $T_3$ are independently selected from the group consisting of CH and N.

As a preferred technical solution of the present disclosure, in general formula (J), the alkyl is selected from the group consisting of methyl, ethyl, n-propyl, isopropyl, n-butyl, isobutyl, sec-butyl, tert-butyl, n-pentyl, sec-pentyl, 1-ethylpropyl, 2-methylbutyl, tert-pentyl, 1,2-dimethylpropyl, isopentyl, neopentyl, n-hexyl, isohexyl, sec-hexyl, tert-hexyl, neohexyl, 2-methylpentyl, 1,2-dimethylbutyl, and 1-ethylbutyl.
As a preferred technical solution of the present disclosure, in general formula (J), the alkoxy is selected from the group consisting of methoxy, ethoxy, n-propoxy, isopropoxy, n-butoxy, isobutoxy, sec-butoxy, and tert-butoxy.
As a preferred technical solution of the present disclosure, in general formula (J), the alkylthio is selected from the group consisting of methylthio, ethylthio, propylthio, isopropylthio, n-butylthio, isobutylthio, sec-butylthio, and tert-butylthio.
As a preferred technical solution of the present disclosure, in general formula (J), the cycloalkyl is selected from the group consisting of cyclopropyl, cyclobutyl, cyclopentyl, and cyclohexyl.
As a preferred technical solution of the present disclosure, in general formula (J), the halogen is selected from the group consisting of fluorine, chlorine, bromine, and iodine.
As a preferred technical solution of the present disclosure, in general formula (J), ring A is selected from

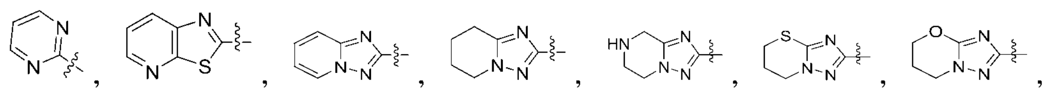

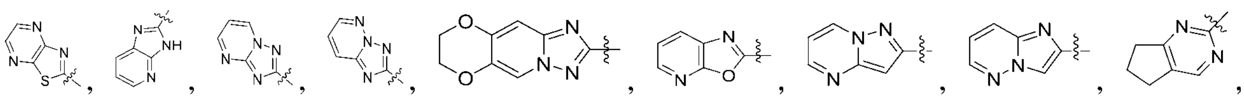

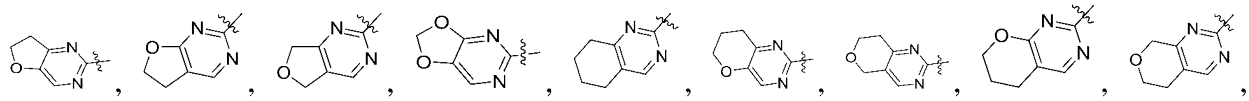

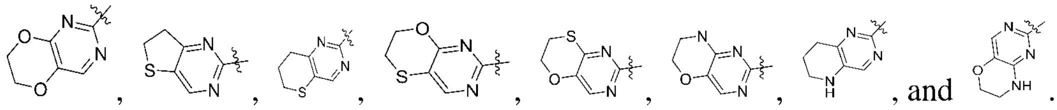

As a preferred technical solution of the present disclosure, in general formula (J), ring B is selected from

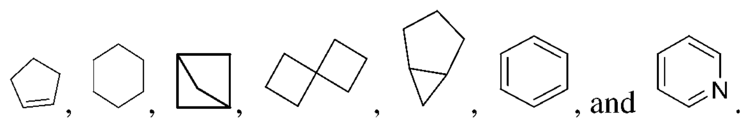

As a preferred technical solution of the present disclosure, in general formula (J), $R_1$ is selected from the group consisting of hydrogen, methyl, cyano, methoxy, cyclopropyl, cyclopropylmethyl, fluorine, chlorine, and $CHF_2$-O-.
As a preferred technical solution of the present disclosure, in general formula (J), $R_2$ is selected from the group consisting of hydrogen, methyl, cyano, methoxy, cyclopropyl, cyclopropylmethyl, fluorine, and chlorine.
As a preferred technical solution of the present disclosure, in general formula (J), ring B is connected in the following way:

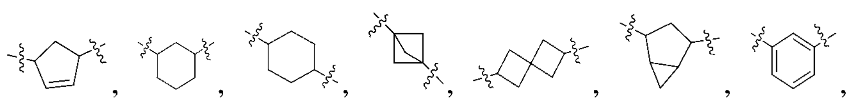

or

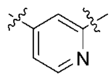

.

As a preferred technical solution of the present disclosure, in general formula (J), the compound, or an isomer thereof, or a racemate thereof, or a pharmaceutically acceptable salt thereof is selected from the group consisting of: J1 to J9.

The present disclosure further provides a pharmaceutical composition, comprising a therapeutically effective amount of the compound, or the isomer thereof, or the racemate thereof, or the pharmaceutically acceptable salt thereof, and a pharmaceutically acceptable carrier.

The present disclosure further provides use of the compound, or the isomer thereof, or the racemate thereof, or the pharmaceutically acceptable salt thereof according to any one of claims 1 to 47 in the manufacture of a medicament for treating a PCSK9 inhibitor-related disease; preferably, the PCSK9 inhibitor-related disease is hypercholesterolemia.

## DETAILED DESCRIPTION

For clarity, general terms used in the description of the compound are defined herein.

Unless otherwise indicated, the following terms and phrases used herein are intended to have the following meanings. A specific term or phrase that is not specifically defined should not be considered indefinite or unclear, but should be understood according to its ordinary meaning. When a trade name appears herein, it is intended to refer to the corresponding commercial product or its active ingredient. The term "pharmaceutically acceptable" as used here refers to those compounds, materials, compositions, and/or dosage forms that are, within the scope of sound medical judgment, suitable for use in contact with human and animal tissues without undue toxicity, irritation, allergic reactions, or other problems or complications, and have a reasonable benefit/risk ratio.

The term "pharmaceutically acceptable salt" refers to a salt of the compound of the present disclosure, prepared from the compound of the present disclosure, which has specific substituents as discovered, with a pharmaceutically acceptable acid or base.

In addition to a salt form, the compound provided by the present disclosure may also exist in a prodrug form. The prodrug of the compound described herein readily undergoes chemical changes under physiological conditions to convert into the compound of the present disclosure. Furthermore, the prodrug can be converted into the compound of the present disclosure through chemical or biochemical methods in an in vivo environment.

Certain compound of the present disclosure may exist in an unsolvated form or solvated form, comprising a hydrated form. In general, the solvated form are equivalent to the unsolvated form, and both are encompassed within the scope of the present disclosure.

The compound of the present disclosure may exist in a specific geometric or stereoisomeric form. The present disclosure contemplates all such compounds, comprising cis and trans isomers, (-)- and (+)- enantiomers, (R)- and (S)-enantiomers, diastereomers, (D)-isomers, (L)-isomers, atropisomers, and their racemic mixtures and other mixtures, such as enantiomerically or diastereomerically enriched mixtures, all of which are encompassed within the scope of the present disclosure. Additional asymmetric carbon atoms may be present in substituents such as alkyl groups. All such isomers and mixtures thereof are encompassed within the scope of the present disclosure.

Optically active (R)- and (S)- isomers, as well as D and L isomers, atropisomers, etc., can be prepared by chiral synthesis or chiral reagents, or by other conventional techniques. If one enantiomer of a compound of the present disclosure is desired, it can be prepared by asymmetric synthesis or by derivation with a chiral auxiliary, wherein the resulting diastereomeric mixture is separated, and the auxiliary group is cleaved to provide the pure desired enantiomer. Alternatively, when the molecule comprises a basic functional group (such as amino) or an acidic functional group (such as carboxyl), diastereomeric salts are formed with an appropriate optically active acid or base, then resolved by conventional methods known in the art to obtain the pure enantiomers, which are then recovered. Furthermore, the separation of enantiomers and diastereomers is usually accomplished using chromatography with a chiral stationary phase, optionally combined with chemical derivatization (e.g., generation of carbamates from amines).

An atom in the molecule of the compound of the present disclosure can be an isotope. Isotopic derivatization often can extend half-life, reduce clearance, improve metabolic stability, and enhance in vivo activity. Moreover, included is an embodiment wherein at least one atom is replaced by an atom having the same atomic number (number of protons) but a different mass number (sum of protons and neutrons). Examples of isotopes comprised in the compounds of the present disclosure comprise hydrogen atom, carbon atom, nitrogen atom, oxygen atom, phosphorus atom, sulfur atom, fluorine atom, chlorine atom, which comprise $^{2}H$, $^{3}H$, $^{13}C$, $^{14}C$, $^{15}N$, $^{17}O$, $^{13}O$, $^{31}P$, $^{32}P$, $^{35}S$, $^{18}F$, and $^{36}Cl$, respectively. In particular, a radioactive isotope that emits radiation upon decay, such as $^{3}H$ or $^{14}C$, can be used for drug formulation or anatomical

testing of the compound in vivo. A stable isotope neither decays nor changes in quantity over time and is non-radioactive, thus it can be used safely. When an atom constituting the molecule of the compound of the present disclosure is an isotope, isotopic conversion can be performed according to general methods by replacing the reagent used in the synthesis with a corresponding isotope-containing reagent.

The compound of the present disclosure may comprise a non-natural proportion of atomic isotopes at one or more atoms constituting the compound. For example, the compound can be labeled with radioactive isotopes, such as deuterium ($^2H$), iodine-125 ($^{125}I$), or carbon-14 ($^{14}C$). All isotopic variations of the compound of the present disclosure, whether radioactive or not, are encompassed within the scope of the present disclosure.

Furthermore, in the compound of the present disclosure, one or more hydrogen atoms are substituted by the isotope deuterium ($^2H$). Deuterium substitution of the compound of the present disclosure results in effects such as extended half-life, reduced clearance, improved metabolic stability, and enhanced in vivo activity.

The preparation method for such isotopic derivatives generally comprises: phase transfer catalysis methods. For example, a preferred deuteration method employs a phase transfer catalyst (e.g., tetraalkylammonium salt, $NBu_4HSO_4$). Using a phase transfer catalyst to exchange the methylene protons of a diphenylmethane compound leads to higher deuterium incorporation compared to reduction with a deuterated silane (e.g., triethylsilyl deuteride) in the presence of an acid (e.g., methanesulfonic acid) or with sodium borodeuteride using a Lewis acid such as aluminum trichloride.

The term "pharmaceutically acceptable carrier" refers to any formulation carrier or medium capable of delivering an effective amount of the active substance of the present disclosure without interfering with the biological activity of the active substance and without toxic side effects to the host or patient. Representative carriers comprise water, oils, vegetable and mineral substances, cream bases, lotion bases, ointment bases, etc. These bases comprise suspending agents, viscosity-increasing agents, transdermal enhancers, etc. Their formulations are well known to those skilled in the art of cosmetics or topical pharmaceuticals. For additional information on carriers, reference can be made to Remington: The Science and Practice of Pharmacy, 21st Ed., Lippincott, Williams & Wilkins (2005), the content of which is incorporated herein by reference.

The term "excipient" generally refers to carriers, diluents, and/or media required for formulating an effective pharmaceutical composition.

With respect to a drug or pharmacologically active agent, the term "effective amount" or "therapeutically effective amount" refers to a sufficient amount of the drug or agent that is non-toxic but achieves the desired effect. For oral dosage forms in the present disclosure, the "effective amount" of an active substance in the composition refers to the amount required to achieve the desired effect when used in combination with another active substance in the composition. The determination of an effective amount varies from person to person, depending on the age and general condition of the recipient, as well as the specific active substance. A suitable effective amount in an individual case can be determined by those skilled in the art through routine experimentation.

The terms "active ingredient," "therapeutic agent," "active substance," or "active agent" refer to a chemical entity that can effectively treat a targeted disorder, disease, or condition.

"Optional" or "optionally" means that the subsequently described event or circumstance may, but does not have to, occur, and the description includes situations where the event or circumstance occurs and situations where it does not occur.

The compound of the present disclosure can be prepared by various synthetic methods well known to those skilled in the art, including the specific embodiments listed below, embodiments formed by combining these with other chemical synthesis methods, and equivalent replacement methods well known to those skilled in the art. Preferred embodiments include, but are not limited to, the examples of the present disclosure.

## EXAMPLES

The present application is further described in detail below with reference to examples, but the embodiments of the present application are not limited thereto.

Example A1

Synthesis of 6'-(((1S,3S)-3-((5-(difluoromethoxy)pyrimidin-2-yl)amino)cyclopentyl)amino)-3-methyl-2H-[1, 3'-bipyridin]-2-one

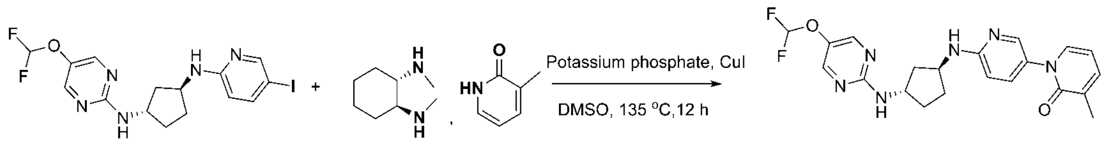


At room temperature, (1S,3S)-$N^1$-(5-(difluoromethoxy)pyrimidin-2-yl)-$N^3$-(5-iodopyridin-2-yl)cyclopentane-1,3-diami ne (300 mg, 0.67 mmol), 3-methylpyridin-2(1H)-one (146 mg, 1.34 mmol), (1S,2S)-$N^1$,$N^2$-dimethylcyclohexane-1,2-diamine (19.0 mg, 0.13 mmol), and potassium phosphate (284.5 mg, 1.34 mmol) were added to DMSO (8 mL). Subsequently, copper(I) iodide (38.2 mg, 0.20 mmol) was added. After completion of addition, the system was purged with nitrogen, and heated to 140 °C and reacted for 12 hours.

After completion of the reaction, the mixture was cooled to room temperature, and water (40 mL) was added to the system. The mixture was extracted with ethyl acetate (20 mL × 3). The organic phases were combined, washed with saturated brine (30 mL), dried, filtered, and concentrated under reduced pressure to dryness to obtain a residue. The residue was purified by normal-phase column chromatography (dichloromethane/methanol = 10:1) to obtain 80 mg of a white product, 6'-(((1S,3S)-3-((5-(difluoromethoxy)pyrimidin-2-yl)amino)cyclopentyl)amino)-3-methyl-2H-[1, 3'-bipyridin]-2-one (yield: 27.8%).

LC-MS: $[M+H]^+$ = 429. $^1H$ NMR (400 MHz, Methanol-$d_4$) δ 8.20-8.18 (s, 2H), 7.96-7.93 (d, J = 2.6 Hz, 1H), 7.52-7.43 (m, 3H), 6.91-6.52 (m, 2H), 6.42-6.37 (t, J = 6.8 Hz, 1H), 4.48-4.30 (m, 2H), 2.36-2.24 (m, 2H), 2.18-2.14 (s, 3H), 2.08-1.97 (m, 2H), 1.71-1.56 (m, 2H).

Example A2

Synthesis of 6'-(((1S,3S)-3-((5-(difluoromethoxy)pyrimidin-2-yl)amino)cyclopentyl)amino)-5-methyl-2H-[1, 3'-bipyridin]-2-one

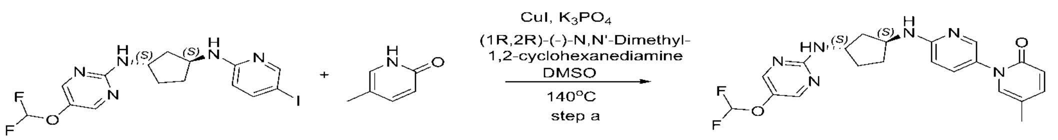


(1S,3S)-$N^1$-(5-(difluoromethoxy)pyrimidin-2-yl)-$N^3$-(5-iodopyridin-2-yl)cyclopentane-1,3-diamine (300 mg, 0.67 mmol) was dissolved in DMSO (5 mL). 5-Methylpyridin-2(1H)-one (73 mg, 0.67 mmol), copper(I) iodide (25 mg, 0.134 mmol), potassium phosphate (284 mg, 1.34 mmol), and (1R,2R)-(-)-N,N'-dimethyl-1,2-cyclohexanediamine (19 mg, 0.134 mmol) were added. Under nitrogen protection, the mixture was reacted at 140 °C for 13 hours.

After completion of the reaction, the mixture was cooled to room temperature, quenched with 10 mL of water, and extracted with ethyl acetate (50 mL × 3). The organic phases were combined, dried over anhydrous sodium sulfate, filtered, and concentrated. The resulting residue was purified by $C_{18}$ column chromatography (water/acetonitrile = 95/5 → acetonitrile) to obtain 130 mg of 6'-(((1S,3S)-3-((5-(difluoromethoxy)pyrimidin-2-yl)amino)cyclopentyl)amino)-5-methyl-2H-[1, 3'-bipyridin]-2-one (yield: 45%).

LC-MS: $[M+H]^+$ = 429. $^1H$ NMR (400 MHz, DMSO-$d_6$) δ 8.23 (s, 2H), 7.90 (d, J = 2.7 Hz, 1H), 7.50 (d, J = 7.2 Hz, 1H), 7.43-7.31 (m, 3H), 7.27-6.83 (m, 2H), 6.51 (d, J = 8.9 Hz, 1H), 6.38 (d, J = 9.2 Hz, 1H), 4.38-4.22 (m, 2H), 2.19-2.05 (m, 2H), 2.03 (d, J = 1.1 Hz, 3H), 1.94-1.79 (m, 2H), 1.58-1.40 (m, 2H).

Example A3

Synthesis of 6'-(((1S,3S)-3-((5-(difluoromethoxy)pyrimidin-2-yl)amino)cyclopentyl)amino)-4-methyl-2H-[1, 3'-bipyridin]-2-one

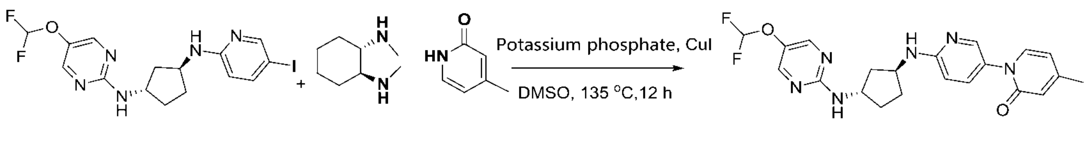

At room temperature, (1S,3S)-N1-(5-(difluoromethoxy)pyrimidin-2-yl)-N3-(5-iodopyridin-2-yl)cyclopentane-1,3-diami ne (300 mg, 0.67 mmol), 4-methylpyridin-2(1H)-one (146 mg, 1.34 mmol), (1S,2S)-N1,N2-dimethylcyclohexane-1,2-diamine (19.0 mg, 0.13 mmol), and potassium phosphate (284.5 mg, 1.34 mmol) were added to DMSO (8 mL). Subsequently, copper(I) iodide (38.2 mg, 0.20 mmol) was added. After completion of addition, the system was purged with nitrogen, and heated to 140 °C and reacted for 12 hours.

After completion of the reaction, the mixture was cooled to room temperature, and water (40 mL) was added to the system. The mixture was extracted with ethyl acetate (20 mL × 3). The organic phases were combined,washed with saturated brine (30 mL), dried, filtered, and concentrated under reduced pressure to dryness to obtain a residue. The residue was purified by normal-phase column chromatography (dichloromethane/methanol = 10:1) to obtain 100 mg of a white product, 6'-(((1S,3S)-3-((5-(difluoromethoxy)pyrimidin-2-yl)amino)cyclopentyl)amino)-4-methyl-2H-[1, 3'-bipyridin]-2-one (yield: 34.7%).

LC-MS: $[M+H]^+$ = 429.

$^1$H NMR (400 MHz, DMSO-$d_6$) δ 8.24-8.22 (s, 2H), 7.90-7.87 (d, J = 2.7 Hz, 1H), 7.52-7.46 (t, J = 6.6 Hz, 2H), 7.38-7.34 (m, 1H), 7.23-6.84 (m, 2H), 6.53-6.49 (d, J = 8.9 Hz, 1H), 6.26-6.24 (m, 1H), 6.16-6.11 (m, 1H), 4.35-4.25 (m, 2H), 2.20-2.07 (m, 5H), 1.95-1.81 (m, 2H), 1.60-1.44 (m, 2H).

Example A4

Synthesis of 6'-(((1S,3S)-3-((5-(difluoromethoxy)-4-methylpyrimidin-2-yl)amino)cyclopentyl)amino)-2H-[1, 3'-bipyridin]-2-one

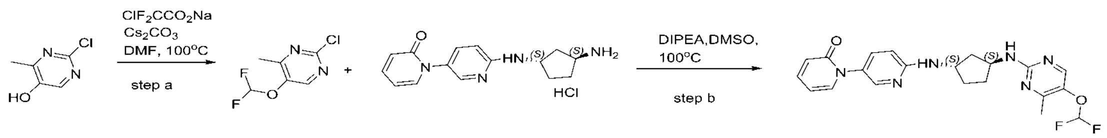


Step A: Synthesis of 2-chloro-5-(difluoromethoxy)-4-methylpyrimidine

2-Chloro-4-methylpyrimidin-5-ol (1 g, 7.06 mmol) was dissolved in DMF (20 mL). Cesium carbonate (3 g, 9.19 mmol) was added, and the mixture was reacted under nitrogen protection at room temperature for 1.5 hours. Subsequently, sodium chlorodifluoroacetate (3.5 g, 22.98 mmol) was added, and the mixture was stirred at 100 °C for 3.5 hours.

After completion of the reaction, the mixture was cooled to room temperature, quenched with 50 mL of water, and extracted with ethyl acetate (60 mL × 3). The organic phases were combined, dried over anhydrous sodium sulfate, filtered, and concentrated. The resulting residue was purified by silica gel column chromatography (PE/EA = 100/1 to 10/1) to obtain 530 mg of 2-chloro-5-(difluoromethoxy)-4-methylpyrimidine (yield: 38%).

Step B: Synthesis of 6'-(((1S,3S)-3-((5-(difluoromethoxy)-4-methylpyrimidin-2-yl)amino)cyclopentyl)amino)-2H-[1, 3'-bipyridin]-2-one

2-Chloro-5-(difluoromethoxy)-4-methylpyrimidine (317 mg, 1.63 mmol) and 6'-(((1S,3S)-3-aminocyclopentyl)amino)-2H-[1,3'-bipyridin]-2-one hydrochloride (500 mg, 1.63 mmol) were dissolved in DMSO (5 mL) and reacted at 100 °C for 6 hours.

After completion of the reaction, the mixture was cooled to room temperature, quenched with 20 mL of water, and extracted with ethyl acetate (60 mL × 3). The organic phases were combined, dried over anhydrous sodium sulfate, filtered, and concentrated. The resulting residue was purified by silica gel column chromatography (PE/EA = 100/1 to EA) to obtain 80 mg of 6'-(((1S,3S)-3-((5-(difluoromethoxy)-4-methylpyrimidin-2-yl)amino)cyclopentyl)amino)-2H-[1, 3'-bipyridin]-2-one (yield: 11%).

LC-MS: $[M+H]^+$ = 429. $^1$H NMR (400 MHz, DMSO-$d_6$) δ 8.08 (s, 1H), 7.92 (d, J = 2.7 Hz, 1H), 7.60 (d, J = 6.8 Hz, 1H), 7.47 (t, J = 7.9 Hz, 1H), 7.43-7.31 (m, 2H), 7.22-6.78 (m, 2H), 6.52 (d, J = 8.9 Hz, 1H), 6.44 (d, J = 9.2 Hz, 1H), 6.27 (t, J = 6.7 Hz, 1H), 4.39-4.23 (m, 2H), 2.24 (s, 3H), 2.18-2.03 (m, 2H), 1.96-1.79 (m, 2H), 1.56-1.42 (m, 2H).

Example A5

Synthesis of 6'-(((1S,3S)-3-((5-(difluoromethoxy)pyrimidin-2-yl)amino)cyclopentyl)amino)-5'-methyl-2H-[1, 3'-bipyridin]-2-one

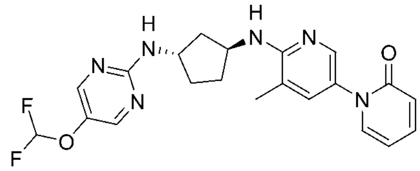

Step A: Synthesis of 6'-chloro-5'-methyl-2H-[1,3'-bipyridin]-2-one

Under nitrogen protection at room temperature, pyridin-2(1H)-one (1.045 g, 11.0 mmol, 1.1 eq), 2-chloro-5-iodo-3-methylpyridine (2.5 g, 10.0 mmol, 1.0 eq), copper(I) iodide (0.286 g, 1.5 mmol, 0.15 eq), (1S,2S)-$N^1$,$N^2$-dimethylcyclohexane-1,2-diamine (0.427 g, 3.0 mmol, 0.3 eq), and potassium phosphate (4.245 g, 20.0 mmol, 2.0 eq) were dissolved in dimethyl sulfoxide (40 mL) and reacted at 120 °C for 14 hours.

After completion of the reaction, cold water (100 mL) was added to quench the reaction, followed by extraction with ethyl acetate (100 mL × 3). The organic phases were combined, dried over anhydrous sodium sulfate, filtered, and concentrated. The resulting residue was purified by silica gel column chromatography (eluent: ethyl acetate/n-hexane = 5/10) to obtain 1.0 g of a yellow solid, 6'-chloro-5'-methyl-2H-[1,3'-bipyridin]-2-one (yield: 45%). LC-MS: $[M+H]^+$ = 221.

Step B: Synthesis of 6'-(((1S,3S)-3-((5-(difluoromethoxy)pyrimidin-2-yl)amino)cyclopentyl)amino)-5'-methyl-2H-[1, 3'-bipyridin]-2-one

Under nitrogen protection at room temperature, 6'-chloro-5'-methyl-2H-[1,3'-bipyridin]-2-one (0.33 g, 1.5 mmol, 1.2 eq), (1S,3S)-$N^1$-(5-(difluoromethoxy)pyrimidin-2-yl)cyclopentane-1,3-diamine (0.305 g, 1.25 mmol, 1.0 eq), methanesulfonato(2-di-tert-butylphosphino-2',4',6'-triisopropyl-1,1'-biphenyl)(2'-amino-1,1'-biph enyl-2-yl)palladium(II) (0.2 g, 0.25 mmol, 0.2 eq), and potassium tert-butoxide (0.281 g, 2.5 mmol, 2.0 eq) were dissolved in 1,4-dioxane (5 mL), and the mixture was heated to 110 °C and reacted overnight.

After completion of the reaction, water (50 mL) was added, followed by extraction with ethyl acetate (50 mL × 3). The organic phases were combined, dried over anhydrous sodium sulfate, filtered, and concentrated. The resulting residue was purified by silica gel column chromatography (eluent: ethyl acetate/methanol = 1/10) to obtain 0.130 g of 6'-(((1S,3S)-3-((5-(difluoromethoxy)pyrimidin-2-yl)amino)cyclopentyl)amino)-5'-methyl-2H-[1, 3'-bipyridin]-2-one (yield: 20%). LC-MS: $[M+H]^+$ = 429.

NMR data: $^1$H NMR (400 MHz, DMSO-$d_6$) δ 8.23 (s, 2H), 7.83 (d, J = 2.6 Hz, 1H), 7.58 (dd, J = 6.9, 2.1 Hz, 1H), 7.54-7.42 (m, 2H), 7.28 (dd, J = 2.6, 1.0 Hz, 1H), 7.03 (t, J = 74.0 Hz, 1H), 6.50-6.40 (m, 1H), 6.26 (td, J = 6.7, 1.4 Hz, 1H), 5.95 (d, J = 7.0 Hz, 1H), 4.53 (h, J = 7.0 Hz, 1H), 4.34 (p, J = 7.0 Hz, 1H), 2.27-2.03 (m, 5H), 1.94 (t, J = 7.1 Hz, 2H), 1.63-1.48 (m, 2H).

Example A6

Synthesis of 6'-(((1S,3S)-3-((5-(difluoromethoxy)pyrimidin-2-yl)amino)cyclopentyl)amino)-4',6-dimethyl-2H -[1,3'-bipyridin]-2-one

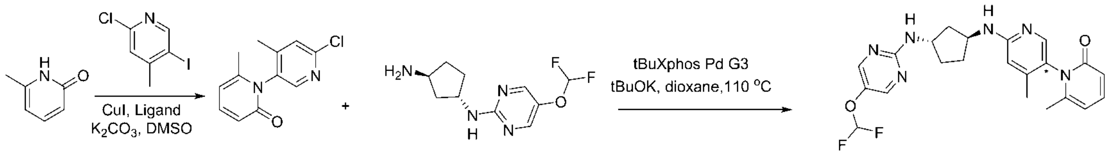


Step A: Synthesis of 6'-chloro-4',6-dimethyl-2H-[1,3'-bipyridin]-2-one

Under nitrogen protection at room temperature, 6-methylpyridin-2(1H)-one (2.0 g, 18.35 mmol, 1.0 eq), 2-chloro-5-iodo-4-methylpyridine (5.0 g, 20.2 mmol, 1.1 eq), copper(I) iodide (1.05 g, 5.5 mmol, 0.3 eq), 8-hydroxyquinoline

(0.88 g, 6.05 mmol, 0.33 eq), and potassium carbonate (8.88 g, 64.22 mmol, 3.5 eq) were dissolved in dimethyl sulfoxide (74 mL) and reacted at 140 °C overnight.

After completion of the reaction, cold water (150 mL) was added to quench the reaction, followed by extraction with ethyl acetate (100 mL × 3). The organic phases were combined, dried over anhydrous sodium sulfate, filtered, and concentrated. The resulting residue was purified by silica gel column chromatography (eluent: ethyl acetate/n-hexane = 5/10) to obtain 0.407 g of a yellow solid, 6'-chloro-4',6-dimethyl-2H-[1,3'-bipyridin]-2-one (yield: 10%). LC-MS: $[M+H]^+$ = 235.

Step B: Synthesis of 6'-(((1S,3S)-3-((5-(difluoromethoxy)pyrimidin-2-yl)amino)cyclopentyl)amino)-4',6-dimethyl-2H -[1,3'-bipyridin]-2-one

Under nitrogen protection at room temperature, 6'-chloro-4',6-dimethyl-2H-[1,3'-bipyridin]-2-one (0.407 g, 1.74 mmol, 1.2 eq), (1S,3S)-$N^1$-(5-(difluoromethoxy)pyrimidin-2-yl)cyclopentane-1,3-diamine (0.472 g, 1.45 mmol, 1.0 eq), methanesulfonato(2-di-tert-butylphosphino-2',4',6'-triisopropyl-1,1'-biphenyl)(2'-amino-1,1'-biph enyl-2-yl)palladium(II) (0.231 g, 0.29 mmol, 0.2 eq), and potassium tert-butoxide (0.325 g, 2.9 mmol, 2.0 eq) were dissolved in 1,4-dioxane (6 mL), and the mixture was heated to 110 °C and reacted overnight.

After completion of the reaction, the organic solvent was directly dried by rotary evaporation. The resulting residue was purified by silica gel column chromatography (eluent: ethyl acetate/methanol = 1/10) to obtain 0.36 g of 6'-(((1S,3S)-3-((5-(difluoromethoxy)pyrimidin-2-yl)amino)cyclopentyl)amino)-4',6-dimethyl-2H -[1,3'-bipyridin]-2-one (yield: 56%). LC-MS: $[M+H]^+$ = 443.

NMR data: $^1$H NMR (400 MHz, DMSO-$d_6$) δ 8.23 (s, 2H), 7.65 (s, 1H), 7.47 (dd, J = 7.2, 3.6 Hz, 1H), 7.39 (dd, J = 9.2, 6.8 Hz, 1H), 7.21-6.84 (m, 1H), 6.76 (d, J = 6.9 Hz, 1H), 6.42 (s, 1H), 6.32 (d, J = 9.2 Hz, 1H), 6.23 (d, J = 6.8 Hz, 1H), 4.30 (dtd, J = 12.1, 7.1, 3.9 Hz, 2H), 2.12 (dtd, J = 12.4, 8.6, 7.7, 3.7 Hz, 2H), 1.92-1.85 (m, 5H), 1.81 (s, 3H), 1.51 (dtd, J = 18.5, 10.6, 9.8, 4.1 Hz, 2H).

After Pre-HPLC separation, using column model IG-3: 4.6*250mm*5um, mobile phase n-hexane/ethanol = 20:80, flow rate 0.6 mL/min, column temperature 35 °C, 22 mg of product with HPLC retention time 12 minutes and purity 96.16% was obtained, named 6A. NMR data: $^1$H NMR (400 MHz, DMSO-$d_6$) δ 8.23 (s, 2H), 7.65 (s, 1H), 7.47 (d, J = 7.3 Hz, 1H), 7.40 (dd, J = 9.2, 6.8 Hz, 1H), 7.12 (d, J = 74.1 Hz, 1H), 6.76 (d, J = 6.9 Hz, 1H), 6.42 (s, 1H), 6.32 (d, J = 9.1 Hz, 1H), 6.23 (dt, J = 6.8, 1.2 Hz, 1H), 4.29 (p, J = 6.6 Hz, 2H), 2.12 (dq, J = 9.7, 5.3, 4.6 Hz, 2H), 1.96-1.79 (m, 8H), 1.51 (tdd, J = 15.8, 12.9, 11.1, 5.3 Hz, 2H).

32 mg of product with HPLC retention time 15.1 minutes and purity 80.37% was obtained, named 6B. NMR data: $^1$H NMR (400 MHz, DMSO-$d_6$) δ 8.23 (s, 2H), 7.65 (s, 1H), 7.47 (d, J = 7.2 Hz, 1H), 7.39 (dd, J = 9.2, 6.7 Hz, 1H), 7.03 (s, 1H), 6.76 (d, J = 6.9 Hz, 1H), 6.42 (s, 1H), 6.32 (d, J = 9.1 Hz, 1H), 6.23 (d, J = 6.8 Hz, 1H), 4.31 (h, J = 7.1, 6.6 Hz, 2H), 2.18-2.07 (m, 2H), 1.89 (d, J = 11.4 Hz, 5H), 1.81 (s, 3H), 1.51 (td, J = 17.8, 16.3, 6.9 Hz, 2H).

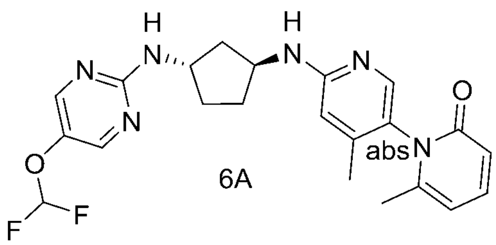
6A

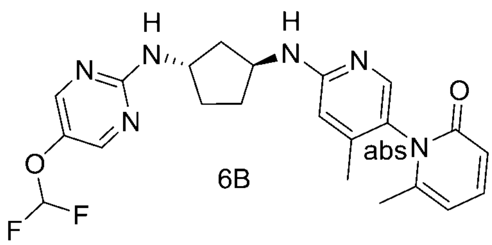
6B

wherein, * represents a racemate, and abs indicates absolute configuration.

Example A7

Synthesis of 3-(benzyloxy)-6'-(((1S,3S)-3-((5-(difluoromethoxy)pyrimidin-2-yl)amino)cyclopentyl)amino)-2 H-[1,3'-bipyridin]-2-one

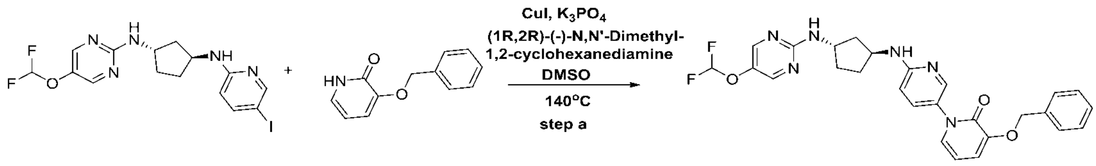


Step A: Synthesis of 3-(benzyloxy)-6'-(((1S,35)-3-((5-(difluoromethoxy)pyrimidin-2-yl)amino)cyclopentyl)amino)-2 H-[1,3'-bipyridin]-2-one

(1S,3S)-N1-(5-(difluoromethoxy)pyrimidin-2-yl)-N3-(5-iodopyridin-2-yl)cyclopentane-1,3-diamine (500 mg, 1.12 mmol) was dissolved in DMSO (5 mL). 3-(benzyloxy)pyridin-2(1H)-one (449 mg, 2.24 mmol), copper(I) iodide (85 mg, 0.447 mmol), potassium phosphate (309 mg, 2.24 mmol), and (1R,2R)-(-)-N,N'-dimethyl-1,2-cyclohexanediamine (32 mg, 0.224 mmol) were added. Under nitrogen protection, the mixture was reacted at 140 °C for 16 hours.

After completion of the reaction, the mixture was cooled to room temperature, quenched with 10 mL of water, and extracted with ethyl acetate (50 mL × 3). The organic phases were combined, dried over anhydrous sodium sulfate, filtered, and concentrated. The resulting residue was purified by $C_{18}$ column chromatography (water/acetonitrile = 95/5 → acetonitrile) to obtain 0.35 g of 3-(benzyloxy)-6'-(((1S,3S)-3-((5-(difluoromethoxy)pyrimidin-2-yl)amino)cyclopentyl)amino)-2 H-[1,3'-bipyridin]-2-one (yield: 60%).

LC-MS: $[M+H]^+$ = 521. NMR data: $^1H$ NMR (400 MHz, DMSO-$d_6$) δ: 8.23 (s, 2H), 7.92 (d, J = 2.7 Hz, 1H), 7.53-7.28 (m, 7H), 7.24-6.81 (m, 4H), 6.52 (d, J = 8.9 Hz, 1H), 6.18 (t, J = 7.2 Hz, 1H), 5.04 (s, 2H), 4.38-4.25 (m, 2H), 2.22-2.05 (m, 2H), 1.96-1.80 (m, 2H), 1.61-1.41 (m, 2H).

Example A8

Synthesis of 6'-(((1S,3S)-3-((5-(difluoromethoxy)pyrimidin-2-yl)amino)cyclopentyl)amino)-3-hydroxy-2H-[1 ,3'-bipyridin]-2-one

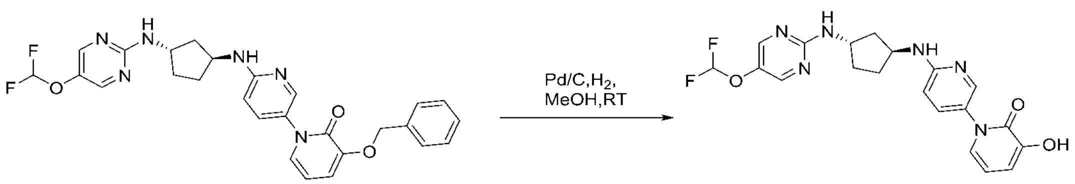


Under nitrogen protection, 3-(benzyloxy)-6'-(((1S,3S)-3-((5-(difluoromethoxy)pyrimidin-2-yl)amino)cyclopentyl)amino)-2 H-[1,3'-bipyridin]-2-one (200 mg, 0.384 mmol) was dissolved in methanol (5 mL). 10% Pd/C (40 mg) was added, and the mixture was reacted under a hydrogen atmosphere at room temperature for 12 hours.

After completion of the reaction, the solid was filtered off, and the filtrate was concentrated. The resulting residue was purified by $C_{18}$ column chromatography (water/acetonitrile = 100/0 → 10/90) to obtain 0.12 g of 6'-(((1S,3S)-3-((5-(difluoromethoxy)pyrimidin-2-yl)amino)cyclopentyl)amino)-3-hydroxy-2H-[1 ,3'-bipyridin]-2-one (yield: 72.7%).

LC-MS: $[M+H]^+$ = 431. $^1H$ NMR (400 MHz, DMSO-$d_6$) δ: 9.14 (s, 1H), 8.22 (s, 2H), 7.93 (d, J = 2.7 Hz, 1H), 7.52-7.32 (m, 2H), 7.22-6.79 (m, 3H), 6.79-6.68 (m, 1H), 6.53 (d, J = 8.9 Hz, 1H), 6.16 (t, J = 7.1 Hz, 1H), 4.38-4.21 (m, 2H), 2.21-2.03 (m, 2H), 1.96-1.78 (m, J = 6.7 Hz, 2H), 1.62-1.40 (m, 2H).

Example A9

Synthesis of 3-chloro-6'-(((1S,3S)-3-((5-(difluoromethoxy)pyrimidin-2-yl)amino)cyclopentyl)amino)-2H-[1,3 '-bipyridin]-2-one

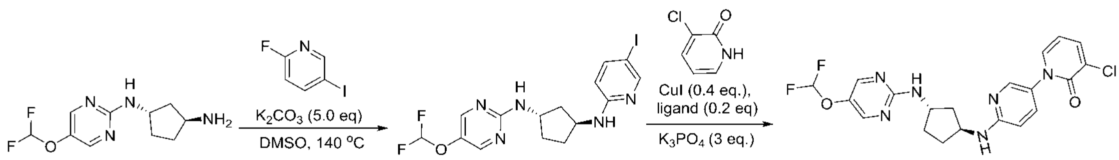


Step A: Synthesis of (1S,3S)-$N^1$-(5-(difluoromethoxy)pyrimidin-2-yl)-$N^3$-(5-iodopyridin-2-yl)cyclopentane-1,3-diami ne

Under nitrogen protection at room temperature, (1S,3S)-$N^1$-(5-(difluoromethoxy)pyrimidin-2-yl)cyclopentane-1,3-diamine (7.32 g, 30.0 mmol, 1.0 eq), 2-fluoro-5-iodopyridine (8.03 g, 36 mmol, 1.2 eq), and potassium carbonate (20.73 g, 150 mmol, 5.0 eq) were dissolved in dimethyl sulfoxide (140 mL) and reacted at 140 °C overnight.

After completion of the reaction, cold water (200 mL) was added to quench the reaction, followed by extraction with ethyl acetate (250 mL × 3). The organic phases were combined, dried over anhydrous sodium sulfate, filtered, and concentrated. The resulting residue was purified by silica gel column chromatography (eluent: ethyl acetate/n-hexane = 5/10) to obtain 10 g of a white solid, (1S,3S)-$N^1$-(5-(difluoromethoxy)pyrimidin-2-yl)-$N^3$-(5-iodopyridin-2-yl)cyclopentane-1,3-diami ne (yield: 75%). LC-MS: $[M+H]^+$ = 448.

Step B: Synthesis of 3-chloro-6'-(((1S,3S)-3-((5-(difluoromethoxy)pyrimidin-2-yl)amino)cyclopentyl)amino)-2H-[1,3 '-bipyridin]-2-one

Under nitrogen protection at room temperature, (1S,3S)-$N^1$-(5-(difluoromethoxy)pyrimidin-2-yl)-$N^3$-(5-iodopyridin-2-yl)cyclopentane-1,3-diami ne (0.448 g, 1.0 mmol, 1.0 eq), 3-chloropyridin-2(1H)-one (0.260 g, 2.0 mmol, 2.0 eq), copper(I) iodide (0.076 g, 0.4 mmol, 0.4 eq), $N^1$,$N^2$-dimethylcyclohexane-1,2-diamine (0.029 g, 0.2 mmol, 0.2 eq), and potassium phosphate (0.673 g, 3.0 mmol, 3.0 eq) were dissolved in dimethyl sulfoxide (4 mL), and the mixture was heated to 140 °C and reacted overnight.

After completion of the reaction, cold water (50 mL) was added to quench the reaction, followed by extraction with ethyl acetate (50 mL × 3). The organic phases were combined, dried over anhydrous sodium sulfate, filtered, and concentrated. The resulting residue was purified by silica gel column chromatography (eluent: ethyl acetate) to obtain 0.1 g of 3-chloro-6'-(((1S,3S)-3-((5-(difluoromethoxy)pyrimidin-2-yl)amino)cyclopentyl)amino)-2H-[1,3 '-bipyridin]-2-one (yield: 22%).

LC-MS: $[M+H]^+$ = 449. NMR data: $^1H$ NMR (400 MHz, DMSO-$d_6$) δ 8.23 (s, 2H), 7.95 (d, J = 2.7 Hz, 1H), 7.81 (dd, J = 7.3, 1.9 Hz, 1H), 7.65 (dd, J = 6.8, 1.9 Hz, 1H), 7.53-7.39 (m, 2H), 7.22-6.84 (m, 2H), 6.53 (d, J = 8.9 Hz, 1H), 6.30 (t, J = 7.1 Hz, 1H), 4.31 (h, J = 6.7 Hz, 2H), 2.12 (dq, J = 12.1, 7.2 Hz, 2H), 1.88 (tdd, J = 13.2, 10.5, 6.3 Hz, 2H), 1.59-1.43 (m, 2H).

Example A10

Synthesis of 3-cyclopropyl-6'-(((1S,3S)-3-((5-(difluoromethoxy)pyrimidin-2-yl)amino)cyclopentyl)amino)-2 H-[1,3'-bipyridin]-2-one

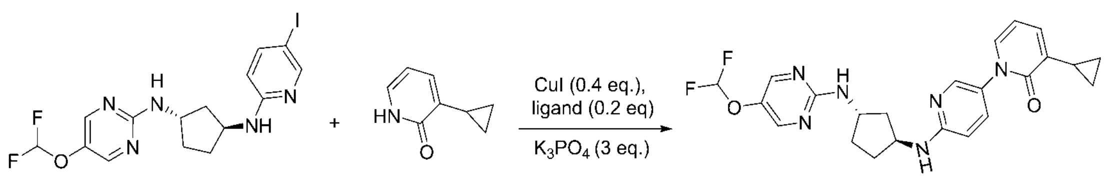


Under nitrogen protection at room temperature, (1S,3S)-$N^1$-(5-(difluoromethoxy)pyrimidin-2-yl)-$N^3$-(5-iodopyridin-2-yl)cyclopentane-1,3-diami ne (0.224 g, 0.5 mmol, 1.0 eq), 3-cyclopropylpyridin-2(1H)-one (0.10 g, 0.735 mmol, 1.5 eq), copper(I) iodide (0.038 g, 0.2 mmol, 0.4 eq), $N^1$,$N^2$-dimethylcyclohexane-1,2-diamine (0.015 g, 0.1 mmol, 0.2 eq), and potassium phosphate (0.320 g, 1.5 mmol, 3.0 eq) were dissolved in dimethyl sulfoxide (2 mL), and the mixture was heated to 140 °C and reacted overnight.

After completion of the reaction, cold water (25 mL) was added to quench the reaction, followed by extraction with ethyl acetate (50 mL × 3). The organic phases were combined, dried over anhydrous sodium sulfate, filtered, and concentrated. The resulting residue was purified by silica gel column chromatography (eluent: ethyl acetate) to obtain 0.077 g of 3-cyclopropyl-6'-(((1S,3S)-3-((5-(difluoromethoxy)pyrimidin-2-yl)amino)cyclopentyl)amino)-2 H-[1,3'-bipyridin]-2-one (yield: 31%).

LC-MS: $[M+H]^+$ = 455. NMR data: $^1H$ NMR (400 MHz, DMSO-$d_6$) δ 8.23 (s, 2H), 7.91 (d, J = 2.7 Hz, 1H), 7.48 (d, J = 7.2 Hz, 1H), 7.43-7.36 (m, 2H), 7.22-6.85 (m, 3H), 6.52 (d, J = 8.9 Hz, 1H), 6.17 (t, J = 6.8 Hz, 1H), 4.30 (p, J = 6.6 Hz, 2H), 2.18-2.08 (m, 2H), 2.01 (ddd, J = 8.5, 5.3, 3.1 Hz, 1H), 1.95-1.83 (m, 2H), 1.51 (ddd, J = 18.9, 13.8, 7.3 Hz, 2H), 0.88-0.81 (m, 2H), 0.67-0.60 (m, 2H).

Example A11

Synthesis of 6'-(((1S,3S)-3-((5-(difluoromethoxy)pyrimidin-2-yl)amino)cyclopentyl)amino)-2',6-dimethyl-2H -[1,3'-bi-pyridin]-2-one

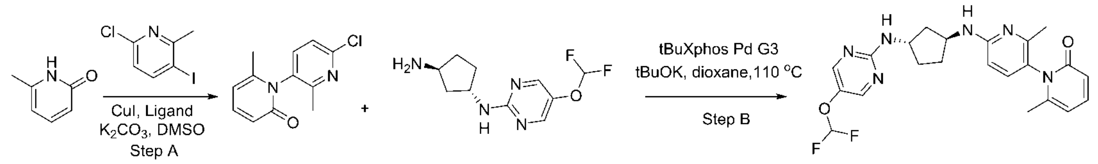


Step A: Synthesis of 6'-chloro-2',6-dimethyl-2H-[1,3'-bipyridin]-2-one

Under nitrogen protection at room temperature, 6-methylpyridin-2(1H)-one (2.0 g, 18.35 mmol, 1.0 eq), 6-chloro-3-iodo-2-methylpyridine (5.0 g, 20.2 mmol, 1.1 eq), copper(I) iodide (1.05 g, 5.5 mmol, 0.3 eq), 8-hydroxyquinoline (0.88 g, 6.05 mmol, 0.33 eq), and potassium carbonate (8.88 g, 64.22 mmol, 3.5 eq) were dissolved in dimethyl sulfoxide (40 mL) and reacted at 140 °C overnight.

After completion of the reaction, cold water (150 mL) was added to quench the reaction, followed by extraction with ethyl acetate (100 mL × 3). The organic phases were combined, dried over anhydrous sodium sulfate, filtered, and concentrated. The resulting residue was purified by silica gel column chromatography (eluent: ethyl acetate/n-hexane = 5/10) to obtain 0.203 g of a yellow solid, 6'-chloro-2',6-dimethyl-2H-[1,3'-bipyridin]-2-one (yield: 5%). LC-MS: $[M+H]^+$ = 235.

Step B: Synthesis of 6'-(((1S,3S)-3-((5-(difluoromethoxy)pyrimidin-2-yl)amino)cyclopentyl)amino)-2',6-dimethyl-2H -[1,3'-bipyridin]-2-one

Under nitrogen protection at room temperature, 6'-chloro-2',6-dimethyl-2H-[1,3'-bipyridin]-2-one (0.203 g, 0.86 mmol, 1.2 eq), (1S,3S)-$N^1$-(5-(difluoromethoxy)pyrimidin-2-yl)cyclopentane-1,3-diamine (0.24 g, 0.72 mmol, 1.0 eq), methanesulfonato(2-di-tert-butylphosphino-2',4',6'-triisopropyl-1,1'-biphenyl)(2'-amino-1,1'-biph enyl-2-yl)palladium(II) (0.12 g, 0.15 mmol, 0.2 eq), and potassium tert-butoxide (0.16 g, 1.45 mmol, 2.0 eq) were dissolved in 1,4-dioxane (8 mL), and the mixture was heated to 110 °C and reacted overnight.

After completion of the reaction, the organic solvent was directly dried by rotary evaporation. The resulting residue was purified by silica gel column chromatography (eluent: ethyl acetate/methanol = 1/10) to obtain 0.18 g of 6'-(((1S,3S)-3-((5-(difluoromethoxy)pyrimidin-2-yl)amino)cyclopentyl)amino)-2',6-dimethyl-2H -[1,3'-bipyridin]-2-one.

LC-MS: $[M+H]^+$ = 443. NMR data: $^1H$ NMR (400 MHz, DMSO-$d_6$) δ 8.26-8.21 (s, 2H), 7.52-7.44 (dd, J = 7.2, 2.3 Hz, 1H), 7.42-7.35 (dd, J = 9.2, 6.8 Hz, 1H), 7.23-7.02 (m, 2H), 6.86-6.75 (m, 1H), 6.41-6.29 (dd, J = 24.5, 8.9 Hz, 2H), 6.25-6.20 (m, 1H), 4.36-4.22 (m, 2H), 2.22-2.05 (m, 2H), 1.95-1.84 (m, 8H), 1.59-1.44 (m, 2H).

After Pre-SFC separation (method: column model IG-3: 4.6*250mm*5um, mobile phase n-hexane/ethanol = 20:80, flow rate 0.6 mL/min, column temperature 35 °C), 25 mg of the front peak (RT = 10.53 min, purity 99%, named A11A) and 35 mg of the rear peak (RT=12.985 min, purity 76%, named A11B) were obtained.

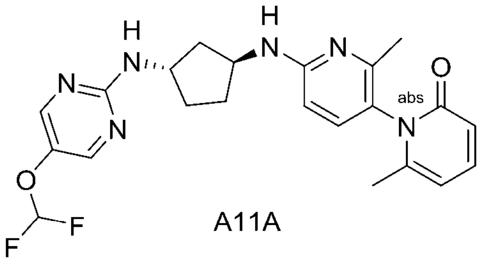


A11A

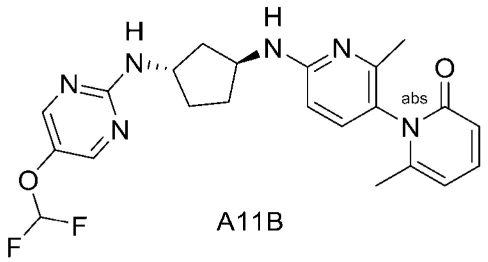


A11B

Example A12

Synthesis of ethyl 6'-(((1S,3S)-3-((5-(difluoromethoxy)pyrimidin-2-yl)amino)cyclopentyl)amino)-2-oxo-2H-[1,3'-b ipyridine]-3-carboxylate

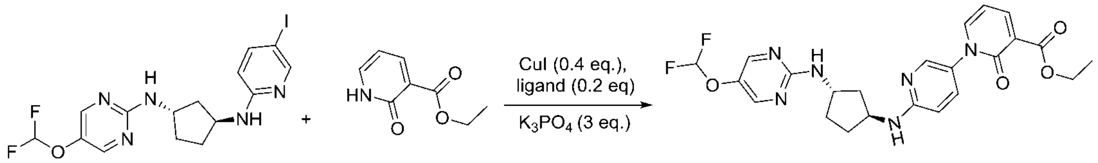


Step A: Synthesis of ethyl 6'-(((1S,3S)-3-((5-(difluoromethoxy)pyrimidin-2-yl)amino)cyclopentyl)amino)-2-oxo-2H-[1,3'-b ipyridine]-3-carboxylate

Under nitrogen protection at room temperature, (1S,3S)-$N^1$-(5-(difluoromethoxy)pyrimidin-2-yl)-$N^3$-(5-iodopyridin-2-yl)cyclopentane-1,3-diami ne (0.642 g, 1.5 mmol, 1.0 eq), ethyl 2-oxo-1,2-dihydropyridine-3-carboxylate (0.502 g, 3 mmol, 2 eq), copper(I) iodide (0.114 g, 0.6 mmol, 0.4 eq), $N^1$,$N^2$-dimethylcyclohexane-1,2-diamine (0.043 g, 0.3 mmol, 0.2 eq), and potassium phosphate (0.320 g, 1.5 mmol, 3.0 eq) were dissolved in dimethyl sulfoxide (6 mL), and the mixture was heated to 140 °C and reacted overnight.

After completion of the reaction, cold water (25 mL) was added to quench the reaction, followed by extraction with ethyl acetate (50 mL × 3). The organic phases were combined, dried over anhydrous sodium sulfate, filtered, and concentrated. The resulting residue was purified by silica gel column chromatography (eluent: ethyl acetate) to obtain 0.19 g of ethyl 6'-(((1S,3S)-3-((5-(difluoromethoxy)pyrimidin-2-yl)amino)cyclopentyl)amino)-2-oxo-2H-[1,3'-b ipyridine]-3-carboxylate.

LC-MS: $[M+H]^+$ = 487. NMR data: $^1H$ NMR (400 MHz, DMSO-$d_6$) δ 8.23 (s, 2H), 8.06 (dd, J = 7.1, 2.2 Hz, 1H), 8.00-7.87 (m, 2H), 7.48 (d, J = 7.2 Hz, 1H), 7.41 (dd, J = 8.9, 2.7 Hz, 1H), 7.21-6.85 (m, 2H), 6.53 (d, J = 8.9 Hz, 1H), 6.36 (t, J = 6.9 Hz, 1H), 4.31 (h, J = 6.7 Hz, 2H), 4.21 (q, J = 7.1 Hz, 2H), 2.13 (ddt, J = 14.6, 12.4, 7.1 Hz, 2H), 1.88 (tdd, J = 13.3, 10.6, 6.4 Hz, 2H), 1.51 (ddd, J = 18.6, 13.9, 7.2 Hz, 2H), 1.25 (t, J = 7.1 Hz, 3H).

Example A13

Synthesis of 6'-(((1S,3S)-3-((5-(difluoromethoxy)pyrimidin-2-yl)amino)cyclopentyl)amino)-2-oxo-2H-[1,3'-b ipyridine]-3-carbonitrile

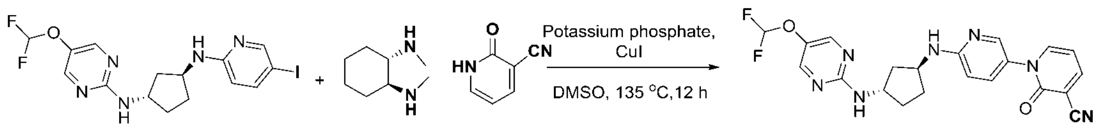


Step A: Synthesis of 6'-(((1S,3S)-3-((5-(difluoromethoxy)pyrimidin-2-yl)amino)cyclopentyl)amino)-2-oxo-2H-[1,3'-b ipyridine]-3-carbonitrile

At room temperature, (1S,3S)-$N^1$-(5-(difluoromethoxy)pyrimidin-2-yl)-$N^3$-(5-iodopyridin-2-yl)cyclopentane-1,3-diami ne (300 mg, 0.67 mmol), 2-oxo-1,2-dihydropyridine-3-carbonitrile (161 mg, 1.34 mmol), (1S,2S)-$N^1$,$N^2$-dimethylcyclohexane-1,2-diamine (19.0 mg, 0.13 mmol), and potassium phosphate (284.5 mg, 1.34 mmol) were added to DMSO (8 mL). Subsequently, copper(I) iodide (38.2 mg, 0.20 mmol) was added. After completion of addition, the system was purged with nitrogen, and then heated to 140 °C and reacted for 12 hours.

After completion of the reaction, the mixture was cooled to room temperature, and water (40 mL) was added to the system. The mixture was extracted with ethyl acetate (20 mL × 3). The organic phases were combined, washed with saturated brine (30 mL), dried, filtered, and concentrated under reduced pressure to dryness to obtain a residue. The residue was purified by normal-phase column chromatography (dichloromethane/methanol = 10:1) to obtain 18 mg of 6'-(((1S,3S)-3-((5-(difluoromethoxy)pyrimidin-2-yl)amino)cyclopentyl)amino)-2-oxo-2H-[1,3'-b ipyridine]-3-carbonitrile.

LC-MS: $[M+H]^+$ = 440. NMR data: $^1H$ NMR (400 MHz, DMSO-$d_6$) δ 8.24-8.20 (m, 3H), 8.06-8.03 (dd, J = 6.8, 2.1

Hz, 1H), 7.99-7.96 (d, J = 2.7 Hz, 1H), 7.51-7.42 (m, 2H), 7.23-6.84 (m, 2H), 6.57-6.51 (d, J = 9.0 Hz, 1H), 6.49-6.43 (t, J = 7.0 Hz, 1H), 4.37-4.26 (m, 2H), 2.20-2.07 (m, 2H), 1.96-1.81 (m, 2H), 1.60-1.43 (m, 2H).

Example A14

Synthesis of 6'-(((1S,3S)-3-((5-(difluoromethoxy)pyrimidin-2-yl)amino)cyclopentyl)amino)-3-fluoro-2H-[1,3' -bipyridin]-2-one

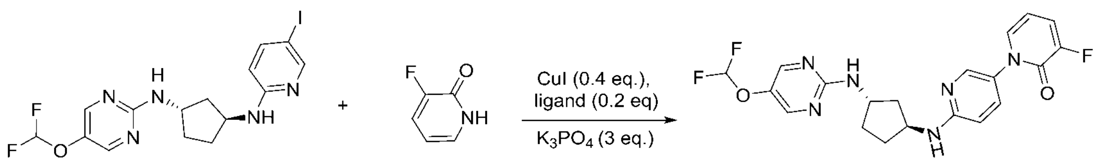


Under nitrogen protection at room temperature, (1S,3S)-N1-(5-(difluoromethoxy)pyrimidin-2-yl)-N3-(5-iodopyridin-2-yl)cyclopentane-1,3-diami ne (0.448 g, 1.0 mmol, 1.0 eq), 3-fluoropyridin-2(1H)-one (0.226 g, 2.0 mmol, 2.0 eq), copper(I) iodide (0.076 g, 0.4 mmol, 0.4 eq), N1,N2-dimethylcyclohexane-1,2-diamine (0.029 g, 0.2 mmol, 0.2 eq), and potassium phosphate (0.673 g, 3.0 mmol, 3.0 eq) were dissolved in dimethyl sulfoxide (4 mL), and the mixture was heated to 140 °C and reacted overnight.

After completion of the reaction, cold water (50 mL) was added to quench the reaction, followed by extraction with ethyl acetate (50 mL × 3). The organic phases were combined, dried over anhydrous sodium sulfate, filtered, and concentrated. The resulting residue was purified by silica gel column chromatography (eluent: ethyl acetate) to obtain 6'-(((1S,3S)-3-((5-(difluoromethoxy)pyrimidin-2-yl)amino)cyclopentyl)amino)-3-fluoro-2H-[1,3' -bipyridin]-2-one. LC-MS: [M+H]+ = 433.

NMR data: $^1$H NMR (400 MHz, DMSO-$d_6$) δ 8.23 (s, 2H), 7.96 (d, J = 2.7 Hz, 1H), 7.54-7.39 (m, 4H), 7.22-6.84 (m, 2H), 6.54 (d, J = 8.9 Hz, 1H), 6.24 (td, J = 7.3, 4.8 Hz, 1H), 4.31 (h, J = 6.8, 6.3 Hz, 2H), 2.19-2.06 (m, 2H), 1.88 (tdd, J = 13.2, 10.4, 6.3 Hz, 2H), 1.58-1.45 (m, 2H).

Example A15

Synthesis of 6'-(((1S,3S)-3-((5-(difluoromethoxy)pyrimidin-2-yl)amino)cyclopentyl)amino)-2'-methyl-2H-[1, 3'-bipyridin]-2-one

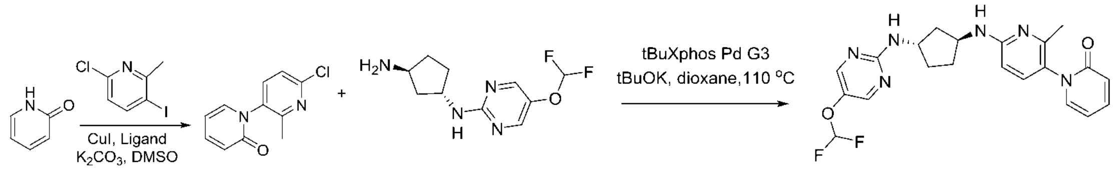


Step A: Synthesis of 6'-chloro-2'-methyl-2H-[1,3'-bipyridin]-2-one

Under nitrogen protection at room temperature, pyridin-2(1H)-one (2.0 g, 21.0 mmol, 1.0 eq), 6-chloro-3-iodo-2-methylpyridine (5.85 g, 23.1 mmol, 1.1 eq), copper(I) iodide (1.21 g, 6.3 mmol, 0.3 eq), 8-hydroxyquinoline (1.0 g, 6.9 mmol, 0.33 eq), and potassium carbonate (10.1 g, 74.7 mmol, 3.5 eq) were dissolved in dimethyl sulfoxide (40 mL) and reacted at 140 °C for 2 hours.

After completion of the reaction, cold water (150 mL) was added to quench the reaction, followed by extraction with ethyl acetate (100 mL × 3). The organic phases were combined, dried over anhydrous sodium sulfate, filtered, and concentrated. The resulting residue was purified by silica gel column chromatography (eluent: ethyl acetate/n-hexane = 5/10) to obtain 0.17 g of a yellow solid, 6'-chloro-2'-methyl-2H-[1,3'-bipyridin]-2-one (yield: 3.6%). LC-MS: [M+H]+ = 221.

Step B: Synthesis of 6'-(((1S,3S)-3-((5-(difluoromethoxy)pyrimidin-2-yl)amino)cyclopentyl)amino)-2'-methyl-2H-[1, 3'-bipyridin]-2-one

Under nitrogen protection at room temperature, 6'-chloro-2'-methyl-2H-[1,3'-bipyridin]-2-one (0.17 g, 0.77 mmol, 1.0 eq), (1S,3S)-$N^1$-(5-(difluoromethoxy)pyrimidin-2-yl)cyclopentane-1,3-diamine (0.207 g, 0.84 mmol, 1.1 eq), methanesulfonato(2-di-tert-butylphosphino-2',4',6'-triisopropyl-1,1'-biphenyl)(2'-amino-1,1'-biph enyl-2-yl)palladium(II) (0.12 g, 0.15 mmol, 0.2 eq), and potassium tert-butoxide (0.17 g, 1.54 mmol, 2.0 eq) were dissolved in 1,4-dioxane (8 mL), and the mixture was heated to 110 °C and reacted overnight.

After completion of the reaction, the organic solvent was directly dried by rotary evaporation. The resulting residue was purified by silica gel column chromatography (eluent: ethyl acetate/methanol = 1/10) to obtain 6'-(((1S,3S)-3-((5-(difluoromethoxy)pyrimidin-2-yl)amino)cyclopentyl)amino)-2'-methyl-2H-[1, 3'-bipyridin]-2-one. LC-MS: $[M+H]^+$ = 429.

NMR data: $^1H$ NMR (400 MHz, DMSO-$d_6$) $\delta$ 7.42-7.38 (s, 2H), 6.69-6.62 (m, 3H), 6.40-6.33 (m, 1H), 6.22-5.85 (m, 2H), 5.66-5.59 (d, J = 9.6 Hz, 1H), 5.56-5.50 (d, J = 8.6 Hz, 1H), 5.46-5.41 (t, J = 6.7 Hz, 1H), 3.52-3.39 (m, 2H), 1.37-1.23 (m, 2H), 1.20-1.13 (s, 3H), 1.10-0.99 (t, J = 6.8 Hz, 2H), 0.77-0.60 (m, 2H).

Example A16

Synthesis of 6'-(((1S,3S)-3-((5-(difluoromethoxy)pyrimidin-2-yl)amino)cyclopentyl)amino)-5'-(trifluorometh yl)-2H-[1,3'-bipyridin]-2-one

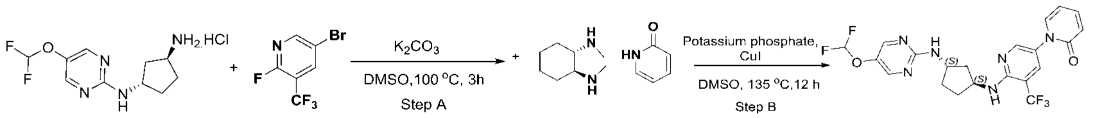


Step A: Synthesis of (1S,3S)-$N^1$-(5-bromo-3-(trifluoromethyl)pyridin-2-yl)-$N^3$-(5-(difluoromethoxy)pyrimidin-2-yl)c yclopentane-1,3-diamine

At room temperature, 5-bromo-2-fluoro-3-(trifluoromethyl)pyridine (500.0 mg, 2.05 mmol) and (1S,3S)-$N^1$-(5-(difluoromethoxy)pyrimidin-2-yl)cyclopentane-1,3-diamine hydrochloride (626 mg, 2.25 mmol) were dissolved in DMSO (10 mL). Subsequently, $K_2CO_3$ (1.7 g, 12.3 mmol) was added. After completion of addition, the system was heated to 100 °C and reacted for 3 hours.

After completion of the reaction, the mixture was cooled to room temperature, and water (40 mL) was added to the system. The mixture was extracted with ethyl acetate (20 mL $\times$ 3). The organic phases were combined, washed with saturated brine (30 mL), dried, filtered, and dried by rotary evaporation to obtain a residue. The resulting residue was purified by normal-phase column chromatography (n-hexane/ethyl acetate = 1:2) to obtain 600 mg of a white product, (1S,3S)-$N^1$-(5-bromo-3-(trifluoromethyl)pyridin-2-yl)-$N^3$-(5-(difluoromethoxy)pyrimidin-2-yl)c yclopentane-1,3-diamine (yield: 62%). LC-MS: $[M+H]^+$ = 468.

Step B: Synthesis of 6'-(((1S,3S)-3-((5-(difluoromethoxy)pyrimidin-2-yl)amino)cyclopentyl)amino)-5'-(trifluorometh yl)-2H-[1,3'-bipyridin]-2-one

At room temperature, (1S,3S)-$N^1$-(5-bromo-3-(trifluoromethyl)pyridin-2-yl)-$N^3$-(5-(difluoromethoxy)pyrimidin-2-yl)c yclopentane-1,3-diamine (300 mg, 0.64 mmol), pyridin-2(1H)-one (122 mg, 1.28 mmol), (1S,2S)-$N^1$,$N^2$-dimethylcyclohexane-1,2-diamine (18.2 mg, 0.13 mmol), and potassium phosphate (408.5 mg, 1.92 mmol) were added to DMSO (8 mL). Subsequently, copper(I) iodide (24.6 mg, 0.128 mmol) was added. After completion of addition, the system was purged with nitrogen, and heated to 140 °C and reacted for 12 hours.

After completion of the reaction, the mixture was cooled to room temperature, and water (40 mL) was added to the system. The mixture was extracted with ethyl acetate (20 mL $\times$ 3). The organic phases were combined, washed with saturated brine (30 mL), dried, filtered, and dried by rotary evaporation to obtain a residue. The resulting residue was purified by normal-phase column chromatography (dichloromethane/methanol = 10:1) to obtain 6'-(((1S,3S)-3-((5-(difluoromethoxy)pyrimidin-2-yl)amino)cyclopentyl)amino)-5'-(trifluorometh yl)-2H-[1,3'-bipyridin]-2-one. LC-MS: $[M+H]^+$ = 483.

NMR data: $^1H$ NMR (400 MHz, DMSO-$d_6$) $\delta$ 8.31-8.28 (d, J = 2.5 Hz, 1H), 8.25-8.22 (s, 2H), 7.90-7.87 (d, J = 2.5 Hz, 1H), 7.71-7.66 (dd, J = 7.0, 2.1 Hz, 1H), 7.53-7.46 (m, 2H), 7.23-6.84 (t, J = 74.0 Hz, 1H), 6.49-6.45 (d, J = 9.2 Hz, 1H),

6.35-6.28 (m, 2H), 4.76-4.65 (m, 1H), 4.39-4.29 (m, 1H), 2.18-2.06 (m, 2H), 2.04-1.90 (m, 2H), 1.70-1.49 (m, 2H).

Example A17

Synthesis of 6'-(((1S,3S)-3-((5-(difluoromethoxy)pyrimidin-2-yl)amino)cyclopentyl)amino)-5'-fluoro-2H-[1,3 '-bipyridin]-2-one

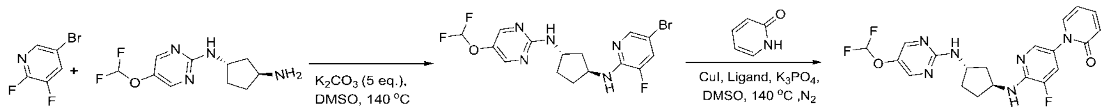


Step A: Synthesis of (1S,3S)-$N^1$-(5-bromo-3-fluoropyridin-2-yl)-$N^3$-(5-(difluoromethoxy)pyrimidin-2-yl)cyclopentan e-1,3-diamine

Under nitrogen protection at room temperature, 5-bromo-2,3-difluoropyridine (0.464 g, 2.4 mmol, 1.2 eq), (1S,3S)-$N^1$-(5-(difluoromethoxy)pyrimidin-2-yl)cyclopentane-1,3-diamine (0.488 g, 2 mmol, 1.0 eq), and potassium carbonate (1.4 g, 10.0 mmol, 5.0 eq) were dissolved in dimethyl sulfoxide (8 mL) and reacted at 140 °C for 14 hours.

After completion of the reaction, cold water (50 mL) was added to quench the reaction, followed by extraction with ethyl acetate (50 mL × 3). The organic phases were combined, dried over anhydrous sodium sulfate, filtered, and concentrated. The resulting residue was purified by silica gel column chromatography (eluent: ethyl acetate/n-hexane = 5/10) to obtain 0.641 g of a yellow solid, (1S,3S)-$N^1$-(5-bromo-3-fluoropyridin-2-yl)-$N^3$-(5-(difluoromethoxy)pyrimidin-2-yl) cyclopentan e-1,3-diamine (yield: 77%). LC-MS: $[M+H]^+$ = 418.

Step B: Synthesis of 6'-(((1S,3S)-3-((5-(difluoromethoxy)pyrimidin-2-yl)amino)cyclopentyl)amino)-5'-fluoro-2H-[1,3 '-bipyridin]-2-one

Under nitrogen protection at room temperature, (1S,3S)-$N^1$-(5-bromo-3-fluoropyridin-2-yl)-$N^3$-(5-(difluoromethoxy)pyrimidin-2-yl)cyclopentan e-1,3-diamine (0.641 g, 1.537 mmol, 1.0 eq), pyridin-2(1H)-one (0.292 g, 3.08 mmol, 2.0 eq), copper(I) iodide (0.117 g, 0.615 mmol, 0.4 eq), $N^1$,$N^2$-dimethylcyclohexane-1,2-diamine (0.044 g, 0.307 mmol, 0.2 eq), and potassium phosphate (0.98 g, 4.6 mmol, 3.0 eq) were dissolved in dimethyl sulfoxide (6 mL), and the mixture was heated to 140 °C and reacted overnight.

After completion of the reaction, cold water (50 mL) was added to quench the reaction, followed by extraction with ethyl acetate (50 mL × 3). The organic phases were combined, dried over anhydrous sodium sulfate, filtered, and concentrated. The resulting residue was purified by silica gel column chromatography (eluent: ethyl acetate) to obtain 6'-(((1S,3S)-3-((5-(difluoromethoxy)pyrimidin-2-yl)amino)cyclopentyl)amino)-5'-fluoro-2H-[1,3 '-bipyridin]-2-one. LC-MS: $[M+H]^+$ = 433.

NMR data: $^1$H NMR (400 MHz, DMSO-$d_6$) δ 8.23 (s, 2H), 7.84 (d, J = 2.1 Hz, 1H), 7.64 (dd, J = 6.9, 2.1 Hz, 1H), 7.60-7.42 (m, 3H), 7.22-6.84 (m, 2H), 6.50-6.42 (m, 1H), 6.28 (td, J = 6.7, 1.4 Hz, 1H), 4.50 (h, J = 6.9 Hz, 1H), 4.33 (p, J = 6.8 Hz, 1H), 2.18-2.07 (m, 2H), 1.99-1.89 (m, 2H), 1.65-1.49 (m, 2H).

Following the preparation methods of Compound A17 and Compound A34, Compound A17-D was prepared:

| | | |
|---|---|---|
| A17-D | 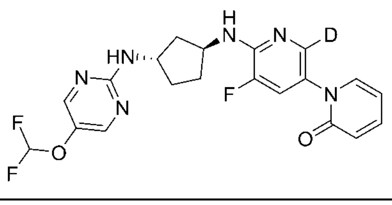 | NMR Data: $^1$H NMR: δ 8.21 (s, 2H), 7.61 (dd, *J* = 1.5, 6.9 Hz, 1H), 7.55 - 7.46 (m, 2H), 7.19 - 6.75 (m, 1H), 6.46 (d, *J* = 8.9 Hz, 1H), 6.31 (dt, *J* = 1.3, 6.7 Hz, 1H), 4.47 (quin, *J* = 6.8 Hz, 1H), 4.29 (quin, *J* = 6.7 Hz, 1H), 2.19 - 2.03 (m, 2H), 2.00 - 1.84 (m, 2H), 1.65 - 1.46 (m, 2H) |

Example A18

Synthesis of 5'-chloro-6'-(((1S,3S)-3-((5-(difluoromethoxy)pyrimidin-2-yl)amino)cyclopentyl)amino)-2H-[1, 3'-bipyridin]-2-one

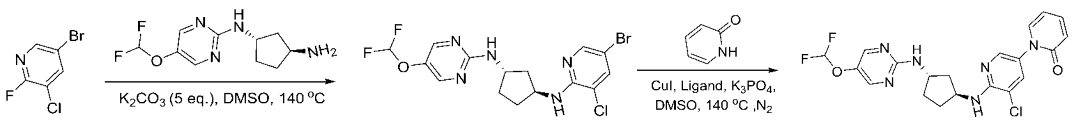


Step A: Synthesis of (1S,3S)-$N^1$-(5-bromo-3-chloropyridin-2-yl)-$N^3$-(5-(difluoromethoxy)pyrimidin-2-yl)cyclopentan e-1,3-diamine

Under nitrogen protection at room temperature, 5-bromo-3-chloro-2-fluoropyridine (0.502 g, 2.4 mmol, 1.2 eq), (1S,3S)-$N^1$-(5-(difluoromethoxy)pyrimidin-2-yl)cyclopentane-1,3-diamine (0.488 g, 2 mmol, 1.0 eq), and potassium carbonate (1.4 g, 10.0 mmol, 5.0 eq) were dissolved in dimethyl sulfoxide (8 mL) and reacted at 140 °C for 14 hours.

After completion of the reaction, cold water (50 mL) was added to quench the reaction, followed by extraction with ethyl acetate (50 mL × 3). The organic phases were combined, dried over anhydrous sodium sulfate, filtered, and concentrated. The resulting residue was purified by silica gel column chromatography (eluent: ethyl acetate/n-hexane = 5/10) to obtain 0.706 g of a yellow solid, (1S,3S)-$N^1$-(5-bromo-3-chloropyridin-2-yl)-$N^3$-(5-(difluoromethoxy)pyrimidin-2-yl)cyclopentan e-1,3-diamine (yield: 82%). LC-MS: $[M+H]^+$ = 434.

Step B: Synthesis of 5'-chloro-6'-(((1S,3S)-3-((5-(difluoromethoxy)pyrimidin-2-yl)amino)cyclopentyl)amino)-2H-[1, 3'-bipyridin]-2-one

Under nitrogen protection at room temperature, (1S,3S)-$N^1$-(5-bromo-3-chloropyridin-2-yl)-$N^3$-(5-(difluoromethoxy)pyrimidin-2-yl)cyclopentan e-1,3-diamine (0.706 g, 1.627 mmol, 1.0 eq), pyridin-2(1H)-one (0.31 g, 3.25 mmol, 2.0 eq), copper(I) iodide (0.124 g, 0.65 mmol, 0.4 eq), $N^1$,$N^2$-dimethylcyclohexane-1,2-diamine (0.046 g, 0.325 mmol, 0.2 eq), and potassium phosphate (1.04 g, 4.9 mmol, 3.0 eq) were dissolved in dimethyl sulfoxide (6.5 mL), and the mixture was heated to 140 °C and reacted overnight.

After completion of the reaction, cold water (50 mL) was added to quench the reaction, followed by extraction with ethyl acetate (50 mL × 3). The organic phases were combined, dried over anhydrous sodium sulfate, filtered, and concentrated. The resulting residue was purified by silica gel column chromatography (eluent: ethyl acetate) to obtain 5'-chloro-6'-(((1S,3S)-3-((5-(difluoromethoxy)pyrimidin-2-yl)amino)cyclopentyl)amino)-2H-[1, 3'-bipyridin]-2-one. LC-MS: $[M+H]^+$ = 449.

NMR data: $^1$H NMR (400 MHz, DMSO-$d_6$) δ 8.23 (s, 2H), 8.00 (d, J = 2.3 Hz, 1H), 7.76 (d, J = 2.3 Hz, 1H), 7.64 (dd, J = 6.9, 2.1 Hz, 1H), 7.49 (ddd, J = 8.7, 6.7, 2.0 Hz, 2H), 7.03 (t, J = 74.0 Hz, 1H), 6.54 (d, J = 7.3 Hz, 1H), 6.49-6.43 (m, 1H), 6.29 (td, J = 6.7, 1.4 Hz, 1H), 4.56 (p, J = 7.1 Hz, 1H), 4.33 (q, J = 6.8 Hz, 1H), 2.12 (qt, J = 7.5, 3.6 Hz, 2H), 2.01-1.90 (m, 2H), 1.67-1.50 (m, 2H).

Example A19

Synthesis of 6'-(((1S,3S)-3-((5-(difluoromethoxy)pyrimidin-2-yl)amino)cyclopentyl)amino)-2-oxo-2H-[1,3'-b ipyridine]-5'-carboxamide

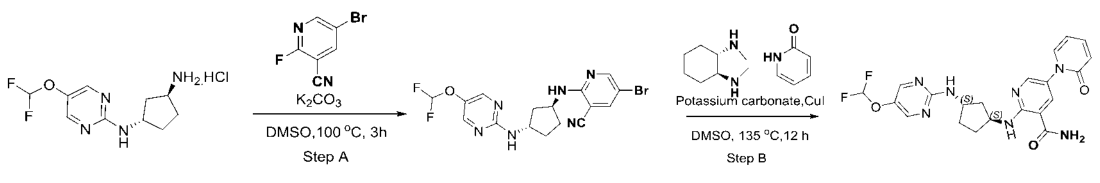


Step A: Synthesis of 5-bromo-2-(((1S,3S)-3-((5-(difluoromethoxy)pyrimidin-2-yl)amino)cyclopentyl)amino)nicotino nitrile

At room temperature, 5-bromo-2-fluoronicotinonitrile (500 mg, 2.5 mmol) and (1S,3S)-$N^1$-(5-(difluoromethoxy) pyrimidin-2-yl)cyclopentane-1,3-diamine hydrochloride (770 mg, 2.75 mmol) were dissolved in DMSO (10 mL). Subsequently, $K_2CO_3$ (1.38 g, 10.0 mmol) was added. After completion of addition, the system was heated to 100 °C and reacted for 30 minutes.

After completion of the reaction, the mixture was cooled to room temperature, and water (40 mL) was added to the system. The mixture was extracted with ethyl acetate (20 mL × 3). The organic phases were combined, washed with

saturated brine (30 mL), dried, filtered, and concentrated under reduced pressure to dryness to obtain a residue. The residue was purified by normal-phase column chromatography (n-hexane/ethyl acetate = 1:2) to obtain 700 mg of 5-bromo-2-(((1S,3S)-3-((5-(difluoromethoxy)pyrimidin-2-yl)amino)cyclopentyl)amino)nicotino nitrile (yield: 66%). LC-MS: $[M+H]^+$ = 425.

Step B: Synthesis of 6'-(((1S,3S)-3-((5-(difluoromethoxy)pyrimidin-2-yl)amino)cyclopentyl)amino)-2-oxo-2H-[1,3'-b ipyridine]-5'-carboxamide

At room temperature, 5-bromo-2-(((1S,3S)-3-((5-(difluoromethoxy)pyrimidin-2-yl)amino)cyclopentyl)amino)nicotino nitrile (300 mg, 0.70 mmol), pyridin-2(1H)-one (134 mg, 1.40 mmol), (1S,2S)-$N^1$,$N^2$-dimethylcyclohexane-1,2-diamine (20.0 mg, 0.14 mmol), and potassium carbonate (195.2 mg, 1.40 mmol) were added to DMSO (8 mL). Subsequently, copper(I) iodide (27.1 mg, 0.14 mmol) was added. After completion of addition, the system was purged with nitrogen, and heated to 140 °C and reacted for 12 hours.

After completion of the reaction, the mixture was cooled to room temperature, and water (40 mL) was added to the system. The mixture was extracted with ethyl acetate (20 mL × 3). The organic phases were combined, washed with saturated brine (30 mL), dried, filtered, and concentrated under reduced pressure to dryness to obtain a residue. The residue was purified by normal-phase column chromatography (dichloromethane/methanol = 10:1) to obtain 6'-(((1S,3S)-3-((5-(difluoromethoxy)pyrimidin-2-yl)amino)cyclopentyl)amino)-2-oxo-2H-[1,3'-b ipyridine]-5'-carboxamide. LC-MS: $[M+H]^+$ = 458.

NMR data: $^1$H NMR (400 MHz, DMSO-$d_6$) δ 8.96-8.90 (d, J = 6.8 Hz, 1H), 8.28-8.22 (s, 2H), 8.20-8.18 (d, J = 2.5 Hz, 1H), 8.09-8.00 (m, 2H), 7.70-7.65 (dd, J = 6.9, 2.1 Hz, 1H), 7.55-7.43 (m, 3H), 7.24-6.84 (t, J = 74.0 Hz, 1H), 6.51-6.45 (d, J = 9.2 Hz, 1H), 6.36-6.29 (td, J = 6.7, 1.4 Hz, 1H), 4.57-4.47 (q, J = 6.7 Hz, 1H), 4.36-4.27 (q, J = 7.0 Hz, 1H), 2.28-2.07 (m, 2H), 2.04-1.93 (m, 1H), 1.91-1.81 (m, 1H), 1.65-1.40 (m, 2H).

Example A20

Synthesis of 5'-((benzyloxy)methyl)-6'-(((1S,3S)-3-((5-(difluoromethoxy)pyrimidin-2-yl)amino)cyclopentyl)a mino)-2H-[1,3'-bipyridin]-2-one

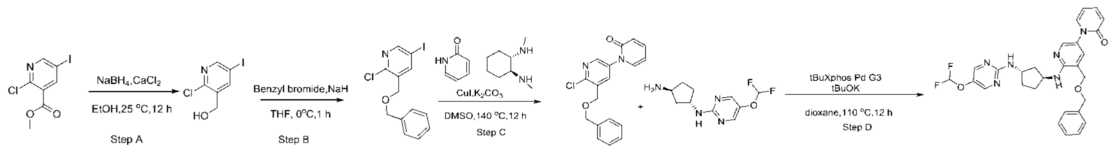


Step A: Synthesis of (2-chloro-5-iodopyridin-3-yl)methanol

Under nitrogen protection at room temperature, methyl 2-chloro-5-iodonicotinate (1.0 g, 3.3 mmol) and anhydrous calcium chloride (1.3 g, 11.5 mmol) were dissolved in anhydrous ethanol (20 mL). The mixture was stirred for 15 minutes, then sodium borohydride solid (0.51 g, 13.2 mmol) was added in batches. The mixture was reacted at room temperature overnight.

After completion of the reaction, saturated aqueous ammonium chloride solution (150 mL) was added to quench the reaction, followed by extraction with ethyl acetate (100 mL × 3). The organic phases were combined, dried over anhydrous sodium sulfate, filtered, and concentrated. The resulting residue was used directly in the next step without further purification, obtaining 0.85 g of a yellow solid, (2-chloro-5-iodopyridin-3-yl)methanol (yield: 94.4%). LC-MS: $[M+H]^+$ = 270.

Step B: Synthesis of 3-((benzyloxy)methyl)-2-chloro-5-iodopyridine

Under nitrogen protection at room temperature, (2-chloro-5-iodopyridin-3-yl)methanol (0.85 g, 3.1 mmol) was dissolved in anhydrous tetrahydrofuran (20 mL). The system was then cooled to 0 °C, and sodium hydride (0.16 g, 4.0 mmol, 60% content) was added in batches. After stirring for 15 minutes, benzyl bromide (0.68 g, 3.7 mmol) was added, and the mixture was reacted at 0 °C for one hour.

After completion of the reaction, saturated aqueous ammonium chloride solution (150 mL) was added to quench the reaction, followed by extraction with ethyl acetate (100 mL × 3). The organic phases were combined, dried over anhydrous sodium sulfate, filtered, and concentrated. The resulting residue was purified by silica gel column chromatography (eluent: ethyl acetate/n-hexane = 1/20) to obtain 0.95 g of a yellow solid, 3-((benzyloxy)methyl)-2-chloro-5-iodopyridine (yield: 84%). LC-MS: $[M+H]^+$ = 360.

Step C: Synthesis of 5'-((benzyloxy)methyl)-6'-chloro-2H-[1,3'-bipyridin]-2-one

At room temperature, 3-((benzyloxy)methyl)-2-chloro-5-iodopyridine (0.95 g, 2.6 mmol), pyridin-2(1H)-one (0.5 g, 5.2 mmol), (1S,2S)-$N^1$,$N^2$-dimethylcyclohexane-1,2-diamine (0.075 g, 0.52 mmol), and potassium phosphate (0.73 g, 5.2 mmol) were added to DMSO (20 mL). Subsequently, copper(I) iodide (0.1 g, 0.52 mmol) was added. After completion of addition, the system was purged with nitrogen, and heated to 140 °C and reacted for 12 hours.

After completion of the reaction, the mixture was cooled to room temperature, and water (40 mL) was added to the system. The mixture was extracted with ethyl acetate (40 mL × 3). The organic phases were combined, washed with saturated brine (100 mL), dried, filtered, and concentrated under reduced pressure to dryness to obtain a residue. The resulting residue was purified by normal-phase column chromatography (ethyl acetate/n-hexane = 4:1) to obtain 200 mg of a white product, 5'-((benzyloxy)methyl)-6'-chloro-2H-[1,3'-bipyridin]-2-one (yield: 23.2%). LC-MS: $[M+H]^+$ = 327.

Step D: Synthesis of 5'-((benzyloxy)methyl)-6'-(((1S,3S)-3-((5-(difluoromethoxy)pyrimidin-2-yl)amino)cyclopentyl)a mino)-2H-[1,3'-bipyridin]-2-one

Under nitrogen protection at room temperature, 5'-((benzyloxy)methyl)-6'-chloro-2H-[1,3'-bipyridin]-2-one (0.2 g, 0.6 mmol), (1S,3S)-$N^1$-(5-(difluoromethoxy)pyrimidin-2-yl)cyclopentane-1,3-diamine (0.179 g, 0.72 mmol), methanesulfonato(2-di-tert-butylphosphino-2',4',6'-triisopropyl-1,1'-biphenyl)(2'-amino-1,1'-biph enyl-2-yl)palladium(II) (0.097 g, 0.12 mmol), and potassium tert-butoxide (0.27 g, 2.4 mmol) were dissolved in 1,4-dioxane (8 mL), and the mixture was heated to 110 °C and reacted overnight.

After completion of the reaction, the organic solvent was directly dried by rotary evaporation. The resulting residue was purified by silica gel column chromatography (eluent: ethyl acetate/methanol = 1/10) to obtain 5'-((benzyloxy) methyl)-6'-(((1S,3S)-3-((5-(difluoromethoxy)pyrimidin-2-yl)amino)cyclopentyl)a mino)-2H-[1,3'-bipyridin]-2-one. LC-MS: $[M+H]^+$ = 535.

NMR data: $^1$H NMR (400 MHz, DMSO-$d_6$) δ 8.24-8.21 (s, 2H), 7.97-7.94 (d, J = 2.6 Hz, 1H), 7.64-7.60 (m, 1H), 7.52-7.44 (m, 3H), 7.41-7.27 (m, 5H), 7.23-6.84 (t, J = 74.0 Hz, 1H), 6.48-6.43 (m, 1H), 6.31-6.25 (td, J = 6.7, 1.4 Hz, 1H), 5.96-5.90 (d, J = 6.9 Hz, 1H), 4.60-4.46 (d, J = 22.1 Hz, 5H), 4.34-4.26 (m, 1H), 2.21-2.04 (m, 2H), 1.99-1.83 (m, 2H), 1.60-1.43 (m, 2H).

Example A21

Synthesis of 6'-(((1S,3S)-3-((5-(difluoromethoxy)pyrimidin-2-yl)amino)cyclopentyl)amino)-2-oxo-2H-[1,3'-b ipyridine]-5'-carbonitrile

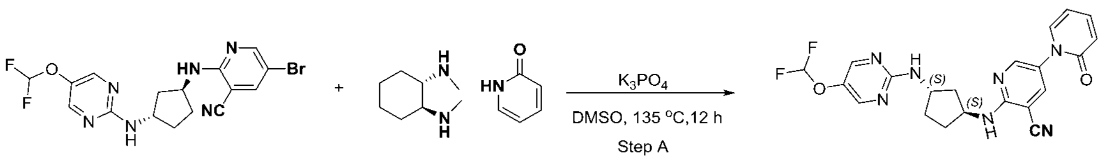


At room temperature, 5-bromo-2-(((1S,3S)-3-((5-(difluoromethoxy)pyrimidin-2-yl)amino)cyclopentyl)amino)nicotino nitrile (300 mg, 0.70 mmol), pyridin-2(1H)-one (134 mg, 1.40 mmol), (1S,2S)-$N^1$,$N^2$-dimethylcyclohexane-1,2-diamine (20.0 mg, 0.14 mmol), and potassium phosphate (300 mg, 1.40 mmol) were added to DMSO (8 mL). Subsequently, copper(I) iodide (27.1 mg, 0.14 mmol) was added. After completion of addition, the system was purged with nitrogen, and heated to 140 °C and reacted for 12 hours.

After completion of the reaction, the mixture was cooled to room temperature, and water (40 mL) was added to the system. The mixture was extracted with ethyl acetate (20 mL × 3). The organic phases were combined, washed with saturated brine (30 mL), dried, filtered, and concentrated under reduced pressure to dryness to obtain a residue. The resulting residue was purified by normal-phase column chromatography (dichloromethane/methanol = 10:1) to obtain 6'-(((1S,3S)-3-((5-(difluoromethoxy)pyrimidin-2-yl)amino)cyclopentyl)amino)-2-oxo-2H-[1,3'-b ipyridine]-5'-carbonitrile. LC-MS: $[M+H]^+$ = 440.

NMR data: $^1$H NMR (400 MHz, DMSO-$d_6$) δ 8.32-8.29 (d, J = 2.6 Hz, 1H), 8.25-8.22 (s, 2H), 8.08-8.06 (d, J = 2.7 Hz, 1H), 7.68-7.64 (dd, J = 7.0, 2.0 Hz, 1H), 7.53-7.46 (m, 2H), 7.37-7.33 (d, J = 7.2 Hz, 1H), 7.23-6.84 (t, J = 74.0 Hz, 1H),

6.50-6.45 (m, 1H), 6.34-6.27 (m, 1H), 4.67-4.54 (q, J = 7.3 Hz, 1H), 4.41-4.30 (q, J = 6.9 Hz, 1H), 2.19-2.06 (m, 2H), 2.05-1.89 (m, 2H), 1.70-1.49 (m, 2H).

Example A22

Synthesis of 6'-(((1S,3S)-3-((5-(difluoromethoxy)pyrimidin-2-yl)amino)cyclopentyl)amino)-2-oxo-2H-[1,3'-b ipyridine]-3-carboxylic acid

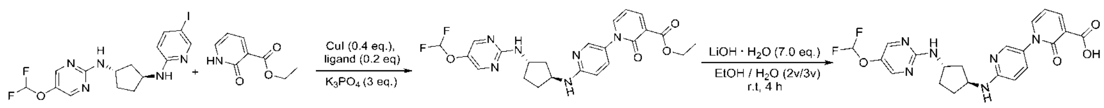


Step A: Synthesis of ethyl 6'-(((1S,3S)-3-((5-(difluoromethoxy)pyrimidin-2-yl)amino)cyclopentyl)amino)-2-oxo-2H-[1,3'-b ipyridine]-3-carboxylate

Under nitrogen protection at room temperature, (1S,3S)-$N^1$-(5-(difluoromethoxy)pyrimidin-2-yl)-$N^3$-(5-iodopyridin-2-yl)cyclopentane-1,3-diami ne (0.642 g, 1.5 mmol, 1.0 eq), ethyl 2-oxo-1,2-dihydropyridine-3-carboxylate (0.502 g, 3 mmol, 2 eq), copper(I) iodide (0.114 g, 0.6 mmol, 0.4 eq), $N^1$,$N^2$-dimethylcyclohexane-1,2-diamine (0.043 g, 0.3 mmol, 0.2 eq), and potassium phosphate (0.320 g, 1.5 mmol, 3.0 eq) were dissolved in dimethyl sulfoxide (6 mL), and the mixture was heated to 140 °C and reacted overnight.

After completion of the reaction, cold water (25 mL) was added to quench the reaction, followed by extraction with ethyl acetate (50 mL × 3). The organic phases were combined, dried over anhydrous sodium sulfate, filtered, and concentrated. The resulting residue was purified by silica gel column chromatography (eluent: ethyl acetate) to obtain 0.19 g of a yellow solid, ethyl 6'-(((1S,3S)-3-((5-(difluoromethoxy)pyrimidin-2-yl)amino)cyclopentyl)amino)-2-oxo-2H-[1,3'-b ipyridine]-3-carboxylate (yield: 26%). LC-MS: $[M+H]^+$ = 487.

Step B: Synthesis of 6'-(((1S,3S)-3-((5-(difluoromethoxy)pyrimidin-2-yl)amino)cyclopentyl)amino)-2-oxo-2H-[1,3'-b ipyridine]-3-carboxylic acid

At room temperature, ethyl 6'-(((1S,3S)-3-((5-(difluoromethoxy)pyrimidin-2-yl)amino)cyclopentyl)amino)-2-oxo-2H-[1,3'-b ipyridine]-3-carboxylate (0.140 g, 0.288 mmol, 1.0 eq) and lithium hydroxide monohydrate (0.085 g, 2.01 mmol, 7.0 eq) were dissolved in a mixed solvent of ethanol (2 mL) and water (3 mL). The mixture was stirred at room temperature for 4 hours.

After completion of the reaction, the pH of the reaction mixture was adjusted to approximately 6 using 1M dilute hydrochloric acid solution, resulting in the precipitation of a large amount of light yellow solid. The resultant was filtrated to obtain 6'-(((1S,3S)-3-((5-(difluoromethoxy)pyrimidin-2-yl)amino)cyclopentyl)amino)-2-oxo-2H-[1,3'-b ipyridine]-3-carboxylic acid. LC-MS: $[M+H]^+$ = 459.

NMR data: $^1$H NMR (400 MHz, DMSO-$d_6$) δ 14.36 (s, 1H), 8.44 (dd, J = 7.3, 2.1 Hz, 1H), 8.40-8.12 (m, 3H), 8.04 (d, J = 2.7 Hz, 1H), 7.66-7.42 (m, 2H), 7.22-6.84 (m, 2H), 6.76 (t, J = 7.0 Hz, 1H), 6.56 (d, J = 8.9 Hz, 1H), 4.46-4.24 (m, 2H), 2.27-2.04 (m, 2H), 2.03-1.78 (m, 2H), 1.52 (tt, J = 13.9, 7.4 Hz, 2H).

Example A23

Synthesis of 6'-(((1S,3S)-3-((5-(difluoromethoxy)pyrimidin-2-yl)amino)cyclopentyl)amino)-2-oxo-2H-[1,3'-b ipyridine]-4-carboxylic acid

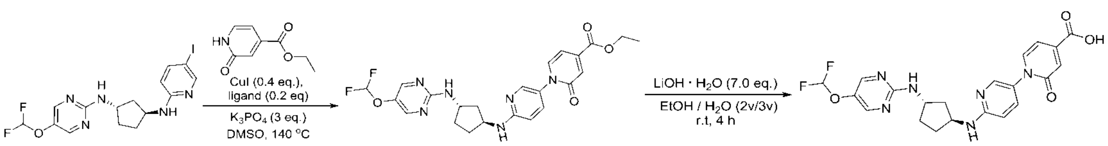

Step A: Synthesis of ethyl 6'-(((1S,3S)-3-((5-(difluoromethoxy)pyrimidin-2-yl)amino)cyclopentyl)amino)-2-oxo-2H-[1,3'-b ipyridine]-4-carboxylate

Under nitrogen protection at room temperature, (1S,3S)-$N^1$-(5-(difluoromethoxy)pyrimidin-2-yl)-$N^3$-(5-iodopyridin-2-yl)cyclopentane-1,3-diami ne (0.642 g, 1.5 mmol, 1.0 eq), ethyl 2-oxo-1,2-dihydropyridine-4-carboxylate (0.502 g, 3 mmol, 2 eq), copper(I) iodide (0.114 g, 0.6 mmol, 0.4 eq), $N^1$,$N^2$-dimethylcyclohexane-1,2-diamine (0.043 g, 0.3 mmol, 0.2 eq), and potassium phosphate (0.320 g, 1.5 mmol, 3.0 eq) were dissolved in dimethyl sulfoxide (6 mL), and the mixture was heated to 140 °C and reacted overnight.

After completion of the reaction, cold water (25 mL) was added to quench the reaction, followed by extraction with ethyl acetate (50 mL × 3). The organic phases were combined, dried over anhydrous sodium sulfate, filtered, and concentrated. The resulting residue was purified by silica gel column chromatography (eluent: ethyl acetate) to obtain 0.146 g of a yellow solid, ethyl 6'-(((1S,3S)-3-((5-(difluoromethoxy)pyrimidin-2-yl)amino)cyclopentyl)amino)-2-oxo-2H-[1,3'-b ipyridine]-4-carboxylate (yield: 22%). LC-MS: $[M+H]^+$ = 487.

Step B: Synthesis of 6'-(((1S,3S)-3-((5-(difluoromethoxy)pyrimidin-2-yl)amino)cyclopentyl)amino)-2-oxo-2H-[1,3'-b ipyridine]-4-carboxylic acid

At room temperature, ethyl 6'-(((1S,3S)-3-((5-(difluoromethoxy)pyrimidin-2-yl)amino)cyclopentyl)amino)-2-oxo-2H-[1,3'-b ipyridine]-4-carboxylate (0.103 g, 0.211 mmol, 1.0 eq) and lithium hydroxide monohydrate (0.085 g, 2.01 mmol, 7.0 eq) were dissolved in a mixed solvent of ethanol (2 mL) and water (3 mL). The mixture was stirred at room temperature for 4 hours.

After completion of the reaction, the pH of the reaction mixture was adjusted to approximately 6 using 1M dilute hydrochloric acid solution, resulting in the precipitation of a large amount of light yellow solid. The resultant was filtrated to obtain 6'-(((1S,3S)-3-((5-(difluoromethoxy)pyrimidin-2-yl)amino)cyclopentyl)amino)-2-oxo-2H-[1,3'-b ipyridine]-4-carboxylic acid. LC-MS: $[M+H]^+$ = 459.

NMR data: $^1H$ NMR (400 MHz, DMSO-$d_6$) δ 13.68 (s, 1H), 8.23 (s, 2H), 7.98 (d, J = 2.7 Hz, 1H), 7.73 (d, J = 7.0 Hz, 1H), 7.49 (d, J = 7.1 Hz, 2H), 7.37-6.78 (m, 3H), 6.60 (dt, J = 7.0, 3.1 Hz, 2H), 4.31 (h, J = 6.7 Hz, 2H), 2.13 (dq, J = 20.4, 7.3, 6.4 Hz, 2H), 1.90 (dq, J = 17.5, 6.6 Hz, 2H), 1.53 (qd, J = 14.7, 13.2, 8.0 Hz, 2H).

Example A24

Synthesis of 6'-(((1S,3S)-3-((5-(difluoromethoxy)pyrimidin-2-yl)amino)cyclopentyl)amino)-3-(hydroxymeth yl)-2H-[1,3'-bipyridin]-2-one

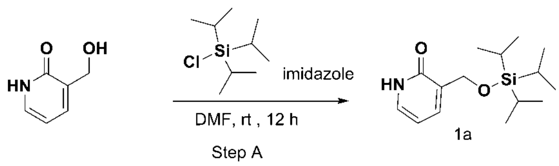


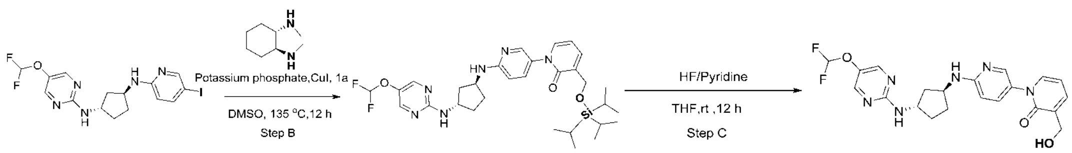


Step A: Synthesis of 3-(((triisopropylsilyl)oxy)methyl)pyridin-2(1H)-one

At room temperature, 3-(hydroxymethyl)pyridin-2(1H)-one (0.5 g, 4.0 mmol) was added to anhydrous DMF (10 mL). Subsequently, imidazole (0.54 g, 4.0 mmol) and triisopropylsilyl chloride (2.0 g, 5.2 mmol) were added to the system. After purging with nitrogen twice, the mixture was reacted at room temperature for 12 hours.

After completion of the reaction, water (40 mL) was added to the system, and the mixture was extracted with ethyl acetate (20 mL × 3). The organic phases were combined, washed twice with saturated brine (30 mL) and twice with 1M aqueous hydrochloric acid solution, dried, and dried by rotary evaporation. Without further purification, 1.05 g of a pale yellow liquid, 3-(((triisopropylsilyl)oxy)methyl)pyridin-2(1H)-one, was obtained (yield: 93%). LC-MS: $[M+H]^+$ = 282.

Step B: Synthesis of 6'-(((1S,3S)-3-((5-(Difluoromethoxy)pyrimidin-2-yl)amino)cyclopentyl)amino)-3-(((triisopropyl silyl)oxy)methyl)-2H-[1,3'-bipyridin]-2-one

At room temperature, (1S,3S)-$N^1$-(5-(difluoromethoxy)pyrimidin-2-yl)-$N^3$-(5-iodopyridin-2-yl)cyclopentane-1,3-diami ne (300 mg, 0.67 mmol), 3-(((triisopropylsilyl)oxy)methyl)pyridin-2(1H)-one (378 mg, 1.34 mmol), (1S,2S)-$N^1$,$N^2$-dimethylcyclohexane-1,2-diamine (19.0 mg, 0.13 mmol), and potassium phosphate (284.5 mg, 1.34 mmol) were added to DMSO (8 mL). Subsequently, copper(I) iodide (38.2 mg, 0.20 mmol) was added. After completion of addition, the system was purged with nitrogen, and heated to 140 °C and reacted for 12 hours.

After completion of the reaction, the mixture was cooled to room temperature, and water (40 mL) was added to the system. The mixture was extracted with ethyl acetate (20 mL × 3). The organic phases were combined, washed with saturated brine (30 mL), dried, filtered, and concentrated under reduced pressure to dryness to obtain a residue. The resulting residue was purified by normal-phase column chromatography (dichloromethane/methanol = 10:1) to obtain 130 mg of a white product, 6'-(((1S,3S)-3-((5-(difluoromethoxy)pyrimidin-2-yl)amino)cyclopentyl)amino)-3-(((triisopropyls ilyl) oxy)methyl)-2H-[1,3'-bipyridin]-2-one (yield: 32%). LC-MS: $[M+Na]^+$ = 623.

Step C: Synthesis of 6'-(((1S,3S)-3-((5-(difluoromethoxy)pyrimidin-2-yl)amino)cyclopentyl)amino)-3-(hydroxymeth yl)-2H-[1,3'-bipyridin]-2-one

At room temperature, 6'-(((1S,3S)-3-((5-(difluoromethoxy)pyrimidin-2-yl)amino)cyclopentyl)amino)-3-(((triisopropyls ilyl)oxy)methyl)-2H-[1,3'-bipyridin]-2-one (130 mg) was dissolved in tetrahydrofuran (5 mL). Subsequently, pyridinium hydrofluoride solution (2.5 mL) was added, and the mixture was reacted at room temperature for 12 hours.

After completion of the reaction, the mixture was first diluted with 20 mL of water, then 2M aqueous potassium carbonate solution was slowly added to adjust the pH to approximately 6. The mixture was then extracted with ethyl acetate (20 mL × 3), dried, and dried by rotary evaporation. The resulting residue was purified by normal-phase column chromatography (dichloromethane/methanol = 10:1) to obtain 6'-(((1S,3S)-3-((5-(difluoromethoxy)pyrimidin-2-yl)amino)cyclopentyl)amino)-3-(hydroxymeth yl)-2H-[1,3'-bipyridin]-2-one. LC-MS: $[M+H]^+$ = 445.

NMR data: $^1$H NMR (400 MHz, DMSO-$d_6$) δ 7.43-7.37 (s, 2H), 7.10-7.07 (d, J = 2.7 Hz, 1H), 6.70-6.62 (t, J = 7.4 Hz, 3H), 6.58-6.53 (dd, J = 8.9, 2.7 Hz, 1H), 6.40-6.00 (m, 2H), 5.72-5.65 (d, J = 8.9 Hz, 1H), 5.52-5.45 (t, J = 6.8 Hz, 1H), 4.30-4.25 (t, J = 5.5 Hz, 1H), 3.53-3.42 (d, J = 5.8 Hz, 4H), 1.37-1.24 (m, 2H), 1.12-0.98 (m, 2H), 0.76-0.62 (m, 2H).

Example A25

Synthesis of 5'-amino-6'-(((1S,3S)-3-((5-(difluoromethoxy)pyrimidin-2-yl)amino)cyclopentyl)amino)-2H-[1,3 '-bipyridin]-2-one

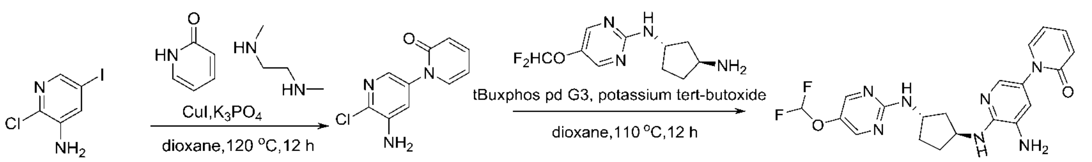


Step A: Synthesis of 5'-amino-6'-chloro-2H-[1,3'-bipyridin]-2-one

At room temperature, pyridin-2(1H)-one (0.82 g, 8.65 mmol, 1.1 eq), 2-chloro-5-iodopyridin-3-amine (2.0 g, 7.87 mmol, 1.0 eq), copper(I) iodide (0.30 g, 1.57 mmol, 0.2 eq), $N^1$,$N^2$-dimethylethane-1,2-diamine (0.27 g, 3.14 mmol, 0.4 eq), and potassium phosphate (5.0 g, 23.61 mmol, 3.0 eq) were added to 1,4-dioxane (20 mL). The solution was purged with nitrogen three times, then reacted at 120 °C for 12 hours.

After completion of the reaction, cold water (100 mL) was added to quench the reaction, followed by extraction with ethyl acetate (100 mL × 3). The organic phases were combined, dried over anhydrous sodium sulfate, filtered, and concentrated. The resulting residue was purified by silica gel column chromatography (eluent: dichloromethane/methanol = 20/1) to obtain 0.6 g of a gray solid, 5'-amino-6'-chloro-2H-[1,3'-bipyridin]-2-one (yield: 34%). LC-MS: $[M+H]^+$ = 222.

Step B: Synthesis of 5'-amino-6'-(((1S,3S)-3-((5-(difluoromethoxy)pyrimidin-2-yl)amino)cyclopentyl)amino)-2H-[1,3 '-bipyridin]-2-one

At room temperature, 5'-amino-6'-chloro-2H-[1,3'-bipyridin]-2-one (0.6 g, 2.7 mmol, 1.0 eq), (1S,3S)-$N^1$-(5-(di-

fluoromethoxy)pyrimidin-2-yl)cyclopentane-1,3-diamine (0.66 g, 2.7 mmol, 1.0 eq), methanesulfonato(2-di-tert-butylphosphino-2'4',6'-triisopropyl-1,1'-biphenyl)(2'-amino-1,1'-biph enyl-2-yl)palladium(II) (0.43 g, 0.54 mmol, 0.2 eq), and potassium tert-butoxide (0.60 g, 5.4 mmol, 2.0 eq) were added to 1,4-dioxane (12 mL). The solution was purged with nitrogen three times, then reacted at 110 °C for 12 hours.

After completion of the reaction, cold water (50 mL) was added to quench the reaction, followed by extraction with ethyl acetate (50 mL × 3). The organic phases were combined, dried over anhydrous sodium sulfate, filtered, and concentrated. The resulting residue was purified by silica gel column chromatography (eluent: dichloromethane/methanol = 10/1) to obtain 0.25 g of a gray solid, 5'-amino-6'-(((1S,3S)-3-((5-(difluoromethoxy)pyrimidin-2-yl)amino)cyclopentyl)amino)-2H-[1,3 '-bipyridin]-2-one (yield: 21.5%).

LC-MS: $[M+H]^+$ = 430. NMR data: $^1H$ NMR (400 MHz, DMSO-$d_6$) δ 8.25-8.22 (s, 2H), 7.56-7.52 (m, 1H), 7.50-7.42 (m, 2H), 7.30-7.27 (d, J = 2.3 Hz, 1H), 7.23-6.84 (t, J = 74.0 Hz, 1H), 6.68-6.64 (d, J = 2.4 Hz, 1H), 6.44-6.39 (m, 1H), 6.26-6.21 (m, 1H), 5.79-5.74 (d, J = 6.5 Hz, 1H), 5.07-5.01 (s, 2H), 4.50-4.41 (q, J = 6.5 Hz, 1H), 4.37-4.29 (p, J = 6.9 Hz, 1H), 2.25-2.06 (m, 2H), 1.97-1.86 (m, 2H), 1.60-1.48 (m, 2H).

Example A26

Synthesis of 6'-(((1S,3S)-3-((5-(difluoromethoxy)pyrimidin-2-yl)amino)cyclopentyl)amino)-5'-hydroxy-2H-[ 1,3'-bipyridin]-2-one

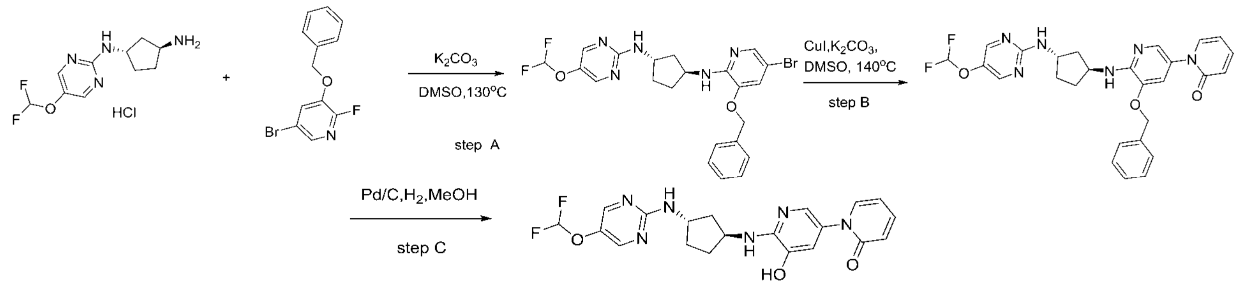


Step A: Synthesis of (1S,3S)-$N^1$-(3-(benzyloxy)-5-bromopyridin-2-yl)-$N^3$-(5-(difluoromethoxy)pyrimidin-2-yl)cyclop entane-1,3-diamine

(1S,3S)-$N^1$-(5-(difluoromethoxy)pyrimidin-2-yl)cyclopentane-1,3-diamine hydrochloride (200 mg, 0.712 mmol) was dissolved in DMSO (5 mL). 3-(benzyloxy)-5-bromo-2-fluoropyridine (201 mg, 0.712 mmol) and potassium carbonate (412 mg, 3.56 mmol) were added. Under nitrogen protection, the reaction system was slowly heated to 130 °C and reacted overnight.

After completion of the reaction, water (50 mL) was added, and the mixture was extracted with ethyl acetate (25 mL × 3). The organic phases were combined and concentrated under reduced pressure. The residue was purified by silica gel column chromatography (dichloromethane → dichloromethane/methanol (10/1)) to obtain 0.2 g of a yellow oil, (1S,3S)-$N^1$-(3-(benzyloxy)-5-bromopyridin-2-yl)-$N^3$-(5-(difluoromethoxy)pyrimidin-2-yl)cyclop entane-1,3-diamine. LC-MS: $[M+H]^+$ = 506.

Step B: Synthesis of 5'-(benzyloxy)-6'-((1S,3S)-3-((5-(difluoromethoxy)pyrimidin-2-yl)amino)cyclopentyl)amino)-2 H-[1,3'-bipyridin]-2-one

At room temperature, (1S,3S)-$N^1$-(3-(benzyloxy)-5-bromopyridin-2-yl)-$N^3$-(5-(difluoromethoxy)pyrimidin-2-yl) cyclop entane-1,3-diamine (0.2 g, 0.395 mmol) was dissolved in DMSO (5 mL). 2-Pyridone (56 mg, 0.592 mmol), (1R,2R)-$N^1$,$N^2$-dimethylcyclohexane-1,2-diamine (11 mg, 0.079 mmol), copper(I) iodide (30 mg, 0.158 mmol), and potassium carbonate (109 mg, 0.79 mmol) were added. Under nitrogen protection, the reaction system was heated to 140 °C and reacted overnight.

After completion of the reaction, water (50 mL) was added, and the mixture was extracted with ethyl acetate (25 mL × 3). The organic phases were combined and concentrated under reduced pressure. The residue was purified by reverse-phase C18 column chromatography (water → acetonitrile) to obtain 120 mg of a yellow solid, 5'-(benzyloxy)-6'-((1S,3S)-3-((5-(difluoromethoxy)pyrimidin-2-yl)amino)cyclopentyl)amino)-2 H-[1,3'-bipyridin]-2-one. LC-MS: $[M+H]^+$ = 521.

Step C: Synthesis of 6'-(((1S,3S)-3-((5-(difluoromethoxy)pyrimidin-2-yl)amino)cyclopentyl)amino)-5'-hydroxy-2H-[ 1,3'-bipyridin]-2-one

5'-(benzyloxy)-6'-((1S,3S)-3-((5-(difluoromethoxy)pyrimidin-2-yl)amino)cyclopentyl)amin o)-2H-[1,3'-bipyridin]-2-one (100 mg, 0.192 mmol) was dissolved in methanol (5 mL). 10% palladium on carbon (20 mg) was added. Under hydrogen protection, the mixture was reacted at room temperature.

After completion of the reaction, the solid was filtered off, and the filtrate was concentrated under reduced pressure. The residue was purified by reverse-phase C18 column chromatography (water → acetonitrile) to obtain 27 mg of a yellow solid, 6'-(((1S,3S)-3-((5-(difluoromethoxy)pyrimidin-2-yl)amino)cyclopentyl)amino)-5'-hydroxy-2H-[ 1,3'-bipyridin]-2-one. LC-MS: $[M+H]^+$ = 431. $^1H$ NMR (400 MHz, DMSO-$d_6$) δ 10.19 (s, 1H), 8.22 (s, 2H), 7.58 (dd, J = 6.8, 2.1 Hz, 1H), 7.47 (ddd, J = 10.4, 5.4, 2.2 Hz, 3H), 7.22-6.79 (m, 2H), 6.47-6.39 (m, 1H), 6.26 (td, J = 6.7, 1.4 Hz, 1H), 5.88 (d, J = 7.3 Hz, 1H), 4.46 (q, J = 6.9 Hz, 1H), 4.30 (q, J = 6.8 Hz, 1H), 2.19-2.04 (m, 2H), 1.91 (t, J = 6.9 Hz, 2H), 1.54 (tdd, J = 13.1, 9.0, 5.9 Hz, 2H).

Example A27

Synthesis of 6'-(((1S,3S)-3-((5-(difluoromethoxy)pyrimidin-2-yl)amino)cyclopentyl)amino)-5'-methoxy-2H-[ 1,3'-bipyridin]-2-one

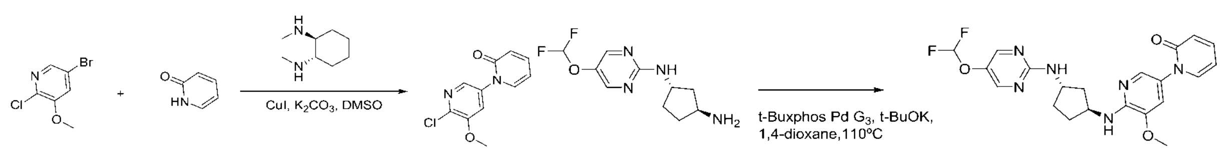


Step A: Synthesis of 6'-chloro-5'-methoxy-2H-[1,3'-bipyridin]-2-one

At room temperature, pyridin-2(1H)-one (0.94 g, 9.89 mmol, 1.1 eq), 5-bromo-2-chloro-3-methoxypyridine (2.0 g, 8.99 mmol, 1.0 eq), copper(I) iodide (0.34 g, 1.8 mmol, 0.2 eq), (1S,2S)-$N^1$,$N^2$-dimethylcyclohexane-1,2-diamine (0.38 g, 2.7 mmol, 0.3 eq), and potassium carbonate (2.48 g, 17.98 mmol, 2.0 eq) were added to dimethyl sulfoxide (20 mL). The solution was purged with nitrogen three times, then reacted at 140 °C for 12 hours.

After completion of the reaction, cold water (100 mL) was added to quench the reaction, followed by extraction with ethyl acetate (100 mL × 3). The organic phases were combined, dried over anhydrous sodium sulfate, filtered, and concentrated. The resulting residue was purified by silica gel column chromatography (eluent: dichloromethane/methanol = 20/1) to obtain 0.6 g of a yellow solid, 6'-chloro-5'-methoxy-2H-[1,3'-bipyridin]-2-one (yield: 28%). LC-MS: $[M+H]^+$ = 237.

Step B: Synthesis of 6'-(((1S,3S)-3-((5-(difluoromethoxy)pyrimidin-2-yl)amino)cyclopentyl)amino)-5'-methoxy-2H-[ 1,3'-bipyridin]-2-one

At room temperature, 6'-chloro-5'-methoxy-2H-[1,3'-bipyridin]-2-one (0.6 g, 2.54 mmol, 1 eq), (1S,3S)-$N^1$-(5-(difluoromethoxy)pyrimidin-2-yl)cyclopentane-1,3-diamine (0.62 g, 2.54 mmol, 1.0 eq), methanesulfonato(2-di-tert-butylphosphino-2',4',6'-triisopropyl-1,1'-biphenyl)(2'-amino-1,1'-biph enyl-2-yl)palladium(II) (0.4 g, 0.51 mmol, 0.2 eq), and potassium tert-butoxide (0.57 g, 5.08 mmol, 2.0 eq) were added to 1,4-dioxane (10 mL). The solution was purged with nitrogen three times, then reacted at 110 °C for 12 hours.

After completion of the reaction, the mixture was diluted with ethyl acetate (50 mL) and filtered through diatomaceous earth. The solid was washed three times with ethyl acetate, and the filtrate was concentrated. The resulting residue was purified by C18 reverse-phase column chromatography (eluent: acetonitrile/water, 45% acetonitrile) to obtain 0.5919 g of a yellow solid, 6'-(((1S,3S)-3-((5-(difluoromethoxy)pyrimidin-2-yl)amino)cyclopentyl)amino)-5'-methoxy-2H-[ 1,3'-bipyridin]-2-one (yield: 52%). LC-MS: $[M+H]^+$ = 445.

NMR data: $^1H$ NMR (400 MHz, DMSO) δ 8.24 (s, 2H), 7.62 (dd, J = 6.8, 2.1 Hz, 1H), 7.56 (d, J = 2.1 Hz, 1H), 7.53-7.44 (m, 2H), 7.25-6.83 (m, 2H), 6.45 (dt, J = 9.2, 1.0 Hz, 1H), 6.28 (td, J = 6.7, 1.4 Hz, 1H), 6.15 (d, J = 7.3 Hz, 1H), 4.51 (p, J = 6.8 Hz, 1H), 4.31 (h, J = 6.7 Hz, 1H), 3.80 (s, 3H), 2.11 (dq, J = 17.6, 7.1, 5.9 Hz, 2H), 1.92 (td, J = 6.8, 1.7 Hz, 2H), 1.68-1.44 (m, 2H).

Example A28

Synthesis of 2-(6-(((1S,3S)-3-((5-(difluoromethoxy)pyrimidin-2-yl)amino)cyclopentyl)amino)-5-fluoropyridi n-3-yl)pyridazin-3(2H)-one

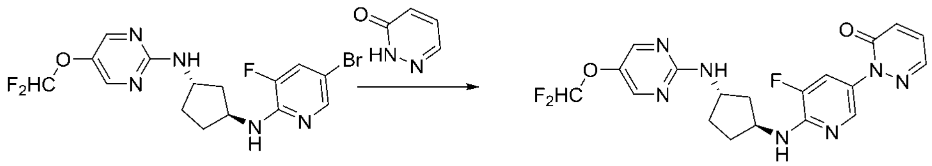

At 20 °C, (1S,3S)-$N^1$-(5-(difluoromethoxy)pyrimidin-2-yl)-$N^3$-(5-iodopyridin-2-yl)cyclopentane-1,3-diami ne (0.4 g, 0.9558 mmol), pyridazin-3(2H)-one (275 mg, 2.867 mmol), and potassium phosphate (608.5 mg, 2.867 mmol) were dissolved in DMSO (20 mL). CuI (72.8 mg, 0.3823 mmol) and (1R,2R)-(-)-N,N'-dimethyl-1,2-cyclohexanediamine (27.19 mg, 0.191 mmol) were added. The mixture was purged with $N_2$ three times, then heated to 130 °C and stirred overnight under a $N_2$ atmosphere.

The reaction mixture was filtered, washed with 50 mL of EA, and then 50 mL of water was added. The mixture was extracted with EA (30 mL × 4). The organic phases were combined, washed with saturated brine (50 mL) and concentrated to obtain the crude product. The crude product was purified by column chromatography (DCM:MeOH = 20:1) to obtain a brown solid, which was further purified by reverse-phase column chromatography to obtain a yellow solid, 2-(6-(((1S,3S)-3-((5-(difluoromethoxy)pyrimidin-2-yl)amino)cyclopentyl)amino)-5-fluoropyridi n-3-yl)pyridazin-3(2H)-one (213 mg, 51.4% yield).

LC-MS: $[M+H]^+$ = 434. $^1H$ NMR (400 MHz, DMSO-$d_6$) δ 8.24 (s, 2H), 8.07-8.00 (m, 2H), 7.63 (dd, J = 12.0, 2.1 Hz, 1H), 7.56-7.45 (m, 2H), 7.24-6.83 (m, 3H), 4.51 (p, J = 7.0 Hz, 1H), 4.33 (p, J = 6.9 Hz, 1H), 2.12 (ddt, J = 15.7, 12.0, 6.0 Hz, 2H), 1.94 (dq, J = 10.7, 6.5 Hz, 2H), 1.69-1.42 (m, 2H).

Example A29

Synthesis of 6'-(((1S,3S)-3-((5-(difluoromethoxy)pyrimidin-2-yl)amino)cyclopentyl)amino)-5'-(difluorometh yl)-2H-[1,3'-bipyridin]-2-one

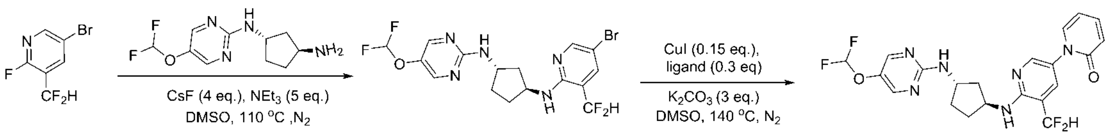


Step A: Synthesis of (1S,3S)-$N^1$-(5-bromo-3-(difluoromethyl)pyridin-2-yl)-$N^3$-(5-(difluoromethoxy)pyrimidin-2-yl)cy clopentane-1,3-diamine

Under nitrogen protection at room temperature, (1S,3S)-$N^1$-(5-(difluoromethoxy)pyrimidin-2-yl)cyclopentane-1,3-diamine (1.16 g, 4.77 mmol, 1.0 eq), 5-bromo-3-(difluoromethyl)-2-fluoropyridine (1.287 g, 5.72 mmol, 1.2 eq), cesium fluoride (2.90 g, 19.08 mmol, 4.0 eq), and triethylamine (2.41 g, 23.85 mmol, 5.0 eq) were dissolved in dimethyl sulfoxide (19 mL). The mixture was heated in an oil bath to 110 °C and reacted overnight.

After completion of the reaction, the mixture was cooled to room temperature, diluted with ethyl acetate (100 mL), and filtered to remove solid impurities. Saturated brine (75 mL) was added for extraction. The separated aqueous phase was further extracted with ethyl acetate (75 mL). The organic phases were combined and concentrated under reduced pressure to dryness. The resulting residue was purified by silica gel column chromatography (eluent: ethyl acetate/n-hexane = 3/1) to obtain 1.89 g of a yellow solid, (1S,3S)-$N^1$-(5-bromo-3-(difluoromethyl)pyridin-2-yl)-$N^3$-(5-(difluoromethoxy)pyrimidin-2-yl)cy clopentane-1,3-diamine (yield: 88%). LC-MS: $[M+H]^+$ = 450.

Step B: Synthesis of 6'-(((1S,3S)-3-((5-(difluoromethoxy)pyrimidin-2-yl)amino)cyclopentyl)amino)-5'-(difluorometh yl)-2H-[1,3'-bipyridin]-2-one

Under nitrogen protection at room temperature, (1S,3S)-$N^1$-(5-bromo-3-(difluoromethyl)pyridin-2-yl)-$N^3$-(5-(difluoromethoxy)pyrimidin-2-yl)cy clopentane-1,3-diamine (1.0 g, 2.23 mmol, 1.0 eq), 2-hydroxypyridine (0.424 g, 4.46

mmol, 2.0 eq), copper(I) iodide (0.064 g, 0.335 mmol, 0.15 eq), $N^1$,$N^2$-bis(2-thienylmethyl)ethanediamide (0.188 g, 0.669 mmol, 0.3 eq), and potassium carbonate (0.923 g, 6.69 mmol, 3.0 eq) were dissolved in dimethyl sulfoxide (9 mL). The mixture was heated in an oil bath to 140 °C and reacted overnight.

After completion of the reaction, the mixture was cooled to room temperature, diluted with ethyl acetate (75 mL), and filtered to remove solid impurities. Saturated brine (50 mL) was added for extraction. The separated aqueous phase was further extracted with ethyl acetate (50 mL). The organic phases were combined and concentrated under reduced pressure to dryness. The resulting residue was purified by silica gel column chromatography (eluent: ethyl acetate) to obtain 0.148 g of a light yellow solid, 6'-(((1S,3S)-3-((5-(difluoromethoxy)pyrimidin-2-yl)amino)cyclopentyl)amino)-5'-(difluorometh yl)-2H-[1,3'-bipyridin]-2-one (yield: 14.3%). LC-MS: $[M+H]^+$ = 465.

Proton NMR: $^1$H NMR (400 MHz, DMSO-$d_6$) δ 8.24 (s, 2H), 8.16 (dd, J = 2.6, 1.4 Hz, 1H), 7.71-7.62 (m, 2H), 7.50 (ddd, J = 8.9, 6.5, 2.0 Hz, 2H), 7.31-6.85 (m, 2H), 6.54-6.43 (m, 2H), 6.29 (td, J = 6.7, 1.3 Hz, 1H), 4.58 (h, J = 7.0 Hz, 1H), 4.33 (h, J = 7.0 Hz, 1H), 2.21-2.07 (m, 2H), 1.94 (t, J = 7.1 Hz, 2H), 1.62-1.49 (m, 2H).

Example A30

Following the method of the preceding Preparation Example, Compound A30 was prepared:

| No. | Structural formula | LC-MS: $[M+H]^+$ | NMR |
|---|---|---|---|
| A30 | 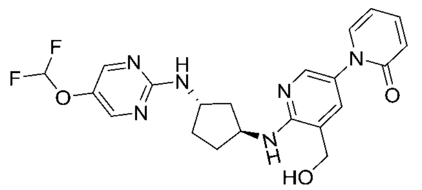 | 445 | $^1$H NMR (400 MHz, DMSO-$d_6$) δ 8.28-8.20 (s, 2H), 7.92-7.87 (d, J = 2.5 Hz, 1H), 7.64-7.37 (m, 4H), 7.24-6.83 (t, J = 74.0 Hz, 1H), 6.49-6.41 (d, J = 9.2 Hz, 1H), 6.31-6.24 (t, J = 6.6 Hz, 1H), 6.03-5.97 (d, J = 6.8 Hz, 1H), 5.45-5.38 (t, J = 5.3 Hz, 1H), 4.57-4.46 (m, 1H), 4.44-4.37 (d, J = 5.4 Hz, 2H), 4.36-4.26 (m, 1H), 2.24-2.04 (m, 2H), 2.01-1.85 (m, 2H), 1.61-1.46 (m, 2H). |

Example A31

Synthesis of 2-(6-(((1S,3S)-3-((5-(difluoromethoxy)pyrimidin-2-yl)amino)cyclopentyl)amino)-5-methylpyridi n-3-yl)pyridazin-3(2H)-one

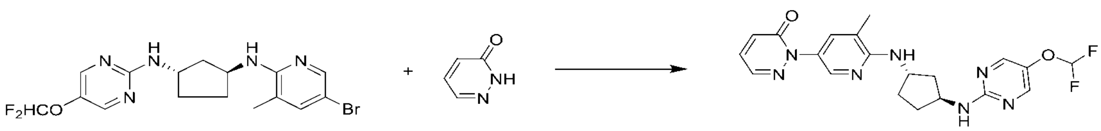

At 25 °C, (1S,3S)-$N^1$-(5-bromo-3-methylpyridin-2-yl)-$N^3$-(5-(difluoromethoxy)pyrimidin-2-yl)cyclopentan e-1,3-diamine (300 mg, 0.729 mmol), pyridazin-3(2H)-one (76.6 mg, 0.797 mmol), potassium phosphate (446 mg, 1.46 mmol), copper(I) iodide (30 mg, 0.146 mmol), and (1R,2R)-(-)-N,N'-dimethyl-1,2-cyclohexanediamine (42 mg, 0.292 mmol) were dissolved in DMSO (10 mL). After addition, the mixture was heated to 140 °C and reacted for 16 hours.

Water (50 mL) was added, followed by extraction with EA (50 mL × 4). The organic phases were combined, washed with saturated brine (50 mL), and concentrated to obtain the crude product. The resulting residue was purified by reverse-phase column chromatography (acetonitrile:water = 5:95 to 60:40) to obtain 130 mg of a white solid, 2-(6-(((1S,3S)-3-((5-(difluoromethoxy)pyrimidin-2-yl)amino)cyclopentyl)amino)-5-methylpyridi n-3-yl)pyridazin-3(2H)-one (0.123 g, 41.8% yield). LC-MS: $[M+H]^+$ = 430.

$^1$H NMR (400 MHz, DMSO-$d_6$) δ 8.24 (s, 2H), 8.04-7.98 (m, 2H), 7.51-7.42 (m, 2H), 7.42-7.37 (m, 1H), 7.07-6.99 (m, 1H), 5.98 (d, J = 7.0 Hz, 1H), 4.54 (h, J = 6.9 Hz, 1H), 4.33 (h, J = 6.9 Hz, 1H), 2.23-2.07 (m, 4H), 1.95 (t, J = 7.1 Hz, 2H), 1.56 (tdd, J = 16.0, 9.3, 4.6 Hz, 2H).

Following the aforementioned method, Compound A31D was prepared: $^1$H NMR (400 MHz, DMSO) δ 8.24 (s, 2H), 8.01 (dd, J = 3.9, 1.6 Hz, 1H), 7.52 - 7.43 (m, 2H), 7.39 (s, 1H), 7.26 - 6.81 (m, 2H), 5.97 (d, J = 7.0 Hz, 1H), 4.55 (q, J = 7.0 Hz, 1H), 4.34 (q, J = 6.9 Hz, 1H), 2.22 - 2.06 (m, 5H), 1.95 (t, J = 7.1 Hz, 2H), 1.66 - 1.48 (m, 2H).

Example A32

Following the method of the preceding preparation example, Compound A32 was prepared:

| | | | |
|---|---|---|---|
| A32 | 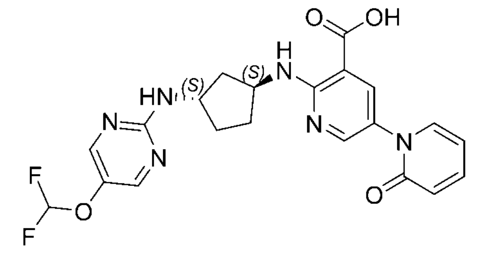 | LC-MS: [M+H]$^+$ =459 | $^1$H NMR (400 MHz, DMSO-$d_6$) δ 8.66-8.50 (s, 1H), 8.27-8.19 (d, J = 11.1 Hz, 3H), 8.05-7.99 (d, J = 2.8 Hz, 1H), 7.69-7.63 (m, 1H), 7.57-7.46 (m, 2H), 7.23-6.83 (t, J = 74.0 Hz, 1H), 6.49-6.43 (d, J = 9.2 Hz, 1H), 6.33-6.26 (m, 1H), 4.62-4.51 (m, 1H), 4.39-4.27 (m ,1H), 2.28-2.08 (m, 2H), 2.06-1.84 (m, 2H), 1.65-1.44 (m, 2H). |

Example A33

Synthesis of 5'-bromo-6'-(((1S,3S)-3-((5-(difluoromethoxy)pyrimidin-2-yl)amino)cyclopentyl)amino)-2H-[1, 3'-bipyridin]-2-one

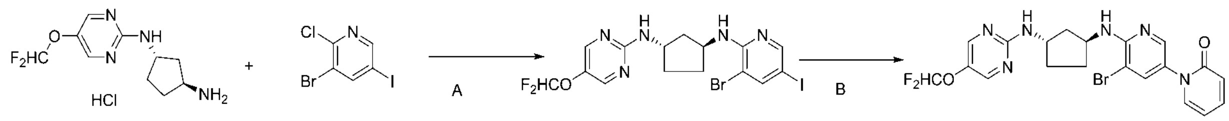


Step A: Synthesis of (1S,3S)-N$^1$-(3-bromo-5-iodopyridin-2-yl)-N$^3$-(5-(difluoromethoxy)pyrimidin-2-yl)cyclopentane-1,3-diamine

At 20 °C, (1S,3S)-N$^1$-(5-(difluoromethoxy)pyrimidin-2-yl)cyclopentane-1,3-diamine hydrochloride (590 mg, 2.10 mmol), 3-bromo-2-chloro-5-iodopyridine (1.00 g, 3.15 mmol), potassium carbonate (0.87 g, 6.30 mmol), and DMSO (20 mL) were added to a 50 mL single-necked flask. The mixture was heated to 100 °C and reacted overnight.

Water (50 mL) was added, followed by extraction with EA (30 mL × 4). The organic phases were combined, washed with saturated brine (50 mL), and concentrated to obtain the crude product. The crude product was purified by column chromatography (DCM:MeOH = 100:3) to obtain a yellow solid, (1S,3S)-N$^1$-(3-bromo-5-iodopyridin-2-yl)-N$^3$-(5-(difluoromethoxy)pyrimidin-2-yl)cyclopentane-1,3-diamine (460 mg, 41.6% yield). LC-MS: [M+H]$^+$ = 526.

Step B: Synthesis of 5'-bromo-6'-(((1S,3S)-3-((5-(difluoromethoxy)pyrimidin-2-yl)amino)cyclopentyl)amino)-2H-[1, 3'-bipyridin]-2-one

(1S,3S)-N$^1$-(3-bromo-5-iodopyridin-2-yl)-N$^3$-(5-(difluoromethoxy)pyrimidin-2-yl)cyclopen tane-1,3-diamine (360 mg, 0.68 mmol), 1,2-dihydropyridin-2-one (0.19 g, 2.04 mmol), potassium phosphate (0.14 g, 0.68 mmol), trans-N,N'-dimethyl-1,2-cyclohexanediamine (0.039 g, 0.27 mmol), CuI (0.052 g, 0.27 mmol), and DMSO (10 mL) were added to a reaction flask. The mixture was heated to 130 °C and reacted overnight.

Water (50 mL) was added, and the mixture was extracted with EA (30 mL × 5). The organic phases were combined, concentrated, and the solvent was removed. The residue was purified by column chromatography (DCM/MeOH = 10/1) to obtain 130 mg of crude product, which was further purified by preparative chromatography to obtain the product, 5'-bromo-6'-(((1S,3S)-3-((5-(difluoromethoxy)pyrimidin-2-yl)amino)cyclopentyl)amino)-2H-[1, 3'-bipyridin]-2-one (25 mg, Yield 7.41%) as a white solid. LC-MS: [M+H]$^+$ = 493.

$^1$H NMR (400 MHz, DMSO-$d_6$) δ 8.24 (s, 2H), 8.04 (d, J = 2.3 Hz, 1H), 7.90 (d, J = 2.3 Hz, 1H), 7.65 (dd, J = 6.9, 2.0 Hz, 1H), 7.53-7.47 (m, 2H), 7.03 (s, 1H), 6.49-6.44 (m, 1H), 6.29 (ddd, J = 8.9, 5.1, 1.8 Hz, 2H), 4.54 (q, J = 7.2 Hz, 1H), 4.39-4.30 (m, 1H), 2.21-2.06 (m, 2H), 2.03-1.92 (m, 2H), 1.69-1.50 (m, 2H).

Example A34 (Compound A31D)

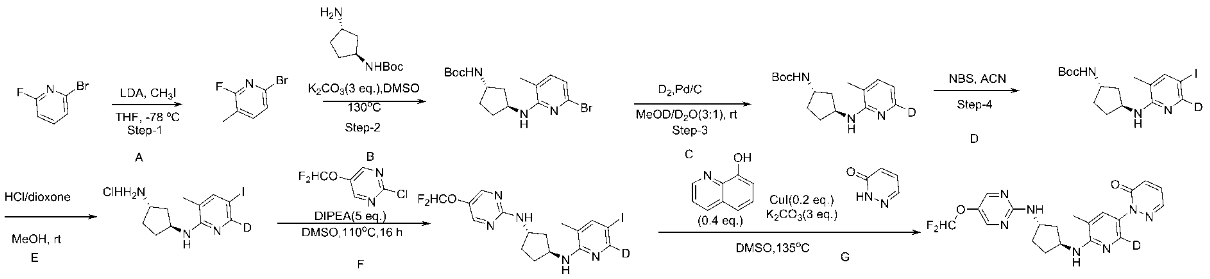


NMR Data: $^{1}$H NMR (400 MHz, DMSO) δ 8.24 (s, 2H), 8.01 (dd, J = 3.9, 1.6 Hz, 1H), 7.52-7.43 (m, 2H), 7.39 (s, 1H), 7.26-6.81 (m, 2H), 5.97 (d, J = 7.0 Hz, 1H), 4.55 (q, J = 7.0 Hz, 1H), 4.34 (q, J = 6.9 Hz, 1H), 2.22-2.06 (m, 5H), 1.95 (t, J = 7.1 Hz, 2H), 1.66-1.48 (m, 2H).

Example A35

Synthesis of 6'-(((1S,3S)-3-((5-(difluoromethoxy)pyrimidin-2-yl)amino)cyclopentyl)amino)-3-(1-hydroxyeth yl)-2H-[1,3'-bipyridin]-2-one (Compound A35)

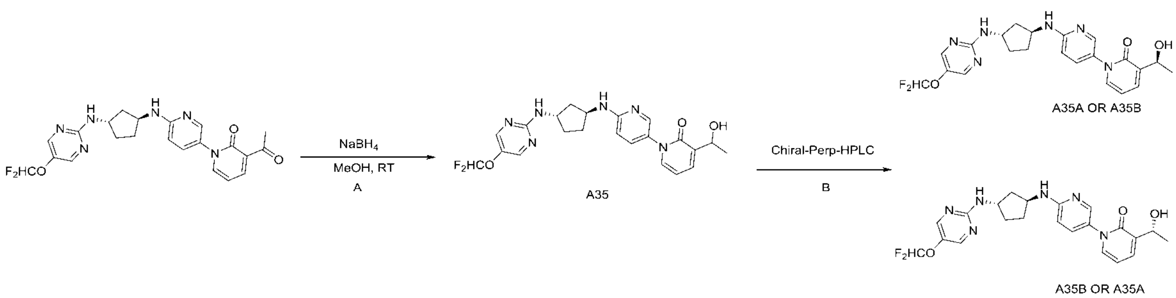


Step A: Synthesis of 6'-(((1S,3S)-3-((5-(difluoromethoxy)pyrimidin-2-yl)amino)cyclopentyl)amino)-3-(1-hydroxyeth yl)-2H-[1,3'-bipyridin]-2-one

At room temperature, 3-acetyl-1-(6-{[(1S,3S)-3-[(5-(difluoromethoxy)pyrimidin-2-yl)amino]cyclopentyl]amino} pyridi n-3-yl)-1,2-dihydropyridin-2-one (800 mg, 1.75 mmol) was dissolved in methanol (10 mL). Sodium borohydride (86.06 mg, 2.27 mmol) was added, and the mixture was maintained at room temperature and reacted for 1 hour.

After completion of the reaction, saturated aqueous ammonium chloride solution (2 mL) was added to quench the reaction. The mixture was then concentrated by rotary evaporation to a residue. The resulting residue was purified by C18 reverse-phase column chromatography (ACN/$H_2O$, 40% ACN) to obtain a light yellow mixture of 6'-(((1S,3S)-3-((5-(difluoromethoxy)pyrimidin-2-yl)amino)cyclopentyl)amino)-3-(1-hydroxyeth yl)-2H-[1,3'-bipyridin]-2-one (570 mg, yield 70%).

Step B: Synthesis of 6'-(((1S,3S)-3-((5-(difluoromethoxy)pyrimidin-2-yl)amino)cyclopentyl)amino)-3-((S)-1-hydroxy ethyl)-2H-[1,3'-bipyridin]-2-one and 6'-(((1S,3S)-3-((5-(difluoromethoxy)pyrimidin-2-yl)amino)cyclopentyl)amino)-3-((R)-1-hydrox yethyl)-2H-[1,3'-bipyridin]-2-one

The mixture obtained from Step A was subjected to chiral separation (column model: CHIRALCEL OD-H 5 um 10 mm*250 mm; mobile phase: n-hexane/ethanol = 70:30; flow rate: 5 mL/min; column temperature: 30 °C) to obtain a white solid front peak (RT=12.69 min) and a rear peak of 213 mg (RT=16.03 min). LC-MS: $[M+H]^+$ = 459.

NMR Data:

Compound with RT=12.69 min: $^{1}$H NMR (400 MHz, DMSO) δ 8.24 (s, 2H), 7.91 (d, J = 2.6 Hz, 1H), 7.58-7.45 (m, 3H), 7.39 (dd, J = 8.9, 2.6 Hz, 1H), 7.26-6.82 (m, 2H), 6.52 (d, J = 8.9 Hz, 1H), 6.32 (t, J = 6.9 Hz, 1H), 5.11 (d, J = 4.5 Hz, 1H), 4.71 (p, J = 6.0 Hz, 1H), 4.31 (q, J = 7.0 Hz, 2H), 2.13 (p, J = 8.1, 7.5 Hz, 2H), 1.90 (hept, J = 6.9 Hz, 2H), 1.53 (d, J = 21.6 Hz, 2H), 1.26 (d, J = 6.4 Hz, 3H).

Compound with RT=16.03 min: $^1$H NMR (400 MHz, DMSO) δ 8.24 (s, 2H), 7.91 (d, J = 2.7 Hz, 1H), 7.52 (td, J = 7.8, 7.2, 2.2 Hz, 3H), 7.39 (dd, J = 8.9, 2.7 Hz, 1H), 7.25-6.82 (m, 2H), 6.52 (d, J = 8.9 Hz, 1H), 6.32 (t, J = 6.8 Hz, 1H), 5.08 (d, J = 4.6 Hz, 1H), 4.71 (p, J = 6.0 Hz, 1H), 4.30 (p, J = 6.9 Hz, 2H), 2.21-2.04 (m, 2H), 1.98-1.80 (m, 2H), 1.52 (td, J = 13.1, 11.9, 6.9 Hz, 2H), 1.26 (d, J = 6.3 Hz, 3H).

Example A36

Synthesis of 6'-(((1S,3S)-3-((5-(difluoromethoxy)pyrimidin-2-yl)amino)cyclopentyl)amino)-2-oxo-2H-[1,3'-b ipyridine]-3-carboxamide

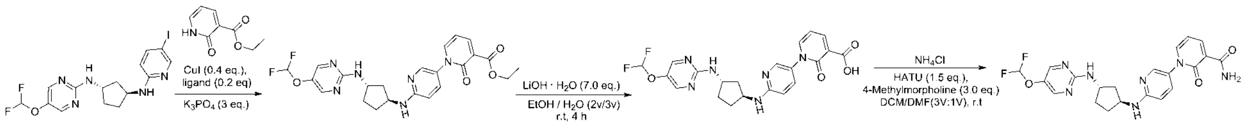


Step A: Synthesis of ethyl 6'-(((1S,3S)-3-((5-(difluoromethoxy)pyrimidin-2-yl)amino)cyclopentyl)amino)-2-oxo-2H-[1,3'-b ipyridine]-3-carboxylate

Under nitrogen protection at room temperature, (1S,3S)-N$^1$-(5-(difluoromethoxy)pyrimidin-2-yl)-N$^3$-(5-iodopyridin-2-yl)cyclopentane-1,3-diami ne (6.71 g, 15 mmol, 1.0 eq), ethyl 2-oxo-1,2-dihydropyridine-3-carboxylate (5.01 g, 30 mmol, 2 eq), copper(I) iodide (1.14 g, 6.0 mmol, 0.4 eq), N$^1$,N$^2$-dimethylcyclohexane-1,2-diamine (0.43 g, 3.0 mmol, 0.2 eq), and potassium phosphate (9.55 g, 45 mmol, 3.0 eq) were dissolved in dimethyl sulfoxide (60 mL), and the mixture was heated to 140 °C and reacted overnight.

After completion of the reaction, cold water (150 mL) was added to quench the reaction, followed by extraction with ethyl acetate (250 mL × 3). The organic phases were combined, dried over anhydrous sodium sulfate, filtered, and concentrated. The resulting residue was purified by silica gel column chromatography (eluent: ethyl acetate) to obtain 2.92 g of a yellow solid, ethyl 6'-(((1S,3S)-3-((5-(difluoromethoxy)pyrimidin-2-yl)amino)cyclopentyl)amino)-2-oxo-2H-[1,3'-b ipyridine]-3-carboxylate. LC-MS: $[M+H]^+$ = 487.

Step B: Synthesis of 6'-(((1S,3S)-3-((5-(difluoromethoxy)pyrimidin-2-yl)amino)cyclopentyl)amino)-2-oxo-2H-[1,3'-b ipyridine]-3-carboxylic acid

At room temperature, ethyl 6'-(((1S,3S)-3-((5-(difluoromethoxy)pyrimidin-2-yl)amino)cyclopentyl)amino)-2-oxo-2H-[1,3'-b ipyridine]-3-carboxylate (2.92 g, 6.37 mmol, 1.0 eq) and lithium hydroxide monohydrate (1.90 g, 44.6 mmol, 7.0 eq) were dissolved in a mixed solvent of ethanol (6 mL) and water (9 mL). The mixture was stirred at room temperature for 4 hours.

After completion of the reaction, the pH of the reaction mixture was adjusted to approximately 6 using 1M dilute hydrochloric acid solution, resulting in the precipitation of a large amount of light yellow solid. The resultant was filtrated to obtain 2.63 g of 6'-(((1S,3S)-3-((5-(difluoromethoxy)pyrimidin-2-yl)amino)cyclopentyl)amino)-2-oxo-2H-[1,3'-b ipyridine]-3-carboxylic acid. LC-MS: $[M+H]^+$ = 459.

Step C: Synthesis of 6'-(((1S,3S)-3-((5-(difluoromethoxy)pyrimidin-2-yl)amino)cyclopentyl)amino)-2-oxo-2H-[1,3'-b ipyridine]-3-carboxamide

At room temperature, 6'-(((1S,3S)-3-((5-(difluoromethoxy)pyrimidin-2-yl)amino)cyclopentyl)amino)-2-oxo-2H-[1,3'-b ipyridine]-3-carboxylic acid (0.25 g, 0.44 mmol, 1.0 eq), O-(7-azabenzotriazol-1-yl)-N,N,N',N'-tetramethyluronium hexafluorophosphate (HATU, 0.251 g, 0.66 mmol, 1.5 eq), and N-methylmorpholine (0.135 g, 1.32 mmol, 3.0 eq) were dissolved in a mixed solvent of dichloromethane (3 mL) and N,N-dimethylformamide (1 mL). The mixture was stirred vigorously for 15 minutes. Subsequently, ammonium chloride (0.036 g, 0.66 mmol, 1.5 eq) was added, and the mixture was stirred at room temperature for 4 hours.

After completion of the reaction, the solvent was removed under reduced pressure by rotary evaporation. The resulting crude product was dissolved in an appropriate amount of dimethyl sulfoxide and purified by C18 reverse-phase column (120 g specification, mobile phase: acetonitrile/water) to obtain 140 mg of a red solid, 6'-(((1S,3S)-3-((5-difluoromethoxy)pyrimidin-2-yl)amino)cyclopentyl)amino)-2-oxo-2H-[1,3'-b ipyridine]-3-carboxamide (70% yield). LC-MS: $[M+H]^+$ = 458.

NMR data: $^1$H NMR (400 MHz, DMSO-$d_6$) δ 8.91 (d, J = 4.4 Hz, 1H), 8.39 (dd, J = 7.3, 2.2 Hz, 1H), 8.23 (s, 2H),

8.05 - 7.92 (m, 2H), 7.61 (d, J = 4.4 Hz, 1H), 7.54 - 7.42 (m, 2H), 7.22 - 6.85 (m, 2H), 6.62 - 6.49 (m, 2H), 4.32 (p, J = 6.7 Hz, 2H), 2.13 (tt, J = 11.9, 7.0 Hz, 2H), 1.97 - 1.83 (m, 2H), 1.52 (ddt, J = 18.9, 14.1, 6.3 Hz, 2H).

Example A37

Synthesis of 6'-(((1S,3S)-3-((5-(difluoromethoxy)pyrimidin-2-yl)amino)cyclopentyl)amino)-N-methyl-2-oxo-2H-[1,3'-bipyridine]-3-carboxamide

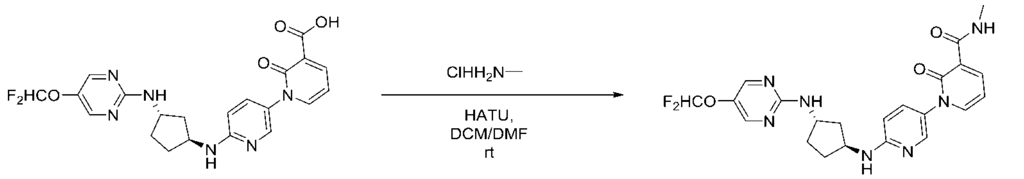


At room temperature, 6'-(((1S,3S)-3-((5-(difluoromethoxy)pyrimidin-2-yl)amino)cyclopentyl)amino)-2-oxo-2H-[1,3'-b ipyridine]-3-carboxylic acid (200 mg, 0.42 mmol) was dissolved in DCM/DMF (6/2 mL). HATU (150 mg, 0.42 mmol) and N-methylmorpholine (200 μL, 2.9 mmol) were added, and the mixture was stirred at room temperature for 10 minutes. Methylamine hydrochloride (30 mg, 0.48 mmol) was then added, and the mixture was stirred at room temperature for 2 hours.

After completion of the reaction, water (20 mL) was added, and the mixture was extracted with DCM (20 mL × 4). The organic phases were combined and purified by reverse-phase column chromatography (CAN/$H_2O$ = 40%) to obtain a yellow solid, 6'-(((1S,3S)-3-((5-(difluoromethoxy)pyrimidin-2-yl)amino)cyclopentyl)amino)-N-methyl-2-oxo-2H-[1,3'-bipyridine]-3-carboxamide, yield 81.27%. LC-MS: $[M+H]^+$ = 472. $^1H$ NMR (400 MHz, DMSO-$d_6$) δ 9.47 (q, J = 4.8 Hz, 1H), 8.40 (dd, J = 7.3, 2.2 Hz, 1H), 8.24 (s, 2H), 7.97 (dd, J = 7.8, 2.5 Hz, 2H), 7.51 (d, J = 7.2 Hz, 1H), 7.47 - 7.38 (m, 1H), 7.25 - 6.70 (m, 2H), 6.61 - 6.51 (m, 2H), 4.33 (p, J = 7.0 Hz, 2H), 2.81 (d, J = 4.8 Hz, 3H), 2.19 - 2.08 (m, 2H), 1.98 - 1.81 (m, 2H), 1.53 (td, J = 13.7, 11.9, 7.0 Hz, 2H).

Example A38

Synthesis of 2'-(((1S,3S)-3-((5-(difluoromethoxy)pyrimidin-2-yl)amino)cyclopentyl)amino)-3-(morpholine-4-carbonyl)-2H-[1,4'-bipyridin]-2-one

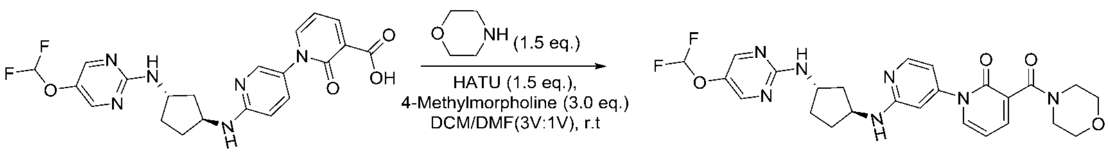


At room temperature, 6'-(((1S,3S)-3-((5-(difluoromethoxy)pyrimidin-2-yl)amino)cyclopentyl)amino)-2-oxo-2H-[1,3'-b ipyridine]-3-carboxylic acid (0.25 g, 0.44 mmol, 1.0 eq), O-(7-azabenzotriazol-1-yl)-N,N,N',N'-tetramethyluronium hexafluorophosphate (HATU, 0.251 g, 0.66 mmol, 1.5 eq), and N-methylmorpholine (0.135 g, 1.32 mmol, 3.0 eq) were dissolved in a mixed solvent of dichloromethane (3 mL) and N,N-dimethylformamide (1 mL). The mixture was stirred vigorously for 15 minutes. Subsequently, morpholine (0.058 g, 0.66 mmol, 1.5 eq) was added, and the mixture was stirred at room temperature for 4 hours.

After completion of the reaction, the solvent was removed under reduced pressure by rotary evaporation. The resulting crude product was dissolved in an appropriate amount of dimethyl sulfoxide and purified by C18 reverse-phase column (120 g specification, mobile phase: acetonitrile/water) to obtain 164 mg of a light yellow solid, 2'-(((1S,3S)-3-((5-(difluoromethoxy)pyrimidin-2-yl)amino)cyclopentyl)amino)-3-(morpholine-4-carbonyl)-2H-[1,4'-bipyridin]-2-one (71% yield). LC-MS: $[M+H]^+$ = 528.

NMR data: $^1H$ NMR (400 MHz, DMSO-$d_6$) δ 8.23 (s, 2H), 7.95 (d, J = 2.7 Hz, 1H), 7.74 (dd, J = 6.8, 2.1 Hz, 1H), 7.63 - 7.37 (m, 3H), 7.23 - 6.85 (m, 2H), 6.53 (d, J = 8.9 Hz, 1H), 6.36 (t, J = 6.8 Hz, 1H), 4.30 (p, J = 6.8 Hz, 2H), 3.56 (ddt, J = 12.6, 9.0, 5.0 Hz, 6H), 3.25 (t, J = 4.7 Hz, 2H), 2.20 - 2.04 (m, 2H), 1.88 (tdd, J = 13.2, 10.4, 6.3 Hz, 2H), 1.60 - 1.43 (m, 2H).

Example A39

Synthesis of 3-acetyl-6'-(((1S,3S)-3-((5-(difluoromethoxy)pyrimidin-2-yl)amino)cyclopentyl)amino)-2H-[1,3' -bipyridin]-2-one

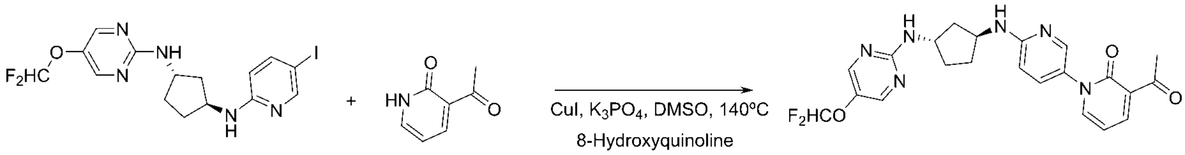


At room temperature, (1S,3S)-$N^1$-(5-(difluoromethoxy)pyrimidin-2-yl)-$N^3$-(5-iodopyridin-2-yl)cyclopentane-1,3-diami ne (2.75 g, 6.15 mmol), 3-acetyl-1,2-dihydropyridin-2-one (1.69 g, 12.3 mmol), copper(I) iodide (0.23 g, 1.23 mmol), 8-hydroxyquinoline (0.36 g, 2.46 mmol), and potassium phosphate (3.92 g, 18.45 mmol) were added to dimethyl sulfoxide (20 mL). The mixture was purged with nitrogen three times, and then heated in an oil bath at 140 °C and reacted for 8 hours.

After completion of the reaction, the reaction mixture was filtered through diatomaceous earth, and the solid was washed with ethyl acetate (3 × 50 mL). Water (150 mL) was added to the filtrate, and the mixture was extracted with ethyl acetate (3 × 80 mL). The organic phases were combined, washed with saturated brine (3 × 50 mL), dried over anhydrous sodium sulfate, filtered, and concentrated by rotary evaporation to a residue. The residue was purified by silica gel column chromatography (eluent: n-hexane/ethyl acetate = 0/100%, V/V) to obtain a yellow-green solid, 3-acetyl-6'-(((1S,3S)-3-((5-(difluoromethoxy)pyrimidin-2-yl)amino)cyclopentyl)amino)-2H-[1,3' -bipyridin]-2-one (1.51 g, Yield 51.11 %, Purity 95%). LC-MS: $[M+H]^+$ = 457.

NMR data: $^1$H NMR (400 MHz, DMSO) δ 8.24 (s, 2H), 8.07 (dd, J = 7.3, 2.2 Hz, 1H), 8.00 (dd, J = 6.6, 2.3 Hz, 1H), 7.97 (d, J = 2.7 Hz, 1H), 7.51 (d, J = 7.2 Hz, 1H), 7.45 (dd, J = 8.9, 2.7 Hz, 1H), 7.26 - 6.82 (m, 2H), 6.55 (d, J = 8.9 Hz, 1H), 6.44 (t, J = 6.9 Hz, 1H), 4.32 (h, J = 6.7 Hz, 2H), 2.53 (s, 3H), 2.23 - 2.06 (m, 2H), 1.89 (tdd, J = 13.2, 10.5, 6.3 Hz, 2H), 1.52 (ddt, J = 16.4, 14.0, 9.2 Hz, 2H).

Example A40

Synthesis of 6'-(((1S,3S)-3-((5-(difluoromethoxy)pyrimidin-2-yl)amino)cyclopentyl)amino)-2'-(difluorometh yl)-2H-[1,3'-bipyridin]-2-one

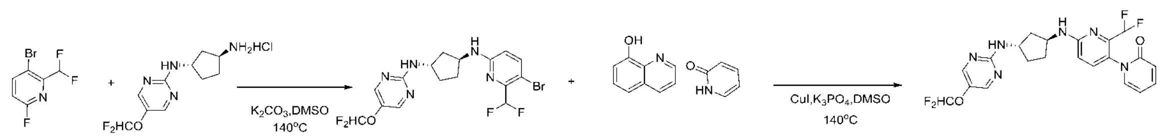


Step A: Synthesis of (1S,3S)-$N^1$-(5-bromo-6-(difluoromethyl)pyridin-2-yl)-$N^3$-(5-(difluoromethoxy)pyrimidin-2-yl)cy clopentane-1,3-diamine

At room temperature, (1S,3S)-$N^1$-(5-(difluoromethoxy)pyrimidin-2-yl)cyclopentane-1,3-diamine hydrochloride (500 mg, 1.5 mmol) was dissolved in DMSO (10 mL). 3-Bromo-2-(difluoromethyl)-6-fluoropyridine (382 mg, 1.7 mmol) and potassium carbonate (1.2 g, 8.6 mmol) were added. Under nitrogen protection, the reaction system was heated to 140 °C and stirred overnight.

After completion of the reaction, water (100 mL) was added, and the mixture was extracted with DCM (50 mL × 4). The organic phases were combined and purified by silica gel column chromatography (EA/Hex = 1:1, 50%) to obtain 635 mg of a yellow solid, (1S,3S)-$N^1$-(5-bromo-6-(difluoromethyl)pyridin-2-yl)-$N^3$-(5-(difluoromethoxy)pyrimidin-2-yl)cy clopentane-1,3-diamine (yield: 94.3%). LC-MS: $[M+H]^+$ = 450.

Step B: Synthesis of 6'-(((1S,3S)-3-((5-(difluoromethoxy)pyrimidin-2-yl)amino)cyclopentyl)amino)-2'-(difluorometh yl)-2H-[1,3'-bipyridin]-2-one

At room temperature, (1S,3S)-$N^1$-(5-bromo-6-(difluoromethyl)pyridin-2-yl)-$N^3$-(5-(difluoromethoxy)pyrimidin-2-yl)cy clopentane-1,3-diamine (615 mg, 1.23 mmol) was dissolved in DMSO (10 mL). Pyridin-2(1H)-one (351 mg, 3.7 mmol), 8-hydroxyquinoline (53 mg, 0.369 mmol), copper(I) iodide (70 mg, 0.37 mmol), and potassium carbonate (389 mg, 2.46 mmol) were added. Under nitrogen protection, the reaction system was slowly heated to 140 °C and reacted overnight.

After completion of the reaction, water (100 mL) was added, and the mixture was extracted with ethyl acetate (80 mL × 3). The organic phases were combined, concentrated under reduced pressure, and the residue was purified by reverse-phase column chromatography (ACN/$H_2O$ = 50%). Lyophilization to obtain 24 mg of a yellow solid product, 6'-(((1S,3S)-3-((5-(difluoromethoxy)pyrimidin-2-yl)amino)cyclopentyl)amino)-2'-(difluorometh yl)-2H-[1,3'-bipyridin]-2-one (yield: 44%). LC-MS: $[M+H]^+$ = 465. $^1H$ NMR (400 MHz, DMSO-$d_6$) δ 8.24 (s, 2H), 7.52 (d, J = 8.1 Hz, 3H), 7.39 (d, J = 8.8 Hz, 1H), 7.31 (d, J = 6.7 Hz, 1H), 7.04 (t, J = 74.0 Hz, 1H), 6.70 (d, J = 8.8 Hz, 1H), 6.62 - 6.33 (m, 2H), 6.29 (t, J = 6.9 Hz, 1H), 4.34 - 4.29 (m, 2H), 2.26 - 2.05 (m, 2H), 1.93 (t, J = 7.0 Hz, 2H), 1.53 (s, 2H).

Example A41

Synthesis of 6'-(((1S,3S)-3-((5-(difluoromethoxy)pyrimidin-2-yl)amino)cyclopentyl)amino)-2'-(fluoromethyl) -2H-[1,3'-bipyridin]-2-one

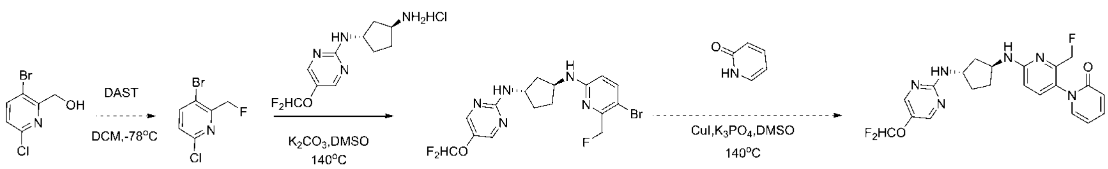


Step A: Synthesis of 3-bromo-6-chloro-2-(fluoromethyl)pyridine

At room temperature, (3-bromo-6-chloropyridin-2-yl)methanol (500 mg, 2.45 mmol) was dissolved in DCM (20 mL). Under ice bath conditions, DAST (789 μL, 4.90 mmol) was added, and the mixture was stirred at 0 °C for 3 hours.

After completion of the reaction, water (100 mL) was added, and the mixture was extracted with DCM (50 mL × 4). The organic phases were combined and purified by silica gel column chromatography (EA/Hex = 1:9, 10%) to obtain 420 mg of a yellow oily liquid, 3-bromo-6-chloro-2-(fluoromethyl)pyridine (yield: 82.8%). LC-MS: $[M+H]^+$ = 207.

Step B: Synthesis of (1S,3S)-$N^1$-(5-bromo-6-(fluoromethyl)pyridin-2-yl)-$N^3$-(5-(difluoromethoxy)pyrimidin-2-yl)cycl opentane-1,3-diamine

At room temperature, (1S,3S)-$N^1$-(5-(difluoromethoxy)pyrimidin-2-yl)cyclopentane-1,3-diamine hydrochloride (380 mg, 1.55 mmol) was dissolved in DMSO (10 mL). 3-Bromo-6-chloro-2-(fluoromethyl)pyridine (420 mg, 1.87 mmol) and potassium carbonate (1.29 g, 9.4 mmol) were added. Under nitrogen protection, the reaction system was heated to 140 °C and stirred overnight.

After completion of the reaction, water (100 mL) was added, and the mixture was extracted with DCM (50 mL × 4). The organic phases were combined and purified by silica gel column chromatography (EA/Hex = 1:1, 50%) to obtain 206 mg of a yellow oily liquid, (1S,3S)-$N^1$-(5-bromo-6-(fluoromethyl)pyridin-2-yl)-$N^3$-(5-(difluoromethoxy)pyrimidin-2-yl)cycl opentane-1,3-diamine (yield: 30.6%). LC-MS: $[M+H]^+$ = 433.

Step C: Synthesis of 6'-(((1S,3S)-3-((5-(difluoromethoxy)pyrimidin-2-yl)amino)cyclopentyl)amino)-2'-(fluoromethyl) -2H-[1,3'-bipyridin]-2-one

At room temperature, (1S,3S)-$N^1$-(5-bromo-6-(fluoromethyl)pyridin-2-yl)-$N^3$-(5-(difluoromethoxy)pyrimidin-2-yl)cycl opentane-1,3-diamine (189 mg, 0.44 mmol) was dissolved in DMSO (10 mL). Pyridin-2(1H)-one (130 mg, 1.3 mmol), 8-hydroxyquinoline (53 mg, 0.369 mmol), copper(I) iodide (25 mg, 0.13 mmol), and potassium carbonate (120 mg, 0.88 mmol) were added. Under nitrogen protection, the reaction system was slowly heated to 140 °C and reacted overnight.

After completion of the reaction, water (100 mL) was added, and the mixture was extracted with ethyl acetate (80 mL × 3). The organic phases were combined, concentrated under reduced pressure, and the residue was purified by reverse-phase column chromatography (ACN/$H_2O$ = 50%). Lyophilization to obtain 20 mg of a yellow solid product, 6'-(((1S,3S)-3-((5-(difluoromethoxy)pyrimidin-2-yl)amino)cyclopentyl)amino)-2'-(fluoromethyl) -2H-[1,3'-bipyridin]-2-one (yield: 10%).

LC-MS: $[M+H]^+$ = 447. $^1$H NMR (400 MHz, DMSO-$d_6$) δ 8.24 (s, 2H), 8.09 (dd, J = 5.0, 2.0 Hz, 1H), 7.83 (ddd, J = 8.7, 7.1, 2.0 Hz, 1H), 7.49 (d, J = 7.2 Hz, 1H), 7.27 (d, J = 8.9 Hz, 1H), 7.09 (dd, J = 7.2, 5.0 Hz, 1H), 7.04 (s, 1H), 7.02 (t, 1H), 6.74 (d, J = 6.8 Hz, 1H), 6.57 (dd, J = 8.9, 2.3 Hz, 1H), 5.14 (d, J = 47.9 Hz, 2H), 4.29 (dq, J = 18.4, 6.8 Hz, 2H), 2.14 (ddd, J = 21.9, 10.6, 4.8 Hz, 2H), 1.90 (td, J = 6.8, 3.0 Hz, 2H), 1.52 (ddt, J = 18.8, 12.8, 8.1 Hz, 2H).

Example A42

Synthesis of 6'-(((1S,3S)-3-((5-(difluoromethoxy)pyrimidin-2-yl)amino)cyclopentyl)amino)-3-(1H-tetrazol-5-yl)-2H-[1,3'-bipyridin]-2-one

Synthetic Route

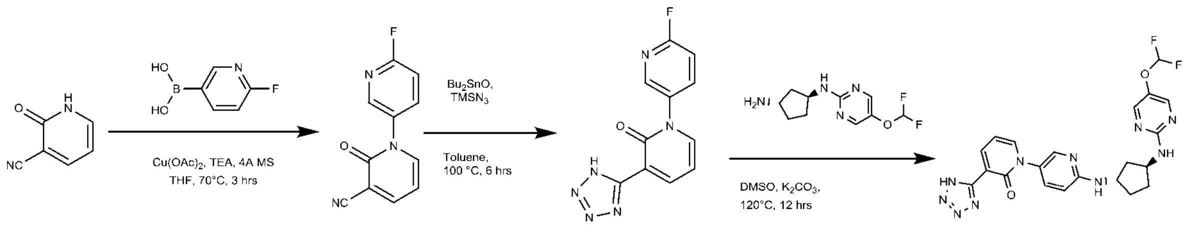


Step A: Synthesis of 6'-fluoro-2-oxo-2H-[1,3'-bipyridine]-3-carbonitrile

2-Oxo-1,2-dihydropyridine-3-carbonitrile (6.50 g, 54.1 mmol, 1 eq), (6-fluoropyridin-3-yl)boronic acid (15.3 g, 108 mmol, 2 eq), copper(II) acetate (19.7 g, 108 mmol, 2 eq), and triethylamine (27.4 g, 271 mmol, 5 eq) were dissolved in tetrahydrofuran (150 mL), and then 4A molecular sieves (1.00 g) were added. After the addition, the mixture was stirred at 70 °C for 3 hours. LCMS detected the MS value of the product (RT = 0.323 min). The reaction mixture was filtered through diatomaceous earth, and the filter cake was washed with dichloromethane several times. The filtrate was concentrated under reduced pressure to obtain a crude product. The crude product was purified by silica gel column chromatography to obtain 6'-fluoro-2-oxo-2H-[1,3'-bipyridine]-3-carbonitrile (450 mg, 2.09 mmol, yield 3.86%). MS (ESI) m/z $[M+1]^+$= 216.1.

Step B: Synthesis of 6'-fluoro-3-(1H-tetrazol-5-yl)-2H-[1,3'-bipyridin]-2-one

6'-Fluoro-2-oxo-2H-[1,3'-bipyridine]-3-carbonitrile (350 mg, 1.63 mmol, 1 eq), trimethylsilyl azide (562 mg, 4.88 mmol, 3 eq), and dibutyltin oxide (203 mg, 813 μmol, 2.5 eq) were dissolved in toluene (10 mL), and then the mixture was stirred at 100 °C for 6 hours. LCMS detected the MS value of the product (RT = 0.342 min). The reaction mixture was concentrated under reduced pressure to obtain a crude product. The crude product was purified by reverse-phase preparative chromatography (Column: Phenomenex luna C18 150*25 mm* 10 um; Mobile phase: [water (TFA)-ACN]; Gradient: 5% to 38% B over min) to obtain 6'-fluoro-3-(1H-tetrazol-5-yl)-2H-[1,3'-bipyridin]-2-one (180 mg, 697 μmol, yield 42.9%). MS (ESI) m/z $[M+1]^+$= 259.1.

Step C: Synthesis of 6'-(((1S,3S)-3-((5-(difluoromethoxy)pyrimidin-2-yl)amino)cyclopentyl)amino)-3-(1H-tetrazol-5-yl)-2H-[1,3'-bipyridin]-2-one

6'-Fluoro-3-(1H-tetrazol-5-yl)-2H-[1,3'-bipyridin]-2-one (180 mg, 697 μmol, 1 eq), (1S,3S)-$N^3$-[5-(difluoromethoxy)pyrimidin-2-yl]cyclopentane-1,3-diamine hydrochloride (235 mg, 837 μmol, 1.2 eq, hydrochloride salt), and potassium carbonate (289 mg, 2.09 mmol, 3 eq) were dissolved in dimethyl sulfoxide (10 mL), and then the mixture was stirred at 120 °C for 12 hours. LCMS detected the MS value of the product (RT = 0.396 min). The reaction mixture was cooled to room temperature, and then filtered. The filtrate was concentrated under reduced pressure to obtain a crude product. The crude product was purified by reverse-phase preparative chromatography (Column: Waters Xbridge 150*25mm* 5um; Mobile phase: [water (ammonium hydroxide v/v)-ACN]; Gradient: 0% to 25% B over 10 min) to obtain 6'-(((1S,3S)-3-((5-(difluoromethoxy)pyrimidin-2-yl)amino)cyclopentyl)amino)-3-(1H-tetrazol-5-yl)-2H-[1,3'-bipyridin]-2-one (68.0 mg, 134 μmol, 19.3% yield, 95.4% purity).

RT = 0.396 min, MS (ESI) m/z $[M+1]^+$ = 483.2, $^1$H NMR: (400 MHz, DMSO-$d_6$): δ 8.46 - 8.39 (m, 1H), 8.23 (s, 2H), 8.04 (d, J = 2.6 Hz, 1H), 7.95 (dd, J = 2.0, 6.6 Hz, 1H), 7.55 - 7.45 (m, 2H), 7.24 - 6.82 (m, 2H), 6.61 - 6.52 (m, 2H), 4.38 - 4.26

(m, 2H), 2.21 - 2.08 (m, 2H), 1.98 - 1.82 (m, 2H), 1.61 - 1.44 (m, 2H).

Example A43

Referring to the aforementioned preparation method, Compound A43 and its isomers A43A, A43B, A43A-D, and A43B-D were prepared:

| | Structural formula | NMR data |
|---|---|---|
| A43 | 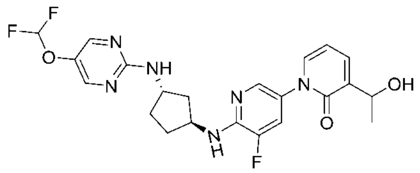 | $^1$H NMR (400 MHz, DMSO-$d_6$) δ 8.24 - 8.21 (s, 2H), 7.84 - 7.82 (d, *J* = 2.2 Hz, 1H), 7.56 - 7.50 (m, 3H), 7.49 - 7.44 (d, *J* = 7.2 Hz, 1H), 7.22 - 7.01 (d, *J* = 73.9 Hz, 1H), 6.93 - 6.83 (m, 1H), 6.36 - 6.30 (t, *J* = 6.8 Hz, 1H), 5.11 - 5.07 (d, *J* = 4.6 Hz, 1H), 4.76 - 4.68 (m, 1H), 4.55 - 4.46 (m, 1H), 4.38 - 4.28 (m, 1H), 2.18 - 2.06 (m, 2H), 2.00 - 1.90 (m, 2H), 1.65 - 1.50 (m, 2H), 1.29 - 1.24 (d, *J* = 6.3 Hz, 3H). |
| A43A or A43B | 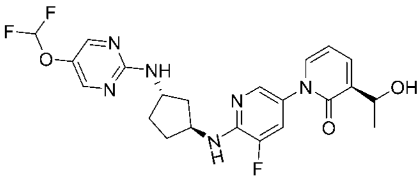 | $^1$H NMR (400 MHz, DMSO-$d_6$) δ 8.25 - 8.20 (s, 2H), 7.85 - 7.81 (d, *J* = 2.2 Hz, 1H), 7.56 - 7.50 (m, 2H), 7.48 - 7.43 (d, *J* = 7.3 Hz, 1H), 7.23 - 6.82 (m, 2H), 6.36 - 6.29 (t, *J* = 6.8 Hz, 1H), 5.12 - 5.07 (d, *J* = 4.6 Hz, 1H), 4.76 - 4.67 (p, *J* = 6.0 Hz, 1H), 4.55 - 4.45 (q, *J* = 6.9 Hz, 1H), 4.38 - 4.27 (q, *J* = 6.9 Hz, 1H), 2.19 - 2.06 (tt, *J* = 11.3, 6.1 Hz, 2H), 1.99 - 1.88 (dq, *J* = 10.7, 6.6 Hz, 2H), 1.66 - 1.49 (m, 2H), 1.31 - 1.24 (d, *J* = 6.4 Hz, 3H). |
| 4A3B or A43A | 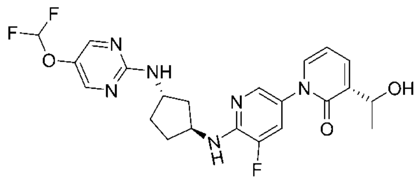 | $^1$H NMR (400 MHz, DMSO-$d_6$) δ 8.26 - 8.21 (s, 2H), 7.85 - 7.82 (d, *J* = 2.1 Hz, 1H), 7.58 - 7.50 (m, 3H), 7.49 - 7.44 (d, *J* = 7.2 Hz, 1H), 7.23 - 6.84 (m, 2H), 6.37 - 6.31 (t, *J* = 6.8 Hz, 1H), 5.12 - 5.07 (d, *J* = 4.6 Hz, 1H), 4.76 - 4.68 (p, *J* = 5.9 Hz, 1H), 4.56 - 4.46 (p, *J* = 7.1 Hz, 1H), 4.38 - 4.28 (q, *J* = 6.9 Hz, 1H), 2.21 - 2.07 (tt, *J* = 11.2, 5.6 Hz, 2H), 1.99 - 1.89 (td, *J* = 6.9, 3.0 Hz, 2H), 1.67 - 1.49 (m, 2H), 1.29 - 1.25 (d, *J* = 6.3 Hz, 3H). |
| A43A-D or A43B-D | 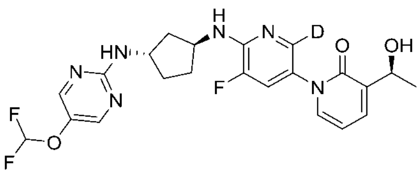 | $^1$H NMR (400 MHz, DMSO-$d_6$) δ 8.23 (s, 2H), 7.68 - 7.37 (m, 4H), 7.24 - 6.82 (m, 2H), 6.33 (t, *J* = 6.8 Hz, 1H), 5.08 (d, *J* = 4.4 Hz, 1H), 4.76 - 4.64 (m, 1H), 4.50 (q, *J* = 7.0 Hz, 1H), 4.32 (q, *J* = 6.9 Hz, 1H), 2.22 - 2.06 (m, 2H), 1.93 (td, *J* = 6.8, 3.2 Hz, 2H), 1.64 - 1.48 (m, 2H), 1.26 (d, *J* = 6.4 Hz, 3H). |
| A43A-D or A43B-D | 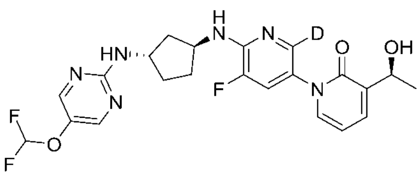 | $^1$H NMR (400 MHz, DMSO-$d_6$) δ 8.23 (s, 2H), 7.68 - 7.37 (m, 4H), 7.24 - 6.82 (m, 2H), 6.33 (t, *J* = 6.8 Hz, 1H), 5.08 (d, *J* = 4.4 Hz, 1H), 4.76 - 4.64 (m, 1H), 4.50 (q, *J* = 7.0 Hz, 1H), 4.32 (q, *J* = 6.9 Hz, 1H), 2.22 - 2.06 (m, 2H), 1.93 (td, *J* = 6.8, 3.2 Hz, 2H), 1.64 - 1.48 (m, 2H), 1.26 (d, *J* = 6.4 Hz, 3H). |

Example A44

Synthesis of 2-(6-(((1S,3S)-3-((5-(difluoromethoxy)-4-methylpyrimidin-2-yl)amino)cyclopentyl)amino)pyridi n-3-yl)pyridazin-3(2H)-one

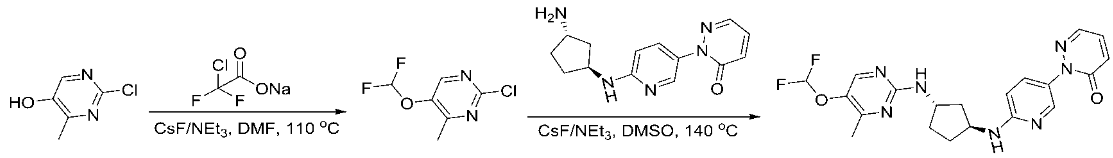


Step A: Synthesis of 2-chloro-5-(difluoromethoxy)-4-methylpyrimidine

Under a nitrogen atmosphere at room temperature, 2-chloro-4-methyl-5-hydroxypyrimidine (2.0 g, 13.84 mmol, 1.0 eq) was dissolved in N,N-dimethylformamide (20 mL). Subsequently, cesium fluoride (4.20 g, 27.68 mmol, 2.0 eq), triethylamine (4.20 g, 41.52 mmol, 3.0 eq), and sodium chlorodifluoroacetate (6.43 g, 41.52 mmol, 3.0 eq) were added. After the mixture was stirred uniformly, the reaction was transferred to an oil bath, and heated to 110 °C and reacted overnight.

After completion of the reaction, ethyl acetate (50 mL) was added to dilute the reaction mixture. Insoluble impurities were removed by filtration. Water (150 mL) was added, and the mixture was extracted with ethyl acetate (80 mL × 3). The organic phases were combined, dried over anhydrous sodium sulfate, filtered, and concentrated. The resulting residue was purified by silica gel column chromatography (eluent: ethyl acetate/n-hexane = 3/10) to obtain 1.52 g of 2-chloro-5-(difluoromethoxy)-4-methylpyrimidine as a yellow oil (yield 53%). LC-MS: $[M+H]^+$ = 195.

Step B: Synthesis of 2-(6-(((1S,3S)-3-((5-(difluoromethoxy)-4-methylpyrimidin-2-yl)amino)cyclopentyl)amino)pyridi n-3-yl)pyridazin-3(2H)-one

Under a nitrogen atmosphere at room temperature, 2-chloro-5-(difluoromethoxy)-4-methylpyrimidine (0.195 g, 1 mmol, 1.0 eq) was dissolved in dimethyl sulfoxide (4 mL). Subsequently, cesium fluoride (0.304 g, 2.0 mmol, 2.0 eq), triethylamine (0.303 g, 3 mmol, 3.0 eq), and 2-(6-(((1S,3S)-3-aminocyclopentyl)amino)pyridin-3-yl)pyridazin-3(2H)-one (0.271 g, 1.0 mmol, 1.0 eq) were added. After the mixture was stirred uniformly, the reaction was transferred to an oil bath, and heated to 140 °C and reacted overnight.

After completion of the reaction, water (50 mL) was added, and the mixture was extracted with ethyl acetate (50 mL × 3). The organic phases were combined, dried over anhydrous sodium sulfate, filtered, and concentrated. The resulting residue was purified by C18 column chromatography (120 g specification, eluent: acetonitrile/water) to obtain 0.050 g of 2-(6-(((1S,3S)-3-((5-(difluoromethoxy)-4-methylpyrimidin-2-yl)amino)cyclopentyl)amino)pyridi n-3-yl)pyridazin-3(2H)-one as a pale yellow solid (yield 12%). LC-MS: $[M+H]^+$ = 430.

NMR data: $^1$H NMR (400 MHz, DMSO-$d_6$) δ 8.21 - 8.03 (m, 2H), 8.01 (dd, J = 3.8, 1.6 Hz, 1H), 7.48 (ddd, J = 19.9, 9.2, 3.3 Hz, 2H), 7.36 (d, J = 7.3 Hz, 1H), 7.19 - 6.81 (m, 3H), 6.52 (d, J = 9.0 Hz, 1H), 4.31 (p, J = 6.6 Hz, 2H), 2.24 (s, 3H), 2.12 (tt, J = 12.8, 6.2 Hz, 2H), 1.87 (ddp, J = 18.7, 13.3, 6.8 Hz, 2H), 1.51 (td, J = 16.3, 8.0 Hz, 2H).

Example A45

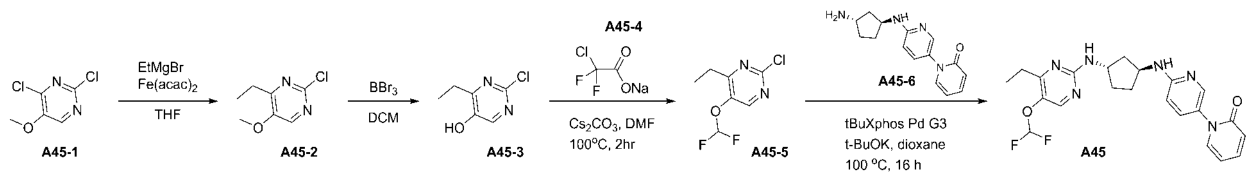


Step A: Synthesis of Compound A45-2

Compound A45-1 (5.0 g, 28 mmol) was dissolved in tetrahydrofuran (50 mL). The atmosphere was purged with nitrogen three times. At 0 °C, iron(III) acetylacetonate (988 mg, 2.8 mmol) and ethylmagnesium bromide (14.5 mL, 29 mmol) were added. The mixture was warmed to room temperature and reacted for 16 hours. The reaction mixture was poured into a saturated ammonium chloride solution and extracted with ethyl acetate (50 mL × 3). The organic phases were combined and dried by rotary evaporation. The resulting material was purified by Flash silica gel column chromatography (0 to 20% ethyl acetate/petroleum ether) to obtain 3.2 g of Compound A45-2 as a colorless oil mixture. LCMS: $[M+H]^+$ = 173.

Step B: Synthesis of Compound A45-3

Compound A45-2 (3.2 g, 18.6 mmol) was dissolved in dichloromethane (30 mL). Boron tribromide (8.9 mL, 93 mmol) was added, and the mixture was stirred at room temperature for 16 hours. The reaction mixture was poured into ice water (50 mL) to quench. The pH was adjusted to 3 with saturated sodium carbonate. The mixture was extracted with ethyl acetate (50 mL × 3). The organic phases were combined and dried by rotary evaporation. The resulting material was purified by Flash silica gel column chromatography (0 to 40% ethyl acetate/petroleum ether) to obtain 500 mg of Compound A45-3 as a white solid (34% yield). LCMS: $[M+H]^+$ = 159.

Step C: Synthesis of Compound A45-5

Compound A45-3 (420 mg, 2.66 mmol) was added to N,N-dimethylformamide (5 mL). Cesium carbonate (1 g, 3.2 mmol) was added, and the mixture was reacted at room temperature for 0.5 hours. Compound A45-4 (1.2 g, 7.98 mmol) was added, and the mixture was reacted at 100 °C for 16 hours. The reaction mixture was poured into saturated ammonium chloride (20 mL) and extracted with ethyl acetate (10 mL × 3). The organic phases were combined and dried by rotary evaporation. The resulting material was purified by Flash silica gel column chromatography (0 to 20% ethyl acetate/petroleum ether) to obtain 380 mg of Compound A45-5 as a yellow solid (68.7% yield). LCMS: $[M+H]^+$ = 209.

Step D: Synthesis of the Target Compound

Compound A45-5 (320 mg, 1.54 mmol) and Compound A45-6 (346 mg, 1.28 mmol) were added to anhydrous 1,4-dioxane (5 mL). Potassium tert-butoxide (287 mg, 2.56 mmol) and (2-dicyclohexylphosphino-2',4',6'-triisopropyl-1,1'-biphenyl)[2-(2'-amino-1,1'-biphenyl)]palladiu m(II) methanesulfonate (203 mg, 0.26 mmol) were added. Under nitrogen protection, the mixture was heated to 80 °C and reacted for 16 hours. The reaction mixture was poured into saturated ammonium chloride (20 mL) and extracted with ethyl acetate (10 mL × 3). The organic phases were combined and dried by rotary evaporation. The resulting material was purified by Flash silica gel column chromatography (0 to 10% methanol/dichloromethane) and further purified by preparative TLC plate to obtain 36 mg of the target compound (5.3% yield). LCMS: $[M+H]^+$ = 443.

$^1H$ NMR (400 MHz, DMSO-$d_6$) δ 8.11 (s, 1H), 7.94 (d, J = 2.7 Hz, 1H), 7.62 (dd, J = 6.7, 2.1 Hz, 1H), 7.50 (ddd, J = 9.0, 6.6, 2.1 Hz, 1H), 7.42 (dd, J = 8.9, 2.7 Hz, 1H), 7.36 (d, J = 7.1 Hz, 1H), 6.93 (d, J = 6.9 Hz, 1H), 6.55 (d, J = 8.9 Hz, 1H), 6.46 (dt, J = 9.1, 1.1 Hz, 1H), 6.29 (td, J = 6.7, 1.4 Hz, 1H), 4.34 (dd, J = 11.8, 6.0 Hz, 2H), 2.61 (q, J = 7.5 Hz, 2H), 2.15 (dq, J = 14.3, 7.1 Hz, 2H), 2.01 - 1.82 (m, 2H), 1.54 (tt, J = 13.7, 8.5 Hz, 2H), 1.18 (t, J = 7.5 Hz, 3H).

Example A46

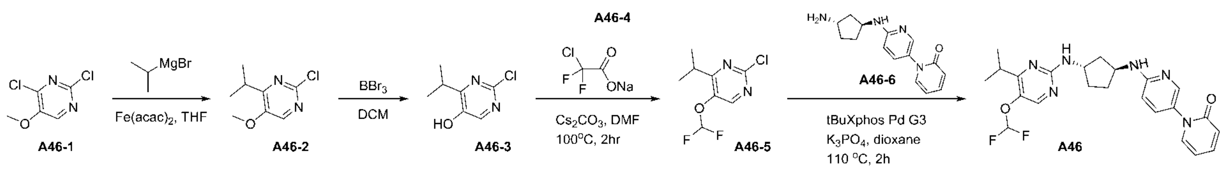


Step A: Synthesis of Compound A46-2

Compound A46-1 (5 g, 27.9 mmol) was dissolved in tetrahydrofuran (30 mL). At 0 °C, iron(III) acetylacetonate (985.4 mg, 2.79 mmol) was added, followed by dropwise addition of a tetrahydrofuran solution of isopropylmagnesium bromide (34 mL, 34 mmol). The reaction was stirred under a nitrogen atmosphere for 12 hours. The reaction mixture was quenched with saturated aqueous ammonium chloride and extracted with dichloromethane (50 mL × 3). The organic phases were combined, dried over anhydrous sodium sulfate and concentrated. The resulting material was purified by Flash silica gel column chromatography (5 to 20% ethyl acetate/petroleum ether) to obtain 2.5 g of Compound A46-2 as a yellow oily liquid mixture (yield: 48.0%). LCMS: $[M+H]^+$ = 187.

Step B: Synthesis of Compound A46-3

The mixture A46-2 (2.5 g, 13.4 mmol) was dissolved in a 1 M solution of boron tribromide in dichloromethane (24.1 mL, 24.1 mmol). Boron tribromide (3.4 mL, 42.9 mmol) was added, and the mixture was reacted at room temperature

for 8 hours. The reaction mixture was quenched by adding it to 20 mL of ice water. The pH was adjusted to 5-6 with saturated aqueous sodium carbonate. The mixture was extracted with dichloromethane (30 mL × 3). The organic phases were combined, dried over anhydrous sodium sulfate, filtered, and the filtrate was dried by rotary evaporation. The resulting material was purified by Flash silica gel column chromatography (0 to 100% ethyl acetate/petroleum ether), followed by purification via reverse-phase column chromatography to obtain 1.2 g of Compound A46-3 as a yellow solid (yield: 52.1%). LCMS: $[M+H]^+$ = 173.

Step C: Synthesis of Compound A46-5

Compound A46-3 (1 g, 5.8 mmol) was dissolved in N,N-dimethylformamide (15 mL). Cesium carbonate (2.28 g, 6.96 mmol) was added, and the mixture was stirred at room temperature for 1 hour. Compound A46-4 (2.65 g, 17.4 mmol) was added, and under a nitrogen atmosphere, the mixture was heated to 100 °C and reacted for 3 hours. The reaction mixture was quenched with aqueous ammonium chloride and extracted with dichloromethane (20 mL × 3). The organic phases were combined, dried, concentrated. The resulting material was purified by Flash silica gel column chromatography (10 to 50% ethyl acetate/petroleum ether) to obtain 700 mg of Compound A46-5 as a yellow oily liquid (yield: 54.2%). LCMS: $[M+H]^+$ =223.

Step D: Synthesis of the Target Compound

Compound A46-5 (222 mg, 1 mmol) and Compound A46-6 (270 mg, 1 mmol) were dissolved in anhydrous dimethyl sulfoxide (8 mL). Potassium phosphate (424 mg, 2 mmol) and methanesulfonato(2-di-tert-butylphosphino-2',4',6'-triisopropyl-1,1'-biphenyl)(2'-amino-1,1'-bi phenyl-2-yl)palladium(II) (159 mg, 0.2 mmol) were added. Under nitrogen protection, the mixture was heated to 110 °C and reacted for 2 hours. The reaction mixture was quenched with aqueous ammonium chloride and extracted with dichloromethane (20 mL × 3). The organic phases were combined, dried, concentrated. The resulting material was purified by preparative reverse-phase column chromatography to obtain 30 mg of the target compound as a yellow solid (yield: 6.5%).

LCMS: $[M+H]^+$ = 457. $^1$H NMR (400 MHz, DMSO-$d_6$) δ 8.10 (s, 1H), 7.92 (d, J = 2.7 Hz, 1H), 7.60 (dd, J = 6.8, 2.1 Hz, 1H), 7.48 (ddd, J = 9.0, 6.6, 2.1 Hz, 1H), 7.40 (dd, J = 8.9, 2.7 Hz, 1H), 7.31 (d, J = 7.0 Hz, 1H), 7.02 (s, 1H), 6.91 (d, J = 6.7 Hz, 1H), 6.53 (d, J = 8.9 Hz, 1H), 6.44 (d, J = 8.9 Hz, 1H), 6.27 (td, J = 6.7, 1.4 Hz, 1H), 4.30 (q, J = 7.1 Hz, 2H), 3.21 - 3.14 (m, 1H), 2.14 (dt, J = 13.4, 5.6 Hz, 2H), 1.90 (dq, J = 28.1, 6.5 Hz, 2H), 1.59 - 1.45 (m, 2H), 1.15 (dd, J = 6.8, 2.7 Hz, 6H).

Example A47

Synthesis of 6'-(((1S,3S)-3-((4-chloro-5-(difluoromethoxy)pyrimidin-2-yl)amino)cyclopentyl)amino)-2H-[1,3 '-bipyridin]-2-one

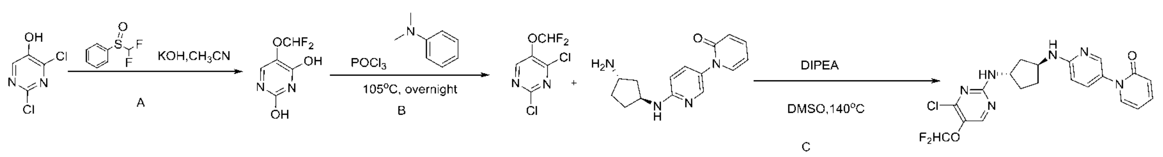


Step A: Synthesis of 5-(difluoromethoxy)pyrimidine-2,4-diol

At room temperature, 2,4-dichloropyrimidin-5-ol (0.5 g, 3.03 mmol) and (difluoromethanesulfonyl)benzene (1.34 g, 6.97 mmol) were dissolved in acetonitrile (20 mL). Potassium hydroxide (1.87 g, 33.33 mmol) and water (6 mL) were added. The reaction system was heated to 50 °C and stirred overnight.

The mixture was concentrated under reduced pressure. Water (20 mL) was added, and the mixture was extracted with EA (30 mL × 3). The aqueous phase was collected, acidified to an acidic pH with 1 M HCl, and extracted with ethyl acetate (20 mL × 3). The organic phases were combined, purified by column chromatography (EA/Hex = 6:4, 60%) to obtain 184 mg of 5-(difluoromethoxy)pyrimidine-2,4-diol as a white solid (yield 34%). LC-MS: $[M+H]^+$ =197.1.

Step B: Synthesis of 2,4-dichloro-5-(difluoromethoxy)pyrimidine

At room temperature, 5-(difluoromethoxy)pyrimidine-2,4-diol (184 mg, 1.03 mmol) was dissolved in phosphorus oxychloride (4 mL). Under nitrogen protection, the mixture was heated to 105 °C and stirred overnight. After completion of

the reaction, the mixture was concentrated under reduced pressure and purified by silica gel column chromatography (EA/Hex = 1:4, 20%) to obtain 115 mg of 2,4-dichloro-5-(difluoromethoxy)pyrimidine as a brown liquid (yield 51.8%).

Step C: Synthesis of 6'-(((1S,35)-3-((4-chloro-5-(difluoromethoxy)pyrimidin-2-yl)amino)cyclopentyl)amino)-2H-[1,3 '-bipyridin]-2-one

At room temperature, 1-(6-{[(1S,3S)-3-aminocyclopentyl]amino}pyridin-3-yl)-1,2-dihydropyridin-2-one (100.02 mg, 0.367 mmol), 2,4-dichloro-5-(difluoromethoxy)pyrimidine (95.45 mg, 0.44 mmol), and DIPEA (0.24 g, 1.85 mmol) were placed in a 100 mL round-bottom flask. Dimethyl sulfoxide (10 mL) was added. Under nitrogen protection, the reaction system was heated to 140 C and reacted overnight.

After completion of the reaction, water (40 mL) was added, and the mixture was extracted with EA (40 mL × 3). The organic phases were combined, purified by reverse-phase column chromatography (ACN/$H_2O$ = 40%), concentrated under reduced pressure, and lyophilized to obtain 115 mg of 6'-(((1S,3S)-3-((4-chloro-5-(difluoromethoxy)pyrimidin-2-yl) amino)cyclopentyl)amino)-2H-[1,3 '-bipyridin]-2-one as a white solid (yield 65.8%).

LC-MS: $[M+H]^+$=449.3. $^1H$ NMR (400 MHz, DMSO-$d_6$) δ 7.96 (d, J = 7.8 Hz, 1H), 7.93 (d, J = 2.7 Hz, 1H), 7.92 (s, 1H), 7.61 (dd, J = 6.8, 2.1 Hz, 1H), 7.48 (ddd, J = 9.0, 6.6, 2.1 Hz, 1H), 7.41 (dd, J = 8.9, 2.7 Hz, 1H), 7.23 (t, J = 72.8 Hz, 1H), 6.97 (d, 1H), 6.53 (d, J = 8.9 Hz, 1H), 6.45 (d, J = 8.9 Hz, 1H), 6.28 (td, J = 6.7, 1.4 Hz, 1H), 4.45 (dq, J = 69.1, 7.0 Hz, 2H), 2.14 (tdd, J = 11.6, 7.1, 3.4 Hz, 2H), 2.08 - 1.85 (m, 2H), 1.69 - 1.45 (m, 2H).

Example A48

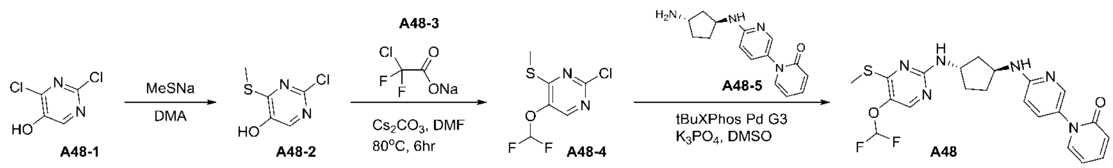


Step A: Synthesis of Compound A48-2

Compound A48-1 (1.2 g, 7.3 mmol) was added to N,N-dimethylacetamide (10 mL). Sodium methanethiolate (0.76 g, 10.9 mmol) was added. The atmosphere was purged with nitrogen, and the reaction was stirred at room temperature for 6 hours. The reaction mixture was added to a saturated aqueous ammonium chloride solution (40 mL). The pH was adjusted to 4 with a 1 M aqueous hydrochloric acid solution. The mixture was extracted with ethyl acetate (25 mL × 3). The organic phases were combined, dried and concentrated. The resulting material was purified by Flash silica gel column chromatography (28 to 32% ethyl acetate/petroleum ether) to obtain 540 mg of Compound A48-2 as a pale yellow solid (42.5% yield). LCMS: $[M+H]^+$ = 177.

Step B: Synthesis of Compound A48-4

Compound A48-2 (540 mg, 3.1 mmol) was added to anhydrous N,N-dimethylformamide (10 mL). Cesium carbonate (1.2 g, 3.7 mmol) and Compound A48-3 (1.4 g, 3.1 mmol) were added. The atmosphere was purged with nitrogen, and the mixture was heated to 80 °C and reacted for 6 hours. The reaction mixture was added to a saturated aqueous ammonium chloride solution (40 mL) and extracted with ethyl acetate (25 mL × 3). The organic phases were combined, dried and concentrated. The resulting material was purified by Flash silica gel column chromatography (1 to 3% ethyl acetate/petroleum ether) to obtain 400 mg of Compound A48-4 as a pale yellow solid (56.9% yield). LCMS: $[M+H]^+$ = 227.

Step C: Synthesis of the Target Compound

Compound A48-4 (360 mg, 1.6 mmol) and Compound A48-5 (519 mg, 1.9 mmol) were dissolved in anhydrous dimethyl sulfoxide (5 mL). Potassium phosphate (679 mg, 3.2 mmol) and methanesulfonato(2-di-tert-butylphosphino-2',4',6'-triisopropyl-1,1'-biphenyl)(2'-amino-1,1'-bi phenyl-2-yl)palladium(II) (254 mg, 0.3 mmol) were added. The atmosphere was purged with nitrogen, and the mixture was heated to 110 C and reacted for 16 hours. The reaction mixture was added to a saturated aqueous ammonium chloride solution (20 mL) and extracted with dichloromethane (10 mL × 3). The organic phases were combined, dried and concentrated. The resulting material was purified by Flash silica

gel column chromatography (2 to 3% methanol/dichloromethane), followed by reverse-phase column chromatography (18 to 23% acetonitrile/water) to obtain 30.7 mg of the target compound as a white solid (4.2% yield).

LCMS: $[M+H]^+$ = 461. $^1H$ NMR (400 MHz, DMSO-$d_6$) δ 7.92 (d, J = 3.7 Hz, 2H), 7.60 (d, J = 6.9 Hz, 1H), 7.48 (td, J = 6.9, 3.5 Hz, 2H), 7.39 (dd, J = 8.9, 2.6 Hz, 1H), 6.93 (d, J = 6.9 Hz, 1H), 6.52 (d, J = 8.9 Hz, 1H), 6.44 (d, J = 9.2 Hz, 1H), 6.27 (t, J = 6.7 Hz, 1H), 4.39 - 4.23 (m, 2H), 2.45 (s, 3H), 2.14 (d, J = 8.8 Hz, 2H), 1.91 (t, J = 7.0 Hz, 2H), 1.62 - 1.44 (m, 2H).

Example A49

Synthesis of 6'-(((1S,3S)-3-((5-(difluoromethoxy)-4,6-dimethylpyrimidin-2-yl)amino)cyclopentyl)amino)-2H -[1,3'-bipyridin]-2-one

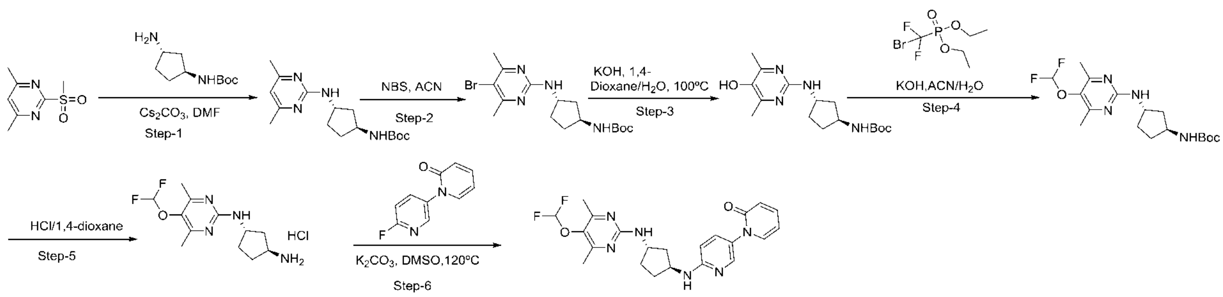


Step A: Synthesis of tert-butyl ((1S,3S)-3-((4,6-dimethylpyrimidin-2-yl)amino)cyclopentyl)carbamate

At room temperature, 4,6-dimethyl-2-(methylsulfonyl)pyrimidine (5 g, 26.8 mmol), tert-butyl ((1S,3S)-3-aminocyclopentyl)carbamate (5.55 g, 28.2 mmol), and cesium carbonate (17.5 g, 53.7 mmol) were added to acetonitrile (100 mL). The mixture was heated to 90 °C and reacted overnight.

After completion of the reaction, the reaction mixture was filtered. The filter cake was washed with ethyl acetate (3 ×50 mL). The filtrate was dried by rotary evaporation to a residue. The resulting residue was purified by silica gel column chromatography (ethyl acetate/n-hexane = 2/3, V/V) to obtain tert-butyl ((1S,3S)-3-((4,6-dimethylpyrimidin-2-yl)amino) cyclopentyl)carbamate as a pale yellow solid (5.05 g, yield 61%). LC-MS: $[M+H]^+$ = 307.

Step B: Synthesis of tert-butyl ((1S,3S)-3-((5-bromo-4,6-dimethylpyrimidin-2-yl)amino)cyclopentyl)carbamate

At room temperature, tert-butyl ((1S,3S)-3-((4,6-dimethylpyrimidin-2-yl)amino)cyclopentyl)carbamate (5.05 g, 16.5 mmol) was dissolved in acetonitrile (50 mL). NBS (4.36 g, 24.5 mmol) was added, and the mixture was stirred at room temperature for 2 hours.

After completion of the reaction, the reaction mixture was quenched with a saturated aqueous sodium bicarbonate solution (150 mL) and extracted with ethyl acetate (3 × 50 mL). The organic phases were combined, washed with brine, dried over anhydrous sodium sulfate, filtered, and dried by rotary evaporation to a residue. The resulting residue was purified by silica gel column chromatography (ethyl acetate/n-hexane = 1/4, V/V) to obtain tert-butyl ((1S,3S)-3-((5-bromo-4,6-dimethylpyrimidin-2-yl)amino)cyclopentyl)carbamate as a pale yellow solid (5.5 g, yield 86%). LC-MS: $[M+H]^+$ = 385.

Step C: Synthesis of tert-butyl ((1S,3S)-3-((5-hydroxy-4,6-dimethylpyrimidin-2-yl)amino)cyclopentyl)carbamate

At room temperature, tert-butyl ((1S,3S)-3-((5-bromo-4,6-dimethylpyrimidin-2-yl)amino)cyclopentyl)carbamate (5.5 g, 14.3 mmol), $Pd_2(dba)_3$ (1.3 g, 1.4 mmol), and potassium hydroxide (2.4 g, 42.9 mmol) were added to a mixed solution of 1,4-dioxane (50 mL) and water (50 mL). The atmosphere was purged with nitrogen three times, and the mixture was heated to 100 °C in an oil bath to react for 3 hours.

After completion of the reaction, water (50 mL) was added. The mixture was extracted with methyl tert-butyl ether (2 × 50 mL). The aqueous phase was collected, adjusted to pH approximately 5-6 with 1 M HCl(aq), and then extracted with ethyl acetate (3 × 50 mL). The organic phases were combined, dried over anhydrous $Na_2SO_4$, filtered, and dried by rotary evaporation to a residue. The resulting residue was purified by silica gel column chromatography (eluent: n-hexane/ethyl acetate = 1/1, V/V) to obtain tert-butyl ((1S,3S)-3-((5-hydroxy-4,6-dimethylpyrimidin-2-yl)amino)cyclopentyl)carbamate as a white solid (1.5 g, yield 32%). LC-MS: $[M+H]^+$ = 323.

Step D: Synthesis of tert-butyl ((1S,3S)-3-((5-(difluoromethoxy)-4,6-dimethylpyrimidin-2-yl)amino)cyclopentyl)carbamate

At room temperature, tert-butyl ((1S,3S)-3-((5-hydroxy-4,6-dimethylpyrimidin-2-yl)amino)cyclopentyl)carbamate (0.3 g, 0.93 mmol) was added to acetonitrile (10 mL). In an ice-water bath, sodium hydroxide (0.52 g, 9.3 mmol) dissolved in water (mL) was added to the above solution, followed by dropwise addition of diethyl (bromodifluoromethyl) phosphonate (0.74 g, 2.79 mmol). After the addition, the mixture was warmed to room temperature and reacted for 2 hours.

After completion of the reaction, water (30 mL) was added. The mixture was extracted with ethyl acetate (3 × 20 mL). The organic phases were combined, dried over anhydrous $Na_2SO_4$, filtered, and dried by rotary evaporation to a residue. The resulting residue was purified by C18 reverse-phase column chromatography (ACN/$H_2O$, 55% ACN) to obtain tert-butyl ((1S,3S)-3-((5-(difluoromethoxy)-4,6-dimethylpyrimidin-2-yl)amino)cyclopentyl)carbamate as a white solid (0.1 g, yield 28.86%). LC-MS: $[M+H]^+$ = 373.

Step E: Synthesis of (1S,3S)-$N^1$-(5-(difluoromethoxy)-4,6-dimethylpyrimidin-2-yl)cyclopentane-1,3-diamine hydrochloride

tert-Butyl ((1S,3S)-3-((5-(difluoromethoxy)-4,6-dimethylpyrimidin-2-yl)amino)cyclopentyl)carbamate (0.1 g, 0.27 mmol) was dissolved in dichloromethane (5 mL). Then, 4 M HCl/1,4-dioxane (2 mL) was added. The mixture was reacted at room temperature for 1 hour. After completion of the reaction, the mixture was directly dried by rotary evaporation and used in the next step. LC-MS: $[M+H]^+$ = 273.

Step F: Synthesis of 6'-(((1S,3S)-3-((5-(difluoromethoxy)-4,6-dimethylpyrimidin-2-yl)amino)cyclopentyl)amino)-2H -[1,3'-bipyridin]-2-one

(1S,3S)-$N^1$-(5-(difluoromethoxy)-4,6-dimethylpyrimidin-2-yl)cyclopentane-1,3-diamine hydrochloride (70 mg, 0.26 mmol), 1-(6-fluoropyridin-3-yl)-1,2-dihydropyridin-2-one (74.17 mg, 0.39 mmol), and potassium carbonate (179.67 mg, 1.3 mmol) were added to DMSO (3 mL). The mixture was reacted at 120 °C for 16 hours.

After completion of the reaction, the mixture was filtered. The filtrate was directly purified by C18 column chromatography (eluent: acetonitrile/water = 45/55, V/V) to obtain 6'-(((1S,3S)-3-((5-(difluoromethoxy)-4,6-dimethylpyrimidin-2-yl)amino)cyclopentyl)amino)-2H -[1,3'-bipyridin]-2-one as a white solid (54.7 mg, yield 47.61%, purity 99%). LC-MS: $[M+H]^+$ = 443.

NMR data: $^1H$ NMR (400 MHz, DMSO) δ 7.92 (d, J = 2.6 Hz, 1H), 7.67 - 7.55 (m, 1H), 7.48 (ddd, J = 8.9, 6.6, 2.1 Hz, 1H), 7.40 (dd, J = 8.9, 2.7 Hz, 1H), 7.24 (d, J = 7.4 Hz, 1H), 7.16 - 6.69 (m, 2H), 6.52 (d, J = 8.9 Hz, 1H), 6.45 (d, J = 9.2 Hz, 1H), 6.27 (t, J = 6.7 Hz, 1H), 4.32 (dp, J = 13.2, 6.7 Hz, 2H), 2.23 (s, 6H), 2.11 (ddd, J = 18.0, 8.6, 4.9 Hz, 2H), 1.87 (dh, J = 26.8, 6.8 Hz, 2H), 1.49 (dq, J = 11.2, 7.1 Hz, 2H).

Example A50

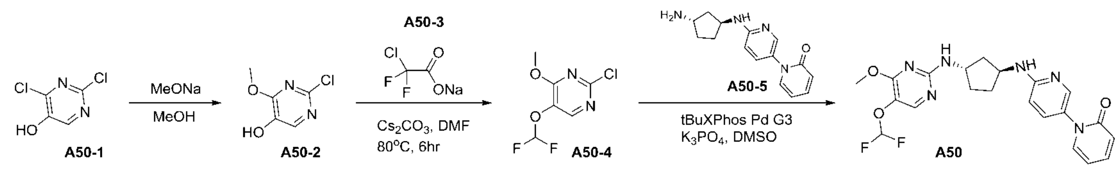


Step A: Synthesis of Compound A50-2

Compound A50-1 (2.0 g, 12.1 mmol) was dissolved in methanol (20 mL). A sodium methoxide methanol solution (3.6 mL, 18.1 mmol) was added. The mixture was reacted at 40 °C for 2 days. The reaction mixture was concentrated under reduced pressure. The resulting material was purified by Flash silica gel column chromatography (0 to 10% methanol/dichloromethane) to obtain 820 mg of Compound A50-2 as a yellow oil (42.4% yield). LCMS: $[M+H]^+$ = 161.

Step B: Synthesis of Compound A50-4

Compound A50-2 (820 mg, 5.13 mmol) was dissolved in N,N-dimethylformamide (10 mL). Cesium carbonate (2 g, 6.16 mmol) was added, and the mixture was stirred at room temperature for 0.5 hours. Compound A50-3 (2.34 g, 15.4

mmol) was added, and the mixture was heated to 100 °C and reacted for 2 hours. Water (20 mL) was added to the reaction mixture, which was then extracted with ethyl acetate (50 mL × 3). The organic phases were combined and dried by rotary evaporation. The resulting material was purified by Flash silica gel column chromatography (0% to 20% ethyl acetate/-petroleum ether) to obtain 230 mg of Compound A50-4 as a yellow oil (21.4% yield). LCMS: $[M+H]^+$ = 211.

Step C: Synthesis of the Target Compound

Compound A50-4 (230 mg, 1.1 mmol) and Compound A50-5 (355 mg, 1.3 mmol) were added to anhydrous dimethyl sulfoxide (5 mL). Potassium phosphate (466 mg, 2.2 mmol) and methanesulfonato(2-di-tert-butylphosphino-2',4',6'-triisopropyl-1,1'-biphenyl)(2'-amino-1,1'-bi phenyl-2-yl)palladium(II) (177 mg, 0.22 mmol) were added. The atmosphere was purged with nitrogen, and the mixture was heated to 100 °C and reacted for 2 hours. The reaction mixture was added to a saturated aqueous ammonium chloride solution and extracted with ethyl acetate (20 mL × 3). The organic phases were combined and dried by rotary evaporation. The resulting material was purified by Flash silica gel column chromatography (0% to 10% methanol/dichloromethane) and further purified by preparative TLC plate to obtain 11 mg of the target compound as a white solid (5.3% yield). LCMS: $[M+H]^+$ = 445.

$^1H$ NMR (400 MHz, DMSO-$d_6$) δ 7.96 (s, 1H), 7.92 (d, J = 2.4 Hz, 1H), 7.60 (dd, J = 6.8, 2.1 Hz, 1H), 7.48 (ddd, J = 9.2, 6.7, 2.1 Hz, 1H), 7.39 (dd, J = 8.8, 2.6 Hz, 2H), 6.93 (d, J = 6.8 Hz, 1H), 6.88 (s, 1H), 6.52 (d, J = 8.8 Hz, 1H), 6.44 (d, J = 9.2 Hz, 1H), 6.27 (t, J = 6.8 Hz, 1H), 4.30 (d, J = 8.0 Hz, 2H), 3.88 (s, 3H), 2.19 - 2.09 (m, 2H), 1.97 - 1.84 (m, 2H), 1.51 (dq, J = 19.2, 7.2 Hz, 2H).

Example A51

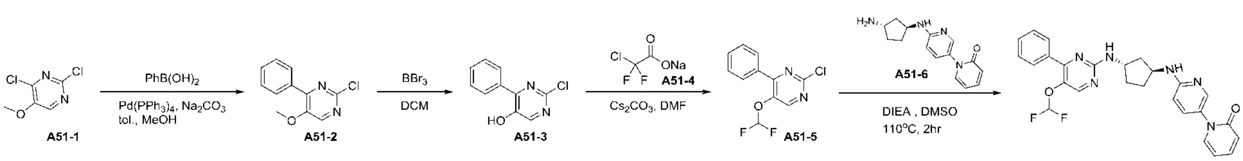


Step A: Synthesis of Compound A51-2

Compound A51-1 (3 g, 16.7 mmol) and phenylboronic acid (3.05 g, 25 mmol) were dissolved in toluene (60 mL) and methanol (12 mL). A 2 M aqueous sodium carbonate solution (16.7 mL, 33.4 mmol) and tetrakis(triphenylphosphine) palladium (1.93 g, 1.67 mmol) were added. Under a nitrogen atmosphere, the mixture was heated to 110 °C and reacted for 3 hours. The reaction mixture was filtered. The filtrate was extracted with ethyl acetate (20 mL × 3). The organic phase was dried over anhydrous sodium sulfate and concentrated under reduced pressure. The resulting material was purified by Flash column chromatography (0 to 20% ethyl acetate/petroleum ether) to obtain 3.3 g of Compound A51-2 as a yellow oily liquid (yield: 88.2%). LCMS: $[M+H]^+$ = 221.

Step B: Synthesis of Compound A51-3

Compound A51-2 (3.3 g, 15.4 mmol) was dissolved in dichloromethane (30 mL). Boron tribromide (7.4 mL, 77 mmol) was added, and the mixture was stirred at room temperature for 16 hours. The reaction mixture was quenched by adding it to 30 mL of ice water. The pH was adjusted to 5-6 with saturated aqueous sodium carbonate. The mixture was extracted with ethyl acetate (30 mL × 3). The organic phases were combined, dried over anhydrous sodium sulfate, filtered, and dried by rotary evaporation. The resulting material was purified by Flash column chromatography (0 to 100% ethyl acetate/petroleum ether) to obtain 2.6 g of Compound A51-3 as a yellow solid (yield: 82.0%). LCMS: $[M+H]^+$ = 207.

Step C: Synthesis of Compound A51-5

Compound A51-3 (2.6 g, 12.6 mmol) was dissolved in N,N-dimethylformamide (25 mL). Cesium carbonate (4.9 g, 15.1 mmol) was added, and the mixture was stirred at room temperature for 1 hour. Compound A51-4 (5.7 g, 37.8 mmol) was added. Under a nitrogen atmosphere, the mixture was heated to 100 °C and reacted for 3 hours. The reaction mixture was cooled to room temperature, quenched with saturated aqueous ammonium chloride, and extracted with dichloromethane (30 mL × 3). The organic phases were combined, dried over anhydrous sodium sulfate, filtered, and dried by rotary evaporation. The resulting material was purified by Flash column chromatography (0 to 30% ethyl acetate/petroleum ether) to obtain 1.2 g of Compound A51-5 as a yellow oily liquid (yield: 37.2%). LCMS: $[M+H]^+$ = 257.

Step D: Synthesis of the Target Compound

Compound A51-5 (80 mg, 0.3 mmol) and Compound A51-6 (81 mg, 0.3 mmol) were dissolved in dimethyl sulfoxide (3 mL). N,N-Diisopropylethylamine (0.16 mL, 0.93 mmol) was added. The mixture was reacted at 110 °C under microwave irradiation for 1 hour. The reaction mixture was quenched with saturated aqueous ammonium chloride and extracted with dichloromethane (10 mL × 3). The organic phases were combined, dried over anhydrous sodium sulfate, filtered, and dried by rotary evaporation. The resulting material was purified by preparative reverse-phase HPLC to obtain 65 mg of the target compound as an off-white solid (yield: 44.2%). LCMS: $[M+H]^+$ = 491.

$^1H$ NMR (400 MHz, DMSO-$d_6$) δ 8.30 (s, 1H), 7.90 (dd, J = 16.7, 4.1 Hz, 3H), 7.64 - 7.55 (m, 2H), 7.53 - 7.45 (m, 4H), 7.40 (dd, J = 8.9, 2.7 Hz, 1H), 7.17 - 6.74 (m, 2H), 6.53 (d, J = 8.9 Hz, 1H), 6.44 (d, J = 9.1 Hz, 1H), 6.27 (td, J = 6.7, 1.3 Hz, 1H), 4.35 (dq, J = 20.8, 6.7 Hz, 2H), 2.17 (dd, J = 9.1, 4.4 Hz, 2H), 2.02 - 1.88 (m, 2H), 1.63 - 1.45 (m, 2H).

Example A52

Synthesis of 6'-(((1S,3S5)-3-((5-(difluoromethoxy)-4-(1H-pyrrol-2-yl)pyrimidin-2-yl)amino)cyclopentyl)amin o)-2H-[1,3'-bipyridin]-2-one

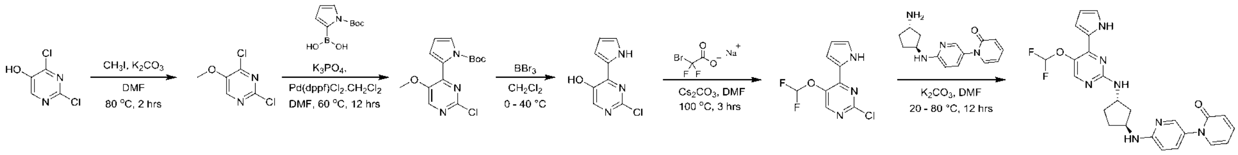


Step 1: Synthesis of 2,4-dichloro-5-methoxypyrimidine

2,4-Dichloropyrimidin-5-ol (5.00 g, 30.3 mmol, 1.00 eq) and potassium carbonate (10.5 g, 75.8 mmol, 2.50 eq) were dissolved in N,N-dimethylformamide (50 mL). Iodomethane (6.45 g, 45.5 mmol, 2.83 mL, 1.50 eq) was added. The mixture was heated to 80 °C and reacted for 2 hours.

TLC (petroleum ether: ethyl acetate = 3:1) showed the formation of a new spot ($R_f$ = 0.60).

Water (200 mL) was slowly added to the reaction mixture to quench the reaction, followed by extraction with ethyl acetate (50 mL * 3). The organic phases were combined, washed with saturated brine (50 mL), dried, and concentrated to obtain 2,4-dichloro-5-methoxypyrimidine (2.50 g, 46.1% yield).

Step 2: Synthesis of tert-butyl 2-(2-chloro-5-methoxypyrimidin-4-yl)pyrrole-1-carboxylate

2,4-Dichloro-5-methoxypyrimidine (2.50 g, 14.0 mmol, 1.00 eq), (1-(tert-butoxycarbonyl)-1H-pyrrol-2-yl)boronic acid (2.95 g, 14.0 mmol, 1.00 eq), [1,1'-bis(diphenylphosphino)ferrocene]palladium(II) dichloride dichloromethane complex (1.14 g, 1.40 mmol, 0.10 eq), and potassium phosphate (17.8 g, 83.8 mmol, 6.00 eq) were dissolved in N,N-dimethylformamide (30 mL). The atmosphere was purged with nitrogen three times. The mixture was heated to 60 °C and reacted for 12 hours. LCMS detected the MS value of the product.

The reaction mixture was diluted with water (200 mL) and then extracted with ethyl acetate (40 mL * 4). The organic phases were combined, washed with saturated brine (30 mL * 3), dried over anhydrous sodium sulfate, filtered, and concentrated to obtain a crude product. The crude product was purified by reverse-phase preparative chromatography (hydrochloric acid system) to obtain tert-butyl 2-(2-chloro-5-methoxypyrimidin-4-yl)pyrrole-1-carboxylate (1.50 g, 34.7% yield). LCMS: MS (ESI) m/z = 480.1 $[M-100+1]^+$.

Step 3: Synthesis of 2-chloro-4-(1H-pyrrol-2-yl)pyrimidin-5-ol

tert-Butyl 2-(2-chloro-5-methoxypyrimidin-4-yl)pyrrole-1-carboxylate (1.50 g, 4.84 mmol, 1.00 eq) was dissolved in dichloromethane (10 mL). The temperature was lowered to 0 °C. Boron tribromide (7.28 g, 29.1 mmol, 6.00 eq) was added dropwise. The mixture was then heated to 40 °C and reacted for 4 hours. TLC (petroleum ether: ethyl acetate = 1:1) showed the formation of a new major spot. Methanol was slowly added dropwise at low temperature to quench the reaction, and the mixture was concentrated to obtain a crude product. The crude product was purified by column chromatography (petroleum ether: ethyl acetate = 10:1 to 1:1) to obtain 2-chloro-4-(1H-pyrrol-2-yl)pyrimidin-5-ol (0.50 g, 52.8% yield).

Step 4: Synthesis of 2-chloro-5-(difluoromethoxy)-4-(1H-pyrrol-2-yl)pyrimidine

At 20 °C, 2-chloro-4-(1H-pyrrol-2-yl)pyrimidin-5-ol (331 mg, 1.69 mmol, 1.00 eq) and cesium carbonate (551 mg, 1.69 mmol, 1.00 eq) were dissolved in N,N-dimethylformamide (5 mL). The mixture was stirred for one hour, then sodium 2-chloro-2,2-difluoroacetate (284 mg, 1.86 mmol, 1.10 eq) was added in batches. After the addition, the mixture was heated to 100 °C and stirred for 2 hours.

TLC (petroleum ether: ethyl acetate = 2:1) showed that the starting material ($R_f$ = 0.1) was completely consumed, and a new major spot ($R_f$ = 0.6) was formed.

The reaction mixture was diluted with 50 mL of water and extracted with ethyl acetate (20 mL * 3). The organic phases were combined, washed with saturated brine (30 mL), dried over anhydrous sodium sulfate, filtered, and concentrated to obtain a crude product. The crude product was purified by column chromatography (petroleum ether: ethyl acetate = 50:1 to 1:1) to obtain 2-chloro-5-(difluoromethoxy)-4-(1H-pyrrol-2-yl)pyrimidine (250 mg, 60.2% yield).

Step 5: Synthesis of 6'-(((1S,3S)-3-((5-(difluoromethoxy)-4-(1H-pyrrol-2-yl)pyrimidin-2-yl)amino)cyclopentyl)amin o)-2H-[1,3'-bipyridin]-2-one

At 20 °C, 2-chloro-5-(difluoromethoxy)-4-(1H-pyrrol-2-yl)pyrimidine (180 mg, 733 μmol, 1.00 eq), 1-[6-[[(1S,3S)-3-aminocyclopentyl]amino]pyridin-3-yl]pyridin-2-one (237.74 mg, 879.43 μmol, 1.2 eq), and potassium carbonate (203 mg, 1.47 mmol, 2.00 eq) were dissolved in N,N-dimethylformamide (1 mL). The mixture was then heated to 80 °C and stirred for 12 hours. LCMS detected the MS value.

The reaction mixture was diluted with water (50 mL) and then extracted with ethyl acetate (20 mL * 3). The organic phases were combined, washed with saturated brine (30 mL * 2), dried over anhydrous sodium sulfate, filtered, and concentrated to obtain a crude product. The crude product was purified by preparative chromatography (Column: Waters Xbridge Prep OBD C18 150*40 mm*10 um; Mobile phase: [water ($NH_4HCO_3$)-ACN]; Gradient: 25% to 55% B over 20 min) to obtain 6'-(((1S,3S)-3-((5-(difluoromethoxy)-4-(1H-pyrrol-2-yl)pyrimidin-2-yl)amino)cyclopentyl)amin o)-2H-[1,3'-bipyridin]-2-one (187 mg, 53.1% yield).

LCMS: MS (ESI) m/z = 480.1 $[M+1]^+$. $^1$H NMR: (400 MHz, $CDCl_3$): δ 9.95 - 9.46 (m, 1H), 8.13 (s, 1H), 8.04 (d, J = 2.4 Hz, 1H), 7.55 (dd, J = 2.4, 8.8 Hz, 1H), 7.40 (ddd, J = 2.0, 6.8, 9.2 Hz, 1H), 7.30 (dd, J = 2.0, 6.8 Hz, 1H), 7.10 (br s, 1H), 7.04 (br s, 1H), 6.66 (d, J = 9.2 Hz, 1H), 6.59 - 6.18 (m, 3H), 6.18 - 6.18 (m, 1H), 5.22 - 5.01 (m, 2H), 4.59 - 4.43 (m, 1H), 4.34 - 4.18 (m, 1H), 2.44 - 2.26 (m, 2H), 2.09 (t, J = 6.8 Hz, 2H), 1.66 - 1.57 (m, 2H).

Example A53

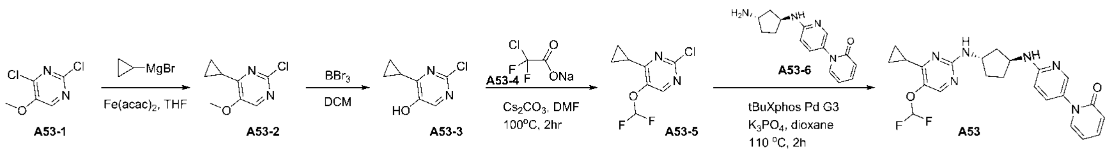


Step A: Synthesis of Compound A53-2

Compound A53-1 (5 g, 27.9 mmol) was dissolved in tetrahydrofuran (30 mL). At 0 °C, iron(III) acetylacetonate (985 mg, 2.79 mmol) was added. Under a nitrogen atmosphere, a tetrahydrofuran solution of cyclopropylmagnesium bromide (34 mL, 1 M, 34 mmol) was added dropwise. The reaction was stirred at room temperature overnight. The reaction mixture was quenched with saturated aqueous ammonium chloride and extracted with dichloromethane (50 mL × 3). The organic phases were combined, dried over anhydrous sodium sulfate and concentrated. The resulting material was purified by Flash column chromatography (5 to 30% ethyl acetate/petroleum ether) to obtain 720 mg of Compound A53-2 as a pale yellow solid (yield: 14%). LCMS: $[M+H]^+$ = 185.

Step B: Synthesis of Compound A53-3

Compound A53-2 (750 mg, 3.9 mmol) was dissolved in a 1 M solution of boron tribromide in dichloromethane (7 mL, 7 mmol). Boron tribromide (1.2 mL, 12.5 mmol) was added. The mixture was stirred at room temperature for 4 hours. The reaction mixture was quenched by adding it to 10 mL of ice water. The pH was adjusted to 5-6 with saturated aqueous sodium carbonate. The mixture was then extracted with ethyl acetate (20 mL × 3). The organic phases were combined, dried over anhydrous sodium sulfate, filtered, and dried by rotary evaporation. The resulting material was purified by Flash

column chromatography (0 to 50% ethyl acetate/petroleum ether) to obtain 300 mg of Compound A53-3 as a pale yellow solid (yield: 45%). LCMS: $[M+H]^+$ = 171.

Step C: Synthesis of Compound A53-5

Compound A53-3 (300 mg, 1.76 mmol) was dissolved in N,N-dimethylformamide (10 mL). Cesium carbonate (687 mg, 2.11 mmol) was added, and the mixture was stirred at room temperature for 1 hour. Compound A53-4 (805 mg, 5.28 mmol) was added. Under a nitrogen atmosphere, the mixture was heated to 100 °C and reacted for 3 hours. The reaction mixture was quenched with saturated aqueous ammonium chloride and extracted with dichloromethane (10 mL × 3). The organic phases were combined, dried, concentrated. The resulting material was purified by Flash column chromatography (0 to 20% ethyl acetate/petroleum ether) to obtain 80 mg of Compound A53-5 as a yellow oil (yield: 20%). LCMS: $[M+H]^+$ = 221.

Step D: Synthesis of the Target Compound

Compound A53-5 (80 mg, 0.36 mmol) and Compound A53-6 (97 mg, 0.36 mmol) were dissolved in anhydrous dimethyl sulfoxide (5 mL). Potassium phosphate (153 mg, 0.72 mmol) and methanesulfonato(2-di-tert-butylphosphino-2',4',6'-triisopropyl-1,1'-biphenyl)(2'-amino-1,1'-bi phenyl-2-yl)palladium(II) (57 mg, 0.072 mmol) were added. Under a nitrogen atmosphere, the mixture was heated to 110 °C and reacted for 2 hours. The reaction mixture was quenched with saturated aqueous ammonium chloride and extracted with dichloromethane (10 mL × 3). The organic phases were combined, dried, concentrated. The resulting material was purified by preparative TLC plate (developing solvent: dichloromethane/methanol = 12:1) to obtain 4 mg of the target compound as an off-white solid (yield: 2.5%). LCMS: $[M+H]^+$ = 455.

$^1H$ NMR (400 MHz, DMSO-$d_6$) δ 8.04 (s, 1H), 7.91 (d, J = 2.7 Hz, 1H), 7.60 (dd, J = 6.8, 2.0 Hz, 1H), 7.48 (ddd, J = 8.9, 6.5, 2.1 Hz, 1H), 7.39 (dd, J = 8.9, 2.7 Hz, 1H), 7.22 (d, J = 7.4 Hz, 1H), 6.91 (d, J = 6.9 Hz, 1H), 6.52 (d, J = 8.9 Hz, 1H), 6.44 (d, J = 9.1 Hz, 1H), 6.27 (t, J = 6.7 Hz, 1H), 4.25 (dq, J = 20.1, 6.8 Hz, 2H), 2.19 - 2.04 (m, 3H), 1.86 (dt, J = 20.3, 6.7 Hz, 2H), 1.55 - 1.44 (m, 2H), 1.23 (s, 2H), 1.01 (s, 2H).

Example A54

Synthesis of 3-(6'-(((1S,3S)-3-((5-(difluoromethoxy)pyrimidin-2-yl)amino)cyclopentyl)amino)-2-oxo-2H-[1,3 '-bipyridin]-3-yl)-1,2,4-oxadiazol-5(4H)-one

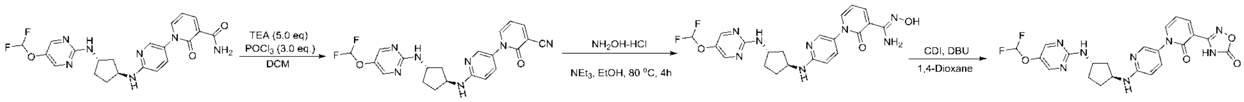


Step A: Synthesis of 6'-(((1S,3S)-3-((5-(difluoromethoxy)pyrimidin-2-yl)amino)cyclopentyl)amino)-2-oxo-2H-[1,3'-b ipyridine]-3-carbonitrile

At room temperature, 6'-(((1S,3S)-3-((5-(difluoromethoxy)pyrimidin-2-yl)amino)cyclopentyl)amino)-2-oxo-2H-[1,3'-b ipyridine]-3-carboxamide (2.2 g, 4.82 mmol, 1.0 eq), triethylamine (2.43 g, 24.1 mmol, 5.0 eq), and phosphorus oxychloride (1.2 g, 14.5 mmol, 3.0 eq) were dissolved in dichloromethane (20 mL). The mixture was stirred at room temperature for 3 hours.

After completion of the reaction, the pH of the reaction mixture was adjusted to 7-8 with a 2 M hydrochloric acid solution. Cold water (30 mL) was added to dilute the mixture, followed by extraction with ethyl acetate (75 mL × 3). The organic phases were combined, dried over anhydrous sodium sulfate, filtered, and concentrated to obtain 1.83 g of 6'-(((1S,3S)-3-((5-(difluoromethoxy)pyrimidin-2-yl)amino)cyclopentyl)amino)-2-oxo-2H-[1,3'-b ipyridine]-3-carbonitrile as a brown foamy solid powder (yield 87%). LC-MS: $[M+H]^+$ = 439.

Step B: Synthesis of (Z)-6'-(((1S,3S)-3-((5-(difluoromethoxy)pyrimidin-2-yl)amino)cyclopentyl)amino)-N'-hydroxy-2-oxo-2H-[1,3'-bipyridine]-3-carboximidamide

At room temperature, 6'-(((1S,3S)-3-((5-(difluoromethoxy)pyrimidin-2-yl)amino)cyclopentyl)amino)-2-oxo-2H-[1,3'-b ipyridine]-3-carbonitrile (0.63 g, 1.44 mmol, 1.0 eq), hydroxylamine hydrochloride (0.8 g, 11.52 mmol,

8.0 eq), and triethylamine (1.16 g, 11.52 mmol, 8.0 eq) were dissolved in ethanol (6 mL). The mixture was heated to 80 °C and stirred for 4 hours.

After completion of the reaction, the organic solvent was removed by concentration under reduced pressure. The crude product was purified by C18 column chromatography (eluent: acetonitrile/water) to obtain 0.493 g of (Z)-6'-(((1S,3S)-3-((5-(difluoromethoxy)pyrimidin-2-yl)amino)cyclopentyl)amino)-N'-hydroxy-2-oxo-2H-[1,3'-bipyridine]-3-carboximidamide as a brown solid powder (yield 73%). LC-MS: $[M+H]^+$ = 473.

Step C: Synthesis of 3-(6'-(((1S,3S)-3-((5-(difluoromethoxy)pyrimidin-2-yl)amino)cyclopentyl)amino)-2-oxo-2H-[1,3 '-bipyridin]-3-yl)-1,2,4-oxadiazol-5(4H)-one

At room temperature, (Z)-6'-(((1S,3S)-3-((5-(difluoromethoxy)pyrimidin-2-yl)amino)cyclopentyl)amino)-N'-hydroxy-2-oxo-2H-[1,3'-bipyridine]-3-carboximidamide (0.4 g, 0.85 mmol, 1.0 eq), N,N'-carbonyldiimidazole (0.152 g, 0.94 mmol, 1.1 eq), and DBU (0.194 g, 1.28 mmol, 1.5 eq) were dissolved in 1,4-dioxane (6 mL). The mixture was stirred vigorously for 6 hours.

After completion of the reaction, the organic solvent was removed by concentration under reduced pressure. The crude product was purified by C18 column chromatography (eluent: acetonitrile/water) to obtain 0.175 g of 3-(6'-(((1S,3S)-3-((5-(difluoromethoxy)pyrimidin-2-yl)amino)cyclopentyl)amino)-2-oxo-2H-[1,3 '-bipyridin]-3-yl)-1,2,4-oxadiazol-5(4H)-one as a yellow solid powder (yield 41%). LC-MS: $[M+H]^+$ = 499.

Proton NMR: $^1$H NMR (400 MHz, DMSO-$d_6$) δ 8.23 (s, 2H), 8.14 - 7.82 (m, 3H), 7.56 - 7.38 (m, 2H), 7.22 - 6.84 (m, 2H), 6.64 - 6.43 (m, 2H), 4.31 (q, J = 7.0 Hz, 2H), 2.13 (tt, J = 13.0, 6.8 Hz, 2H), 1.89 (qt, J = 12.9, 6.7 Hz, 2H), 1.51 (ddd, J = 19.0, 14.1, 7.1 Hz, 2H).

Examples A55 to A73

| | | |
|---|---|---|
| A55 | 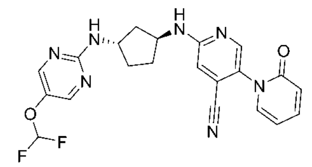 | $^1$H NMR (400 MHz, DMSO-$d_6$) δ 8.24 (s, 2H), 8.17 - 8.04 (m, 2H), 7.94 - 7.83 (m, 1H), 7.51 (d, *J* = 7.2 Hz, 1H), 7.25 - 6.80 (m, 5H), 4.28 (dq, *J* = 21.3, 6.6 Hz, 2H), 2.22 - 2.02 (m, 2H), 1.89 (dq, *J* = 22.5, 6.5 Hz, 2H), 1.60 - 1.41 (m, 2H). |
| A56 | 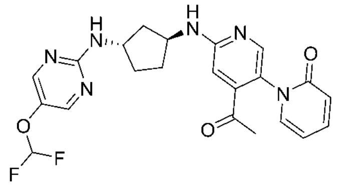 | $^1$H NMR (400 MHz, DMSO-$d_6$) δ 8.25 (s, 2H), 8.11 - 8.04 (m, 1H), 7.92 (s, 1H), 7.85 (ddd, *J* = 9.0, 7.2, 2.0 Hz, 1H), 7.51 (d, *J* = 7.2 Hz, 1H), 7.25 - 6.85 (m, 4H), 6.73 (s, 1H), 4.31 (dq, *J* = 13.5, 6.8 Hz, 2H), 2.35 (s, 4H), 2.14 (dq, *J* = 14.2, 7.3 Hz, 2H), 1.90 (h, *J* = 6.7 Hz, 2H), 1.51 (dq, *J* = 22.2, 9.3, 8.1 Hz, 2H). |
| A57 | 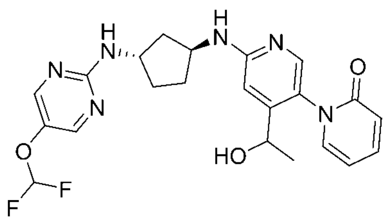 | $^1$H NMR (400 MHz, DMSO-$d_6$) δ 8.25 (s, 2H), 8.11 (dd, *J* = 5.2, 2.0 Hz, 1H), 7.83 (ddd, *J* = 8.7, 7.2, 2.0 Hz, 1H), 7.67 (s, 1H), 7.49 (d, *J* = 7.3 Hz, 1H), 7.27 - 6.82 (m, 3H), 6.69 (s, 1H), 6.56 (dd, *J* = 6.9, 2.4 Hz, 1H), 5.21 (d, *J* = 4.0 Hz, 1H), 4.57 (t, *J* = 5.4 Hz, 1H), 4.29 (dt, *J* = 12.3, 6.5 Hz, 2H), 2.12 (dt, *J* = 11.4, 5.8 Hz, 2H), 1.88 (t, *J* = 6.9 Hz, 2H), 1.51 (t, *J* = 12.7 Hz, 2H), 1.18 (d, *J* = 6.5 Hz, 3H). |
| A58 | 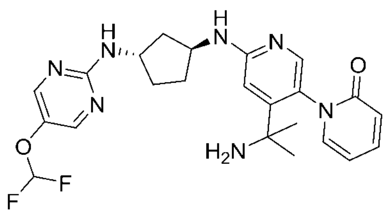 | $^1$H NMR (400 MHz, DMSO-$d_6$) δ 8.63 (s, 1H), 8.24 (s, 2H), 7.86 (dd, *J* = 5.3, 1.8 Hz, 1H), 7.49 - 7.39 (m, 2H), 7.34 (ddd, *J* = 8.8, 7.0, 2.0 Hz, 1H), 7.05 (t, *J* = 74.0 Hz, 1H), 6.54 - 6.43 (m, 3H), 6.38 (s, 1H), 5.80 (d, *J* = 6.7 Hz, 1H), 4.25 (q, *J* = 7.0 Hz, 1H), 4.07 (q, *J* = 6.4 Hz, 1H), 2.10 - 1.99 (m, 2H), 1.77 (t, *J* = 7.1 Hz, 2H), 1.68 (s, 6H), 1.54 - 1.33 (m, 2H). |

(continued)

| | | |
|---|---|---|
| A59 | 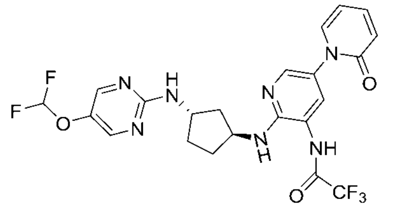 | $^1$H NMR (400 MHz, DMSO-$d_6$) δ 8.26 - 8.20 (s, 2H), 7.95 - 7.86 (s, 1H), 7.67 - 7.58 (dd, J = 6.9, 2.1 Hz, 2H), 7.55 - 7.45 (m, 2H), 7.24 - 6.84 (t, J = 74.0 Hz, 1H), 6.50 - 6.41 (t, J = 7.2 Hz, 2H), 6.34 - 6.25 (m, 1H), 4.54 - 4.44 (m, 1H), 4.36 - 4.25 (m, 1H), 2.24 - 2.03 (m, 2H), 2.02 - 1.85 (m, 2H), 1.60 - 1.47 (m, 2H). |
| A60 | 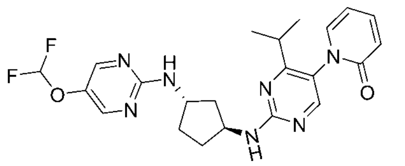 | $^1$H NMR (400 MHz, DMSO-$d_6$) δ 8.23 (s, 2H), 8.13 - 8.06 (m, 1H), 8.02 (s, 1H), 7.84 (ddd, *J* = 8.3, 7.3, 2.0 Hz, 1H), 7.46 (d, *J* = 7.2 Hz, 1H), 7.21 - 6.84 (m, 4H), 4.30 (h, *J* = 6.8 Hz, 2H), 2.87 (p, *J* = 6.8 Hz, 1H), 2.16 - 2.05 (m, 2H), 1.95 - 1.86 (m, 2H), 1.59 - 1.49 (m, 2H), 1.05 (dd, *J* = 6.8, 3.3 Hz, 6H). |
| A61 | 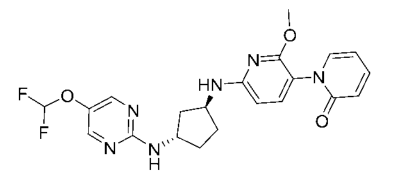 | $^1$H NMR (400 MHz, DMSO-$d_6$) δ 8.26 - 8.23 (s, 2H), 7.53 - 7.48 (d, J = 7.2 Hz, 1H), 7.47 - 7.40 (m, 2H), 7.27 - 7.22 (m, 1H), 7.06 - 6.85 (m, 2H), 6.44 - 6.39 (m, 1H), 6.23 - 6.18 (m, 1H), 6.13 - 6.07 (d, J = 8.4 Hz, 1H), 4.38 - 4.22 (m, 2H), 3.79 - 3.73 (s, 3H), 2.24 - 2.06 (m, 2H), 2.03 - 1.83 (m, 2H), 1.60 - 1.48 (m, 2H). |
| A62 | 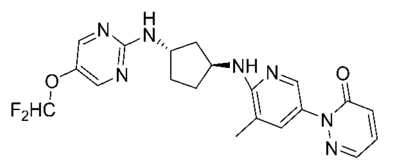 | $^1$H NMR (400 MHz, DMSO-$d_6$) δ 8.24 (s, 2H), 8.04 - 7.98 (m, 2H), 7.51 - 7.42 (m, 2H), 7.42 - 7.37 (m, 1H), 7.07 - 6.99 (m, 1H), 5.98 (d, J = 7.0 Hz, 1H), 4.54 (h, J = 6.9 Hz, 1H), 4.33 (h, J = 6.9 Hz, 1H), 2.23 - 2.07 (m, 4H), 1.95 (t, J = 7.1 Hz, 2H), 1.56 (tdd, J = 16.0, 9.3, 4.6 Hz, 2H). |
| A63 | 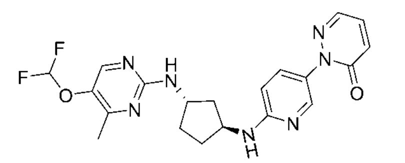 | $^1$H NMR (400 MHz, DMSO-$d_6$) δ 8.21 - 8.03 (m, 2H), 8.01 (dd, *J* = 3.8, 1.6 Hz, 1H), 7.48 (ddd, *J* = 19.9, 9.2, 3.3 Hz, 2H), 7.36 (d, *J* = 7.3 Hz, 1H), 7.19 - 6.81 (m, 3H), 6.52 (d, *J* = 9.0 Hz, 1H), 4.31 (p, *J* = 6.6 Hz, 2H), 2.24 (s, 3H), 2.12 (tt, *J* = 12.8, 6.2 Hz, 2H), 1.87 (ddp, *J* = 18.7, 13.3, 6.8 Hz, 2H), 1.51 (td, *J* = 16.3, 8.0 Hz, 2H). |
| A64 | 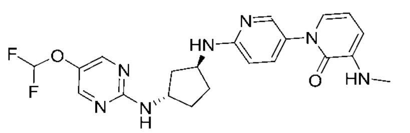 | $^1$H NMR (400 MHz, DMSO-$d_6$) δ 8.25 - 8.21 (s, 2H), 7.93 - 7.90 (d, J = 2.6 Hz, 1H), 7.50 - 7.47 (d, J = 7.2 Hz, 1H), 7.40 - 7.36 (m, 1H), 7.23 - 7.02 (d, J = 74.0 Hz, 1H), 6.91 - 6.76 (m, 2H), 6.55 - 6.51 (d, J = 8.9 Hz, 1H), 6.22 - 6.12 (m, 2H), 5.64 - 5.58 (m 1H), 4.38 - 4.26 (m, 2H), 2.73 - 2.68 (d, J = 5.2 Hz, 3H), 2.19 - 2.07 (m, 2H), 1.95 - 1.81 (m, 2H), 1.59 - 1.45 (m, 2H). |
| A65 | 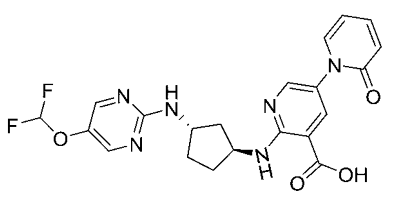 | $^1$H NMR (400 MHz, DMSO-$d_6$) δ 8.69 - 8.49 (s, 1H), 8.28 - 8.19 (d, J = 11.1 Hz, 3H), 8.05 - 7.99 (d, J = 2.8 Hz, 1H), 7.70 - 7.64 (dd, J = 6.8, 2.1 Hz, 1H), 7.57 - 7.46 (m, 2H), 7.24 - 6.83 (s, 1H), 6.51 - 6.43 (d, J = 9.2 Hz, 1H), 6.33 - 6.26 (m, 1H), 4.62 - 4.51 (q, J = 6.7 Hz, 1H), 4.39 - 4.27 (m, 1H), 2.28 - 2.19 (m, 1H), 2.18 - 2.08 (m, 4.6 Hz, 1H), 2.06 - 1.96 (m, 1H), 1.94 - 1.83 (m, 1H), 1.65 - 1.45 (m, 2H). |
| A66 | 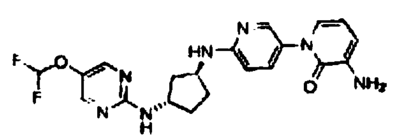 | $^1$H NMR (400 MHz, DMSO-$d_6$) δ 8.25 - 8.22 (s, 2H), 7.93 - 7.90 (d, J = 2.7 Hz, 1H), 7.51 - 7.47 (d, J = 7.2 Hz, 1H), 7.40 - 7.36 (m, 1H), 7.23 - 7.02 (d, J = 74.0 Hz, 1H), 6.91 - 6.78 (m, 2H), 6.55 - 6.46 (m, 2H), 6.12 - 6.07 (t, J = 7.0 Hz, 1H), 5.21 - 5.15 (s, 2H), 4.36 - 4.26 (m, 2H), 2.20 - 2.06 (m, 2H), 1.95 - 1.82 (m, 2H), 1.59 - 1.43 (m, 2H). |
| A67 | 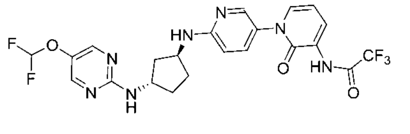 | $^1$H NMR (400 MHz, DMSO-$d_6$) δ 8.25 - 8.21 (s, 2H), 8.03 - 7.95 (m, 2H), 7.60 - 7.55 (d, J = 6.8 Hz, 1H), 7.51 - 7.40 (m, 2H), 7.23 - 6.83 (m, 3H), 6.57 - 6.51 (d, J = 9.0 Hz, 1H), 6.43 - 6.37 (t, J = 7.1 Hz, 1H), 4.37 - 4.26 (m, 2H), 2.20 - 2.07 (m, 2H), 1.95 - 1.81 (m, 2H), 1.58 - 1.45 (m, 2H). |

(continued)

| | | |
|---|---|---|
| A68 | 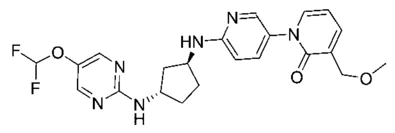 | $^1$H NMR (400 MHz, DMSO-$d_6$) δ 8.25 - 8.21 (s, 2H), 7.93 - 7.90 (d, J = 2.7 Hz, 1H), 7.57 - 7.53 (dd, J = 6.8, 2.1 Hz, 1H), 7.50 - 7.43 (m, 2H), 7.42 - 7.37 (dd, J = 8.9, 2.7 Hz, 1H), 7.23 - 7.02 (d, J = 73.9 Hz, 1H), 6.94 - 6.83 (m, 1H), 6.54 - 6.50 (d, J = 8.9 Hz, 1H), 6.33 - 6.28 (t, J = 6.8 Hz, 1H), 4.36 - 4.26 (m, 2H), 4.25 - 4.22 (s, 2H), 2.18 - 2.07 m, 2H), 1.94 - 1.81 (m, 2H), 1.59 - 1.42 (m, 2H). |
| A69 | 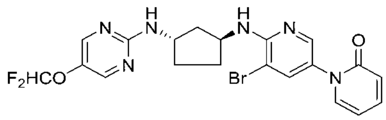 | $^1$H NMR (400 MHz, DMSO-$d_6$) δ 8.24 (s, 2H), 8.04 (d, J = 2.3 Hz, 1H), 7.90 (d, J = 2.3 Hz, 1H), 7.65 (dd, J = 6.9, 2.0 Hz, 1H), 7.53 - 7.47 (m, 2H), 7.03 (s, 1H), 6.49 - 6.44 (m, 1H), 6.29 (ddd, J = 8.9, 5.1, 1.8 Hz, 2H), 4.54 (q, J = 7.2 Hz, 1H), 4.39 - 4.30 (m, 1H), 2.21 - 2.06 (m, 2H), 2.03 - 1.92 (m, 2H), 1.69 - 1.50 (m, 2H). |
| A70 | 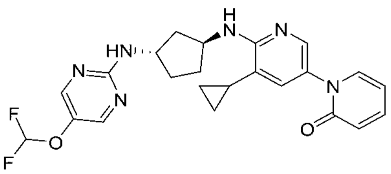 | $^1$H NMR: (400 MHz, $CDCl_3$)<br>*δ* 8.19 (s, 2H), 7.97 (d, *J* = 2.4 Hz, 1H), 7.39 (ddd, *J* = 2.1, 6.8, 9.1 Hz, 1H), 7.35 (d, *J* = 1.6 Hz, 1H), 7.29 (br d, *J* = 2.0 Hz, 1H), 6.65 (d, *J* = 9.3 Hz, 1H), 6.61 - 6.18 (m, 2H), 5.53 - 5.30 (m, 1H), 5.11 (br d, *J* = 4.1 Hz, 1H), 4.73 - 4.58 (m, 1H), 4.52 - 4.36 (m, 1H), 2.50 - 2.28 (m, 2H), 2.20 - 2.02 (m, 2H), 1.62 - 1.55 (m, 3H), 1.00 - 0.93 (m, 2H), 0.67 - 0.59 (m, 2H) |
| A71 | 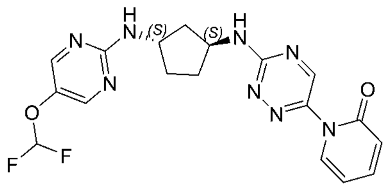 | $^1$H NMR: (DMSO-$d_6$, 400 MHz)<br>8.60 (s, 1H), 8.24 (s, 2H), 7.83 (dd, J = 1.5, 7.0 Hz, 1H), 7.58 (ddd, J = 2.1, 6.8, 9.1 Hz, 1H), 7.51 (d, J = 7.1 Hz, 1H), 7.03 (t, J = 74.0 Hz, 1H), 6.52 (d, J = 9.1 Hz, 1H), 6.41 (dt, J = 1.1, 6.8 Hz, 1H), 4.54 - 4.27 (m, 2H), 2.20 - 2.08 (m, 2H), 1.96 (br t, J = 6.8 Hz, 2H), 1.67 - 1.51 (m, 2H) |
| A72 | 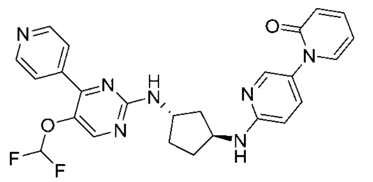 | $^1$H NMR: (400 MHz, MeOD) δ 8.81 - 8.54 (m, 2H), 8.39 - 8.24 (m, 1H), 8.10 - 7.86 (m, 3H), 7.64 - 7.55 (m, 2H), 7.50 - 7.37 (m, 1H), 6.89 - 6.42 (m, 4H), 4.59 - 4.28 (m, 2H), 2.41 - 2.21 (m, 2H), 2.17 - 1.95 (m, 2H), 1.79 - 1.53 (m, 2H). |
| A73 | 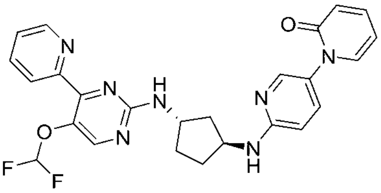 | $^1$H NMR: (400 MHz, DMSO-$d_6$)δ8.68 (d, *J* = 4.8 Hz, 1H), 8.32 (s, 1H), 8.03 - 7.83 (m, 3H), 7.68 (br d, *J* = 7.0 Hz, 1H), 7.60 (dd, *J* = 1.6, 6.8 Hz, 1H), 7.55 - 7.44 (m, 2H), 7.40 (dd, *J* = 2.6, 8.9 Hz, 1H), 7.26 - 6.80 (m, 2H), 6.53 (d, *J* = 8.9 Hz, 1H), 6.44 (d, *J* = 8.9 Hz, 1H), 6.27 (dt, *J* = 1.3, 6.7 Hz, 1H), 4.45 - 4.26 (m, 2H), 2.22 - 2.10 (m, 2H), 2.03 - 1.85 (m, 2H), 1.66 - 1.44 (m, 2H). |

Example B1 (Compound B1)

Synthesis of 6'-(((1S,35)-3-((6,7-dihydro-5H-cyclopenta[d]pyrimidin-2-yl)amino)cyclopentyl)amino)-2H-[1,3 '-bipyridin]-2-one

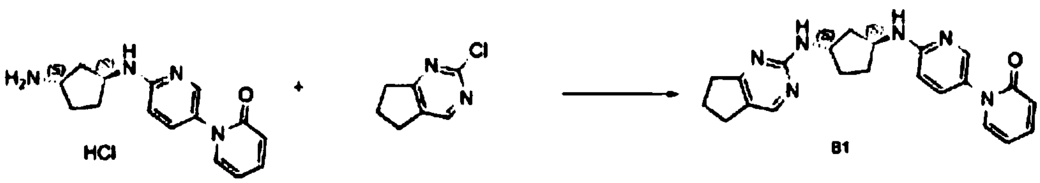

At 25 °C, 6'-(((1S,3S)-3-aminocyclopentyl)amino)-2H-[1,3'-bipyridin]-2-one hydrochloride (342 mg, 1.0 mmol), 2-chloro-6,7-dihydro-5H-cyclopenta[d]pyrimidine (0.2 g, 1.29 mmol), and TEA (1.01 g, 10 mmol) were dissolved in DMSO

(15 mL). After the addition, the mixture was heated to 110 °C and reacted for 2 days.

Water (100 mL) was added, followed by extraction with ethyl acetate (50 mL * 4). The organic phases were combined, washed with saturated brine (50 mL) and concentrated to obtain a crude product. The crude product was purified by column chromatography (DCM:MeOH = 10:1) to obtain an intermediate, which was further purified by reverse-phase column chromatography and then by preparative chromatography to obtain 100 mg of 6'-(((1S,35)-3-((6,7-dihydro-5H-cyclopenta[d]pyrimidin-2-yl)amino)cyclopentyl)amino)-2H-[1,3 '-bipyridin]-2-one.

LC-MS: $[M+H]^+$ = 389. $^1$H NMR (400 MHz, DMSO-$d_6$) δ 8.07 (s, 1H), 7.92 (d, J = 2.6 Hz, 1H), 7.61 (s, 1H), 7.48 (ddd, J = 9.0, 6.6, 2.1 Hz, 1H), 7.38 (d, J = 2.7 Hz, 1H), 6.94 (dd, J = 10.7, 7.1 Hz, 2H), 6.52 (d, J = 8.9 Hz, 1H), 6.44 (d, J = 9.1 Hz, 1H), 6.27 (d, J = 1.4 Hz, 1H), 4.33 (dp, J = 20.4, 6.7 Hz, 2H), 2.70 (td, J = 7.5, 4.2 Hz, 4H), 2.19 - 2.05 (m, 2H), 2.03 - 1.79 (m, 4H), 1.49 (td, J = 12.4, 7.0 Hz, 2H).

Example B2 (Compound B2)

Synthesis of 6'-(((1S,3S)-3-((5,6,7,8-tetrahydroquinazolin-2-yl)amino)cyclopentyl)amino)-2H-[1,3'-bipyridin] -2-one

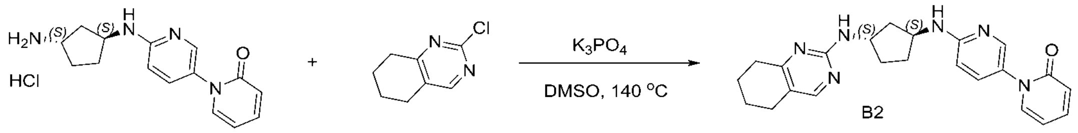

B2

At 25 °C, 6'-(((1S,3S)-3-aminocyclopentyl)amino)-2H-[1,3'-bipyridin]-2-one hydrochloride (172 mg, 0.5 mmol) and 2-chloro-5,6,7,8-tetrahydroquinazoline (126 mg, 0.75 mmol) were dissolved in DMSO (5 mL). Potassium phosphate (634 mg, 3 mmol) was added. After the addition, the mixture was heated to 140 °C and reacted overnight.

Water (10 mL) was added, followed by extraction with EA (10 mL * 3). The organic phases were combined and concentrated to obtain a crude product. The crude product was purified by column chromatography (EA:MeOH = 13:1) to obtain 6'-(((1S,3S)-3-((5,6,7,8-tetrahydroquinazolin-2-yl)amino)cyclopentyl)amino)-2H-[1,3'-bipyridin] -2-one.

LC-MS: $[M+H]^+$ = 403.2. $^1$H NMR (400 MHz, Chloroform-d) δ 8.02 (d, J = 2.6 Hz, 1H), 7.98 (s, 1H), 7.50 (dd, J = 8.9, 2.7 Hz, 1H), 7.42 - 7.34 (m, 1H), 7.29 (dd, J = 6.9, 2.1 Hz, 1H), 6.64 (dd, J = 9.2, 1.2 Hz, 1H), 6.43 (d, J = 8.9 Hz, 1H), 6.23 (t, J = 6.7 Hz, 1H), 5.03 (d, J = 7.3 Hz, 1H), 4.90 (d, J = 6.8 Hz, 1H), 4.43 (q, J = 6.8 Hz, 1H), 4.21 (q, J = 6.4 Hz, 1H), 2.65 (t, J = 6.4 Hz, 2H), 2.56 (t, J = 6.2 Hz, 2H), 2.35 - 2.24 (m, 2H), 2.03 - 1.99 (m, 2H), 1.85 - 1.71 (m, 4H), 1.61 - 1.50 (m, 2H).

Example B3 (Compound B3)

Synthesis of 6'-(((1S,3S)-3-((6,7-dihydro-[1,4]dioxino[2,3-d]pyrimidin-2-yl)amino)cyclopentyl)amino)-2H-[1 ,3'-bipyridin]-2-one

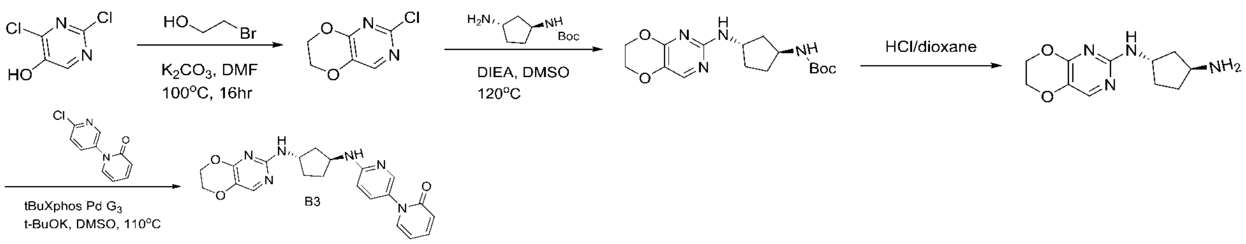

B3

Step A: Synthesis of 2-chloro-6,7-dihydro-[1,4]dioxino[2,3-d]pyrimidine

2,4-Dichloropyrimidin-5-ol (3 g, 18.2 mmol) was added to anhydrous N,N-dimethylformamide (30 mL). Potassium carbonate (12.5 g, 90.9 mmol) was added. The atmosphere was purged with nitrogen, and the mixture was stirred at room temperature for 30 minutes. 2-Bromoethanol (6.8 g, 54.6 mmol) was added, and the mixture was reacted at 100 °C for 16 hours.

The reaction mixture was added to a saturated aqueous ammonium chloride solution (100 mL) and extracted with dichloromethane (40 mL × 3). The organic phases were combined, dried and concentrated.

The resulting material was purified by Flash silica gel column chromatography (9 to 13% ethyl acetate/petroleum

ether) to obtain 1 g of 2-chloro-6,7-dihydro-[1,4]dioxino[2,3-d]pyrimidine as a pale yellow solid (yield: 31.9%). LCMS: Rt = 0.946 min, $[M+H]^+$ = 173.

Step B: Synthesis of tert-butyl ((1S,3S)-3-((6,7-dihydro-[1,4]dioxino[2,3-d]pyrimidin-2-yl)amino)cyclopentyl)carbamate

2-Chloro-6,7-dihydro-[1,4]dioxino[2,3-d]pyrimidine (1 g, 5.8 mmol) was added to anhydrous dimethyl sulfoxide (10 mL). N,N-Diisopropylethylamine (3.2 mL, 18.2 mmol) and tert-butyl ((1S,3S)-3-aminocyclopentyl)carbamate (1.16 g, 5.8 mmol) were added. Under nitrogen protection, the mixture was heated to 120 C and reacted for 16 hours. The reaction mixture was added to a saturated aqueous ammonium chloride solution (50 mL) and extracted with ethyl acetate (20 mL × 3). The organic phases were combined, dried and concentrated. The resulting material was purified by Flash silica gel column chromatography (20 to 35% ethyl acetate/petroleum ether) to obtain 400 mg of tert-butyl ((1S,3S)-3-((6,7-dihydro-[1,4]dioxino[2,3-d]pyrimidin-2-yl)amino)cyclopentyl)carbamate as a pale yellow solid (yield: 20.7%). LCMS: Rt =1.128 min, $[M+H]^+$ = 337.

Step C: Synthesis of (1S,3S)-$N^1$-(6,7-dihydro-[1,4]dioxino[2,3-d]pyrimidin-2-yl)cyclopentane-1,3-diamine

tert-Butyl ((1S,3S)-3-((6,7-dihydro-[1,4]dioxino[2,3-d]pyrimidin-2-yl)amino)cyclopentyl)carbamate (400 mg, 1.2 mmol) was dissolved in dichloromethane (5 mL). A 4 M hydrogen chloride solution in 1,4-dioxane (4 mL, 16 mmol) was added. The mixture was reacted at room temperature for 2 hours. The reaction mixture was concentrated under reduced pressure. The residue was dissolved in methanol (3 mL), and the pH was adjusted to 8 using a hydroxide ion exchange resin. The mixture was filtered, and the filtrate was concentrated to obtain 340 mg of (1S,3S)-$N^1$-(6,7-dihydro-[1,4]dioxino[2,3-d]pyrimidin-2-yl)cyclopentane-1,3-diamine as a pale yellow solid. LCMS: Rt=0.334 min, $[M+H]^+$ = 237.

Step D: Synthesis of 6'-(((1S,3S)-3-((6,7-dihydro-[1,4]dioxino[2,3-d]pyrimidin-2-yl)amino)cyclopentyl)amino)-2H-[1 ,3'-bipyridin]-2-one

(1S,3S)-$N^1$-(6,7-dihydro-[1,4]dioxino[2,3-d]pyrimidin-2-yl)cyclopentane-1,3-diamine (340 mg crude, 1.2 mmol) and Compound 6 (297.5 mg, 1.44 mmol) were added to anhydrous dimethyl sulfoxide (5 mL). Potassium tert-butoxide (269.3 mg, 2.4 mmol) and methanesulfonato(2-di-tert-butylphosphino-2',4',6'-triisopropyl-1,1'-biphenyl)(2'-amino-1,1'-biphenyl-2-yl)palladium(II) (190.6 mg, 0.24 mmol) were added. The atmosphere was purged with nitrogen, and the mixture was heated to 110 °C and reacted for 16 hours.

The reaction mixture was added to a saturated aqueous ammonium chloride solution (20 mL) and extracted with dichloromethane (10 mL × 3). The organic phases were combined, dried and concentrated. The resulting material was purified by Flash silica gel column chromatography (3 to 4% methanol/dichloromethane) and further purified by reverse-phase column chromatography (17 to 20% acetonitrile/water) to obtain 93.9 mg of 6'-(((1S,3S)-3-((6,7-dihydro-[1,4]dioxino[2,3-d]pyrimidin-2-yl)amino)cyclopentyl)amino)-2H-[1 ,3'-bipyridin]-2-one. LCMS: Rt =0.827 min, $[M+H]^+$ = 407.

$^1$H NMR (400 MHz, DMSO-$d_6$) δ 7.91 (d, J = 2.6 Hz, 1H), 7.83 (s, 1H), 7.61 (dd, J = 6.9, 2.0 Hz, 1H), 7.48 (ddd, J = 8.9, 6.5, 2.1 Hz, 1H), 7.39 (dd, J = 8.9, 2.7 Hz, 1H), 6.91 (d, J = 6.9 Hz, 1H), 6.80 (d, J = 7.2 Hz, 1H), 6.51 (d, J = 8.9 Hz, 1H), 6.44 (d, J = 9.2 Hz, 1H), 6.27 (td, J = 6.7, 1.2 Hz, 1H), 4.39 (dd, J = 5.5, 2.7 Hz, 2H), 4.24 (dq, J = 27.3, 6.7 Hz, 2H), 4.16 - 4.09 (m, 2H), 2.16 - 2.02 (m, 2H), 1.83 (q, J = 7.1 Hz, 2H), 1.52 - 1.40 (m, 2H).

Example B4 (Compound B4)

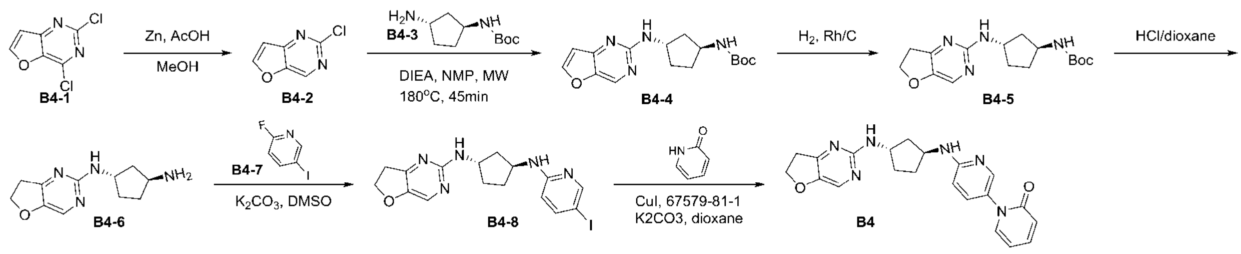


Step A: Synthesis of Compound B4-2

Compound B4-1 (1.89 g, 10 mmol) was dissolved in methanol (20 mL). Acetic acid (1.8 g, 30 mmol) was added. Zinc powder (1.3 g, 20 mmol) was added in batches under a water bath. The reaction was continued for 2 hours. The

reaction mixture was filtered, and the filtrate was concentrated. The resulting material was purified by Flash silica gel column chromatography (0 to 30% ethyl acetate/petroleum ether) to obtain 1.16 g of Compound B4-2 as a white solid (yield: 75%). LCMS: $[M+H]^+$ = 155.

Step B: Synthesis of Compound B4-4

Compound B4-2 (464 mg, 3 mmol) was dissolved in N-methylpyrrolidone (12 mL). Compound B4-3 (600 mg, 3 mmol) and N,N-diisopropylethylamine (1.5 mL, 9 mmol) were added. The atmosphere was purged with nitrogen, and the mixture was reacted at 180 °C under microwave irradiation for 45 minutes. A total of 6 batches were run. The reaction mixtures were poured into a saturated aqueous ammonium chloride solution and extracted with ethyl acetate (50 mL × 3). The organic phases were combined, dried and concentrated. The resulting material was purified by Flash silica gel column chromatography (0 to 60% ethyl acetate/petroleum ether) to obtain 0.5 g of Compound B4-4 as a white solid (yield: 8.7%). LCMS: $[M+H]^+$ = 319.

Step C: Synthesis of Compound B4-5

Compound B4-4 (580 mg, 1.8 mmol) was dissolved in methanol (10 mL). The atmosphere was purged with nitrogen, and rhodium on carbon (100 mg) was added. The mixture was reacted under a hydrogen atmosphere at room temperature overnight. The reaction mixture was filtered, and the filtrate was concentrated. The resulting material was purified by Flash silica gel column chromatography (0 to 60% ethyl acetate/petroleum ether) to obtain 110 mg of Compound B4-5 as a white solid (yield: 18.8%). LCMS: $[M+H]^+$ = 321.

Step D: Synthesis of Compound B4-8

Compound B4-5 (110 mg, 0.34 mmol) was dissolved in methanol (2 mL). A 4 M hydrogen chloride solution in 1,4-dioxane (2 mL, 8 mmol) was added. The mixture was reacted at room temperature for 2 hours. The reaction mixture was directly dried by rotary evaporation for use in the next step. The above residue was dissolved in dimethyl sulfoxide (2 mL). Compound B4-7 (115 mg, 0.51 mmol) and potassium carbonate (138 mg, 1 mmol) were added. The atmosphere was purged with nitrogen, and the mixture was reacted at 120 °C overnight. The reaction mixture was poured into a saturated aqueous ammonium chloride solution and extracted with ethyl acetate (20 mL × 3). The organic phases were combined, dried and concentrated. The resulting material was purified by Flash silica gel column chromatography (0 to 70% ethyl acetate/petroleum ether) to obtain 47 mg of Compound B4-8 as a pale yellow solid (yield: 32.6%). LCMS: $[M+H]^+$ = 424.

Step E: Synthesis of Compound B4

Compound B4-8 (47 mg, 0.11 mmol) and 2-pyridone (16 mg, 0.17 mmol) were dissolved in anhydrous 1,4-dioxane (2 mL). Potassium carbonate (31 mg, 0.22 mmol), copper(I) iodide (5 mg, 0.02 mmol), and trans-(1R,2R)-N,N'-dimethylcyclohexane-1,2-diamine (3 mg, 0.02 mmol) were added. The atmosphere was purged with nitrogen, and the mixture was reacted at 110 °C for 3 hours. The reaction mixture was cooled to room temperature, filtered, and the filtrate was concentrated. The resulting material was purified by silica gel preparative TLC plate (developing solvent: dichloromethane/methanol = 10/1) to obtain 19.2 mg of Compound B4 as an off-white solid (yield: 44%). LCMS: Rt = 0.832 min, $[M+H]^+$ = 391.

$^1H$ NMR (400 MHz, DMSO-$d_6$) δ 7.97 (d, J = 2.6 Hz, 1H), 7.84 (s, 1H), 7.66 (dd, J = 6.9, 2.0 Hz, 1H), 7.54 (ddd, J = 8.9, 6.6, 2.1 Hz, 1H), 7.45 (dd, J = 8.9, 2.7 Hz, 1H), 6.98 (d, J = 6.9 Hz, 1H), 6.81 (d, J = 7.3 Hz, 1H), 6.58 (d, J = 8.9 Hz, 1H), 6.50 (d, J = 9.2 Hz, 1H), 6.37 - 6.29 (m, 1H), 4.54 (t, J = 8.7 Hz, 2H), 4.34 (p, J = 7.2 Hz, 2H), 3.17 (t, J = 8.7 Hz, 2H), 2.18 (ddd, J = 16.9, 8.3, 5.1 Hz, 2H), 1.98 - 1.85 (m, 2H), 1.54 (td, J = 12.4, 7.0 Hz, 2H).

Example B5 (Compound B5)

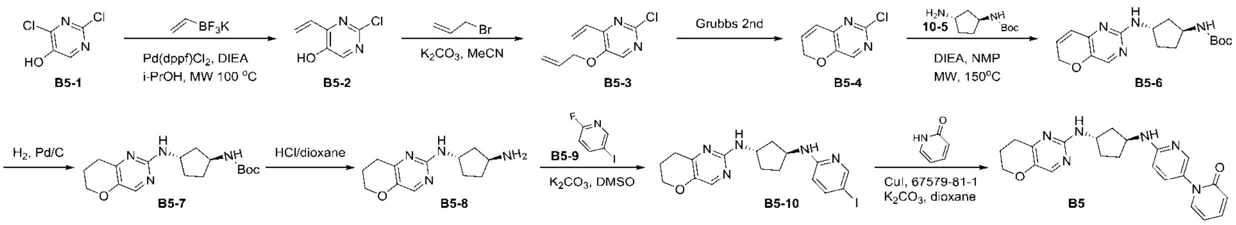


Step A: Synthesis of Compound B5-2

Compound B5-1 (330 mg, 2 mmol) and potassium vinyltrifluoroborate (281 mg, 2.1 mmol) were added to isopropanol (15 mL). N,N-Diisopropylethylamine (0.7 mL, 4 mmol) was added. The atmosphere was purged with nitrogen. [1,1'-Bis(diphenylphosphino)ferrocene]palladium(II) dichloride (73 mg, 0.1 mmol) was added. The mixture was reacted at 100 C under microwave irradiation for 0.5 hours. A total of 10 batches were run. The reaction mixtures were filtered through a silica gel pad, and the filtrates were concentrated to obtain 7 g of Compound B5-2 as a pale yellow liquid (crude). LCMS: $[M+H]^+$ = 157.

Step B: Synthesis of Compound B5-3

Compound B5-2 (7 g, crude) was added to acetonitrile (100 mL). Potassium carbonate (5.5 g, 40 mmol) and allyl bromide (2.9 g, 24 mmol) were added. The mixture was reacted at room temperature for 16 hours. The reaction mixture was diluted with ethyl acetate, filtered, and the filtrate was concentrated. The resulting material was purified by Flash silica gel column chromatography (0 to 10% ethyl acetate/petroleum ether) to obtain 1.92 g of Compound B5-3 as a pale yellow solid (two-step yield: 4.9%). LCMS: $[M+H]^+$ = 197.

Step C: Synthesis of Compound B5-4

Compound B5-3 (1.92 g, 9.7 mmol) was dissolved in 1,2-dichloroethane (800 mL). Nitrogen was bubbled through the solution for 10 minutes. Grubbs catalyst II (1 g) was added. Under nitrogen protection, the mixture was reacted at 90 °C for 16 hours. The reaction mixture was concentrated under reduced pressure. The resulting material was purified by Flash silica gel column chromatography (0 to 15% ethyl acetate/petroleum ether) to obtain 1.37 g of Compound B5-4 as an earthy yellow solid (yield: 81%). LCMS: $[M+H]^+$ = 169.

Step D: Synthesis of Compound B5-6

Compound B5-4 (700 mg, 4.1 mmol) was dissolved in N-methylpyrrolidone (10 mL). Compound B5-5 (820 mg, 4.1 mmol) and N,N-Diisopropylethylamine (2.1 mL, 12.4 mmol) were added. The atmosphere was purged with nitrogen, and the mixture was reacted at 150 °C under microwave irradiation for 1 hour. The reaction mixture was poured into a saturated aqueous ammonium chloride solution and extracted with ethyl acetate (50 mL × 3). The organic phases were combined, dried and concentrated. The resulting material was purified by Flash silica gel column chromatography (0 to 60% ethyl acetate/petroleum ether) to obtain 125 mg of Compound B5-6 as a white solid (yield: 9.1%). LCMS: $[M+H]^+$ = 333.

Step E: Synthesis of Compound B5-7

Compound B5-6 (125 mg, 0.38 mmol) was dissolved in methanol (5 mL). The atmosphere was purged with nitrogen, and palladium on carbon (20 mg) was added. The mixture was reacted under a hydrogen atmosphere at room temperature overnight. The reaction mixture was filtered, and the filtrate was concentrated to obtain 90 mg of Compound B5-7 as a grayish-white solid (yield: 72%). LCMS: $[M+H]^+$ = 335.

Step F: Synthesis of Compound B5-10

Compound B5-7 (90 mg, 0.27 mmol) was dissolved in methanol (2 mL). A 4 M hydrogen chloride solution in 1,4-dioxane (2 mL, 8 mmol) was added. The mixture was reacted at room temperature for 2 hours. The reaction mixture was directly dried by rotary evaporation for use in the next step. The above residue was dissolved in dimethyl sulfoxide (2 mL). Compound B5-9 (90 mg, 0.4 mmol) and potassium carbonate (112 mg, 0.8 mmol) were added. The atmosphere was purged with nitrogen, and the mixture was reacted at 120 °C overnight. The reaction mixture was poured into a saturated

aqueous ammonium chloride solution and extracted with ethyl acetate (20 mL × 3). The organic phases were combined, dried and concentrated. The resulting material was purified by Flash silica gel column chromatography (0 to 70% ethyl acetate/petroleum ether) to obtain 43 mg of Compound B5-10 as a pale yellow solid (yield: 37%). LCMS: $[M+H]^+$ = 438.

Step G: Synthesis of Compound B5

Compound B5-10 (43 mg, 0.1 mmol) and 2-pyridone (14 mg, 0.15 mmol) were dissolved in anhydrous 1,4-dioxane (2 mL). Potassium carbonate (28 mg, 0.22 mmol), copper(I) iodide (5 mg, 0.02 mmol), and trans-(1R,2R)-N,N'-dimethylcyclohexane-1,2-diamine (3 mg, 0.02 mmol) were added. The atmosphere was purged with nitrogen, and the mixture was reacted at 110 °C for 3 hours. The reaction mixture was cooled to room temperature, filtered, and the filtrate was concentrated. The resulting material was purified by silica gel preparative TLC plate (developing solvent: dichloromethane/methanol = 10/1) to obtain 8.9 mg of Compound B5 as a yellow solid (yield: 22%). LCMS: $[M+H]^+$ = 405.

$^1$H NMR (400 MHz, Methanol-$d_4$) δ 7.93 (d, J = 2.6 Hz, 1H), 7.80 (s, 1H), 7.65 - 7.56 (m, 2H), 7.43 (dd, J = 9.0, 2.7 Hz, 1H), 6.65 - 6.55 (m, 2H), 6.46 (td, J = 6.8, 1.3 Hz, 1H), 4.32 (td, J = 6.6, 4.3 Hz, 2H), 4.13 - 4.06 (m, 2H), 2.73 (t, J = 6.6 Hz, 2H), 2.29 - 2.19 (m, 2H), 2.07 - 2.02 (m, 2H), 1.97 (t, J = 6.7 Hz, 2H), 1.61 - 1.54 (m, 2H).

Example B6 (Compound B6)

Synthesis of 6'-(((1S,3S)-3-((6,7-dihydrothieno[3,2-d]pyrimidin-2-yl)amino)cyclopentyl)amino)-2H-[1,3'-bip yridin]-2-one

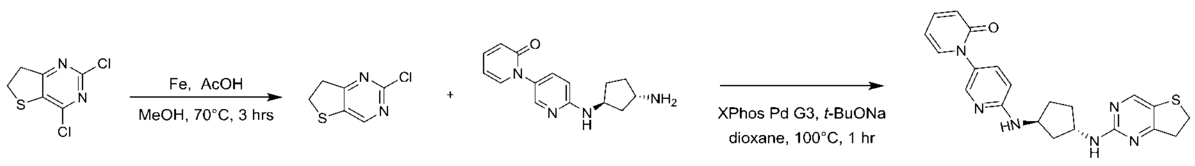


B6

Step 1: Synthesis of 2-chloro-6,7-dihydrothieno[3,2-d]pyrimidine

2,4-Dichloro-6,7-dihydrothieno[3,2-d]pyrimidine (1.00 g, 4.83 mmol, 1 eq) and acetic acid (1.74 g, 29.0 mmol, 6 eq) were dissolved in methanol (20 mL). Iron powder (1.08 g, 19.3 mmol, 4 eq) was then added to the reaction mixture in batches. After the addition, the mixture was heated to 70 °C and reacted for 3 hours. TLC (petroleum ether/ethyl acetate = 3/1) showed complete reaction. The reaction mixture was filtered, and the filtrate was concentrated under reduced pressure to obtain a crude product. The crude product was purified by silica gel column chromatography to obtain 2-chloro-6,7-dihydrothieno[3,2-d]pyrimidine (570 mg, 3.30 mmol, 68.4% yield).

Step 2: Synthesis of 6'-(((1S,3S)-3-((6,7-dihydrothieno[3,2-d]pyrimidin-2-yl)amino)cyclopentyl)amino)-2H-[1,3'-bip yridin]-2-one

6'-(((1S,3S)-3-Aminocyclopentyl)amino)-2H-[1,3'-bipyridin]-2-one (313 mg, 1.16 mmol, 1 eq) and 2-chloro-6,7-dihydrothieno[3,2-d]pyrimidine (200 mg, 1.16 mmol, 1 eq) were dissolved in 1,4-dioxane (5 mL). Sodium tert-butoxide (278 mg, 2.90 mmol, 2.5 eq) and XPhos Pd G3 (490 mg, 579 μmol, 0.5 eq) were then added. After the addition, the mixture was heated to 100 °C and reacted for 1 hour. LCMS detected the MS value of the product (RT = 0.372 min). The reaction mixture was filtered. The filtrate was diluted with water (20 mL) and extracted with ethyl acetate (10 mL * 2). The organic phases were combined, washed with saturated brine (50 mL * 2), dried over anhydrous sodium sulfate, filtered, and concentrated to obtain a crude product. The crude product was purified by reverse-phase preparative chromatography (column: YMC-Actus Triart C18 150*30 mm*7 um; mobile phase: [water (FA)-ACN]; gradient: 8% to 38% B over 10 min) to obtain 6'-(((1S,3S)-3-((6,7-dihydrothieno[3,2-d]pyrimidin-2-yl)amino)cyclopentyl)amino)-2H-[1,3'-bip yridin]-2-one (10.0 mg, 23.5 μmol, 2.03% yield, 95.6% purity). LCMS (ESI) m/z = 407.2 $[M+1]^+$.

$^1$H NMR: (400 MHz, DMSO-$d_6$): δ 8.07 (s, 1H), 7.91 (d, J = 2.6 Hz, 1H), 7.59 (dd, J = 1.6, 6.8 Hz, 1H), 7.47 (ddd, J = 2.1, 6.8, 9.1 Hz, 1H), 7.38 (dd, J = 2.8, 8.9 Hz, 1H), 7.04 (d, J = 7.4 Hz, 1H), 6.89 (d, J = 6.9 Hz, 1H), 6.52 (d, J = 8.9 Hz, 1H), 6.44 (d, J = 8.9 Hz, 1H), 6.27 (dt, J = 1.3, 6.7 Hz, 1H), 4.30 (td, J = 6.4, 12.7 Hz, 2H), 3.28 (s, 2H), 3.13 - 3.04 (m, 2H), 2.19 - 2.03 (m, 2H), 1.95 - 1.78 (m, 2H), 1.56 - 1.41 (m, 2H).

Example B7 (Compound B7)

Synthesis of 6'-(((1S,3S)-3-((7,8-dihydro-6H-thiopyrano[3,2-d]pyrimidin-2-yl)amino)cyclopentyl)amino)-2H -[1,3'-bipyridin]-2-one

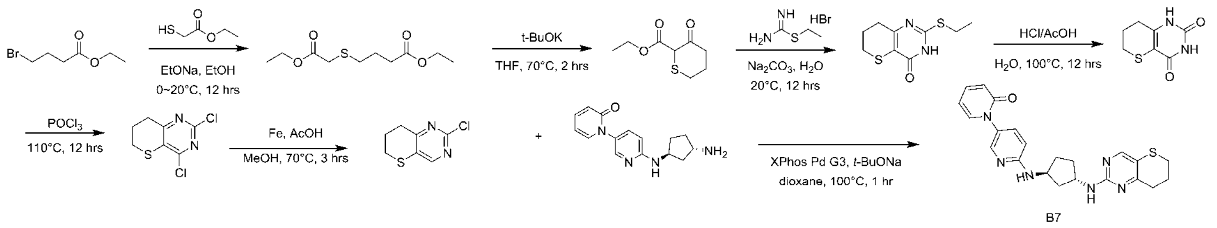


Step 1: Synthesis of ethyl 4-((2-ethoxy-2-oxoethyl)thio)butanoate

Sodium ethoxide (6.98 g, 103 mmol, 1 eq) was dissolved in ethanol (200 mL). The temperature was then lowered to 0 °C. Ethyl 2-sulfanylacetate (12.3 g, 103 mmol, 1 eq) and ethyl 4-bromobutanoate (20.0 g, 103 mmol, 1 eq) were added in batches. After the addition, the mixture was stirred at room temperature for 12 hours. TLC (petroleum ether/ethyl acetate = 5/1) showed complete reaction. The solvent was removed by concentration under reduced pressure. The residue was diluted with water (200 mL) and extracted with ethyl acetate (100 mL × 3). The organic phases were combined, washed with saturated brine (200 mL × 2), dried over anhydrous sodium sulfate, filtered, and concentrated to obtain ethyl 4-((2-ethoxy-2-oxoethyl)thio)butanoate (24.0 g, 102 mmol, 99.9% yield). This was used directly in the next step without purification.

Step 2: Synthesis of ethyl 3-oxotetrahydro-2H-thiopyran-2-carboxylate

Ethyl 4-((2-ethoxy-2-oxoethyl)thio)butanoate (24.0 g, 102 mmol, 1 eq) and potassium tert-butoxide (23.0 g, 205 mmol, 2 eq) were dissolved in tetrahydrofuran (240 mL). After the addition, the mixture was heated to 70 °C and reacted for 2 hours. TLC (petroleum ether/ethyl acetate = 10/1) showed complete reaction. The solvent was removed by concentration under reduced pressure. The residue was diluted with water (200 mL), and the aqueous phase was adjusted to pH = 5 with 2 M hydrochloric acid. The aqueous phase was then extracted with ethyl acetate (100 mL × 2). The organic phases were combined, washed with saturated brine (500 mL × 2), dried over anhydrous sodium sulfate, filtered, and concentrated to obtain ethyl 3-oxotetrahydro-2H-thiopyran-2-carboxylate (16.0 g, 85.0 mmol, 83.0% yield). This was used directly in the next step without purification.

Step 3: Synthesis of 2-(ethylthio)-7,8-dihydro-3H-thiopyrano[3,2-d]pyrimidin-4(6H)-one

Sodium carbonate (9.01 g, 85.0 mmol, 1 eq) and ethyl 3-oxotetrahydro-2H-thiopyran-2-carboxylate (16.0 g, 85.0 mmol, 1 eq) were added to an aqueous solution (150 mL) of S-ethylisothiouronium bromide (15.73 g, 85.00 mmol, 1 eq). After the addition, the mixture was stirred at 20 °C in the dark for 12 hours. TLC (petroleum ether/ethyl acetate = 3/1) showed complete reaction. The resulting solid precipitate was collected by filtration, washed with water (50 mL) and petroleum ether (50 mL), and dried under reduced pressure to obtain 2-(ethylthio)-7,8-dihydro-3H-thiopyrano[3,2-d]pyrimidin-4(6H)-one (14.0 g, 61.3 mmol, 72.1% yield). This was used directly in the next step without purification.

Step 4: Synthesis of 7,8-dihydro-1H-thiopyrano[3,2-d]pyrimidine-2,4(3H,6H)-dione

2-(Ethylthio)-7,8-dihydro-3H-thiopyrano[3,2-d]pyrimidin-4(6H)-one (14.0 g, 61.3 mmol, 1 eq) was dissolved in water (150 mL). Concentrated hydrochloric acid (21.8 mL, 4.26 eq) and acetic acid (19.6 g, 326 mmol, 5.32 eq) were added. The mixture was then stirred at 100 C for 12 hours. TLC (petroleum ether/ethyl acetate = 3/1) showed complete reaction. The reaction mixture was cooled to room temperature and then filtered. The filter cake was dried under reduced pressure to obtain 7,8-dihydro-1H-thiopyrano[3,2-d]pyrimidine-2,4(3H,6H)-dione (9.40 g, 51.0 mmol, 83.2% yield).

Step 5: Synthesis of 2,4-dichloro-7,8-dihydro-6H-thiopyrano[3,2-d]pyrimidine

7,8-Dihydro-1H-thiopyrano[3,2-d]pyrimidine-2,4(3H,6H)-dione (5.00 g, 27.1 mmol, 1 eq) was dissolved in phosphorus oxychloride (20 mL). The mixture was then stirred at 110 °C for 12 hours. LCMS detected the MS value of the product (RT = 0.594 min). The reaction mixture was cooled to 20 °C, slowly poured into ice water (20 mL), and stirred

for 10 minutes. The mixture was extracted with dichloromethane (20 mL × 2). The organic phases were combined, washed with aqueous sodium bicarbonate (50 mL × 2) and saturated brine (50 mL × 2), dried over anhydrous sodium sulfate, filtered, and concentrated to obtain a crude product. The crude product was purified by silica gel column chromatography to obtain 2,4-dichloro-7,8-dihydro-6H-thiopyrano[3,2-d]pyrimidine (4.70 g, 21.3 mmol, 78.3% yield).

Step 6: Synthesis of 2-chloro-7,8-dihydro-6H-thiopyrano[3,2-d]pyrimidine

2,4-Dichloro-7,8-dihydro-6H-thiopyrano[3,2-d]pyrimidine (4.70 g, 21.3 mmol, 1 eq) and acetic acid (7.66 g, 128 mmol, 6 eq) were dissolved in methanol (50 mL). Iron powder (4.75 g, 85.0 mmol, 4 eq) was then added to the reaction mixture in batches. After the addition, the mixture was heated to 70 °C and reacted for 3 hours. TLC (petroleum ether/ethyl acetate = 3/1) showed complete reaction. The reaction mixture was filtered, and the filtrate was concentrated under reduced pressure to obtain a crude product. The crude product was purified by silica gel column chromatography to obtain 2-chloro-7,8-dihydro-6H-thiopyrano[3,2-d]pyrimidine (2.50 g, 13.4 mmol, 63.0% yield).

Step 7: Synthesis of 6'-(((1S,3S)-3-((7,8-dihydro-6H-thiopyrano[3,2-d]pyrimidin-2-yl)amino)cyclopentyl)amino)-2H -[1,3'-bipyridin]-2-one

6'-(((1S,3S)-3-Aminocyclopentyl)amino)-2H-[1,3'-bipyridin]-2-one (434 mg, 1.61 mmol, 1 eq) and 2-chloro-7,8-dihydro-6H-thiopyrano[3,2-d]pyrimidine (300 mg, 1.61 mmol, 1 eq) were dissolved in 1,4-dioxane (8 mL). Sodium tert-butoxide (386 mg, 4.02 mmol, 2.5 eq) and XPhos Pd G3 (680 mg, 804 μmol, 0.5 eq) were then added. After the addition, the mixture was heated to 100 °C and reacted for 1 hour. LCMS detected the MS value of the product (RT = 0.372 min). The reaction mixture was filtered. The filtrate was diluted with water (10 mL) and extracted with ethyl acetate (10 mL × 2). The organic phases were combined, washed with saturated brine (50 mL × 2), dried over anhydrous sodium sulfate, filtered, and concentrated to obtain a crude product. The crude product was purified by reverse-phase preparative chromatography (column: Phenomenex luna C18 150*40mm* 15um; mobile phase: [water (FA)-ACN]; gradient: 5% to 35% B over 15 min) to obtain 6'-(((1S,3S)-3-((7,8-dihydro-6H-thiopyrano[3,2-d]pyrimidin-2-yl)amino)cyclopentyl)amino)-2H -[1,3'-bipyridin]-2-one (36.0 mg, 84.2 μmol, 5.24% yield, 98.4% purity). LCMS (ESI) m/z = 421.2 $[M+1]^+$.

$^1H$ NMR: (400 MHz, DMSO-$d_6$): δ 7.95 (s, 1H), 7.91 (d, J = 2.6 Hz, 1H), 7.59 (dd, J = 1.8, 6.8 Hz, 1H), 7.47 (ddd, J = 2.1, 6.7, 9.0 Hz, 1H), 7.39 (dd, J = 2.7, 8.9 Hz, 1H), 6.98 (d, J = 7.4 Hz, 1H), 6.89 (d, J = 6.8 Hz, 1H), 6.52 (d, J = 8.9 Hz, 1H), 6.44 (d, J = 9.0 Hz, 1H), 6.26 (dt, J = 1.3, 6.7 Hz, 1H), 4.29 (sext, J = 6.7 Hz, 2H), 2.99 - 2.92 (m, 2H), 2.67 (t, J = 6.4 Hz, 2H), 2.17 - 2.02 (m, 4H), 1.94 - 1.77 (m, 2H), 1.56 - 1.41 (m, 2H).

Example B8 (Compound B8)

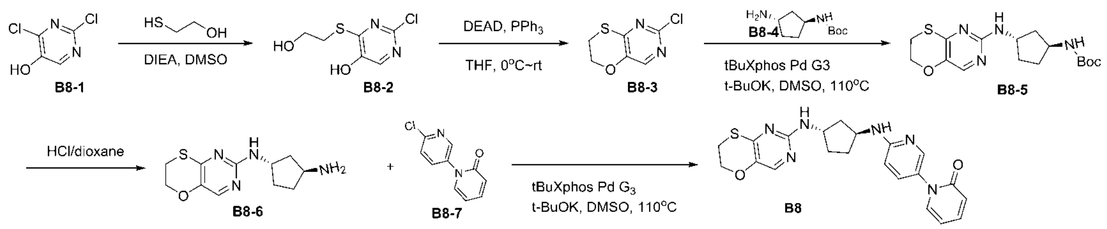


Step A: Synthesis of Compound B8-2

Compound B8-1 (1.3 g, 7.9 mmol) was added to anhydrous dimethyl sulfoxide (15 mL). N,N-Diisopropylethylamine (6.9 mL, 39.5 mmol) and 2-mercaptoethanol (1.9 g, 23.7 mmol) were added. Under nitrogen protection, the mixture was heated to 100 °C and reacted for 12 hours. The reaction mixture was cooled and added to a saturated aqueous ammonium chloride solution (60 mL). The mixture was extracted with ethyl acetate (20 mL × 3). The organic phases were combined, dried and concentrated. The resulting material was purified by Flash silica gel column chromatography (1 to 2% methanol/dichloromethane) to obtain 1.2 g of Compound B8-2 as a pale yellow oil (yield: 73.5%). LCMS: $[M+H]^+$ = 207.

Step B: Synthesis of Compound B8-3

Compound B8-2 (1.2 g, 5.8 mmol) was added to anhydrous tetrahydrofuran (15 mL). Triphenylphosphine (2.3 g, 8.7 mmol) was added. The atmosphere was purged with nitrogen. In an ice-water bath, diethyl azodicarboxylate (1.5 g, 8.7

mmol) was added. The mixture was warmed to room temperature and reacted for 3 hours. The reaction mixture was added to a saturated aqueous sodium bicarbonate solution (40 mL) and extracted with ethyl acetate (25 mL × 3). The organic phases were combined, dried and concentrated. The resulting material was purified by Flash silica gel column chromatography (35 to 37% ethyl acetate/petroleum ether) to obtain 420 mg of Compound B8-3 as a pale yellow oil (yield: 35.2%). LCMS: $[M+H]^+$ = 189.

Step C: Synthesis of Compound B8-5

Compound B8-3 (420 mg, 2.2 mmol) and Compound B8-4 (444 mg, 2.2 mmol) were added to dimethyl sulfoxide (5 mL). Potassium tert-butoxide (497 mg, 4.4 mmol) was added. Under nitrogen protection, methanesulfonato(2-di-tert-butylphosphino-2',4',6'-triisopropyl-1,1'-biphenyl)(2'-amino-1,1'-bi phenyl-2-yl)palladium(II) (351 mg, 0.44 mmol) was added. The mixture was reacted at 110 °C overnight. The reaction mixture was quenched with saturated aqueous ammonium chloride and extracted with dichloromethane (20 mL × 3). The organic phases were combined, dried, and dried by rotary evaporation. The resulting material was purified by Flash silica gel column chromatography (0 to 10% methanol/dichloromethane) to obtain 300 mg of Compound B8-5 as an off-white solid (yield: 38.1%). LCMS: $[M+H]^+$ = 353.

Step D: Synthesis of Compound B8-6

Compound B8-5 (300 mg, 0.85 mmol) was dissolved in dichloromethane (10 mL). A 4 M hydrogen chloride solution in 1,4-dioxane (5 mL) was added. The mixture was stirred at room temperature for 1 hour. The reaction mixture was concentrated. The residue was dissolved in methanol (10 mL), and the pH was adjusted to basic using a hydroxide ion exchange resin. The mixture was filtered and concentrated to obtain 214 mg of Compound B8-6 as a brown oil (crude). LCMS: $[M+H]^+$ = 253.

Step E: Synthesis of Compound B8

Compound B8-6 (214 mg, 0.85 mmol) and Compound B8-7 (171 mg, 0.85 mmol) were added to dimethyl sulfoxide (5 mL). Potassium tert-butoxide (192.0 mg, 1.7 mmol) was added. Under nitrogen protection, methanesulfonato(2-di-tert-butylphosphino-2',4',6'-triisopropyl-1,1'-biphenyl)(2'-amino-1,1'-bi phenyl-2-yl)palladium(II) (135 mg, 0.17 mmol) was added. The mixture was reacted at 110 °C overnight. The reaction mixture was quenched with saturated aqueous ammonium chloride and extracted with dichloromethane (20 mL × 3). The organic phases were combined, dried, and dried by rotary evaporation. The resulting material was purified by Flash silica gel column chromatography (0 to 10% methanol/dichloromethane) and further purified by preparative TLC plate (developing solvent: dichloromethane/methanol = 10:1) to obtain 16.1 mg of Compound B8 as a white solid (two-step yield: 4.5%). LCMS: $[M+H]^+$ = 423.

$^1$H NMR (400 MHz, DMSO-$d_6$) δ 7.91 (d, J = 2.7 Hz, 1H), 7.75 (s, 1H), 7.60 (ddd, J = 6.8, 2.1, 0.7 Hz, 1H), 7.48 (ddd, J = 9.0, 6.6, 2.1 Hz, 1H), 7.39 (dd, J = 8.9, 2.7 Hz, 1H), 6.90 (t, J = 7.0 Hz, 2H), 6.51 (d, J = 8.9 Hz, 1H), 6.44 (dt, J = 9.1, 1.0 Hz, 1H), 6.27 (td, J = 6.6, 1.4 Hz, 1H), 4.31 - 4.16 (m, 4H), 3.31 - 3.25 (m, 2H), 2.15 - 2.03 (m, 2H), 1.91 - 1.78 (m, 2H), 1.47 (dp, J = 8.6, 5.8, 3.9 Hz, 2H).

Example B9 (Compound B9)

Synthesis of 6'-(((1S,3S)-3-((6,6-difluoro-6,7-dihydro-[1,4]dioxino[2,3-d]pyrimidin-2-yl)amino)cyclopentyl)a mino)-2H-[1,3'-bipyridin]-2-one

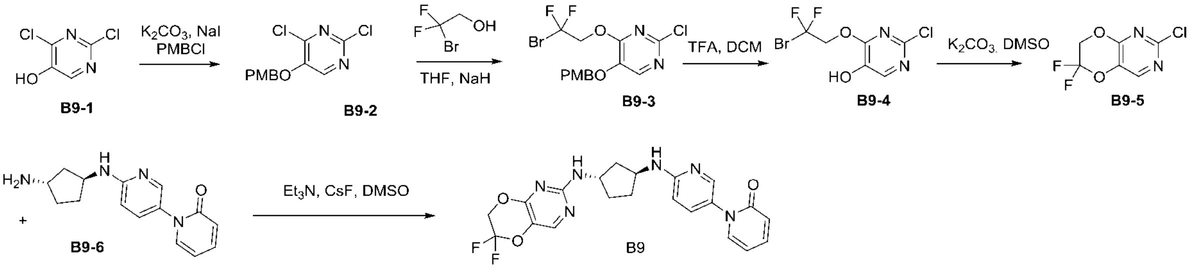

Step 1: Synthesis of 2,4-dichloro-5-((4-methoxybenzyl)oxy)pyrimidine

At room temperature, Compound B9-1 (2.0 g, 12.2 mmol), potassium carbonate (3.4 g, 24.4 mmol, 2 eq), sodium iodide (1.8 g, 12.2 mmol, 1 eq), and acetonitrile (100 mL) were sequentially added to a 250 mL round-bottom flask. PMBCl (2.8 g, 18.3 mmol, 1.5 eq) was slowly added. The reaction mixture was stirred at room temperature overnight.

LC-MS indicated the disappearance of the starting material and formation of the target product. EA (100 mL) was added to dilute the mixture. The reaction mixture was then filtered and washed with EA. The organic phase was concentrated and purified by silica gel column chromatography (petroleum ether:ethyl acetate = 10:1) to obtain Compound B9-2 (3 g, 86.6% yield) as a pale yellow oily liquid.

Step 2: Synthesis of 4-(2-bromo-2,2-difluoroethoxy)-2-chloro-5-((4-methoxybenzyl)oxy)pyrimidine

At room temperature, a 250 mL round-bottom flask was purged with nitrogen. THF (100 mL) and sodium hydride (632 mg, 15.8 mmol, 60 wt%, 1.5 eq) were sequentially added. The mixture was cooled to 0 °C. 2-bromo-2,2-difluoroethanol (2.5 g, 15.8 mmol, 1.5 eq) was slowly added dropwise. After stirring at 0 °C for 30 minutes, Compound B9-2 (3 g, 10.5 mmol, 1 eq) was slowly added. After the addition, the reaction mixture was warmed to room temperature and stirred for three hours.

LCMS detected the formation of the target product. 30 mL saturated aqueous ammonium chloride was slowly added to quench the reaction. The mixture was extracted with EA, dried over anhydrous sodium sulfate, filtered, concentrated, and purified by silica gel column chromatography (petroleum ether:ethyl acetate = 10:1) to obtain Compound B9-3 (4.0 g, 94% yield) as a pale yellow oily liquid.

Step 3: Synthesis of 4-(2-bromo-2,2-difluoroethoxy)-2-chloropyrimidin-5-ol

At room temperature, Compound B9-3 (4.0 g, 9.8 mmol, 1.00 eq), DCM (50 mL), and TFA (2.2 g, 19.6 mmol, 2 eq) were added to a 250 mL round-bottom flask. The mixture was stirred at room temperature overnight. LCMS detected the formation of the target product. The reaction mixture was directly dried by rotary evaporation. The resultant was purified by silica gel column chromatography (petroleum ether:ethyl acetate = 2:1) to obtain Compound B9-4 (0.6 g, 21% yield) as a white solid.

Step 4: Synthesis of 2-chloro-6,6-difluoro-6,7-dihydro-[1,4]dioxino[2,3-d]pyrimidine

At room temperature, Compound B9-4 (0.6 g, 2.1 mmol, 1.0 eq), dimethyl sulfoxide (10 mL), and potassium carbonate (0.4 g, 3.2 mmol, 1.5 eq) were sequentially added to a 100 mL round-bottom flask. The mixture was stirred at room temperature overnight.

LCMS detected the formation of the target product. 100 mL EA and 50 mL water were added to the reaction system. The organic and aqueous phases were separated. The aqueous phase was further extracted with EA twice. The organic phases were combined, washed with water and saturated brine, dried over anhydrous sodium sulfate, filtered, concentrated, and purified by silica gel column chromatography (petroleum ether:ethyl acetate = 10:1) to obtain Compound B9-5 (140 mg, 32% yield) as a pale yellow oily liquid.

Step 5: Synthesis of 6'-(((1S,3S)-3-((6,6-difluoro-6,7-dihydro-[1,4]dioxino[2,3-d]pyrimidin-2-yl)amino)cyclopentyl)a mino)-2H-[1,3'-bipyridin]-2-one

At room temperature, Compound B9-5 (60 mg, 0.29 mmol, 1.0 eq), Compound B9-6 (94 mg, 0.35 mmol, 1.2 eq), cesium fluoride (220 mg, 1.45 mmol, 5.0 eq), DMSO (2 mL), and triethylamine (118 mg, 1.16 mmol, 4.0 eq) were sequentially added to a 25 mL round-bottom flask. The reaction mixture was stirred at 120 °C overnight.

LCMS detected the formation of the target product. The reaction mixture was filtered through diatomite, and the filtrate was concentrated under reduced pressure. The resultant was purified by C18 reverse-phase column chromatography (mobile phase: water (FA)-ACN; gradient: 0% to 80% B over 50 min) to obtain 6'-(((1S,3S)-3-((6,6-difluoro-6,7-dihydro-[1,4]dioxino[2,3-d]pyrimidin-2-yl)amino)cyclopentyl)a mino)-2H-[1,3'-bipyridin]-2-one (92 mg).

$^1$H NMR (400 MHz, DMSO-$d_6$) δ 8.17 (s, 1H), 7.91 (d, J = 2.7 Hz, 1H), 7.60 (dd, J = 6.8, 2.1 Hz, 1H), 7.47 (ddd, J = 9.0, 6.6, 2.1 Hz, 1H), 7.39 (dd, J = 8.8, 2.7 Hz, 1H), 7.35 (d, J = 7.2 Hz, 1H), 6.90 (d, J = 6.9 Hz, 1H), 6.52 (d, J = 8.9 Hz, 1H), 6.44 (d, J = 9.2 Hz, 1H), 6.27 (td, J = 6.7, 1.4 Hz, 1H), 4.77 (t, J = 6.4 Hz, 2H), 4.28 (dt, J = 17.7, 7.7 Hz, 2H), 2.20 - 2.07 (m, 2H), 1.95 - 1.78 (m, 2H), 1.49 (td, J = 13.6, 7.5 Hz, 2H).

Examples B10 to B11 (Compounds B10 and B11)

Referring to the preparation methods B1 and B2 and the preparation methods of the Examples of the present application, the following Compounds were prepared:

| No. | Compound | No. | Compound |
|---|---|---|---|
| B10 | 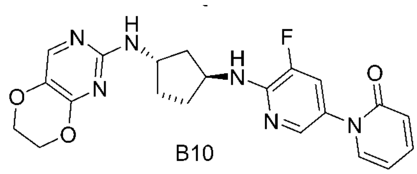 B10 $^1$H NMR (400 MHz, DMSO-$d_6$) δ 7.85 - 7.81 (m, 2H), 7.66 - 7.62 (dd, $J$ = 6.9, 2.1 Hz, 1H), 7.56 - 7.46 (m, 2H), 6.91 - 6.86 (d, $J$ = 7.1 Hz, 1H), 6.77 - 6.73 (d, $J$ = 7.2 Hz, 1H), 6.49 - 6.44 (d, $J$ = 9.2 Hz, 1H), 6.31 - 6.26 (td, $J$ = 6.7, 1.4 Hz, 1H), 4.52 - 4.44 (q, $J$ = 7.0 Hz, 1H), 4.41 - 4.36 (m, 2H), 4.27 - 4.17 (q, $J$ = 6.8 Hz, 1H), 4.16 - 4.09 (m, 2H), 2.16 - 2.02 (m, 2H), 1.93 - 1.84 (td, $J$ = 6.9, 4.4 Hz, 2H), 1.64 - 1.43 (m, 2H). | B11 | 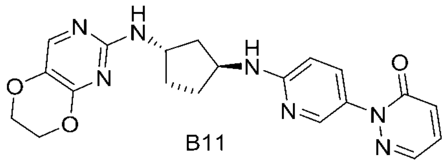 B11 $^1$H NMR (400 MHz, DMSO-$d_6$) δ 8.10 - 8.08 (m, 1H), 8.02 - 7.99 (dd, $J$ = 3.8, 1.6 Hz, 1H), 7.84 - 7.81 (s, 1H), 7.53 - 7.43 (m, 2H), 7.04 - 7.00 (dd, $J$ = 9.5, 1.6 Hz, 1H), 6.91 - 6.87 (d, $J$ = 6.9 Hz, 1H), 6.77 - 6.73 (d, $J$ = 7.2 Hz, 1H), 6.54 - 6.49 (dd, $J$ = 8.9, 0.7 Hz, 1H), 4.41 - 4.37 (m, 2H), 4.31 - 4.19 (m, 2H), 4.16 - 4.12 (m, 2H), 2.17 - 2.02 (m, 2H), 1.91 - 1.78 (m, 2H), 1.52 - 1.42 (m, 2H). |

Example B12 (Compound B12)

Synthesis of 6'-(((1S,3S)-3-(quinazolin-2-ylamino)cyclopentyl)amino)-2H-[1,3'-bipyridin]-2-one

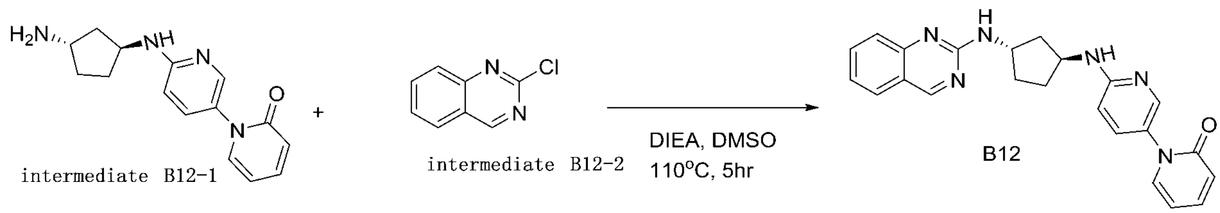


intermediate B12-1 + intermediate B12-2 → B12

Intermediate Compound B12-2 (50 mg, 0.3 mmol) was added to 5 mL dimethyl sulfoxide. Intermediate Compound B12-1 (100 mg, 0.37 mmol) and N,N-diisopropylethylamine (0.2 mL, 1.16 mmol) were added. Under nitrogen protection, the mixture was reacted at 110 °C for 5 hours. The reaction mixture was poured into a saturated aqueous ammonium chloride solution and extracted with ethyl acetate (10 mL × 2). The organic phase was dried over anhydrous sodium sulfate, filtered, and concentrated. The resultant was purified by reverse-phase column chromatography to obtain 51.3 mg of 6'-(((1S,3S)-3-(quinazolin-2-ylamino)cyclopentyl)amino)-2H-[1,3'-bipyridin]-2-one.

LCMS: Rt = 0.879 min, $[M+H]^+$ = 399. $^1$H NMR (400 MHz, DMSO- $d_6$) δ 9.10 (s, 1H), 7.92 (d, J = 2.6 Hz, 1H), 7.78 (d, J = 7.9 Hz, 1H), 7.67 (dd, J = 8.6, 6.8 Hz, 1H), 7.61 (dd, J = 6.8, 1.9 Hz, 1H), 7.55 - 7.38 (m, 4H), 7.21 (t, J = 7.5 Hz, 1H), 6.98 (d, J = 6.9 Hz, 1H), 6.54 (d, J = 8.9 Hz, 1H), 6.44 (d, J = 9.2 Hz, 1H), 6.29 - 6.24 (m, 1H), 4.51 (d, J = 7.3 Hz, 1H), 4.34 (d, J = 5.6 Hz, 1H), 2.17 (d, J = 10.1 Hz, 2H), 2.02 - 1.91 (m, 2H), 1.62 - 1.50 (m, 2H).

Example B13 (Compound B13)

Synthesis of 6'-(((1S,3S)-3-(pyrido[2,3-d]pyrimidin-2-ylamino)cyclopentyl)amino)-2H-[1,3'-bipyridin]-2-one

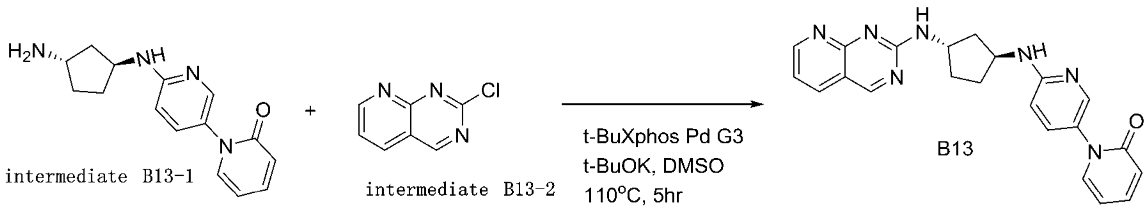


Intermediate Compound B13-2 (50 mg, 0.3 mmol) was added to 5 mL dimethyl sulfoxide. Intermediate Compound B13-1 (100 mg, 0.37 mmol), potassium tert-butoxide (67 mg, 0.6 mmol), and methanesulfonato(2-di-tert-butylphosphino-2',4',6'-triisopropyl-1,1'-biphenyl)(2'-amino-1,1'-bi phenyl-2-yl)palladium(II) (48 mg, 0.06 mmol) were added. Under nitrogen protection, the mixture was reacted at 110 °C for 5 hours.

The reaction mixture was poured into a saturated aqueous ammonium chloride solution and extracted with ethyl acetate (10 mL × 2). The organic phase was dried over anhydrous sodium sulfate, filtered, and concentrated.

The resultant was purified by reverse-phase column chromatography to obtain 60.2 mg of 6'-(((1S,3S)-3-(pyrido [2,3-d]pyrimidin-2-ylamino)cyclopentyl)amino)-2H-[1,3'-bipyridin]-2-one. LCMS: Rt = 0.725 min, $[M+H]^+$ = 400.

$^1$H NMR (400 MHz, DMSO-$d_6$) δ 9.16 (s, 1H), 8.84 (s, 1H), 8.21 (dd, J = 7.8, 2.1 Hz, 1H), 8.01 - 7.89 (m, 2H), 7.66 - 7.58 (m, 1H), 7.48 (ddd, J = 9.0, 6.6, 2.1 Hz, 1H), 7.40 (dd, J = 8.9, 2.7 Hz, 1H), 7.22 (dd, J = 7.9, 4.5 Hz, 1H), 7.01 (d, J = 6.9 Hz, 1H), 6.55 (d, J = 8.9 Hz, 1H), 6.44 (d, J = 9.1 Hz, 1H), 6.27 (td, J = 6.7, 1.3 Hz, 1H), 4.52 (d, J = 6.6 Hz, 1H), 4.35 (q, J = 6.6 Hz, 1H), 2.26 - 2.13 (m, 2H), 1.98 (dt, J = 13.0, 6.7 Hz, 2H), 1.67 - 1.51 (m, 2H).

Example B14 (Compound B14)

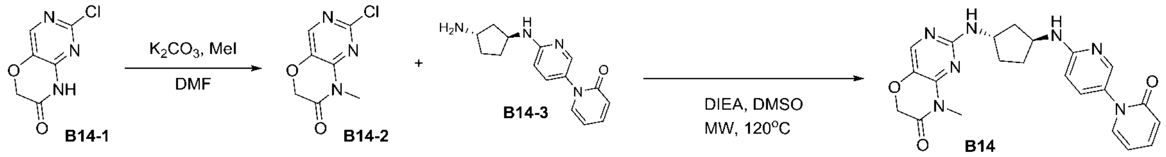


Step A: Synthesis of Compound B14-2

Compound B14-1 (400 mg, 2.1 mmol) was dissolved in N,N-dimethylformamide (5 mL). Potassium carbonate (870 mg, 6.3 mmol) and iodomethane (300 mg, 2.1 mmol) were added. The reaction was stirred at room temperature for 16 hours. The reaction mixture was slowly added to water and extracted with ethyl acetate (15 mL × 3). The organic phases were combined and dried by rotary evaporation. The resulting crude product was purified by silica gel column chromatography (dichloromethane:methanol = 99:1) to obtain 250 mg of Compound B14-2 as a white solid (yield: 59%). LCMS: $[M+H]^+$ = 200.

Step B: Synthesis of Compound B14

Compound B14-2 (200 mg, 1.0 mmol) and Compound B14-3 (270 mg, 1.0 mmol) were dissolved in dimethyl sulfoxide (5 mL). N,N-Diisopropylethylamine (387 mg, 3.0 mmol) was added. The atmosphere was purged with nitrogen, and the mixture was reacted at 120 °C under microwave irradiation for 1 hour. The reaction mixture was quenched with saturated aqueous ammonium chloride and extracted with ethyl acetate (50 mL × 3). The organic phases were combined, dried and concentrated. The resultant was purified by silica gel column chromatography (dichloromethane:methanol = 95:5) to obtain 23 mg of Compound B14 as a pale yellow solid (yield: 5.3%). LCMS: $[M+H]^+$ = 434.

$^1$H NMR (400 MHz, DMSO-$d_6$) δ 7.96 (d, J = 3.0 Hz, 2H), 7.64 (dd, J = 7.0, 2.1 Hz, 1H), 7.52 (ddd, J = 9.0, 6.6, 2.1 Hz, 1H), 7.43 (dd, J = 8.9, 2.7 Hz, 1H), 7.10 (d, J = 7.0 Hz, 1H), 6.96 (d, J = 6.8 Hz, 1H), 6.56 (d, J = 8.9 Hz, 1H), 6.48 (dt, J = 9.1, 1.1 Hz, 1H), 6.31 (td, J = 6.7, 1.4 Hz, 1H), 4.68 (s, 2H), 4.33 (p, J = 6.5 Hz, 2H), 3.29 (s, 3H), 2.16 (h, J = 6.4, 5.6 Hz, 2H), 1.93 (dq, J = 22.2, 6.4 Hz, 2H), 1.54 (tt, J = 12.7, 6.5 Hz, 2H).

Example B15 (Compound B15)

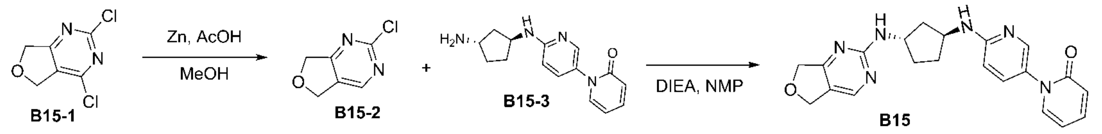

B15-1 B15-2 B15-3 B15

Step A: Synthesis of Compound B15-2

Compound B15-1 (1 g, 5.2 mmol) was added to methanol (10 mL). Glacial acetic acid (0.9 mL, 15.6 mmol) was added. Zinc powder (680 mg, 10.4 mmol) was added in batches under a water bath. The reaction was stirred at room temperature for 4 hours. The reaction mixture was filtered, and the filtrate was concentrated. The resultant was purified by Flash silica gel column chromatography (5 to 30% ethyl acetate/petroleum ether) to obtain 200 mg of Compound B15-2 as a pale yellow solid (yield: 24.5%). LCMS: $[M+H]^+$ = 157.

Step B: Synthesis of Compound B15

Compound B15-2 (150 mg, 0.95 mmol) and Compound B15-3 (256.8 mg, 0.95 mmol) were added to N-methylpyrrolidone (4 mL). N,N-Diisopropylethylamine (0.5 mL, 2.97 mmol) was added. After the atmosphere was purged with nitrogen, the mixture was reacted at 150 °C under microwave irradiation for 30 minutes. The reaction mixture was quenched with saturated aqueous ammonium chloride and extracted with ethyl acetate (20 mL × 3). The organic phases were combined, dried and concentrated. The resultant was purified by preparative C18 reverse-phase column chromatography to obtain 90 mg of Compound B15 as a pale yellow solid (yield: 24.2%). LCMS: $[M+H]^+$ = 391.

$^1$H NMR (400 MHz, DMSO-$d_6$) δ 8.23 (s, 1H), 7.92 (d, J = 2.7 Hz, 1H), 7.60 (dd, J = 6.8, 2.1 Hz, 1H), 7.48 (ddd, J = 9.0, 6.6, 2.1 Hz, 1H), 7.39 (dd, J = 8.9, 2.7 Hz, 1H), 7.33 (d, J = 7.3 Hz, 1H), 6.93 (d, J = 6.9 Hz, 1H), 6.52 (d, J = 8.9 Hz, 1H), 6.49 - 6.39 (m, 1H), 6.27 (td, J = 6.7, 1.3 Hz, 1H), 4.91 (d, J = 2.0 Hz, 2H), 4.71 (t, J = 1.8 Hz, 2H), 4.34 (dq, J = 24.4, 6.9 Hz, 2H), 2.18 - 2.06 (m, 2H), 1.94 - 1.81 (m, 2H), 1.50 (ddd, J = 16.4, 12.8, 7.0 Hz, 2H).

Example B16 (Compound B16)

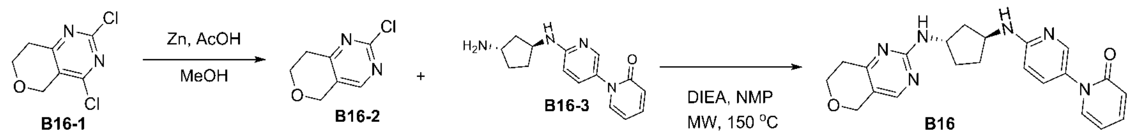

B16-1 B16-2 B16-3 B16

Step A: Synthesis of Compound B16-2

Compound B16-1 (1 g, 4.9 mmol) was dissolved in methanol (10 mL). Acetic acid (0.84 mL, 14.7 mmol) was added. Zinc powder (637 mg, 9.8 mmol) was added in batches. The reaction was stirred at room temperature for 2 hours. The reaction mixture was filtered, and the filtrate was concentrated. The resultant was purified by Flash silica gel column chromatography (5 to 30% ethyl acetate/petroleum ether) to obtain 350 mg of Compound B16-2 as a yellow oil product (yield: 39.4%). LCMS: $[M+H]^+$ = 171.

Step B: Synthesis of Compound B16

Compound B16-2 (170 mg, 1 mmol) and Compound B16-3 (270 mg, 1 mmol) were dissolved in N-methylpyrrolidone (5 mL). N,N-Diisopropylethylamine (0.35 mL, 2 mmol) was added. The atmosphere was purged with nitrogen, and the mixture was reacted at 150 °C under microwave irradiation for 0.5 hours. The reaction mixture was quenched with saturated aqueous ammonium chloride and extracted with ethyl acetate (20 mL × 3). The organic phases were combined, dried and concentrated.

The resultant was purified by Flash silica gel column chromatography (0% to 10% methanol/dichloromethane) to obtain 56.8 mg of Compound B16 as a yellow solid (yield: 14.1%).

LCMS: $[M+H]^+$ = 405. $^1$H NMR (400 MHz, DMSO-$d_6$) δ 8.34 (s, 2H), 8.26 (s, 2H), 7.73 (d, J = 7.6 Hz, 1H), 7.68 (dd, J = 6.8, 2.0 Hz, 1H), 7.56 (d, J = 7.6 Hz, 1H), 7.51 (ddd, J = 8.8, 6.6, 2.0 Hz, 1H), 7.06 (s, 1H), 6.51 - 6.44 (m, 1H), 6.32 (td, J = 6.6, 1.2 Hz, 1H), 5.96 (s, 2H), 5.14 (q, J = 6.4, 5.8 Hz, 1H), 5.10 - 5.03 (m, 1H), 2.14 (t, J = 6.0 Hz, 2H).

Example B17 (Compound B17)

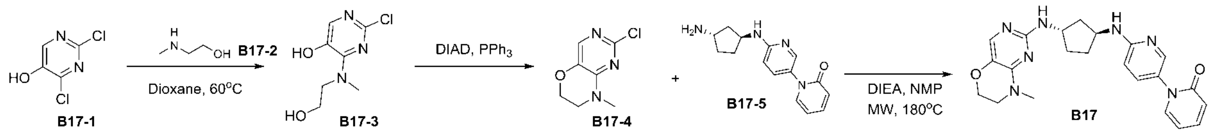


Step A: Synthesis of Compound B17-3

Compound B17-1 (1 g, 6.1 mmol) was dissolved in 10 mL 1,4-dioxane. Compound B17-2 (457 mg, 12.2 mmol) was added. The mixture was reacted at 60 °C for 16 hours. The resultant was dried by rotary evaporation. The resultant was purified by Flash silica gel column chromatography (0 to 50% ethyl acetate/petroleum ether) to obtain 900 mg of Compound B17-3 as a white solid (yield: 75%). LCMS: $[M+H]^+$ = 204.

Step B: Synthesis of Compound B17-4

Compound B17-3 (900 mg, 5.4 mmol) was dissolved in N,N-dimethylformamide (10 mL). Triphenylphosphine (2.1 g, 8.1 mmol) and diisopropyl azodicarboxylate (1.6 mL, 8.1 mmol) were added. Under nitrogen protection, the reaction was stirred at room temperature for 2 hours. The reaction mixture was added to water (20 mL) and extracted with ethyl acetate (20 mL × 3). The organic phases were combined and dried by rotary evaporation. The resultant was purified by Flash silica gel column chromatography (0 to 30% ethyl acetate/petroleum ether) to obtain 170 mg of Compound B17-4 as a white solid (yield: 17%). LCMS: $[M+H]^+$ = 186.

Step C: Synthesis of Compound B17

Compound B17-4 (160 mg, 0.86 mmol) and Compound B17-5 (232 mg, 0.86 mmol) were dissolved in N-methylpyrrolidone (1 mL). N,N-Diisopropylethylamine (0.3 mL, 1.73 mmol) was added. The atmosphere was purged with nitrogen, and the mixture was reacted at 180 °C under microwave irradiation for 1 hour. The reaction mixture was quenched with saturated aqueous ammonium chloride and extracted with ethyl acetate (20 mL × 3). The organic phases were combined, dried and concentrated. The resultant was purified by C18 reverse-phase column chromatography (0 to 30% acetonitrile/water) to obtain 5.8 mg of Compound B17 as a white solid (yield: 1.6%). LCMS: $[M+H]^+$ = 420.

$^1$H NMR (400 MHz, DMSO-$d_6$) δ 7.92 (d, J = 2.7 Hz, 1H), 7.60 (dd, J = 7.0, 2.1 Hz, 1H), 7.52 - 7.33 (m, 3H), 7.00 (d, J = 6.5 Hz, 1H), 6.54 (d, J = 8.9 Hz, 1H), 6.44 (d, J = 9.1 Hz, 1H), 6.27 (td, J = 6.8, 1.4 Hz, 1H), 4.29 (d, J = 7.2 Hz, 2H), 4.10 (d, J = 5.0 Hz, 2H), 3.54 (s, 2H), 3.11 (s, 3H), 2.13 (t, J = 5.8 Hz, 2H), 1.90 (t, J = 6.8 Hz, 2H), 1.51 (s, 2H).

Example B18 (Compound B18)

Synthesis of 6'-(((1S,3S)-3-((5-oxido-6,7-dihydrothieno[3,2-d]pyrimidin-2-yl)amino)cyclopentyl)amino)-2H-[1,3'-bipyridin]-2-one

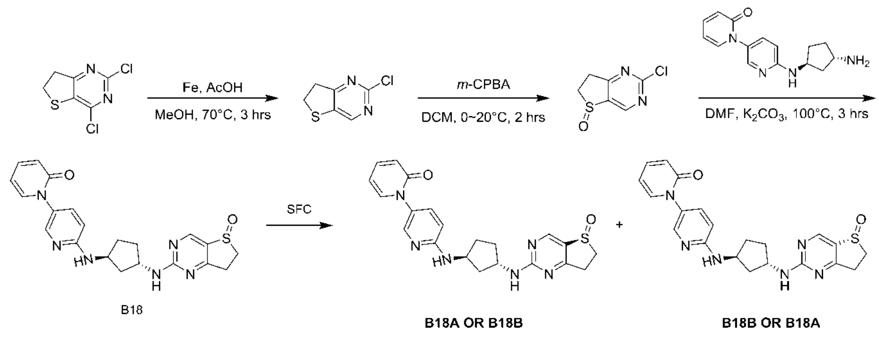

Step 1: Synthesis of 2-chloro-6,7-dihydrothieno[3,2-d]pyrimidine

2,4-dichloro-6,7-dihydrothieno[3,2-d]pyrimidine (1.00 g, 4.83 mmol, 1 eq) and acetic acid (1.74 g, 29.0 mmol, 6 eq) were dissolved in methanol (20 mL). Iron powder (1.08 g, 19.3 mmol, 4 eq) was then added to the reaction mixture in batches. After the addition, the mixture was heated to 70 °C and reacted for 3 hours. TLC (petroleum ether/ethyl acetate = 3/1) indicated the completion of the reaction. The reaction mixture was filtered, and the filtrate was concentrated under reduced pressure to obtain a crude product. The crude product was purified by silica gel column chromatography to obtain 2-chloro-6,7-dihydrothieno[3,2-d]pyrimidine (570 mg, 3.30 mmol, 68.4% yield).

Step 2: Synthesis of 2-chloro-6,7-dihydrothieno[3,2-d]pyrimidine 5-oxide

At 0 °C, a solution of m-chloroperoxybenzoic acid (811 mg, 4.00 mmol, 85% purity, 1.15 eq) in dichloromethane (5 mL) was added to a solution of 2-chloro-6,7-dihydrothieno[3,2-d]pyrimidine (600 mg, 3.48 mmol, 1 eq) in dichloromethane (10 mL). After the addition, the mixture was stirred at 20 °C for 2 hours. LCMS detected the MS value of the product (RT = 0.328 min). The reaction mixture was quenched with a 10% $Na_2SO_3$ solution and extracted with dichloromethane (20 mL × 2). The organic phases were combined, washed with saturated brine (50 mL × 2), dried over anhydrous sodium sulfate, filtered, and concentrated to obtain a crude product. The crude product was purified by silica gel column chromatography to obtain 2-chloro-6,7-dihydrothieno[3,2-d]pyrimidine 5-oxide (450 mg, 2.39 mmol, 68.6% yield).

Step 3: Synthesis of 6'-(((1S,3S)-3-((5-oxido-6,7-dihydrothieno[3,2-d]pyrimidin-2-yl)amino)cyclopentyl)amino)-2H-[1,3'-bipyridin]-2-one

6'-(((1S,3S)-3-aminocyclopentyl)amino)-2H-[1,3'-bipyridin]-2-one (516 mg, 1.91 mmol, 1 eq) and 2-chloro-6,7-dihydrothieno[3,2-d]pyrimidine 5-oxide (360 mg, 1.91 mmol, 1 eq) were dissolved in N,N-dimethylformamide (5 mL). Potassium carbonate (791 mg, 5.73 mmol, 3 eq) was then added. After the addition, the reaction mixture was stirred at 100 °C for 3 hours. LCMS detected the MS value of the product (RT = 0.412 min). The reaction mixture was filtered, and the filtrate was concentrated under reduced pressure to obtain a crude product. The crude product was purified by reverse-phase preparative chromatography (Column: Waters Xbridge 150*25mm* 5um; Mobile phase: [water (ammonium hydroxide v/v)-ACN]; Gradient: 0% to 22% B over 10 min) to obtain 6'-(((1S,3S)-3-((5-oxido-6,7-dihydrothieno[3,2-d]pyrimidin-2-yl)amino)cyclopentyl)amino)-2H-[1,3'-bipyridin]-2-one (100 mg, 232 μmol, 12.2% yield, 98.0% purity). LCMS: MS (ESI) m/z = 423.1 $[M+1]^+$.

Step 4: SFC Separation

6'-(((1S,3S)-3-((5-oxido-6,7-dihydrothieno[3,2-d]pyrimidin-2-yl)amino)cyclopentyl)amino) -2H-[1,3'-bipyridin]-2-one (100 mg, 232 μmol, 1 eq) was subjected to SFC separation (Column: DAICEL CHIRALPAK AD (250 mm*30 mm, 10 um); Mobile phase: [CO2-ACN/i-PrOH (0.1% NH3H2O)]; B%: 60%, isocratic elution mode). The two separated peaks were further purified by reverse-phase column chromatography (Column: Waters Xbridge 150*25mm* 5um; Mobile phase: [water (ammonium hydroxide v/v)-ACN]; Gradient: 0% to 25% B over 10 min) to obtain B18A (peak 1, 10.24 mg, 25.7 μmol, 22.2% yield, 98.7% purity) and B18B (peak 2, 11.06 mg, 28.2 μmol, 24.3% yield, 99.1% purity).

B18A: LCMS MS (ESI) m/z = 423.1 $[M+1]^+$. $^1H$ NMR: (400 MHz, DMSO-$d_6$):
δ 8.72 (s, 1H), 7.94 (d, J = 2.6 Hz, 1H), 7.88 - 7.80 (m, 1H), 7.54 (dd, J = 1.9, 6.9 Hz, 1H), 7.45 (ddd, J = 2.1, 6.7, 9.1 Hz, 1H), 7.39 (dd, J = 2.8, 8.9 Hz, 1H), 6.67 (br d, J = 6.5 Hz, 1H), 6.55 (d, J = 8.9 Hz, 1H), 6.43 (d, J = 9.4 Hz, 1H), 6.25 (dt, J = 1.3, 6.7 Hz, 1H), 4.55 - 4.45 (m, 1H), 4.42 - 4.31 (m, 1H), 3.61 - 3.47 (m, 1H), 3.38 (td, J = 8.1, 13.6 Hz, 1H), 3.12 (br s, 1H), 2.98 (ddd, J = 2.7, 7.6, 13.6 Hz, 1H), 2.25 - 2.12 (m, 2H), 2.07 - 1.88 (m, 2H), 1.68 - 1.47 (m, 2H).

B18B: LCMS m/z = 423.1 $[M+1]^+$. $^1H$ NMR: (400 MHz, DMSO-$d_6$):
δ 8.72 (s, 1H), 7.94 (d, J = 2.6 Hz, 1H), 7.84 (br d, J = 6.5 Hz, 1H), 7.54 (dd, J = 1.9, 6.8 Hz, 1H), 7.45 (ddd, J = 2.1, 6.7, 9.1 Hz, 1H), 7.39 (dd, J = 2.8, 8.9 Hz, 1H), 6.67 (br d, J = 6.9 Hz, 1H), 6.55 (d, J = 8.9 Hz, 1H), 6.43 (d, J = 9.3 Hz, 1H), 6.25 (dt, J = 1.3, 6.7 Hz, 1H), 4.55 - 4.44 (m, 1H), 4.36 (sext, J = 6.5 Hz, 1H), 3.60 - 3.49 (m, 1H), 3.38 (td, J = 8.2, 13.6 Hz, 1H), 3.12 (br d, J = 2.4 Hz, 1H), 2.98 (ddd, J = 2.6, 7.6, 13.6 Hz, 1H), 2.27 - 2.11 (m, 2H), 2.07 - 1.87 (m, 2H), 1.66 - 1.49 (m, 2H).

Example B19 (Compound B19)

Synthesis of 6'-(((1S,3S)-3-((5-oxido-7,8-dihydro-6H-thiopyrano[3,2-d]pyrimidin-2-yl)amino)cyclopentyl)amino)-2H-[1,3'-bipyridin]-2-one

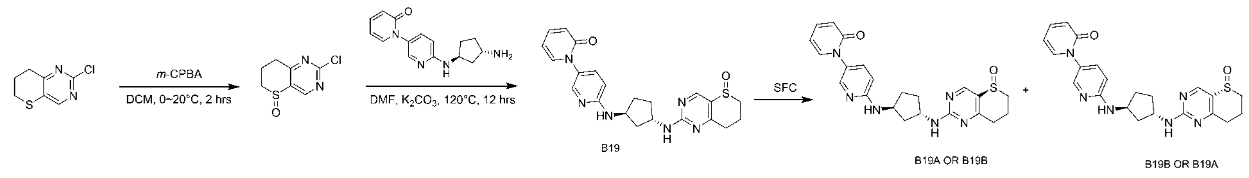


B19 B19A OR B19B + B19B OR B19A

Step 1: Synthesis of 2-chloro-7,8-dihydro-6H-thiopyrano[3,2-d]pyrimidine 5-oxide

At 0 °C, a solution of m-chloroperoxybenzoic acid (750 mg, 3.70 mmol, 85% purity, 1.15 eq) in dichloromethane (8 mL) was added to a solution of 2-chloro-7,8-dihydro-6H-thiopyrano[3,2-d]pyrimidine (600 mg, 3.21 mmol, 1 eq) in dichloromethane (10 mL). After the addition, the mixture was stirred at 20 °C for 2 hours. LCMS detected the MS value of the product (RT = 0.255 min). The reaction mixture was quenched with a 10% $Na_2SO_3$ solution and extracted with dichloromethane (20 mL × 2). The organic phases were combined, washed with saturated brine (50 mL × 2), dried over anhydrous sodium sulfate, filtered, and concentrated to obtain a crude product. The crude product was purified by silica gel column chromatography to obtain 2-chloro-7,8-dihydro-6H-thiopyrano[3,2-d]pyrimidine 5-oxide (350 mg, 1.73 mmol, 53.7% yield, 100% purity).

Step 2: Synthesis of 6'-(((1S,3S)-3-((5-oxido-7,8-dihydro-6H-thiopyrano[3,2-d]pyrimidin-2-yl)amino)cyclopentyl)am ino)-2H-[1,3'-bipyridin]-2-one

6'-(((1S,3S)-3-Aminocyclopentyl)amino)-2H-[1,3'-bipyridin]-2-one (400 mg, 1.48 mmol, 1 eq) and 2-chloro-7,8-dihydro-6H-thiopyrano[3,2-d]pyrimidine 5-oxide (300 mg, 1.48 mmol, 1 eq) were dissolved in N,N-dimethylformamide (5 mL). Potassium carbonate (614 mg, 4.44 mmol, 3 eq) was then added. After the addition, the reaction mixture was stirred at 120 °C for 12 hours. LCMS detected the MS value of the product (RT = 0.400 min). The reaction mixture was filtered, and the filtrate was concentrated under reduced pressure to obtain a crude product. The crude product was purified by reverse-phase preparative chromatography (Column: Waters Xbridge 150*25mm* 5um; Mobile phase: [water (ammonium hydroxide v/v)-ACN]; Gradient: 0% to 22% B over 10 min) to obtain 6'-(((1S,3S)-3-((5-oxido-7,8-dihydro-6H-thiopyrano[3,2-d]pyrimidin-2-yl)amino)cyclopentyl)am ino)-2H-[1,3'-bipyridin]-2-one (330 mg, 756 μmol, 51.1% yield, 100% purity).

Step 3: SFC Separation

6'-(((1S,3S)-3-((5-oxido-7,8-dihydro-6H-thiopyrano[3,2-d]pyrimidin-2-yl)amino)cyclopent yl)amino)-2H-[1,3'-bipyridin]-2-one (330 mg, 756 μmol, 1 eq) was subjected to SFC separation (Column: DAICEL CHIRALCEL OD (250mm*50mm, 10um); Mobile phase: [CO2-ACN/EtOH (0.1% NH3H2O)]; B%: 48%, isocratic elution mode) to obtain B19A (peak 1, 92.95 mg, 213 μmol, 56.4% yield, 100% purity) and B19B (peak 2, 126.02 mg, 287 μmol, 76.0% yield, 98.8% purity).

B19A: LCMS (ESI) m/z = 437.1 $[M+1]^+$.

$^1$H NMR (400 MHz, DMSO-$d_6$): δ 8.54 - 8.41 (m, 1H), 8.02 - 7.86 (m, 2H), 7.59 (dd, J = 1.6, 6.8 Hz, 1H), 7.47 (ddd, J = 2.1, 6.8, 9.1 Hz, 1H), 7.39 (dd, J = 2.8, 8.9 Hz, 1H), 6.92 (d, J = 6.9 Hz, 1H), 6.52 (d, J = 8.9 Hz, 1H), 6.44 (d, J = 9.3 Hz, 1H), 6.27 (dt, J = 1.3, 6.7 Hz, 1H), 4.48 - 4.23 (m, 2H), 3.13 - 3.02 (m, 1H), 2.96 - 2.83 (m, 1H), 2.82 - 2.70 (m, 2H), 2.20 - 1.77 (m, 6H), 1.64 - 1.42 (m, 2H).

B19B: LCMS (ESI) m/z = 437.1 $[M+1]^+$.

$^1$H NMR (400 MHz, DMSO-$d_6$): δ 8.55 - 8.40 (m, 1H), 8.03 - 7.86 (m, 2H), 7.59 (dd, J = 1.7, 6.8 Hz, 1H), 7.47 (ddd, J = 2.0, 6.7, 9.0 Hz, 1H), 7.39 (dd, J = 2.6, 8.9 Hz, 1H), 6.93 (d, J = 6.9 Hz, 1H), 6.52 (d, J = 8.9 Hz, 1H), 6.44 (d, J = 9.1 Hz, 1H), 6.27 (dt, J = 1.2, 6.7 Hz, 1H), 4.47 - 4.26 (m, 2H), 3.08 (br dd, J = 3.6, 13.2 Hz, 1H), 2.89 (dt, J = 1.9, 13.3 Hz, 1H), 2.82 - 2.64 (m, 2H), 2.21 - 1.78 (m, 6H), 1.60 - 1.44 (m, 2H).

Example B20 (Compound B20)

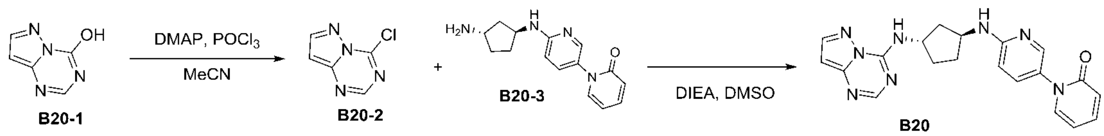


B20-1 B20-2 + B20-3 B20

Step A: Synthesis of the Target Compound

Compound B20-1 (272 mg, 2 mmol) and benzotriazole (600 mg, 5 mmol) were dissolved in 3 mL acetonitrile. Triethylamine (0.84 mL, 6 mmol) was added, followed by dropwise addition of phosphorus oxychloride (0.28 mL, 3 mmol). Under nitrogen protection, the mixture was heated to 80 °C and reacted for 6 hours. The reaction mixture was dried by rotary evaporation. The residue was dissolved in 5 mL dimethyl sulfoxide. N,N-Diisopropylethylamine (780 mg, 6 mmol) and Compound B20-3 (270 mg, 1 mmol) were added, and the mixture was reacted at room temperature overnight. The reaction mixture was added to 20 mL saturated aqueous ammonium chloride solution, extracted with ethyl acetate (10 mL × 3), dried over anhydrous sodium sulfate, filtered, and concentrated. The resultant was purified by silica gel column chromatography (eluent: 0 to 10% methanol/dichloromethane) to obtain 55 mg of the target compound as an off-white solid (yield: 14%).

LCMS: $[M+H]^+$ = 389. $^1$H NMR (400 MHz, DMSO-$d_6$) δ 8.96 (d, J = 8.0 Hz, 1H), 8.20 - 8.09 (m, 2H), 7.93 (d, J = 2.7 Hz, 1H), 7.61 (dd, J = 6.9, 2.0 Hz, 1H), 7.48 (ddd, J = 9.0, 6.6, 2.1 Hz, 1H), 7.41 (dd, J = 8.9, 2.7 Hz, 1H), 7.00 (d, J = 6.8 Hz, 1H), 6.55 (d, J = 8.9 Hz, 1H), 6.49 - 6.40 (m, 2H), 6.27 (td, J = 6.7, 1.4 Hz, 1H), 4.76 (h, J = 7.6 Hz, 1H), 4.39 (q, J = 6.4 Hz, 1H), 2.17 (ddd, J = 13.2, 10.9, 6.5 Hz, 3H), 1.95 (ddd, J = 13.1, 8.1, 5.0 Hz, 1H), 1.85 - 1.76 (m, 1H), 1.55 (dt, J = 15.6, 7.6 Hz, 1H).

Example B21 (Compound B21)

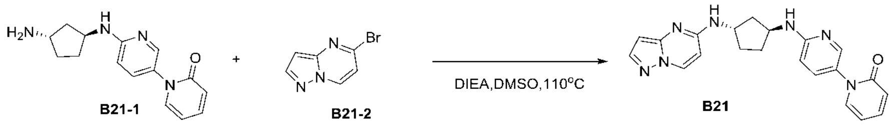


B21-1 + B21-2 → B21

Step A: Synthesis of the Target Compound

Compound B21-1 (100 mg, 0.37 mmol) was dissolved in 3 mL dimethyl sulfoxide. Compound B21-2 (72.9 mg, 0.37 mmol) and N,N-diisopropylethylamine (142 mg, 1.1 mmol) were added. The mixture was reacted at 120 °C for 2 hours. The reaction mixture was added to 20 mL saturated aqueous ammonium chloride solution, extracted with ethyl acetate (10 mL × 3), dried over anhydrous sodium sulfate, filtered, and concentrated. The resultant was purified by preparative silicone plate (developing agent: dichloromethane/methanol = 10/1) to obtain 12.6 mg of the target compound as a white solid (yield: 8.8%). LCMS: $[M+H]^+$ = 388.

$^1$H NMR (400 MHz, DMSO-$d_6$) δ 8.48 (d, J = 7.6 Hz, 1H), 7.99 (d, J = 2.8 Hz, 1H), 7.81 (d, J = 2.0 Hz, 1H), 7.67 (dd, J = 6.8, 2.0 Hz, 1H), 7.62 - 7.50 (m, 2H), 7.47 (dd, J = 8.8, 2.8 Hz, 1H), 7.06 (d, J = 6.8 Hz, 1H), 6.60 (d, J = 8.8 Hz, 1H), 6.50 (d, J = 9.2 Hz, 1H), 6.40 - 6.23 (m, 2H), 6.01 (d, J = 2.0 Hz, 1H), 4.45 (dq, J = 28.4, 6.8 Hz, 2H), 2.34 - 2.17 (m, 1H), 1.97 (t, J = 6.8 Hz, 2H), 1.57 (dd, J = 7.2, 4.2 Hz, 1H).

Example B22 (Compound B22)

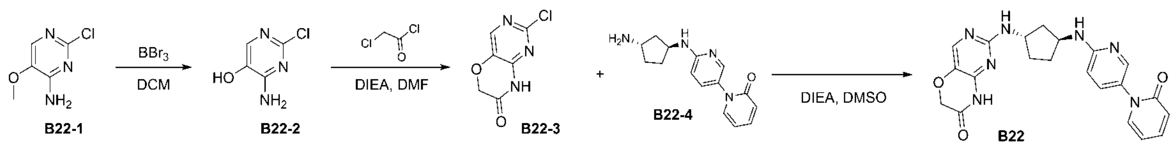


B22-1 → B22-2 → B22-3; + B22-4 → B22

Step A: Synthesis of Compound B22-2

Compound B22-1 (9 g, 56.6 mmol) was dissolved in dichloromethane (1 L). Boron tribromide (87 mL) was added. The mixture was stirred at room temperature for 16 hours. The reaction mixture was slowly added to ice water to quench the reaction, and then extracted with dichloromethane (1 L × 3). The aqueous phase was adjusted to weakly basic with ammonia water and dried by rotary evaporation. The resulting solid was extracted with dichloromethane/methanol = 10:1 (1 L), filtered, and the filtrate was concentrated. The resulting crude product was purified by silica gel column chromatography (dichloromethane:methanol = 95:5) to obtain 5.5 g of Compound B22-2 as a white solid (yield: 67%). LCMS: $[M+H]^+$ = 146.

Step B: Synthesis of Compound B22-3

Compound B22-2 (3 g, 20.6 mmol) was dissolved in N,N-dimethylformamide (30 mL). N,N-Diisopropylethylamine (7.9 g, 61.8 mmol) was added. At 0 °C, chloroacetyl chloride (2.3 g, 20.6 mmol) was added. The mixture was stirred at room temperature for 16 hours. The reaction mixture was quenched with saturated aqueous ammonium chloride and extracted with ethyl acetate (50 mL × 3). The organic phases were combined, dried and concentrated. The resultant was purified by Flash silica gel column chromatography (dichloromethane:methanol = 97:3) to obtain 1.5 g of Compound B22-3 as a gray solid (yield: 39%). LCMS: $[M+H]^+$ = 186.

Step C: Synthesis of the Target Compound

Compound B22-3 (150 mg, 0.81 mmol) and Compound B22-4 (218 mg, 0.81 mmol) were dissolved in dimethyl sulfoxide (5 mL). N,N-Diisopropylethylamine (0.42 mL, 2.43 mmol) was added. Under a nitrogen atmosphere, the mixture was heated to 120 °C and reacted for 12 hours. The reaction mixture was added to a saturated aqueous ammonium chloride solution and extracted with ethyl acetate (10 mL × 3). The organic phases were combined, dried and concentrated. The resultant was purified by preparative silicone plate (dichloromethane:methanol = 10:1) to obtain 4.8 mg of the target compound as a white solid (yield: 1.4%).

LCMS: $[M+H]^+$ = 420. $^1$H NMR (400 MHz, DMSO-$d_6$) δ 11.28 (s, 1H), 8.25 - 7.87 (m, 2H), 7.66 (dd, J = 6.8, 2.0 Hz, 1H), 7.54 (ddd, J = 8.8, 6.4, 2.0 Hz, 1H), 7.45 (dd, J = 8.8, 2.8 Hz, 1H), 6.94 (dd, J = 13.2, 7.2 Hz, 2H), 6.58 (d, J = 8.8 Hz, 1H), 6.50 (d, J = 9.2 Hz, 1H), 6.33 (td, J = 6.8, 1.2 Hz, 1H), 4.59 (s, 2H), 4.33 (q, J = 7.2, 6.4 Hz, 2H), 2.31 - 2.11 (m, 2H), 2.02 - 1.81 (m, 1H), 1.55 (td, J = 13.2, 11.6, 7.2 Hz, 2H).

Example B23 (Compound B23)

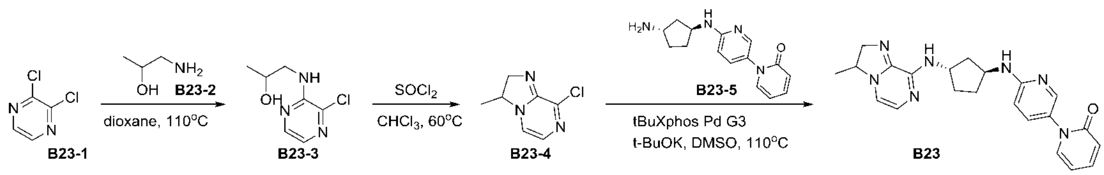


Step A: Synthesis of Compound B23-3

Compound B23-1 (10 g, 67.6 mmol) was dissolved in 100 mL 1,4-dioxane. Compound B23-2 (10 g, 133.4 mmol) was added. The mixture was heated to 110 °C and reacted for 16 hours. The reaction mixture was dried by rotary evaporation. The resultant was purified by Flash column chromatography (0 to 40% ethyl acetate/petroleum ether) to obtain 11.2 g of Compound B23-3 as a yellow oily product (yield: 88.2%). LCMS: $[M+H]^+$ = 188.

Step B: Synthesis of Compound B23-4

Compound B23-3 (940 mg, 5.0 mmol) was dissolved in 30 mL chloroform. Thionyl chloride (1.1 mL, 15 mmol) was added. The mixture was reacted at 60 °C for 16 hours. The reaction mixture was dried by rotary evaporation. The residue was dissolved in methanol, and the pH was adjusted to 8 using a hydroxide ion exchange resin. The mixture was filtered and dried by rotary evaporation. The resultant was purified by Flash column chromatography (0 to 10% methanol/dichloromethane) to obtain 320 mg of Compound B23-4 as a white solid (yield: 37.9%). LCMS: $[M+H]^+$ = 170.

Step C: Synthesis of the Target Compound

Compound B23-4 (320 mg, 1.9 mmol) and Compound B23-5 (612 mg, 2.27 mmol) were added to 5 mL anhydrous dimethyl sulfoxide. Potassium phosphate (806 mg, 3.8 mmol) and methanesulfonato(2-di-tert-butylphosphino-2',4',6'-triisopropyl-1,1'-biphenyl)(2'-amino-1,1'-bi phenyl-2-yl)palladium(II) (302 mg, 0.38 mmol) were added. Under nitrogen protection, the mixture was reacted at 100 °C for 16 hours. The reaction mixture was poured into saturated ammonium chloride (20 mL) and extracted with ethyl acetate (10 mL × 3). The organic phases were combined and dried by rotary evaporation. The resultant was purified by C18 column chromatography (0 to 20% acetonitrile/water) to obtain 7.1 mg of the target compound as a pale yellow solid (yield: 1%). LCMS: $[M+H]^+$ = 404.

$^1$H NMR (400 MHz, DMSO-$d_6$) δ 10.48 (s, 1H), 8.38 (d, J = 6.4 Hz, 1H), 7.93 (d, J = 2.8 Hz, 1H), 7.61 (dd, J = 6.8,

2.0 Hz, 1H), 7.48 (ddd, J = 9.2, 6.6, 2.0 Hz, 1H), 7.44 - 7.36 (m, 3H), 6.99 (d, J = 6.8 Hz, 1H), 6.55 (d, J = 8.8 Hz, 1H), 6.48 - 6.41 (m, 1H), 6.27 (td, J = 6.8, 1.2 Hz, 1H), 5.00 - 4.85 (m, 1H), 4.45 (h, J = 6.4 Hz, 1H), 4.34 (p, J = 6.4 Hz, 1H), 4.16 (t, J = 11.2 Hz, 1H), 3.62 (dd, J = 11.2, 8.4 Hz, 1H), 2.18 (ddd, J = 12.0, 9.2, 5.6 Hz, 2H), 2.06 (dq, J = 13.2, 6.4 Hz, 1H), 1.97 - 1.88 (m, 1H), 1.69 (dt, J = 14.4, 6.0 Hz, 1H), 1.53 (d, J = 6.4 Hz, 4H).

Examples B24, B25, B27 to B33

Referring to the above preparation methods for the Compounds, the following Compounds were prepared:

| | | | |
|---|---|---|---|
| B24 | 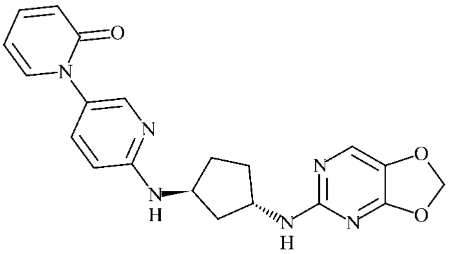<br>$^1$H NMR (400 MHz, DMSO-$d_6$) δ = 7.94 - 7.89 (m, 1H), 7.71 (s, 1H), 7.60 (dd, J = 1.8, 6.9 Hz, 1H), 7.47 (ddd, J = 2.1, 6.7, 9.0 Hz, 1H), 7.39 (dd, J = 2.6, 8.9 Hz, 1H), 6.97 (d, J = 7.0 Hz, 1H), 6.91 (d, J = 6.9 Hz, 1H), 6.51 (d, J = 8.9 Hz, 1H), 6.44 (d, J = 9.0 Hz, 1H), 6.26 (dt, J = 1.1, 6.7 Hz, 1H), 6.00 (s, 2H), 4.40 - 4.15 (m, 2H), 2.19 - 2.02 (m, 2H), 1.92 - 1.76 (m, 2H), 1.56 - 1.39 (m, 2H). | B25 | 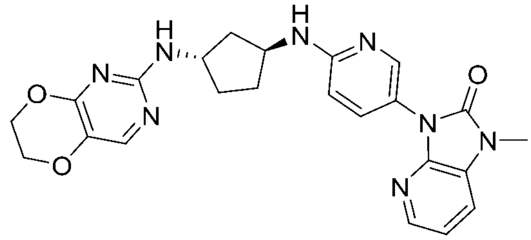<br>$^1$H NMR (400 MHz, DMSO-$d_6$) δ 8.08 (d, *J* = 2.6 Hz, 1H), 7.93 (dd, *J* = 5.2, 1.4 Hz, 1H), 7.83 (s, 1H), 7.56 (dd, *J* = 7.7, 1.4 Hz, 1H), 7.49 (dd, *J* = 8.9, 2.6 Hz, 1H), 7.13 (dd, *J* = 7.7, 5.2 Hz, 1H), 6.88 (d, *J* = 6.9 Hz, 1H), 6.78 (d, *J* = 7.2 Hz, 1H), 6.58 (d, *J* = 8.9 Hz, 1H), 4.45 - 4.35 (m, 2H), 4.29 (q, *J* = 6.6 Hz, 1H), 4.22 (q, *J* = 6.9 Hz, 1H), 4.17 - 4.10 (m, 2H), 3.40 (s, 3H), 2.19 - 2.02 (m, 2H), 1.93 - 1.78 (m, 2H), 1.48 (qd, *J* = 6.5, 3.0 Hz, 2H). |
| B27 | 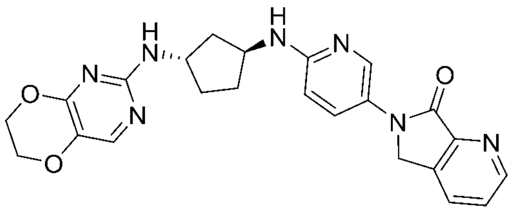<br>$^1$H NMR (400 MHz, DMSO-$d_6$) δ 8.75 (dd, *J* = 4.8, 1.5 Hz, 1H), 8.35 (d, *J* = 2.7 Hz, 1H), 8.11 (dd, *J* = 7.7, 1.5 Hz, 1H), 7.87 (dd, *J* = 9.0, 2.7 Hz, 1H), 7.83 (s, 1H), 7.62 (dd, *J* = 7.7, 4.8 Hz, 1H), 6.76 (d, *J* = 7.2 Hz, 1H), 6.66 (d, *J* = 6.9 Hz, 1H), 6.55 (d, *J* = 9.0 Hz, 1H), 4.93 (s, 2H), 4.43 - 4.37 (m, 2H), 4.23 (dq, J = 20.7, 6.7 Hz, 2H), 4.17 - 4.09 (m, 2H), 2.17 - 1.98 (m, 3H), 1.83 (ddt, *J* = 13.2, 7.5, 4.2 Hz, 2H), 1.53 - 1.41 (m, 2H). | B28 | 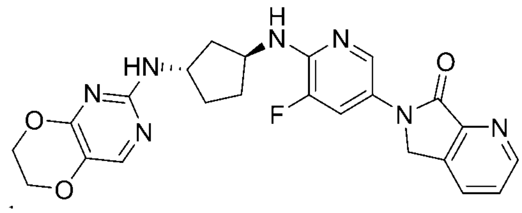<br>$^1$H NMR (400 MHz, DMSO-$d_6$) δ 8.77 (dd, *J* = 4.8, 1.4 Hz, 1H), 8.24 (d, *J* = 2.2 Hz, 1H), 8.13 (dd, *J* = 7.8, 1.4 Hz, 1H), 8.02 (dd, *J* = 13.1, 2.2 Hz, 1H), 7.83 (s, 1H), 7.63 (dd, *J* = 7.7, 4.7 Hz, 1H), 6.75 (d, *J* = 7.2 Hz, 1H), 6.63 (dd, *J* = 7.1, 1.5 Hz, 1H), 4.97 (s, 2H), 4.45 (q, *J* = 7.0 Hz, 1H), 4.42 - 4.35 (m, 2H), 4.22 (q, *J* = 6.9 Hz, 1H), 4.17 - 4.08 (m, 2H), 2.17 - 2.05 (m, 2H), 1.89 (t, *J* = 7.4 Hz, 2H), 1.62 - 1.45 (m, 2H). |

(continued)

| | | | |
|---|---|---|---|
| B29 | 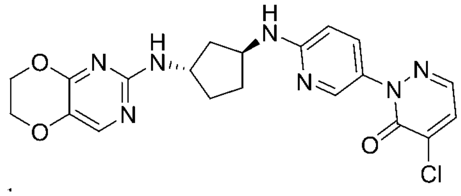 $^1$H NMR (400 MHz, DMSO-$d_6$) δ 8.13 - 8.08 (d, $J$ = 2.7 Hz, 1H), 8.00 - 7.97 (d, $J$ = 4.5 Hz, 1H), 7.85 - 7.81 (m, 2H), 7.55 - 7.49 (dd, $J$ = 9.0, 2.6 Hz, 1H), 7.01 - 6.94 (d, $J$ = 6.9 Hz, 1H), 6.80 - 6.74 (d, $J$ = 7.2 Hz, 1H), 6.55 - 6.49 (d, $J$ = 9.0 Hz, 1H), 4.42 - 4.37 (t, $J$ = 4.1 Hz, 2H), 4.32 - 4.17 (dd, $J$ = 30.3, 7.1 Hz, 2H), 4.17 - 4.10 (t, $J$ = 4.2 Hz, 2H), 2.18 - 1.95 (m, 2H), 1.91 - 1.74 (hept, J = 6.7 Hz, 2H), 1.53 - 1.38 (d, $J$ = 15.0 Hz, 2H). | B30 | 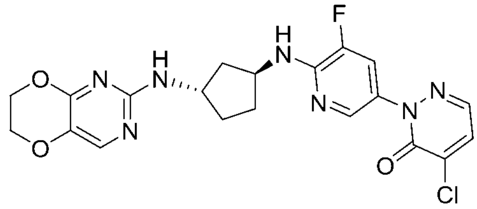 $^1$H NMR (400 MHz, DMSO-$d_6$) δ 8.04 - 8.02 (d, $J$ = 2.1 Hz, 1H), 8.01 - 7.98 (d, $J$ = 4.4 Hz, 1H), 7.86 - 7.84 (d, $J$ = 4.5 Hz, 1H), 7.83 - 7.82 (s, 1H), 7.66 - 7.63 (d, $J$ = 2.1 Hz, 1H), 7.00 - 6.96 (dd, $J$ = 7.2, 1.7 Hz, 1H), 6.78 - 6.73 (d, $J$ = 7.3 Hz, 1H), 4.54 - 4.44 (p, $J$ = 7.1 Hz, 1H), 4.41 - 4.37 (m, 2H), 4.27 - 4.18 (p, $J$ = 7.0 Hz, 1H), 4.16 - 4.11 (m, 2H), 2.17 - 2.02 (m, 2H), 1.93 - 1.83 (m, 2H), 1.61 - 1.43 (m, 2H). |
| B31 | 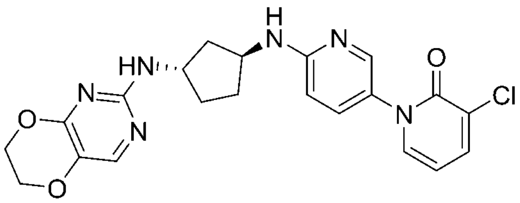 $^1$H NMR (400 MHz, DMSO-$d_6$) δ 7.95 (d, $J$ = 2.7 Hz, 1H), 7.83 (s, 1H), 7.81 (dd, $J$ = 7.3, 1.9 Hz, 1H), 7.66 (dd, $J$ = 6.9, 1.9 Hz, 1H), 7.42 (dd, $J$ = 8.9, 2.7 Hz, 1H), 6.96 (d, $J$ = 6.9 Hz, 1H), 6.77 (d, $J$ = 7.2 Hz, 1H), 6.52 (d, $J$ = 9.0 Hz, 1H), 6.30 (t, $J$ = 7.1 Hz, 1H), 4.44 - 4.37 (m, 2H), 4.28 (q, $J$ = 6.6 Hz, 1H), 4.21 (q, $J$ = 7.0 Hz, 1H), 4.17 - 4.10 (m, 2H), 2.18 - 2.03 (m, 2H), 1.85 (hept, J = 6.8 Hz, 2H), 1.54 - 1.39 (m, 2H). | B32 | 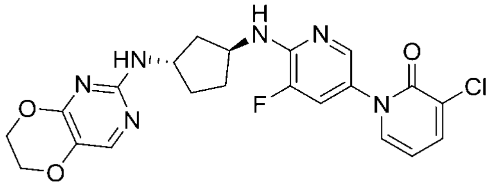 $^1$H NMR (400 MHz, DMSO-$d_6$) δ 7.88 (d, $J$ = 2.2 Hz, 1H), 7.86 - 7.78 (m, 2H), 7.70 (dd, $J$ = 6.8, 1.9 Hz, 1H), 7.59 (dd, $J$ = 11.9, 2.2 Hz, 1H), 6.96 (dd, $J$ = 7.2, 1.6 Hz, 1H), 6.76 (d, $J$ = 7.2 Hz, 1H), 6.32 (t, $J$ = 7.1 Hz, 1H), 4.48 (q, $J$ = 7.1 Hz, 1H), 4.43 - 4.35 (m, 2H), 4.22 (q, $J$ = 6.9 Hz, 1H), 4.17 - 4.09 (m, 2H), 2.15 - 2.05 (m, 2H), 1.95 - 1.84 (m, 2H), 1.64 - 1.53 (m, 1H), 1.53 - 1.40 (m, 1H). |
| B33 | 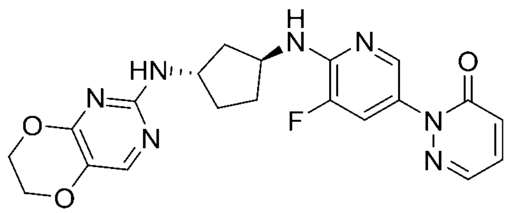 | $^1$H NMR (400 MHz, DMSO-$d_6$) δ 8.03 (dd, $J$ = 3.7, 1.8 Hz, 2H), 7.83 (s, 1H), 7.61 (dd, $J$ = 12.1, 2.1 Hz, 1H), 7.48 (dd, $J$ = 9.5, 3.9 Hz, 1H), 7.05 (dd, $J$ = 9.5, 1.6 Hz, 1H), 6.91 (dd, $J$ = 7.1, 1.7 Hz, 1H), 6.76 (d, $J$ = 7.2 Hz, 1H), 4.48 (q, $J$ = 7.1 Hz, 1H), 4.43 - 4.36 (m, 2H), 4.21 (p, $J$ = 7.1 Hz, 1H), 4.17 - 4.07 (m, 2H), 2.13 - 2.06 (m, 2H), 1.94 - 1.83 (m, 2H), 1.62 - 1.52 (m, 1H), 1.48 (ddd, $J$ = 9.3, 7.0, 4.7 Hz, 1H). | |

Example B26 (Compound B26)

Synthesis of 3-(6-(((1S,3S)-3-((6,7-dihydro-[1,4]dioxino[2,3-d]pyrimidin-2-yl)amino)cyclopentyl)amino)-5-fl uoropyridin-3-yl)-1-methyl-1,3-dihydro-2H-imidazo[4,5-b]pyridin-2-one

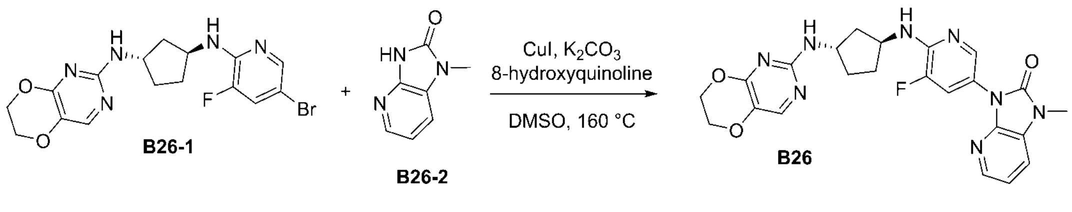


B26-1 B26-2 B26

At room temperature, Compound B26-1 (200 mg, 0.49 mmol), Compound B26-2 (110 mg, 0.73 mmol, 1.5 eq), potassium carbonate (200 mg, 1.47 mmol, 3 eq), copper(I) iodide (47 mg, 0.24 mmol, 0.5 eq), 8-hydroxyquinoline (36 mg,

0.24 mmol, 0.5 eq), and DMSO (3 mL) were sequentially added to a 10 mL microwave reaction tube. The reaction system was purged with nitrogen atmosphere, then heated to 160 °C and reacted under microwave irradiation for 2 hours.

LC-MS detected the formation of the target product. The reaction mixture was filtered and concentrated. The resultant was purified by C18 reverse-phase column chromatography (mobile phase: water (FA)-ACN; gradient: 0% to 80% B over 50 min) to obtain the target Compound (99 mg).

$^1$H NMR (400 MHz, DMSO-$d_6$) δ 8.04 (d, J = 2.1 Hz, 1H), 7.95 (dd, J = 5.2, 1.4 Hz, 1H), 7.83 (s, 1H), 7.64 - 7.56 (m, 2H), 7.16 (dd, J = 7.7, 5.2 Hz, 1H), 6.87 (d, J = 7.0 Hz, 1H), 6.76 (d, J = 7.2 Hz, 1H), 4.49 (h, J = 7.0 Hz, 1H), 4.43 - 4.34 (m, 2H), 4.22 (dt, J = 14.1, 6.9 Hz, 1H), 4.17 - 4.10 (m, 2H), 3.41 (s, 4H), 2.12 (dq, J = 13.5, 4.5 Hz, 2H), 1.91 (td, J = 6.7, 3.6 Hz, 2H), 1.58 (ddd, J = 16.7, 14.2, 8.6 Hz, 1H), 1.53 - 1.42 (m, 1H).

Example C1

Synthesis of 6'-(((1S,3S)-3-((5-(difluoromethoxy)thiazolo[5,4-b]pyridin-2-yl)amino)cyclopentyl)amino)-2H-[ 1,3'-bipyridin]-2-one

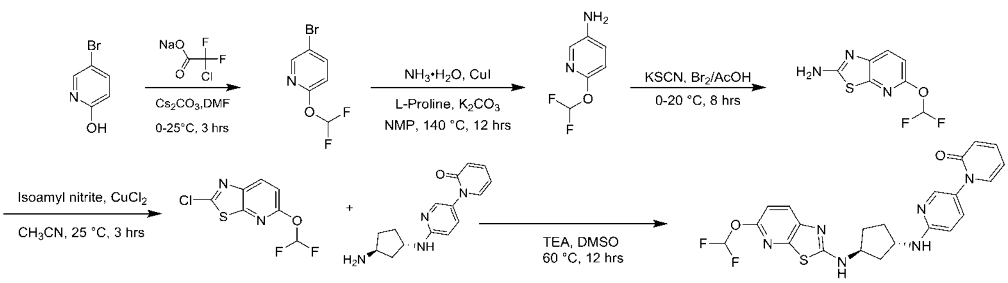


Step 1: Synthesis of 5-bromo-2-(difluoromethoxy)pyridine

At room temperature, 5-bromopyridin-2-ol (10.0 g, 57.5 mmol, 1.00 eq) was dissolved in DMF (200 mL). Cesium carbonate (22.5 g, 69.0 mmol, 1.20 eq) was added. Under nitrogen protection, the mixture was stirred at 25 °C for 1.5 hours. Sodium 2-chloro-2,2-difluoroacetate (26.3 g, 172 mmol, 3.00 eq) was then added. The mixture was heated to 100 °C and stirred for 1.5 hours.

LCMS showed the MS of the target product (RT = 0.537 min, m/z = 223.9 $[M+H]^+$). The mixture was cooled to room temperature. 600 mL water was added, and the mixture was extracted with ethyl acetate (600 mL × 5). The organic phases were combined, washed with saturated sodium chloride solution, dried over anhydrous sodium sulfate, and concentrated under reduced pressure. The resultant was mixed with silica gel and purified by column chromatography, eluting with petroleum ether to obtain 5-bromo-2-(difluoromethoxy)pyridine as a colorless oily liquid (3.01 g, 13.4 mmol, 23.38% yield). LCMS (ESI) m/z = 223.9 $[M+H]^+$.

Step 2: Synthesis of 6-(difluoromethoxy)pyridin-3-amine

At room temperature, 5-bromo-2-(difluoromethoxy)pyridine (3.00 g, 13.4 mmol, 1.00 eq), ammonia water (2.35 g, 20.1 mmol, 2.58 mL, 30% purity, 1.50 eq), copper(I) iodide (510 mg, 2.68 mmol, 0.2 eq), potassium carbonate (2.78 g, 20.09 mmol, 1.50 eq), and L-proline (616.76 mg, 5.36 mmol, 0.40 eq) were added to N-methylpyrrolidone (30 mL). The mixture was heated to 140 °C in a sealed vessel and stirred for 12 hours.

TLC (petroleum ether/ethyl acetate = 5/1) indicated the reaction of the starting material was complete. 20 mL water was added, and the mixture was extracted with ethyl acetate (30 mL × 4). The organic phases were combined, washed with saturated sodium chloride solution, dried over anhydrous sodium sulfate, and concentrated under reduced pressure. The resultant was mixed with silica gel and purified by column chromatography (petroleum ether/ethyl acetate = 5/1) to obtain 6-(difluoromethoxy)pyridin-3-amine as a yellow oily product (1.3 g, 8.12 mmol, 60.62% yield).

Step 3: Synthesis of 5-(difluoromethoxy)thiazolo[5,4-b]pyridin-2-amine

In an ice bath at 0 °C, 6-(difluoromethoxy)pyridin-3-amine (1.00 g, 6.25 mmol, 1.00 eq) and potassium thiocyanate (4.86 g, 50.0 mmol, 4.84 mL, 8.00 eq) were dissolved in glacial acetic acid (3.00 mL). A solution of bromine (2.99 g, 18.8 mmol, 965 μL, 3.00 eq) in glacial acetic acid (1.00 mL) was slowly added dropwise, keeping the reaction temperature below 0 °C. After the addition, the mixture was allowed to warm naturally to 20 °C and stirred at 20 °C for 8

hours.

TLC (petroleum ether/ethyl acetate = 2/1) indicated the reaction of the starting material was complete. 5 mL water was added, and the mixture was heated to 85 °C. The mixture was filtered while hot. The filter cake was added to acetic acid (1.5 mL), heated to 85 °C, and filtered while hot. The two filtrates were combined, and the pH was adjusted to 8 with ammonia water in an ice-water bath, resulting in the precipitation of a large amount of yellow solid. The mixture was filtered under reduced pressure, and the filter cake was dried to obtain 5-(difluoromethoxy)thiazolo[5,4-b]pyridin-2-amine as a yellow solid (0.9 g, 4.14 mmol, 66.35% yield).

Step 4: Synthesis of 2-chloro-5-(difluoromethoxy)thiazolo[5,4-b]pyridine

At room temperature, 5-(difluoromethoxy)thiazolo[5,4-b]pyridin-2-amine (0.90 g, 4.14 mmol, 1.00 eq) was dissolved in acetonitrile (15 mL). Under nitrogen protection, isoamyl nitrite (728 mg, 6.22 mmol, 837 μL, 1.50 eq) and copper(II) chloride (669 mg, 4.97 mmol, 161 μL, 1.20 eq) were added. The mixture was stirred at 25 °C for 3 hours.

TLC (petroleum ether/ethyl acetate = 2/1) indicated the reaction of the starting material was complete. 8 mL saturated ammonium chloride solution was added, and the mixture was stirred for 10 minutes. 25 mL water was added to dilute the mixture, followed by extraction with ethyl acetate (80 mL × 4). The organic phases were combined, washed with saturated sodium chloride solution, dried over anhydrous sodium sulfate, and concentrated under reduced pressure. The resultant was mixed with silica gel and purified by column chromatography (petroleum ether/ethyl acetate = 1/0 to 0/1) to obtain 2-chloro-5-(difluoromethoxy)thiazolo[5,4-b]pyridine as a white solid (0.7 g, 2.96 mmol, 71.39% yield). LCMS (ESI) m/z = 236.9 $[M+H]^+$.

Step 5: Synthesis of 6'-(((1S,3S)-3-((5-(difluoromethoxy)thiazolo[5,4-b]pyridin-2-yl)amino)cyclopentyl)amino)-2H-[ 1,3'-bipyridin]-2-one

At room temperature, 2-chloro-5-(difluoromethoxy)thiazolo[5,4-b]pyridine (200 mg, 845 μmol, 1 eq), 6'-(((1S,3S)-3-aminocyclopentyl)amino)-2H-[1,3'-bipyridin]-2-one hydrochloride (259 mg, 845 μmol, 1.00 eq, HCl), and triethylamine (257 mg, 2.54 mmol, 352 μL, 3.00 eq) were added to DMSO (10.0 mL). Under nitrogen protection, the mixture was heated to 60 °C and reacted for 12 hours.

LCMS showed the MS of the target product (RT = 0.423 min, m/z = 471.1 $[M+H]^+$). 30 mL water was added to the reaction mixture to quench the reaction. The mixture was extracted with ethyl acetate (60 mL × 4). The organic phases were combined, washed with saturated sodium chloride solution, dried over anhydrous sodium sulfate, and concentrated under reduced pressure. The resultant was mixed with silica gel and purified by column chromatography (petroleum ether/ethyl acetate = 1/0 to 1/1) to obtain 6'-(((1S,3S)-3-((5-(difluoromethoxy)thiazolo[5,4-b]pyridin-2-yl)amino)cyclopentyl)amino)-2H-[ 1,3'-bipyridin]-2-one as a yellow solid (124 mg, 264 μmol, 31.18% yield).

LCMS (ESI) m/z = 471.1 $[M+H]^+$.

$^1$H NMR (400 MHz, CDCl3): δ 8.41 (d, J = 6.6 Hz, 1H), 7.93 (d, J = 2.6 Hz, 1H), 7.84 - 7.77 (m, 1H), 7.64 - 7.56 (m, 1H), 7.47 (ddd, J = 2.1, 6.8, 9.1 Hz, 1H), 7.44 - 7.37 (m, 1H), 7.02 - 6.88 (m, 2H), 6.53 (d, J = 9.0 Hz, 1H), 6.44 (d, J = 9.1 Hz, 1H), 6.27 (dt, J = 1.2, 6.7 Hz, 1H), 4.47 - 4.26 (m, 2H), 2.26 - 2.11 (m, 2H), 1.96 (tq, J = 6.6, 13.7 Hz, 2H), 1.65 - 1.49 (m, 2H).

Example C2

Synthesis of 6'-(((1S,3S)-3-((7-(difluoromethoxy)-[1,2,4]triazolo[1,5-a]pyridin-2-yl)amino)cyclopentyl)amin o)-2H-[1,3'-bipyridin]-2-one

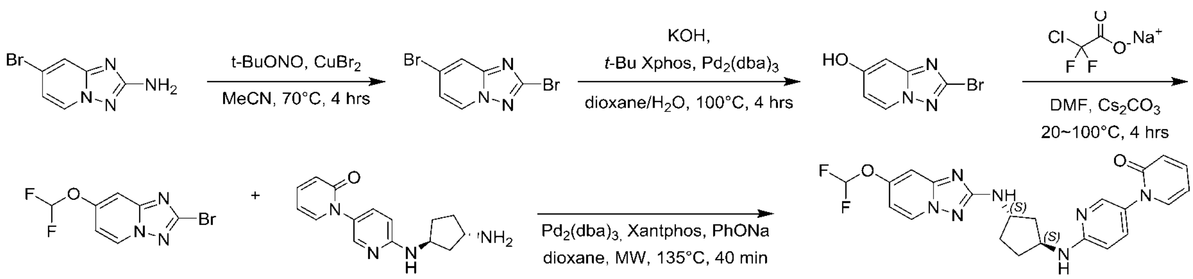

Step 1: Synthesis of 2,7-dibromo-[1,2,4]triazolo[1,5-a]pyridine

7-Bromo-[1,2,4]triazolo[1,5-a]pyridin-2-amine (2.00 g, 9.39 mmol, 1 eq), copper(II) bromide (3.15 g, 14.1 mmol, 1.5 eq), and tert-butyl nitrite (1.45 g, 14.1 mmol, 1.5 eq) were dissolved in acetonitrile (120 mL). The mixture was stirred at 80 °C for 3 hours. LCMS detected the MS value of the product (RT = 0.452 min). The reaction mixture was cooled to room temperature, diluted with water (200 mL), and extracted with ethyl acetate (80 mL × 2). The organic phases were combined, washed with saturated brine (200 mL × 2), dried over anhydrous sodium sulfate, filtered, and concentrated to obtain 2,7-dibromo-[1,2,4]triazolo[1,5-a]pyridine (2.00 g, 7.22 mmol, 76.9% yield). This was used directly in the next step without purification. LCMS (ESI) m/z = 277.9 $[M+1]^+$.

Step 2: Synthesis of 2-bromo-[1,2,4]triazolo[1,5-a]pyridin-7-ol

2,7-dibromo-[1,2,4]triazolo[1,5-a]pyridine (2.00 g, 7.22 mmol, 1 eq), potassium hydroxide (1.2 g, 21.7 mmol, 3 eq), t-Bu XPhos (675 mg, 1.59 mmol, 0.22 eq), and $Pd_2(dba)_3$ (728 mg, 794 μmol, 0.11 eq) were dissolved in 1,4-dioxane (20 mL) and water (5 mL). The mixture was stirred at 100 °C for 4 hours. LCMS detected the MS value of the product (RT = 0.358 min). The reaction mixture was cooled to room temperature. Water (60 mL) and ethyl acetate (40 mL) were added to dilute the mixture. The organic phase was separated and discarded. The aqueous phase was adjusted to pH = 5 with dilute hydrochloric acid and then extracted with ethyl acetate (60 mL × 2). The organic phases were combined, washed with saturated brine (100 mL × 2), dried over anhydrous sodium sulfate, filtered, and concentrated to obtain 2-bromo-[1,2,4]triazolo[1,5-a]pyridin-7-ol (860 mg, 3.97 mmol, 54.9% yield, 98.7% purity). This was used directly in the next step without purification. LCMS (ESI) m/z = 215.9 $[M+1]^+$.

Step 3: Synthesis of 2-bromo-7-(difluoromethoxy)-[1,2,4]triazolo[1,5-a]pyridine

2-Bromo-[1,2,4]triazolo[1,5-a]pyridin-7-ol (400 mg, 1.84 mmol, 1 eq) and cesium carbonate (721 mg, 2.21 mmol, 1.2 eq) were dissolved in N,N-dimethylformamide (10 mL). The mixture was stirred at 20 °C for 1 hour. Sodium chlorodifluoroacetate (844 mg, 5.53 mmol, 3 eq) was then added to the reaction mixture. The mixture was stirred at 100 °C for 3 hours. TLC (petroleum ether/ethyl acetate = 3/1) indicated the completion of the reaction. The reaction mixture was cooled to room temperature, diluted with water (100 mL), and extracted with ethyl acetate (20 mL × 2). The organic phases were combined, washed with saturated brine (50 mL × 2), dried over anhydrous sodium sulfate, filtered, and concentrated to obtain a crude product. The crude product was purified by reverse-phase preparative chromatography (Column: C18 150×30mm; Mobile phase: [water (FA)-ACN]; Gradient: 30% to 60% B over 7 min) to obtain 2-bromo-7-(difluoromethoxy)-[1,2,4]triazolo[1,5-a]pyridine (230 mg, 871 μmol, 47.2% yield, 100% purity). LCMS (ESI) m/z = 263.9 $[M+1]^+$.

Step 4: Synthesis of 6'-(((1S,3S)-3-((7-(difluoromethoxy)-[1,2,4]triazolo[1,5-a]pyridin-2-yl)amino)cyclopentyl)amin o)-2H-[1,3'-bipyridin]-2-one

2-Bromo-7-(difluoromethoxy)-[1,2,4]triazolo[1,5-a]pyridine (100 mg, 379 μmol, 1 eq), 6'-(((1S,3S)-3-aminocyclopentyl)amino)-2H-[1,3'-bipyridin]-2-one (123 mg, 455 μmol, 1.2 eq), XantPhos (17.5 mg, 30.3 μmol, 0.08 eq), sodium phenoxide (66.0 mg, 568 μmol, 1.5 eq), and Pd2(dba)3 (13.9 mg, 15.2 μmol, 0.04 eq) were dissolved in 1,4-dioxane (4 mL). The mixture was stirred at 135 °C under microwave irradiation for 40 minutes. LCMS detected the MS value of the product (RT = 0.391 min). The reaction mixture was filtered. The filtrate was concentrated to obtain a crude product. The crude product was purified by reverse-phase preparative chromatography (Column: C18 150×30mm; Mobile phase: [water (FA)-ACN]; Gradient: 12% to 42% B over 7 min) to obtain 6'-(((1S,3S)-3-((7-(difluoromethoxy)-[1,2,4]triazolo[1,5-a]pyridin-2-yl)amino)cyclopentyl)amin o)-2H-[1,3'-bipyridin]-2-one (49.0 mg, 103 μmol, 27.1% yield, 95.1% purity).

LCMS (ESI) m/z = 454.2 $[M+1]^+$.

$^1H$ NMR: (400 MHz, DMSO-$d_6$): δ 8.63 (d, J = 7.3 Hz, 1H), 7.92 (d, J = 2.5 Hz, 1H), 7.63 - 7.22 (m, 4H), 7.14 (d, J = 2.6 Hz, 1H), 6.91 (d, J = 6.9 Hz, 1H), 6.78 - 6.70 (m, 2H), 6.52 (d, J = 9.0 Hz, 1H), 6.44 (d, J = 8.9 Hz, 1H), 6.26 (dt, J = 1.3, 6.7 Hz, 1H), 4.38 - 4.26 (m, 1H), 4.15 (qd, J = 6.8, 13.4 Hz, 1H), 2.22 - 2.09 (m, 2H), 2.02 - 1.82 (m, 2H), 1.64 - 1.43 (m, 2H).

Example C3

Synthesis of 6'-(((1S,3S)-3-((6-(difluoromethoxy)thiazolo[5,4-b]pyridin-2-yl)amino)cyclopentyl)amino)-2H-[ 1,3'-bipyridin]-2-one

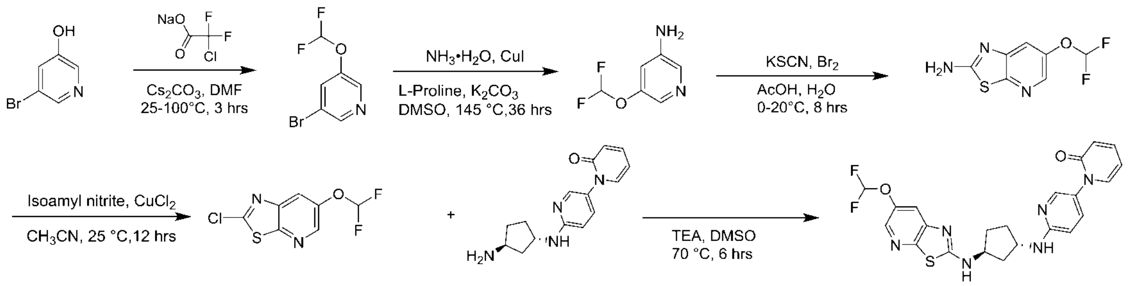


Step 1: Synthesis of 3-bromo-5-(difluoromethoxy)pyridine

At room temperature, 3-bromo-5-hydroxypyridine (40.0 g, 230 mmol, 1.00 eq) was dissolved in DMF (800 mL). $Cs_2CO_3$ (89.9 g, 276 mmol, 1.20 eq) was added. Under nitrogen protection, the mixture was stirred at 25 °C for 1.5 hours. Sodium 2-chloro-2,2-difluoroacetate (105 g, 690 mmol, 3.00 eq) was then added. The mixture was heated to 100 °C and stirred for 1.5 hours.

TLC (petroleum ether/ethyl acetate = 5/1) indicated the reaction of the starting material was complete and the target product formed. 60 mL water was added. The mixture was extracted with ethyl acetate (50 mL × 3). The organic phases were combined, washed with saturated sodium chloride solution, dried over anhydrous sodium sulfate, and concentrated under reduced pressure to obtain a crude product. The resultant was mixed with silica gel and purified by column chromatography, eluting with pure petroleum ether to obtain 3-bromo-5-(difluoromethoxy)pyridine as a yellow oily product (6.3 g, 28.1 mmol, 12.23% yield).

Step 2: Synthesis of 5-(difluoromethoxy)pyridin-3-amine

At room temperature, 3-bromo-5-(difluoromethoxy)pyridine (5.00 g, 22.3 mmol, 1.00 eq), ammonia water (3.91 g, 33.5 mmol, 4.30 mL, 30% purity, 1.50 eq), copper(I) iodide (850 mg, 4.46 mmol, 0.20 eq), $K_2CO_3$ (4.63 g, 33.48 mmol, 1.5 eq), and N,N'-bis(2-furylmethyl)oxalamide (2.22 g, 8.93 mmol, 0.4 eq) were added to NMP (10 mL). The mixture was reacted in a sealed vessel. The mixture was heated to 145 °C and stirred for 36 hours.

TLC (petroleum ether/ethyl acetate = 1/1) indicated the reaction of the starting material was complete and a new main spot formed. 100 mL water was added to the reaction mixture to quench the reaction. The mixture was extracted with ethyl acetate (100 mL × 4). The organic phases were combined, washed with saturated sodium chloride solution, dried over anhydrous sodium sulfate, and concentrated under reduced pressure to obtain a crude product. The crude product was purified by column chromatography (petroleum ether/ethyl acetate = 1/1) to obtain the target product 5-(difluoromethoxy)pyridin-3-amine (3 g, 18.7 mmol, 83.94% yield).

Step 3: Synthesis of 6-(difluoromethoxy)thiazolo[5,4-b]pyridin-2-amine

In an ice bath at 0 °C, 5-(difluoromethoxy)pyridin-3-amine (2.00 g, 12.5 mmol, 1.00 eq) and KSCN (9.71 g, 100 mmol, 9.68 mL, 8.00 eq) were dissolved in AcOH (6 mL). A solution of $Br_2$ (5.99 g, 37.5 mmol, 1.93 mL, 3.00 eq) in AcOH (2 mL) was slowly added dropwise, keeping the reaction temperature below 0 °C. After the addition, the mixture was allowed to warm naturally to 20 °C and stirred for 8 hours.

TLC (petroleum ether/ethyl acetate = 2/1) indicated the reaction of the starting material was incomplete and a new spot for the target product formed. 5 mL water was added, and the mixture was heated to 85 °C. The mixture was filtered while hot. The filter cake was added to 1.5 mL acetic acid, heated to 85 °C, and filtered while hot. The two filtrates were combined, and the pH was adjusted to 8 with ammonia water in an ice-water bath, resulting in the precipitation of a large amount of yellow solid. The mixture was filtered under reduced pressure, and the filter cake was dried. The resultant was mixed with silica gel and purified by column chromatography (petroleum ether/ethyl acetate = 2/1) to obtain the target solid product 6-(difluoromethoxy)thiazolo[5,4-b]pyridin-2-amine as a yellow solid (460 mg, 2.12 mmol, 16.96% yield). LCMS (ESI) m/z = 218.0 $[M+H]^+$.

Step 4: Synthesis of 2-chloro-6-(difluoromethoxy)thiazolo[5,4-b]pyridine

At room temperature, 6-(difluoromethoxy)thiazolo[5,4-b]pyridin-2-amine (450 mg, 2.07 mmol, 1.00 eq) was dissolved in acetonitrile (10 mL). Under nitrogen protection, isoamyl nitrite (364 mg, 3.11 mmol, 418 μL, 1.50 eq) and $CuCl_2$ (334 mg, 2.49 mmol, 1.20 eq) were added. The mixture was stirred at 25 °C for 12 hours.

LCMS showed the MS of the target product (RT = 0.524 min, m/z = 236.9 $[M+H]^+$). 20 mL saturated ammonium chloride solution was added, and the mixture was stirred for 10 minutes. 100 mL water was added to dilute the mixture,

followed by extraction with ethyl acetate (150 mL × 4). The organic phases were combined, washed with saturated sodium chloride solution, dried over anhydrous sodium sulfate, and concentrated under reduced pressure. The resultant was purified by column chromatography (petroleum ether/ethyl acetate = 5/1) to obtain the target product 2-chloro-6-(difluoromethoxy)thiazolo[5,4-b]pyridine as a white solid (275 mg, 1.16 mmol, 56.09% yield). LCMS (ESI) m/z = 236.9 $[M+H]^+$.

Step 5: Synthesis of 6'-(((1S,3S)-3-((6-(difluoromethoxy)thiazolo[5,4-b]pyridin-2-yl)amino)cyclopentyl)amino)-2H-[ 1,3'-bipyridin]-2-one

At room temperature, 2-chloro-6-(difluoromethoxy)thiazolo[5,4-b]pyridine (100 mg, 423 μmol, 1.00 eq), 6'-(((1S,3S)-3-aminocyclopentyl)amino)-2H-[1,3'-bipyridin]-2-one (114 mg, 423 μmol, 1 eq), and TEA (128 mg, 1.27 mmol, 176 μL, 3.00 eq) were added to DMSO (2.00 mL). The mixture was stirred at 70 °C for 6 hours.

LCMS showed the MS of the target product (RT = 0.417 min, m/z = 471.2 $[M+H]^+$). The mixture was cooled to room temperature. 10 mL water was added to dilute the mixture, followed by extraction with ethyl acetate (60 mL × 4). The organic phases were combined, washed with saturated sodium chloride solution, dried over anhydrous sodium sulfate, and concentrated under reduced pressure. The resultant was purified by column chromatography (petroleum ether/ethyl acetate = 0/1) to obtain the target product 6'-(((1S,3S)-3-((6-(difluoromethoxy)thiazolo[5,4-b]pyridin-2-yl)amino)cyclopentyl)amino)-2H-[ 1,3'-bipyridin]-2-one as a pale yellow solid (198 mg, 421 μmol, 99.58% yield).

LCMS (ESI) m/z = 471.2 $[M+H]^+$.

$^1H$ NMR (400 MHz, DMSO-$d_6$): δ 8.67 (d, J = 6.8 Hz, 1H), 8.01 (d, J = 2.4 Hz, 1H), 7.93 (d, J = 2.6 Hz, 1H), 7.60 (dd, J = 1.6, 6.8 Hz, 1H), 7.56 (d, J = 2.5 Hz, 1H), 7.51 - 7.07 (m, 3H), 6.97 (d, J = 6.9 Hz, 1H), 6.53 (d, J = 8.9 Hz, 1H), 6.44 (d, J = 8.9 Hz, 1H), 6.27 (dt, J = 1.2, 6.7 Hz, 1H), 4.46 - 4.28 (m, 2H), 2.28 - 2.11 (m, 2H), 2.06 - 1.87 (m, 2H), 1.69 - 1.45 (m, 2H).

Example C4

Synthesis of 6'-(((1S,3S)-3-((5,6,7,8-tetrahydro-[1,2,4]triazolo[1,5-a]pyridin-2-yl)amino)cyclopentyl)amino)-2H-[1,3'-bipyridin]-2-one

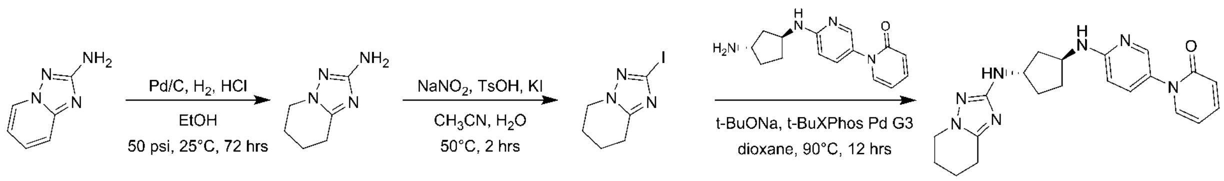


Step 1: Synthesis of 5,6,7,8-tetrahydro-[1,2,4]triazolo[1,5-a]pyridin-2-amine

At 25 °C, [1,2,4]triazolo[1,5-a]pyridin-2-amine (2.50 g, 18.6 mmol, 1.00 eq), wet palladium on carbon (0.40 g, 10% purity), and HCl (3.65 mL, 36.5% purity, 2.00 eq) were dissolved in ethanol (30 mL). The atmosphere was then replaced with hydrogen three times. The mixture was reacted under a hydrogen atmosphere (50 psi) for 72 hours.

LCMS detected the MS value of the product. After completion of the reaction, the mixture was filtered and concentrated to obtain 5,6,7,8-tetrahydro-[1,2,4]triazolo[1,5-a]pyridin-2-amine (2.50 g, 97.1% yield). MS (ESI) m/z = 139.1 $[M+1]^+$.

Step 2: Synthesis of 2-iodo-5,6,7,8-tetrahydro-[1,2,4]triazolo[1,5-a]pyridine

5,6,7,8-Tetrahydro-[1,2,4]triazolo[1,5-a]pyridin-2-amine (0.20 g, 1.45 mmol, 1.00 eq), potassium iodide (601 mg, 3.62 mmol, 2.50 eq), p-toluenesulfonic acid (997 mg, 5.79 mmol, 4.00 eq), and sodium nitrite (200 mg, 2.89 mmol, 2.00 eq) were dissolved in acetonitrile (2 mL) and water (0.40 mL). The mixture was then heated to 50 °C and reacted for 2 hours.

TLC (dichloromethane:methanol = 20:1) indicated the starting material was completely consumed ($R_f$ = 0.24).

The reaction mixture was concentrated, then diluted with ethyl acetate (50 mL) and water (70 mL). The phases were separated. The aqueous phase was extracted with ethyl acetate (20 mL × 3). The organic phases were combined, washed with saturated brine (30 mL × 2), dried over anhydrous sodium sulfate, filtered, and concentrated to obtain a crude product. The crude product was purified by column chromatography (petroleum ether:ethyl acetate = 50:1 to 1:1) to obtain 2-iodo-5,6,7,8-tetrahydro-[1,2,4]triazolo[1,5-a]pyridine (250 mg, 69.4% yield).

Step 3: Synthesis of 6'-(((1S,3S)-3-((5,6,7,8-tetrahydro-[1,2,4]triazolo[1,5-a]pyridin-2-yl)amino)cyclopentyl)amino)-2H-[1,3'-bipyridin]-2-one

2-Iodo-5,6,7,8-tetrahydro-[1,2,4]triazolo[1,5-a]pyridine (200 mg, 1.00 eq), 6'-(((1S,3S)-3-aminocyclopentyl) amino)-2H-[1,3'-bipyridin]-2-one (217 mg, 1.00 eq), sodium tert-butoxide (232 mg, 2.41 mmol, 3.00 eq), and t-BuXPhos Pd G3 (63.8 mg, 0.10 eq) were dissolved in 1,4-dioxane (15 mL). The atmosphere was purged with nitrogen three times. The mixture was then heated to 90 °C and reacted for 12 hours.
LCMS detected the MS value of the product.
The reaction mixture was concentrated to obtain a crude product. The crude product was purified by reverse-phase preparative chromatography (Column: C18 150×30mm; Mobile phase: [water (FA)-ACN]; Gradient: 2% to 32% B over 7 min) to obtain 6'-(((1S,3S)-3-((5,6,7,8-tetrahydro-[1,2,4]triazolo[1,5-a]pyridin-2-yl)amino)cyclopentyl)amino)-2H-[1,3'-bipyridin]-2-one (45 mg, 13.80% yield, 96.4% purity).
LCMS (ESI) m/z = 392.2 $[M+1]^+$.
$^1$H NMR (400 MHz, CD3OD): δ 7.98 - 7.89 (m, 1H), 7.66 - 7.54 (m, 2H), 7.49 - 7.36 (m, 1H), 6.61 (dd, J = 5.2, 8.8 Hz, 2H), 6.47 (dt, J = 1.2, 6.8 Hz, 1H), 4.40 - 4.27 (m, 1H), 4.06 (quintet, J = 6.4 Hz, 1H), 3.94 (t, J = 6.0 Hz, 2H), 2.74 (t, J = 6.4 Hz, 2H), 2.66 (s, 1H), 2.35 - 2.16 (m, 2H), 2.09 - 1.87 (m, 5H), 1.66 - 1.51 (m, 2H).

Example C5

Synthesis of 6'-(((1S,3S)-3-((7-cyclopropyl-5,6,7,8-tetrahydro-[1,2,4]triazolo[1,5-a]pyrazin-2-yl)amino)cyclo pentyl) amino)-2H-[1,3'-bipyridin]-2-one

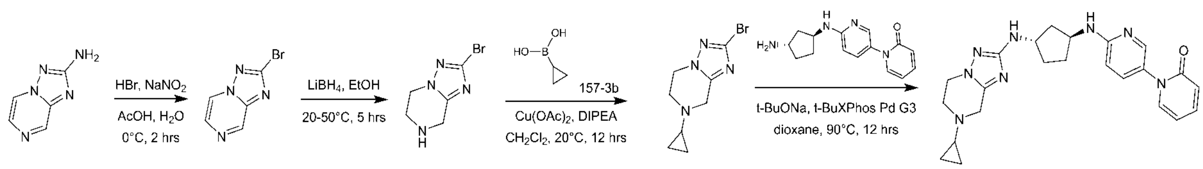


Step 1: Synthesis of 2-bromo-[1,2,4]triazolo[1,5-a]pyrazine

At 0 °C, [1,2,4]triazolo[1,5-a]pyrazin-2-amine (7.00 g, 51.8 mmol, 1.00 eq) and hydrobromic acid (29.3 mL, 48% purity, 5.00 eq) were dissolved in acetic acid (40 mL). A solution of sodium nitrite (7.15 g, 2.00 eq) in water (40 mL) was then added dropwise to the reaction mixture. After the addition, the reaction was maintained for 2 hours.
LCMS detected the MS value of the product.
The reaction mixture was concentrated, then diluted with water (100 mL) and ethyl acetate (60 mL). The aqueous phase was extracted with ethyl acetate (20 mL × 3). The organic phases were combined, washed with saturated brine (30 mL × 3), dried over anhydrous sodium sulfate, filtered, and concentrated to obtain a crude product. The crude product was purified by column chromatography (petroleum ether:ethyl acetate = 20:1 to 1:1) to obtain 2-bromo-[1,2,4]triazolo[1,5-a] pyrazine (3.50 g, 33.9% yield). LCMS (ESI) m/z = 200.9 $[M+1]^+$.

Step 2: Synthesis of 2-bromo-5,6,7,8-tetrahydro-[1,2,4]triazolo[1,5-a]pyrazine

At 20 °C, 2-bromo-[1,2,4]triazolo[1,5-a]pyrazine (3.00 g, 1.00 eq) was dissolved in ethanol (20 mL). Lithium borohydride (2.00 M, 30.2 mL, 4.00 eq) was then added to the solution. After the addition, the reaction mixture was heated to 50 °C and reacted for 5 hours.
LCMS detected the MS value of the product.
After completion of the reaction, the mixture was quenched with 1 M hydrochloric acid (800 mL). The mixture was then washed with ethyl acetate (30 mL × 2), and the organic phases were discarded. The aqueous phase was adjusted to pH = 9 with saturated sodium carbonate solution and then extracted with dichloromethane (40 mL × 5). The organic phases from the second extraction were washed with saturated brine (30 mL × 2), dried over anhydrous sodium sulfate, filtered, and concentrated to obtain a crude product. The crude product was purified by reverse-phase preparative chromatography (Column: Waters Xbridge 150*25mm 5um; Mobile phase: [water ($NH_4HCO_3$)-ACN]; Gradient: 1% to 30% B over 9 min) to obtain 2-bromo-5,6,7,8-tetrahydro-[1,2,4]triazolo[1,5-a]pyrazine (1.60 g, 52.3% yield). MS (ESI) m/z = 203.0 $[M+1]^+$.

Step 3: Synthesis of 2-bromo-7-cyclopropyl-6,8-dihydro-5H-[1,2,4]triazolo[1,5-a]pyrazine

2-Bromo-5,6,7,8-tetrahydro-[1,2,4]triazolo[1,5-a]pyrazine (0.70 g, 3.45 mmol, 1.00 eq), cyclopropylboronic acid (4441 mg, 5.17 mmol, 1.50 eq), copper(II) acetate (1.25 g, 6.90 mmol, 2.00 eq), and N,N-diisopropylethylamine (891 mg, 1.20 mL, 2.00 eq) were dissolved in dichloromethane (2 mL). The mixture was then stirred at 20 °C for 12 hours.
LCMS detected the MS value of the product.
The reaction mixture was diluted with dichloromethane (50 mL) and water (30 mL). The aqueous phase was then extracted with dichloromethane (10 mL × 4). The organic phases were combined, washed with saturated brine (20 mL), dried over anhydrous sodium sulfate, filtered, and concentrated to obtain a crude product. The crude product was purified by column chromatography (petroleum ether:ethyl acetate = 20:1 to 1:1) to obtain 2-bromo-7-cyclopropyl-6,8-dihydro-5H-[1,2,4]triazolo[1,5-a]pyrazine (160 mg, 19.09% yield). MS (ESI) m/z = 243.0 $[M+1]^+$.

Step 4: Synthesis of 6'-(((1S,3S)-3-((7-cyclopropyl-5,6,7,8-tetrahydro-[1,2,4]triazolo[1,5-a]pyrazin-2-yl)amino)cyclo pentyl)amino)-2H-[1,3'-bipyridin]-2-one

2-Bromo-7-cyclopropyl-6,8-dihydro-5H-[1,2,4]triazolo[1,5-a]pyrazine (140 mg, 1.00 eq), 6'-(((1S,3S)-3-aminocyclopentyl)amino)-2H-[1,3'-bipyridin]-2-one (156 mg, 1.00 eq), sodium tert-butoxide (166 mg, 3.00 eq), and t-BuXPhos Pd G3 (45.7 mg, 0.10 eq) were dissolved in 1,4-dioxane (12 mL). The atmosphere was purged with nitrogen three times. The mixture was then heated to 90 °C and reacted for 12 hours.
LCMS detected the MS value of the product.
The reaction mixture was concentrated to obtain a crude product. The crude product was purified by reverse-phase preparative chromatography (Column: C18 150×30mm; Mobile phase: [water (FA)-ACN]; Gradient: 5% to 35% B over 7 min and Column: C18 150×30mm; Mobile phase: [water (FA)-ACN]; Gradient: 5% to 35% B over 7 min) to obtain 6'-(((1S,3S)-3-((7-cyclopropyl-5,6,7,8-tetrahydro-[1,2,4]triazolo[1,5-a]pyrazin-2-yl)amino)cyclo pentyl)amino)-2H-[1,3'-bipyridin]-2-one (20 mg, 8.03% yield).
LCMS (ESI) m/z = 433.2 $[M+1]^+$.
$^1$H NMR (400 MHz, $CDCl_3$): δ 8.03 (d, J = 2.4 Hz, 1H), 7.52 (dd, J = 2.4, 8.8 Hz, 1H), 7.39 (ddd, J = 2.4, 6.8, 9.2 Hz, 1H), 7.30 (dd, J = 2.0, 7.2 Hz, 1H), 6.65 (d, J = 9.2 Hz, 1H), 6.44 (d, J = 8.8 Hz, 1H), 6.23 (dt, J = 1.2, 6.8 Hz, 1H), 4.77 (br dd, J = 1.2, 5.6 Hz, 1H), 4.28 - 4.12 (m, 2H), 4.07 - 3.95 (m, 3H), 3.79 (s, 2H), 3.12 (t, J = 5.6 Hz, 2H), 2.39 - 2.23 (m, 2H), 2.11 - 1.97 (m, 2H), 1.95 - 1.86 (m, 1H), 0.62 - 0.55 (m, 2H), 0.54 - 0.48 (m, 2H).

Example C6

Synthesis of 1-(5-(((1S,3S)-3-((5,6,7,8-tetrahydro-[1,2,4]triazolo[1,5-a]pyridin-2-yl)amino)cyclopentyl)amino )pyrazin-2-yl)pyridin-2(1H)-one

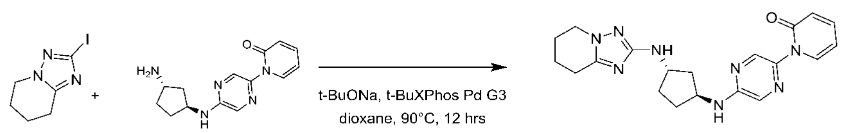


Step 1: Synthesis of 1-(5-(((1S,3S)-3-((5,6,7,8-tetrahydro-[1,2,4]triazolo[1,5-a]pyridin-2-yl)amino)cyclopentyl)amino ) pyrazin-2-yl)pyridin-2(1H)-one

2-Iodo-5,6,7,8-tetrahydro-[1,2,4]triazolo[1,5-a]pyridine (300 mg, 1.00 eq), 1-(5-(((1S,3S)-3-aminocyclopentyl) amino)pyrazin-2-yl)pyridin-2(1H)-one (327 mg, 1.00 eq), sodium tert-butoxide (347 mg, 3.61 mmol, 3.00 eq), and t-BuXPhos Pd G3 (95.7 mg, 0.10 eq) were dissolved in 1,4-dioxane (10 mL). The atmosphere was purged with nitrogen three times. The mixture was then heated to 90 °C and reacted for 12 hours.
LCMS detected the MS value of the product.
The reaction mixture was concentrated to obtain a crude product. The crude product was purified by reverse-phase preparative chromatography (Column: Waters Xbridge 150*25mm* 5um; Mobile phase: [water (ammonium hydroxide v/v)-ACN]; Gradient: 0% to 25% B over 10 min) to obtain 1-(5-(((1S,3S)-3-((5,6,7,8-tetrahydro-[1,2,4]triazolo[1,5-a]pyridin-2-yl)amino)cyclopentyl)amino )pyrazin-2-yl)pyridin-2(1H)-one (30 mg, 6.35% yield).
LCMS (ESI) m/z = 391.1 [M-1]+.
$^1$H NMR: (400 MHz, CDCl3): δ 7.76 (d, J = 1.2 Hz, 1H), 7.63 (dd, J = 2.0, 6.8 Hz, 1H), 7.38 (ddd, J = 2.0, 6.8, 9.2 Hz, 1H), 6.64 (d, J = 9.2 Hz, 1H), 6.32 - 6.22 (m, 1H), 4.93 (br d, J = 6.8 Hz, 1H), 4.41 - 4.29 (m, 1H), 4.24 - 4.12 (m, 1H), 4.04 (br

d, J = 6.8 Hz, 1H), 3.97 (t, J = 6.0 Hz, 2H), 2.78 (t, J = 6.4 Hz, 2H), 2.42 - 2.25 (m, 2H), 2.12 - 1.99 (m, 4H), 1.96 - 1.87 (m, 2H), 1.60 - 1.51 (m, 2H).

Example C7

Synthesis of 6'-(((1S,3S)-3-([1,2,4]triazolo[1,5-a]pyridin-2-ylamino)cyclopentyl)amino)-2H-[1,3'-bipyridin]-2 -one

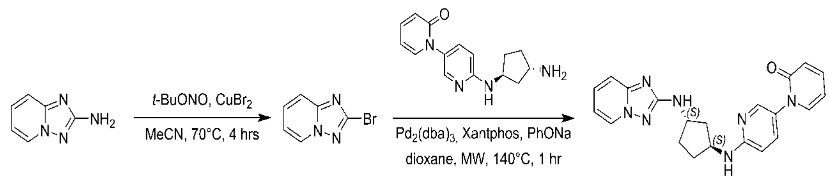

,
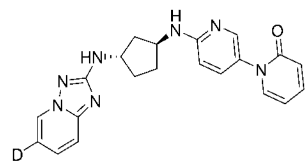

Step 1: Synthesis of 2-bromo-[1,2,4]triazolo[1,5-a]pyridine

[1,2,4]Triazolo[1,5-a]pyridin-2-amine (1.90 g, 14.2 mmol, 1.0 eq), copper(II) bromide (4.75 g, 21.3 mmol, 1.5 eq), and tert-butyl nitrite (2.19 g, 21.3 mmol, 1.50 eq) were dissolved in acetonitrile (30 mL). The mixture was stirred at 70 °C for 4 hours. TLC (petroleum ether/ethyl acetate = 1/1) indicated the completion of the reaction. The reaction mixture was cooled to room temperature, diluted with water (50 mL), and extracted with ethyl acetate (30 mL × 2). The organic phases were combined, washed with saturated brine (100 mL × 2), dried over anhydrous sodium sulfate, filtered, and concentrated to obtain 2-bromo-[1,2,4]triazolo[1,5-a]pyridine (2.17 g, 11.0 mmol, 77.4% yield). This was used directly in the next step without purification. LCMS MS (ESI) m/z = 200.0 $[M+1]^+$.

Step 2: Synthesis of 6'-(((1S,3S)-3-([1,2,4]triazolo[1,5-a]pyridin-2-ylamino)cyclopentyl)amino)-2H-[1,3'-bipyridin]-2 -one

2-Bromo-[1,2,4]triazolo[1,5-a]pyridine (100 mg, 505 μmol, 1.0 eq), 6'-(((1S,3S)-3-aminocyclopentyl)amino)-2H-[1,3'-bipyridin]-2-one (150 mg, 556 μmol, 1.1 eq), XantPhos (23.4 mg, 40.4 μmol, 0.08 eq), sodium phenoxide (87.9 mg, 758 μmol, 1.5 eq), and $Pd_2(dba)_3$ (18.5 mg, 20.2 μmol, 0.04 eq) were dissolved in 1,4-dioxane (4 mL). The mixture was stirred at 140 °C under microwave irradiation for 1 hour. LCMS detected the MS value of the product (RT = 0.347 min). The reaction mixture was filtered. The filtrate was concentrated to obtain a crude product. The crude product was first purified by reverse-phase preparative chromatography (Column: Waters Xbridge 150*25mm* 5um; Mobile phase: [water ($NH_4HCO_3$)-ACN]; Gradient: 12% to 42% B over 9 min) to obtain 6'-(((1S,3S)-3-([1,2,4]triazolo[1,5-a]pyridin-2-ylamino)cyclopentyl)amino)-2H-[1,3'-bipyridin]-2 -one (a total of 4 parallel batches under microwave irradiation, 83.1 mg, 211 μmol, 10.5% yield, 98.4% purity).

LCMS MS (ESI) m/z = 388.2 $[M+1]^+$.

$^1H$ NMR, (400 MHz, DMSO-$d_6$): δ 8.61 - 8.55 (m, 1H), 7.92 (d, J = 2.8 Hz, 1H), 7.60 (dd, J = 1.6, 6.8 Hz, 1H), 7.47 (ddd, J = 2.1, 6.7, 9.1 Hz, 1H), 7.44 - 7.34 (m, 2H), 6.92 (d, J = 6.9 Hz, 1H), 6.85 (dt, J = 1.6, 6.8 Hz, 1H), 6.64 (d, J = 7.3 Hz, 1H), 6.53 (d, J = 8.8 Hz, 1H), 6.44 (d, J = 8.8 Hz, 1H), 6.26 (dt, J = 1.3, 6.7 Hz, 1H), 4.40 - 4.26 (m, 1H), 4.18 (sext, J = 6.7 Hz, 1H), 2.21 - 2.09 (m, 2H), 2.00 - 1.84 (m, 2H), 1.63 - 1.43 (m, 2H).

Wherein, using this preparation method combined with a deuteration preparation method, the deuterated Compound C7A of Compound C7 was prepared, with the structure as follows:
Identification data: $^1H$ NMR (400 MHz, DMSO-$d_6$) δ 8.59 (s, 1H), 7.92 (d, J = 2.7 Hz, 1H), 7.61 (dd, J = 6.8, 2.1 Hz, 1H), 7.53 - 7.46 (m, 1H), 7.46 - 7.33 (m, 3H), 6.94 (d, J = 6.9 Hz, 1H), 6.66 (d, J = 7.3 Hz, 1H), 6.53 (d, J = 8.9 Hz, 1H), 6.49 - 6.40 (m, 1H), 6.27 (td, J = 6.7, 1.4 Hz, 1H), 4.33 (q, J = 6.6 Hz, 1H), 4.19 (q, J = 6.7 Hz, 1H), 2.15 (ddt, J = 12.1, 7.0, 4.5 Hz, 2H), 1.97 (dt, J = 13.4, 6.8 Hz, 1H), 1.93 - 1.83 (m, 1H), 1.65 - 1.40 (m, 2H).

Example C8

Synthesis of 6'-(((1S,3S)-3-((7-methoxy-[1,2,4]triazolo[1,5-a]pyridin-2-yl)amino)cyclopentyl)amino)-2H-[1,3 '-bipyridin]-2-one

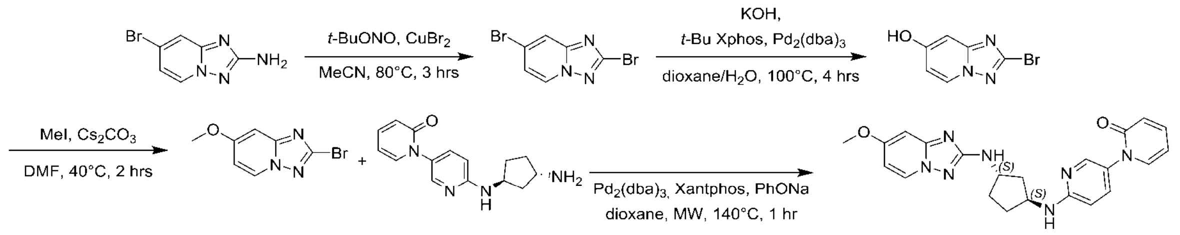


Step 1: Synthesis of 2,7-dibromo-[1,2,4]triazolo[1,5-a]pyridine

7-Bromo-[1,2,4]triazolo[1,5-a]pyridin-2-amine (2.00 g, 9.39 mmol, 1.0 eq), copper(II) bromide (3.15 g, 14.1 mmol, 1.5 eq), and tert-butyl nitrite (1.45 g, 14.1 mmol, 1.5 eq) were dissolved in acetonitrile (120 mL). The mixture was stirred at 80 °C for 3 hours. LCMS detected the MS value of the product (RT = 0.452 min). The reaction mixture was cooled to room temperature, diluted with water (200 mL), and extracted with ethyl acetate (80 mL × 2). The organic phases were combined, washed with saturated brine (200 mL × 2), dried over anhydrous sodium sulfate, filtered, and concentrated to obtain 2,7-dibromo-[1,2,4]triazolo[1,5-a]pyridine (2.00 g, 7.22 mmol, 76.9% yield). This was used directly in the next step without purification. LCMS MS (ESI) m/z = 277.9 $[M+1]^+$.

Step 2: Synthesis of 2-bromo-[1,2,4]triazolo[1,5-a]pyridin-7-ol

2,7-Dibromo-[1,2,4]triazolo[1,5-a]pyridine (2.00 g, 7.22 mmol, 1.0 eq), potassium hydroxide (1.20 g, 21.7 mmol, 3.0 eq), t-Bu XPhos (675 mg, 1.59 mmol, 0.22 eq), and Pd2(dba)3 (728 mg, 794 μmol, 0.11 eq) were dissolved in 1,4-dioxane (20 mL) and water (5 mL). The mixture was stirred at 100 °C for 4 hours. LCMS detected the MS value of the product (RT = 0.358 min). The reaction mixture was cooled to room temperature. Water (60 mL) and ethyl acetate (40 mL) were added to dilute the mixture. The organic phase was separated and discarded. The aqueous phase was adjusted to pH = 5 with dilute hydrochloric acid and then extracted with ethyl acetate (60 mL × 2). The organic phases were combined, washed with saturated brine (100 mL × 2), dried over anhydrous sodium sulfate, filtered, and concentrated to obtain 2-bromo-[1,2,4]triazolo[1,5-a]pyridin-7-ol (860 mg, 3.97 mmol, 54.9% yield, 98.7% purity). This was used directly in the next step without purification. LCMS MS (ESI) m/z = 215.9 $[M+1]^+$.

Step 3: Synthesis of 2-bromo-7-methoxy-[1,2,4]triazolo[1,5-a]pyridine

2-Bromo-[1,2,4]triazolo[1,5-a]pyridin-7-ol (1.30 g, 6.07 mmol, 1.0 eq) and cesium carbonate (4.95 g, 15.2 mmol, 2.5 eq) were dissolved in N,N-dimethylformamide (20 mL). Iodomethane (1.72 g, 12.2 mmol, 2.0 eq) was then added to the reaction mixture. After the addition, the mixture was stirred at 40 °C for 2 hours. TLC (petroleum ether/ethyl acetate = 3/1) indicated the completion of the reaction. The reaction mixture was cooled to room temperature, diluted with water (100 mL), and extracted with ethyl acetate (30 mL × 2). The organic phases were combined, washed with saturated brine (50 mL × 2), dried over anhydrous sodium sulfate, filtered, and concentrated to obtain a crude product. The crude product was purified by silica gel column chromatography to obtain 2-bromo-7-methoxy-[1,2,4]triazolo[1,5-a]pyridine (1.00 g, 4.39 mmol, 72.2% yield).

Step 4: Synthesis of 6'-(((1S,3S)-3-((7-methoxy-[1,2,4]triazolo[1,5-a]pyridin-2-yl)amino)cyclopentyl)amino)-2H-[1,3 '-bipyridin]-2-one

2-Bromo-7-methoxy-[1,2,4]triazolo[1,5-a]pyridine (100 mg, 439 μmol, 1 eq), 6'-(((1S,3S)-3-aminocyclopentyl) amino)-2H-[1,3'-bipyridin]-2-one (130 mg, 482 μmol, 1.1 eq), XantPhos (20.3 mg, 35.1 μmol, 0.08 eq), sodium phenoxide (76.4 mg, 658 μmol, 1.5 eq), and Pd2(dba)3 (16.1 mg, 17.5 μmol, 0.04 eq) were dissolved in 1,4-dioxane (5 mL). The mixture was stirred at 140 °C under microwave irradiation for 1 hour. LCMS detected the MS value of the product (RT = 0.366 min). The reaction mixture was filtered. The filtrate was concentrated to obtain a crude product. The crude product was first purified by reverse-phase preparative chromatography under acidic conditions (Column: Phenomenex luna C18 150*40mm* 15um; Mobile phase: [water (FA)-ACN]; Gradient: 20% to 50% B over 15 min), and then further purified by reverse-phase preparative chromatography under basic conditions (Column: Waters Xbridge 150*25mm* 5um; Mobile phase: [water (ammonium hydroxide v/v)-ACN]; Gradient: 0% to 30% B over 10 min) to obtain 6'-(((1S,3S)-3-((7-methoxy-[1,2,4]triazolo[1,5-a]pyridin-2-yl)amino)cyclopentyl)amino)-2H-[1,3 '-bipyridin]-2-one (a total of 8 parallel batches under microwave irradiation, 69.9 mg, 166 μmol, 4.72% yield, 98.9% purity).

LCMS MS (ESI) m/z = 418.2 $[M+1]^+$. $^1H$ NMR, (400 MHz, DMSO-$d_6$): δ 8.39 (d, J = 7.4 Hz, 1H), 7.91 (d, J = 2.6 Hz, 1H), 7.59 (dd, J = 1.7, 6.8 Hz, 1H), 7.47 (ddd, J = 2.0, 6.7, 9.1 Hz, 1H), 7.39 (dd, J = 2.6, 8.9 Hz, 1H), 6.89 (d, J = 6.9 Hz, 1H),

6.81 (d, J = 2.6 Hz, 1H), 6.58 - 6.38 (m, 4H), 6.26 (dt, J = 1.3, 6.7 Hz, 1H), 4.31 (sext, J = 6.4 Hz, 1H), 4.13 (sext, J = 6.7 Hz, 1H), 3.82 (s, 3H), 2.19 - 2.08 (m, 2H), 1.99 - 1.82 (m, 2H), 1.62 - 1.44 (m, 2H).

Example C9

Synthesis of 1-(5-(((1S,3S)-3-([1,2,4]triazolo[1,5-a]pyridin-2-ylamino)cyclopentyl)amino)pyrazin-2-yl)pyridi n-2(1H)-one

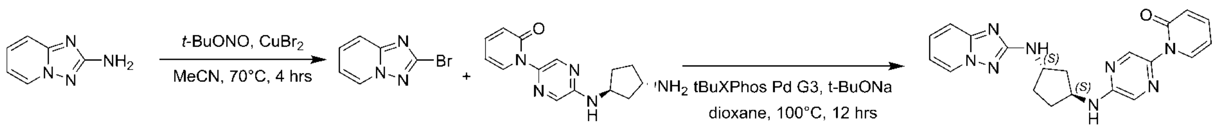


Step 1: Synthesis of 2-bromo-[1,2,4]triazolo[1,5-a]pyridine

[1,2,4]Triazolo[1,5-a]pyridin-2-amine (1.90 g, 14.2 mmol, 1.0 eq), copper(II) bromide (4.75 g, 21.3 mmol, 1.5 eq), and tert-butyl nitrite (2.19 g, 21.3 mmol, 1.50 eq) were dissolved in acetonitrile (30 mL). The mixture was stirred at 70 °C for 4 hours. TLC (petroleum ether/ethyl acetate = 1/1) indicated the completion of the reaction. The reaction mixture was cooled to room temperature, diluted with water (50 mL), and extracted with ethyl acetate (30 mL × 2). The organic phases were combined, washed with saturated brine (100 mL × 2), dried over anhydrous sodium sulfate, filtered, and concentrated to obtain 2-bromo-[1,2,4]triazolo[1,5-a]pyridine (2.17 g, 11.0 mmol, 77.4% yield). This was used directly in the next step without purification. LCMS MS (ESI) m/z = 200.0 $[M+1]^+$.

Step 2: Synthesis of 1-(5-(((1S,3S)-3-([1,2,4]triazolo[1,5-a]pyridin-2-ylamino)cyclopentyl)amino)pyrazin-2-yl)pyridi n-2(1H)-one

2-Bromo-[1,2,4]triazolo[1,5-a]pyridine (300 mg, 1.51 mmol, 1.0 eq), 1-(5-((((1S,3S)-3-aminocyclopentyl)amino) pyrazin-2-yl)pyridin-2(1H)-one (411 mg, 1.51 mmol, 1.0 eq), sodium tert-butoxide (437 mg, 4.54 mmol, 3.0 eq), and tBuXPhos Pd G3 (120 mg, 152 μmol, 0.10 eq) were dissolved in 1,4-dioxane (10 mL). The mixture was stirred at 100 °C for 12 hours. LCMS detected the MS value of the product (RT = 0.394 min). The reaction mixture was filtered. The filtrate was concentrated to obtain a crude product. The crude product was purified by reverse-phase preparative chromatography (Column: Phenomenex luna C18 150*40mm* 15um; Mobile phase: [water (FA)-ACN]; Gradient: 10% to 40% B over 15 min) to obtain 1-(5-(((1S,3S)-3-([1,2,4]triazolo[1,5-a]pyridin-2-ylamino)cyclopentyl)amino)pyrazin-2-yl)pyridi n-2(1H)-one (79.6 mg, 201 μmol, 13.2% yield, 97.9% purity).

LCMS MS (ESI) m/z = 389.2 $[M+1]^+$. $^1H$ NMR, (400 MHz, DMSO-$d_6$): δ 8.62 - 8.54 (m, 1H), 8.25 (d, J = 1.4 Hz, 1H), 7.87 (d, J = 1.3 Hz, 1H), 7.72 (dd, J = 1.5, 6.9 Hz, 1H), 7.56 - 7.32 (m, 4H), 6.87 (dt, J = 1.5, 6.8 Hz, 1H), 6.66 (d, J = 7.3 Hz, 1H), 6.47 (d, J = 9.0 Hz, 1H), 6.32 (dt, J = 1.3, 6.7 Hz, 1H), 4.41 - 4.15 (m, 2H), 2.25 - 2.11 (m, 2H), 2.06 - 1.86 (m, 2H), 1.67 - 1.47 (m, 2H).

Example C10

Synthesis of 1-(5-(((1S,3S)-3-((6,7-dihydro-5H-[1,2,4]triazolo[5,1-b][1,3]thiazin-2-yl)amino)cyclopentyl)ami no)pyrazin-2-yl)pyridin-2(1H)-one

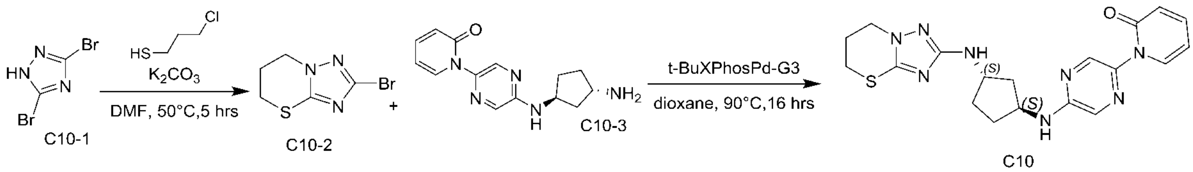


Step 1: Synthesis of 2-bromo-6,7-dihydro-5H-[1,2,4]triazolo[5,1-b][1,3]thiazine

Compound C10-1 (3.00 g, 13.2 mmol, 1.00 eq), 3-chloropropane-1-thiol (1.76 g, 15.8 mmol, 1.55 mL, 1.20 eq),

and $K_2CO_3$ (3.60 g, 26.5 mmol, 2.00 eq) were dissolved in DMF (20 mL). The reaction mixture was stirred at 50 °C for 5 hours.

LCMS detected the formation of the target product. 200 mL water was added to quench the reaction. The mixture was extracted with ethyl acetate (100 mL × 3). The organic phases were combined, washed with saturated brine (100 mL × 2), dried over anhydrous sodium sulfate, filtered, and concentrated. The solid of organic phases reaction mixture was purified by normal-phase silica gel column chromatography (petroleum ether:ethyl acetate = 1:1) to obtain Compound C10-2 (800 mg, 3.63 mmol, yield: 27.5%) as a white solid.

Step 2: Synthesis of 1-(5-(((1S,3S)-3-((6,7-dihydro-5H-[1,2,4]triazolo[5,1-b][1,3]thiazin-2-yl)amino)cyclopentyl)ami no) pyrazin-2-yl)pyridin-2(1H)-one

Under nitrogen protection, Compound C10-3 (300 mg, 1.11 mmol, 1.00 eq), Compound C10-2 (4988 mg, 2.27 mmol, 2.05 eq), t-BuONa (318 mg, 3.32 mmol, 3.00 eq), and [2-(2-aminophenyl)phenyl]methylsulfonyloxy-palladium; ditert-butyl-[2-(2,4,6-triisopropylphenyl)phenyl]phosphine (175 mg, 221 μmol, 0.20 eq) were dissolved in 1,4-dioxane (20 mL). The reaction mixture was stirred at 90 °C for 16 hours.

LCMS and HPLC detected the formation of the target product. 200 mL water was added to quench the reaction. The mixture was extracted with ethyl acetate (100 mL × 3). The organic phases were combined, washed with saturated brine (100 mL × 2), dried over anhydrous sodium sulfate, filtered, and concentrated. The organic phase was purified by reverse-phase preparative chromatography (Column: Phenomenex Luna C18 150*25mm*10um; Mobile phase: [water (TFA)-ACN]; Gradient: 12% to 42% B over 11 min) to obtain the target Compound (60.0 mg, 146 μmol, yield: 13.2%) as a pale yellow gummy compound. LC-MS: $[M+H]^+$ = 411.

$^1$H NMR (400 MHz, Chloroform-d) δ 8.52 (s, 1H), 7.73 (d, J = 1.6 Hz, 1H), 7.62 (dd, J = 7.2, 2.1 Hz, 1H), 7.41 - 7.33 (m, 1H), 6.62 (d, J = 9.3 Hz, 1H), 6.33 - 6.21 (m, 1H), 5.01 (d, J = 6.8 Hz, 1H), 4.31 (q, J = 6.6 Hz, 1H), 4.22 - 4.04 (m, 4H), 3.23 - 3.06 (m, 2H), 2.43 - 2.22 (m, 4H), 2.07 - 1.99 (m, 2H), 1.63 - 1.49 (m, 3H).

Example C11

Synthesis of 1-(5-(((1S,3S)-3-((4-oxido-6,7-dihydro-5H-[1,2,4]triazolo[5,1-b][1,3]thiazin-2-yl)amino)cyclope ntyl)amino) pyrazin-2-yl)pyridin-2(1H)-one

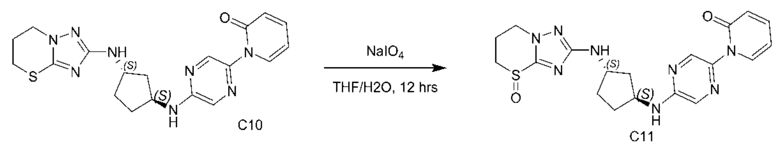


Compound C10 (20.0 mg, 48.7 μmol, 1.00 eq) was dissolved in THF (8.00 mL). $NaIO_4$ (10.4 mg, 48.7 μmol, 2.70 μL, 1.00 eq) dissolved in $H_2O$ (3 mL) was added. The reaction mixture was stirred at 70 °C for 12 hours.

LCMS and HPLC detected the formation of the target product. The reaction mixture was directly concentrated under reduced pressure. The compound was purified by reverse-phase preparative chromatography (Column: Phenomenex Luna C18 150*25mm*10um; Mobile phase: [water (TFA)-ACN]; Gradient: 10% to 40% B over 11 min) to obtain the target Compound (18.0 mg, 42.2 μmol, yield: 86.6%) as a white solid. LC-MS: $[M-H]^-$ = 427.1.

$^1$H NMR, (CHLOROFORM-d, 400 MHz): δ 8.54 (dd, 1H, J =1.0, 4.3 Hz), 8.04 (d, 1H, J =5.6 Hz), 7.7-7.8 (m, 1H), 7.52 (ddd, 1H, J =1.9, 6.9, 9.0 Hz), 6.81 (d, 1H, J =9.1 Hz), 6.46 (dt, 1H, J =0.9, 6.8 Hz), 4.3-4.4 (m, 2H), 4.1-4.2 (m, 2H), 3.42 (dd, 1H, J=5.9, 12.6 Hz), 2.9-3.2 (m, 2H), 2.3-2.5 (m, 3H), 2.0-2.2 (m, 2H), 1.6-1.8 (m, 2H).

Example C12

Referring to the preparation methods of the above Examples C1 and C3, Compounds C12, C12A, and C12B were prepared. The synthetic route is as follows:

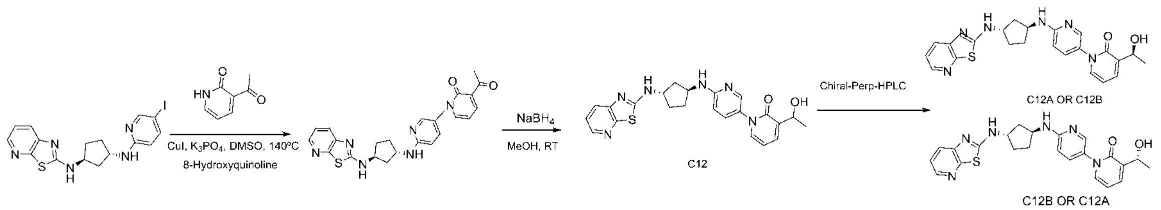


Compound C12 was subjected to chiral separation (Column: CHIRALCEL OD-H 5um 10mm*250mm; Mobile phase: n-hexane/ethanol = 70:30; Flow rate: 5 mL/min; Column temperature: 30 °C) to obtain white solids C12A and C12B: Compound C12A, $^1H$ NMR: (400 MHz, DMSO-$d_6$): δ 8.59 - 8.33 (m, 1H), 8.08 (dd, J = 1.6, 4.8 Hz, 1H), 7.92 (d, J = 2.4 Hz, 1H), 7.66 (dd, J = 1.4, 8.0 Hz, 1H), 7.55 - 7.48 (m, 2H), 7.40 (dd, J = 2.8, 8.9 Hz, 1H), 7.24 (dd, J = 4.8, 8.0 Hz, 1H), 6.96 (d, J = 6.9 Hz, 1H), 6.53 (d, J = 8.9 Hz, 1H), 6.31 (t, J = 6.8 Hz, 1H), 5.06 (br s, 1H), 4.79 - 4.62 (m, 1H), 4.37 (dt, J = 6.4, 13.8 Hz, 2H), 2.27 - 2.12 (m, 2H), 2.04 - 1.90 (m, 2H), 1.65 - 1.48 (m, 2H), 1.26 (d, J = 6.4 Hz, 3H).

Compound C12B, $^1H$ NMR: (400 MHz, DMSO-$d_6$): δ 8.54 - 8.37 (m, 1H), 8.08 (dd, J = 1.4, 4.8 Hz, 1H), 7.92 (d, J = 2.6 Hz, 1H), 7.66 (dd, J = 1.4, 8.1 Hz, 1H), 7.55 - 7.48 (m, 2H), 7.40 (dd, J = 2.6, 8.9 Hz, 1H), 7.24 (dd, J = 4.8, 8.0 Hz, 1H), 6.96 (d, J = 6.9 Hz, 1H), 6.53 (d, J = 8.9 Hz, 1H), 6.31 (t, J = 6.8 Hz, 1H), 5.06 (d, J = 4.6 Hz, 1H), 4.75 - 4.67 (m, 1H), 4.44 - 4.29 (m, 2H), 2.27 - 2.13 (m, 2H), 2.03 - 1.91 (m, 2H), 1.66 - 1.49 (m, 2H), 1.25 (d, J = 6.4 Hz, 3H).

Example C45

Synthesis of 6'-(((1S,3S)-3-((7,8-dihydro-[1,4]dioxino[2,3-d][1,2,4]triazolo[1,5-a]pyridin-2-yl)amino)cyclope ntyl)amino)-2H-[1,3'-bipyridin]-2-one

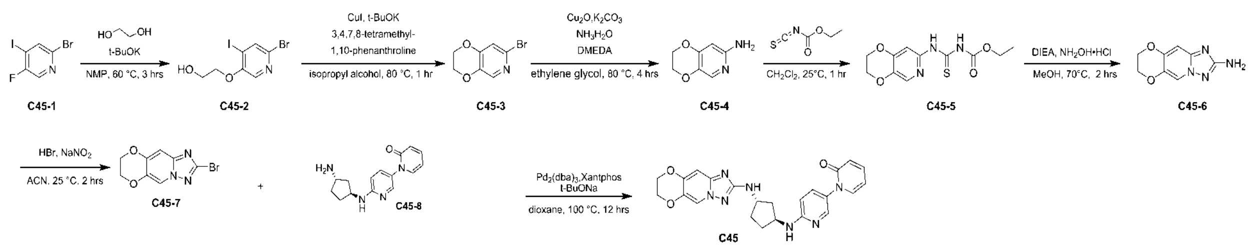


Step 1: Synthesis of 2-((6-bromo-4-iodopyridin-3-yl)oxy)ethan-1-ol

To a solution of C45-1 (8.00 g, 26.5 mmol, 1.00 eq) and ethylene glycol (55.6 g, 896 mmol, 50 mL, 33.8 eq) in NMP (50 mL) was added t-BuOK (5.95 g, 53.0 mmol, 2.00 eq). The mixture was stirred at 60 °C for 3 hours.

LCMS detected the formation of the target product. The reaction mixture was poured into water and extracted with ethyl acetate. The organic phase was washed with saturated sodium chloride solution, dried over anhydrous sodium sulfate, filtered, and concentrated and dried by rotary evaporation. The crude product was purified by reverse-phase HPLC (0.1% FA condition) to obtain Compound C45-2 (3.50 g, 10.1 mmol, 38.4% yield) as a white solid.

Step 2: Synthesis of 7-bromo-2,3-dihydro-[1,4]dioxino[2,3-c]pyridine

To a solution of C45-2 (3.50 g, 10.1 mmol, 1.00 eq) in isopropanol (35 mL) was added CuI (116 mg, 610 μmol, 0.06 eq), t-BuOK (1.60 g, 14.2 mmol, 1.40 eq), and 3,4,7,8-tetramethyl-1,10-phenanthroline (192 mg, 814 μmol, 0.08 eq). Under nitrogen protection, the mixture was stirred at 80 °C for 1 hour.

LCMS detected the formation of the target product. The reaction mixture was poured into water and extracted with ethyl acetate. The organic phase was washed with saturated sodium chloride solution, dried over anhydrous sodium sulfate, filtered, and concentrated and dried by rotary evaporation to obtain Compound C45-3 (2.20 g, 9.12 mmol, 89.6% yield) as a brown solid.

Step 3: Synthesis of 2,3-dihydro-[1,4]dioxino[2,3-c]pyridin-7-amine

$Cu_2O$ (14.5 mg, 101 μmol, 10.4 μL, 0.01 eq), $NH_3H_2O$ (50.9 g, 407 mmol, 56.0 mL, 40.0 eq), $K_2CO_3$ (281 mg, 2.04 mmol, 0.200 eq), and N',N'-dimethylethane-1,2-diamine (89.7 mg, 1.02 mmol, 111 μL, 0.100 eq) were added to a solution

of C45-3 (2.20 g, 10.1 mmol, 1.00 eq) in ethylene glycol (20 mL). The mixture was stirred at 80 °C for 4 hours.

LCMS detected the formation of the target product. The reaction mixture was poured into water and extracted with ethyl acetate. The organic phase was washed with saturated sodium chloride solution, dried over anhydrous sodium sulfate, filtered, and concentrated to dryness to obtain Compound C45-4 (1.50 g, crude) as a yellow oily product.

Step 4: Synthesis of ethyl N-((2,3-dihydro-[1,4]dioxino[2,3-c]pyridin-7-yl)carbamothioyl)carbamate

Ethyl N-(thioiminocarbamate) (1.29 g, 9.86 mmol, 1.00 eq) was added to a solution of C45-4 (1.50 g, 9.86 mmol, 1.00 eq) in dichloromethane (15 mL). The mixture was stirred at 25 °C for 1 hour.

LCMS detected the formation of the target product. The reaction mixture was poured into water and extracted with dichloromethane. The organic phase was washed with saturated sodium chloride solution, dried over anhydrous sodium sulfate, filtered, and concentrated and dried by rotary evaporation to obtain Compound C45-5 (2.20 g, 7.77 mmol, 78.7% yield) as a yellow solid.

Step 5: Synthesis of 7,8-dihydro-[1,4]dioxino[2,3-d][1,2,4]triazolo[1,5-a]pyridin-2-amine

$NH_2OH.HCl$ (2.43 g, 34.9 mmol, 4.50 eq) and DIEA (3.01 g, 23.30 mmol, 4.06 mL, 3 eq) were added to a solution of C45-5 (2.20 g, 7.77 mmol, 1.00 eq) in methanol (15 mL). The mixture was stirred at 70 °C for 2 hours.

LCMS detected the formation of the target product. The reaction mixture was cooled to 25 °C and filtered. The filter cake was Compound C45-6 (1.30 g, 6.76 mmol, 87.1% yield) as a white solid.

Step 6: Synthesis of 2-bromo-7,8-dihydro-[1,4]dioxino[2,3-d][1,2,4]triazolo[1,5-a]pyridine

At 0 °C, $NaNO_2$ (359 mg, 5.20 mmol, 2.00 eq) and HBr (1.21 g, 5.98 mmol, 812 μL, 2.30 eq) were added to a solution of C45-6 (500 mg, 2.60 mmol, 1.00 eq) in acetonitrile (5 mL). The mixture was stirred at 25 °C for 2 hours.

LCMS detected the formation of the target product. The reaction mixture was poured into water and extracted with ethyl acetate. The organic phase was washed with saturated sodium chloride solution, dried over anhydrous sodium sulfate, filtered, and concentrated and dried by rotary evaporation to obtain Compound C45-7 (300 mg, 1.17 mmol, 45.0% yield) as a yellow solid.

Step 7: Synthesis of 6'-(((1S,3S)-3-((7,8-dihydro-[1,4]dioxino[2,3-d][1,2,4]triazolo[1,5-a]pyridin-2-yl)amino)cyclope ntyl)amino)-2H-[1,3'-bipyridin]-2-one

t-BuONa (225 mg, 2.34 mmol, 2.00 eq), $Pd_2(dba)_3$ (107 mg, 117 μmol, 0.100 eq), and XantPhos (135 mg, 234 μmol, 0.200 eq) were added to a solution of C45-7 (300 mg, 1.17 mmol, 1.00 eq) and C45-8 (316 mg, 1.17 mmol, 1.00 eq) in 1,4-dioxane (5 mL). The mixture was stirred under nitrogen protection at 100 °C for 12 hours.

LCMS detected the formation of the target product. The reaction mixture was poured into water and extracted with ethyl acetate. The organic phase was washed with saturated sodium chloride solution, dried over anhydrous sodium sulfate, filtered, and concentrated and dried by rotary evaporation. The crude product was purified by reverse-phase preparative chromatography (Column: Waters Xbridge Prep OBD C18 150*40mm*10um; Mobile phase: [water ($NH_4HCO_3$)-ACN]; Gradient: 10% to 40% B over 52 min) and (Column: Phenomenex luna C18 150*25mm* 10um; Mobile phase: [water (FA)-ACN]; Gradient: 1% to 25% B over 10 min) to obtain the target Compound (36.6 mg).

LC-MS: $[M+H]^+$ = 446.1. $^1H$ NMR: (DMSO-$d_6$, 400 MHz): δ (ppm) = 8.38 (s, 1H), 7.91 (d, J = 2.4 Hz, 1H), 7.60 (dd, J = 2.0, 6.8 Hz, 1H), 7.49 - 7.45 (m, 1H), 7.39 (dd, J = 2.8, 8.8 Hz, 1H), 6.90 (d, J = 6.8 Hz, 1H), 6.78 (s, 1H), 6.52 (d, J = 9.2 Hz, 1H), 6.44 (d, J = 9.2 Hz, 1H), 6.36 (d, J = 7.2 Hz, 1H), 6.26 (dt, J = 1.2, 6.8 Hz, 1H), 4.37 - 4.24 (m, 5H), 4.18 - 4.05 (m, 1H), 2.20 - 2.06 (m, 2H), 1.99 - 1.78 (m, 2H), 1.64 - 1.41 (m, 2H).

Example C46

Synthesis of 6'-(((1S,3S)-3-((7,8-dihydro-[1,4]dioxino[2,3-d][1,2,4]triazolo[1,5-a]pyridin-2-yl)amino)cyclope ntyl)amino)-5'-fluoro-2H-[1,3'-bipyridin]-2-one

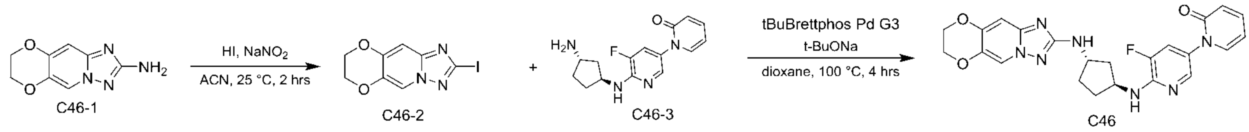


Step 1: Synthesis of 2-iodo-7,8-dihydro-[1,4]dioxino[2,3-d][1,2,4]triazolo[1,5-a]pyridine

At 0 °C, $NaNO_2$ (215 mg, 3.12 mmol, 2.00 eq) and HI (1.02 g, 3.59 mmol, 600 μL, 2.30 eq) were added to a solution of C46-1 (300 mg, 1.56 mmol, 1.00 eq) in acetonitrile (5 mL). The mixture was stirred at 25 °C for 2 hours.

LCMS detected the formation of the target product. The reaction mixture was poured into water and extracted with ethyl acetate. The organic phase was washed with aqueous sodium bicarbonate, dried over anhydrous sodium sulfate, filtered, and concentrated and dried by rotary evaporation to obtain Compound C46-2 (480 mg, 1.11 mmol, 71.0% yield) as a brown solid.

Step 2: Synthesis of 6'-(((1S,3S)-3-((7,8-dihydro-[1,4]dioxino[2,3-d][1,2,4]triazolo[1,5-a]pyridin-2-yl)amino)cyclopentyl)amino)-5'-fluoro-2H-[1,3'-bipyridin]-2-one

t-BuONa(190.26 mg, 1.98 mmol, 2 eq) and tBuBrettPhos Pd G3 (84.5 mg, 98.9 μmol, 0.100 eq) were added to a solution of C46-2 (300 mg, 989 μmol, 1.00 eq) and C46-3 (285 mg, 989 μmol, 1.00 eq) in 1,4-dioxane (5 mL). Under nitrogen protection, the mixture was stirred at 100 °C for 4 hours.

LCMS detected the formation of the target product. The reaction mixture was filtered, and the filtrate was concentrated and dried by rotary evaporation. The crude product was purified by reverse-phase preparative chromatography (Column: Waters Xbridge Prep OBD C18 150*40 mm*10 um; Mobile phase: [water ($NH_4HCO_3$)-ACN]; Gradient: 16% to 46% B over 15 min) to obtain the target Compound (20.0 mg).

LC-MS: $[M+H]^+$ = 464.1. $^1$H NMR: (DMSO-$d_6$, 400 MHz): δ (ppm) = 8.38 (s, 1H), 7.84 (d, J = 2.0 Hz, 1H), 7.64 (dd, J = 1.6, 6.8 Hz, 1H), 7.57 - 7.45 (m, 2H), 6.90 (br d, J = 7.2 Hz, 1H), 6.78 (s, 1H), 6.46 (d, J = 8.8 Hz, 1H), 6.35 (d, J = 7.2 Hz, 1H), 6.28 (t, J = 6.4 Hz, 1H), 4.57 - 4.47 (m, 1H), 4.35 (br dd, J = 2.0, 5.2 Hz, 2H), 4.31 - 4.24 (m, 2H), 4.17 - 4.07 (m, 1H), 2.18 - 2.06 (m, 2H), 1.99 - 1.90 (m, 2H), 1.63 - 1.50 (m, 2H).

Example C47

Synthesis of 2-(6-(((1S,3S)-3-([1,2,4]triazolo[1,5-a]pyridin-2-ylamino)cyclopentyl)amino)-5-fluoropyridin-3-yl)pyridazin-3(2H)-one

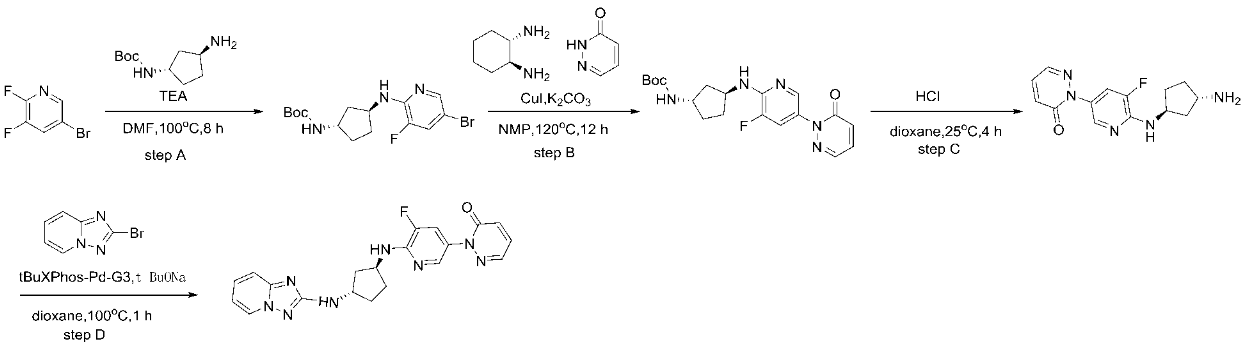


Step A: Synthesis of tert-butyl ((1S,3S)-3-((5-bromo-3-fluoropyridin-2-yl)amino)cyclopentyl)carbamate

At room temperature, 5-bromo-2,3-difluoropyridine (100.0 g, 0.52 mol) and tert-butyl ((1S,3S)-3-aminocyclopentyl)carbamate (114.58 g, 0.57 mol) were added to DMF (800 mL). Triethylamine (156.99 g, 1.55 mol) was then added to the system. The mixture was heated to 100 °C and reacted for 8 hours.

After completion of the reaction, the mixture was cooled to room temperature. Water (500 mL) was added to the system, followed by extraction with ethyl acetate (400 mL × 3). The organic phases were combined, washed twice with saturated brine (300 mL), dried, filtered, and concentrated under reduced pressure. The residue was triturated with isopropyl ether (300 mL) to obtain 130 g of tert-butyl ((1S,3S)-3-((5-bromo-3-fluoropyridin-2-yl)amino)cyclopentyl)carbamate as a white solid (yield 67%). LC-MS: [M+H]+ = 374.

Step B: Synthesis of tert-butyl ((1s,3s)-3-((3-fluoro-5-(6-oxopyridazin-1(6h)-yl)pyridin-2-yl)amino)cyclopentyl)carbamate

At room temperature, tert-butyl ((1S,3S)-3-((5-bromo-3-fluoropyridin-2-yl)amino)cyclopentyl)carbamate (120 g, 0.32 mol), pyridazin-3(2H)-one (40 g, 0.41 mol), (1S,2S)-$N^1$,$N^2$-dimethylcyclohexane-1,2-diamine (9.1 g, 0.064 mol), and potassium carbonate (133.19 g, 0.96 mol) were added to NMP (960 mL). CuI (12.3 g, 0.064 mol) was then added. After the addition, the system was purged with nitrogen and the mixture was heated to 120 °C and reacted for 12 hours.

After completion of the reaction, the mixture was cooled to room temperature. Water (1000 mL) was added to the system, followed by extraction with ethyl acetate (400 mL × 3). The organic phases were combined, washed three times with saturated brine (400 mL), dried, filtered, and concentrated under reduced pressure. The residue was purified by normal-phase column chromatography (dichloromethane/methanol = 50:1) to obtain 70 g of tert-butyl ((1S,3S)-3-((3-fluoro-5-(6-oxopyridazin-1(6H)-yl)pyridin-2-yl)amino)cyclopentyl)carbamate as a black oil (yield 56%). LC-MS: [M+H]+ = 390.

Step C: Synthesis of 2-(6-(((1S,3S)-3-aminocyclopentyl)amino)-5-fluoropyridin-3-yl)pyridazin-3(2H)-one

At room temperature, tert-butyl ((1S,3S)-3-((3-fluoro-5-(6-oxopyridazin-1(6H)-yl)pyridin-2-yl)amino)cyclopentyl)carbamate (70 g, 0.17 mol) was dissolved in a hydrochloric acid/1,4-dioxane solution (2 M, 300 mL), reacted at room temperature for 4 hours.

After completion of the reaction, the mixture was concentrated to dryness to obtain 2-(6-(((1S,3S)-3-aminocyclopentyl)amino)-5-fluoropyridin-3-yl)pyridazin-3(2H)-one hydrochloride. This was dissolved in methanol (200 mL), and the pH of the system was adjusted to approximately 9 using a basic ion exchange resin. The mixture was filtered, and the filter cake was washed three times with methanol (100 mL). The filtrates were combined and concentrated to dryness to obtain 55 g of 2-(6-(((1S,3S)-3-aminocyclopentyl)amino)-5-fluoropyridin-3-yl)pyridazin-3(2H)-one as a brown solid. This was used directly in the next step without further treatment. LC-MS: [M+H]+ = 290.

Step D: Synthesis of 2-(6-(((1S,3S)-3-([1,2,4]triazolo[1,5-a]pyridin-2-ylamino)cyclopentyl)amino)-5-fluoropyridin-3-yl) pyridazin-3(2H)-one

At room temperature, 2-(6-(((1S,3S)-3-aminocyclopentyl)amino)-5-fluoropyridin-3-yl)pyridazin-3(2H)-one (55 g, 0.19 mol) and 2-bromo-[1,2,4]triazolo[1,5-a]pyridine (37.4 g, 0.19 mol) were dissolved in 1,4-dioxane (550 mL). Sodium tert-butoxide (36.5 g, 0.38 mol) and methanesulfonato(2-di-tert-butylphosphino-2',4',6'-triisopropyl-1,1'-biphenyl)(2'-amino-1,1'-bi phenyl-2-yl)palladium(II) (30 g, 0.037 mol) were then added. After the addition, the system was purged with nitrogen, and the mixture was slowly heated to 100 °C and reacted for 1 hour.

After completion of the reaction, the mixture was cooled to room temperature. Water (400 mL) was added to the system, followed by extraction with ethyl acetate (300 mL × 3). The organic phases were combined, washed with saturated brine (200 mL), dried, filtered, and concentrated under reduced pressure. The residue was purified by normal-phase column chromatography (dichloromethane/methanol = 10:1) to obtain 20.5 g of 2-(6-(((1S,3S)-3-([1,2,4]triazolo [1,5-a]pyridin-2-ylamino)cyclopentyl)amino)-5-fluoropyridin-3-yl)pyridazin-3(2H)-one. LC-MS: $[M+H]^+$ = 407.

$^1$H NMR (400 MHz, DMSO-$d_6$) δ 8.61 - 8.58 (dd, J = 6.7, 1.1 Hz, 1H), 8.06 - 8.04 (q, J = 1.7 Hz, 2H), 7.67 - 7.61 (dd, J = 12.1, 2.2 Hz, 1H), 7.52 - 7.47 (dd, J = 9.5, 3.9 Hz, 1H), 7.46 - 7.41 (m, 1H), 7.40 - 7.37 (dt, J = 8.8, 1.3 Hz, 1H), 7.09 - 7.05 (dd, J = 9.5, 1.6 Hz, 1H), 6.99 - 6.94 (m, 1H), 6.90 - 6.84 (td, J = 6.7, 1.6 Hz, 1H), 6.68 - 6.63 (d, J = 7.2 Hz, 1H), 4.61 - 4.51 (p, J = 7.0 Hz, 1H), 4.26 - 4.17 (p, J = 6.6 Hz, 1H), 2.22 - 2.12 (m, 2H), 2.02 - 1.97 (m, 2H), 1.67 - 1.54 (m, 2H).

Example C54

Synthesis of 2-(6-(((1S,3S)-3-((7,8-dihydro-[1,4]dioxino[2,3-d][1,2,4]triazolo[1,5-a]pyridin-2-yl)amino)cyclo pentyl)amino)pyridin-3-yl)pyridazin-3(2H)-one

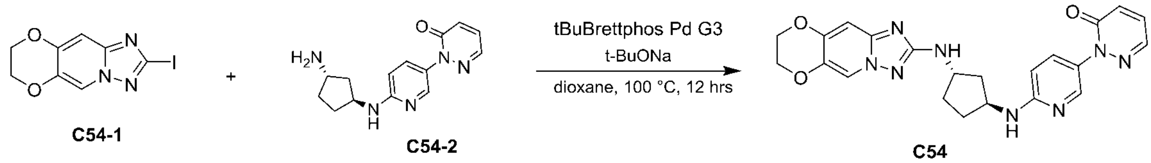


C54-1 C54-2 C54

Step 1: Synthesis of 2-(6-(((1S,3S)-3-((7,8-dihydro-[1,4]dioxino[2,3-d][1,2,4]triazolo[1,5-a]pyridin-2-yl)amino)cyclo pentyl)amino)pyridin-3-yl)pyridazin-3(2H)-one

Sodium tert-butoxide (190 mg, 1.98 mmol, 5.00 eq) and tBuBrettPhos Pd G3 (33.8 mg, 39.60 μmol, 0.100 eq) was added to a solution of C54-1 (120 mg, 395 μmol, 1.00 eq) and C54-2 (107 mg, 395 μmol, 1.00 eq) in 1,4-dioxane (4 mL). Under nitrogen protection, the mixture was stirred at 100 °C for 12 hours.

LCMS detected the formation of the target product. The reaction mixture was filtered, and the filtrate was concentrated to dryness. The crude product was purified by reverse-phase preparative chromatography (Column: Phenomenex luna C18 150*25 mm* 10um; Mobile phase: [water (FA)-ACN]; Gradient: 1% to 30% B over 10 min) to obtain the target Compound (18.6 mg).

LC-MS: [M+H]+ = 447.1. $^1$H NMR: (DMSO-$d_6$, 400 MHz): δ (ppm) = 8.37 (s, 1H), 8.09 (d, J = 2.4 Hz, 1H), 8.01 (dd, J = 1.6, 4.0 Hz, 1H), 7.51 (dd, J = 2.4, 8.8 Hz, 1H), 7.46 (dd, J = 4.0, 9.6 Hz, 1H), 7.02 (dd, J = 1.6, 9.6 Hz, 1H), 6.91 (d, J = 7.2 Hz, 1H), 6.77 (s, 1H), 6.52 (d, J = 8.8 Hz, 1H), 6.35 (d, J = 7.6 Hz, 1H), 4.37 - 4.25 (m, 5H), 4.16 - 4.07 (m, 1H), 2.18 - 2.08 (m, 2H), 1.99 - 1.83 (m, 2H), 1.56 - 1.44 (m, 2H).

Example C13

Referring to the preparation methods of the aforementioned Examples C1, C3, and C12, Compounds C13, C13A, and C13B were prepared:

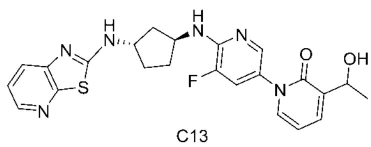

C13

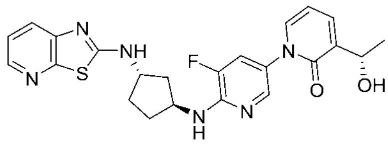

C13A or C13B

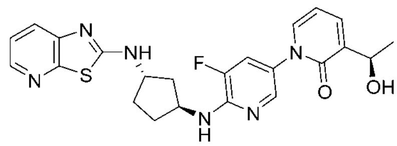

C13B or C13A

Compound C13A, $^1$H NMR: (400 MHz, DMSO-$d_6$): δ 8.49 (br d, J = 6.4 Hz, 1H), 8.16 - 8.08 (m, 1H), 7.89 (d, J = 2.0 Hz, 1H), 7.70 (dd, J = 1.2, 8.0 Hz, 1H), 7.62 - 7.55 (m, 3H), 7.28 (dd, J = 4.8, 8.0 Hz, 1H), 7.03 (br d, J = 7.2 Hz, 1H), 6.37 (t, J = 6.8 Hz, 1H), 5.12 (d, J = 4.4 Hz, 1H), 4.76 (quintet, J = 5.6 Hz, 1H), 4.66 - 4.53 (m, 1H), 4.51 - 4.39 (m, 1H), 2.31 - 2.16 (m, 2H), 2.11 - 2.02 (m, 2H), 1.74 - 1.60 (m, 2H), 1.30 (d, J = 6.4 Hz, 3H).

Compound C13B, $^1$H NMR: (400 MHz, DMSO-$d_6$): δ 8.45 (br d, J = 4.4 Hz, 1H), 8.08 (dd, J = 1.2, 4.8 Hz, 1H), 7.84 (d, J = 2.0 Hz, 1H), 7.66 (dd, J = 1.2, 8.0 Hz, 1H), 7.60 - 7.51 (m, 3H), 7.24 (dd, J = 4.8, 8.0 Hz, 1H), 6.98 (br d, J = 6.8 Hz, 1H), 6.33 (t, J = 6.8 Hz, 1H), 5.08 (d, J = 4.4 Hz, 1H), 4.80 - 4.65 (m, 1H), 4.55 (sextet, J = 7.2 Hz, 1H), 4.46 - 4.35 (m, 1H), 2.27 - 2.15 (m, 2H), 2.02 (t, J = 7.2 Hz, 2H), 1.69 - 1.56 (m, 2H), 1.26 (d, J = 6.4 Hz, 3H).

Example C14

Referring to the preparation method of the aforementioned Example C2, Compound C14 was prepared:

| | |
|---|---|
| 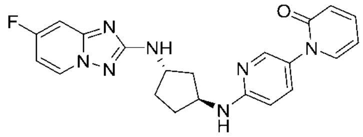 C14 | $^1$H NMR (DMSO-$d_6$, 400 MHz) δ 8.65 (dd, 1H, J=5.7, 7.3 Hz), 7.92 (d, 1H, J=2.6 Hz), 7.59 (dd, 1H, J=1.7, 6.8 Hz), 7.47 (ddd, 1H, J=2.1, 6.7, 9.1 Hz), 7.39 (dd, 1H, J=2.7, 8.8 Hz), 7.26 (dd, 1H, J=2.7, 9.7 Hz), 6.91 (d, 1H, J=6.9 Hz), 6.86 (dt, 1H, J=2.8, 7.6 Hz), 6.74 (d, 1H, J=7.1 Hz), 6.52 (d, 1H, J=9.0 Hz), 6.44 (d, 1H, J=9.0 Hz), 6.26 (dt, 1H, J=1.3, 6.7 Hz), 4.2-4.4 (m, 1H), 4.1-4.2 (m, 1H), 2.1-2.2 (m, 2H), 1.9-2.0 (m, 1H), 1.8-1.9 (m, 1H), 1.4-1.6 (m, 2H). |

Example C17

Synthesis of 6'-(((1S,3S)-3-([1,2,4]triazolo[1,5-a]pyridin-2-ylamino)cyclopentyl)amino)-5'-fluoro-2H-[1,3'-bi pyridin]-2-one

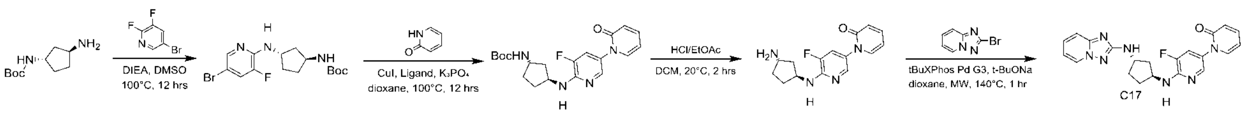


C17

Step 1: Synthesis of tert-butyl ((1S,3S)-3-((5-bromo-3-fluoropyridin-2-yl)amino)cyclopentyl)carbamate

Tert-butyl ((1S,3S)-3-aminocyclopentyl)carbamate (3.00 g, 15.0 mmol, 1 eq), 5-bromo-2,3-difluoropyridine (2.91 g, 15.0 mmol, 1.0 eq), and N,N-diisopropylethylamine (4.84 g, 37.5 mmol, 2.5 eq) were dissolved in dimethyl sulfoxide (40 mL). The mixture was stirred at 100 °C for 12 hours. LCMS showed the MS value of the product (RT = 0.568 min). The reaction solution was cooled to room temperature. Then the reaction mixture was diluted with water (200 mL) and extracted with ethyl acetate (100 mL * 2). The organic phases were combined, washed with saturated brine (500 mL * 2), dried over anhydrous sodium sulfate, filtered, and concentrated to obtain the crude product. The crude product was purified by silica gel column chromatography to obtain tert-butyl ((1S,3S)-3-((5-bromo-3-fluoropyridin-2-yl)amino)cyclopentyl)carbamate (4.00 g). LCMS: RT = 0.568 min, MS (ESI) m/z = 374.0 $[M+1]^+$

Step 2: Synthesis of tert-butyl ((1S,3S)-3-((5'-fluoro-2-oxo-2H-[1,3'-bipyridin]-6'-yl)amino)cyclopentyl)carbamate

Tert-butyl ((1S,3S)-3-((5-bromo-3-fluoropyridin-2-yl)amino)cyclopentyl)carbamate (1.5 g, 4.01 mmol, 1.0 eq), pyridin-2(1H)-one (457 mg, 4.81 mmol, 1.2 eq), Potassium phosphate (2.55 g, 12.0 mmol, 3.0 eq), CuI (153 mg, 802 μmol, 0.20 eq), and (1S,2S)-$N^1$,$N^2$-dimethylcyclohexane-1,2-diamine (114 mg, 802 μmol, 0.2 eq) were dissolved in 1,4-dioxane (20 mL). The mixture was stirred at 100 °C for 12 hours. TLC (petroleum ether/ethyl acetate = 3/1) showed the reaction was complete. The reaction mixture was cooled to room temperature. Then the reaction mixture was diluted with water (100 mL) and extracted with ethyl acetate (30 mL * 2). The organic phases were combined, washed with saturated brine (100 mL * 2), dried over anhydrous sodium sulfate, filtered, and concentrated to obtain the crude product. The crude product was purified by silica gel column chromatography to obtain tert-butyl ((1S,3S)-3-((5'-fluoro-2-oxo-2H-[1,3'-bipyridin]-6'-yl) amino)cyclopentyl)carbamate (1.00 g).

Step 3: Synthesis of 6'-(((1S,3S)-3-aminocyclopentyl)amino)-5'-fluoro-2H-[1,3'-bipyridin]-2-one

Tert-butyl ((1S,3S)-3-((5'-fluoro-2-oxo-2H-[1,3'-bipyridin]-6'-yl)amino)cyclopentyl)carbamate (1.00 g, 2.57 mmol, 1.0 eq) was dissolved in dichloromethane (10 mL). Then HCl/EtOAc (2 M, 20 mL, 15.5 eq) was added. The mixture was stirred at 20 °C for 2 hours. LCMSshowed the MS value of the product (RT = 0.320 min). The reaction mixture was concentrated directly under reduced pressure to dryness to obtain 6'-(((1S,3S)-3-aminocyclopentyl)amino)-5'-fluoro-2H-[1,3'-bipyridin]-2-one (600 mg, 2.08 mmol, 80.8% yield). It was used in the next step without further purification. LCMS: RT = 0.320 min, MS (ESI) m/z = 577.3 $[2M+1]^+$

Step 4: Synthesis of 6'-(((1S,3S)-3-([1,2,4]triazolo[1,5-a]pyridin-2-ylamino)cyclopentyl)amino)-5'-fluoro-2H-[1,3'-bi pyridin]-2-one

6'-(((1S,3S)-3-aminocyclopentyl)amino)-5'-fluoro-2H-[1,3'-bipyridin]-2-one (120 mg, 416 μmol, 1.0 eq), 2-bromo-[1,2,4]triazolo[1,5-a]pyridine (90.7 mg, 458 μmol, 1.1 eq), sodium tert-butoxide (120 mg, 1.25 mmol, 3.0 eq), and tBuXPhos Pd G3 (33.1 mg, 41.6 μmol, 0.1 eq) were dissolved in 1,4-dioxane (4 mL). The mixture was stirred at 140 °C for 1 hour in a microwave reactor. LCMS showed the MS value of the product (RT = 0.399 min). The reaction mixture was filtered. The filtrate was concentrated to obtain the crude product. The crude product was purified by reverse-phase preparative chromatography (column: Phenomenex luna C18 150*40mm*15um; mobile phase: [water(FA)-ACN]; gradient: 15% to 45% B over 15 min) twice to obtain 6'-(((1S,3S)-3-([1,2,4]triazolo[1,5-a]pyridin-2-ylamino)cyclopentyl) amino)-5'-fluoro-2H-[1,3'-bi pyridin]-2-one (36.6 mg). LCMS: RT = 0.399 min, MS (ESI) m/z = 406.1 $[M+1]^+$.

Example C15 to C44, C48 to C53, and C55 to C95

According to the aforementioned preparation methods, Compounds C15 to C44, C48 to C53, and C55to C95 were prepared.

| | | | |
|---|---|---|---|
| C15 | 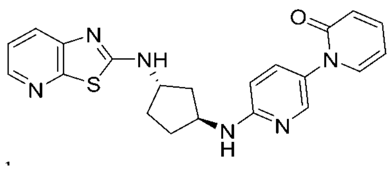<br>$^1$H NMR (400 MHz, DMSO-$d_6$) δ 8.48 (d, *J* = 6.8 Hz, 1H), 8.09 (dd, *J* = 4.9, 1.5 Hz, 1H), 7.94 (d, *J* = 2.7 Hz, 1H), 7.66 (dd, *J* = 8.0, 1.5 Hz, 1H), 7.60 (dd, *J* = 6.8, 2.1 Hz, 1H), 7.48 (ddd, *J* = 9.0, 6.6, 2.1 Hz, 1H), 7.41 (dd, *J* = 8.9, 2.7 Hz, 1H), 7.24 (dd, *J* = 8.0, 4.8 Hz, 1H), 6.99 (d, *J* = 6.9 Hz, 1H), 6.54 (d, *J* = 8.9 Hz, 1H), 6.45 (d, *J* = 9.1 Hz, 1H), 6.28 (td, *J* = 6.7, 1.4 Hz, 1H), 4.43 - 4.34 (m, 2H), 2.25 - 2.13 (m, 2H), 1.97 (dq, *J* = 20.2, 6.6 Hz, 2H), 1.64 - 1.50 (m, 2H). | C16 | 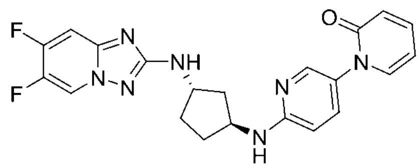<br>$^1$H NMR (METHANOL-$d_4$, 400 MHz) *δ* 8.77 (dd, 1H, *J*=5.2, 6.8 Hz), 7.94 (br d, 1H, *J*=2.3 Hz), 7.5-7.6 (m, 2H), 7.44 (dd, 1H, *J*=2.6, 9.0 Hz), 7.27 (br dd, 1H, *J*=7.4, 10.3 Hz), 6.62 (br d, 2H, *J*=9.0 Hz), 6.46 (dt, 1H, *J*=1.2, 6.7 Hz), 4.35 (td, 1H, *J*=6.5, 13.0 Hz), 4.2-4.3 (m, 1H), 2.2-2.3 (m, 2H), 2.0-2.1 (m, 2H), 1.6-1.7 (m, 2H) |
| C17 | 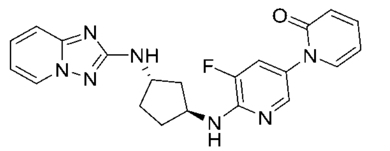<br>$^1$H NMR (400 MHz, DMSO-$d_6$) δ = 8.58 (d, *J* = 6.9 Hz, 1H), 7.84 (d, *J* = 2.0 Hz, 1H), 7.64 (dd, *J* = 1.7, 6.7 Hz, 1H), 7.54 (dd, *J* = 2.1, 11.9 Hz, 1H), 7.49 (ddd, *J* = 2.1, 6.8, 9.0 Hz, 1H), 7.44 - 7.34 (m, 2H), 6.94 (br d, *J* = 6.9 Hz, 1H), 6.85 (dt, *J* = 1.4, 6.7 Hz, 1H), 6.64 (d, *J* = 7.3 Hz, 1H), 6.46 (d, *J* = 9.0 Hz, 1H), 6.32 - 6.25 (m, 1H), 4.57 - 4.47 (m, 1H), 4.25 - 4.14 (m, 1H), 2.19 - 2.10 (m, 2H), 2.01 - 1.94 (m, 2H), 1.65 - 1.51 (m, 2H). | C18 | 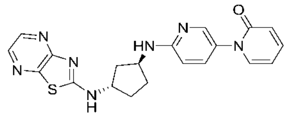<br>$^1$H NMR (400 MHz, DMSO-$d_6$) δ 9.10 (s, 1H), 8.24 (d, *J* = 2.8 Hz, 1H), 8.04 (d, *J* = 2.8 Hz, 1H), 7.94 (d, *J* = 2.7 Hz, 1H), 7.61 (dd, *J* = 6.9, 2.1 Hz, 1H), 7.48 (ddd, *J* = 9.0, 6.6, 2.1 Hz, 1H), 7.41 (dd, *J* = 8.9, 2.7 Hz, 1H), 7.02 (d, *J* = 6.9 Hz, 1H), 6.55 (d, *J* = 8.9 Hz, 1H), 6.45 (d, *J* = 9.2 Hz, 1H), 6.30 - 6.26 (m, 1H), 4.50 - 4.31 (m, 2H), 2.29 - 2.16 (m, 2H), 2.01 (dt, *J* = 9.9, 6.5 Hz, 2H), 1.60 (ddt, *J* = 25.7, 12.6, 6.9 Hz, 2H). |
| C17A: | 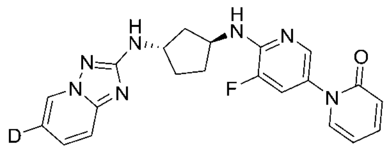 | 1H NMR (400 MHz, DMSO-d6) δ 8.59 (s, 1H), 7.85 (d, J = 2.1 Hz, 1H), 7.65 (dd, J = 6.8, 2.0 Hz, 1H), 7.55 (dd, J = 11.9, 2.2 Hz, 1H), 7.50 (ddd, J = 8.9, 6.6, 2.1 Hz, 1H), 7.42 (dd, J = 8.8, 1.3 Hz, 1H), 7.37 (d, J = 8.8 Hz, 1H), 7.00 - 6.88 (m, 1H), 6.65 (d, J = 7.2 Hz, 1H), 6.52 - 6.41 (m, 1H), 6.29 (td, J = 6.7, 1.3 Hz, 1H), 4.53 (q, J = 6.9 Hz, 1H), 4.20 (q, J = 6.6 Hz, 1H), 2.21 - 2.10 (m, 2H), 1.98 (td, J = 6.7, 2.8 Hz, 2H), 1.68 - 1.50 (m, 2H). | |
| C19 | 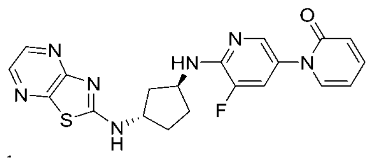<br>$^1$H NMR (400 MHz, DMSO-$d_6$) *δ* 9.08 (s, 1H), 8.25 (d, *J* = 2.8 Hz, 1H), 8.04 (d, *J* = 2.8 Hz, 1H), 7.87 (d, *J* = 2.2 Hz, 1H), 7.66 (dd, *J* = 6.9, 2.1 Hz, 1H), 7.57 (dd, *J* = 11.9, 2.2 Hz, 1H), 7.50 (ddd, *J* = 9.0, 6.6, 2.1 Hz, 1H), 7.07 - 6.97 (m, 1H), 6.47 (d, *J* = 9.2 Hz, 1H), 6.29 (td, *J* = 6.7, 1.4 Hz, 1H), 4.56 (dt, *J* = 23.1, 11.6 Hz, 2H), 2.30 - 2.17 (m, 2H), 2.11 - 2.02 (m, 2H), 1.71 - 1.61 (m, 2H). | C20 | 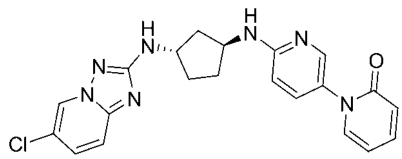<br>$^1$H NMR (400 MHz, DMSO-$d_6$) δ 8.93 (d, *J* = 2.0 Hz, 1H), 7.92 (d, *J* = 2.7 Hz, 1H), 7.60 (dd, *J* = 6.9, 2.1 Hz, 1H), 7.54 - 7.45 (m, 2H), 7.40 (dd, *J* = 9.0, 2.9 Hz, 2H), 6.93 (d, *J* = 6.9 Hz, 1H), 6.82 (d, *J* = 7.2 Hz, 1H), 6.53 (d, *J* = 8.9 Hz, 1H), 6.45 (d, *J* = 9.1 Hz, 1H), 6.27 (td, *J* = 6.7, 1.3 Hz, 1H), 4.32 (q, *J* = 6.5 Hz, 1H), 4.18 (q, *J* = 6.7 Hz, 1H), 2.22 - 2.09 (m, 2H), 1.97 (dt, *J* = 13.4, 6.8 Hz, 1H), 1.89 (td, *J* = 7.3, 3.6 Hz, 1H), 1.63 - 1.43 (m, 2H). |

(continued)

| | | | |
|---|---|---|---|
| C21 | 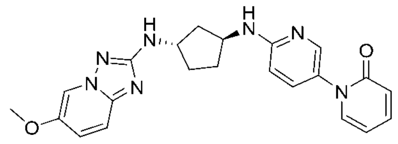 $^1$H NMR (400 MHz, DMSO-$d_6$) δ 8.39 (d, *J* = 2.4 Hz, 1H), 7.92 (d, *J* = 2.7 Hz, 1H), 7.61 (dd, *J* = 6.9, 2.1 Hz, 1H), 7.54 - 7.44 (m, 1H), 7.40 (dd, *J* = 8.9, 2.7 Hz, 1H), 7.31 (d, *J* = 9.5 Hz, 1H), 7.21 (dd, *J* = 9.5, 2.5 Hz, 1H), 6.93 (d, *J* = 6.8 Hz, 1H), 6.51 (dd, *J* = 14.2, 8.0 Hz, 2H), 6.45 (d, *J* = 8.8 Hz, 1H), 6.27 (td, *J* = 6.7, 1.4 Hz, 1H), 4.32 (q, *J* = 6.6 Hz, 1H), 4.15 (q, *J* = 6.7 Hz, 1H), 3.79 (s, 3H), 2.15 (qd, *J* = 7.6, 2.8 Hz, 2H), 1.96 (dt, *J* = 13.2, 6.7 Hz, 1H), 1.92 - 1.80 (m, 1H), 1.65 - 1.43 (m, 2H). | C22 | 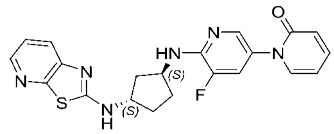 $^1$H NMR (400 MHz, DMSO-$d_6$) δ 8.50 (d, *J* = 6.8 Hz, 1H), 8.26 (d, *J* = 1.4 Hz, 1H), 8.09 (dd, *J* = 4.8, 1.5 Hz, 1H), 7.88 (d, *J* = 1.3 Hz, 1H), 7.72 (dd, *J* = 7.0, 2.1 Hz, 1H), 7.67 (dd, *J* = 8.0, 1.5 Hz, 1H), 7.56 (d, *J* = 6.8 Hz, 1H), 7.50 (ddd, *J* = 8.9, 6.5, 2.1 Hz, 1H), 7.25 (dd, *J* = 8.0, 4.8 Hz, 1H), 6.47 (dd, *J* = 9.2, 1.2 Hz, 1H), 6.33 (td, *J* = 6.8, 1.4 Hz, 1H), 4.39 (dq, *J* = 26.9, 6.5 Hz, 2H), 2.27 - 2.16 (m, 2H), 2.01 (tt, *J* = 13.3, 6.7 Hz, 2H), 1.61 (ddt, *J* = 21.4, 12.8, 5.7 Hz, 2H). |
| C23 | 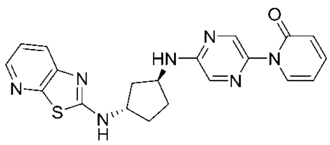 $^1$H NMR (400 MHz, DMSO-$d_6$) δ 8.50 (d, *J* = 6.8 Hz, 1H), 8.26 (d, *J* = 1.4 Hz, 1H), 8.09 (dd, *J* = 4.8, 1.5 Hz, 1H), 7.88 (d, *J* = 1.3 Hz, 1H), 7.72 (dd, *J* = 7.0, 2.1 Hz, 1H), 7.67 (dd, *J* = 8.0, 1.5 Hz, 1H), 7.56 (d, *J* = 6.8 Hz, 1H), 7.51 (td, *J* = 6.7, 3.2 Hz, 1H), 7.25 (dd, *J* = 8.0, 4.8 Hz, 1H), 6.47 (dd, *J* = 9.2, 1.2 Hz, 1H), 6.33 (td, *J* = 6.8, 1.4 Hz, 1H), 4.45 - 4.34 (m, 2H), 2.23 (td, *J* = 10.7, 10.1, 5.6 Hz, 2H), 2.01 (dt, *J* = 13.2, 6.6 Hz, 2H), 1.61 (ddd, *J* = 16.7, 13.9, 7.0 Hz, 2H). | C24 | 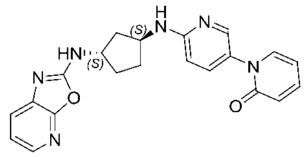 $^1$H NMR (400 MHz, DMSO-$d_6$) δ 8.44 - 8.39 (d, *J* = 7.0 Hz, 1H), 7.96 - 7.92 (d, *J* = 2.7 Hz, 1H), 7.86 - 7.81 (dd, *J* = 5.1, 1.5 Hz, 1H), 7.63 - 7.56 (m, 2H), 7.51 - 7.38 (m, 2H), 7.19 - 7.14 (dd, *J* = 7.6, 5.1 Hz, 1H), 7.01 - 6.97 (d, *J* = 6.8 Hz, 1H), 6.57 - 6.51 (d, *J* = 8.9 Hz, 1H), 6.49 - 6.42 (d, *J* = 9.2 Hz, 1H), 6.30 - 6.24 (td, *J* = 6.7, 1.3 Hz, 1H), 4.40 - 4.23 (m, 2H), 2.25 - 2.13 (m, 2H), 2.06 - 1.91 (m, 2H), 1.72 - 1.49 (m, 2H). |
| C25 | 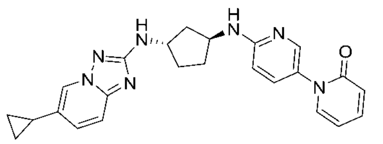 $^1$H NMR (400 MHz, DMSO-$d_6$) δ 8.42 (s, 1H), 7.92 (d, *J* = 2.7 Hz, 1H), 7.61 (dd, *J* = 6.9, 2.1 Hz, 1H), 7.48 (ddd, *J* = 8.9, 6.6, 2.1 Hz, 1H), 7.40 (dd, *J* = 8.9, 2.8 Hz, 1H), 7.27 (d, *J* = 9.0 Hz, 1H), 7.22 - 7.11 (m, 1H), 6.93 (d, *J* = 6.9 Hz, 1H), 6.54 (t, *J* = 9.0 Hz, 2H), 6.45 (d, *J* = 9.2 Hz, 1H), 6.27 (t, *J* = 6.7 Hz, 1H), 4.31 (d, *J* = 6.9 Hz, 1H), 4.23 - 4.10 (m, 1H), 2.15 (dt, *J* = 11.6, 6.4 Hz, 2H), 2.04 - 1.91 (m, 2H), 1.87 (q, *J* = 6.6 Hz, 1H), 1.52 (ddd, *J* = 24.9, 13.0, 6.8 Hz, 2H), 0.91 (dt, *J* = 8.7, 3.2 Hz, 2H), 0.77 - 0.59 (m, 2H). | C26 | 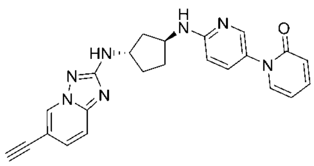 $^1$H NMR (400 MHz, DMSO-$d_6$) δ 8.88 (t, *J* = 1.2 Hz, 1H), 7.92 (d, *J* = 2.7 Hz, 1H), 7.61 (dd, *J* = 6.9, 2.1 Hz, 1H), 7.53 - 7.44 (m, 2H), 7.44 - 7.32 (m, 2H), 6.95 (d, *J* = 6.9 Hz, 1H), 6.90 (d, *J* = 7.3 Hz, 1H), 6.53 (d, *J* = 8.9 Hz, 1H), 6.49 - 6.40 (m, 1H), 6.27 (td, *J* = 6.6, 1.4 Hz, 1H), 4.33 (q, *J* = 6.6 Hz, 1H), 4.27 (s, 1H), 4.18 (q, *J* = 6.7 Hz, 1H), 2.23 - 2.11 (m, 2H), 1.97 (dt, *J* = 13.3, 6.9 Hz, 1H), 1.88 (ddd, *J* = 13.2, 7.5, 5.8 Hz, 1H), 1.64 - 1.46 (m, 2H). |

(continued)

| | | | |
|---|---|---|---|
| C27 | 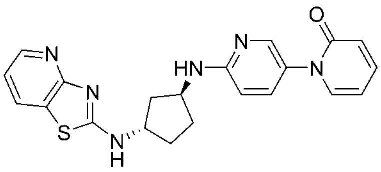<br>$^{1}$H NMR (400 MHz, DMSO-$d_6$) δ 8.58 (d, *J* = 6.8 Hz, 1H), 8.23 (d, *J* = 4.9 Hz, 1H), 8.07 (d, *J* = 7.6 Hz, 1H), 7.94 (d, *J* = 2.6 Hz, 1H), 7.62 (dd, *J* = 6.9, 2.0 Hz, 1H), 7.48 (ddd, *J* = 8.9, 6.6, 2.1 Hz, 1H), 7.41 (dd, *J* = 8.9, 2.7 Hz, 1H), 7.02 (d, *J* = 6.9 Hz, 1H), 6.98 (dd, *J* = 7.7, 4.9 Hz, 1H), 6.54 (d, *J* = 8.9 Hz, 1H), 6.45 (d, *J* = 9.2 Hz, 1H), 6.28 (t, *J* = 6.7 Hz, 1H), 4.37 (dt, *J* = 13.4, 6.7 Hz, 2H), 2.21 (ddt, *J* = 17.8, 12.3, 6.0 Hz, 2H), 1.98 (dt, *J* = 13.9, 6.9 Hz, 2H), 1.65 - 1.53 (m, 2H). | C28 | 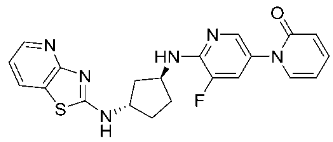<br>$^{1}$H NMR (400 MHz, DMSO-$d_6$) δ 8.60 (d, *J* = 6.8 Hz, 1H), 8.23 (dd, *J* = 4.9, 1.6 Hz, 1H), 8.06 (dd, *J* = 7.7, 1.7 Hz, 1H), 7.86 (s, 1H), 7.66 (dd, *J* = 6.9, 2.1 Hz, 1H), 7.57 (dd, *J* = 11.9, 2.2 Hz, 1H), 7.50 (ddd, *J* = 9.0, 6.6, 2.1 Hz, 1H), 7.04 (d, *J* = 7.1 Hz, 1H), 6.97 (dd, *J* = 7.7, 4.9 Hz, 1H), 6.47 (d, *J* = 9.2 Hz, 1H), 6.31 - 6.27 (m, 1H), 4.56 (q, *J* = 7.1 Hz, 1H), 4.41 (q, *J* = *7.1,* 6.6 Hz, 1H), 2.21 (ddt, *J* = 19.9, 12.4, 5.0 Hz, 2H), 2.04 (h, *J* = 5.8, 5.1 Hz, 2H), 1.64 (dt, *J* = 11.5, 7.6 Hz, 2H). |
| C29 | 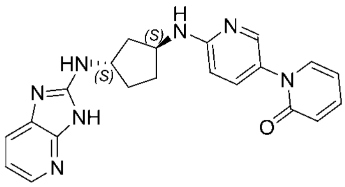<br>1H NMR (400 MHz, DMSO-d6) δ 9.10 - 8.90 (s, 1H), 8.08 - 8.02 (d, J = 6.0 Hz, 1H), 8.00 - 7.95 (d, J = 2.6 Hz, 1H), 7.84 - 7.79 (d, J = 7.6 Hz, 1H), 7.63 - 7.58 (dd, J = 6.9, 2.1 Hz, 1H), 7.54 - 7.46 (m, 2H), 7.41 - 7.25 (s, 1H), 7.24 - 7.16 (m 1H), 6.65 - 6.60 (d, J = 9.0 Hz, 1H), 6.48 - 6.43 (d, J = 9.2 Hz, 1H), 6.33 - 6.26 (t, J = 6.7 Hz, 1H), 4.41 - 4.25 (t, J = 7.0 Hz, 2H), 2.28 - 2.18 (m, 2H), 2.08 - 2.00 (m, 2H), 1.72 - 1.56 (m, 2H). | C30 | 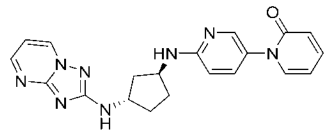<br>1H NMR (400 MHz, DMSO-d6) δ 9.01 (dd, J = 6.5, 1.9 Hz, 1H), 8.49 (dd, J = 4.5, 1.9 Hz, 1H), 7.92 (d, J = 2.7 Hz, 1H), 7.61 (dd, J = 7.0, 2.0 Hz, 1H), 7.51 - 7.46 (m, 1H), 7.40 (dd, J = 8.8, 2.7 Hz, 1H), 7.10 (d, J = 7.2 Hz, 1H), 7.01 (dd, J = 6.5, 4.6 Hz, 1H), 6.95 (d, J = 6.9 Hz, 1H), 6.53 (d, J = 8.9 Hz, 1H), 6.46 - 6.43 (m, 1H), 6.27 (td, J = 6.7, 1.4 Hz, 1H), 4.36 - 4.30 (m, 1H), 4.23 - 4.16 (m, 1H), 2.17 (dt, J = 8.4, 5.0 Hz, 2H), 2.00 - 1.91 (m, 2H), 1.56 (ddd, J = 26.4, 12.5, 6.7 Hz, 2H). |
| C31 | 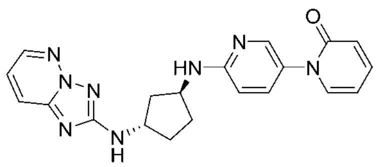<br>$^{1}$H NMR (400 MHz, DMSO-$d_6$) δ 8.34 (dd, *J* = 4.6, 1.7 Hz, 1H), 7.94 (dd, *J* = 8.8, 2.0 Hz, 2H), 7.61 (dd, *J* = 6.8, 2.2 Hz, 1H), 7.50 - 7.46 (m, 1H), 7.40 (dd, *J* = 8.6, 3.3 Hz, 2H), 7.03 (d, *J* = 7.2 Hz, 1H), 6.96 (d, *J* = 6.9 Hz, 1H), 6.54 (d, *J* = 8.9 Hz, 1H), 6.46 - 6.43 (m, 1H), 6.29 - 6.25 (m, 1H), 4.34 (q, *J* = 6.6 Hz, 1H), 4.23 (q, *J* = 6.7 Hz, 1H), 2.17 (ddd, *J* = 10.7, 7.9, 3.5 Hz, 2H), 2.00 - 1.89 (m, 2H), 1.62 - 1.50 (m, 2H). | C32 | 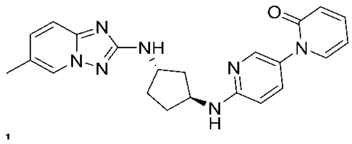<br>,<br>$^{1}$H NMR (400 MHz, DMSO-$d_6$) δ 8.44 (s, 1H), 7.92 (d, *J* = 2.7 Hz, 1H), 7.61 (dd, *J* = 6.9, 2.1 Hz, 1H), 7.48 (ddd, *J* = 8.9, 6.5, 2.1 Hz, 1H), 7.40 (dd, *J* = 8.9, 2.7 Hz, 1H), 7.29 (s, 2H), 6.94 (d, *J* = 6.8 Hz, 1H), 6.54 (t, *J* = 8.4 Hz, 2H), 6.44 (d, *J* = 9.2 Hz, 1H), 6.27 (t, *J* = 6.7 Hz, 1H), 4.32 (q, *J* = 6.6 Hz, 1H), 4.16 (q, *J* = 6.7 Hz, 1H), 2.27 (s, 3H), 2.15 (dq, *J* = 10.5, 5.1 Hz, 2H), 1.93 (ddd, *J* = 28.0, 13.6, 7.0 Hz, 2H), 1.62 - 1.39 (m, 2H). |

(continued)

| | | | |
|---|---|---|---|
| C33 | 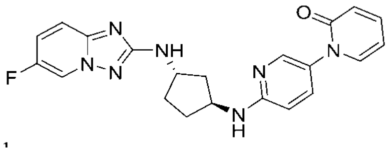<br>$^1$H NMR (400 MHz, DMSO-$d_6$) δ 8.99 - 8.87 (m, 1H), 7.92 (d, *J* = 2.7 Hz, 1H), 7.60 (ddd, *J* = 6.9, 2.1, 0.7 Hz, 1H), 7.55 - 7.44 (m, 2H), 7.44 - 7.37 (m, 2H), 6.92 (d, *J* = 6.8 Hz, 1H), 6.72 (d, *J* = 7.2 Hz, 1H), 6.53 (d, *J* = 8.9 Hz, 1H), 6.45 (dt, *J* = 9.1, 1.1 Hz, 1H), 6.27 (t d, *J* = 6.7, 1.4 Hz, 1H), 4.32 (q, *J* = 6.6 Hz, 1H), 4.17 (q, *J* = 6.7 Hz, 1H), 2.15 (dq, *J* = 11.9, 5.0 Hz, 2H), 1.97 (dt, *J* = 13.4, 6.8 Hz, 1H), 1.93 - 1.83 (m, 1H), 1.64 - 1.43 (m, 2H). | C34 | 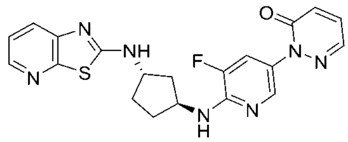<br>$^1$H NMR (400 MHz, DMSO-$d_6$) δ 8.46 (d, *J* = 6.8 Hz, 1H), 8.10 (dd, *J* = 4.8, 1.5 Hz, 1H), 8.07 - 8.04 (m, 2H), 7.69 - 7.64 (m, 2H), 7.49 (dd, *J* = 9.5, 3.9 Hz, 1H), 7.25 (dd, *J* = 8.1, 4.8 Hz, 1H), 7.07 (dd, *J* = 9.5, 1.6 Hz, 1H), 7.04 - 7.01 (m, 1H), 4.59 - 4.35 (m, 2H), 2.27 - 2.18 (m, 2H), 2.05 (td, *J* = 6.7, 2.1 Hz, 2H), 1.70 - 1.61 (m, 2H). |
| C35 | 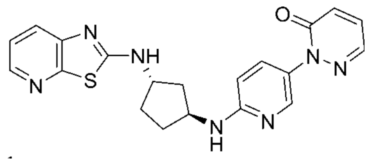<br>$^1$H NMR (400 MHz, DMSO-$d_6$) δ 8.47 (d, *J* = 6.8 Hz, 1H), 8.14 (d, *J* = 2.7 Hz, 1H), 8.10 (dd, *J* = 4.8, 1.5 Hz, 1H), 8.03 (dd, *J* = 3.8, 1.7 Hz, 1H), 7.68 (dd, *J* = 8.1, 1.5 Hz, 1H), 7.55 (dd, J = 8.9, 2.7 Hz, 1H), 7.48 (dd, *J* = 9.5, 3.8 Hz, 1H), 7.25 (dd, *J* = 8.1, 4.8 Hz, 1H), 7.04 (dd, *J* = 9.5, 1.6 Hz, 1H), 7.00 (d, *J* = 6.9 Hz, 1H), 6.56 (d, *J* = 8.9 Hz, 1H), 4.39 (dt, *J* = 13.7, 7.3 Hz, 2H), 2.22 (ddt, *J* = 18.0, 13.0, 5.8 Hz, 2H), 1.99 (tt, *J* = 13.3, 6.3 Hz, 2H), 1.67 - 1.53 (m, 2H). | C36 | 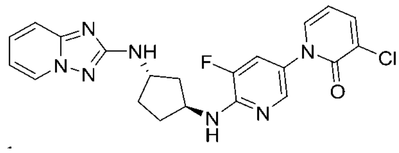<br>$^1$H NMR (400 MHz, DMSO-$d_6$) δ 8.66 - 8.47 (m, 1H), 7.90 (d, *J* = 2.2 Hz, 1H), 7.84 (dd, *J* = 7.4, 1.9 Hz, 1H), 7.72 (dd, *J* = 6.9, 1.9 Hz, 1H), 7.62 (dd, *J* = 11.9, 2.2 Hz, 1H), 7.49 - 7.34 (m, 2H), 7.01 (dd, *J* = 7.2, 1.7 Hz, 1H), 6.87 (td, *J* = 6.7, 1.5 Hz, 1H), 6.66 (d, *J* = 7.2 Hz, 1H), 6.33 (t, *J* = 7.1 Hz, 1H), 4.54 (p, *J* = 6.9 Hz, 1H), 4.22 (p, *J* = 6.5 Hz, 1H), 2.16 (ddt, *J* = 9.5, 6.6, 3.1 Hz, 2H), 2.00 (td, *J* = 6.9, 6.5, 2.6 Hz, 2H), 1.61 (dtd, *J* = 14.4, 8.8, 4.5 Hz, 2H). |
| C37 | 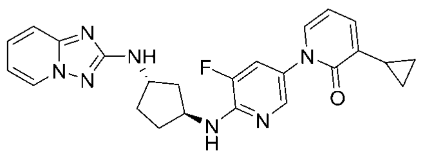<br>$^1$H NMR (400 MHz, DMSO-$d_6$) δ 8.60 (d, *J* = 6.6 Hz, 1H), 7.86 (d, *J* = 2.2 Hz, 1H), 7.57 (dd, *J* = 12.0, 2.2 Hz, 1H), 7.50 - 7.34 (m, 3H), 7.03 (dd, *J* = 7.0, 2.0 Hz, 1H), 6.97 - 6.90 (m, 1H), 6.90 - 6.82 (m, 1H), 6.67 (d, *J* = 7.2 Hz, 1H), 6.20 (t, *J* = 6.8 Hz, 1H), 4.55 (q, *J* = 6.9 Hz, 1H), 4.22 (q, *J* = 6.5 Hz, 1H), 2.17 (q, *J* = 6.1, 5.6 Hz, 2H), 2.02 (dtd, *J* = 13.9, 7.1, 6.2, 3.2 Hz, 3H), 1.61 (q, *J* = 5.3 Hz, 2H), 0.92 - 0.80 (m, 2H), 0.73 - 0.57 (m, 2H). | C38 | 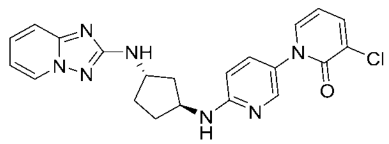<br>$^1$H NMR (400 MHz, DMSO-$d_6$) δ 8.62 (d, *J* = 6.8 Hz, 1H), 7.98 (s, 1H), 7.91 - 7.75 (m, 1H), 7.68 (d, *J* = 6.7 Hz, 1H), 7.44 (dt, *J* = 11.4, 5.5 Hz, 3H), 7.01 (d, *J* = 6.8 Hz, 1H), 6.88 (t, *J* = 6.5 Hz, 1H), 6.69 (s, 1H), 6.56 (d, *J* = 8.9 Hz, 1H), 6.32 (t, *J* = 7.0 Hz, 1H), 4.35 (q, *J* = 6.8 Hz, 1H), 4.20 (s, 1H), 2.18 (q, *J* = 7.1 Hz, 2H), 1.95 (dq, *J* = 30.0, 6.6 Hz, 2H), 1.72 - 1.40 (m, 2H). |

(continued)

| | | | |
|---|---|---|---|
| C39 | 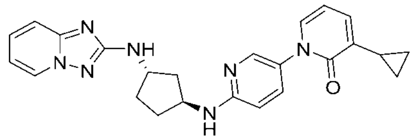<br>$^{1}$H NMR (400 MHz, DMSO-$d_6$) δ 8.60 (d, $J$ = 6.6 Hz, 1H), 7.94 (d, $J$ = 2.6 Hz, 1H), 7.42 (dq, $J$ = 10.0, 2.7 Hz, 4H), 7.02 (dd, $J$ = 6.9, 2.0 Hz, 1H), 6.92 (d, $J$ = 6.9 Hz, 1H), 6.90 - 6.83 (m, 1H), 6.67 (d, $J$ = 7.1 Hz, 1H), 6.55 (d, $J$ = 8.9 Hz, 1H), 6.19 (t, $J$ = 6.9 Hz, 1H), 4.34 (p, $J$ = 6.7 Hz, 1H), 4.21 (q, $J$ = 6.7 Hz, 1H), 2.25 - 2.12 (m, 2H), 2.08 - 1.94 (m, 2H), 1.90 (ddd, $J$ = 13.2, 7.5, 5.8 Hz, 1H), 1.67 - 1.40 (m, 2H), 0.94 - 0.79 (m, 2H), 0.70 - 0.60 (m, 2H). | C40 | 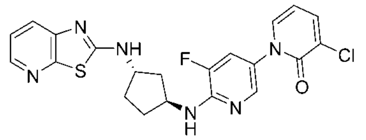<br>$^{1}$H NMR (400 MHz, DMSO-$d_6$) δ 8.48 (d, $J$ = 6.8 Hz, 1H), 8.11 - 8.09 (m, 1H), 7.91 (d, $J$ = 2.2 Hz, 1H), 7.85 (dd, $J$ = 7.3, 1.9 Hz, 1H), 7.73 - 7.70 (m, 1H), 7.68 (dd, $J$ = 8.0, 1.5 Hz, 1H), 7.64 (dd, $J$ = 11.9, 2.2 Hz, 1H), 7.28 - 7.24 (m, 1H), 7.09 (d, $J$ = 7.2 Hz, 1H), 6.33 (d, $J$ = 7.1 Hz, 1H), 4.51 (dq, $J$ = 56.0, 6.8 Hz, 2H), 2.25 - 2.17 (m, 2H), 2.06 (d, $J$ = 7.1 Hz, 2H), 1.65 (td, $J$ = 12.3, 11.4, 6.8 Hz, 2H). |
| C41 | 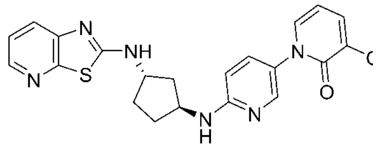<br>$^{1}$H NMR (400 MHz, DMSO-$d_6$) δ 8.48 (d, $J$ = 6.8 Hz, 1H), 8.10 (dd, $J$ = 4.8, 1.5 Hz, 1H), 7.99 (d, $J$ = 2.7 Hz, 1H), 7.83 (dd, $J$ = 7.3, 1.9 Hz, 1H), 7.69 (t, $J$ = 1.4 Hz, 1H), 7.67 (d, $J$ = 1.7 Hz, 1H), 7.46 (dd, $J$ = 8.9, 2.7 Hz, 1H), 7.26 (dd, $J$ = 8.0, 4.8 Hz, 1H), 7.06 (d, $J$ = 6.9 Hz, 1H), 6.56 (d, $J$ = 8.9 Hz, 1H), 6.32 (t, $J$ = 7.1 Hz, 1H), 4.39 (dt, $J$ = 13.8, 7.1 Hz, 2H), 2.22 (ddd, $J$ = 18.8, 9.9, 6.2 Hz, 2H), 2.03 - 1.95 (m, 2H), 1.66 - 1.53 (m, 2H). | C42 | 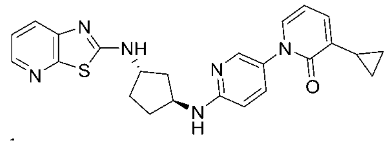<br>$^{1}$H NMR (400 MHz, DMSO-$d_6$) δ 8.48 (d, $J$ = 6.8 Hz, 1H), 8.11 (d, $J$ = 4.4 Hz, 1H), 7.95 (s, 1H), 7.68 (dd, $J$ = 8.0, 1.4 Hz, 1H), 7.45 - 7.43 (m, 1H), 7.41 (d, $J$ = 2.2 Hz, 1H), 7.27 - 7.24 (m, 1H), 7.04 - 7.02 (m, 1H), 6.98 (d, $J$ = 6.8 Hz, 1H), 6.56 (d, $J$ = 8.9 Hz, 1H), 6.20 (d, $J$ = 6.8 Hz, 1H), 4.44 - 4.37 (m, 2H), 2.22 (ddd, $J$ = 11.3, 8.3, 5.5 Hz, 2H), 2.05 - 2.01 (m, 2H), 1.98 (d, $J$ = 7.6 Hz, 1H), 1.66 - 1.55 (m, 2H), 0.87 (dd, $J$ = 6.1, 2.4 Hz, 2H), 0.68 - 0.64 (m, 2H). |
| C43 | 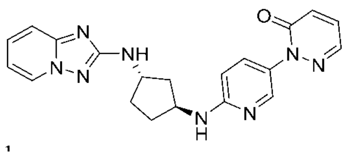<br>$^{1}$H NMR (400 MHz, DMSO-$d_6$) δ 8.61 - 8.58 (dt, $J$ = 6.6, 1.2 Hz, 1H), 8.13 - 8.11 (m, 1H), 8.04 - 8.02 (dd, $J$ = 3.8, 1.6 Hz, 1H), 7.55 - 7.51 (dd, $J$ = 8.9, 2.7 Hz, 1H), 7.50 - 7.45 (m, 1H), 7.44 - 7.41 (m, 1H), 7.40 - 7.37 (dt, $J$ = 8.7, 1.4 Hz, 1H), 7.06 - 7.02 (dd, $J$ = 9.5, 1.6 Hz, 1H), 6.98 - 6.93 (d, $J$ = 6.9 Hz, 1H), 6.91 - 6.84 (td, $J$ = *6.7,* 1.6 Hz, 1H), 6.69 - 6.63 (d, $J$ = 7.2 Hz, 1H), 6.58 - 6.52 (dd, $J$ = 9.0, 0.7 Hz, 1H), 4.39 - 4.30 (q, $J$ = 6.6 Hz, 1H), 4.24 - 4.17 (q, $J$ = 6.7 Hz, 1H), 2.23 - 2.11 (td, $J$ = 11.4, 10.8, 4.9 Hz, 2H), 1.98 - 1.86 (ddd, $J$ = 19.1, 14.1, 6.7 Hz, 2H), 1.66 - 1.45 (ddt, $J$ = 25.0, 12.9, 6.7 Hz, 2H). | C44 | 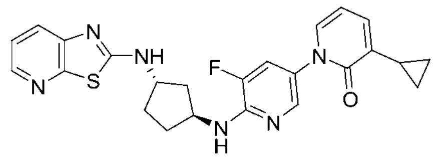<br>$^{1}$H NMR (400 MHz, DMSO-$d_6$) δ 8.48 (d, $J$ = 6.8 Hz, 1H), 8.10 (dd, $J$ = 4.8, 1.5 Hz, 1H), 7.87 (d, $J$ = 2.2 Hz, 1H), 7.68 (dd, $J$ = 8.0, 1.5 Hz, 1H), 7.58 (dd, $J$ = 11.9, 2.2 Hz, 1H), 7.47 (dd, $J$ = 6.8, 2.0 Hz, 1H), 7.26 (dd, $J$ = 8.1, 4.8 Hz, 1H), 7.02 (ddd, $J$ = 13.1, 7.1, 1.8 Hz, 2H), 6.21 (t, $J$ = 6.9 Hz, 1H), 4.58 (q, $J$ = 7.0 Hz, 1H), 4.44 (q, $J$ = 6.3 Hz, 1H), 2.23 (ddd, $J$ = 18.2, 9.7, 5.0 Hz, 2H), 2.08 - 2.04 (m, 2H), 2.03 (d, $J$ = 1.9 Hz, 1H), 1.64 (ddd, $J$ = 11.8, 7.0, 3.7 Hz, 2H), 0.90 - 0.85 (m, 2H), 0.66 (dd, $J$ = 5.4, 2.1 Hz, 2H). |

(continued)

| | | | |
|---|---|---|---|
| C48 | 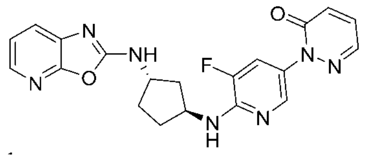 $^1$H NMR (400 MHz, DMSO-$d_6$) δ 8.43 - 8.37 (d, *J* = 7.0 Hz, 1H), 8.07 - 8.01 (t, *J* = 3.4 Hz, 2H), 7.85 - 7.80 (d, *J* = 5.1 Hz, 1H), 7.67 - 7.55 (m, 2H), 7.52 - 7.44 (dd, *J* = 9.5, 3.9 Hz, 1H), 7.19 - 7.12 (dd, *J* = 7.7, 5.1 Hz, 1H), 7.09 - 6.99 (m, 2H), 4.61 - 4.51 (m, 1H), 4.34 - 4.24 (d, *J* = 6.4 Hz, 1H), 2.26 - 2.13 (td, *J* = 11.6, 11.2, 3.8 Hz, 2H), 2.07 - 1.99 (t, *J* = 6.8 Hz, 2H), 1.70 - 1.58 (d, *J* = 11.6 Hz, 2H). | C49 | 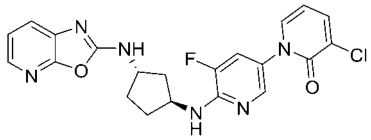 $^1$H NMR (400 MHz, DMSO-$d_6$) δ 8.46 - 8.40 (d, *J* = 7.0 Hz, 1H), 7.94 - 7.82 (m, 3H), 7.76 - 7.58 (m, 3H), 7.22 - 7.07 (m, 2H), 6.38 - 6.29 (t, *J* = 7.0 Hz, 1H), 4.65 - 4.51 (q, *J* = 7.0 Hz, 1H), 4.35 - 4.23 (q, *J* = *6.6* Hz, 1H), 2.27 - 2.11 (dt, *J* = 18.0, 9.0 Hz, 2H), 2.11 - 1.91 (t, *J* = 6.8 Hz, 2H), 1.74 - 1.60 (tt, *J* = 7.4, 3.6 Hz, 2H). |
| C50 | 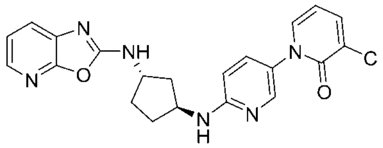 $^1$H NMR (400 MHz, DMSO-$d_6$) δ 8.45 - 8.37 (t, *J* = 5.5 Hz, 1H), 8.01 - 7.94 (s, 1H), 7.87 - 7.78 (d, *J* = 7.5 Hz, 2H), 7.69 - 7.64 (dd, *J* = 6.4, 3.6 Hz, 1H), 7.61 - 7.52 (dd, *J* = 8.0, 3.9 Hz, 1H), 7.48 - 7.41 (dt, *J* = 8.9, 3.2 Hz, 1H), 7.21 - 7.11 (dt, *J* = 8.3, 4.4 Hz, 1H), 7.08 - 6.99 (t, *J* = *5.5* Hz, 1H), 6.59 - 6.50 (dd, *J* = 9.0, 3.9 Hz, 1H), 6.35 - 6.26 (td, *J* = 7.1, 3.7 Hz, 1H), 4.42 - 4.21 (d, *J* = 34.1 Hz, 2H), 2.25 - 2.12 (s, 2H), 2.07 - 1.87 (m, 2H), 1.70 - 1.45 (d, *J* = 42.2 Hz, 2H). | C51 | 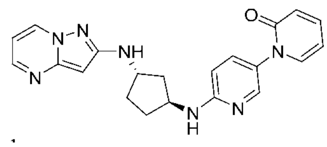 $^1$H NMR (400 MHz, DMSO-$d_6$) δ 8.72 (d, *J* = 6.7 Hz, 1H), 8.22 (dd, *J* = 4.3, 1.8 Hz, 1H), 7.94 (d, *J* = 2.7 Hz, 1H), 7.67 - 7.58 (m, 1H), 7.49 (ddd, *J* = 9.0, 6.6, 2.0 Hz, 1H), 7.42 (dd, *J* = 8.9, 2.9 Hz, 1H), 6.99 - 6.89 (m, 1H), 6.64 (dd, *J* = 6.8, 4.3 Hz, 1H), 6.54 (d, *J* = 9.0 Hz, 1H), 6.46 (d, *J* = 9.2 Hz, 1H), 6.35 (d, *J* = 7.1 Hz, 1H), 6.29 (t, *J* = 6.6 Hz, 1H), 5.75 (s, 1H), 4.34 (q, *J* = 6.5 Hz, 1H), 4.16 - 3.99 (m, 1H), 2.18 (p, *J* = 4.9 Hz, 2H), 1.95 (ddd, *J* = 22.3, 14.0, 6.8 Hz, 2H), 1.54 (dd, *J* = 10.8, 5.6 Hz, 2H). |
| C52 | 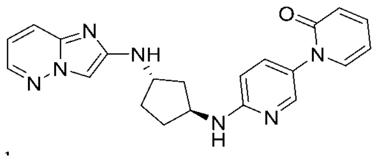 $^1$H NMR (400 MHz, DMSO-$d_6$) δ 8.20 (dd, *J* = 4.8, 1.6 Hz, 1H), 7.94 (d, *J* = 2.6 Hz, 1H), 7.74 - 7.65 (m, 1H), 7.62 (dd, *J* = 6.9, 2.0 Hz, 1H), 7.49 (ddd, *J* = 9.0, 6.6, 2.1 Hz, 1H), 7.42 (q, *J* = 4.4, 3.6 Hz, 2H), 7.06 - 6.88 (m, 2H), 6.54 (d, *J* = 8.9 Hz, 1H), 6.46 (d, *J* = 9.2 Hz, 1H), 6.28 (t, *J* = 6.7 Hz, 1H), 6.11 (d, *J* = 7.2 Hz, 1H), 4.42 - 4.30 (m, 1H), 4.02 (dt, *J* = 13.2, 6.8 Hz, 1H), 2.17 (tt, *J* = 12.6, 6.2 Hz, 2H), 2.06 - 1.87 (m, 2H), 1.55 (dtd, *J* = 19.8, 13.9, 11.3, 7.2 Hz, 2H). | C53 | 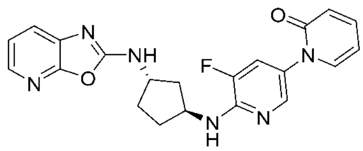 $^1$H NMR (400 MHz, DMSO-$d_6$) δ 8.43 - 8.38 (d, *J* = 7.1 Hz, 1H), 7.87 - 7.81 (m, 2H), 7.67 - 7.63 (ddd, *J* = 6.8, 2.1, 0.8 Hz, 1H), 7.60 - 7.54 (m, 2H), 7.52 - 7.47 (ddd, *J* = 9.0, 6.6, 2.1 Hz, 1H), 7.19 - 7.13 (dd, *J* = 7.6, 5.1 Hz, 1H), 7.02 - 6.98 (d, *J* = 7.0 Hz, 1H), 6.48 - 6.43 (dt, *J* = 9.0, 1.1 Hz, 1H), 6.32 - 6.26 (td, *J* = 6.7, 1.4 Hz, 1H), 4.60 - 4.51 (q, *J* = 6.9 Hz, 1H), 4.34 - 4.24 (q, *J* = 6.5 Hz, 1H), 2.25 - 2.13 (tt, *J* = 11.3, 5.6 Hz, 2H), 2.08 - 2.00 (t, *J* = 6.8 Hz, 2H), 1.71 - 1.58 (m, 2H). |

(continued)

| | | | |
|---|---|---|---|
| C55 | 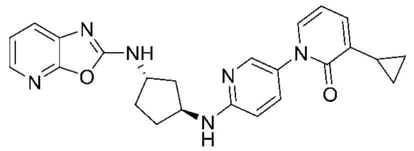 $^1$H NMR (400 MHz, DMSO-$d_6$) δ 8.43 - 8.39 (d, *J* = 7.0 Hz, 1H), 7.94 - 7.91 (d, *J* = 2.7 Hz, 1H), 7.86 - 7.82 (dd, *J* = 5.1, 1.5 Hz, 1H), 7.61 - 7.56 (dd, *J* = 7.6, 1.5 Hz, 1H), 7.44 - 7.38 (m, 2H), 7.18 - 7.14 (dd, *J* = 7.6, 5.1 Hz, 1H), 7.04 - 6.94 (m, 2H), 6.56 - 6.51 (d, *J* = 8.9 Hz, 1H), 6.20 - 6.16 (t, *J* = 6.9 Hz, 1H), 4.40 - 4.23 (dp, *J* = 33.2, 6.6 Hz, 2H), 2.25 - 2.13 (tt, *J* = 12.4, 6.2 Hz, 2H), 2.07 - 2.01 (dd, *J* = 8.0, 5.3 Hz, 1H), 1.98 - 1.89 (ddd, *J* = 13.3, 7.5, 6.0 Hz, 1H), 1.71 - 1.48 (m, 2H), 0.88 - 0.80 (m, 2H), 0.68 - 0.60 (m, 2H). | C56 | 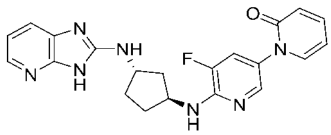 $^1$H NMR (400 MHz, DMSO-$d_6$) δ 7.91 (s, 1H), 7.87 (d, *J* = 2.2 Hz, 1H), 7.67 (dd, *J* = 6.9, 2.1 Hz, 1H), 7.58 (dd, *J* = 11.9, 2.2 Hz, 1H), 7.55 - 7.32 (m, 3H), 6.98 (d, *J* = 7.0 Hz, 1H), 6.95 - 6.86 (m, 1H), 6.48 (d, *J* = *9.1* Hz, 1H), 6.31 (td, *J* = 6.7, 1.4 Hz, 1H), 4.57 (q, *J* = 7.0 Hz, 1H), 4.39 - 4.25 (m, 1H), 2.37 - 2.14 (m, 2H), 2.10 - 1.96 (m, 3H), 1.64 (td, *J* = 12.7, 7.1 Hz, 2H). |
| C57 | 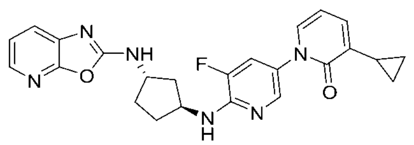 $^1$H NMR (400 MHz, DMSO-$d_6$) δ 8.44 - 8.41 (d, *J* = 7.0 Hz, 1H), 7.87 - 7.84 (m, 2H), 7.62 - 7.55 (m, 2H), 7.48 - 7.44 (dd, *J* = 6.8, 1.9 Hz, 1H), 7.21 - 7.16 (dd, *J* = 7.7, 5.1 Hz, 1H), 7.06 - 6.97 (ddd, *J* = 19.6, 7.2, 1.7 Hz, 2H), 6.25 - 6.19 (t, *J* = 6.9 Hz, 1H), 4.61 - 4.53 (q, *J* = 6.9 Hz, 1H), 4.36 - 4.26 (q, *J* = 6.6 Hz, 1H), 2.27 - 2.14 (m, 2H), 2.09 - 2.02 (m, 3H), 1.75 - 1.59 (m, 2H), 0.89 - 0.85 (m, 2H), 0.70 - 0.61 (m, 2H). | C58 | 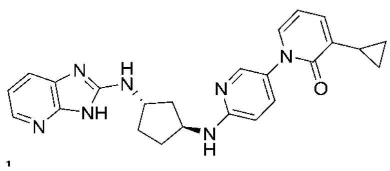 $^1$H NMR (400 MHz, DMSO-$d_6$) δ 7.95 (d, *J* = 2.7 Hz, 2H), 7.53 (ddd, *J* = 11.3, 6.9, 2.1 Hz, 3H), 7.43 (dd, *J* = 8.9, 2.7 Hz, 1H), 6.98 (d, *J* = 6.7 Hz, 2H), 6.70 - 6.51 (m, 2H), 6.44 (dd, *J* = 15.8, 1.8 Hz, 1H), 6.30 (t, *J* = 6.9 Hz, 1H), 4.35 (dq, *J* = 24.7, 6.6 Hz, 2H), 2.22 (ddd, *J* = 15.7, 10.5, 6.1 Hz, 2H), 2.07 - 1.94 (m, 2H), 1.84 (dd, *J* = 6.6, 1.6 Hz, 3H), 1.75 - 1.48 (m, 2H). |
| C59 | 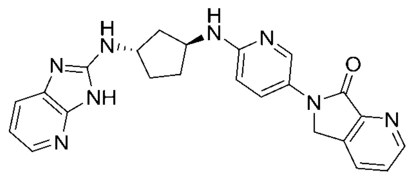 $^1$H NMR (400 MHz, DMSO-$d_6$) δ 8.76 (dd, *J* = 4.7, 1.5 Hz, 1H), 8.37 (d, *J* = 2.7 Hz, 1H), 8.14 (s, 1H), 8.11 (dd, *J* = 7.8, 1.5 Hz, 1H), 7.94 - 7.82 (m, 2H), 7.62 (dd, *J* = 7.7, 4.8 Hz, 1H), 7.47 (dd, *J* = 7.6, 1.4 Hz, 2H), 6.92 (dd, *J* = 7.7, 5.2 Hz, 1H), 6.73 (d, *J* = 6.7 Hz, 1H), 6.57 (d, *J* = 9.0 Hz, 1H), 4.93 (s, 2H), 4.31 (dq, *J* = 14.1, 6.8 Hz, 2H), 2.20 (tp, *J* = 12.4, 6.7, 6.0 Hz, 2H), 1.97 (dh, *J* = 19.9, 6.6 Hz, 2H), 1.75 - 1.40 (m, 2H). | C60 | 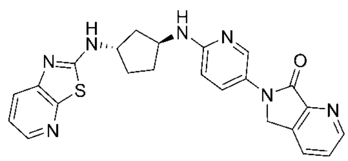 $^1$H NMR (400 MHz, DMSO-$d_6$) δ 8.76 (d, *J* = 4.7 Hz, 1H), 8.47 (d, *J* = 6.7 Hz, 1H), 8.37 (d, *J* = 2.7 Hz, 1H), 8.09 (dd, *J* = 4.6, 2.9 Hz, 2H), 7.89 (dd, *J* = 9.0, 2.7 Hz, 1H), 7.66 (d, *J* = 6.6 Hz, 1H), 7.63 - 7.60 (m, 1H), 7.25 (t, *J* = 3.0 Hz, 1H), 6.75 (d, *J* = 6.9 Hz, 1H), 6.57 (d, *J* = 9.0 Hz, 1H), 4.93 (s, 2H), 4.41 - 4.32 (m, 2H), 2.24 - 2.18 (m, 2H), 1.98 (d, *J* = 7.5 Hz, 2H), 1.61 - 1.51 (m, 2H). |

(continued)

| | | | |
|---|---|---|---|
| C61 | 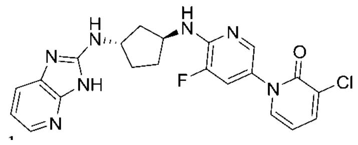<br>$^1$H NMR (400 MHz, DMSO-$d_6$) δ 8.15 (s, 1H), 7.92 - 7.80 (m, 3H), 7.70 (dd, *J* = 6.8, 1.9 Hz, 1H), 7.62 (dd, *J* = 11.9, 2.2 Hz, 1H), 7.41 - 7.33 (m, 1H), 7.13 (s, 1H), 7.02 (dd, *J* = 7.0, 1.6 Hz, 1H), 6.84 (t, *J* = 6.4 Hz, 1H), 6.32 (t, *J* = 7.1 Hz, 1H), 4.55 (q, *J* = 7.0 Hz, 1H), 4.30 (q, *J* = 6.7 Hz, 1H), 2.26 - 2.14 (m, 2H), 2.02 (tq, *J* = 11.5, 6.5, 5.9 Hz, 2H), 1.61 (td, *J* = 7.4, 6.9, 4.1 Hz, 2H). | C62 | 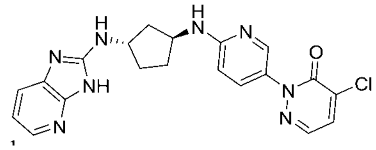<br>$^1$H NMR (400 MHz, DMSO-$d_6$) δ 8.20 - 8.07 (m, 1H), 7.99 (d, *J* = 4.5 Hz, 1H), 7.84 (d, *J* = 4.5 Hz, 2H), 7.54 (dd, *J* = 9.0, 2.7 Hz, 1H), 7.41 (s, 1H), 7.20 (s, 1H), 7.04 (d, *J* = 6.7 Hz, 1H), 6.88 (s, 1H), 6.54 (d, *J* = 8.9 Hz, 1H), 4.33 (d, *J* = 26.9 Hz, 2H), 2.19 (s, 2H), 1.98 (td, *J* = 16.8, 16.0, 7.1 Hz, 2H), 1.67 - 1.40 (m, 2H). |
| C63 | 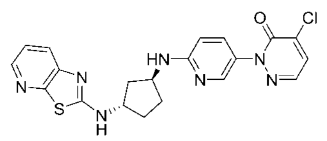 | $^1$H NMR (400 MHz, DMSO-$d_6$) δ 8.48 (d, *J* = 6.8 Hz, 1H), 8.15 - 8.09 (m, 2H), 8.00 (d, *J* = 4.5 Hz, 1H), 7.85 (d, *J* = 4.5 Hz, 1H), 7.68 (dd, *J* = 8.1, 1.5 Hz, 1H), 7.56 (dd, *J* = 8.9, 2.7 Hz, 1H), 7.28 - 7.24 (m, 1H), 7.07 (d, *J* = 6.9 Hz, 1H), 6.56 (d, *J* = 8.9 Hz, 1H), 4.40 (dd, *J* = 13.9, 7.0 Hz, 2H), 2.22 (ddt, *J* = 17.8, 12.9, 6.9 Hz, 2H), 1.98 (dd, *J* = 13.6, 6.7 Hz, 2H), 1.70 - 1.29 (m, -2H). | |
| C64 | 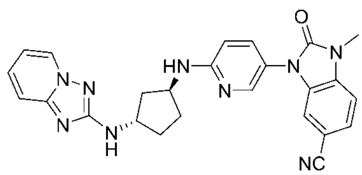<br>$^1$H NMR (400 MHz, DMSO-$d_6$) δ 8.19 (s, 1H), 8.13 (d, *J* = 2.6 Hz, 1H), 8.01 (d, *J* = 4.5 Hz, 1H), 7.86 (d, *J* = 4.5 Hz, 1H), 7.55 (dd, *J* = 9.0, 2.7 Hz, 2H), 7.04 (d, *J* = 7.0 Hz, 1H), 6.55 (d, *J* = 9.0 Hz, 1H), 4.37 (dt, *J* = 13.2, 6.7 Hz, 2H), 2.40 (s, 3H), 2.17 (dt, *J* = 9.9, 5.7 Hz, 2H), 1.99 - 1.91 (m, 2H), 1.63 - 1.52 (m, 2H). | C65 | 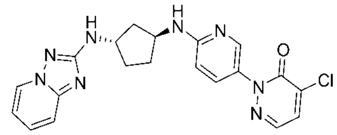<br>$^1$H NMR (400 MHz, DMSO-$d_6$) δ 8.62 (s, 1H), 8.15 (d, *J* = 6.0 Hz, 1H), 8.01 (d, *J* = 4.5 Hz, 1H), 7.85 (d, *J* = 4.5 Hz, 1H), 7.55 (dd, *J* = 9.0, 2.5 Hz, 1H), 7.43 (d, *J* = 7.0 Hz, 2H), 7.03 (d, *J* = 6.9 Hz, 1H), 6.88 (t, *J* = 6.6 Hz, 1H), 6.68 (s, 1H), 6.56 (d, *J* = 9.0 Hz, 1H), 4.35 (d, *J* = 8.1 Hz, 1H), 4.20 (s, 1H), 2.17 (dt, *J* = 11.5, 6.0 Hz, 2H), 1.99 (dt, *J* = 13.4, 6.8 Hz, 1H), 1.91 (q, *J* = 6.6 Hz, 1H), 1.55 (ddd, *J* = 25.0, 12.7, 6.6 Hz, 2H). |
| C66 | 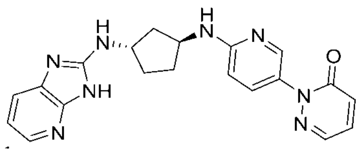<br>$^1$H NMR (400 MHz, DMSO-$d_6$) δ 8.13 (d, *J* = 2.6 Hz, 1H), 8.03 (dd, *J* = 3.9, 1.6 Hz, 1H), 7.93 (d, *J* = 5.3 Hz, 1H), 7.63 (s, 1H), 7.55 (dd, *J* = 9.0, 2.7 Hz, 1H), 7.53 - 7.44 (m, 2H), 7.04 (dd, *J* = 9.5, 1.6 Hz, 1H), 6.99 (d, *J* = 6.8 Hz, 1H), 6.95 (dd, *J* = 7.7, 5.3 Hz, 1H), 6.56 (d, *J* = 9.0 Hz, 1H), 4.34 (dq, J = 24.0, 6.7 Hz, 2H), 2.21 (dq, *J* = 11.6, 6.1 Hz, 2H), 2.00 (dq, *J* = 23.2, 6.6 Hz, 2H), 1.69 - 1.48 (m, 2H). | C67 | 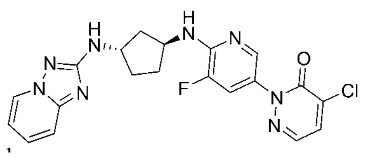<br>$^1$H NMR (400 MHz, DMSO-$d_6$) δ 8.58 (dt, *J* = 6.7, 1.2 Hz, 1H), 8.10 - 7.97 (m, 2H), 7.86 (d, *J* = 4.5 Hz, 1H), 7.64 (dd, *J* = 12.0, 2.1 Hz, 1H), 7.47 - 7.31 (m, 2H), 7.01 (dd, *J* = 7.2, 1.7 Hz, 1H), 6.85 (td, *J* = 6.7, 1.6 Hz, 1H), 6.64 (d, *J* = 7.3 Hz, 1H), 4.54 (q, *J* = 6.9 Hz, 1H), 4.20 (q, *J* = 6.5 Hz, 1H), 2.25 - 2.11 (m, 2H), 1.98 (td, *J* = 7.2, 6.7, 1.8 Hz, 2H), 1.70 - 1.51 (m, 2H). |

(continued)

| | | | |
|---|---|---|---|
| C68 | 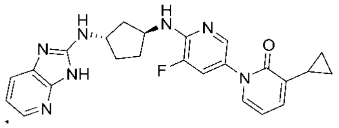<br>$^1$H NMR (400 MHz, DMSO-$d_6$) δ 7.97 - 7.81 (m, 2H), 7.63 - 7.49 (m, 3H), 7.45 (d, $J$ = 7.8 Hz, 1H), 7.01 - 6.82 (m, 2H), 6.60 (dd, $J$ = 15.8, 6.7 Hz, 1H), 6.42 (dd, $J$ = 15.8, 1.8 Hz, 1H), 6.29 (t, $J$ = 6.9 Hz, 1H), 4.55 (q, $J$ = 7.0 Hz, 1H), 4.31 (q, $J$ = 6.5 Hz, 1H), 2.19 (ddd, $J$ = 11.8, 7.0, 4.1 Hz, 2H), 2.02 (dd, $J$ = 14.5, 7.9 Hz, 2H), 1.83 (dd, $J$ = 6.7, 1.7 Hz, 3H), 1.63 (dt, $J$ = 10.3, 6.2 Hz, 2H). | C69 | 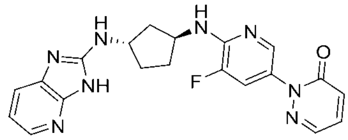<br>$^1$H NMR (400 MHz, DMSO-$d_6$) δ 8.11 - 7.98 (m, 2H), 7.92 (d, $J$ = 5.3 Hz, 1H), 7.64 (dd, $J$ = 12.1, 2.1 Hz, 2H), 7.55 - 7.41 (m, 2H), 7.06 (dd, $J$ = 9.5, 1.6 Hz, 1H), 6.97 (ddd, $J$ = 13.0, 7.4, 3.5 Hz, 2H), 4.56 (q, $J$ = 7.1 Hz, 1H), 4.31 (q, $J$ = 6.6 Hz, 1H), 2.19 (ddt, $J$ = 12.4, 9.3, 4.5 Hz, 2H), 2.11 - 1.98 (m, 2H), 1.63 (dtt, $J$ = 10.9, 7.1, 3.1 Hz, 2H). |
| C70 | 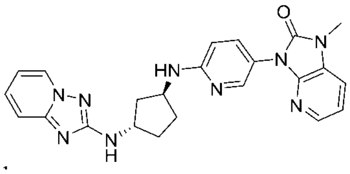<br>$^1$H NMR (400 MHz, DMSO-$d_6$) δ 8.61 - 8.57 (dt, $J$ = 6.7, 1.2 Hz, 1H), 8.10 - 8.07 (d, $J$ = 2.6 Hz, 1H), 7.95 - 7.91 (dd, $J$ = 5.2, 1.4 Hz, 1H), 7.59 - 7.54 (dd, $J$ = 7.7, 1.4 Hz, 1H), 7.52 - 7.47 (dd, $J$ = 8.9, 2.6 Hz, 1H), 7.44 - 7.34 (m, 2H), 7.16 - 7.09 (dd, $J$ = 7.7, 5.2 Hz, 1H), 6.93 - 6.82 (m, 2H), 6.67 - 6.56 (dd, $J$ = 21.8, 8.1 Hz, 2H), 4.38 - 4.29 (q, $J$ = 6.1 Hz, 1H), 4.24 - 4.14 (p, $J$ = 6.7 Hz, 1H), 3.41 - 3.39 (s, 3H), 2.26 - 2.10 (m, 2H), 2.05 - 1.84 (m, 2H), 1.64 - 1.43 (m, 2H). | C71 | 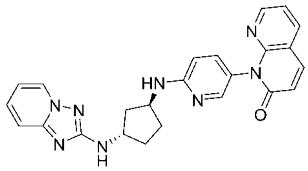<br>$^1$H NMR (400 MHz, DMSO-$d_6$) δ 8.63 - 8.56 (d, $J$ = 6.7 Hz, 1H), 8.48 - 8.38 (d, $J$ = 4.8 Hz, 1H), 8.24 - 8.14 (d, $J$ = 7.7 Hz, 1H), 8.07 - 7.94 (d, $J$ = 9.5 Hz, 1H), 7.82 - 7.75 (d, $J$ = 2.6 Hz, 1H), 7.46 - 7.35 (dt, $J$ = 14.9, 8.5 Hz, 2H), 7.32 - 7.21 (ddd, $J$ = 16.1, 8.4, 3.8 Hz, 2H), 6.91 - 6.72 (m, 3H), 6.70 - 6.53 (dd, $J$ = 29.1, 8.0 Hz, 2H), 4.40 - 4.29 (m, 1H), 4.27 - 4.11 (q, $J$ = 6.9 Hz, 1H), 2.26 - 2.09 (m, 2H), 2.05 - 1.83 (qt, $J$ = 12.8, 5.7 Hz, 2H), 1.67 - 1.46 (m, 2H). |
| C72 | 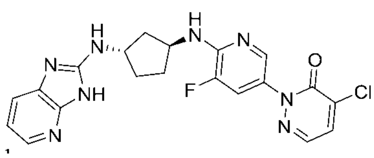<br>$^1$H NMR (400 MHz, DMSO-$d_6$) δ 8.15 (s, 1H), 8.05 (d, $J$ = 2.2 Hz, 1H), 8.01 (d, $J$ = 4.5 Hz, 1H), 7.86 (d, $J$ = 4.5 Hz, 2H), 7.65 (dd, $J$ = 12.0, 2.2 Hz, 1H), 7.37 (d, $J$ = 7.6 Hz, 1H), 7.04 (d, $J$ = 7.1 Hz, 2H), 6.84 (s, 1H), 4.56 (h, $J$ = 7.0 Hz, 1H), 4.30 (h, $J$ = 6.5 Hz, 1H), 2.18 (dt, $J$ = 12.6, 6.1 Hz, 2H), 2.01 (td, $J$ = 8.0, 7.4, 3.7 Hz, 2H), 1.61 (tt, $J$ = 12.0, 7.0 Hz, 2H). | C73 | 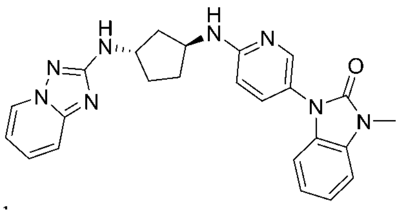<br>$^1$H NMR (400 MHz, DMSO-$d_6$) δ 8.58 (dt, $J$ = 6.7, 1.1 Hz, 1H), 8.04 (d, $J$ = 2.7 Hz, 1H), 7.46 (dd, $J$ = 8.9, 2.7 Hz, 1H), 7.44 - 7.34 (m, 2H), 7.22 (dd, $J$ = 7.8, 1.2 Hz, 1H), 7.12 (td, $J$ = 7.6, 1.2 Hz, 1H), 7.03 (td, $J$ = 7.6, 1.2 Hz, 1H), 6.96 (d, $J$ = 6.9 Hz, 1H), 6.93 - 6.81 (m, 2H), 6.70 - 6.56 (m, 2H), 4.35 (q, $J$ = 6.6 Hz, 1H), 4.20 (q, $J$ = 6.8 Hz, 1H), 3.38 (s, 3H), 2.23 - 2.12 (m, 2H), 2.03 - 1.87 (m, 2H), 1.66 - 1.44 (m, 2H). |

(continued)

| | | | |
|---|---|---|---|
| C74 | 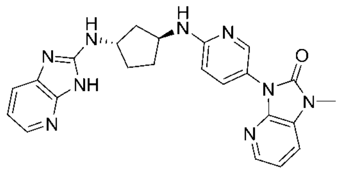<br>$^1$H NMR (400 MHz, DMSO-$d_6$) δ 9.01 (s, 1H), 8.16 - 8.09 (m, 1H), 8.05 (d, *J* = 6.0 Hz, 1H), 7.94 (dd, *J* = 5.2, 1.4 Hz, 1H), 7.81 (d, *J* = 7.7 Hz, 1H), 7.57 (ddd, *J* = 8.3, 5.5, 2.0 Hz, 2H), 7.20 (dd, *J* = 7.6, 5.9 Hz, 1H), 7.15 (dd, *J* = 7.7, 5.2 Hz, 1H), 7.07 (s, 1H), 6.64 (d, *J* = 8.9 Hz, 1H), 4.45 - 4.24 (m, 2H), 3.41 (s, 3H), 2.35 - 2.13 (m, 2H), 2.05 (dp, *J* = 20.2, 7.0 Hz, 2H), 1.69 (ddd, *J* = 14.6, 9.0, 6.0 Hz, 1H), 1.60 (qd, *J* = 8.7, 8.1, 5.7 Hz, 1H). | C75 | 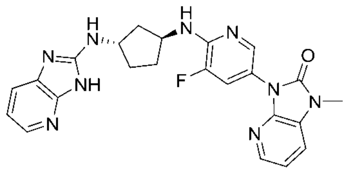<br>$^1$H NMR (400 MHz, DMSO-$d_6$) δ 8.06 (d, *J* = 2.0 Hz, 1H), 7.96 (d, *J* = 5.2 Hz, 1H), 7.89 (s, 1H), 7.64 (dd, *J* = 12.1, 2.1 Hz, 1H), 7.59 (d, *J* = 7.7 Hz, 1H), 7.43 (s, 1H), 7.33 (d, *J* = 12.2 Hz, 1H), 7.16 (dd, *J* = 7.7, 5.2 Hz, 1H), 6.94 (d, *J* = 6.9 Hz, 1H), 6.89 (s, 1H), 4.57 (q, *J* = 7.0 Hz, 1H), 4.31 (q, *J* = 6.6 Hz, 1H), 3.41 (s, 3H), 2.20 (dt, *J* = 12.4, 6.0 Hz, 2H), 2.04 (td, *J* = 15.6, 13.7, 7.3 Hz, 2H), 1.74 - 1.57 (m, 2H). |
| C76 | 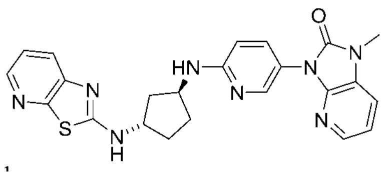<br>$^1$H NMR (400 MHz, DMSO-$d_6$) δ 8.47 (d, *J* = 6.8 Hz, 1H), 8.10 (d, *J* = 2.7 Hz, 1H), 8.08 (dd, *J* = 4.8, 1.5 Hz, 1H), 7.93 (dd, *J* = 5.2, 1.4 Hz, 1H), 7.67 (dd, *J* = 8.0, 1.5 Hz, 1H), 7.57 (dd, *J* = 7.7, 1.4 Hz, 1H), 7.52 (dd, *J* = 8.9, 2.6 Hz, 1H), 7.40 (d, *J* = 7.7 Hz, 1H), 7.24 (dd, *J* = 8.0, 4.8 Hz, 1H), 7.14 (dd, *J* = 7.8, 5.2 Hz, 1H), 7.00 - 6.95 (m, 1H), 6.60 (d, *J* = 8.9 Hz, 1H), 4.37 (dd, *J* = 14.8, 8.2 Hz, 2H), 3.40 (s, 3H), 2.22 (dq, *J* = 11.6, 6.7, 5.9 Hz, 2H), 2.02 - 1.97 (m, 2H), 1.66 - 1.53 (m, 2H). | C77 | 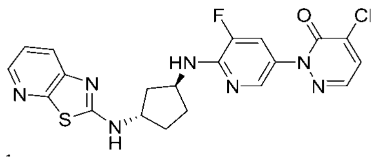<br>$^1$H NMR (400 MHz, DMSO-$d_6$) δ 8.48 (d, *J* = 6.9 Hz, 1H), 8.10 (s, 1H), 8.07 (s, 1H), 8.03 (s, 1H), 7.87 (s, 1H), 7.69 (s, 1H), 7.67 (d, *J* = 1.5 Hz, 1H), 7.26 (s, 1H), 7.11 (s, 1H), 4.63 - 4.41 (m, 2H), 2.21 (d, *J* = 5.2 Hz, 2H), 2.05 (s, 2H), 1.65 (d, *J* = 10.8 Hz, 2H). |
| C78 | 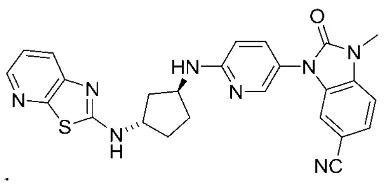<br>$^1$H NMR (400 MHz, DMSO-$d_6$) δ 8.47 (d, *J* = 6.8 Hz, 1H), 8.10 - 8.07 (m, 2H), 7.69 - 7.65 (m, 1H), 7.62 - 7.59 (m, 1H), 7.50 (d, *J* = 3.0 Hz, 1H), 7.42 (d, *J* = 8.2 Hz, 1H), 7.31 (s, 1H), 7.24 (dd, *J* = 8.1, 4.8 Hz, 1H), 7.08 (d, *J* = 6.8 Hz, 1H), 6.62 (d, *J* = 8.9 Hz, 1H), 4.41 (dd, *J* = 12.8, 6.6 Hz, 2H), 3.43 (s, 3H), 2.21 (q, *J* = 7.6, 6.2 Hz, 2H), 1.99 (t, *J* = 8.0 Hz, 2H), 1.65 (d, *J* = 7.6 Hz, 2H). | C79 | 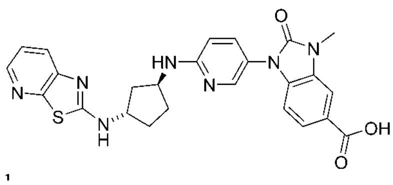<br>$^1$H NMR (400 MHz, DMSO-$d_6$) δ 8.47 (d, *J* = 6.8 Hz, 1H), 8.08 (s, 2H), 7.77 - 7.70 (m, 2H), 7.67 (d, *J* = 8.0 Hz, 1H), 7.49 (dd, *J* = 8.8, 2.4 Hz, 1H), 7.25 (dd, *J* = 8.0, 4.4 Hz, 1H), 7.07 (s, 1H), 6.96 (d, *J* = 7.9 Hz, 1H), 6.63 (d, *J* = 8.9 Hz, 1H), 4.40 (dd, *J* = 12.9, 6.5 Hz, 2H), 3.44 (s, 3H), 2.21 (dq, *J* = 12.5, 6.0, 5.1 Hz, 2H), 1.99 (dd, *J* = 11.7, 6.3 Hz, 2H), 1.59 (ddd, *J* = 13.7, 10.1, 4.7 Hz, 2H). |

(continued)

| | | | |
|---|---|---|---|
| C80 | 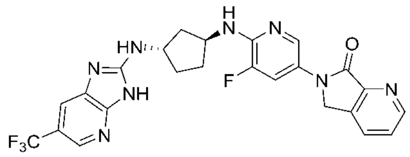 $^1$H NMR (400 MHz, DMSO-$d_6$) δ 8.77 (dd, *J* = 4.8, 1.5 Hz, 1H), 8.26 (d, *J* = 2.2 Hz, 2H), 8.13 (dd, *J* = 7.8, 1.4 Hz, 1H), 8.04 (dd, *J* = 13.0, 2.2 Hz, 1H), 7.75 - 7.38 (m, 3H), 6.72 (s, 1H), 4.97 (s, 2H), 4.53 (q, *J* = 6.9 Hz, 1H), 4.34 (q, *J* = 6.8 Hz, 1H), 2.19 (t, *J* = 6.3 Hz, 2H), 2.02 (d, *J* = 7.3 Hz, 2H), 1.62 (dt, *J* = 11.4, 6.2 Hz, 2H). | C81 | 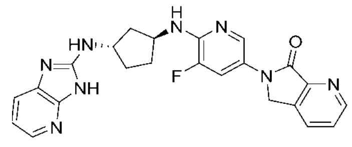 $^1$H NMR (400 MHz, DMSO-$d_6$) δ 8.77 (dd, *J* = 4.7, 1.5 Hz, 1H), 8.26 (d, *J* = 2.2 Hz, 1H), 8.12 (dd, *J* = 7.9, 1.5 Hz, 1H), 8.04 (dd, *J* = 13.1, 2.2 Hz, 1H), 7.92 (dd, *J* = 5.4, 1.4 Hz, 1H), 7.64 (dd, *J* = 7.7, 4.7 Hz, 2H), 7.50 (dd, *J* = 7.7, 1.4 Hz, 1H), 6.94 (dd, *J* = 7.6, 5.3 Hz, 1H), 6.70 (dd, *J* = 7.1, 1.6 Hz, 1H), 4.97 (s, 2H), 4.53 (q, *J* = 6.9 Hz, 1H), 4.31 (q, *J* = 6.6 Hz, 1H), 2.27 - 2.11 (m, 2H), 2.03 (td, *J* = 6.7, 2.3 Hz, 2H), 1.63 (qd, *J* = 6.4, 2.2 Hz, 2H). |
| C82 | 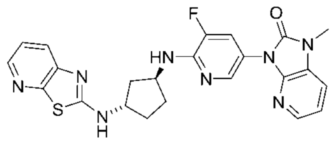 $^1$H NMR (400 MHz, DMSO-$d_6$) δ 8.47 (d, *J* = 6.8 Hz, 1H), 8.09 (s, 1H), 8.06 (d, *J* = 2.1 Hz, 1H), 7.96 (dd, *J* = 5.2, 1.4 Hz, 1H), 7.68 - 7.66 (m, 1H), 7.66 - 7.62 (m, 1H), 7.59 (dd, *J* = 7.8, 1.4 Hz, 1H), 7.26 - 7.23 (m, 1H), 7.16 (dd, *J* = 7.8, 5.2 Hz, 1H), 7.01 - 6.97 (m, 1H), 4.57 (q, *J* = 7.1 Hz, 1H), 4.43 (q, *J* = 6.4 Hz, 1H), 3.41 (s, 3H), 2.21 (dt, *J* = 12.8, 6.5 Hz, 2H), 2.08 - 2.03 (m, 2H), 1.64 (tt, *J* = 12.7, 8.0 Hz, 2H). | C83 | 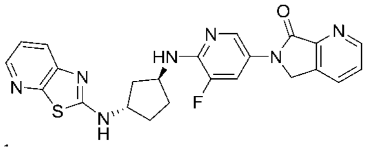 $^1$H NMR (400 MHz, DMSO-$d_6$) δ 8.77 (d, *J* = 4.7 Hz, 1H), 8.53 (d, *J* = 4.7 Hz, 1H), 8.26 (d, *J* = 2.1 Hz, 1H), 8.13 (d, *J* = 7.8 Hz, 1H), 8.09 (dd, *J* = 4.8, 1.6 Hz, 1H), 8.05 (dd, *J* = 13.0, 2.2 Hz, 1H), 7.67 - 7.65 (m, 1H), 7.65 - 7.61 (m, 1H), 7.26 - 7.23 (m, 1H), 6.75 (d, *J* = 7.1 Hz, 1H), 4.97 (s, 2H), 4.47 (dt, *J* = 41.5, 6.6 Hz, 2H), 2.21 (dd, *J* = 13.2, 6.0 Hz, 2H), 2.04 (d, *J* = 6.8 Hz, 2H), 1.63 (dd, *J* = 12.4, 6.2 Hz, 2H). |
| C84 | 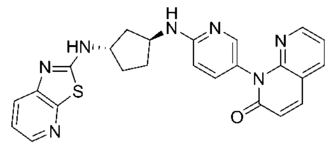 $^1$H NMR (400 MHz, DMSO-$d_6$) δ 8.47 (d, *J* = 6.8 Hz, 1H), 8.44 (dd, *J* = 4.7, 1.9 Hz, 1H), 8.20 (dd, *J* = 7.8, 1.8 Hz, 1H), 8.09 (dd, *J* = 4.8, 1.5 Hz, 1H), 8.04 (d, *J* = 9.6 Hz, 1H), 7.80 (d, *J* = 2.5 Hz, 1H), 7.68 (dd, *J* = 8.0, 1.5 Hz, 1H), 7.31 - 7.23 (m, 3H), 6.89 (d, *J* = 6.8 Hz, 1H), 6.76 (d, *J* = 9.5 Hz, 1H), 6.59 (d, *J* = 8.8 Hz, 1H), 4.40 (dq, *J* = 19.4, 6.6 Hz, 2H), 2.22 (ddd, *J* = 20.3, 12.5, 5.1 Hz, 2H), 2.03 - 1.97 (m, 2H), 1.67 - 1.55 (m, 2H). | C85 | 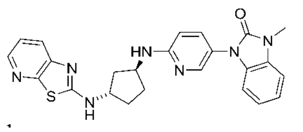 $^1$H NMR (400 MHz, DMSO-$d_6$) δ 8.02 (dd, *J* = 4.8, 1.5 Hz, 1H), 7.99 (d, *J* = 2.6 Hz, 1H), 7.60 (dd, *J* = 8.0, 1.5 Hz, 1H), 7.40 (dd, *J* = 8.9, 2.6 Hz, 1H), 7.20 - 7.16 (m, 1H), 7.16 - 7.14 (m, 1H), 7.05 (td, J = 7.7, 1.2 Hz, 1H), 6.99 - 6.94 (m, 2H), 6.81 (dd, *J* = 7.8, 1.1 Hz, 1H), 6.55 (d, *J* = 8.9 Hz, 1H), 4.31 (dd, *J* = 14.0, 7.4 Hz, 2H), 3.32 (s, 3H), 2.14 (tdd, *J* = 12.4, 8.2, 5.2 Hz, 2H), 1.99 - 1.89 (m, 2H), 1.61 - 1.47 (m, 2H). |
| C86 | 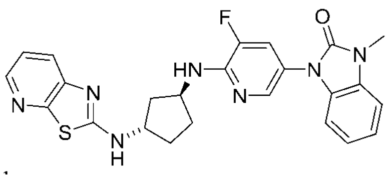 $^1$H NMR (400 MHz, DMSO-$d_6$) δ 8.40 (d, *J* = 6.8 Hz, 1H), 8.04 (s, 1H), 7.92 (d, *J* = 2.1 Hz, 1H), 7.60 (d, *J* = 8.0 Hz, 1H), 7.53 (dd, *J* = 11.9, 2.1 Hz, 1H), 7.17 (dt, *J* = 7.9, 2.8 Hz, 2H), 7.09 - 7.05 (m, 1H), 7.00 - 6.95 (m, 2H), 6.90 - 6.87 (m, 1H), 4.44 (dq, J = 59.6, 6.8 Hz, 2H), 3.32 (s, 3H), 2.14 (dtd, *J* = 14.9, 7.3, 4.0 Hz, 2H), 2.01 - 1.96 (m, 2H), 1.64 - 1.53 (m, 2H). | C87 | 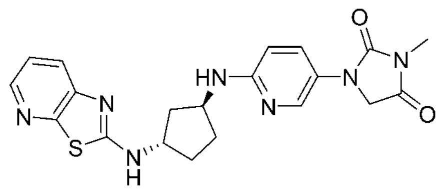 $^1$H NMR (400 MHz, DMSO-$d_6$) δ 8.44 (d, *J* = 6.8 Hz, 1H), 8.10 (d, *J* = 2.7 Hz, 1H), 8.08 (dd, *J* = 4.8, 1.5 Hz, 1H), 7.65 (ddd, *J* = 9.0, 5.1, 2.1 Hz, 2H), 7.24 (dd, *J* = 8.1, 4.8 Hz, 1H), 6.66 (d, *J* = 6.9 Hz, 1H), 6.50 (d, *J* = 9.0 Hz, 1H), 4.39 (s, 2H), 4.38 (s, 2H), 2.91 (s, 3H), 2.21 - 2.10 (m, 2H), 2.00 - 1.90 (m, 2H), 1.61 - 1.47 (m, 2H). |

(continued)

| | | | |
|---|---|---|---|
| C88 | 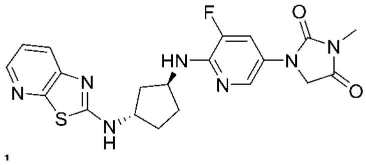 $^1$H NMR (400 MHz, DMSO-$d_6$) δ 8.44 (d, *J* = 6.9 Hz, 1H), 8.08 (dd, *J* = *4.8,* 1.5 Hz, 1H), 8.00 (d, *J* = 2.2 Hz, 1H), 7.75 (dd, *J* = 12.9, 2.2 Hz, 1H), 7.65 (dd, *J* = 8.0, 1.4 Hz, 1H), 7.24 (dd, *J* = 8.0, 4.7 Hz, 1H), 6.64 (dd, *J* = 7.1, 1.5 Hz, 1H), 4.50 - 4.40 (m, 2H), 4.40 (s, 2H), 2.92 (s, 3H), 2.23 - 2.11 (m, 2H), 1.99 (t, *J* = 6.8 Hz, 2H), 1.63 - 1.56 (m, 2H). | C89 | 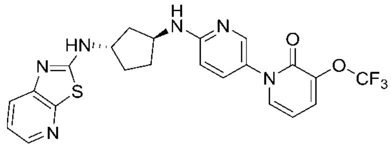 $^1$H NMR (400 MHz, DMSO-$d_6$) δ 8.09 - 8.08 (m, 1H), 7.98 (d, *J* = 2.7 Hz, 1H), 7.71 (ddd, *J* = 7.0, 5.1, 1.7 Hz, 2H), 7.67 - 7.65 (m, 1H), 7.45 (dd, *J* = 8.9, 2.7 Hz, 1H), 7.24 (dd, *J* = 4.0, 2.1 Hz, 1H), 7.06 (d, *J* = 6.9 Hz, 1H), 6.57 - 6.54 (m, 1H), 6.46 - 6.41 (m, 1H), 6.33 (t, *J* = 7.2 Hz, 1H), 4.41 - 4.34 (m, 2H), 2.22 - 2.16 (m, 2H), 1.98 (dd, *J* = 14.3, 7.4 Hz, 2H), 1.62 - 1.52 (m, 2H). |
| C90 | 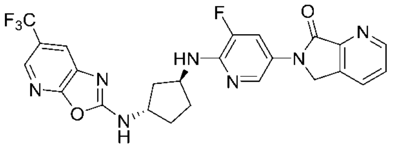 $^1$H NMR (400 MHz, DMSO-$d_6$) δ 8.83 - 8.79 (d, *J* = 7.1 Hz, 1H), 8.78 - 8.75 (m, 1H), 8.27 - 8.24 (dd, *J* = 5.2, 2.0 Hz, 2H), 8.15 - 8.11 (m, 1H), 8.07 - 8.02 (dd, *J* = 13.1, 2.2 Hz, 1H), 7.95 - 7.92 (d, *J* = 2.1 Hz, 1H), 7.66 - 7.61 (dd, *J* = 7.7, 4.7 Hz, 1H), 6.78 - 6.74 (d, *J* = 7.2 Hz, 1H), 4.99 - 4.95 (s, 2H), 4.59 - 4.49 (q, *J* = 6.7 Hz, 1H), 4.38 - 4.28 (q, *J* = 6.7 Hz, 1H), 2.27 - 2.14 (dt, *J* = 11.4, 5.7 Hz, 2H), 2.09 - 2.00 (t, *J* = 6.9 Hz, 2H), 1.74 - 1.58 (tt, *J* = 13.1, 7.5 Hz, 2H). | C91 | 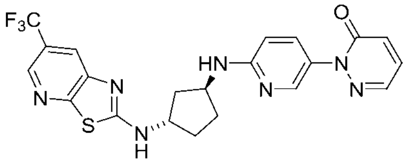 $^1$H NMR (400 MHz, DMSO-$d_6$) δ 8.84 (d, *J* = 6.8 Hz, 1H), 8.45 - 8.44 (m, 1H), 8.12 (d, *J* = 2.7 Hz, 1H), 8.02 (dd, *J* = 3.8, 1.7 Hz, 1H), 7.98 (d, *J* = 2.0 Hz, 1H), 7.53 (dd, *J* = 8.9, 2.7 Hz, 1H), 7.46 (dd, *J* = 9.5, 3.8 Hz, 1H), 7.03 (dd, *J* = 9.5, 1.7 Hz, 2H), 6.54 (d, *J* = 8.9 Hz, 1H), 4.46 - 4.35 (m, 2H), 2.26 - 2.17 (m, 2H), 2.03 - 1.93 (m, 2H), 1.59 (ddt, *J* = 21.2, 9.8, 4.1 Hz, 2H). |
| C92 | 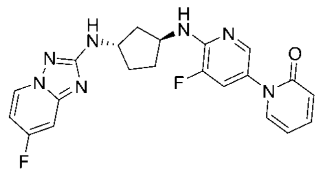 $^1$H NMR (400 MHz, DMSO-$d_6$) δ 8.66 (dd, *J* = 7.4, 5.6 Hz, 1H), 7.84 (d, *J* = 2.1 Hz, 1H), 7.64 (dd, *J* = 6.9, 2.1 Hz, 1H), 7.54 (dd, *J* = 11.9, 2.2 Hz, 1H), 7.49 (ddd, *J* = 8.9, 6.6, 2.1 Hz, 1H), 7.27 (dd, *J* = 9.7, 2.8 Hz, 1H), 6.92 (dd, *J* = 7.2, 1.6 Hz, 1H), 6.86 (td, *J* = 7.6, 2.8 Hz, 1H), 6.74 (d, *J* = 7.2 Hz, 1H), 6.46 (d, *J* = 9.2 Hz, 1H), 6.29 (td, *J* = 6.7, 1.3 Hz, 1H), 4.51 (p, *J* = 6.8 Hz, 1H), 4.17 (h, *J* = 6.4 Hz, 1H), 2.15 (dt, *J* = 9.1, 4.1 Hz, 2H), 1.97 (td, *J* = 7.1, 6.7, 2.7 Hz, 2H), 1.65 - 1.52 (m, 2H). | C93 | 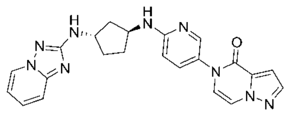 $^1$H NMR (400 MHz, DMSO-$d_6$) δ 8.58 (d, *J* = 6.6 Hz, 1H), 8.02 (d, *J* = 2.7 Hz, 1H), 7.96 (d, *J* = 2.2 Hz, 1H), 7.82 (d, *J* = 6.0 Hz, 1H), 7.49 - 7.43 (m, 1H), 7.43 - 7.33 (m, 2H), 7.11 (d, *J* = 6.0 Hz, 1H), 7.08 (d, *J* = 2.2 Hz, 1H), 6.96 (d, *J* = 6.9 Hz, 1H), 6.86 (td, *J* = 6.7, 1.5 Hz, 1H), 6.64 (d, *J* = 7.3 Hz, 1H), 6.56 (d, *J* = 8.9 Hz, 1H), 4.33 (q, *J* = 6.6 Hz, 1H), 4.19 (q, *J* = 6.7 Hz, 1H), 2.16 (dq, *J* = 11.7, 6.0 Hz, 2H), 1.98 (dt, *J* = 13.4, 7.0 Hz, 1H), 1.89 (dt, *J* = 13.2, 6.7 Hz, 1H), 1.64 - 1.44 (m, 2H). |

(continued)

| | | | |
|---|---|---|---|
| C94 | 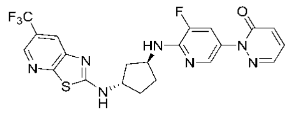 $^1$H NMR (400 MHz, DMSO-$d_6$) δ 8.83 (d, *J* = 6.8 Hz, 1H), 8.45 (dd, *J* = 2.1, 1.0 Hz, 1H), 8.06 - 8.03 (m, 2H), 7.98 (d, *J* = 2.0 Hz, 1H), 7.65 (dd, *J* = 12.1, 2.1 Hz, 1H), 7.48 (dd, *J* = 9.5, 3.9 Hz, 1H), 7.08 - 7.03 (m, 2H), 4.60 - 4.43 (m, 2H), 2.26 - 2.17 (m, 2H), 2.06 (ddd, *J* = 6.9, 4.8, 2.6 Hz, 2H), 1.65 (ddd, *J* = 12.1, 8.5, 4.8 Hz, 2H). | C95 | 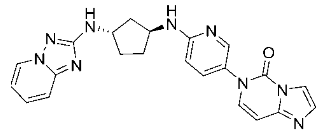 $^1$H NMR (400 MHz, DMSO-$d_6$) δ 8.59 (d, *J* = 6.6 Hz, 1H), 8.06 (d, *J* = 2.8 Hz, 1H), 7.86 (s, 1H), 7.56 - 7.32 (m, 5H), 7.00 (d, *J* = 6.8 Hz, 1H), 6.86 (t, *J* = 6.7 Hz, 1H), 6.72 (s, 1H), 6.65 (s, 1H), 6.56 (d, *J* = 8.9 Hz, 1H), 4.34 (q, *J* = 6.6 Hz, 1H), 4.19 (q, *J* = 6.6 Hz, 1H), 2.17 (dt, *J* = 9.7, 5.7 Hz, 2H), 1.98 (dt, *J* = 13.3, 6.8 Hz, 1H), 1.89 (dt, *J* = 13.2, 6.6 Hz, 1H), 1.66 - 1.46 (m, 2H). |

Example D1

Synthesis of 6'-(((1S,3S)-3-(pyrimidin-2-ylamino)cyclopentyl)amino)-2H-[1,3'-bipyridin]-2-one

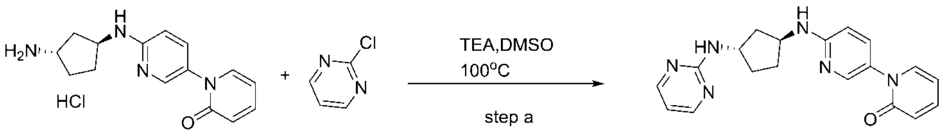


Step A: Synthesis of 6'-(((1S,3S)-3-(pyrimidin-2-ylamino)cyclopentyl)amino)-2H-[1,3'-bipyridin]-2-one

Compound 1 (0.25 g, 0.814 mmol) was dissolved in DMSO (5 mL). Compound 2 (93 mg, 0.814 mmol) and triethylamine (0.412 g, 4.07 mmol) were added. Under a nitrogen atmosphere, the mixture was reacted at 100 °C for 16 hours.

After completion of the reaction, the mixture was cooled to room temperature and quenched with water (10 mL). The mixture was extracted with ethyl acetate (50 mL × 3). The organic phases were combined, dried over anhydrous sodium sulfate, filtered, and concentrated. The resulting residue was purified by C18 column chromatography (water/-acetonitrile = 95/5 → acetonitrile) to obtain 50 mg of 6'-(((1S,35)-3-(pyrimidin-2-ylamino)cyclopentyl)amino)-2H-[1,3'-bipyridin]-2-one.

LC-MS: $[M+H]^+$ = 349. $^1$H NMR (400 MHz, DMSO-$d_6$) δ 8.25 (d, J = 4.7 Hz, 2H), 7.92 (d, J = 2.6 Hz, 1H), 7.60 (dd, J = 6.8, 2.1 Hz, 1H), 7.48 (ddd, J = 9.0, 6.7, 2.2 Hz, 1H), 7.40 (dd, J = 8.9, 2.7 Hz, 1H), 7.22 (d, J = 7.2 Hz, 1H), 6.93 (d, J = 6.9 Hz, 1H), 6.57 - 6.50 (m, 2H), 6.45 (d, J = 9.2 Hz, 1H), 6.28 (t, J = 6.7 Hz, 1H), 4.39 - 4.28 (m, 2H), 2.21 - 2.07 (m, 2H), 1.98 - 1.80 (m, 2H), 1.59 - 1.44 (m, 2H).

Example D2

Synthesis of 6'-(((1S,3S)-3-((5-fluoropyrimidin-2-yl)amino)cyclopentyl)amino)-2H-[1,3'-bipyridin]-2-one

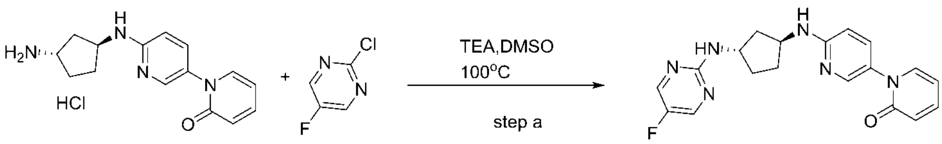


Step A: Synthesis of 6'-(((1S,3S)-3-((5-fluoropyrimidin-2-yl)amino)cyclopentyl)amino)-2H-[1,3'-bipyridin]-2-one

Compound 1 (0.3 g, 0.977 mmol) was dissolved in DMSO (5 mL). Compound 2 (129 mg, 0.977 mmol) and triethylamine (0.492 g, 4.89 mmol) were added. Under a nitrogen atmosphere, the mixture was reacted at 100 °C for 12

hours.

After completion of the reaction, the mixture was cooled to room temperature and quenched with water (10 mL). The mixture was extracted with ethyl acetate (50 mL × 3). The organic phases were combined, dried over anhydrous sodium sulfate, filtered, and concentrated. The resulting residue was purified by C18 column chromatography (water/-acetonitrile = 95/5 → acetonitrile) to obtain 60 mg of 6'-(((1S,3S)-3-((5-fluoropyridin-2-yl)amino)cyclopentyl)amino)-2H-[1,3'-bipyridin]-2-one.

LC-MS: $[M+H]^+$ = 367. $^1H$ NMR (400 MHz, DMSO-$d_6$) δ 8.33 (s, 2H), 7.91 (d, J = 2.7 Hz, 1H), 7.59 (dd, J = 6.9, 2.1 Hz, 1H), 7.52 - 7.26 (m, 3H), 6.92 (d, J = 6.9 Hz, 1H), 6.52 (d, J = 8.9 Hz, 1H), 6.44 (d, J = 9.2 Hz, 1H), 6.27 (t, J = 6.7 Hz, 1H), 4.39 - 4.18 (m, 2H), 2.22 - 2.03 (m, 2H), 1.95 - 1.79 (m, 2H), 1.61 - 1.40 (m, 2H).

Example D3

Synthesis of 1-(5-(((1S,3S)-3-(pyrimidin-2-ylamino)cyclopentyl)amino)pyrazin-2-yl)pyridin-2(1H)-one

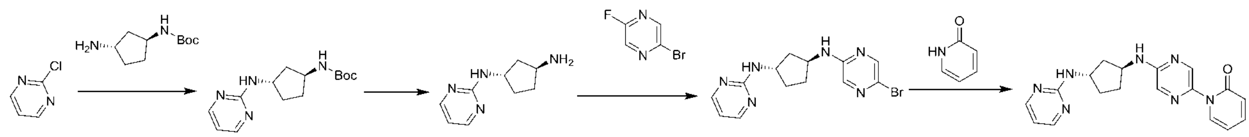

Step 1: Synthesis of tert-butyl ((1S,3S)-3-(pyrimidin-2-ylamino)cyclopentyl)carbamate

At 25 °C, 2-chloropyrimidine (2.00 g, 17.48 mmol), tert-butyl ((1S,3S)-3-aminocyclopentyl)carbamate (3.50 g, 17.48 mmol), and DIPEA (11.3 g, 81.4 mmol) were dissolved in DMSO (20 mL). After addition, the mixture was heated to 100 °C and reacted for 16 hours.

Water (100 mL) was added. Then the mixture was extracted with EA (100 mL * 2). The organic phases were combined, washed with saturated brine (50 mL) and concentrated to obtain the crude product. The resulting residue was purified by normal phase column chromatography (n-hexane:ethyl acetate 10:1 to 1:1) to obtain 3.4 g of tert-butyl ((1S,3S)-3-(pyrimidin-2-ylamino)cyclopentyl)carbamate (3.4 g, 69.9% yield) as a yellow solid. LC-MS: $[M+H]^+$ = 279.

Step 2: Synthesis of (1S,3S)-$N^1$-(pyrimidin-2-yl)cyclopentane-1,3-diamine

At 25 °C, tert-butyl ((1S,3S)-3-(pyrimidin-2-ylamino)cyclopentyl)carbamate (3.40 g, 12.21 mmol) was dissolved in HCl/dioxane (50 mL). Then the mixture was reacted at room temperature for 3 hours. After the completion of the reaction, the reaction mixture was concentrated directly under reduced pressure to dryness to obtain the crude product, which was used directly in the next step. LC-MS: $[M+H]^+$ = 179.

Step 3: Synthesis of (1S,3S)-$N^1$-(5-bromopyrazin-2-yl)-$N^3$-(pyrimidin-2-yl)cyclopentane-1,3-diamine

At 25 °C, (1S,3S)-$N^1$-(pyrimidin-2-yl)cyclopentane-1,3-diamine 2.5 hydrochloride (1.8 g, 6.68 mmol), 2-bromo-5-fluoropyrazine (1.42 g, 8.02 mmol), and potassium carbonate (4.62 g, 33.4 mmol) were dissolved in DMSO (20 mL). The mixture was reacted at 140 °C for 12 hours.

Water (100 mL) was added. Then the mixture was extracted with EA (100 mL * 2). The organic phases were combined, washed with saturated brine (50 mL) and concentrated to obtain the crude product. The resulting residue was purified by normal phase column chromatography (n-hexane:ethyl acetate 10:1 to 1:1) to obtain 1.6 g of (1S,3S)-$N^1$-(5-bromopyrazin-2-yl)-$N^3$-(pyrimidin-2-yl)cyclopentane-1,3-diamine (1.6 g, 71.4% yield) as a yellow solid. LC-MS: $[M+H]^+$ = 336.

Step 4: Synthesis of 1-(5-(((1S,3S)-3-(pyrimidin-2-ylamino)cyclopentyl)amino)pyrazin-2-yl)pyridin-2(1H)-one

At 25 °C, (1S,3S)-$N^1$-(5-bromopyrazin-2-yl)-$N^3$-(pyrimidin-2-yl)cyclopentane-1,3-diamine (750 mg, 2.24 mmol), pyridin-2(1H)-one (430 mg, 4.48 mmol), potassium phosphate (950 mg, 4.48 mmol), CuI (85 mg, 0.45 mmol), and (1R,2R)-N,N'-dimethylcyclohexane-1,2-diamine (130 mg, 0.9 mmol) were dissolved in DMSO (20 mL). Under a nitrogen atmosphere, the mixture was stirred at 130 °C for 12 hours.

Water (100 mL) was added. Then the mixture was extracted with EA (100 mL * 2). The organic phases were combined, washed with saturated brine (50 mL) and concentrated to obtain the crude product. The crude product was

purified by preparative chromatography using formic acid system to obtain 340 mg of 1-(5-(((1S,3S)-3-(pyrimidin-2-ylamino)cyclopentyl)amino)pyrazin-2-yl)pyridin-2(1H)-one (340 mg, 43.4% yield) as a light yellow solid. LC-MS: $[M+H]^+$ = 350.

$^1$H NMR (400 MHz, DMSO-$d_6$) δ 8.29 - 8.22 (m, 3H), 7.86 (d, J = 1.5 Hz, 1H), 7.72 (dd, J = 7.0, 2.1 Hz, 1H), 7.50 (ddd, J = 8.6, 6.6, 1.9 Hz, 2H), 7.25 (d, J = 7.3 Hz, 1H), 4.35 (dh, J = 25.8, 6.7 Hz, 2H), 2.25 - 2.05 (m, 1H), 1.91 (tdd, J = 13.2, 10.5, 6.2 Hz, 2H), 1.54 (ddd, J = 17.8, 12.7, 6.5 Hz, 2H).

Example D4

Synthesis of 3-chloro-1-(5-(((1S,3S)-3-(pyrimidin-2-ylamino)cyclopentyl)amino)pyrazin-2-yl)pyridin-2(1H)-one

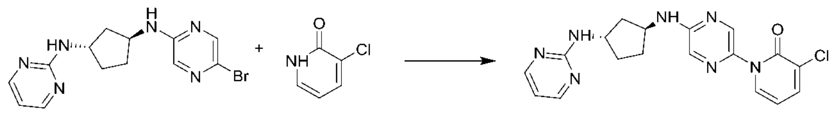

Step 1: Synthesis of 3-chloro-1-(5-(((1S,3S)-3-(pyrimidin-2-ylamino)cyclopentyl)amino)pyrazin-2-yl)pyridin-2(1H)-one

At 25 °C, (1S,3S)-$N^1$-(5-bromopyrazin-2-yl)-$N^3$-(pyrimidin-2-yl)cyclopentane-1,3-diamine (750 mg, 2.24 mmol), 3-chloropyridin-2(1H)-one (580 mg, 4.48 mmol), potassium phosphate (950 mg, 4.48 mmol), CuI (85 mg, 0.45 mmol), and (1R,2R)-N,N'-dimethylcyclohexane-1,2-diamine (130 mg, 0.9 mmol) were dissolved in DMSO (20 mL). Under a nitrogen atmosphere, the mixture was stirred at 130 °C for 12 hours.

Water (100 mL) was added. Then the mixture was extracted with EA (100 mL * 2). The organic phases were combined, washed with saturated brine (50 mL) and concentrated to obtain the crude product. The crude product was purified by preparative chromatography using formic acid system to obtain 310 mg of 3-chloro-1-(5-(((1S,3S)-3-(pyrimidin-2-ylamino)cyclopentyl)amino)pyrazin-2-yl)pyridin-2(1H)-one (310 mg, 34.2% yield) as a white solid. LC-MS: $[M+H]^+$ = 384.

$^1$H NMR (400 MHz, DMSO-$d_6$) δ 8.26 (d, J = 4.7 Hz, 3H), 7.85 (d, J = 8.8 Hz, 2H), 7.76 (d, J = 6.8 Hz, 1H), 7.57 (d, J = 6.9 Hz, 1H), 7.24 (d, J = 7.4 Hz, 1H), 6.54 (t, J = 4.8 Hz, 1H), 6.36 (t, J = 7.1 Hz, 1H), 4.35 (dq, J = 20.5, 6.9 Hz, 2H), 2.21 - 2.13 (m, 2H), 1.93 (dq, J = 12.4, 6.7 Hz, 2H), 1.61 - 1.52 (m, 2H).

Example D5

Synthesis of 1-(2-(((1S,3S)-3-((5-ethynylpyrimidin-2-yl)amino)cyclopentyl)amino)pyrimidin-5-yl)pyridin-2( 1H)-one

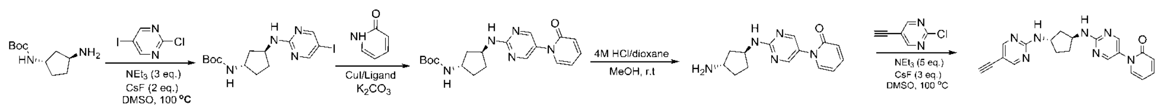


Step A: Synthesis of tert-butyl ((1S,3S)-3-((5-iodopyrimidin-2-yl)amino)cyclopentyl)carbamate

At room temperature, tert-butyl ((1S,3S)-3-aminocyclopentyl)carbamate (2 g, 9.99 mmol, 1.0 eq), 2-chloro-5-iodopyrimidine (2.88 g, 11.99 mmol, 1.2 eq), triethylamine (3.03 g, 29.97 mmol, 3.0 eq), and cesium fluoride (3.03 g, 19.98 mmol, 2.0 eq) were added to dimethyl sulfoxide (40 mL). After stirring evenly, the reaction mixture was transferred to an oil bath and heated to 100 °C. The mixture was reacted overnight.

After completion of the reaction, ethyl acetate (100 mL) was added to dilute the reaction mixture. Solid impurities were filtered off. Water (75 mL * 3) was added for extraction. The organic phases were combined and concentrated under reduced pressure. The crude product was purified by silica gel column chromatography (80 g silica gel, eluent: n-hexane/ethyl acetate = 10/3) to obtain 1.341 g of tert-butyl ((1S,3S)-3-((5-iodopyrimidin-2-yl)amino)cyclopentyl)carbamate (33.22% yield) as a white solid.

LC-MS: $[M+H]^+$ = 405.

Step B: Synthesis of tert-butyl ((1S,3S)-3-((5-(2-oxopyridin-1(2H)-yl)pyrimidin-2-yl)amino)cyclopentyl)carbamate

Under a nitrogen atmosphere, tert-butyl ((1S,3S)-3-((5-iodopyrimidin-2-yl)amino)cyclopentyl)carbamate (1.341 g, 3.32 mmol, 1.0 eq), pyridin-2(1H)-one (0.35 g, 3.65 mmol, 1.1 eq), N,N'-dimethylcyclohexane-1,2-diamine (0.094 g, 0.66 mmol, 0.2 eq), CuI (0.13 g, 0.66 mmol, 0.2 eq), and potassium carbonate (0.92 g, 6.64 mmol, 2.0 eq) were added to 1,4-dioxane (13 mL). After stirring evenly, the mixture was transferred to an oil bath, heated to 110 °C and reacted overnight.

After completion of the reaction, ethyl acetate (50 mL) was added to dilute the reaction mixture. Solid impurities were filtered off. The organic phase was concentrated under reduced pressure. The crude product was purified by silica gel column chromatography (80 g silica gel, eluent: ethyl acetate) to obtain 1.11 g of tert-butyl ((1S,3S)-3-((5-(2-oxopyridin-1(2H)-yl)pyrimidin-2-yl)amino)cyclopentyl)carbamate (90.09% yield) as a yellow solid. LC-MS: $[M+H]^+$ = 372.

Step C: Synthesis of 1-(2-(((1S,3S)-3-aminocyclopentyl)amino)pyrimidin-5-yl)pyridin-2(1H)-one

At room temperature, tert-butyl ((1S,3S)-3-((5-(2-oxopyridin-1(2H)-yl)pyrimidin-2-yl)amino)cyclopentyl)carbamate (1.11 g, 2.99 mmol, 1.0 eq) was dissolved in methanol (5 mL). A 4 M HCl solution in 1,4-dioxane (7.5 mL, 29.90 mmol, 10.0 eq) was added dropwise. The mixture was stirred vigorously at room temperature for about 4 hours.

After completion of the reaction, the organic solvent was evaporated under reduced pressure to obtain 0.81 g of 1-(2-(((1S,3S)-3-aminocyclopentyl)amino)pyrimidin-5-yl)pyridin-2(1H)-one (99.90% yield) as a yellow solid. LC-MS: $[M+H]^+$ = 272.

Step D: Synthesis of 1-(2-(((1S,3S)-3-((5-ethynylpyrimidin-2-yl)amino)cyclopentyl)amino)pyrimidin-5-yl)pyridin-2( 1H)-one

At room temperature, 1-(2-(((1S,3S)-3-aminocyclopentyl)amino)pyrimidin-5-yl)pyridin-2(1H)-one (0.19 g, 0.70 mmol, 1.0 eq), 2-chloro-5-ethynylpyrimidine (0.12 g, 0.84 mmol, 1.2 eq), triethylamine (0.35 g, 3.5 mmol, 5.0 eq), and cesium fluoride (0.32 g, 2.1 mmol, 3.0 eq) were dissolved in dimethyl sulfoxide (3 mL). After stirring evenly, the mixture was transferred to an oil bath, heated to 100 °C and reacted overnight.

After completion of the reaction, ethyl acetate (50 mL) was added to dilute the reaction mixture. Solid impurities were filtered off. The crude product was dissolved in a suitable amount of DMSO and purified by C18 column chromatography (120 g C18 silica gel, eluent: acetonitrile/water) to obtain 162 mg of 1-(2-(((1S,3S)-3-((5-ethynylpyrimidin-2-yl)amino)cyclopentyl)amino)pyrimidin-5-yl)pyridin-2( 1H)-one (61.23% yield) as a yellow solid.

LC-MS: $[M+H]^+$ = 374. NMR data: H NMR (400 MHz, DMSO-$d_6$) $\delta$ 8.35 (d, J = 27.7 Hz, 4H), 7.77 (d, J = 7.3 Hz, 1H), 7.69 - 7.60 (m, 2H), 7.50 (ddd, J = 8.9, 6.6, 2.1 Hz, 1H), 6.47 (dd, J = 9.2, 1.2 Hz, 1H), 6.31 (td, J = 6.7, 1.3 Hz, 1H), 4.42 - 4.31 (m, 2H), 4.22 (s, 1H), 2.11 (qd, J = 10.6, 10.2, 5.8 Hz, 2H), 1.91 (t, J = 6.9 Hz, 2H), 1.61 - 1.49 (m, 2H).

Example D6

Synthesis of 6'-(((1S,3S)-3-((5-ethynylpyrimidin-2-yl)amino)cyclopentyl)amino)-5'-fluoro-2H-[1,3'-bipyridin ]-2-one

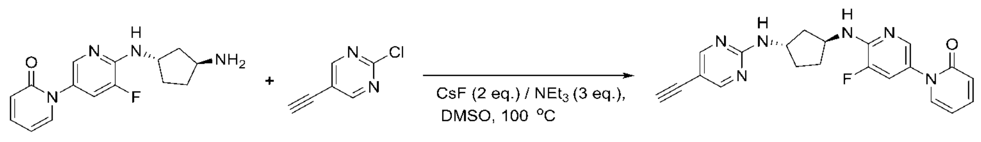


Step A: Synthesis of 6'-(((1S,3S)-3-((5-ethynylpyrimidin-2-yl)amino)cyclopentyl)amino)-5'-fluoro-2H-[1,3'-bipyridin ]-2-one

At room temperature under a nitrogen atmosphere, 6'-(((1S,3S)-3-aminocyclopentyl)amino)-5'-fluoro-2H-[1,3'-bipyridin]-2-one (0.19 g, 0.7 mmol, 1.0 eq), 2-chloro-5-ethynylpyrimidine (0.116 g, 0.84 mmol, 1.2 eq), triethylamine (0.212 g, 2.1 mmol, 3.0 eq), and cesium fluoride (0.213 g, 1.4 mmol, 2.0 eq) were dissolved in dimethyl sulfoxide (4 mL). The mixture was reacted at 100 C overnight.

After completion of the reaction, cold water (50 mL) was added to quench the reaction. The mixture was extracted with ethyl acetate (50 mL × 3). The organic phases were combined, dried over anhydrous sodium sulfate, filtered, and concentrated. The resulting residue was purified by silica gel column chromatography (eluent: ethyl acetate) to obtain 0.10

g of 6'-(((1S,3S)-3-((5-ethynylpyrimidin-2-yl)amino)cyclopentyl)amino)-5'-fluoro-2H-[1,3'-bipyridin ]-2-one (37% yield) as a yellow solid.

LC-MS: $[M+H]^+$ = 391. NMR data: H NMR (400 MHz, DMSO-$d_6$) δ 8.38 (s, 2H), 7.89 - 7.70 (m, 2H), 7.64 (dd, J = 6.8, 2.0 Hz, 1H), 7.59 - 7.45 (m, 2H), 6.93 (d, J = 7.0 Hz, 1H), 6.51 - 6.43 (m, 1H), 6.29 (td, J = 6.7, 1.4 Hz, 1H), 4.50 (p, J = 7.0 Hz, 1H), 4.39 (q, J = 6.9 Hz, 1H), 4.22 (s, 1H), 2.18 - 2.07 (m, 2H), 1.94 (td, J = 6.9, 2.9 Hz, 2H), 1.64 - 1.50 (m, 2H).

Example D7

Synthesis of 6'-(((1S,3S)-3-((5-ethynylpyrimidin-2-yl)amino)cyclopentyl)amino)-2H-[1,3'-bipyridin]-2-one

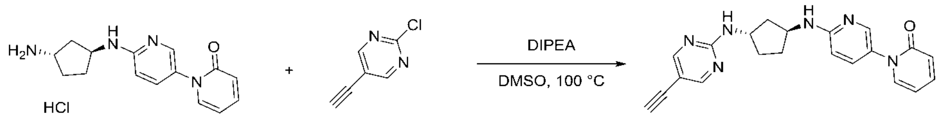


At room temperature, 6'-(((1S,3S)-3-aminocyclopentyl)amino)-2H-[1,3'-bipyridin]-2-one (0.34 g, 1 mmol), 2-chloro-5-ethynylpyrimidine (0.21 g, 1.48 mmol), DMSO (5 mL), and DIPEA (0.64 g, 4.95 mmol) were added to a 25 mL round-bottom flask sequentially. The reaction system was purged with a nitrogen atmosphere and heated to 100 °C. The reaction was stirred overnight. After completion of the reaction, the reaction system was cooled to room temperature. Water (5 mL) was added to quench the reaction. The mixture was extracted with EA (20 mL x 3). The organic phases were combined, dried over anhydrous sodium sulfate, filtered, and concentrated. The crude product was purified by C18 reverse phase column chromatography (water/acetonitrile = 3:1) to obtain 6'-(((1S,3S)-3-((5-ethynylpyrimidin-2-yl) amino)cyclopentyl)amino)-2H-[1,3'-bipyridin]-2-one (86 mg, yield: 23%, Purity: 99%) as a white solid.

LC-MS: $[M+H]^+$ = 373.1. $^1$H NMR (400 MHz, DMSO-$d_6$) δ 8.38 (s, 2H), 7.92 (d, J = 2.7 Hz, 1H), 7.77 (d, J = 7.3 Hz, 1H), 7.59 (dd, J = 6.8, 2.1 Hz, 1H), 7.47 (ddd, J = 9.0, 6.6, 2.1 Hz, 1H), 7.39 (dd, J = 8.9, 2.7 Hz, 1H), 6.92 (d, J = 6.9 Hz, 1H), 6.52 (d, J = 8.9 Hz, 1H), 6.44 (d, J = 9.1 Hz, 1H), 6.27 (td, J = 6.6, 1.4 Hz, 1H), 4.34 (dq, J = 25.5, 6.8 Hz, 2H), 4.22 (s, 1H), 2.22 - 2.04 (m, 2H), 2.00 - 1.81 (m, 2H), 1.52 (m, 2H).

Example D8

Synthesis of 2-(6-(((1S,3S)-3-((6-ethynyl-1,2,4-triazin-3-yl)amino)cyclopentyl)amino)pyridin-3-yl)pyridazin-3-(2H)-one

| | |
|---|---|
| 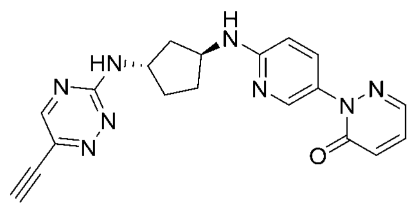 | $^1$H NMR (400 MHz, DMSO-$d_6$) δ 8.35 (s, 1H), 8.07 (d, J = 2.6 Hz, 1H), 8.01 (dd, J = 3.9, 1.6 Hz, 1H), 7.51 (dd, J = 8.9, 2.7 Hz, 1H), 7.45 (dd, J = 9.5, 3.9 Hz, 1H), 7.02 (dd, J = 9.5, 1.6 Hz, 1H), 6.54 (d, J = 8.9 Hz, 1H), 4.53 (s, 1H), 4.46 - 4.17 (m, 2H), 2.17 (d, J = 7.9 Hz, 2H), 2.04 - 1.83 (m, 2H), 1.67 - 1.43 (m, 2H). |

Example D9

Synthesis of 6'-(((1S,3S)-3-((5-(2,2-difluorocyclopropyl)pyrimidin-2-yl)amino)cyclopentyl)amino)-2H-[1,3'-bipyridin]-2-one

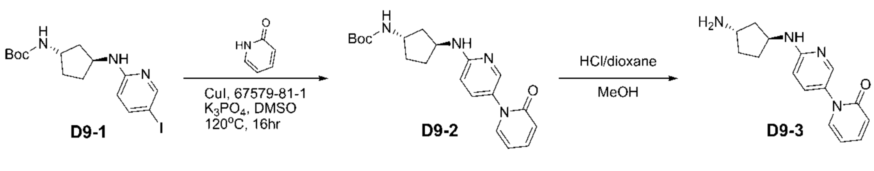

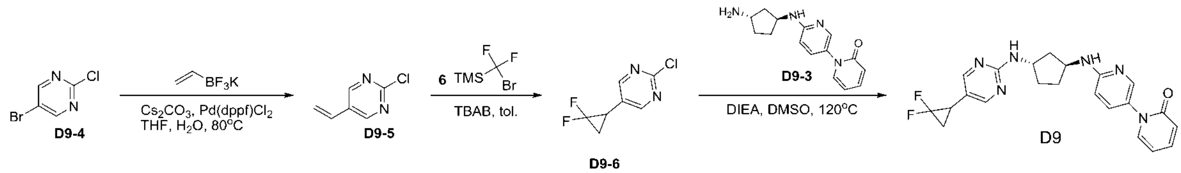


Step A: Synthesis of Compound D9-2

Compound D9-1 (1 g, 2.48 mmol) was dissolved in dimethyl sulfoxide (20 mL). Potassium phosphate (1.05 g, 5.0 mmol), pyridin-2(1H)-one (238.2 mg, 2.48 mmol), CuI (47.2 mg, 0.25 mmol), and trans-N,N'-dimethylcyclohexane-1,2-diamine (70.4 mg, 0.5 mmol) were added sequentially. Under a nitrogen atmosphere, the mixture was reacted at 120 C overnight. The reaction mixture was quenched with saturated ammonium chloride aqueous solution and extracted with dichloromethane (10 mL × 3). The organic phases were combined, washed with saturated sodium chloride aqueous solution three times. The organic phase was separated, dried, and concentrated. The resulting material was purified by Flash column chromatography (0 to 100% ethyl acetate/petroleum ether) to obtain 900 mg of Compound D9-2 (yield: 98%) as an off-white solid. LCMS: $[M+H]^+$ = 371.

Step B: Synthesis of Compound D9-3

Compound D9-2 (0.9 g, 2.43 mmol) was dissolved in methanol (5 mL). A 4 M HCl solution in 1,4-dioxane (5 mL) was added. The mixture was reacted at room temperature for 2 hours.

The reaction mixture was concentrated. The residue was dissolved in methanol. OH-type ion exchange resin was added to adjust the pH to 7. The mixture was filtered, and the filtrate was concentrated to obtain 430 mg of Compound D9-3 (yield: 65%) as a brownish solid. LCMS: $[M+H]^+$ = 271.

Step C: Synthesis of Compound D9-5

Compound D9-4 (5.0 g, 25.9 mmol) was dissolved in a mixed solvent of tetrahydrofuran (50 mL) and water (10 mL). Potassium vinyltrifluoroborate (3.47 g, 25.9 mmol), cesium carbonate (25.27 g, 77.6 mmol), and [1,1'-bis(diphenylphosphino)ferrocene]dichloropalladium(II) dichloromethane complex (0.95 g, 1.30 mmol) were added sequentially. Under a nitrogen atmosphere, the mixture was reacted at 80 °C for 16 hours. The reaction mixture was poured into water (70 mL) and extracted with ethyl acetate (50 mL × 3). The organic phases were combined, washed with saturated brine, dried, and concentrated. The resulting material was purified by Flash column chromatography (0 to 30% ethyl acetate/petroleum ether) to obtain 1.75 g of a mixture D9-5 (yield: 48%) as an off-white solid. LCMS: $[M+H]^+$ = 141.

Step D: Synthesis of Compound D9-6

Compound D9-5 (1.76 g, 12.6 mmol) was dissolved in toluene (50 mL). Compound D9-6 (7.68 g, 37.8 mmol) and tetrabutylammonium bromide (244 mg, 0.756 mmol) were added. Under a nitrogen atmosphere, the mixture was reacted at 110 °C for 16 hours. The reaction mixture was concentrated directly under reduced pressure. The resulting material was purified by Flash column chromatography (0 to 50% ethyl acetate/petroleum ether) to obtain 1.84 g of a mixture 7 (yield: 77%) as an off-white solid. LCMS: $[M+H]^+$ = 191.

Step E: Synthesis of Target Compound

Mixture D9-6 (320 mg, 1.68 mmol) was dissolved in dimethyl sulfoxide (8 mL). Diisopropylethylamine (300 mg, 2.28 mmol) and Compound D9-3 (200 mg, 0.74 mmol) were added. Under a nitrogen atmosphere, the mixture was reacted at 110 °C for 12 hours. The reaction mixture was quenched with saturated ammonium chloride aqueous solution and extracted with dichloromethane (10 mL × 3). The organic phases were combined, washed with saturated sodium chloride aqueous solution three times. The organic phase was separated, dried, and concentrated. The resulting material was purified by Flash column chromatography (0 to 100% ethyl acetate/petroleum ether), followed by reverse-phase C18 column chromatography to obtain 40 mg of the target compound (yield: 5.6%) as an off-white solid. LCMS: $[M+H]^+$ = 425.

H NMR (400 MHz, Methanol-d4): δ 8.21 (s, 2H), 8.02 - 7.94 (m, 1H), 7.70 - 7.60 (m, 2H), 7.48 (dd, J = 8.9, 2.7 Hz, 1H), 6.65 (dd, J = 9.0, 0.9 Hz, 2H), 6.50 (td, J = 6.8, 1.4 Hz, 1H), 4.41 (dp, J = 19.5, 6.7 Hz, 2H), 2.73 (td, J = 12.2, 7.9 Hz, 1H), 2.31 (dtd, J = 9.9, 5.1, 3.1 Hz, 2H), 2.00 (m, 2H), 1.91 (tdd, J = 12.0, 8.0, 5.4 Hz, 1H), 1.77 - 1.57 (m, 3H).

Example D10

Synthesis of 6'-(((1S,3S)-3-((5-ethoxypyrimidin-2-yl)amino)cyclopentyl)amino)-2H-[1,3'-bipyridin]-2-one

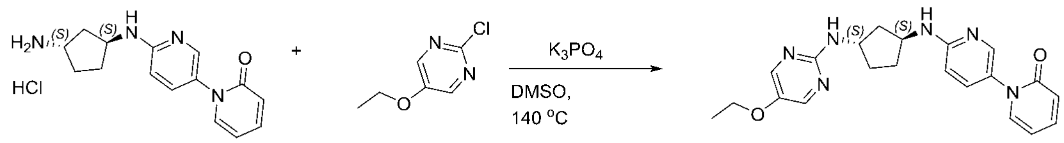


Step 1: Synthesis of 6'-(((1S,3S)-3-((5-ethoxypyrimidin-2-yl)amino)cyclopentyl)amino)-2H-[1,3'-bipyridin]-2-one

At 25 °C, 6'-(((1S,3S)-3-aminocyclopentyl)amino)-2H-[1,3'-bipyridin]-2-one hydrochloride (172 mg, 0.5 mmol) and 2-chloro-5-ethoxypyrimidine (120 mg, 0.75 mmol) were dissolved in DMSO (5 mL). Potassium phosphate (634 mg, 3 mmol) was added. After addition, the mixture was heated to 140 °C and reacted overnight.

Water (10 mL) was added. Then the mixture was extracted with EA (10 mL * 3). The organic phases were combined and concentrated to obtain the crude product. The crude product was purified by column chromatography (EA:MeOH = 13:1) to obtain 41 mg of 6'-(((1S,3S)-3-((5-ethoxypyrimidin-2-yl)amino)cyclopentyl)amino)-2H-[1,3'-bipyridin]-2-one (41 mg, 21% yield) as a light yellow solid. LC-MS: $[M+H]^+$ = 393.2.

H NMR (400 MHz, Chloroform-d) δ 8.08 - 8.00 (m, 3H), 7.51 (dd, J = 8.9, 2.7 Hz, 1H), 7.42 - 7.35 (m, 1H), 7.29 (dd, J = 6.8, 2.1 Hz, 1H), 6.64 (d, J = 9.2 Hz, 1H), 6.43 (d, J = 8.9 Hz, 1H), 6.23 (td, J = 6.7, 1.4 Hz, 1H), 4.95 (d, J = 7.0 Hz, 1H), 4.86 (d, J = 6.8 Hz, 1H), 4.41 - 4.32 (m, 1H), 4.29 - 4.19 (m, 1H), 3.99 (q, J = 7.0 Hz, 2H), 2.31 (d, J = 5.1 Hz, 2H), 2.02 (d, J = 6.8 Hz, 2H), 1.63 - 1.50 (m, 2H), 1.38 (t, J = 7.0 Hz, 3H).

Example D11

Synthesis of 6'-(((1S,3S)-3-((5-(dimethylphosphoryl)pyrimidin-2-yl)amino)cyclopentyl)amino)-2H-[1,3'-bipy ridin]-2-one

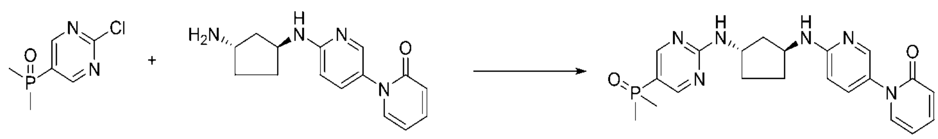

Step 1: Synthesis of 6'-(((1S,3S)-3-((5-(dimethylphosphoryl)pyrimidin-2-yl)amino)cyclopentyl)amino)-2H-[1,3'-bipy ridin]-2-one

At 20 °C, 6'-(((1S,3S)-3-aminocyclopentyl)amino)-2H-[1,3'-bipyridin]-2-one (280 mg, 0.91 mmol), (2-chloropyrimidin-5-yl)dimethylphosphine oxide (0.23 g, 1.18 mmol), triethylamine (0.2 g, 9.1 mmol), and DMF (10 mL) were added to a 50 mL single-neck flask. The mixture was heated to 100 °C and reacted overnight.

The solvent was removed by concentration. The crude product was purified by column chromatography (DCM/MeOH = 10/1). The resulting material was further purified by reverse-phase column chromatography (DCM:MeOH = 100:3) to obtain 6'-(((1S,3S)-3-((5-(dimethylphosphoryl)pyrimidin-2-yl)amino)cyclopentyl)amino)-2H-[1,3'-bipy ridin]-2-one (253 mg, 65.3% yield) as an off-white solid. LC-MS: $[M+H]^+$ = 425.

H NMR (400 MHz, DMSO-$d_6$) δ 8.07 (s, 1H), 7.92 (d, J = 2.6 Hz, 1H), 7.61 (s, 1H), 7.48 (ddd, J = 9.0, 6.6, 2.1 Hz, 1H), 7.38 (d, J = 2.7 Hz, 1H), 6.94 (dd, J = 10.7, 7.1 Hz, 2H), 6.52 (d, J = 8.9 Hz, 1H), 6.44 (d, J = 9.1 Hz, 1H), 6.27 (d, J = 1.4 Hz, 1H), 4.33 (dp, J = 20.4, 6.7 Hz, 2H), 2.70 (td, J = 7.5, 4.2 Hz, 4H), 2.19 - 2.05 (m, 2H), 2.03 - 1.79 (m, 4H), 1.49 (td, J = 12.4, 7.0 Hz, 2H).

Example D12

Synthesis of 6'-(((1S,3S)-3-((5-(methylsulfinyl)pyrimidin-2-yl)amino)cyclopentyl)amino)-2H-[1,3'-bipyridin] -2-one

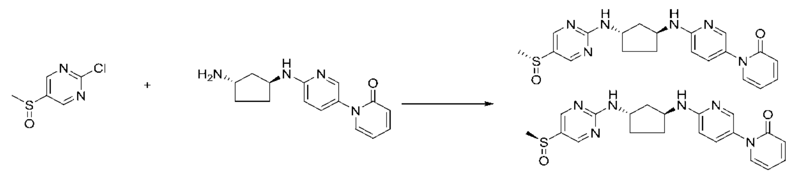

Step 1 : Synthesis of 6'-(((1S,3S)-3-((5-(methylsulfinyl)pyrimidin-2-yl)amino)cyclopentyl)amino)-2H-[1,3'-bipyridin] -2-one

At 20 °C, 6'-(((1S,3S)-3-aminocyclopentyl)amino)-2H-[1,3'-bipyridin]-2-one (414.5 mg, 1.351 mmol), 2-chloro-5-(methylsulfinyl)pyrimidine (0.48 g, 2.7 mmol), triethylamine (1.37 g, 13.5 mmol), and DMF (20 mL) were added to a 50 mL single-neck flask. The mixture was heated to 100 °C and reacted overnight.

The mixture was concentrated. Then it was purified by column chromatography (DCM:MeOH = 20:1), followed by reverse-phase column chromatography to obtain 430 mg of a mixture. The mixture was separated to obtain Peak 1 (3 mg, 41.6% yield). H NMR (400 MHz, DMSO-$d_6$) δ 8.57 (s, 2H), 8.04 (d, J = 7.3 Hz, 1H), 7.92 (d, J = 2.7 Hz, 1H), 7.60 (dd, J = 6.8, 2.1 Hz, 1H), 7.48 (ddd, J = 9.0, 6.6, 2.1 Hz, 1H), 7.40 (dd, J = 8.9, 2.7 Hz, 1H), 6.95 (d, J = 6.8 Hz, 1H), 6.53 (d, J = 8.9 Hz, 1H), 6.45 (d, J = 9.2 Hz, 1H), 6.28 (td, J = 6.7, 1.3 Hz, 1H), 4.43 (q, J = 7.0 Hz, 1H), 4.33 (q, J = 6.5 Hz, 1H), 2.83 (s, 3H), 2.14 (dp, J = 12.9, 6.5, 5.9 Hz, 2H), 2.00 - 1.87 (m, 2H), 1.54 (ddt, J = 18.6, 13.5, 7.1 Hz, 2H). LC-MS: $[M+H]^+$ = 411 (front peak).

Peak 2 (51 mg, 41.6% yield) was afforded. H NMR (400 MHz, DMSO-$d_6$) δ 8.57 (s, 2H), 8.04 (d, J = 7.3 Hz, 1H), 7.93 (d, J = 2.7 Hz, 1H), 7.61 (s, 1H), 7.48 (s, 1H), 7.44 - 7.37 (m, 1H), 6.95 (d, J = 6.9 Hz, 1H), 6.53 (d, J = 8.9 Hz, 1H), 6.48 - 6.42 (m, 1H), 6.28 (d, J = 1.4 Hz, 1H), 4.43 (q, J = 7.0 Hz, 1H), 4.33 (q, J = 6.5 Hz, 1H), 2.83 (s, 3H), 2.21 - 2.09 (m, 2H), 2.01 - 1.87 (m, 2H), 1.54 (ddt, J = 18.4, 13.1, 6.7 Hz, 2H). LC-MS: $[M+H]^+$ = 411 (back peak).

Example D13

Synthesis of 6'-(((1S,3S)-3-((5-(dimethylamino)pyrimidin-2-yl)amino)cyclopentyl)amino)-2H-[1,3'-bipyridin] -2-one

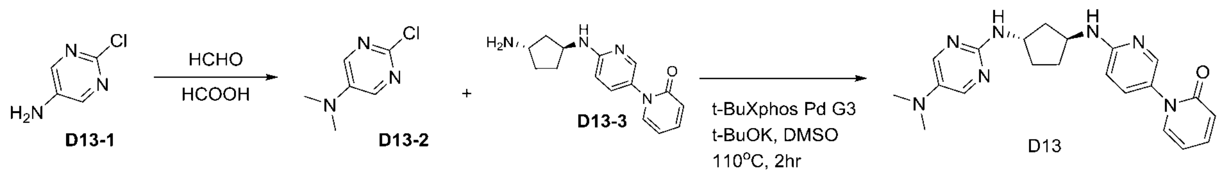


Step A: Synthesis of Compound D13-2

Compound D13-1 (2.0 g, 15.5 mmol) was dissolved in formic acid (3.24 mL). A 37% formaldehyde aqueous solution (3.47 mL, 279 mmol) was added. The mixture was reacted at 105 °C for 4 hours. The reaction mixture was concentrated under reduced pressure. The residue was neutralized with saturated sodium bicarbonate aqueous solution and filtered. The resulting solid was purified by Flash column chromatography (0 to 50% ethyl acetate/petroleum ether) to obtain 760 mg of Compound D13-2 (yield: 32.5%) as a white solid.

LCMS: $[M+H]^+$ = 158.

Step B: Synthesis of Target Compound

Compound D13-2 (70 mg, 0.44 mmol) and Compound D13-3 (100 mg, 0.37 mmol) were dissolved in dimethyl sulfoxide (2 mL). Potassium tert-butoxide (157 mg, 0.74 mmol) and (2-dicyclohexylphosphino-2',4',6'-triisopropyl-1,1'-biphenyl)[2-(2-aminoethyl)phenyl]palladium( II) methanesulfonate (59 mg, 0.074 mmol) were added. Under a nitrogen atmosphere, the mixture was reacted at 110 C for 2 hours. The reaction mixture was poured into saturated ammonium chloride aqueous solution (20 mL) and extracted with ethyl acetate (15 mL × 3). The organic phases were combined and dried by rotary evaporation. The resulting material was purified by Flash column chromatography (0% to 10% methanol/dichloromethane) to obtain 16.3 mg of the target compound (yield: 11.3%) as a white solid.

LCMS: $[M+H]^+$ = 392. H NMR (400 MHz, DMSO-$d_6$) δ 8.01 (s, 2H), 7.91 (d, J = 2.7 Hz, 1H), 7.60 (dd, J = 6.7, 2.1 Hz, 1H), 7.48 (ddd, J = 9.1, 6.6, 2.1 Hz, 1H), 7.39 (dd, J = 8.9, 2.7 Hz, 1H), 6.91 (d, J = 6.9 Hz, 1H), 6.54 (dd, J = 17.5, 8.1 Hz, 2H), 6.44 (d, J = 9.2 Hz, 1H), 6.27 (t, J = 6.8 Hz, 1H), 4.27 (dq, J = 13.7, 6.6 Hz, 2H), 2.72 (s, 6H), 2.10 (dq, J = 12.5, 6.1 Hz,

2H), 1.86 (q, J = 6.3 Hz, 2H), 1.48 (td, J = 13.0, 12.3, 7.2 Hz, 2H).

Example D14

Synthesis of 3-chloro-6'-(((1S,3S)-3-((5-ethoxypyrimidin-2-yl)amino)cyclopentyl)amino)-2H-[1,3'-bipyridin] -2-one

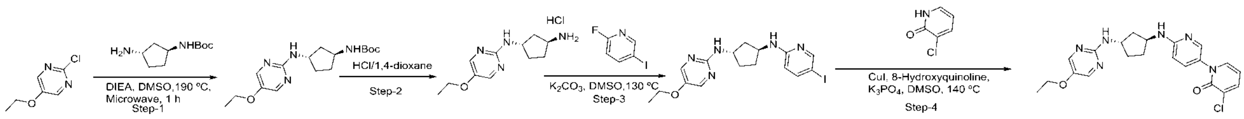


Step 1: Synthesis of tert-butyl ((1S,3S)-3-((5-ethoxypyrimidin-2-yl)amino)cyclopentyl)carbamate

At room temperature, 2-chloro-5-ethoxypyrimidine (1 g, 6.31 mmol), tert-butyl ((1S,3S)-3-aminocyclopentyl) carbamate (1.39 g, 6.94 mmol), and DIEA (2.45 g, 18.93 mmol) were added to DMSO (50 mL). The mixture was heated to 190 °C and reacted for 60 minutes using a microwave synthesizer.

After completion of the reaction, the solution was purified by C18 column chromatography (eluent: acetonitrile/-water = 55/45, V/V) to obtain tert-butyl ((1S,3S)-3-((5-ethoxypyrimidin-2-yl)amino)cyclopentyl)carbamate (680 mg, yield: 33.45%) as a light yellow solid. LC-MS: $[M+H]^+$ = 323.

Step 2: Synthesis of (1S,3S)-$N^1$-(5-ethoxypyrimidin-2-yl)cyclopentane-1,3-diamine hydrochloride

At room temperature, tert-butyl ((1S,3S)-3-((5-ethoxypyrimidin-2-yl)amino)cyclopentyl)carbamate (0.68 g, 2.11 mmol) was dissolved in methanol (10 mL), and then 4 M HCl/1,4-dioxane (5 mL) was added. The mixture was stirred at room temperature for 1 hour. After completion of the reaction, the mixture was directly concentrated by rotary evaporation to a residue. The residue was used in the next step.

Step 3: Synthesis of (1S,3S)-$N^1$-(5-ethoxypyrimidin-2-yl)-$N^3$-(5-iodopyridin-2-yl)cyclopentane-1,3-diamine

At room temperature, (1S,3S)-$N^1$-(5-ethoxypyrimidin-2-yl)cyclopentane-1,3-diamine hydrochloride (0.55 g, 2.13 mmol), 2-fluoro-5-iodopyridine (0.71 g, 3.19 mmol), and potassium carbonate (1.47 g, 10.65 mmol) were added to DMSO (10 mL). The mixture was irradiated with microwaves and heated to 130 °C for 2 hours.

After completion of the reaction, water (50 mL) was added. The mixture was extracted with ethyl acetate (3 × 30 mL). The organic phases were combined, washed with brine (3 × 20 mL), dried over anhydrous $Na_2SO_4$, filtered, and dried by rotary evaporation to a residue. The resulting residue was purified by silica gel column chromatography (eluent: n-hexane/ethyl acetate = 55/45, V/V) to obtain (1S,3S)-$N^1$-(5-ethoxypyrimidin-2-yl)-$N^3$-(5-iodopyridin-2-yl)cyclopentane-1,3-diamine (0.495 g, yield: 54.76%) as a light yellow solid. LC-MS: $[M+H]^+$ = 426.

Step 4: Synthesis of 3-chloro-6'-(((1S,3S)-3-((5-ethoxypyrimidin-2-yl)amino)cyclopentyl)amino)-2H-[1,3'-bipyridin] -2-one

At room temperature, (1S,3S)-$N^1$-(5-ethoxypyrimidin-2-yl)-$N^3$-(5-iodopyridin-2-yl)cyclopentane-1,3-diamine (200 mg, 0.47 mmol), 3-chloropyridin-2(1H)-one (182.65 mg, 1.41 mmol), CuI (17.90 mg, 0.094 mmol), 8-hydroxyquinoline (27.29 mg, 0.19 mmol), and potassium phosphate (299.30 mg, 1.41 mmol) were added to DMSO (2 mL). The atmosphere was purged with nitrogen three times. The mixture was placed in an oil bath, heated to 135 °C and reacted overnight.

After completion of the reaction, water (30 mL) was added. The mixture was extracted with ethyl acetate (3 × 20 mL). The organic phases were combined, washed with brine (2 × 20 mL), dried over anhydrous $Na_2SO_4$, filtered, and dried by rotary evaporation to a residue. The resulting residue was purified by C18 reverse-phase column chromatography (ACN/$H_2O$, 45% ACN) to obtain 3-chloro-6'-(((1S,3S)-3-((5-ethoxypyrimidin-2-yl)amino)cyclopentyl)amino)-2H-[1,3'-bipyridin] -2-one (112 mg, yield: 54.67%) as a yellow solid. LC-MS: $[M+H]^+$ = 427.

H NMR (400 MHz, DMSO) δ 8.09 (s, 2H), 7.96 (d, J = 2.6 Hz, 1H), 7.82 (dd, J = 7.3, 1.8 Hz, 1H), 7.66 (dd, J = 6.8, 1.8 Hz, 1H), 7.43 (dd, J = 8.9, 2.6 Hz, 1H), 6.98 (d, J = 6.8 Hz, 1H), 6.85 (d, J = 6.3 Hz, 1H), 6.53 (d, J = 8.9 Hz, 1H), 6.31 (t, J = 7.0 Hz, 1H), 4.29 (dq, J = 13.8, 6.8, 6.3 Hz, 2H), 3.99 (q, J = 6.9 Hz, 2H), 2.21 - 2.03 (m, 2H), 1.96 - 1.80 (m, J = 6.8 Hz, 2H), 1.50 (tt, J = 14.8, 7.6 Hz, 2H), 1.28 (t, J = 6.9 Hz, 3H).

Example D15

Synthesis of 6'-(((1S,3S)-3-((5-(azetidin-1-yl)pyrimidin-2-yl)amino)cyclopentyl)amino)-2H-[1,3'-bipyridin]-2 -one

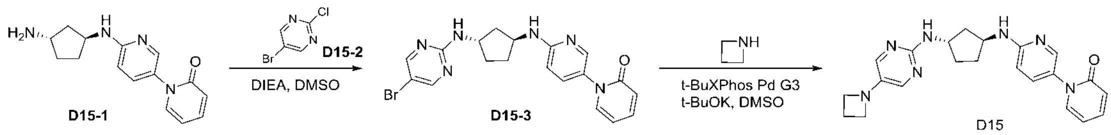


D15-1 D15-3 D15

Step A: Synthesis of Compound D15-2

Compound D15-1 (500 mg, 1.85 mmol) was dissolved in dimethyl sulfoxide (5 mL). Compound D15-2 (533 mg, 2.78 mmol) and N,N-diisopropylethylamine (716 mg, 5.56 mmol) were added. Under a nitrogen atmosphere, the mixture was heated to 120 C and reacted for 16 hours. The reaction mixture was poured into saturated ammonium chloride aqueous solution and extracted with ethyl acetate (10 mL × 3). The organic phases were combined, dried and concentrated. The resulting material was purified by Flash silica gel column chromatography (0% to 10% methanol/dichloromethane) to obtain 740 mg of Compound D15-3 (yield: 94%) as a brown oil. LCMS: $[M+H]^+$ = 427/429.

Step B: Synthesis of Target Compound

Compound D15-3 (150 mg, 0.35 mmol) was dissolved in anhydrous dimethyl sulfoxide (2 mL). Azetidine (24 mg, 0.42 mmol), potassium tert-butoxide (78 mg, 0.7 mmol), and (2-dicyclohexylphosphino-2',4',6'-triisopropyl-1,1'-biphenyl) [2-(2-aminoethyl)phenyl]palladium( II) methanesulfonate (14 mg, 0.0175 mmol) were added. Under a nitrogen atmosphere, the mixture was heated to 110 °C and reacted for 8 hours. The reaction mixture was poured into saturated ammonium chloride aqueous solution and extracted with ethyl acetate (10 mL × 3). The organic phases were combined, dried and concentrated. The resulting material was purified by Flash silica gel column chromatography (0% to 10% methanol/dichloromethane) to obtain 13 mg of the target compound (yield: 9%) as a light yellow solid. LCMS: $[M+H]^+$ = 404.

H NMR (400 MHz, Chloroform-d) δ 7.90 (d, J = 2.7 Hz, 1H), 7.68 (s, 2H), 7.59 (dd, J = 6.8, 2.1 Hz, 1H), 7.47 (ddd, J = 8.9, 6.6, 2.1 Hz, 1H), 7.38 (dd, J = 8.9, 2.7 Hz, 1H), 6.90 (d, J = 6.9 Hz, 1H), 6.52 (dd, J = 11.2, 8.1 Hz, 2H), 6.43 (dd, J = 9.3, 1.2 Hz, 1H), 6.26 (td, J = 6.7, 1.4 Hz, 1H), 4.25 (dq, J = 16.7, 6.7 Hz, 2H), 3.70 (t, J = 7.1 Hz, 4H), 2.26 (p, J = 7.1 Hz, 2H), 2.09 (ddt, J = 19.8, 7.7, 5.2 Hz, 2H), 1.85 (hept, J = 7.0 Hz, 2H), 1.46 (td, J = 12.1, 7.0 Hz, 2H).

Example D16

Synthesis of 6'-(((1S,3S)-3-((5-(2-oxoazetidin-1-yl)pyrimidin-2-yl)amino)cyclopentyl)amino)-2H-[1,3'-bipyri din]-2-one

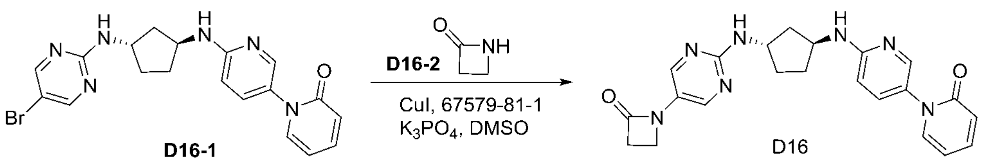


D16-1 D16

Step A: Synthesis of Target Compound

Compound D16-1 (300 mg, 0.70 mmol) and Compound D16-2 (60 mg, 0.85 mmol) were dissolved in dimethyl sulfoxide (5 mL). Potassium phosphate (297 mg, 1.4 mmol), CuI (13 mg, 0.07 mmol), and trans-N,N'-dimethylcyclohexane-1,2-diamine (20 mg, 0.14 mmol) were added. Under a nitrogen atmosphere, the mixture was heated to 110 C and reacted for 16 hours. The reaction mixture was poured into saturated ammonium chloride aqueous solution and extracted with ethyl acetate (15 mL × 3). The organic phases were combined, dried and concentrated.

The resulting material was purified by Flash silica gel column chromatography (0 to 10% methanol/dichloromethane) to obtain 10.3 mg of the target compound (yield: 3.4%) as a white solid.

LCMS: $[M+H]^+$ = 418. H NMR (400 MHz, DMSO-$d_6$) δ 8.43 (s, 2H), 7.98 (d, J = 2.7 Hz, 1H), 7.66 (dd, J = 6.8, 2.1

Hz, 1H), 7.54 (ddd, J = 9.0, 6.6, 2.1 Hz, 1H), 7.45 (dd, J = 8.9, 2.7 Hz, 1H), 7.33 (d, J = 7.2 Hz, 1H), 6.99 (d, J = 6.9 Hz, 1H), 6.58 (d, J = 8.9 Hz, 1H), 6.50 (d, J = 9.0 Hz, 1H), 6.33 (td, J = 6.7, 1.4 Hz, 1H), 4.43 - 4.32 (m, 2H), 3.65 (t, J = 4.4 Hz, 2H), 3.13 (t, J = 4.3 Hz, 2H), 2.28 - 2.13 (m, 2H), 2.01 - 1.87 (m, 2H), 1.57 (ddt, J = 14.9, 12.1, 8.3 Hz, 2H).

Example D17

Synthesis of 6'-(((1S,35)-3-((5-((1-methylazetidin-3-yl)oxy)pyrimidin-2-yl)amino)cyclopentyl)amino)-2H-[1, 3'-bipyridin]-2-one

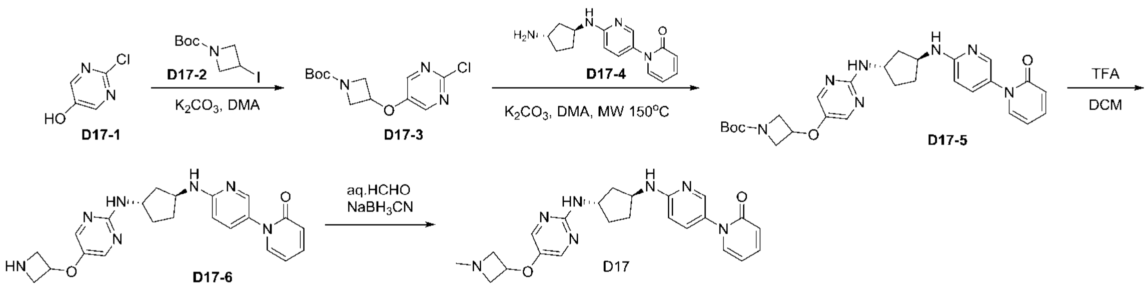


Step A: Synthesis of Compound D17-3

Compound D17-2 (2.1 g, 7.4 mmol) was dissolved in N,N-dimethylacetamide (20 mL). Compound D17-1 (1.4 g, 11.1 mmol) and potassium carbonate (2.1 g, 14.8 mmol) were added. Under a nitrogen atmosphere, the mixture was heated to 60 °C and reacted overnight. The reaction mixture was cooled to room temperature and poured into saturated ammonium chloride aqueous solution to quench. The mixture was extracted with ethyl acetate (40 mL × 3). The organic phases were dried, filtered, and concentrated. The resulting material was purified by Flash column chromatography (0 to 80% ethyl acetate/petroleum ether) to obtain 1.6 g of Compound D17-3 (yield: 75.6%) as a white solid. LCMS: $[M+H-56]^+$ = 230.

Step B: Synthesis of Compound D17-5

Compound D17-4 (135 mg, 0.5 mmol) was dissolved in N,N-dimethylacetamide (3 mL). Compound D17-3 (171 mg, 0.6 mmol) and potassium carbonate (138 mg, 1 mmol) were added. The atmosphere was purged with nitrogen. The mixture was irradiated with microwaves at 150 °C for 40 minutes. Two additional batches of Compound D17-4 (210 mg) were processed similarly. The reaction mixtures were poured into saturated ammonium chloride aqueous solution to quench and extracted with ethyl acetate (20 mL × 3). The organic phases were dried, filtered, and concentrated. The combined crude product was purified by Flash column chromatography (0 to 10% methanol/dichloromethane) to obtain 140 mg of Compound D17-5 (yield: 13.1%) as a light yellow solid. LCMS: $[M+H]^+$ = 520.

Step C: Synthesis of Compound D17-6

Compound D17-5 (140 mg, 0.27 mmol) was dissolved in dichloromethane (2 mL). Trifluoroacetic acid (0.4 mL) was added. The reaction was stirred at room temperature for 2 hours. The reaction mixture was concentrated without heating. The residue was dissolved in methanol, neutralized with OH-type ion exchange resin, filtered, and concentrated to obtain 90 mg of Compound D17-6 (yield: 79.4%) as a light yellow oil. LCMS: $[M+H]^+$ = 420.

Step D: Synthesis of Target Compound

Compound D17-6 (90 mg, 0.21 mmol) was dissolved in methanol (2 mL). A 37% formaldehyde aqueous solution (21 mg, 0.26 mmol) and one drop of acetic acid were added. The reaction was stirred at room temperature for 1 hour. Sodium cyanoborohydride (20 mg, 0.32 mmol) was added. The mixture was reacted at room temperature for 2 hours. The reaction mixture was concentrated. The resulting material was purified by preparative silica gel TLC (dichloromethane/-methanol = 10:1) to obtain 31.7 mg of the target compound (yield: 34.8%) as a yellow solid. LCMS: $[M+H]^+$ = 434.

H NMR (400 MHz, DMSO-$d_6$) δ 8.11 (s, 2H), 7.97 (t, J = 4.2 Hz, 1H), 7.66 (dd, J = 6.8, 2.1 Hz, 1H), 7.54 (ddd, J = 9.0, 6.5, 2.1 Hz, 1H), 7.45 (dd, J = 8.8, 2.8 Hz, 1H), 7.14 - 6.81 (m, 2H), 6.58 (d, J = 8.9 Hz, 1H), 6.50 (d, J = 9.2 Hz, 1H), 6.34

(t, J = 6.7 Hz, 1H), 4.86 (p, J = 5.7 Hz, 1H), 4.33 (tt, J = 14.2, 7.0 Hz, 2H), 4.17 (dd, J = 9.9, 6.3 Hz, 1H), 3.65 (dd, J = 10.3, 4.9 Hz, 1H), 2.66 (s, 3H), 2.49 (s, 1H), 2.18 (qd, J = 13.7, 10.7, 5.2 Hz, 3H), 1.92 (q, J = 6.6 Hz, 2H), 1.55 (td, J = 13.3, 12.3, 6.8 Hz, 2H).

Example D18

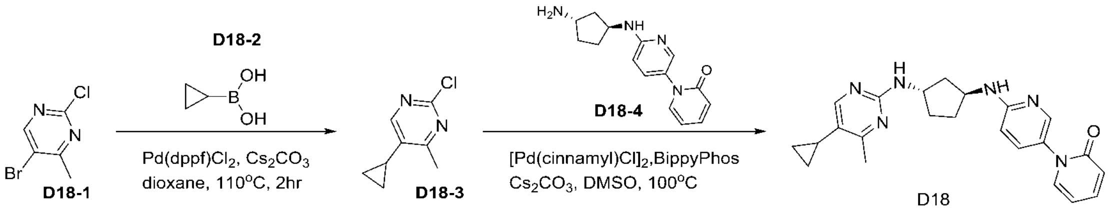


D18-1 D18-3 D18

Step A: Synthesis of Compound D18-3

Compound D18-1 (3 g, 14.5 mmol) and Compound D18-2 (1.5 g, 17.4 mmol) were added to 30 mL of anhydrous 1,4-dioxane. Cesium carbonate (7.1 g, 21.8 mmol) was added. Under a nitrogen atmosphere, [1,1'-bis(diphenylphosphino)ferrocene]dichloropalladium(II) (0.3 g, 0.4 mmol) was added. The mixture was heated to 110 °C and reacted for 16 hours. The reaction mixture was poured into saturated ammonium chloride aqueous solution (100 mL) and extracted with dichloromethane (40 mL × 3). The organic phases were combined, dried and concentrated. The resulting material was purified by Flash silica gel column chromatography (7 to 9% ethyl acetate/petroleum ether) to obtain 1.2 g of Compound D18-3 (49.1% yield) as a light yellow solid. LCMS: $[M+H]^+$ = 169.

Step B: Synthesis of Target Compound

Compound D18-3 (300 mg, 1.8 mmol) and Compound D18-4 (481.0 mg, 1.8 mmol) were added to 5 mL of anhydrous dimethyl sulfoxide. Cesium carbonate (1.2 g, 3.6 mmol) was added. Under a nitrogen atmosphere, chloro(π-cinnamyl)palladium(II) dimer (91 mg, 0.18 mmol) and 5-di-tert-butylphosphino-1',3',5'-triphenyl-1'H-[1,4']bipyrazole (180 mg, 0.36 mmol) were added. The mixture was heated to 100 °C and reacted for 16 hours. The reaction mixture was poured into saturated ammonium chloride aqueous solution (20 mL) and extracted with ethyl acetate (10 mL × 3). The organic phases were combined, dried and concentrated. The resulting material was purified by Flash silica gel column chromatography (4 to 5% methanol/dichloromethane), followed by reverse-phase column chromatography (13 to 17% acetonitrile/water) to obtain 26 mg of the target compound (3.6% yield) as a white solid.

LCMS: $[M+H]^+$ = 403. H NMR (400 MHz, DMSO-$d_6$) δ 7.94 (d, J = 2.7 Hz, 1H), 7.90 (s, 1H), 7.65 - 7.60 (m, 1H), 7.50 (ddd, J = 9.0, 6.6, 2.1 Hz, 1H), 7.42 (dd, J = 8.9, 2.7 Hz, 1H), 6.93 (t, J = 6.8 Hz, 2H), 6.55 (d, J = 8.9 Hz, 1H), 6.47 (dd, J = 9.3, 1.1 Hz, 1H), 6.30 (td, J = 6.7, 1.4 Hz, 1H), 4.34 (dt, J = 15.2, 7.2 Hz, 2H), 2.38 (s, 3H), 2.21 - 2.07 (m, 2H), 1.88 (qt, J = 13.1, 7.0 Hz, 2H), 1.70 (ddd, J = 13.6, 8.3, 5.3 Hz, 1H), 1.51 (td, J = 12.4, 7.0 Hz, 2H), 0.86 - 0.80 (m, 2H), 0.57 - 0.50 (m, 2H).

Example D19

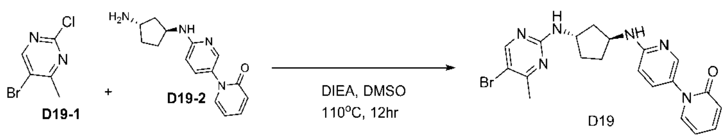


D19-1 + D19-2 D19

Compound D19-1 (200 mg, 1.0 mmol) and Compound D19-2 (270 mg, 1.0 mmol) were added to 5 mL of anhydrous dimethyl sulfoxide. N,N-diisopropylethylamine (0.5 mL, 3.1 mmol) was added. Under a nitrogen atmosphere, the mixture was heated to 110 C and reacted for 12 hours. The reaction mixture was poured into saturated ammonium chloride aqueous solution (20 mL) and extracted with dichloromethane (10 mL × 3). The organic phases were combined, dried and concentrated. The resulting material was purified by Flash silica gel column chromatography (2 to 3% methanol/dichloromethane), followed by reverse-phase column chromatography (23 to 26% acetonitrile/water) to obtain 280 mg of the target compound (63.4% yield) as a white solid.

LCMS: $[M+H]^+$ = 441. H NMR (400 MHz, DMSO-$d_6$) δ 8.26 (s, 1H), 7.92 (d, J = 2.7 Hz, 1H), 7.60 (dd, J = 6.8, 2.0

Hz, 1H), 7.48 (ddd, J = 9.0, 6.4, 2.2 Hz, 2H), 7.40 (dd, J = 9.0, 2.6 Hz, 1H), 6.95 (s, 1H), 6.52 (d, J = 8.9 Hz, 1H), 6.44 (d, J = 9.2 Hz, 1H), 6.27 (dd, J = 7.4, 6.1 Hz, 1H), 4.37 - 4.24 (m, 2H), 2.34 (s, 3H), 2.16 - 2.06 (m, 2H), 1.94 - 1.79 (m, 2H), 1.50 (ddd, J = 15.5, 12.6, 7.1 Hz, 2H).

Example D20

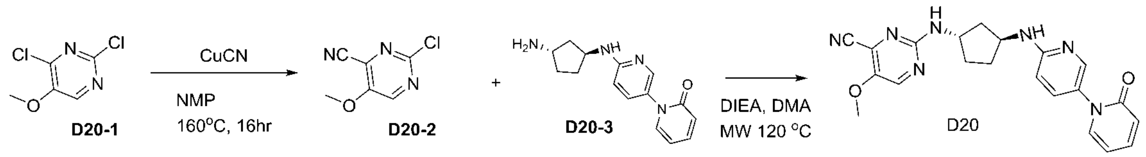


Step A: Synthesis of Compound D20-2

Compound D20-1 (2 g, 11.2 mmol) was dissolved in 20 mL of N-methylpyrrolidinone. Cuprous cyanide (2 g, 22.3 mmol) was added. The mixture was heated to 160 C and reacted for 16 hours. The reaction mixture was poured into saturated ammonium chloride solution and extracted with ethyl acetate (50 mL) three times. The organic phases were combined and dried by rotary evaporation. The resulting material was purified by Flash column chromatography (0 to 30% ethyl acetate/petroleum ether) to obtain 640 mg of Compound D20-2 (yield: 34%) as a white solid. LCMS: $[M+H]^+$ = 170.

Step B: Synthesis of Target Compound

Compound D20-2 (200 mg, 1.18 mmol) was added to 2 mL of N,N-dimethylacetamide. Compound D20-3 (320 mg, 1.18 mmol) and N,N-diisopropylethylamine (0.4 mL, 2.36 mmol) were added. The mixture was irradiated with microwaves at 120 C for 1 hour. The reaction mixture was poured into saturated ammonium chloride solution (20 mL) and extracted with ethyl acetate (10 mL $\times$ 3). The organic phases were combined and dried by rotary evaporation. The resulting material was purified by Flash column chromatography (0 to 80% ethyl acetate/petroleum ether) to obtain 13.3 mg of the target compound (yield: 2.7%) as a light yellow solid. LCMS: $[M+H]^+$ = 404.

H NMR (400 MHz, DMSO-$d_6$) $\delta$ 8.61 (s, 1H), 7.91 (d, J = 2.8 Hz, 1H), 7.60 (dd, J = 6.8, 2.0 Hz, 1H), 7.54 (d, J = 7.2 Hz, 1H), 7.47 (ddd, J = 9.2, 6.4, 2.0 Hz, 1H), 7.39 (dd, J = 8.8, 2.8 Hz, 1H), 6.94 (d, J = 6.8 Hz, 1H), 6.52 (d, J = 8.8 Hz, 1H), 6.44 (d, J = 9.2 Hz, 1H), 6.27 (td, J = 6.8, 1.6 Hz, 1H), 4.30 (q, J = 6.4 Hz, 1H), 4.23 (q, J = 6.8 Hz, 1H), 3.90 (s, 3H), 3.33 (s, 1H), 2.18 - 2.07 (m, 2H), 1.96 - 1.87 (m, 1H), 1.87 - 1.81 (m, 1H), 1.50 (dq, J = 11.6, 6.8 Hz, 2H).

Example D21

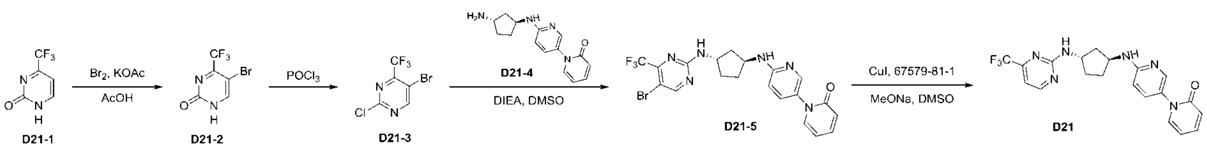


Step A: Synthesis of Compound D21-2

Compound D21-1 (5 g, 30.5 mmol) was dissolved in acetic acid (30 mL). Potassium acetate (9 g, 91.5 mmol) and liquid bromine (1.56 mL, 30.5 mmol) were added. The mixture was reacted at 80 °C for 2 hours. The reaction mixture was concentrated. Water (50 mL) was added. The mixture was extracted with ethyl acetate (50 mL $\times$ 3). The organic phases were combined and dried by rotary evaporation. The resulting material was purified by Flash column chromatography (0 to 50% ethyl acetate/petroleum ether) to obtain 2.5 g of Compound D21-2 (yield: 34%) as a pale yellow solid. LCMS: $[M+H]^+$ = 243/245.

Step B: Synthesis of Compound D21-3

Compound D21-2 (1.8 g, 7.44 mmol) was dissolved in phosphorus oxychloride (10 mL). The mixture was heated to 110 °C and reacted for 45 minutes. The reaction mixture was dried and concentrated. The resulting material was purified

by Flash column chromatography (0% to 20% ethyl acetate/petroleum ether) to obtain 1.2 g of Compound D21-3 as a yellow oil (yield: 62%).

Step C: Synthesis of Compound D21-5

Compound D21-3 (260 mg, 1.0 mmol) and Compound D21-4 (270 mg, 1.0 mmol) were added to dimethyl sulfoxide (5 mL). N,N-diisopropylethylamine (0.35 mL, 2.0 mmol) was added. The atmosphere was purged with nitrogen. The mixture was heated to 80 °C and reacted for 2 hours. The reaction mixture was poured into saturated ammonium chloride aqueous solution and extracted with ethyl acetate (20 mL × 3). The organic phases were combined, dried and concentrated. The resulting material was purified by Flash column chromatography (0 to 10% methanol/dichloromethane) to obtain 280 mg of Compound D21-5 as a yellow oil (yield: 56%). LCMS: $[M+H]^+$ = 495/497.

Step D: Synthesis of Target Compound

Compound D21-5 (50 mg, 0.1 mmol) was added to anhydrous dimethyl sulfoxide (1 mL). Sodium methoxide (22 mg, 0.4 mmol), copper(I) iodide (2 mg, 0.01 mmol), and trans-(1R,2R)-N,N'-dimethylcyclohexane-1,2-diamine (3 mg, 0.02 mmol) were added. The atmosphere was purged with nitrogen. The mixture was reacted at 110 C for 16 hours. The reaction mixture was poured into saturated ammonium chloride aqueous solution and extracted with ethyl acetate (10 mL × 3). The organic phases were combined, dried and concentrated. The resulting material was purified by preparative silica gel TLC (dichloromethane/methanol = 10:1) to obtain 12.6 mg of the target compound as a white solid (yield: 30%).

LCMS: $[M+H]^+$ = 417. $^1$H NMR (400 MHz, DMSO-d6) δ 8.34 (s, 2H), 8.26 (s, 2H), 7.73 (d, J = 7.6 Hz, 1H), 7.68 (dd, J = 6.8, 2.1 Hz, 1H), 7.56 (d, J = 7.6 Hz, 1H), 7.51 (ddd, J = 8.8, 6.6, 2.0 Hz, 1H), 7.06 (s, 1H), 6.51 - 6.44 (m, 1H), 6.32 (td, J = 6.6, 1.2 Hz, 1H), 5.96 (s, 2H), 5.14 (q, J = 6.4, 5.4 Hz, 1H), 5.10 - 5.03 (m, 1H), 2.14 (t, J = 6.0 Hz, 2H).

Example D22

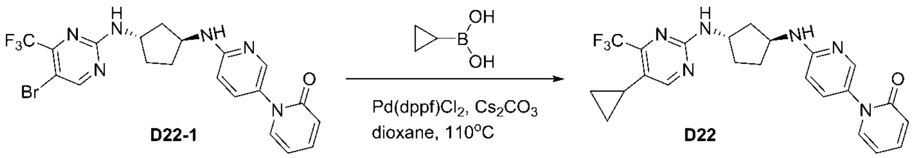


D22-1 D22

Compound D22-1 (280 mg, 0.57 mmol) was added to 1,4-dioxane (3 mL). Cyclopropylboronic acid (58 mg, 0.68 mmol), cesium carbonate (279 mg, 0.86 mmol), and [1,1'-bis(diphenylphosphino)ferrocene]dichloropalladium(II) (44 mg, 0.06 mmol) were added. The atmosphere was purged with nitrogen. The mixture was heated to 110 °C and reacted for 2 hours. The reaction mixture was dried and concentrated. The resulting material was purified by Flash column chromatography (0 to 10% methanol/dichloromethane) to obtain 62.8 mg of the target compound as a white solid (yield: 24%).

LCMS: $[M+H]^+$ = 457. H NMR (400 MHz, DMSO-$d_6$) δ 8.31 (s, 1H), 7.92 (d, J = 2.8 Hz, 1H), 7.77 (d, J = 6.8 Hz, 1H), 7.60 (dd, J = 6.8, 2.0 Hz, 1H), 7.48 (ddd, J = 9.2, 6.6, 2.1 Hz, 1H), 7.39 (dd, J = 8.8, 2.8 Hz, 1H), 6.93 (d, J = 6.8 Hz, 1H), 6.52 (d, J = 9.2 Hz, 1H), 6.44 (dd, J = 9.2, 1.1 Hz, 1H), 6.27 (td, J = 6.8, 1.4 Hz, 1H), 4.30 (dd, J = 13.6, 7.2 Hz, 2H), 2.12 (ddt, J = 16.0, 8.4, 5.2 Hz, 2H), 1.97 - 1.80 (m, 3H), 1.52 (ddt, J = 18.0, 12.4, 7.2 Hz, 2H), 0.93 - 0.82 (m, 2H), 0.71 (dt, J = 5.6, 3.2 Hz, 2H).

Example D23

Synthesis of 3-chloro-6'-(((1S,3S)-3-(pyrimidin-2-ylamino)cyclopentyl)amino)-2H-[1,3'-bipyridin]-2-one

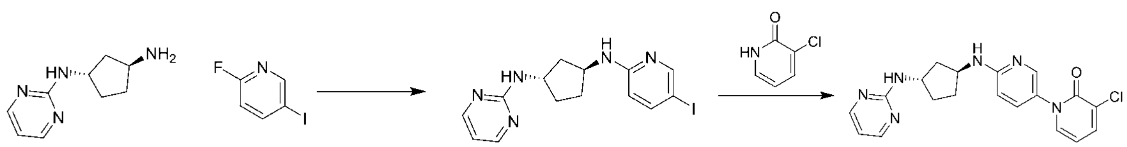

Step 1: Synthesis of (1S,3S)-$N^1$-(5-iodopyridin-2-yl)-$N^3$-(pyrimidin-2-yl)cyclopentane-1,3-diamine

At 25 °C, (1S,3S)-$N^1$-(pyrimidin-2-yl)cyclopentane-1,3-diamine 2.5 hydrochloride (900 mg, 3.34 mmol), 2-fluoro-5-iodopyridine (890 mg, 4.01 mmol), and potassium carbonate (2.31 g, 16.7 mmol) were dissolved in DMSO (20 mL). The mixture was stirred at 140 °C for 12 hours.

Water (100 mL) was added. Then the mixture was extracted with EA (100 mL * 2). The organic phases were combined, washed with saturated brine (50 mL) and concentrated to obtain the crude product. The resulting residue was purified by normal phase column chromatography (n-hexane:ethyl acetate 10:1 to 1:1) to obtain 590 mg of (1S,3S)-$N^1$-(5-iodopyridin-2-yl)-$N^3$-(pyrimidin-2-yl)cyclopentane-1,3-diamine as a pale yellow solid (590 mg, 46.3% yield).LC-MS: $[M+H]^+$ = 382.

Step 2: Synthesis of 3-chloro-6'-(((1S,3S)-3-(pyrimidin-2-ylamino)cyclopentyl)amino)-2H-[1,3'-bipyridin]-2-one

At 25 °C, (1S,3S)-$N^1$-(5-iodopyridin-2-yl)-$N^3$-(pyrimidin-2-yl)cyclopentane-1,3-diamine (540 mg, 1.42 mmol), 3-chloropyridin-2(1H)-one (580 mg, 2.48 mmol), potassium phosphate (600 mg, 2.48 mmol), copper(I) iodide (54 mg, 0.28 mmol), and (1R,2R)-N,N'-dimethylcyclohexane-1,2-diamine (87 mg, 0.57 mmol) were dissolved in DMSO (20 mL). The mixture was stirred at 130 C for 12 hours under nitrogen protection.

Water (100 mL) was added. Then the mixture was extracted with EA (100 mL * 2). The organic phases were combined, washed with saturated brine (50 mL) and concentrated to obtain the crude product. The crude product was purified by preparative chromatography using formic acid system to obtain 140 mg of 3-chloro-6'-(((1S,3S)-3-(pyrimidin-2-ylamino)cyclopentyl)amino)-2H-[1,3'-bipyridin]-2-one as a pale yellow solid (140 mg, 25.8% yield). LC-MS: [M+H]+ = 383.

H NMR (400 MHz, DMSO-d6) δ 8.25 (d, J = 4.8 Hz, 2H), 7.96 (d, J = 2.7 Hz, 1H), 7.84 - 7.78 (m, 1H), 7.69 - 7.63 (m, 1H), 7.43 (dd, J = 8.9, 2.7 Hz, 1H), 7.22 (d, J = 7.3 Hz, 1H), 6.99 (d, J = 6.9 Hz, 1H), 6.53 (dd, J = 6.7, 4.0 Hz, 2H), 6.31 (t, J = 7.0 Hz, 1H), 4.35 (dp, J = 14.8, 6.8 Hz, 2H), 2.13 (tt, J = 14.8, 6.3 Hz, 2H), 1.98 - 1.81 (m, 2H), 1.52 (tt, J = 13.8, 7.4 Hz, 2H).

Example D24

According to the aforementioned preparation methods, Compound D24 was prepared and separated to obtain Compounds D24A and D24B:

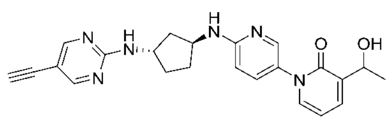

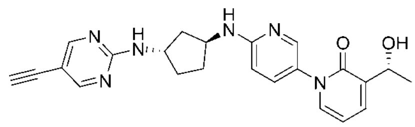

D24A or D24B

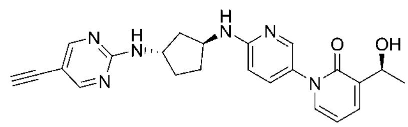

D24B or D24A

D24A: $^1$H NMR (400 MHz, DMSO-$d_6$) δ = 8.38 (br s, 2H), 7.91 (d, *J* = 2.6 Hz, 1H), 7.83 - 7.76 (m, 1H), 7.55 - 7.47 (m, 2H), 7.38 (dd, *J* = 2.6, 8.9 Hz, 1H), 6.92 (d, *J* = 6.9 Hz, 1H), 6.52 (d, *J* = 8.9 Hz, 1H), 6.31 (t, *J* = 6.8 Hz, 1H), 5.07 (d, *J* = 4.5 Hz, 1H), 4.71 (quin, *J* = 5.8 Hz, 1H), 4.43 - 4.27 (m, 2H), 4.24 (s, 1H), 2.20 - 2.05 (m, 2H), 1.96 - 1.81 (m, 2H), 1.61 - 1.43 (m, 2H), 1.25 (d, *J* = 6.4 Hz, 3H).

D24B: $^1$H NMR (400 MHz, DMSO-$d_6$) δ = 8.39 (br s, 2H), 7.91 (d, *J* = 2.6 Hz, 1H), 7.80 (d, *J* = 7.4 Hz, 1H), 7.55 - 7.48 (m, 2H), 7.39 (dd, *J* = 2.6, 8.9 Hz, 1H), 6.93 (d, *J* = 6.9 Hz, 1H), 6.52 (d, *J* = 8.9 Hz, 1H), 6.31 (t, *J* = 6.8 Hz, 1H), 5.07 (d, *J* = 4.5 Hz, 1H), 4.76 - 4.66 (m, 1H), 4.45 - 4.26 (m, 2H), 4.24 (s, 1H), 2.19 - 2.06 (m, 2H), 1.96 - 1.82 (m, 2H), 1.62 - 1.43 (m, 2H), 1.25 (d, *J* = 6.4 Hz, 3H)

Example D25

According to the aforementioned preparation methods, Compound D25 was prepared and separated to obtain Compounds D25A and D25B:

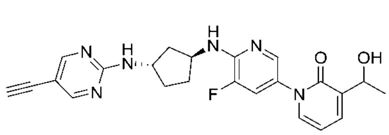

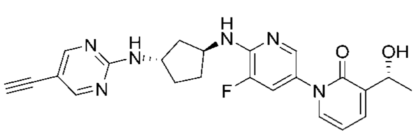

D25A or D25B

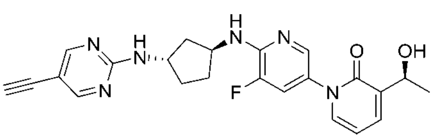

D25B or D25A

D25A: $^1$H NMR (400 MHz, DMSO-$d_6$) δ = 8.38 (br s, 2H), 7.83 (d, *J* = 2.1 Hz, 1H), 7.79 (d, *J* = 7.4 Hz, 1H), 7.57 - 7.50 (m, 3H), 6.94 (d, *J* = 6.5 Hz, 1H), 6.33 (t, *J* = 6.8 Hz, 1H), 5.09 (d, *J* = 4.6 Hz, 1H), 4.71 (quin, *J* = 5.8 Hz, 1H), 4.56 - 4.45 (m, 1H), 4.44 - 4.34 (m, 1H), 4.24 (s, 1H), 2.19 - 2.06 (m, 2H), 2.00 - 1.88 (m, 2H), 1.66 - 1.48 (m, 2H), 1.25 (d, *J* = 6.4 Hz, 3H).

D25B: $^1$H NMR (400 MHz, DMSO-$d_6$) δ = 8.38 (br s, 2H), 7.83 (d, *J* = 2.1 Hz, 1H), 7.79 (d, *J* = 7.4 Hz, 1H), 7.59 - 7.49 (m, 3H), 6.94 (d, *J* = 6.5 Hz, 1H), 6.33 (t, *J* = 6.8 Hz, 1H), 5.09 (d, *J* = 4.5 Hz, 1H), 4.71 (quin, *J* = 5.8 Hz, 1H), 4.50 (sxt, *J* = 7.0 Hz, 1H), 4.44 - 4.34 (m, 1H), 4.24 (s, 1H), 2.18 - 2.08 (m, 2H), 2.00 - 1.88 (m, 2H), 1.67 - 1.49 (m, 2H), 1.25 (d, *J* = 6.4 Hz, 3H).

Example D26 to D28

| | | | |
|---|---|---|---|
| D26 | 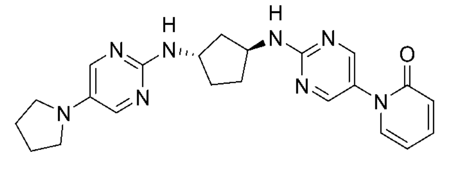<br>$^1$H NMR (400 MHz, DMSO-$d_6$)<br>δ 8.31 (s, 2H), 7.82 (s, 2H), 7.71 - 7.57 (m, 2H), 7.50 (ddd, *J* = 9.0, 6.6, 2.1 Hz, 1H), 6.47 (d, *J* = 9.2 Hz, 1H), 6.41 - 6.25 (m, 2H), 4.34 (p, *J* = 6.9 Hz, 1H), 4.23 (h, *J* = 6.7 Hz, 1H), 3.18 - 3.06 (m, 4H), 2.14 - 2.03 (m, 2H), 1.99 - 1.76 (m, 6H), 1.59 - 1.44 (m, 2H). | D27 | 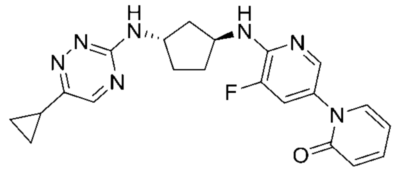<br>$^1$H NMR: (400 MHz, DMSO-$d_6$)<br>δ 8.17 - 8.11 (m, 1H), 7.79 (d, *J* = 2.0 Hz, 1H), 7.64 - 7.57 (m, 1H), 7.54 - 7.46 (m, 2H), 6.94 (br d, *J* = 6.0 Hz, 1H), 6.47 (d, *J* = 9.4 Hz, 1H), 6.32 (t, *J* = 6.4 Hz, 1H), 4.55 - 4.42 (m, 1H), 4.37 - 4.27 (m, 1H), 2.18 - 2.08 (m, 2H), 2.02 (dt, *J* = 4.3, 8.6 Hz, 1H), 1.97 - 1.89 (m, 2H), 1.64 - 1.48 (m, 2H), 0.99 - 0.91 (m, 2H), 0.88 - 0.82 (m, 2H) |
| D28 | 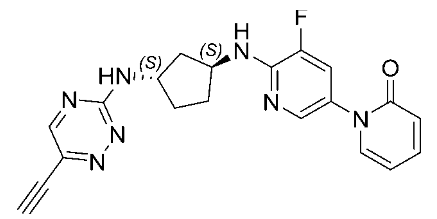 | $^1$H NMR($CDCl_3$, 400 MHz)<br><br>δ (ppm) = 8.22 (s, IH), 7.88 (s, 1H), 7.44 - 7.36 (m, 2H), 7.36 - 7.29 (m, 2H), 6.66 (d, *J* = 9.6 Hz, 1H), 6.25 (t, *J* = 7.2 Hz, 1H), 4.89 - 4.75 (m, 1H), 4.70 - 4.50 (m, 2H), 3.44 (s, 1H), 2.48 - 2.36 (m, 2H), 2.14 (t, *J* = 6.4 Hz, 2H), 1.75 - 1.67 (m, 2H). | |

Example D29

Synthesis of 5-cyclopropyl-2-(((1S,3S)-3-((2-oxo-2H-[1,3'-bipyridin]-6'-yl)amino)cyclopentyl)amino)pyrimid ine-4-carbonitrile

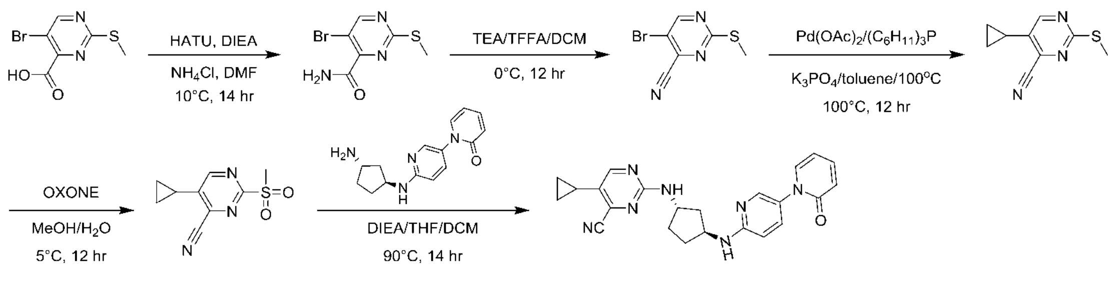


Step 1: Synthesis of 5-bromo-2-(methylsulfonyl)pyrimidine-4-carboxamide

5-Bromo-2-(methylthio)-4-pyrimidinecarboxylic acid (5 g, 20.1 mmol, 1 eq) was dissolved in N,N-dimethylformamide (50 mL). Ammonium chloride (5.4 g, 101 mmol, 5.03 eq), O-(7-azabenzotriazol-1-yl)-N,N,N,N-tetramethyluronium hexafluorophosphate (11.5 g, 30.24 mmol, 1.51 eq), and N,N-diisopropylethylamine (13.4 g, 103 mmol, 18 mL, 5.15

eq) were added. The mixture was reacted at 10 °C for 14 hours. Ice water (300 mL) was added to the reaction mixture to quench. Ethyl acetate (50 mL) was added and the mixture was stirred for 10 min. The precipitated solid was filtered. The filter cake was collected and dried under vacuum at 45 °C to obtain the product 5-bromo-2-(methylsulfonyl)pyrimidine-4-carboxamide (4.63 g).
LCMS: RT = 0.502 min, MS (ESI) m/z = 248.0, 250.0 $[M+1]^+$

Step 2: Synthesis of 5-bromo-2-(methylthio)pyrimidine-4-carbonitrile

5-Bromo-2-(methylsulfonyl)pyrimidine-4-carboxamide (1 g, 4.03 mmol, 1 eq) was dissolved in dichloromethane (30 mL). Triethylamine (2.18 g, 21.55 mmol, 3 mL, 5.35 eq) and trifluoroacetic anhydride (2.27 g, 10.79 mmol, 1.5 mL, 2.68 eq) were added at 0 °C. The reaction was reacted at 0 °C for 14 hours. Water (50 mL) was added to the reaction mixture. The mixture was extracted with dichloromethane (50 mL × 3). The organic phases were combined, washed with saturated brine (50 mL × 2), dried over anhydrous sodium sulfate, filtered, and concentrated to obtain the product 5-bromo-2-(methylthio)pyrimidine-4-carbonitrile (830 mg). LCMS: RT = 0.680 min, MS (ESI) m/z = 231.9 $[M+1]^+$

Step 3: Synthesis of 5-cyclopropyl-2-(methylthio)pyrimidine-4-carbonitrile

5-Bromo-2-(methylthio)pyrimidine-4-carbonitrile (1 g, 4.35 mmol, 1 eq) and cyclopropylboronic acid (1.2 g, 13.97 mmol, 3.21 eq) were dissolved in toluene (20 mL) and water (2 mL). Tricyclohexylphosphine (500 mg, 1.78 mmol, 0.41 eq), potassium phosphate (5.7 g, 26.85 mmol, 6.18 eq), and palladium(II) acetate (200 mg, 0.89 mmol, 0.205 eq) were added. the mixture was stirred at 100 °C for 12 hours under a nitrogen atmosphere. The reaction mixture was concentrated. The resulting crude product was purified by silica gel column chromatography (petroleum ether/ethyl acetate, 1/0 to 20/1, V/V) to obtain the product 5-cyclopropyl-2-(methylthio)pyrimidine-4-carbonitrile (200 mg, 24.1% yield). LCMS: RT = 0.539 min, MS (ESI) m/z = 192.1 $[M+1]^+$

Step 4: Synthesis of 5-cyclopropyl-2-(methylsulfonyl)pyrimidine-4-carbonitrile

5-Cyclopropyl-2-(methylthio)pyrimidine-4-carbonitrile (100 mg, 0.523 mmol, 1 eq) was dissolved in methanol (12.5 mL). An aqueous solution of potassium peroxymonosulfate (2.00 g, 3.25 mmol, 6.22 eq) in water (2.5 mL) was added. The reaction was reacted at 5 °C for 12 hours. The organic solvent was evaporated. Dichloromethane (20 mL) was added. The mixture was extracted with dichloromethane (10 mL × 3). The organic phases were combined, washed with saturated sodium sulfite aqueous solution (50 mL) and saturated brine (50 mL), dried over anhydrous sodium sulfate, filtered, and concentrated to obtain the product 5-cyclopropyl-2-(methylsulfonyl)pyrimidine-4-carbonitrile (120 mg). LCMS: RT = 0.428 min, MS (ESI) m/z = 224.0 $[M+1]^+$

Step 5: Synthesis of 5-cyclopropyl-2-(((1S,3S)-3-((2-oxo-2H-[1,3'-bipyridin]-6'-yl)amino)cyclopentyl)amino)pyrimid ine-4-carbonitrile

5-Cyclopropyl-2-(methylsulfonyl)pyrimidine-4-carbonitrile (95 mg, 0.43 mmol, 1 eq) and 6'-(((1S,3S)-3-aminocyclopentyl)amino)-2H-[1,3'-bipyridin]-2-one (146 mg, 0.54 mmol, 1.27 eq) were dissolved in tetrahydrofuran (6 mL) and dichloromethane (3 mL). N,N-Diisopropylethylamine (0.37 g, 2.87 mmol, 0.5 mL, 6.75 eq) was added. The mixture was reacted at 90 °C for 14 hours. After concentration, the reaction mixture was directly purified by preparative chromatography (column: Phenomenex luna C18 150 × 25 mm × 10 μm; mobile phase: formic acid in water-acetonitrile; gradient: acetonitrile 18% to 38%, 10 min) to obtain 5-cyclopropyl-2-(((1S,3S)-3-((2-oxo-2H-[1,3'-bipyridin]-6'-yl)amino)cyclopentyl)amino)pyrimid ine-4-carbonitrile (35.8 mg).

$^1H$ NMR: (400 MHz, MeOD) δ 8.18 (s, 1H), 7.94 (d, J = 2.4 Hz, 1H), 7.65 - 7.54 (m, 2H), 7.44 (dd, J = 2.6, 9.0 Hz, 1H), 6.67 - 6.58 (m, 2H), 6.50 - 6.42 (m, 1H), 4.50 - 4.23 (m, 2H), 2.37 - 2.16 (m, 2H), 2.10 - 1.88 (m, 3H), 1.69 - 1.52 (m, 2H), 1.11 - 1.00 (m, 2H), 0.80 - 0.69 (m, 2H).

Example E1

Synthesis of 1-(2-(((1S,3S)-3-((5-(difluoromethoxy)pyrimidin-2-yl)amino)cyclopentyl)amino)benzo[d]thiazol -5-yl)pyridin-2(1H)-one

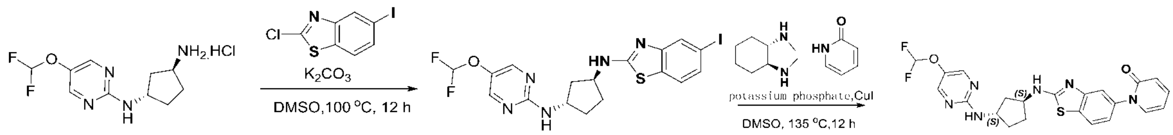


Step A: Synthesis of (1S,3S)-N1-(5-(difluoromethoxy)pyrimidin-2-yl)-N3-(5-iodobenzo[d]thiazol-2-yl)cyclopentane-1 ,3-diamine

At room temperature, 2-chloro-5-iodobenzo[d]thiazole (500.0 mg, 1.69 mmol) and (1S,3S)-N1-(5-(difluoromethoxy)pyrimidin-2-yl)cyclopentane-1,3-diamine hydrochloride (522 mg, 1.86 mmol) were dissolved in DMSO (10 mL). Potassium carbonate (935 mg, 6.76 mmol) was added. After addition, the system was heated to 100 °C and reacted for 12 hours.

After completion of the reaction, the mixture was cooled to room temperature. Water (40 mL) was added to the system. The mixture was extracted with ethyl acetate (20 mL × 3). The organic phases were combined, washed with saturated brine (30 mL), dried, filtered, and concentrated under reduced pressure to dryness to obtain the residue. The residue was purified by normal phase column chromatography (n-hexane/ethyl acetate = 1:2) to obtain 500 mg of (1S,3S)-N1-(5-(difluoromethoxy)pyrimidin-2-y1)-N3-(5-iodobenzo[d]thiazol-2-yl)cyclopentane-1 ,3-diamine as a white solid (yield: 59%). LC-MS: $[M+H]^+$ = 504.

Step B: Synthesis of 1-(2-(((1S,3S)-3-((5-(difluoromethoxy)pyrimidin-2-yl)amino)cyclopentyl)amino)benzo[d]thiazol -5-yl)pyridin-2(1H)-one

At room temperature, (1S,3S)-N1-(5-(difluoromethoxy)pyrimidin-2-yl)-N3-(5-iodobenzo[d]thiazol-2-yl)cyclopentane-1 ,3-diamine (300 mg, 0.59 mmol), pyridin-2(1H)-one (113 mg, 1.18 mmol), (1S,2S)-N1,N2-dimethylcyclohexane-1,2-diamine (16.9 mg, 0.12 mmol), and potassium phosphate (379.3 mg, 1.77 mmol) were added to DMSO (8 mL). Cuprous iodide (22.9 mg, 0.12 mmol) was added. After addition, the atmosphere was purged with nitrogen. The system was heated to 140 °C andreacted for 12 hours.

After completion of the reaction, the mixture was cooled to room temperature. Water (40 mL) was added to the system. The mixture was extracted with ethyl acetate (20 mL × 3). The organic phases were combined, washed with saturated brine (30 mL), dried, filtered, and concentrated under reduced pressure to dryness to obtain the residue. The residue was purified by normal phase column chromatography (dichloromethane/methanol = 10:1) to obtain 80 mg of 1-(2-(((1S,3S)-3-((5-(difluoromethoxy)pyrimidin-2-yl)amino)cyclopentyl)amino)benzo[d]thiazol -5-yl)pyridin-2(1H)-one as a white solid (yield: 28.5%).

LC-MS: [M+H]+ = 471. H NMR (400 MHz, DMSO-d6) δ 8.33 - 8.29 (d, J = 6.8 Hz, 1H), 8.26 - 8.22 (s, 2H), 7.79 - 7.75 (d, J = 8.2 Hz, 1H), 7.67 - 7.63 (m, 1H), 7.55 - 7.47 (m, 2H), 7.34 - 7.32 (d, J = 2.0 Hz, 1H), 7.22 - 6.84 (m, 2H), 6.49 - 6.44 (d, J = 9.2 Hz, 1H), 6.32 - 6.27 (m, 1H), 4.39 - 4.27 (m, 2H), 2.25 - 2.06 (m, 2H), 1.99 - 1.93 (t, J = 6.7 Hz, 2H), 1.63 - 1.51 (m, 2H).

Example E2

Synthesis of 1-(2-(((1S,3S)-3-((5-(difluoromethoxy)pyrimidin-2-yl)amino)cyclopentyl)amino)benzo[d]thiazol -6-yl)pyridin-2(1H)-one

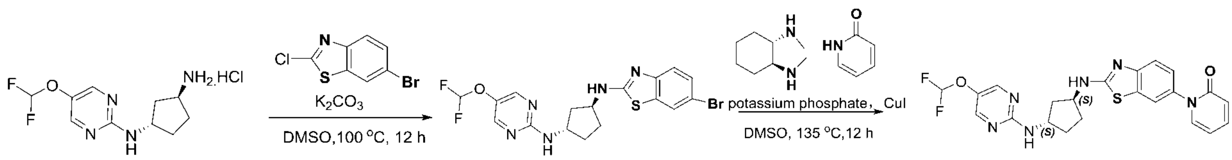


Step A: Synthesis of (1S,3S)-N1-(6-bromobenzo[d]thiazol-2-yl)-N3-(5-(difluoromethoxy)pyrimidin-2-yl)cyclopentane -1,3-diamine

At room temperature, 6-bromo-2-chlorobenzo[d]thiazole (500.0 mg, 2.02 mmol) and (1S,3S)-N1-(5-(difluoromethoxy)pyrimidin-2-yl)cyclopentane-1,3-diamine hydrochloride (623 mg, 2.22 mmol) were dissolved in DMSO (10 mL). Potassium carbonate (1.1 g, 8.08 mmol) was added. After addition, the system was heated to 100 °C and reacted for 12

hours.

After completion of the reaction, the mixture was cooled to room temperature. Water (40 mL) was added to the system. The mixture was extracted with ethyl acetate (20 mL × 3). The organic phases were combined, washed with saturated brine (30 mL), dried, filtered, and concentrated under reduced pressure to dryness to obtain the residue. The residue was purified by normal phase column chromatography (n-hexane/ethyl acetate = 1:2) to obtain 600 mg of (1S,3S)-$N^1$-(6-bromobenzo[d]thiazol-2-yl)-$N^3$-(5-(difluoromethoxy)pyrimidin-2-yl)cyclopentane -1,3-diamine as a white solid (yield: 65%).

LC-MS: $[M+H]^+$ = 456.

Step B: Synthesis of 1-(2-(((1S,3S)-3-((5-(difluoromethoxy)pyrimidin-2-yl)amino)cyclopentyl)amino)benzo[d]thiazol -6-yl)pyridin-2(1H)-one

At room temperature, (1S,3S)-$N^1$-(6-bromobenzo[d]thiazol-2-yl)-$N^3$-(5-(difluoromethoxy)pyrimidin-2-yl)cyclopentane -1,3-diamine (300 mg, 0.65 mmol), pyridin-2(1H)-one (125 mg, 1.3 mmol), (1S,2S)-$N^1$,$N^2$-dimethylcyclohexane-1,2-diamine (18.7 mg, 0.13 mmol), and potassium phosphate (379.3 mg, 1.95 mmol) were added to DMSO (8 mL). Cuprous iodide (25.3 mg, 0.13 mmol) was added. After addition, the atmosphere was purged with nitrogen. The system was heated to 140 °C and reacted for 12 hours.

After completion of the reaction, the mixture was cooled to room temperature. Water (40 mL) was added to the system. The mixture was extracted with ethyl acetate (20 mL × 3). The organic phases were combined, washed with saturated brine (30 mL), dried, filtered, and concentrated under reduced pressure to dryness to obtain the residue. The residue was purified by normal phase column chromatography (dichloromethane/methanol = 10:1) to obtain 130 mg of 1-(2-(((1S,3S)-3-((5-(difluoromethoxy)pyrimidin-2-yl)amino)cyclopentyl)amino)benzo[d]thiazol -6-yl)pyridin-2(1H)-one as a white solid (yield: 42.0%).

LC-MS: $[M+H]^+$ = 471. H NMR (400 MHz, DMSO-d6) δ 8.30 - 8.22 (m, 3H), 7.74 - 7.71 (d, J = 2.2 Hz, 1H), 7.66 - 7.62 (m, 1H), 7.55 - 7.41 (m, 3H), 7.23 - 6.84 (m, 2H), 6.49 - 6.44 (m, 1H), 6.31 - 6.26 (m, 1H), 4.40 - 4.28 (m, 2H), 2.26 - 2.06 (m, 2H), 2.00 - 1.93 (t, J = 6.7 Hz, 2H), 1.63 - 1.50 (m, 2H).

Examples E3 to E11

According to the general preparation methods and the preparation methods of Examples E1 and E2, the following Compounds were prepared:

| No. | Compound | No. | Compound |
|---|---|---|---|
| E3 | 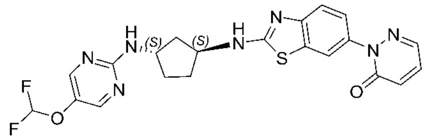 1H NMR (400 MHz, DMSO-d6) δ 8.37 - 8.16 (d, J = 20.1 Hz, 3H), 8.08 - 8.01 (m, 1H), 7.89 - 7.81 (s, 1H), 7.58 - 7.29 (m, 4H), 7.23 - 6.83 (m, 2H), 4.43 - 4.27 (p, J = 6.9 Hz, 2H), 2.26 - 2.07 (m, 2H), 2.02 - 1.91 (t, J = 6.8 Hz, 2H), 1.66 - 1.50 (m, 2H). | E4 | 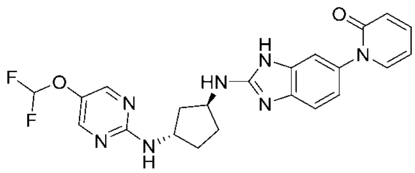 $^1$H NMR (400 MHz, DMSO-$d_6$) δ (ppm) = 10.86 - 10.76 (m, 1H), 8.24 (s, 2H), 7.65 - 7.57 (m, 1H), 7.52 - 7.44 (m, 2H), 7.25 - 7.00 (m, 3H), 6.93 - 6.73 (m, 2H), 6.44 (br d, *J* = 8.8 Hz, 1H), 6.26 (t, *J* = 7.2 Hz, 1H), 4.36 - 4.29 (m, 1H), 4.28 - 4.21 (m, 1H), 2.23 - 2.06 (m, 2H), 1.94 (t, *J* = 7.2 Hz, 2H), 1.61 - 1.50 (m, 2H). |

(continued)

| No. | Compound | No. | Compound |
|---|---|---|---|
| E5 | 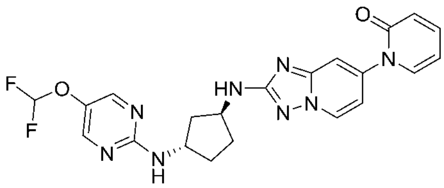<br>$^1$H NMR (METHANOL-$d_4$, 400 MHz) δ 8.56 (br d, 1H, *J*=4.5 Hz), 8.17 (br s, 2H), 7.6-7.7 (m, 2H), 7.46 (br s, 1H), 7.02 (br d, 1H, *J*=6.6 Hz), 6.6-6.9 (m, 2H), 6.5-6.5 (m, 1H), 4.4-4.5 (m, 1H), 4.26 (br d, 1H, *J*=5.8 Hz), 2.2-2.4 (m, 3H), 2.0-2.1 (m, 2H), 1.6-1.7 (m, 2H), 1.2-1.3 (m, 2H). | E6 | 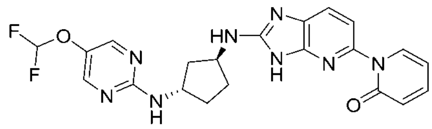<br>$^1$H NMR (400 MHz, DMSO-$d_6$) δ (ppm) = 11.46 - 10.75 (m, 1H), 8.24 (s, 2H), 7.76 (dd, *J* = 1.6, 7.2 Hz, 1H), 7.54 - 7.44 (m, 3H), 7.22 - 6.85 (m, 2H), 6.46 (d, *J* = 9.6 Hz, 1H), 6.30 (dt, *J* = 1.2, 6.4 Hz, 1H), 4.40 - 4.19 (m, 2H), 2.26 - 2.07 (m, 2H), 2.00 - 1.90 (m, 2H), 1.68 - 1.49 (m, 2H). |
| E7 | 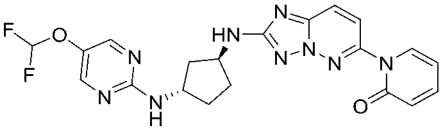<br>1H NMR (400 MHz, DMSO-d6) δ = 8.23 (s, 2H), 8.06 (d, J = 9.4 Hz, 1H), 7.84 (dd, J = 1.4, 6.9 Hz, 1H), 7.70 (d, J = 9.4 Hz, 1H), 7.60 (ddd, J = 2.0, 6.8, 9.1 Hz, 1H), 7.49 (d, J = 7.1 Hz, 1H), 7.23 - 7.15 (m, 1H), 7.05 - 6.82 (m, 1H), 6.56 (d, J = 9.3 Hz, 1H), 6.41 (dt, J = 1.2, 6.8 Hz, 1H), 4.37 - 4.26 (m, 1H), 4.25 - 4.15 (m, 1H), 2.21 - 2.05 (m, 2H), 2.00 - 1.87 (m, 2H), 1.64 - 1.49 (m, 2H). | E8 | 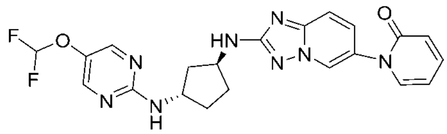<br>$^1$H NMR (METHANOL-$d_4$, 400 MHz) δ 8.73 (d, 1H, *J*=1.8 Hz), 8.17 (s, 2H), 7.6-7.7 (m, 2H), 7.5-7.6 (m, 1H), 7.46 (d, 1H, *J*=9.4 Hz), 6.4-6.9 (m, 3H), 4.3-4.5 (m, 1H), 4.2-4.3 (m, 1H), 2.27 (td, 2H, *J*=5.3, 10.6 Hz), 2.0-2.1 (m, 2H), 1.5-1.7 (m, 2H). |
| E9 | 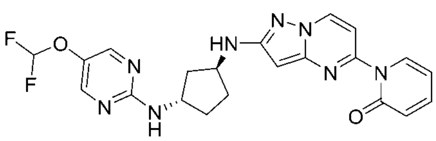<br>1H NMR (400 MHz, DMSO-d6) δ = 8.83 (d, J = 7.1 Hz, 1H), 8.23 (s, 2H), 7.91 (dd, J = 1.7, 7.1 Hz, 1H), 7.54 (ddd, J = 2.1, 6.7, 9.1 Hz, 1H), 7.48 (d, J = 7.3 Hz, 1H), 7.23 - 6.83 (m, 2H), 6.51 (d, J = 9.3 Hz, 1H), 6.47 (d, J = 7.3 Hz, 1H), 6.38 (dt, J = 1.2, 6.8 Hz, 1H), 5.82 (s, 1H), 4.31 (sxt, J = 7.0 Hz, 1H), 4.15 - 4.00 (m, 1H), 2.19 - 2.06 (m, 2H), 1.91 (t, J = 6.7 Hz, 2H), 1.58 - 1.47 (m, 2H). | E10 | 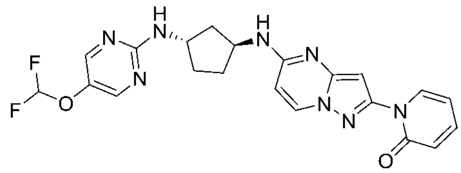<br>1H NMR (400 MHz, DMSO-d6) δ = 8.50 - 8.41 (m, 1H), 8.24 (s, 2H), 8.01 (dd, J = 1.7, 7.1 Hz, 1H), 7.66 (d, J = 7.0 Hz, 1H), 7.56 - 7.44 (m, 2H), 7.24 - 6.83 (m, 1H), 6.50 (d, J = 9.0 Hz, 1H), 6.41 (s, 1H), 6.38 - 6.26 (m, 2H), 4.47 - 4.28 (m, 2H), 2.23 - 2.05 (m, 2H), 1.99 - 1.86 (m, 2H), 1.62 - 1.44 (m, 2H). |
| E11 | 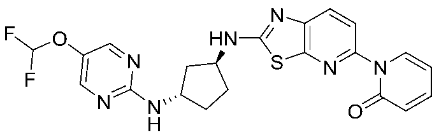 | $^1$H NMR: (400 MHz, DMSO-*d6*)δ 8.68 - 8.51 (m, 1H), 8.25 (s, 2H), 7.86 - 7.75 (m, 2H), 7.60 - 7.47 (m, 3H), 7.26 - 6.84 (m, 1H), 6.50 (d, *J* = 9.4 Hz, 1H), 6.37 - 6.29 (m, 1H), 4.50 - 4.27 (m, 2H), 2.29 - 2.07 (m, 2H), 1.98 (br t, *J* = 6.6 Hz, 2H), 1.66 - 1.50 (m, 2H). | | |

Example E12

Synthesis of 1-(1-(((1S,3S)-3-((5-(difluoromethoxy)pyrimidin-2-yl)amino)cyclopentyl)amino)isoquinolin-4-y 1)pyridin-2(1H)-one

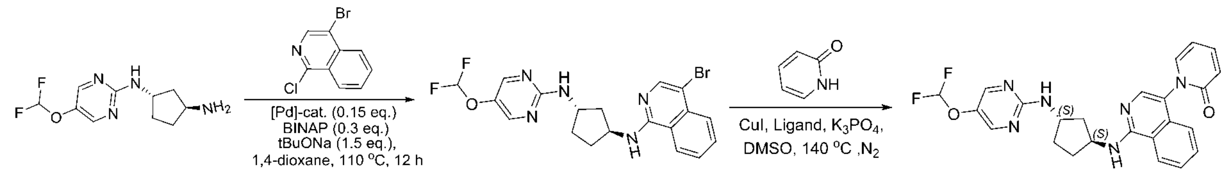


Step A: Synthesis of (1S,3S)-N1-(4-bromoisoquinolin-1-yl)-N3-(5-(difluoromethoxy)pyrimidin-2-yl)cyclopentane-1,3 -diamine

At room temperature under nitrogen protection, (1S,3S)-N1-(5-(difluoromethoxy)pyrimidin-2-yl)cyclopentane-1,3-diamine (1.04 g, 4.25 mmol, 1.0 eq), 4-bromo-1-chloroisoquinoline (1.23 g, 5.1 mmol, 1.2 eq), tris(dibenzylideneacetone)dipalladium (0.584 g, 0.64 mmol, 0.15 eq), S-(-)-1,1'-binaphthyl-2,2'-bis(diphenylphosphine) (BINAP, 0.8 g, 1.28 mmol, 0.3 eq), and sodium tert-butoxide (0.61 g, 6.4 mmol, 1.5 eq) were dissolved in 1,4-dioxane (20 mL). The mixture was heated to 110 °C and reacted overnight.

After completion of the reaction, cold water (50 mL) was added to quench the reaction. The mixture was extracted with ethyl acetate (75 mL × 3). The organic phases were combined, dried over anhydrous sodium sulfate, filtered, and concentrated. The resulting residue was purified by silica gel column chromatography (eluent: ethyl acetate/hexane = 5/10) to obtain 0.74 g of (1S,3S)-N1-(4-bromoisoquinolin-1-yl)-N3-(5-(difluoromethoxy)pyrimidin-2-yl)cyclopentane-1,3 -diamine as a yellow solid (yield: 40%). LC-MS: $[M+H]^+$ = 450.

Step B: Synthesis of 1-(1-(((1S,3S)-3-((5-(difluoromethoxy)pyrimidin-2-yl)amino)cyclopentyl)amino)isoquinolin-4-y 1) pyridin-2(1H)-one

At room temperature under nitrogen protection, (1S,3S)-N1-(4-bromoisoquinolin-1-yl)-N3-(5-(difluoromethoxy) pyrimidin-2-yl)cyclopentane-1,3 -diamine (0.63 g, 1.40 mmol, 1.0 eq), pyridin-2(1H)-one (0.40 g, 4.2 mmol, 3.0 eq), copper(I) iodide (0.80 g, 0.42 mmol, 0.3 eq), N1,N2-bis(2-thiophenmethyl)-ethylenediamine (0.12 g, 0.42 mmol, 0.3 eq), and potassium phosphate (0.89 g, 4.2 mmol, 3.0 eq) were dissolved in dimethyl sulfoxide (6 mL). The mixture was heated to 140 °C and reacted overnight.

After completion of the reaction, cold water (50 mL) was added to quench the reaction. The mixture was extracted with ethyl acetate (75 mL × 3). The organic phases were combined, dried over anhydrous sodium sulfate, filtered, and concentrated. The resulting residue was purified by silica gel column chromatography (eluent: ethyl acetate/methanol = 10/1) to obtain 0.045 g of 1-(1-(((1 S,3S)-3-((5-(difluoromethoxy)pyrimidin-2-yl)amino)cyclopentyl)amino)isoquinolin-4-y 1)pyridin-2(1H)-one as a white solid (yield: 7%).

LC-MS: $[M+H]^+$ = 465. H NMR (400 MHz, DMSO-d6) δ 8.45 (d, J = 8.3 Hz, 1H), 8.24 (s, 2H), 7.82 (s, 1H), 7.72 - 7.46 (m, 6H), 7.21 - 6.85 (m, 2H), 6.55 - 6.49 (m, 1H), 6.38 - 6.31 (m, 1H), 4.73 (q, J = 6.9 Hz, 1H), 4.39 (q, J = 7.0 Hz, 1H), 2.27 - 2.13 (m, 2H), 2.10 - 1.98 (m, 2H), 1.74 - 1.56 (m, 2H).

Examples E13 to E18

According to the aforementioned preparation methods, the following Compounds were prepared:

| | | | |
|---|---|---|---|
| E13 | 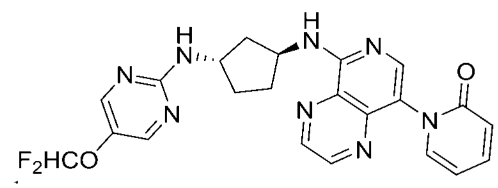 $^1$H NMR (400 MHz, DMSO-$d_6$) δ 9.02 (d, *J* = 2.0 Hz, 1H), 8.89 (d, *J* = 2.0 Hz, 1H), 8.26 (s, 2H), 8.18 (s, 1H), 8.14 (d, *J* = 7.7 Hz, 1H), 7.64 (dd, *J* = 6.8, 2.1 Hz, 1H), 7.60 - 7.51 (m, 2H), 7.05 (t, *J* = 74.0 Hz, 1H), 6.50 (d, *J* = 9.2 Hz, 1H), 6.32 (td, *J* = 6.7, 1.4 Hz, 1H), 4.78 (p, *J* = 7.5 Hz, 1H), 4.50 - 4.29 (m, 1H), 2.19 (ddd, *J* = 14.7, 9.5, 4.8 Hz, 2H), 2.14 - 2.08 (m, 1H), 2.07 - 1.97 (m, 1H), 1.84 - 1.70 (m, 1H), 1.63 (dt, *J* = 13.3, 7.8 Hz, 1H). | E14 | 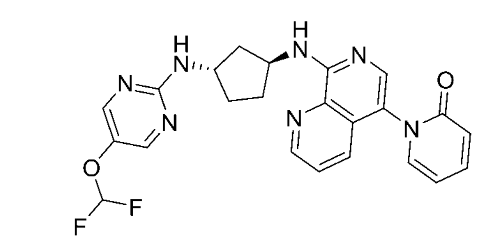 $^1$H NMR (400 MHz, DMSO-$d_6$) δ 8.90 - 8.81 (m, 1H), 8.26 (s, 2H), 8.03 (dd, *J* = 5.0, 1.9 Hz, 1H), 7.96 - 7.83 (m, 3H), 7.69 (dd, *J* = 8.4, 4.3 Hz, 1H), 7.56 (d, *J* = 7.2 Hz, 1H), 7.42 (d, *J* = 7.5 Hz, 1H), 7.26 - 6.85 (m, 3H), 4.69 (h, *J* = 7.2 Hz, 1H), 4.39 (h, *J* = 6.8 Hz, 1H), 2.30 - 2.14 (m, 2H), 2.05 (dq, *J* = 16.4, 6.4 Hz, 2H), 1.80 - 1.67 (m, 1H), 1.62 (dq, *J* = 15.3, 7.8 Hz, 1H). |

(continued)

| | | | |
|---|---|---|---|
| E15 | 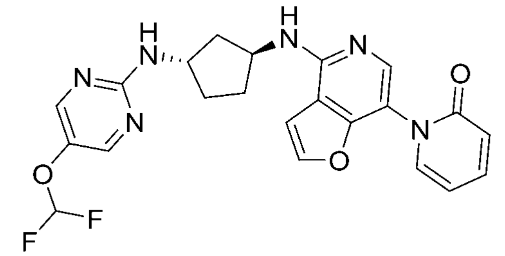 $^1$H NMR (400 MHz, DMSO-$d_6$) δ 8.26 (s, 2H), 7.86 (d, *J* = 17.4 Hz, 2H), 7.69 (dd, *J* = 6.8, 2.0 Hz, 1H), 7.57 (ddd, *J* = 8.7, 6.7, 2.1 Hz, 2H), 7.41 - 6.84 (m, 3H), 6.51 (d, *J* = 9.3 Hz, 1H), 6.34 (t, *J* = 6.7 Hz, 1H), 4.62 (d, *J* = 7.2 Hz, 1H), 4.38 (q, *J* = 7.0 Hz, 1H), 2.29 - 2.11 (m, 2H), 2.00 (t, *J* = 7.0 Hz, 2H), 1.70 - 1.53 (m, 2H). | E16 | 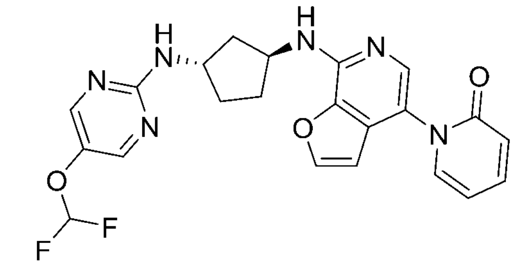 $^1$H NMR (400 MHz, DMSO-$d_6$) δ 8.25 (s, 2H), 8.11 (d, *J* = 2.1 Hz, 1H), 7.76 (s, 1H), 7.69 (d, *J* = 6.8 Hz, 1H), 7.60 - 7.47 (m, 2H), 7.31 - 6.83 (m, 2H), 6.64 (d, *J* = 2.1 Hz, 1H), 6.52 (d, *J* = 9.2 Hz, 1H), 6.33 (t, *J* = 6.7 Hz, 1H), 4.65 (p, *J* = 7.1 Hz, 1H), 4.37 (q, *J* = 6.9 Hz, 1H), 2.17 (dh, *J* = 12.5, 6.3 Hz, 2H), 2.00 (qt, *J* = 13.4, 6.9 Hz, 2H), 1.68 (dt, *J* = 13.8, 7.2 Hz, 1H), 1.63 - 1.52 (m, 1H). |
| E17 | 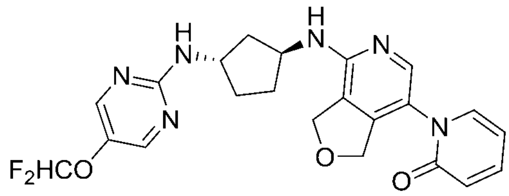 $^1$H NMR (400 MHz, DMSO-$d_6$) δ 8.25 (s, 2H), 8.11 (dd, *J* = 5.1, 2.0 Hz, 1H), 7.84 (ddd, *J* = 8.8, 7.1, 2.0 Hz, 1H), 7.78 (s, 1H), 7.47 (d, *J* = 7.2 Hz, 1H), 7.25 - 6.82 (m, 3H), 6.18 (d, *J* = 6.9 Hz, 1H), 4.91 (d, *J* = 2.7 Hz, 2H), 4.73 (t, *J* = 2.6 Hz, 2H), 4.46 (q, *J* = 6.8 Hz, 1H), 4.33 (q, *J* = 6.9 Hz, 1H), 2.14 (ddt, *J* = 16.1, 7.0, 4.7 Hz, 2H), 1.93 (td, *J* = 6.9, 1.5 Hz, 2H), 1.54 (ddd, *J* = 9.6, 6.9, 4.9 Hz, 2H). | E18 | 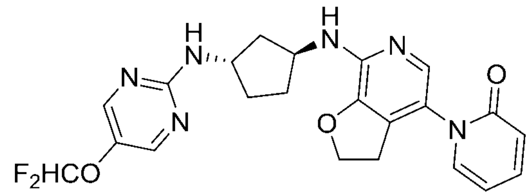 $^1$H NMR (400 MHz, DMSO-$d_6$) δ 8.23 (s, 2H), 7.61 (dd, *J* = 6.9, 2.1 Hz, 1H), 7.54 - 7.47 (m, 2H), 7.45 (d, *J* = 7.2 Hz, 1H), 7.03 (t, *J* = 74.0 Hz, 1H), 6.46 (d, *J* = 9.2 Hz, 1H), 6.28 (td, *J* = 6.7, 1.3 Hz, 1H), 6.17 (d, *J* = 7.3 Hz, 1H), 4.59 (t, *J* = 9.0 Hz, 2H), 4.48 (q, *J* = 7.0 Hz, 1H), 4.30 (q, *J* = 6.8 Hz, 1H), 3.01 (t, *J* = 8.9 Hz, 2H), 2.16 - 2.04 (m, 2H), 1.91 (p, *J* = 6.5 Hz, 2H), 1.63 - 1.47 (m, 2H). |

Example F1

Synthesis of 1-(5-(((1S,3S)-3-((5-(difluoromethoxy)pyrimidin-2-yl)amino)cyclopentyl)amino)pyrazin-2-yl)py ridin-2(1 H)-one

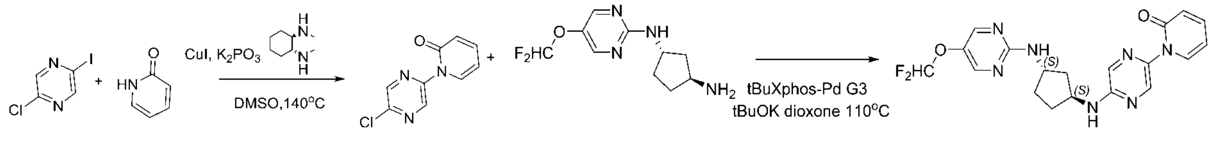


Step A: Synthesis of 1-(5-chloropyrazin-2-yl)pyridin-2(1H)-one

At room temperature, 2-chloro-5-iodopyrazine (500 mg, 2.09 mmol) was dissolved in DMSO (5 mL). Pyridin-2(1H)-one (218 mg, 2.3 mmol), (1R,2R)-$N^1$,$N^2$-dimethylcyclohexane-1,2-diamine (59 mg, 0.42 mmol), copper(I) iodide (159 mg, 0.84 mmol), and potassium phosphate (1.33 g, 6.27 mmol) were added. The reaction system was slowly heated to 140 C and reacted overnight under nitrogen protection.

After completion of the reaction, water (50 mL) was added. The mixture was extracted with ethyl acetate (25 mL × 3). The organic phases were combined and concentrated under reduced pressure. The residue was purified by silica gel column chromatography (EA/Hex = 30%) to obtain 179 mg of 1-(5-chloropyrazin-2-yl)pyridin-2(1H)-one as a yellow flocculent solid.

LC-MS: $[M+H]^+$ = 208. H NMR (400 MHz, DMSO-d6) δ 9.00 (d, J = 1.3 Hz, 1H), 8.89 (d, J = 1.3 Hz, 1H), 7.89 (ddd,

J = 7.1, 2.1, 0.8 Hz, 1H), 7.60 (ddd, J = 9.3, 6.5, 2.1 Hz, 1H), 6.57 (dt, J = 9.3, 1.0 Hz, 1H), 6.46 (td, J = 6.8, 1.3 Hz, 1H).

Step B: Synthesis of 1-(5-(((1S,3S)-3-((5-(difluoromethoxy)pyrimidin-2-yl)amino)cyclopentyl)amino)pyrazin-2-yl)py ridin-2(1H)-one

At room temperature, 1-(5-chloropyrazin-2-yl)pyridin-2(1H)-one (179 mg, 0.95 mmol) was dissolved in 1,4-dioxane (5 Ml). (1S,3S)-$N^1$-(5-(difluoromethoxy)pyrimidin-2-yl)cyclopentane-1,3-diamine (222 mg, 0.79 mmol), tBuXPhos Pd-G3 (125 mg, 0.16 mmol), and potassium tert-butoxide (176.9 mg, 1.58 mmol) were added. The reaction system was heated to 110 °C and reacted overnight under nitrogen protection.

After completion of the reaction, silica gel powder was added for filtration. The mixture was washed several times with EA. The filtrate was concentrated under reduced pressure. The residue was purified by reverse-phase column chromatography to obtain 104 mg of 1-(5-(((1S,3S)-3-((5-(difluoromethoxy)pyrimidin-2-yl)amino)cyclopentyl)amino)pyrazin-2-yl)py ridin-2(1H)-one as a white solid.

LC-MS: $[M+H]^+$ = 416. H NMR (400 MHz, DMSO-d6) $\delta$ 8.24 (s, 3H), 7.86 (s, 1H), 7.72 (d, J = 6.9 Hz, 1H), 7.54 - 7.50 (m, 3H), 7.04 (t, J = 74.0 Hz, 1H), 6.47 (d, J = 9.2 Hz, 1H), 6.33 (t, J = 6.8 Hz, 1H), 4.35 - 4.30 (m, 2H), 2.14 (td, J = 13.8, 7.0 Hz, 2H), 1.93 (tq, J = 14.1, 7.1 Hz, 2H), 1.55 (tq, J = 13.5, 7.8, 6.2 Hz, 2H).

Example F2

Synthesis of 1-(6-(((1S,3S)-3-((5-(difluoromethoxy)pyrimidin-2-yl)amino)cyclopentyl)amino)-1,2,4-triazin-3 -yl)pyridin-2(1H)-one

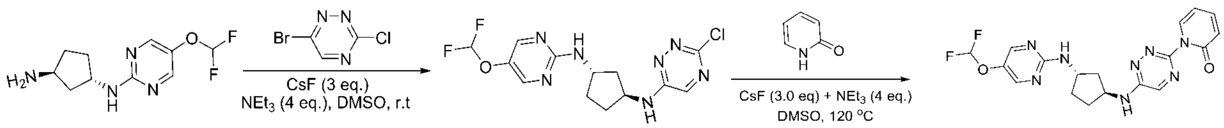


Step A: Synthesis of (1S,3S)-$N^1$-(3-chloro-1,2,4-triazin-6-yl)-$N^3$-(5-(difluoromethoxy)pyrimidin-2-yl)cyclopentane-1, 3-diamine

At room temperature under nitrogen protection, (1S,3S)-$N^1$-(5-(difluoromethoxy)pyrimidin-2-yl)cyclopentane-1,3-diamine (0.488 g, 2 mmol, 1.0 eq), 6-bromo-3-chloro-1,2,4-triazine (0.464 g, 2.4 mmol, 1.2 eq), cesium fluoride (0.912 g, 6 mmol, 3.0 eq), and triethylamine (0.816 g, 8 mmol, 4.0 eq) were dissolved in dimethyl sulfoxide (8 mL). The reaction was reacted overnight.

After completion of the reaction, ethyl acetate (75 mL) was added to dilute the reaction mixture. Solid impurities were filtered off. Saturated brine (50 mL) was added for extraction. The separated aqueous phase was extracted again with ethyl acetate (50 mL). The organic phases were combined, dried and concentrated under vacuum to dryness. The resulting residue was purified by silica gel column chromatography (eluent: ethyl acetate/n-hexane = 3/1) to obtain 0.675 g of (1S,3S)-$N^1$-(3-chloro-1,2,4-triazin-6-yl)-$N^3$-(5-(difluoromethoxy)pyrimidin-2-yl)cyclopentane-1, 3-diamine as a yellow solid (yield: 90%). LC-MS: $[M+H]^+$ = 358.

Step B: Synthesis of 1-(6-(((1S,3S)-3-((5-(difluoromethoxy)pyrimidin-2-yl)amino)cyclopentyl)amino)-1,2,4-triazin-3 -yl) pyridin-2(1H)-one

At room temperature under nitrogen protection, (1S,3S)-$N^1$-(3-chloro-1,2,4-triazin-6-yl)-$N^3$-(5-(difluoromethoxy)pyrimidin-2-yl)cyclopentane-1, 3-diamine (0.40 g, 1.12 mmol, 1.0 eq), pyridin-2(1H)-one (0.162 g, 1.68 mmol, 1.5 eq), cesium fluoride (0.51 g, 3.36 mmol, 3.0 eq), and triethylamine (0.457 g, 4.48 mmol, 4.0 eq) were dissolved in dimethyl sulfoxide (5 mL). The mixture was heated in an oil bath to 120 °C and reacted overnight.

After completion of the reaction, ethyl acetate (75 mL) was added to dilute the reaction mixture. Solid impurities were filtered off. Saturated brine (50 mL) was added for extraction. The separated aqueous phase was extracted again with ethyl acetate (50 mL). The organic phases were combined, dried and concentrated under vacuum to dryness. The resulting residue was purified by silica gel column chromatography (eluent: ethyl acetate) to obtain 0.139 g of 1-(6-(((1S,3S)-3-((5-(difluoromethoxy)pyrimidin-2-yl)amino)cyclopentyl)amino)-1,2,4-triazin-3 -yl)pyridin-2(1H)-one.

H NMR (400 MHz, DMSO-d6) $\delta$ 8.60 (s, 1H), 8.24 (s, 3H), 7.86 - 7.79 (m, 1H), 7.58 (ddd, J = 8.9, 6.6, 2.1 Hz, 1H), 7.52 (d, J = 7.1 Hz, 1H), 7.03 (t, J = 74.0 Hz, 1H), 6.52 (dt, J = 9.3, 1.1 Hz, 1H), 6.41 (td, J = 6.7, 1.3 Hz, 1H), 4.51 - 4.29 (m, 2H), 2.20 - 2.08 (m, 2H), 1.96 (t, J = 6.8 Hz, 2H), 1.66 - 1.53 (m, 2H).

Example F3

Synthesis of 3-cyclopropyl-1-(2-(((1S,3S)-3-((5-(difluoromethoxy)pyrimidin-2-yl)amino)cyclopentyl)amino) pyrimidin-5-yl)pyridin-2(1H)-one

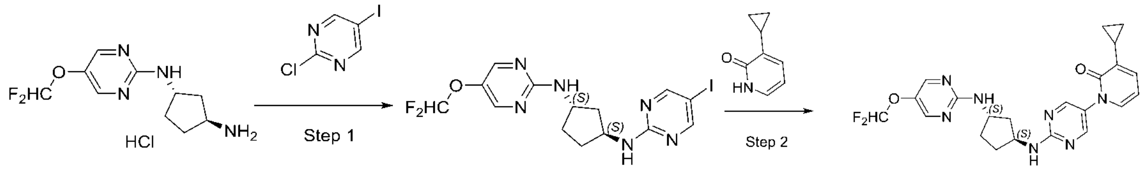


Step 1 : Synthesis of (1S,3S)-$N^1$-(5-(difluoromethoxy)pyrimidin-2-yl)-$N^3$-(5-iodopyrimidin-2-yl)cyclopentane-1,3-dia mine

At 25 °C, (1S,3S)-$N^1$-(5-(difluoromethoxy)pyrimidin-2-yl)cyclopentane-1,3-diamine hydrochloride (0.4 g, 1.1 mmol), 2-chloro-5-iodopyrimidine (0.405 g, 1.68 mmol), and potassium carbonate (0.775 g, 5.61 mmol) were dissolved in DMF (10 mL). After addition, the mixture was heated to 110 °C and reacted overnight.

Water (50 mL) was added. Then the mixture was extracted with EA (50 mL * 3). The organic phases were combined, washed with saturated brine (50 mL) and concentrated to obtain the crude product. The crude product was purified by column chromatography (DCM:MeOH = 20:1) to obtain (1S,3S)-$N^1$-(5-(difluoromethoxy)pyrimidin-2-yl)-$N^3$-(5-iodopyrimidin-2-yl)cyclopentane-1,3-dia mine as a yellow solid (0.497 g, 100% yield). LC-MS: [M+H]+ = 449.

Step 2: Synthesis of 3-cyclopropyl-1-(2-(((1S,3S)-3-((5-(difluoromethoxy)pyrimidin-2-yl)amino)cyclopentyl)amino) pyrimidin-5-yl)pyridin-2(1H)-one

At 20 °C, (1S,3S)-$N^1$-(5-(difluoromethoxy)pyrimidin-2-yl)-$N^3$-(5-iodopyrimidin-2-yl)cyclopentane-1,3-dia mine (0.3 g, 0.668 mmol), 3-cyclopropylpyridin-2(1H)-one (181.3 mg, 1.33 mmol), and potassium phosphate (282.6 mg, 1.33 mmol) were dissolved in DMSO (20 mL). Cuprous iodide (39.9 mg, 0.20 mmol) and (1R,2R)-(-)-N,N'-dimethylcyclohexane-1,2-diamine (18.8 mg, 0.133 mmol) were added. The atmosphere was purged with nitrogen three times. Under a nitrogen atmosphere, the mixture was heated to 130 °C and stirred overnight.

Water (50 mL) was added. Then the mixture was extracted with EA (30 mL * 4). The organic phases were combined, washed with saturated brine (30 mL) and concentrated to obtain the crude product. The crude product was purified by column chromatography (DCM:MeOH = 20:1) to afford3-cyclopropyl-1-(2-(((1S,3S)-3-((5-(difluoromethoxy) pyrimidin-2-yl)amino)cyclopentyl)a mino)pyrimidin-5-yl)pyridin-2(1H)-one.

H NMR (400 MHz, DMSO-d6): δ 8.32 (s, 2H), 8.24 (s, 2H), 7.64 (d, J = 7.2 Hz, 1H), 7.53 - 7.46 (m, 2H), 7.23 - 6.85 (m, 2H), 6.22 (t, J = 6.8 Hz, 1H), 4.34 (dq, J = 24.4, 6.6 Hz, 2H), 2.12 (d, J = 3.3 Hz, 2H), 2.02 (s, 1H), 1.92 (t, J = 7.0 Hz, 2H), 1.63 - 1.49 (m, 2H), 0.86 (dt, J = 8.4, 3.0 Hz, 2H), 0.65 (dt, J = 5.4, 2.9 Hz, 2H).

Example F4

Synthesis of 3-cyclopropyl-1-(5-(((1S,3S)-3-((5-(difluoromethoxy)pyrimidin-2-yl)amino)cyclopentyl)amino) pyrazin-2-yl)pyridin-2(1H)-one

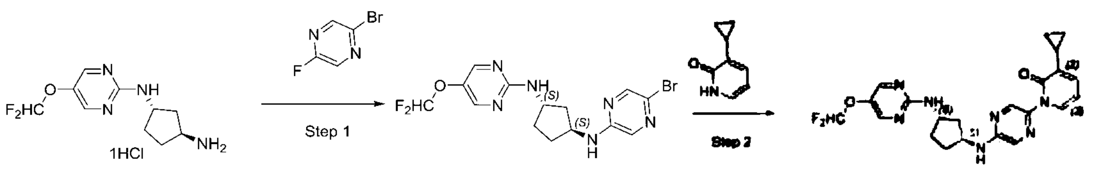


Step 1: Synthesis of (1S,3S)-$N^1$-(5-bromopyrazin-2-yl)-$N^3$-(5-(difluoromethoxy)pyrimidin-2-yl)cyclopentane-1,3-dia mine

At 25 °C, (1S,3S)-$N^1$-(5-(difluoromethoxy)pyrimidin-2-yl)cyclopentane-1,3-diamine hydrochloride (0.4 g, 1.1

mmol), 2-bromo-5-fluoropyrazine (0.238 g, 1.35 mmol), and TEA (1.123 g, 11.1 mmol) were dissolved in DMSO (10 mL). After addition, the mixture was heated to 110 °C and reacted for 3 hours.

Water (50 mL) was added. Then the mixture was extracted with EA (50 mL * 4). The organic phases were combined, washed with saturated brine (50 mL) and concentrated to obtain the crude product. The crude product was purified by column chromatography (DCM:MeOH = 20:1) to obtain (1S,3S)-$N^1$-(5-bromopyrazin-2-yl)-$N^3$-(5-(difluoromethoxy)pyrimidin-2-yl)cyclopentane-1,3-dia mine as a yellow solid (0.44 g, 100% yield). LC-MS: [M+H]+ = 401.

Step 2: Synthesis of 3-cyclopropyl-1-(5-(((1S,3S)-3-((5-(difluoromethoxy)pyrimidin-2-yl)amino)cyclopentyl)amino) pyrazin-2-yl)pyridin-2(1H)-one

At 20 C, (1S,3S)-$N^1$-(5-bromopyrazin-2-yl)-$N^3$-(5-(difluoromethoxy)pyrimidin-2-yl)cyclopentane-1,3-dia mine (0.5 g, 1.24 mmol), 3-cyclopropylpyridin-2(1H)-one (339 mg, 2.49 mmol), and potassium phosphate (530 mg, 2.49 mmol) were dissolved in DMSO (20 mL). Cuprous iodide (70.84 mg, 0.372 mmol) and (1R,2R)-(-)-N,N'-dimethylcyclohexane-1,2-diamine (35.41 mg, 0.249 mmol) were added. The atmosphere was purged with nitrogen three times. Under a nitrogen atmosphere, the mixture was heated to 130 °C and stirred for 10 hours.

The mixture was filtered under vacuum and washed with EA (50 mL). Water (50 mL) was added to the filtrate. Then the mixture was extracted with EA (30 mL * 4). The organic phases were combined, washed with saturated brine (50 mL) and concentrated to obtain the crude product. The crude product was purified by column chromatography (DCM:MeOH = 20:1) to obtain 3-cyclopropyl-1-(5-(((1S,3S)-3-((5-(difluoromethoxy)pyrimidin-2-yl)amino)cyclopentyl) amino) pyrazin-2-yl)pyridin-2(1H)-one.

H NMR (400 MHz, DMSO-d6): δ 8.24 (d, J = 2.0 Hz, 3H), 7.85 (d, J = 1.4 Hz, 1H), 7.57 - 7.44 (m, 3H), 7.24 - 6.85 (m, 2H), 6.23 (t, J = 6.9 Hz, 1H), 4.33 (p, J = 6.7 Hz, 2H), 2.24 - 2.08 (m, 2H), 2.06 - 1.97 (m, 1H), 1.97 - 1.85 (m, 2H), 1.54 (tt, J = 13.1, 6.9 Hz, 2H), 0.90 - 0.82 (m, 2H), 0.69 - 0.61 (m, 2H).

Example F5

Synthesis of 1-(2-(((1S,3S)-3-((5-(difluoromethoxy)pyrimidin-2-yl)amino)cyclopentyl)amino)pyrimidin-5-yl) pyridin-2(1H)-one

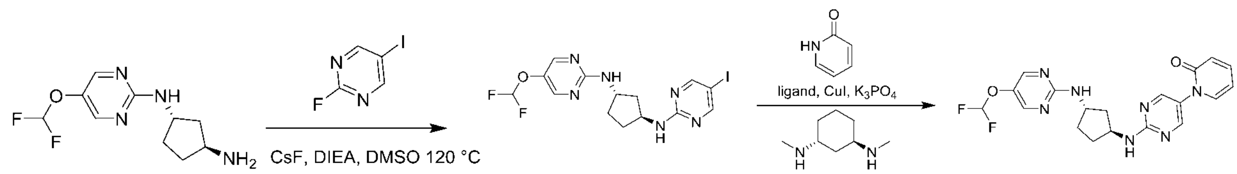


Step A: Synthesis of (1S,3S)-$N^1$-(5-(difluoromethoxy)pyrimidin-2-yl)-$N^3$-(5-iodopyrimidin-2-yl)cyclopentane-1,3-dia mine

At room temperature, (1S,3S)-$N^1$-(5-(difluoromethoxy)pyrimidin-2-yl)cyclopentane-1,3-diamine hydrochloride (0.5 g, 2.05 mmol, 1.0 eq), 2-fluoro-5-iodopyrimidine (0.5 g, 2.05 mmol, 1.0 eq), cesium fluoride (0.8 g, 5.12 mmol, 2.5 eq), and N-ethyl-N-isopropylpropan-2-amine (0.6 g, 5.12 mmol, 2.5 eq) were mixed in dimethyl sulfoxide (10 mL). Then the mixture was reacted at 120 °C for 6 hours.

After completion of the reaction, the mixture was cooled to room temperature and filtered. The filtrate was directly purified by reverse-phase column chromatography (C18, 330 g silica gel, eluent: water/acetonitrile = 5/13, flow rate: 80 mL/min) to obtain 0.66 g of (1S,3S)-$N^1$-(5-(difluoromethoxy)pyrimidin-2-yl)-$N^3$-(5-iodopyrimidin-2-yl)cyclopentane-1,3-dia mine (yield: 72%) as a gray solid. LC-MS: [M+H]+ = 449.

Step B: Synthesis of 1-(2-(((1S,3S)-3-((5-(difluoromethoxy)pyrimidin-2-yl)amino)cyclopentyl)amino)pyrimidin-5-yl) pyridin-2(1H)-one

At room temperature, (1S,3S)-$N^1$-(5-(difluoromethoxy)pyrimidin-2-yl)-$N^3$-(5-iodopyrimidin-2-yl)cyclopentane-1,3-dia mine (0.5 g, 1.12 mmol, 1.0 eq), pyridin-2(1H)-one (0.212 g, 2.23 mmol, 2.0 eq), (1R,3R)-$N^1$,$N^3$-dimethylcyclohexane-1,3-diamine (16 mg, 112 μmol, 0.1 eq), potassium phosphate (0.47 g, 2.23 mmol, 2.0 eq), and copper(I) iodide (42 mg, 223 μmol, 0.2 eq) were added to dimethyl sulfoxide (6 mL). After nitrogen exchange for three times, the solution was reacted at 130 °C for 16 hours.

After completion of the reaction, the mixture was cooled to room temperature. Cold water (60 mL) was added to quench the reaction. The mixture was extracted with ethyl acetate (50 mL × 3). The organic phases were combined, dried over anhydrous sodium sulfate, filtered, and concentrated. The resulting residue was purified by reverse-phase column chromatography (C18, 330 g silica gel, eluent: water/acetonitrile = 5/13, flow rate: 80 mL/min) to obtain 70 mg of 1-(2-(((1S,3S)-3-((5-(difluoromethoxy)pyrimidin-2-yl)amino)cyclopentyl)amino)pyrimidin-5-yl) pyridin-2(1H)-one.

H NMR (400 MHz, DMSO-d6): δ 8.32 (s, 2H), 8.24 (s, 2H), 7.67 (dd, J = 6.9, 2.3 Hz, 1H), 7.51 (ddd, J = 8.7, 6.6, 2.0 Hz, 1H), 7.04 (t, J = 74.0 Hz, 1H), 6.55 - 6.44 (m, 1H), 6.32 (td, J = 6.7, 1.4 Hz, 1H), 4.34 (ddt, J = 30.5, 13.4, 6.7 Hz, 2H), 2.18 - 2.05 (m, 2H), 1.97 - 1.81 (m, 2H), 1.55 (ddp, J = 13.0, 9.0, 4.0 Hz, 2H).

Example F6

Synthesis of 3-cyclopropyl-1-(6-(((1S,3S)-3-((5-(difluoromethoxy)pyrimidin-2-yl)amino)cyclopentyl)amino) pyridazin-3-yl)pyridin-2(1H)-one

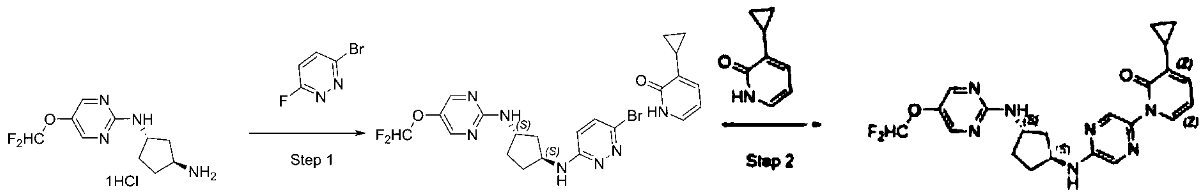


Step 1: Synthesis of (1S,3S)-$N^1$-(6-bromopyridazin-3-yl)-$N^3$-(5-(difluoromethoxy)pyrimidin-2-yl)cyclopentane-1,3-d iamine

At 25 °C, (1S,3S)-$N^1$-(5-(difluoromethoxy)pyrimidin-2-yl)cyclopentane-1,3-diamine hydrochloride (0.4 g, 1.1 mmol), 3-bromo-6-fluoropyridazine (0.238 g, 1.35 mmol), and TEA (1.123 g, 11.1 mmol) were dissolved in DMSO (10 mL). After addition, the mixture was heated to 110 °C and reacted for 3 hours.

Water (50 mL) was added. Then the mixture was extracted with EA (50 mL * 4). The organic phases were combined, washed with saturated brine (50 mL) and concentrated to obtain the crude product. The crude product was purified by column chromatography (DCM:MeOH = 20:1) to obtain (1S,3S)-$N^1$-(6-bromopyridazin-3-yl)-$N^3$-(5-(difluoromethoxy)pyrimidin-2-yl)cyclopentane-1,3-d iamine (0.44 g, 90.9% yield) as a yellow solid. LC-MS: [M+H]+ = 401.

Step 2: Synthesis of 3-cyclopropyl-1-(6-(((1S,3S)-3-((5-(difluoromethoxy)pyrimidin-2-yl)amino)cyclopentyl)amino) pyridazin-3-yl)pyridin-2(1H)-one

At 20 °C, (1S,3S)-$N^1$-(6-bromopyridazin-3-yl)-$N^3$-(5-(difluoromethoxy)pyrimidin-2-yl)cyclopentane-1,3-d iamine (0.3 g, 0.75 mmol), 3-cyclopropylpyridin-2(1H)-one (181.3 mg, 1.5 mmol), and potassium phosphate (282.6 mg, 1.5 mmol) were dissolved in DMSO (20 mL). Cuprous iodide (39.9 mg, 0.225 mmol) and (1R,2R)-(-)-N,N'-dimethylcyclohexane-1,2-diamine (18.88 mg, 0.15 mmol) were added. The atmosphere was purged with nitrogen three times. Under a nitrogen atmosphere, the mixture was heated to 130 °C and reacted overnight.

The mixture was filtered under vacuum and washed with EA (50 mL). Water (50 mL) was added to the filtrate. Then the mixture was extracted with EA (30 mL * 4). The organic phases were combined, washed with saturated brine (50 mL) and concentrated to obtain the crude product. The crude product was purified by column chromatography (DCM:MeOH = 20:1) to obtain 3-cyclopropyl-1-(6-(((1S,3S)-3-((5-(difluoromethoxy)pyrimidin-2-yl)amino)cyclopentyl) amino) pyridazin-3-yl)pyridin-2(1H)-one.

H NMR (400 MHz, DMSO-d6): δ 8.25 (s, 2H), 7.65 - 7.48 (m, 3H), 7.28 (d, J = 6.7 Hz, 1H), 7.25 - 6.84 (m, 3H), 6.26 (t, J = 6.9 Hz, 1H), 4.48 - 4.37 (m, 1H), 4.34 (d, J = 6.9 Hz, 1H), 2.16 (ddt, J = 27.6, 15.8, 7.5 Hz, 2H), 2.07 - 1.85 (m, 3H), 1.57 (dq, J = 10.0, 6.3, 5.3 Hz, 2H), 0.93 - 0.82 (m, 2H), 0.71 - 0.63 (m, 2H).

Example F7

Synthesis of 1-(6-(((1S,3S)-3-((5-(difluoromethoxy)pyrimidin-2-yl)amino)cyclopentyl)amino)pyridazin-3-yl) pyridin-2(1H)-one

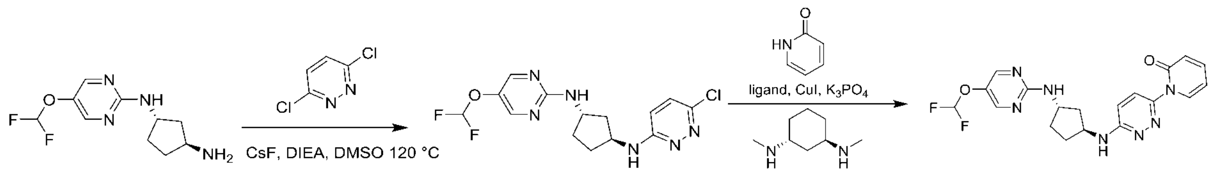


Step A: Synthesis of (1S,3S)-$N^1$-(6-chloropyridazin-3-yl)-$N^3$-(5-(difluoromethoxy)pyrimidin-2-yl)cyclopentane-1,3-d iamine

At room temperature, (1S,3S)-$N^1$-(5-(difluoromethoxy)pyrimidin-2-yl)cyclopentane-1,3-diamine hydrochloride (0.5 g, 2.05 mmol, 1.0 eq), 2,6-dichloropyridazine (0.335 g, 2.25 mmol, 1.0 eq), cesium fluoride (0.8 g, 5.12 mmol, 2.5 eq), and N-ethyl-N-isopropylpropan-2-amine (0.6 g, 5.12 mmol, 2.5 eq) were mixed in dimethyl sulfoxide (10 mL). Then the mixture was reacted at 120 C for 6 hours.

After completion of the reaction, the mixture was cooled to room temperature and filtered. The filtrate was directly purified by reverse-phase column chromatography (C18, 330 g silica gel, eluent: water/acetonitrile = 5/13, flow rate: 80 mL/min) to obtain 0.55 g of (1S,3S)-$N^1$-(6-chloropyridazin-3-yl)-$N^3$-(5-(difluoromethoxy)pyrimidin-2-yl)cyclopentane-1,3-d iamine as a gray solid (yield: 75%). LC-MS: [M+H]+ = 449.

Step B: Synthesis of 1-(6-(((1S,3S)-3-((5-(difluoromethoxy)pyrimidin-2-yl)amino)cyclopentyl)amino)pyridazin-3-yl) pyridin-2(1H)-one

At room temperature, (1S,3S)-$N^1$-(6-chloropyridazin-3-yl)-$N^3$-(5-(difluoromethoxy)pyrimidin-2-yl)cyclopentane-1,3-d iamine (0.4 g, 1.12 mmol, 1.0 eq), pyridin-2(1H)-one (0.32 g, 3.36 mmol, 3.0 eq), (1R,3R)-$N^1$,$N^3$-dimethylcyclohexane-1,3-diamine (16 mg, 112 μmol, 0.1 eq), potassium phosphate (0.714 g, 3.36 mmol, 3.0 eq), and copper(I) iodide (107 mg, 561 μmol, 0.5 eq) were added to dimethyl sulfoxide (6 mL). After nitrogen exchange for three times, the solution was was reacted at 130 C for 16 hours.

After completion of the reaction, the mixture was cooled to room temperature. Cold water (60 mL) was added to quench the reaction. The mixture was extracted with ethyl acetate (50 mL × 3). The organic phases were combined, dried over anhydrous sodium sulfate, filtered, and concentrated. The resulting residue was purified by reverse-phase column chromatography (C18, 330 g silica gel, eluent: water/acetonitrile = 5/13, flow rate: 80 mL/min) to obtain 10 mg of 1-(6-(((1S,3S)-3-((5-(difluoromethoxy)pyrimidin-2-yl)amino)cyclopentyl)amino)pyridazin-3-yl) pyridin-2(1H)-one.

$^1H$ NMR (400 MHz, DMSO-$d_6$): δ 8.24 (s, 2H), 7.80 (dd, J = 7.0, 2.1 Hz, 1H), 7.54 (ddd, J = 13.1, 10.2, 7.9 Hz, 3H), 7.31 (d, J = 6.7 Hz, 1H), 7.13 (d, J = 74.0 Hz, 1H), 6.96 - 6.83 (m, 1H), 6.49 (d, J = 9.2 Hz, 1H), 6.36 (td, J = 6.7, 1.3 Hz, 1H), 4.37 (dq, J = 35.5, 6.8 Hz, 2H), 2.17 (ddt, J = 25.2, 12.5, 7.4 Hz, 2H), 2.00 - 1.86 (m, 2H), 1.64 - 1.51 (m, 2H).

Example F8

Synthesis of 3-(6-(((1S,3S)-3-((5-(difluoromethoxy)pyrimidin-2-yl)amino)cyclopentyl)amino)pyridin-3-yl)pi peridine-2,6-dione (Compound F8), and (R)-3-(6-(((1S,3S)-3-((5-(difluoromethoxy)pyrimidin-2-yl)amino)cyclopentyl)amino)pyridin-3-y 1)-1-methylpiperidine-2,6-dione (Compound F8A) and (S)-3-(6-(((1S,3S)-3-((5-(difluoromethoxy)pyrimidin-2-yl) amino)cyclopentyl)amino)pyridin-3-y 1)-1-methylpiperidine-2,6-dione (Compound F8B)

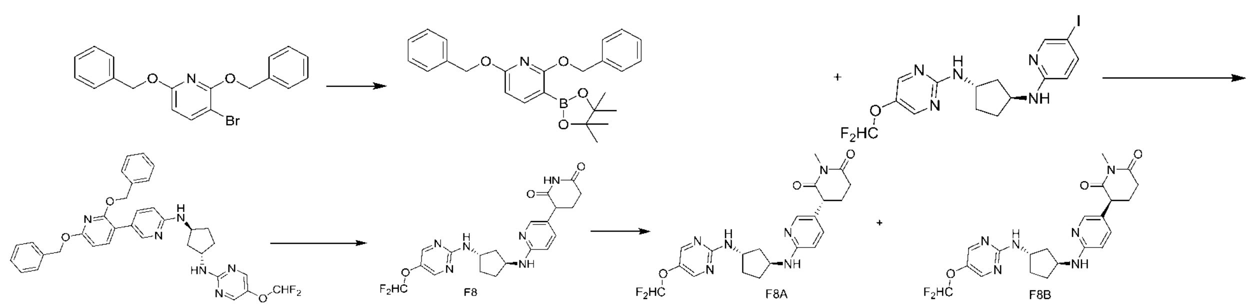

F8 F8A F8B

Step A: Synthesis of 2,6-bis(benzyloxy)-3-(4,4,5,5-tetramethyl-1,3,2-dioxaborolan-2-yl)pyridine

At room temperature, 2,6-bis(benzyloxy)-3-bromopyridine (1.50 g, 4.05 mmol) was dissolved in 1,4-dioxane (20 mL). Bis(pinacolato)diboron (1.54 g, 6.08 mmol), cesium acetate (2.33 g, 12.2 mmol), and [1,1'-bis(diphenylphosphino) ferrocene]dichloropalladium(II) (300 mg, 0.41 mmol) were added at room temperature. The mixture was stirred at 100 C for 12 hours under nitrogen protection.

After completion of the reaction, silica gel was added to the reaction mixture and concentrated. The resulting crude product was purified by normal phase column chromatography (n-hexane:ethyl acetate 100:0 to 70:30) to obtain 880 mg of 2,6-bis(benzyloxy)-3-(4,4,5,5-tetramethyl-1,3,2-dioxaborolan-2-yl)pyridine as a yellow solid (yield: 52%). LC-MS: [M+H]+ = 418.

Step B: Synthesis of (1S,3S)-$N^1$-(2,6-bis(benzyloxy)-[3,3'-bipyridin]-6-yl)-$N^3$-(5-(difluoromethoxy)pyrimidin-2-yl)cy clopentane-1,3-diamine

At room temperature, 2,6-bis(benzyloxy)-3-(4,4,5,5-tetramethyl-1,3,2-dioxaborolan-2-yl)pyridine (880 mg, 2.11 mmol), (1S,3S)-$N^1$-(5-iodopyridin-2-yl)-$N^3$-(5-(difluoromethoxy)pyrimidin-2-yl)cyclopentane-1,3-diami ne (800 mg, 1.79 mmol), potassium carbonate (740 mg, 5.37 mmol), and [1,1'-bis(diphenylphosphino)ferrocene]dichloropalladium(II) (140 mg, 0.18 mmol) were added to a mixture of 1,4-dioxane (20 mL) and water (10 mL). The mixture was stirred at 100 °C for 12 hours under nitrogen protection.

After completion of the reaction, silica gel was added to the reaction mixture and dried and concentrated. The resulting crude product was purified by normal phase column chromatography (n-hexane:ethyl acetate 100:0 to 0:100) to obtain 900 mg of (1S,3S)-$N^1$-(2,6-bis(benzyloxy)-[3,3'-bipyridin]-6-yl)-$N^3$-(5-(difluoromethoxy)pyrimidin-2-yl)cy clopentane-1,3-diamine as a yellow solid (yield: 90%). LC-MS: [M+H]+ = 611.

Step C: Synthesis of 3-(6-(((1S,3S)-3-((5-(difluoromethoxy)pyrimidin-2-yl)amino)cyclopentyl)amino)pyridin-3-yl)pi peridine-2,6-dione

At room temperature, (1S,3S)-$N^1$-(2,6-bis(benzyloxy)-[3,3'-bipyridin]-6-yl)-$N^3$-(5-(difluoromethoxy)pyrimidin-2-yl)cy clopentane-1,3-diamine (900 mg, 1.47 mmol) was added to methanol (20 mL). Wet palladium on carbon (500 mg, 0.70 mmol) was added at room temperature, the mixture was stirred at 50 °C for 16 hours under hydrogen protection.

After completion of the reaction, the reaction mixture was filtered directly. The filter cake was washed with methanol. The filtrate was dried and concentrated. The resulting residue was purified by reverse-phase column chromatography (acetonitrile:water 5:95 to 60:40) to obtain 325 mg of 3-(6-(((1S,3S)-3-((5-(difluoromethoxy)pyrimidin-2-yl)amino)cyclopentyl)amino)pyridin-3-yl)pi peridine-2,6-dione as a white solid (yield: 51%). LC-MS: [M-H]+ = 431.

NMR: H NMR (400 MHz, DMSO-d6) δ 10.80 (s, 1H), 8.23 (s, 2H), 7.79 (d, J = 2.4 Hz, 1H), 7.48 (d, J = 7.2 Hz, 1H), 7.21 (dd, J = 8.8, 2.3 Hz, 1H), 7.04 (s, 1H), 6.52 (d, J = 6.8 Hz, 1H), 6.42 (d, J = 8.6 Hz, 1H), 4.27 (ddt, J = 22.4, 12.8, 6.6 Hz, 2H), 3.65 (dd, J = 12.0, 4.9 Hz, 1H), 2.67 (ddd, J = 17.3, 12.1, 5.3 Hz, 1H), 2.47 (d, J = 4.0 Hz, 1H), 2.12 (dddd, J = 19.1, 13.8, 10.0, 3.8 Hz, 3H), 2.00 - 1.91 (m, 1H), 1.91 - 1.76 (m, 2H), 1.50 (ddt, J = 18.8, 13.2, 8.6 Hz, 2H).

Step D: Synthesis of (R)-3-(6-(((1S,3S)-3-((5-(difluoromethoxy)pyrimidin-2-yl)amino)cyclopentyl)amino)pyridin-3-y 1)-1-methylpiperidine-2,6-dione, and (S)-3-(6-(((1S,3S)-3-((5-(difluoromethoxy)pyrimidin-2-yl)amino)cyclopentyl)amino)pyridin-3-y 1)-1-methylpiperidine-2,6-dione

At room temperature, 3-(6-(((1S,3S)-3-((5-(difluoromethoxy)pyrimidin-2-yl)amino)cyclopentyl)amino)pyridin-3-yl)pi peridine-2,6-dione (200 mg, 0.462 mmol) was added to N,N-dimethylformamide (10 mL). Then iodomethane (132 mg, 0.942 mmol) and potassium carbonate (192 mg, 1.39 mmol) were added. The reaction was reacted at room temperature for 2 hours.

After completion of the reaction, the reaction mixture was filtered. The filter cake was washed with N,N-dimethylformamide. The filtrate was dried and concentrated to obtain the crude product. The crude product was purified by reverse-phase column chromatography (acetonitrile:water 5:95 to 65:35) to obtain a mixture. The mixture was separated by Prep-HPLC using a CHIRALCEL OJ-H column; 10 mm * 250 mm * 5 μm, mobile phase: n-hexane/ethanol = 10:90, flow rate: 3.5 mL/min, column temperature: 35 °C. Two compounds with retention times of 18.8 minutes and 29.8 minutes were obtained: (R)-3-(6-(((1S,3S)-3-((5-(difluoromethoxy)pyrimidin-2-yl)amino)cyclopentyl)amino)pyridin-3-y 1)-1-methylpiperidine-2,6-dione and (S)-3-(6-(((1S,3S)-3-((5-(difluoromethoxy)pyrimidin-2-yl)amino)cyclopentyl)amino)pyridin-3-y 1)-1-methylpiperidine-2,6-dione.

Compound with retention time of 18.8 minutes: H NMR (400 MHz, DMSO-d6) δ 8.23 (s, 2H), 7.79 (d, J = 2.4 Hz, 1H), 7.47 (d, J = 7.2 Hz, 1H), 7.25 - 7.18 (m, 1H), 7.04 (s, 1H), 6.47 (dd, J = 37.9, 7.8 Hz, 2H), 4.34 - 4.20 (m, 2H), 3.75 (dd, J = 11.9, 4.9 Hz, 1H), 3.02 (s, 3H), 2.77 (ddd, J = 17.2, 12.0, 5.3 Hz, 1H), 2.70 - 2.54 (m, 1H), 2.21 - 2.06 (m, 3H), 2.00 - 1.91 (m,

1H), 1.50 (ddt, J = 18.9, 12.9, 8.6 Hz, 2H).

Examples F9 to F37

According to the aforementioned Compound preparation methods, Compounds F9-F37 were prepared:

| No. | Compound Structure | No. | Compound Structure |
|---|---|---|---|
| F9 | 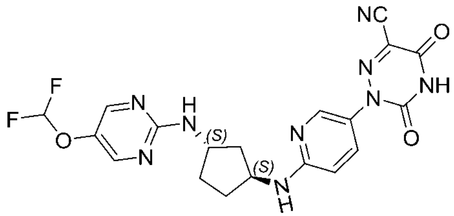 $^1$H NMR (400 MHz, DMSO-$d_6$) δ 8.24 - 8.22 (s, 2H), 8.00 - 7.97 (d, *J* = 2.7 Hz, 1H), 7.49 - 7.44 (d, *J* = 7.2 Hz, 1H), 7.42 - 7.38 (dd, *J* = 8.9, 2.7 Hz, 1H), 7.29 - 7.17 (m, 1H), 7.06 - 6.82 (m, 2H), 6.52 - 6.46 (d, *J* = 9.0 Hz, 1H), 4.35 - 4.24 (d, *J* = 7.6 Hz, 2H), 2.20 - 2.07 (m, 2H), 2.05 - 1.95 (m, 2H), 1.93 - 1.81 (dt, *J* = 16.7, 6.9 Hz, 2H). | F10 | 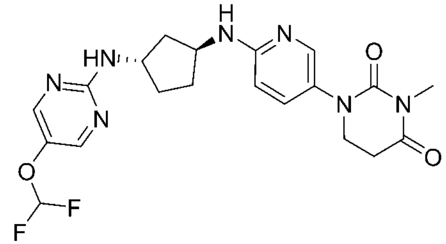 $^1$H NMR (400 MHz, DMSO-$d_6$) δ 8.20 (s, 2H), 7.88 (d, *J* = 2.7 Hz, 1H), 7.43 (d, *J* = 7.2 Hz, 1H), 7.29 (dd, *J* = 8.9, 2.7 Hz, 1H), 7.00 (t, *J* = 74.0 Hz, 1H), 6.65 (d, *J* = 6.9 Hz, 1H), 6.44 (d, *J* = 8.8 Hz, 1H), 4.25 (dq, *J* = 14.0, 6.8 Hz, 2H), 3.61 (t, *J* = 6.7 Hz, 2H), 3.00 (s, 3H), 2.77 (t, *J* = 6.7 Hz, 2H), 2.17 - 1.99 (m, 2H), 1.96 - 1.72 (m, 2H), 1.47 (dddd, *J* = 18.5, 12.4, 5.8, 2.6 Hz, 2H). |
| F11 | 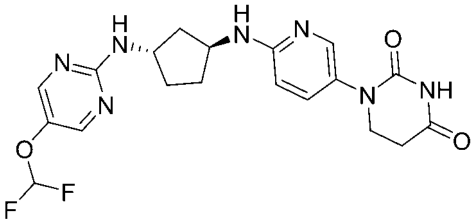 $^1$H NMR (400 MHz, DMSO-$d_6$) δ 10.30 (s, 1H), 8.23 (s, 2H), 7.90 (d, *J* = 2.6 Hz, 1H), 7.47 (d, *J* = 7.2 Hz, 1H), 7.32 (dd, *J* = 8.9, 2.7 Hz, 1H), 7.03 (t, *J* = 74.0 Hz, 2H), 6.67 (d, *J* = 6.9 Hz, 1H), 6.46 (d, *J* = 8.9 Hz, 1H), 4.28 (dq, *J* = 19.2, 6.7 Hz, 2H), 3.66 (t, *J* = 6.7 Hz, 2H), 2.68 (t, *J* = 6.7 Hz, 2H), 2.21 - 2.02 (m, 2H), 1.86 (tdd, *J* = 13.2, 10.2, 6.2 Hz, 2H), 1.49 (ddt, *J* = 18.8, 13.1, 7.0 Hz, 2H). | F12 | 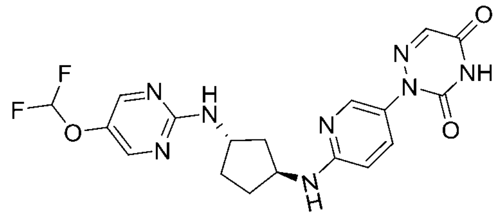 $^1$H NMR (400 MHz, DMSO-$d_6$) δ 12.83 - 12.31 (s, 1H), 8.25 - 8.21 (s, 2H), 7.85 - 7.82 (d, *J* = 2.5 Hz, 1H), 7.59 - 7.56 (s, 1H), 7.52 - 7.47 (d, *J* = 7.2 Hz, 1H), 7.30 - 7.25 (dd, *J* = 8.9, 2.6 Hz, 1H), 7.22 - 7.02 (d, *J* = 74.0 Hz, 1H), 6.93 - 6.83 (m, 1H), 6.53 - 6.48 (d, *J* = 8.9 Hz, 1H), 4.36 - 4.24 (m, 2H), 2.18 - 2.05 (m, 2H), 1.93 - 1.83 (m, 2H), 1.61 - 1.43 (m, 2H). |

(continued)

| No. | Compound Structure | No. | Compound Structure |
|---|---|---|---|
| F13 | 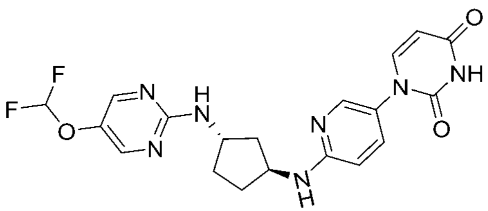 $^1H$ NMR (400 MHz, DMSO-$d_6$) δ 11.43 - 11.37 (s, 1H), 8.26 - 8.23 (s, 2H), 7.97 - 7.94 (d, *J* = 2.7 Hz, 1H), 7.67 - 7.62 (d, *J* = 7.9 Hz, 1H), 7.52 - 7.46 (d, *J* = 7.1 Hz, 1H), 7.43 - 7.38 (dd, *J* = 8.9, 2.8 Hz, 1H), 6.97 - 6.85 (m, 1H), 6.56 - 6.47 (d, *J* = 8.9 Hz, 1H), 5.66 - 5.60 (d, *J* = 7.8 Hz, 1H), 4.37 - 4.24 (m, 2H), 2.20 - 2.06 (d, *J* = 13.2 Hz, 2H), 1.95 - 1.83 (q, *J* = 7.3 Hz, 2H), 1.62 - 1.42 (m, 2H). | F14 | 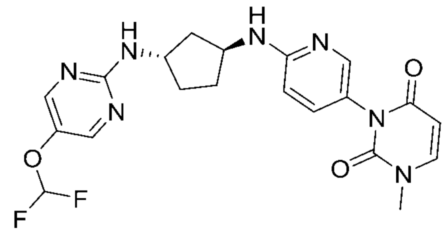 $^1H$ NMR (400 MHz, DMSO-$d_6$) δ 8.25 (s, 2H), 7.82 - 7.68 (m, 2H), 7.50 (d, *J* = 7.2 Hz, 1H), 7.27 - 6.85 (m, 2H), 6.82 (d, *J* = 6.8 Hz, 1H), 6.52 (d, *J* = 8.8 Hz, 1H), 5.76 (d, *J* = 7.8 Hz, 1H), 4.32 (dt, *J* = 11.0, 6.7 Hz, 2H), 3.32 (s, 3H), 2.14 (ddt, *J* = 15.4, 8.2, 5.3 Hz, 2H), 1.91 (p, *J* = 6.3 Hz, 2H), 1.53 (ddd, *J* = 18.7, 13.4, 7.0 Hz, 2H). |
| F15 | 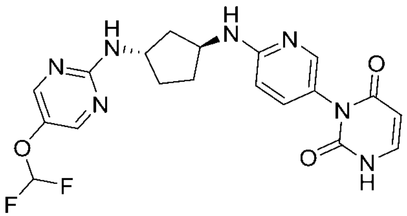 $^1H$ NMR (400 MHz, DMSO-$d_6$) δ 8.25 (s, 2H), 7.75 (d, *J* = 2.6 Hz, 1H), 7.51 (t, *J* = 8.0 Hz, 2H), 7.28 - 6.85 (m, 2H), 6.81 (d, *J* = 6.8 Hz, 1H), 6.51 (d, *J* = 8.8 Hz, 1H), 5.68 (d, *J* = 7.6 Hz, 1H), 4.31 (dq, *J* = 12.5, 6.5 Hz, 2H), 2.14 (dd, *J* = 13.1, 8.6 Hz, 2H), 1.90 (q, *J* = 6.3 Hz, 2H), 1.61 - 1.45 (m, 2H). | F16 | 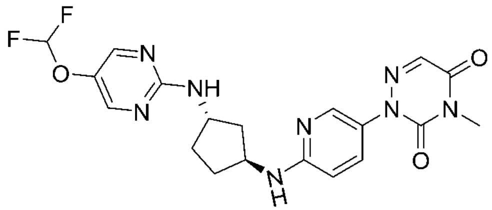 $^1H$ NMR (400 MHz, DMSO-$d_6$) δ 8.26 - 8.21 (s, 2H), 7.84 - 7.80 (d, *J* = 2.5 Hz, 1H), 7.65 - 7.61 (s, 1H), 7.50 - 7.45 (d, *J* = 7.2 Hz, 1H), 7.27 - 7.20 (dd, *J* = 10.9, 8.3 Hz, 1H), 7.05 - 6.83 (m, 1H), 6.54 - 6.49 (d, *J* = 8.9 Hz, 1H), 4.37 - 4.23 (q, *J* = 7.0 Hz, 2H), 3.53 - 3.52 (s, 3H), 2.19 - 2.06 (t, *J* = 11.0 Hz, 2H), 1.94 - 1.82 (q, *J* = 6.5 Hz, 2H), 1.59 - 1.42 (t, *J* = 17.0 Hz, 2H). |

(continued)

| No. | Compound Structure | No. | Compound Structure |
|---|---|---|---|
| F17 | 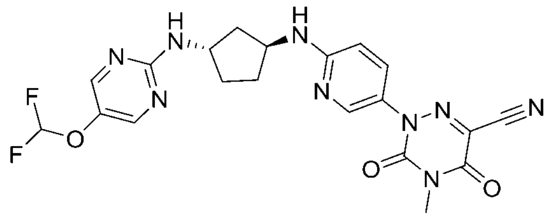<br>$^1$H NMR (400 MHz, DMSO-$d_6$) δ 8.26 - 8.24 (s, 2H), 8.05 - 8.02 (d, $J$ = 2.6 Hz, 1H), 7.52 - 7.43 (m, 2H), 7.26 - 6.83 (t, $J$ = 74.0 Hz, -1H), 6.60 - 6.55 (d, $J$ = 9.0 Hz, 1H), 4.37 - 4.28 (q, $J$ = 6.8 Hz, 2H), 3.24 - 3.23 (s, 3H), 2.20 - 2.10 (ddd, $J$ = 18.3, 10.2, 5.1 Hz, 2H), 1.96 - 1.85 (m, 2H), 1.61 - 1.43 (td, $J$ = 19.0, 18.5, 6.9 Hz, 2H). | F18 | 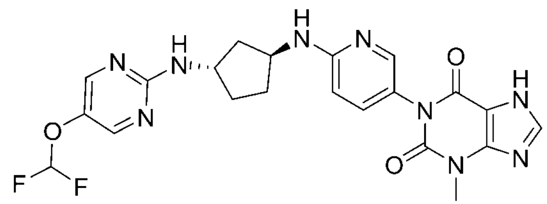<br>$^1$H NMR: (400 MHz, DMSO-d6) δ 8.24 (s, 2H), 7.93 - 7.83 (m, 1H), 7.75 (d, $J$ = 2.4 Hz, 1H), 7.48 (d, $J$ = 7.3 Hz, 1H), 7.25 - 6.83 (m, 2H), 6.75 (d, $J$ = 6.9 Hz, 1H), 6.51 (d, $J$ = 8.8 Hz, 1H), 4.38 - 4.25 (m, 2H), 3.44 (s, 3H), 2.19 - 2.08 (m, 2H), 1.97 - 1.85 (m, 2H), 1.60 - 1.46 (m, 2H). |
| F19 | 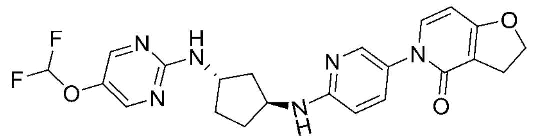<br>$^1$H NMR (400 MHz, DMSO-$d_6$) δ 8.23 (s, 2H), 7.87 (d, $J$ = 2.6 Hz, 1H), 7.52 (dd, $J$ = 17.5, 7.3 Hz, 2H), 7.34 (dd, $J$ = 8.9, 2.7 Hz, 1H), 7.22 - 6.85 (m, 2H), 6.50 (d, $J$ = 8.8 Hz, 1H), 6.12 (d, $J$ = 7.3 Hz, 1H), 4.65 (t, $J$ = 9.1 Hz, 2H), 4.30 (ddt, $J$ = 9.8, 6.6, 3.4 Hz, 2H), 2.94 (t, $J$ = 9.1 Hz, 2H), 2.16 - 2.07 (m, 2H), 1.87 (tdd, $J$ = 13.2, 10.4, 6.3 Hz, 2H), 1.58 - 1.44 (m, 2H). | F20 | 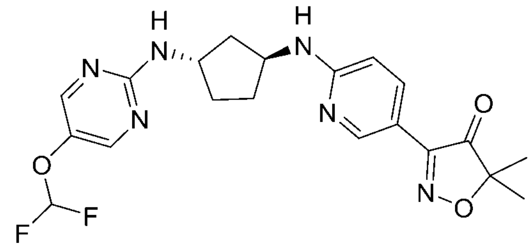<br>$^1$H NMR (400 MHz, DMSO-d6) δ 8.60 - 8.45 (m, 1H), 8.27 - 8.22 (m, 2H), 8.08 (br d, $J$ = 8.0 Hz, 1H), 7.52 (br d, $J$ = 2.0 Hz, 1H), 7.27 - 6.79 (m, 2H), 4.32 (br d, $J$ = 1.6 Hz, 2H), 2.31 - 2.10 (m, 2H), 2.06 - 1.86 (m, 2H), 1.66 - 1.51 (m, 2H), 1.46 - 1.31 (m, 6H) |
| F21 | 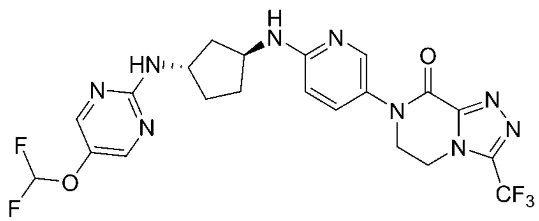<br>$^1$H NMR: (400 MHz, DMSO-d6) δ 8.33 - 8.15 (m, 2H), 8.01 (d, $J$ = 2.6 Hz, 1H), 7.49 (d, $J$ = 7.3 Hz, 1H), 7.41 (dd, $J$ = 2.6, 8.9 Hz, 1H), 7.26 - 6.85 (m, 1H), 6.83 (d, $J$ = 6.8 Hz, 1H), 6.53 (d, $J$ = 8.9 Hz, 1H), 4.59 (t, $J$ = 5.9 Hz, 2H), 4.30 (td, $J$ = 7.1, 14.6 Hz, 2H), 4.21 - 4.06 (m, 2H), 2.21 - 1.98 (m, 2H), 1.98 - 1.80 (m, 2H), 1.61 - 1.38 (m, 2H) | F22 | 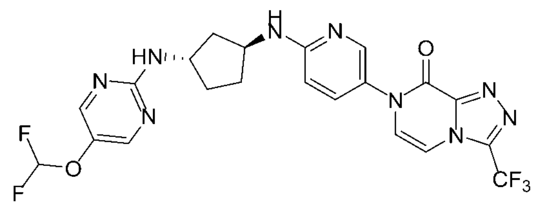<br>$^1$H NMR (400 MHz, DMSO-d6) δ 8.29 - 8.22 (m, 2H), 8.01 (d, $J$ = 2.5 Hz, 1H), 7.65 (d, $J$ = 5.9 Hz, 1H), 7.50 (d, $J$ = 7.1 Hz, 1H), 7.45 (dd, $J$ = 2.5, 8.9 Hz, 1H), 7.36 (d, $J$ = 5.9 Hz, 1H), 7.24 - 6.85 (m, 2H), 6.58 (d, $J$ = 9.0 Hz, 1H), 4.41 - 4.25 (m, 2H), 2.23 - 2.07 (m, 2H), 1.97 - 1.83 (m, 2H), 1.60 - 1.47 (m, 2H) |

(continued)

| No. | Compound Structure | No. | Compound Structure |
| --- | --- | --- | --- |
| F23 | 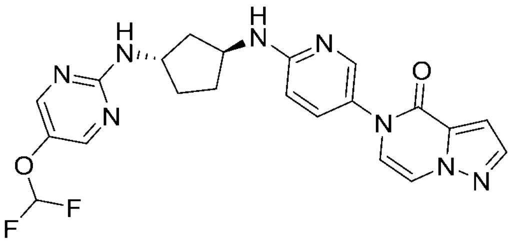 $^1$H NMR (400 MHz, DMSO-$d_6$) δ 8.24 (s, 2H), 8.02 (d, $J$ = 2.7 Hz, 1H), 7.96 (d, $J$ = 2.2 Hz, 1H), 7.82 (d, $J$ = 6.0 Hz, 1H), 7.55 - 7.42 (m, 2H), 7.25 - 6.81 (m, 4H), 6.56 (d, $J$ = 8.9 Hz, 1H), 4.32 (h, $J$ = 6.7 Hz, 2H), 2.13 (dddd, J = 19.6, 12.5, 7.4, 3.9 Hz, 2H), 1.98 - 1.84 (m, 2H), 1.63 - 1.43 (m, 2H). | F24 | 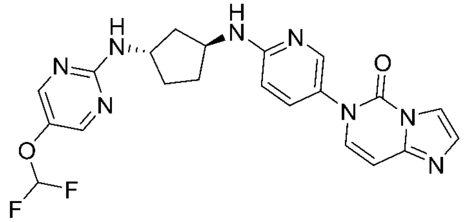 $^1$H NMR (400 MHz, DMSO-$d_6$) δ 8.24 (s, 2H), 8.06 (d, $J$ = 2.7 Hz, 1H), 7.83 (d, $J$ = 1.6 Hz, 1H), 7.55 - 7.47 (m, 2H), 7.47 - 7.38 (m, 2H), 7.25 - 6.81 (m, 2H), 6.72 (d, $J$ = 7.7 Hz, 1H), 6.56 (d, $J$ = 8.9 Hz, 1H), 4.33 (p, $J$ = 6.8 Hz, 2H), 2.21 - 2.09 (m, 2H), 1.90 (tdd, $J$ = 13.2, 10.4, 6.3 Hz, 2H), 1.62 - 1.44 (m, 2H). |
| F25 | 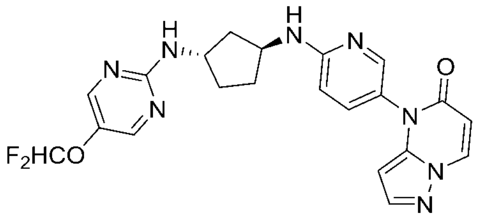 $^1$H NMR (400 MHz, DMSO-$d_6$) δ 8.58 (d, $J$ = 7.9 Hz, 1H), 8.24 (s, 2H), 7.99 (d, $J$ = 2.6 Hz, 1H), 7.74 (d, $J$ = 2.0 Hz, 1H), 7.49 (d, $J$ = 7.2 Hz, 1H), 7.42 (dd, $J$ = 8.9, 2.7 Hz, 1H), 7.28 - 6.82 (m, 2H), 6.59 (d, $J$ = 8.9 Hz, 1H), 6.13 (d, $J$ = 7.9 Hz, 1H), 5.53 (d, $J$ = 2.0 Hz, 1H), 4.32 (h, $J$ = 6.9 Hz, 2H), 2.27 - 2.03 (m, 2H), 1.90 (q, $J$ = 6.4 Hz, 2H), 1.53 (ddd, $J$ = 15.2, 12.5, 7.1 Hz, 2H). | F26 | 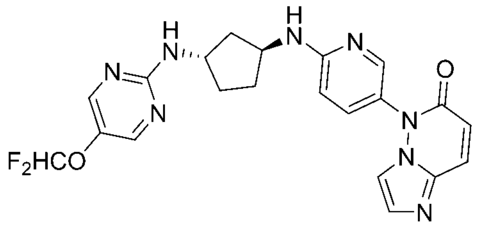 $^1$H NMR (400 MHz, DMSO-$d_6$) δ 8.24 (s, 2H), 8.09 (d, $J$ = 2.6 Hz, 1H), 7.86 (d, $J$ = 10.1 Hz, 1H), 7.55 - 7.45 (m, 2H), 7.32 - 7.23 (m, 2H), 7.24 - 6.83 (m, 2H), 6.65 (d, $J$ = 2.4 Hz, 1H), 6.63 (d, $J$ = 3.5 Hz, 1H), 4.34 (dt, $J$ = 13.7, 6.7 Hz, 2H), 2.22 - 2.08 (m, 2H), 1.97 - 1.86 (m, 2H), 1.62 - 1.48 (m, 2H). |

(continued)

| No. | Compound Structure | No. | Compound Structure |
|---|---|---|---|
| F27 | 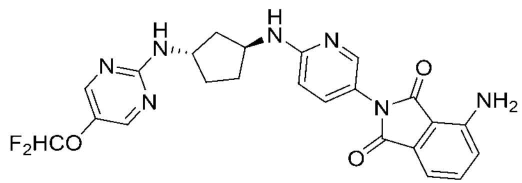 $^1$H NMR (400 MHz, DMSO-$d_6$) δ 8.24 (s, 2H), 7.92 (d, *J* = 2.6 Hz, 1H), 7.52 - 7.43 (m, 2H), 7.35 (dd, *J* = 8.9, 2.6 Hz, 1H), 7.23 - 6.82 (m, 4H), 6.57 - 6.45 (m, 3H), 4.32 (q, *J* = 6.3, 5.8 Hz, 2H), 2.21 - 2.04 (m, 2H), 1.95 - 1.85 (m, 2H), 1.52 (td, *J* = 12.7, 11.1, 6.8 Hz, 2H). | F28 | 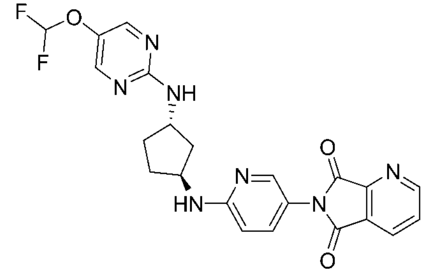 $^1$H NMR: (400 MHz, DMSO-d6) δ 9.02 (dd, *J* = 1.3, 4.9 Hz, 1H), 8.36 (dd, *J* = 1.3, 7.7 Hz, 1H), 8.24 (s, 2H), 7.98 (d, *J* = 2.5 Hz, 1H), 7.84 (dd, *J* = 5.0, 7.6 Hz, 1H), 7.48 (d, *J* = 7.3 Hz, 1H), 7.40 (dd, *J* = 2.5, 8.9 Hz, 1H), 7.25 - 6.82 (m, 2H), 6.57 (d, *J* = 8.9 Hz, 1H), 4.44 - 4.20 (m, 2H), 2.23 - 2.03 (m, 2H), 1.97 - 1.83 (m, 2H), 1.61 - 1.45 (m, 2H) |
| F29 | 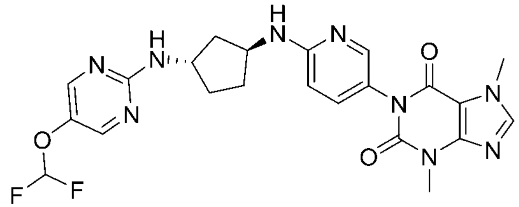 $^1$H NMR: δ 8.23 (s, 2H), 8.06 (s, 1H), 7.76 (d, *J* = 2.5 Hz, 1H), 7.48 (d, *J* = 7.3 Hz, 1H), 7.26 - 6.83 (m, 2H), 6.79 (d, *J* = 6.9 Hz, 1H), 6.51 (d, *J* = 8.9 Hz, 1H), 4.31 (td, *J* = 7.2, 14.4 Hz, 2H), 3.86 (s, 3H), 3.42 (s, 3H), 2.21 - 2.03 (m, 2H), 1.97 - 1.81 (m, 2H), 1.62 - 1.39 (m, 2H) | F30 | 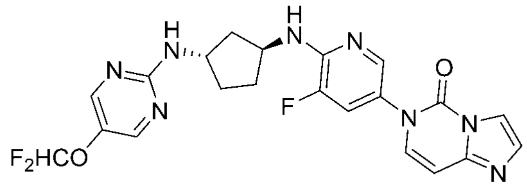 $^1$H NMR (400 MHz, DMSO-$d_6$) δ 8.23 (s, 2H), 8.00 (d, *J* = 2.1 Hz, 1H), 7.85 (d, *J* = 1.5 Hz, 1H), 7.66 (dd, *J* = 11.8, 2.2 Hz, 1H), 7.51 - 7.40 (m, 3H), 7.24 - 6.81 (m, 2H), 6.73 (d, *J* = 7.7 Hz, 1H), 4.52 (p, *J* = 7.0 Hz, 1H), 4.33 (h, *J* = 6.9 Hz, 1H), 2.21 - 2.07 (m, 2H), 2.03 - 1.89 (m, 2H), 1.68 - 1.51 (m, 2H). |

(continued)

| No. | Compound Structure | No. | Compound Structure |
|---|---|---|---|
| F31 | 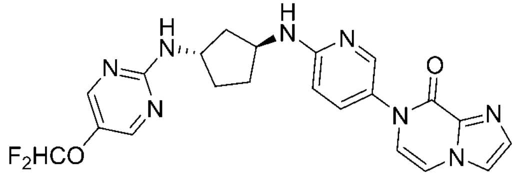 $^1H$ NMR (400 MHz, DMSO-$d_6$) δ 8.24 (s, 2H), 8.00 (d, $J$ = 2.7 Hz, 1H), 7.86 (s, 1H), 7.62 (d, $J$ = 5.9 Hz, 1H), 7.57 - 7.40 (m, 3H), 7.24 - 6.83 (m, 3H), 6.55 (d, $J$ = 8.9 Hz, 1H), 4.31 (p, $J$ = 6.8 Hz, 2H), 2.14 (tt, $J$ = 11.1, 6.6 Hz, 2H), 2.02 - 1.83 (m, 2H), 1.51 (ddt, $J$ = 22.3, 16.0, 8.1 Hz, 2H). | F32 | 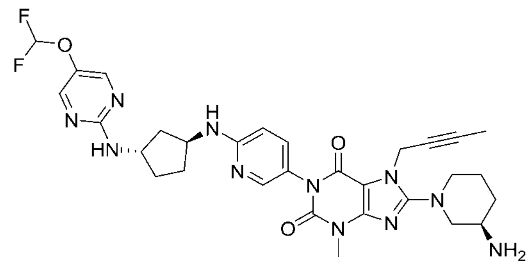 $^1H$ NMR (400 MHz, DMSO-d6) δ8.23 (s, 2H), 7.74 (d, $J$ = 2.5 Hz, 1H), 7.47 (d, $J$ = 7.3 Hz, 1H), 7.23 - 6.82 (m, 2H), 6.76 (d, $J$ = 6.8 Hz, 1H), 6.50 (d, $J$ = 8.8 Hz, 1H), 4.86 (br s, 2H), 4.38 - 4.22 (m, 2H), 3.67 - 3.53 (m, 2H), 3.02 - 2.93 (m, 1H), 2.86 - 2.77 (m, 1H), 2.77 - 2.69 (m, 1H), 2.19 - 2.07 (m, 2H), 1.96 - 1.72 (m, 8H), 1.68 - 1.43 (m, 4H), 1.28 - 1.16 (m, 1H). |
| F33 | 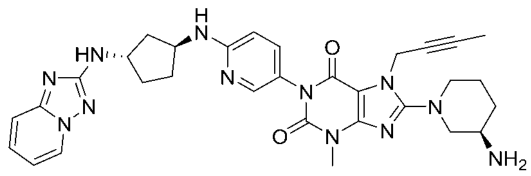 $^1H$ NMR, (400 MHz, DMSO-d6) δ8.58 (d, $J$ = 6.6 Hz, 1H), 7.74 (d, $J$ = 2.5 Hz, 1H), 7.45 - 7.34 (m, 2H), 7.19 (dd, $J$ = 2.6, 8.8 Hz, 1H), 6.85 (dt, $J$ = 1.6, 6.7 Hz, 1H), 6.76 (d, $J$ = 6.8 Hz, 1H), 6.62 (d, $J$ = 7.1 Hz, 1H), 6.51 (d, $J$ = 8.8 Hz, 1H), 4.87 (br s, 2H), 4.36 - 4.25 (m, 1H), 4.24 - 4.13 (m, 1H), 3.68 - 3.53 (m, 2H), 3.37 (s, 3H), 3.03 - 2.92 (m, 1H), 2.86 - 2.78 (m, 1H), 2.77 - 2.70 (m, 1H), 2.22 - 2.10 (m, 2H), 2.02 - 1.93 (m, 1H), 1.92 - 1.72 (m, 7H), 1.68 - 1.44 (m, 4H), 1.29 - 1.15 (m, 1H). | F34 | 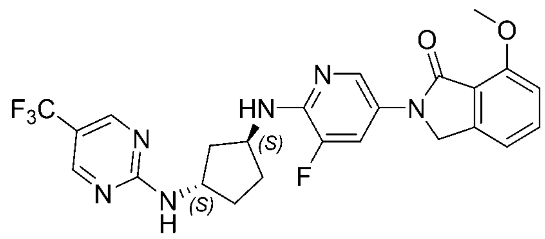 $^1H$ NMR (400 MHz, DMSO-$d_6$) δ 8.61 (d, $J$ = 16.6 Hz, 2H), 8.20 - 8.16 (m, 2H), 7.96 (dd, $J$ = 13.2, 2.2 Hz, 1H), 7.59 (t, $J$ = 7.9 Hz, 1H), 7.15 (d, $J$ = 7.5 Hz, 1H), 7.07 (d, $J$ = 8.3 Hz, 1H), 6.57 (d, $J$ = 7.0 Hz, 1H), 4.85 (s, 2H), 4.46 (dq, J = 17.8, 6.8 Hz, 2H), 3.88 (s, 3H), 2.14 (td, $J$ = 10.5, 4.5 Hz, 2H), 1.96 (t, $J$ = 7.0 Hz, 2H), 1.63 - 1.54 (m, 2H). |

(continued)

| No. | Compound Structure | No. | Compound Structure |
|---|---|---|---|
| F35 | 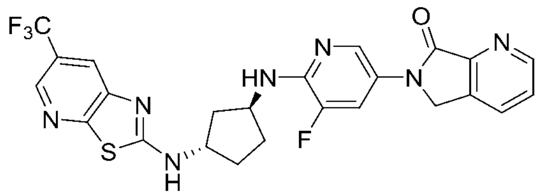<br>$^1$H NMR (400 MHz, DMSO-$d_6$) δ 8.77 (d, $J$ = 6.9 Hz, 1H), 8.70 (dd, $J$ = 4.7, 1.4 Hz, 1H), 8.38 (dd, $J$ = 2.0, 0.9 Hz, 1H), 8.19 (d, $J$ = 2.2 Hz, 1H), 8.06 (dd, $J$ = 7.8, 1.4 Hz, 1H), 7.98 (dd, $J$ = 13.0, 2.2 Hz, 1H), 7.91 (d, $J$ = 2.1 Hz, 1H), 7.57 (dd, $J$ = 7.7, 4.8 Hz, 1H), 6.72 - 6.68 (m, 1H), 4.90 (s, 2H), 4.52 - 4.33 (m, 2H), 2.14 (ddd, $J$ = 17.2, 12.2, 6.8 Hz, 2H), 1.99 (d, $J$ = 6.7 Hz, 2H), 1.56 (dt, $J$ = 12.3, 6.2 Hz, 2H). | F36 | 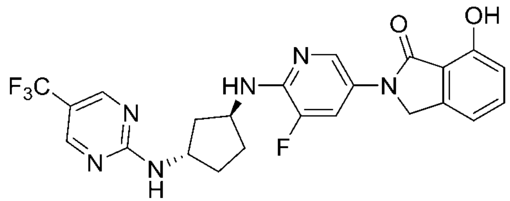<br>$^1$H NMR (400 MHz, DMSO-$d_6$) δ 8.54 (d, $J$ = 16.3 Hz, 2H), 8.11 (d, $J$ = 2.0 Hz, 2H), 7.90 (dd, $J$ = 13.2, 2.2 Hz, 1H), 7.37 (t, $J$ = 7.8 Hz, 1H), 6.95 (d, $J$ = 7.4 Hz, 1H), 6.79 (d, $J$ = 8.1 Hz, 1H), 6.52 (dd, $J$ = 7.0, 1.5 Hz, 1H), 4.78 (s, 2H), 4.38 (dt, $J$ = 17.9, 6.8 Hz, 2H), 2.08 (tt, $J$ = 6.2, 3.1 Hz, 2H), 1.89 (t, $J$ = 7.0 Hz, 2H), 1.53 (dd, $J$ = 11.8, 6.7 Hz, 2H). |
| F37 | 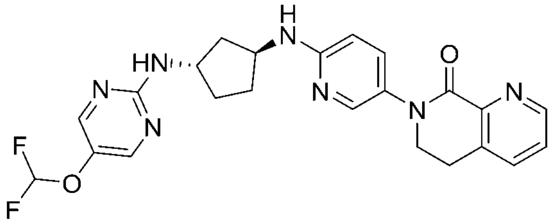 | $^1$H NMR (400 MHz, DMSO-$d_6$) δ (ppm) = 8.61 (br d, $J$ = 4.0 Hz, 1H), 8.23 (s, 2H), 7.99 (d, $J$ = 2.0 Hz, 1H), 7.82 (d, $J$ = 7.2 Hz, 1H), 7.55 - 7.45 (m, 2H), 7.40 (dd, $J$ = 2.4, 8.8 Hz, 1H), 7.03 (t, $J$ = 74.0 Hz, 1H), 6.69 (br d, $J$ = 6.4 Hz, 1H), 6.50 (d, $J$ = 8.8 Hz, 1H), 4.36 - 4.24 (m, 2H), 3.87 (t, $J$ = 6.4 Hz, 2H), 3.15 (t, $J$ = 6.4 Hz, 2H), 2.19 - 2.05 (m, 2H), 1.95 - 1.80 (m, 2H), 1.61 - 1.41 (m, 2H) | | |

Example G1

Synthesis of 3-((6-(((1S,3S)-3-((5-(difluoromethoxy)pyrimidin-2-yl)amino)cyclopentyl)amino)pyridin-3-yl)m ethyl)-1,2,4-oxadiazol-5(4H)-one

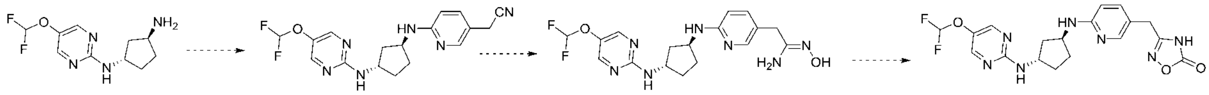

Step 1: Synthesis of 2-(6-(((1S,3S)-3-((5-(difluoromethoxy)pyrimidin-2-yl)amino)cyclopentyl)amino)pyridin-3-yl)ac etonitrile (2)

(1S,3S)-$N^1$-(5-(difluoromethoxy)pyrimidin-2-yl)cyclopentane-1,3-diamine (2 g, 8.19 mmol), 2-(6-bromopyridin-3-yl)acetonitrile (2.42 g, 12.29 mmol), potassium tert-butoxide (1.84 g, 16.38 mmol), and methanesulfonato(2-di-tert-butylphosphino-2',4',6'-triisopropyl-1,1'-biphenyl)(2'-amino-1,1'-bi phenyl-2-yl)palladium(II) (1.30 g, 1.64 mmol) were added sequentially to 1,4-dioxane (10 mL). The mixture was degassed three times and then reacted at 110 °C under nitrogen protection. MS monitoring showed product formation. The mixture was filtered through diatomaceous earth. The residue was purified by silica gel column chromatography and concentrated to obtain 2-(6-(((1S,3S)-3-((5-(difluoromethoxy)pyrimidin-2-yl)amino)cyclopentyl)amino)pyridin-3-yl)ac etonitrile as a light yellow viscous substance (0.2 g, yield: 7.79%). LC-MS: [M+H]+ = 361.0

Step 2: Synthesis of (Z)-2-(6-(((1S,3S)-3-((5-(difluoromethoxy)pyrimidin-2-yl)amino)cyclopentyl)amino)pyridin-3-y l)-N'-hydroxyacetimidamide (3)

2-(6-(((1S,3S)-3-((5-(Difluoromethoxy)pyrimidin-2-yl)amino)cyclopentyl)amino)pyridin-3-yl)acetonitrile (0.1 g, 0.28 mmol, Purity 100%), hydroxylamine hydrochloride (0.049 g, 0.70 mmol), and triethylamine (0.12 g, 0.84 mmol) were added sequentially to dry methanol (5 mL). The reaction was reacted at room temperature. MS monitoring showed product formation. The reaction mixture was concentrated to obtain (Z)-2-(6-(((1S,3S)-3-((5-(difluoromethoxy)pyrimidin-2-yl)amino)cyclopentyl)amino)pyridin-3-y l)-N'-hydroxyacetimidamide as a brown viscous substance (30 mg, yield: 27.48%). LC-MS: [M+H]+ = 394.2

Step 3: Synthesis of 3-((6-(((1S,3S)-3-((5-(difluoromethoxy)pyrimidin-2-yl)amino)cyclopentyl)amino)pyridin-3-yl)m ethyl)-1,2,4-oxadiazol-5(4H)-one

(Z)-2-(6-(((1S,3S)-3-((5-(Difluoromethoxy)pyrimidin-2-yl)amino)cyclopentyl)amino)pyridi n-3-yl)-N'-hydroxyacetimidamide (0.1 g, 0.25 mmol), CDI (0.061 g, 0.38 mmol), and DBU (0.042 g, 0.28 mmol) were added sequentially to 1,4-dioxane (10 mL). The mixture was reacted at 110 °C. MS monitoring showed product formation. The reaction mixture was concentrated. The residue was purified by Flash column chromatography and concentrated. The residue was further purified by reverse-phase column chromatography, concentrated, and lyophilized to obtain 3-((6-(((1S,3S)-3-((5-(difluoromethoxy)pyrimidin-2-yl)amino)cyclopentyl)amino)pyridin-3-yl)m ethyl)-1,2,4-oxadiazol-5(4H)-one as a white solid (20 mg, yield: 18.76%).

H NMR (400 MHz, DMSO-$d_6$) δ 8.23 (s, 2H), 7.83 (d, J = 2.3 Hz, 1H), 7.47 (d, J = 7.2 Hz, 1H), 7.26 (dd, J = 8.5, 2.3 Hz, 1H), 7.03 (s, 1H), 6.45 (d, J = 6.9 Hz, 1H), 6.39 (d, J = 8.5 Hz, 1H), 4.30 - 4.22 (m, 2H), 2.12 - 2.07 (m, 2H), 1.91 - 1.84 (m, 2H), 1.51 - 1.43 (m, 2H), 1.24 (d, J = 3.5 Hz, 2H).

Example G2

Synthesis of (1S,3S)-$N^1$-(4-((1,3,4-oxadiazol-2-yl)methyl)phenyl)-$N^3$-(5-(difluoromethoxy)pyrimidin-2-yl)cy clopentane-1,3-diamine

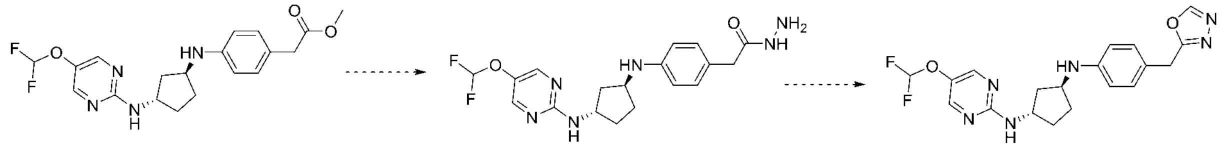

Step 1: Synthesis of 2-(4-(((1S,3S)-3-((5-(difluoromethoxy)pyrimidin-2-yl)amino)cyclopentyl)amino)phenyl)acetohy drazide (2)

At room temperature, methyl 2-(4-(((1S,3S)-3-((5-(difluoromethoxy)pyrimidin-2-yl)amino)cyclopentyl)amino) phenyl)acetate (0.3 g, 0.76 mmol) and hydrazine hydrate (0.046 g, 0.91 mmol) were added sequentially to methanol (5 mL). The mixture was reacted at 65 °C. After completion of the reaction, the reaction mixture was concentrated. Water (10 mL) was added to the residue. The mixture was extracted with dichloromethane (10 mL × 3). The organic phase was concentrated to obtain 2-(4-(((1S,3S)-3-((5-(difluoromethoxy)pyrimidin-2-yl)amino)cyclopentyl)amino)phenyl)acetohy drazide as a pale yellow solid (0.2 g, yield: 66.67%, Purity: 100%). LC-MS: [M+H]+ = 394.2

Step 2: Synthesis of (1S,3S)-$N^1$-(4-((1,3,4-oxadiazol-2-yl)methyl)phenyl)-$N^3$-(5-(difluoromethoxy)pyrimidin-2-yl)cy clopentane-1,3-diamine (SAL-0139-0705-1)

2-(4-(((1S,3S)-3-((5-(difluoromethoxy)pyrimidin-2-yl)amino)cyclopentyl)amino)phenyl)ac etohydrazide (0.1 g, 0.25 mmol) and p-toluenesulfonic acid (0.043 g, 0.25 mmol) were added to triethyl orthoformate (2 mL). The mixture was refluxed. MS monitoring showed product formation. The reaction mixture was purified by preparative HPLC, concentrated, and lyophilized to obtain (1S,3S)-$N^1$-(4-((1,3,4-oxadiazol-2-yl)methyl)phenyl)-$N^3$-(5-(difluoromethoxy)pyrimidin-2-yl)cy clopentane-1,3-diamine as a white solid (30 mg, yield: 29.26%).

LC-MS: [M+H]+ = 404.2. H NMR (400 MHz, DMSO-$d_6$) δ 9.13 (s, 1H), 8.24 (s, 2H), 7.93 (d, J = 2.4 Hz, 1H), 7.49 (d, J = 5.8 Hz, 1H), 7.31 (dd, J = 8.6, 2.4 Hz, 1H), 6.95 (d, J = 74.0 Hz, 1H), 6.60 (d, J = 6.9 Hz, 1H), 6.45 (d, J = 8.6 Hz, 1H), 4.31 - 4.25 (m, 2H), 4.09 (s, 2H), 2.11 (dq, J = 11.8, 4.6 Hz, 2H), 1.91 - 1.83 (m, 2H), 1.50 (ddd, J = 20.2, 9.6, 5.9 Hz, 2H).

Examples G3 to G15

According to the aforementioned Compound preparation methods, Compounds 3 to 15 were prepared:

| No. | Compound | No. | Compound |
|---|---|---|---|
| G3 | 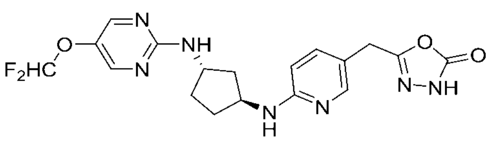<br>$^1$H NMR (400 MHz, DMSO-$d_6$) δ 8.23 (s, 2H), 7.87 (s, 1H), 7.46 (d, $J$ = 7.3 Hz, 1H), 7.27 (d, $J$ = 8.6 Hz, 1H), 7.03 (s, 1H), 6.58 (d, $J$ = 6.8 Hz, 1H), 6.44 (d, $J$ = 8.7 Hz, 1H), 4.27 (dq, $J$ = 14.2, 6.8 Hz, 2H), 3.71 (s, 2H), 2.09 (dt, $J$ = 13.8, 6.6 Hz, 2H), 1.83 (tt, $J$ = 13.1, 6.6 Hz, 2H), 1.48 (td, $J$ = 12.7, 6.2 Hz, 2H). | G6 | 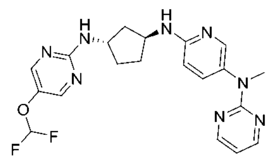<br>$^1$H NMR (400 MHz, DMSO-$d_6$) δ 8.32 (d, $J$ = 4.8 Hz, 2H), 8.24 (s, 2H), 7.89 (d, $J$ = 2.7 Hz, 1H), 7.48 (d, $J$ = 7.2 Hz, 1H), 7.30 (dd, $J$ = 8.8, 2.7 Hz, 1H), 7.04 (t, $J$ = 74.0 Hz, 1H), 6.67 (t, $J$ = 4.7 Hz, 1H), 6.60 (d, $J$ = 6.8 Hz, 1H), 6.48 (d, $J$ = 8.8 Hz, 1H), 4.42 - 4.19 (m, 2H), 3.37 (s, 3H), 2.12 (dq, $J$ = 12.1, 7.0 Hz, 2H), 1.95 - 1.81 (m, 2H), 1.60 - 1.42 (m, 2H). |

(continued)

| No. | Compound | No. | Compound |
|---|---|---|---|
| G5 | 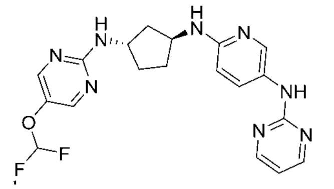<br>$^1$H NMR (400 MHz, DMSO-$d_6$) δ 9.18 (s, 1H), 8.38 (d, *J* = 4.7 Hz, 2H), 8.25 (s, 3H), 7.68 (d, *J* = 8.9 Hz, 1H), 7.47 (d, *J* = 7.1 Hz, 1H), 7.05 (t, *J* = 74.0 Hz, 1H), 6.74 (t, *J* = 4.7 Hz, 1H), 6.55 (s, 2H), 4.31 (q, *J* = 6.9 Hz, 1H), 4.22 (s, 1H), 2.24 - 2.04 (m, 2H), 1.89 (tdd, *J* = 13.2, 10.4, 6.1 Hz, 2H), 1.53 (dtd, *J* = 21.6, 13.0, 11.3, 6.1 Hz, 2H). | G8 | 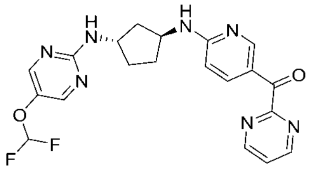<br>$^1$H NMR (400 MHz, DMSO-$d_6$) δ 8.99 (d, *J* = 4.9 Hz, 2H), 8.45 (d, *J* = 2.4 Hz, 1H), 8.25 (s, 2H), 7.88 (t, *J* = 8.7 Hz, 2H), 7.71 (t, *J* = 4.9 Hz, 1H), 7.52 (d, *J* = 7.2 Hz, 1H), 7.05 (t, *J* = 74.0 Hz, 1H), 6.58 (d, *J* = 9.0 Hz, 1H), 4.42 (d, *J* = 35.8 Hz, 1H), 4.33 (q, *J* = 6.9 Hz, 1H), 2.14 (dddd, J = 14.4, 9.7, 7.7, 4.1 Hz, 2H), 2.02 - 1.83 (m, 2H), 1.68 - 1.45 (m, 2H). |
| G7 | 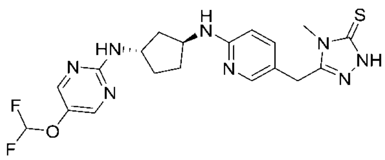<br>$^1$H NMR (400 MHz, DMSO-$d_6$) δ 13.54 (s, 1H), 8.24 (s, 2H), 7.88 (d, *J* = 2.4 Hz, 1H), 7.47 (d, *J* = 7.2 Hz, 1H), 7.24 - 7.22 (m, 1H), 7.21 - 6.84 (m, 1H), 6.58 (d, *J* = 6.9 Hz, 1H), 6.44 (d, *J* = 8.6 Hz, 1H), 4.28 (dt, *J* = 17.2, 7.0 Hz, 2H), 3.89 (s, 2H), 2.11 (ddd, *J* = 11.5, 7.8, 4.5 Hz, 2H), 1.89 - 1.81 (m, 2H), 1.56 - 1.45 (m, 2H). | G10 | 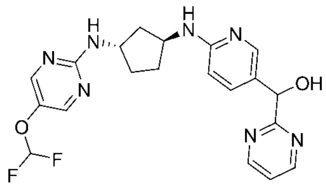<br>$^1$H NMR (400 MHz, DMSO-$d_6$) δ 8.79 (d, *J* = 4.9 Hz, 2H), 8.23 (s, 2H), 7.98 (d, *J* = 2.3 Hz, 1H), 7.49 - 7.36 (m, 3H), 7.25 - 6.85 (m, 1H), 6.52 (d, *J* = 7.0 Hz, 1H), 6.39 (d, *J* = 8.7 Hz, 1H), 5.74 (d, *J* = 5.5 Hz, 1H), 5.60 (d, *J* = 5.5 Hz, 1H), 4.26 (td, *J* = 13.9, 6.7 Hz, 2H), 2.19 - 2.05 (m, 2H), 1.92 - 1.76 (m, 2H), 1.50 (dt, *J* = 19.5, 7.1 Hz, 2H). |
| G9 | 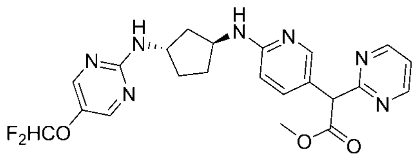<br>$^1$H NMR (400 MHz, DMSO-$d_6$) δ 8.79 (d, *J* = 4.9 Hz, 2H), 8.24 (s, 2H), 7.94 (d, *J* = 2.4 Hz, 1H), 7.47 (d, *J* = 7.2 Hz, 1H), 7.45 - 7.37 (m, 2H), 7.04 (t, *J* = 74.0 Hz, 1H), 6.60 (d, *J* = 6.9 Hz, 1H), 6.42 (d, *J* = 8.7 Hz, 1H), 5.23 (s, 1H), 4.27 (dq, *J* = 18.1, 6.7 Hz, 2H), 3.65 (s, 3H), 2.10 (dq, *J* = 10.4, 6.2, 4.7 Hz, 2H), 1.85 (h, *J* = 6.5 Hz, 2H), 1.59 - 1.40 (m, 2H). | G12 | 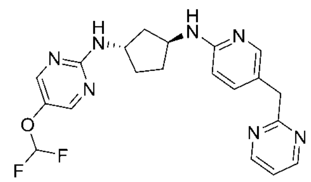<br>$^1$H NMR (400 MHz, DMSO-$d_6$) δ 8.73 (d, *J* = 4.9 Hz, 2H), 8.24 (d, *J* = 5.6 Hz, 2H), 7.90 (d, *J* = 2.3 Hz, 1H), 7.48 (d, *J* = 7.2 Hz, 1H), 7.39 - 7.27 (m, 2H), 7.04 (t, *J* = 74.0 Hz, 1H), 6.42 (dd, *J* = 18.5, 7.7 Hz, 2H), 4.26 (dq, *J* = 20.3, 6.7 Hz, 2H), 4.00 (s, 2H), 2.10 (tt, *J* = 10.6, 4.4 Hz, 2H), 1.84 (tdd, *J* = 16.9, 12.3, 8.2 Hz, 2H), 1.58 - 1.39 (m, 2H). |
| G11 | 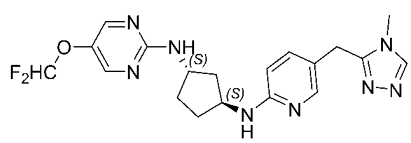<br>$^1$H NMR (400 MHz, DMSO-$d_6$) δ 8.35 (s, -1H), 8.24 (s, 2H), 7.86 (s, -1H), 7.46 (d, *J* = 7.1 Hz, -1H), 7.23 (s, -1H), 7.04 (s, -1H), 6.86 (s, -1H), 6.46 (d, *J* = 22.5 Hz, -2H), 4.29 (d, *J* = 6.7 Hz, -2H), 3.52 (s, 2H), 2.15 - 1.79 (m, -2H), 1.50 (d, *J* = 18.3 Hz, -2H), 1.26 (s, 3H), 1.11 - 0.99 (m, -2H). | G14 | 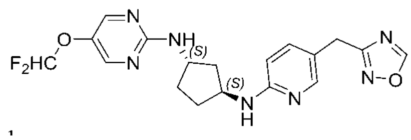<br>,<br>$^1$H NMR (400 MHz, DMSO-$d_6$) δ 9.60 (s, 1H), 8.27 (s, 2H), 7.95 (s, 1H), 7.87 (d, J = 9.5 Hz, 1H), 7.54 (d, *J* = 6.9 Hz, 1H), 7.06 (d, *J* = 5.1 Hz, 1H), 7.03 (s, 1H), 6.88 (s, -1H), 4.26 (d, *J* = 49.2 Hz, 2H), 4.15 (s, 2H), 2.26 - 2.02 (m, 2H), 2.02 - 1.90 (m, 2H), 1.61 (q, *J* = 5.8, 4.5 Hz, 2H). |

(continued)

| No. | Compound | No. | Compound |
|---|---|---|---|
| G13 | 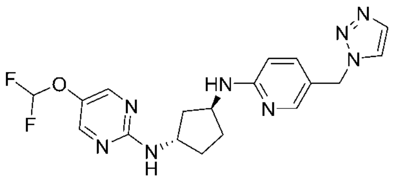 $^1$H NMR (400 MHz, DMSO-$d_6$) δ 8.24 (s, 2H), 8.13 (s, 1H), 8.04 (d, *J* = 2.4 Hz, 1H), 7.72 (s, 1H), 7.46 (d, *J* = 7.2 Hz, 1H), 7.36 (dd, *J* = 8.7, 2.4 Hz, 1H), 7.04 (s, -1H), 6.72 (d, *J* = 6.8 Hz, 1H), 6.44 (d, *J* = 8.6 Hz, 1H), 5.41 (s, 2H), 4.31 - 4.25 (m, 2H), 2.10 (dt, *J* = 7.5, 3.8 Hz, 2H), 1.91 - 1.83 (m, 2H), 1.50 (ddd, *J* = 15.4, 11.3, 6.1 Hz, 2H). | G16 | 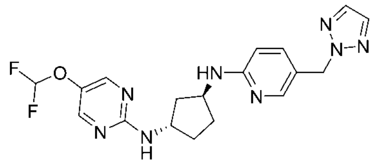 $^1$H NMR (400 MHz, DMSO-$d_6$) δ 8.24 (s, 2H), 7.77 (s, 2H), 7.46 (d, *J* = 7.2 Hz, 1H), 7.34 (dd, *J* = 8.6, 2.3 Hz, 1H), 7.13 (d, *J* = 74.0 Hz, -1H), 6.70 (d, *J* = 7.0 Hz, 1H), 6.43 (d, *J* = 8.6 Hz, 1H), 5.44 (s, 2H), 4.28 (dt, *J* = 11.6, 6.1 Hz, 2H), 2.09 (tt, *J* = 7.8, 3.2 Hz, 2H), 1.84 (ddd, *J* = 18.9, 13.2, 7.2 Hz, 2H), 1.49 (ddt, *J* = 17.2, 12.1, 5.9 Hz, 2H). |
| G15 | 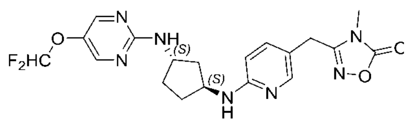 $^1$H NMR (400 MHz, DMSO-$d_6$) δ 8.24 (s, 4H), 7.90 (s, 1H), 7.49 (s, 1H), 7.47 (s, 1H), 7.03 (s, -1H), 4.30 (q, *J* = 6.8 Hz, 2H), 3.10 (s, 3H), 2.16 - 2.09 (m, 2H), 1.91 (d, *J* = 7.6 Hz, 2H), 1.54 (d, *J* = 11.5 Hz, 2H), 1.24 (s, 2H). | G18 | 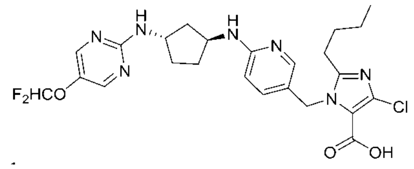 $^1$H NMR: (METHANOL-$d_4$, 400 MHz) δ 8.16 (s, 2H), 7.74 (s, 1H), 7.40 (dd, 1H, *J*=1.9, 8.9 Hz), 6.5-6.9 (m, 2H), 5.49 (s, 2H), 4.3-4.4 (m, 1H), 4.22 (quin, 1H, *J*=6.3 Hz), 2.7-2.8 (m, 2H), 2.25 (br dd, 2H, *J*=6.9, 12.6 Hz), 1.9-2.1 (m, 2H), 1.5-1.7 (m, 4H), 1.3-1.4 (m, 2H), 0.90 (t, 3H, *J*=7.4 Hz) |
| G17 | 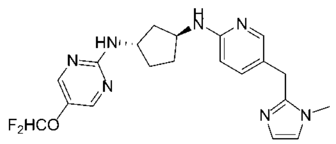 $^1$H NMR: (400 MHz, DMSO-d6) δ 8.25 - 8.21 (m, 2H), 8.21 - 8.19 (m, 1H), 7.81 (d, *J* = 2.0 Hz, 1H), 7.45 (d, *J* = 7.1 Hz, 1H), 7.25 - 6.82 (m, 4H), 6.74 (d, *J* = 1.1 Hz, 1H), 6.49 - 6.32 (m, 2H), 4.36 - 4.18 (m, 2H), 3.82 (s, 2H), 3.51 (s, 3H), 2.16 - 2.01 (m, 2H), 1.91 - 1.75 (m, 2H), 1.60 - 1.38 (m, 2H) | G20 | 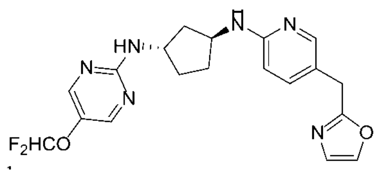 $^1$H NMR: (400 MHz, DMSO-d6) δ 8.30 - 8.25 (m, 1H), 8.24 - 8.20 (m, 2H), 7.85 (d, *J* = 2.0 Hz, 1H), 7.79 (d, *J* = 0.8 Hz, 1H), 7.45 (d, *J* = 7.3 Hz, 1H), 7.24 (dd, *J* = 2.4, 8.5 Hz, 1H), 7.22 - 6.82 (m, 1H), 6.44 - 6.33 (m, 2H), 4.36 - 4.17 (m, 2H), 3.62 (s, 2H), 2.17 - 2.02 (m, 2H), 1.94 - 1.74 (m, 2H), 1.58 - 1.37 (m, 2H) |
| G19 | 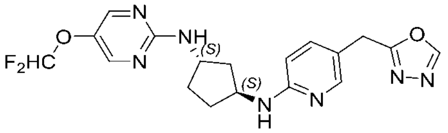 $^1$H NMR (400 MHz, DMSO-$d_6$) δ 9.13 (s, 1H), 8.24 (s, 2H), 7.93 (d, *J* = 2.4 Hz, 1H), 7.49 (d, *J* = 5.8 Hz, 1H), 7.31 (dd, *J* = 8.6, 2.4 Hz, 1H), 6.95 (d, *J* = 74.0 Hz, 1H), 6.60 (d, *J* = 6.9 Hz, 1H), 6.45 (d, *J* = 8.6 Hz, 1H), 4.31 - 4.25 (m, 2H), 4.09 (s, 2H), 2.11 (dq, *J* = 11.8, 4.6 Hz, 2H), 1.91 - 1.83 (m, 2H), 1.50 (ddd, *J* = 20.2, 9.6, 5.9 Hz, 2H). | G6 | 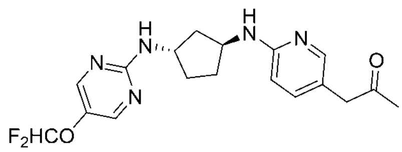 $^1$H NMR (400 MHz, DMSO-$d_6$) δ 8.22 (s, 2H), 7.85 - 7.68 (m, 1H), 7.45 (d, *J* = 7.3 Hz, 1H), 7.27 - 6.82 (m, 2H), 6.42 (dd, *J* = 15.5, 7.7 Hz, 2H), 4.38 - 4.16 (m, 2H), 3.54 (s, 2H), 2.10 (s, 5H), 1.94 - 1.77 (m, 2H), 1.49 (d, *J* = 20.9 Hz, 2H). |

(continued)

| No. | Compound | No. | Compound |
|---|---|---|---|
| G21 | 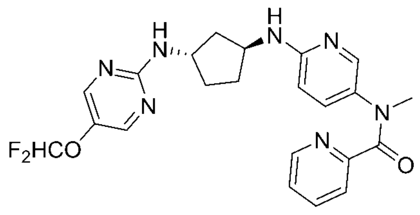 | $^1$H NMR (400 MHz, DMSO-$d_6$) δ 8.33 (d, $J$ = 4.8 Hz, 1H), 8.22 (s, 2H), 7.72 (td, $J$ = 7.8, 1.6 Hz, 1H), 7.62 (d, $J$ = 2.7 Hz, 1H), 7.41 (t, $J$ = 8.2 Hz, 2H), 7.27 - 6.81 (m, 3H), 6.61 (d, $J$ = 6.9 Hz, 1H), 6.28 (d, $J$ = 8.8 Hz, 1H), 4.24 (q, $J$ = 7.0 Hz, 1H), 4.12 (q, $J$ = 6.6 Hz, 1H), 3.29 (s, 3H), 2.03 (tt, $J$ = 13.3, 6.0 Hz, 2H), 1.77 (ddt, $J$ = 26.4, 13.1, 6.8 Hz, 2H), 1.46 (q, $J$ = 8.0, 6.8 Hz, 1H), 1.35 (d, $J$ = 11.1 Hz, 1H). | |

Example H1

Synthesis of 6'-(((1S,3S)-3-((1-(cyclopropylmethyl)-1H-1,2,4-triazol-3-yl)amino)cyclopentyl)amino)-2H-[1,3' -bipyridin]-2-one

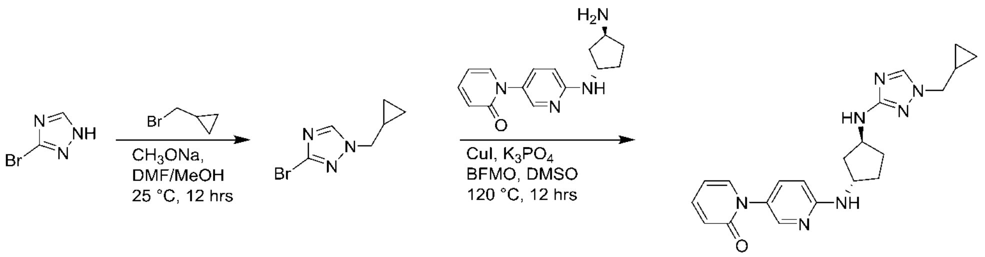


Step 1: Synthesis of 3-bromo-1-(cyclopropylmethyl)-1H-1,2,4-triazole

At room temperature, 3-bromo-1H-1,2,4-triazole (1.00 g, 6.76 mmol, 1.00 eq) was dissolved in methanol (10 mL). Sodium methoxide (365 mg, 6.76 mmol, 1.00 eq) was added. The mixture was stirred at 25 °C for 10 min under nitrogen protection. The mixture was concentrated by rotary evaporation under reduced pressure. The concentrated liquid was dissolved in DMF (5 mL). (Bromomethyl)cyclopropane (912 mg, 6.76 mmol, 645 μL, 1.00 eq) was added. The mixture was stirred at 25 °C for 12 hours under nitrogen protection.

LCMS showed the MS of the target product (RT = 0.410 min, m/z = 202.0 $[M+H]^+$). The reaction was quenched with saturated ammonium chloride solution (15 mL) and extracted with ethyl acetate (20 mL × 3). The organic phases were combined, washed with saturated sodium chloride aqueous solution, dried over anhydrous sodium sulfate, and concentrated under reduced pressure to obtain the crude product. The crude product was purified by column chromatography (PE:EA = 1:0 to 5:1) to obtain 3-bromo-1-(cyclopropylmethyl)-1H-1,2,4-triazole as a colorless oil (452 mg, 2.24 mmol, 33.10% yield). MS (ESI) m/z = 202.0 [M+H]+

Step 2: Synthesis of 6'-(((1S,3S)-3-((1-(cyclopropylmethyl)-1H-1,2,4-triazol-3-yl)amino)cyclopentyl)amino)-2H-[1,3' -bipyridin]-2-one

At room temperature, 3-bromo-1-(cyclopropylmethyl)-1H-1,2,4-triazole (200 mg, 990 μmol, 1.00 eq), 6'-(((1S,3S)-3-aminocyclopentyl)amino)-2H-[1,3'-bipyridin]-2-one hydrochloride (304 mg, 990 μmol, 1.00 eq), CuI (37.7 mg, 198 μmol, 0.20 eq), $K_3PO_4$ (630 mg, 2.97 mmol, 3.00 eq), and N,N'-bis(2-furfuryl)oxalamide (98.3 mg, 396 μmol, 0.40 eq) were added to DMSO (2.00 mL). The mixture was heated to 120 °C and stirred for 12 hours under nitrogen protection.

LCMS showed the MS of the target product (RT = 0.858 min, m/z = 392.2 $[M+H]^+$). The reaction was complex. Saturated ammonium chloride solution (5 mL) was added to quench the reaction. The mixture was extracted with ethyl acetate (15 mL × 3). The organic phases were combined, washed with saturated sodium chloride aqueous solution, dried over anhydrous sodium sulfate, and concentrated under reduced pressure to obtain the crude product. The crude product was purified by reverse-phase preparative chromatography (column: Waters Xbridge 150*25mm*5μm; mobile phase: [water (ammonia hydroxide v/v)-ACN]; gradient: 8% to 38% B over 10 min) to obtain 6'-(((1S,3S)-3-((1-(cyclopropylmethyl)-1H-1,2,4-triazol-3-yl)amino)cyclopentyl)amino)-2H-[1,3' -bipyridin]-2-one as a light yellow solid (20 mg, 51.1

μmol, 5.16% yield).

MS (ESI) m/z = 392.2 [M+H]+
H NMR: (400 MHz, DMSO-$d_6$ + TFA): δ 9.40 (s, 1H), 8.19 (s, 1H), 7.98 (br d, J = 10.4 Hz, 1H), 7.65 (dd, J = 1.7, 6.8 Hz, 1H), 7.53 (ddd, J = 1.9, 6.8, 9.1 Hz, 1H), 7.12 (br d, J = 9.0 Hz, 1H), 6.50 (d, J = 9.1 Hz, 1H), 6.35 (t, J = 6.2 Hz, 1H), 4.37 - 4.22 (m, 1H), 4.15 - 4.03 (m, 1H), 3.97 (d, J = 7.3 Hz, 2H), 2.36 - 1.98 (m, 5H), 1.73 - 1.52 (m, 2H), 1.33 - 1.19 (m, 2H), 0.63 - 0.56 (m, 2H), 0.45 - 0.38 (m, 2H).

Example H2

Synthesis of 5'-fluoro-6'-(((1S,3S)-3-((5-methyl-1,2,4-oxadiazol-3-yl)amino)cyclopentyl)amino)-2H-[1,3'-bip yridin]-2-one

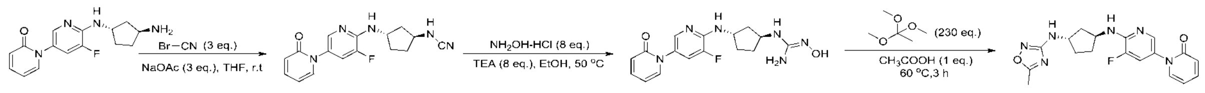


Step 1: Synthesis of (N-((1S,3S)-3-((5'-fluoro-2-oxo-2H-[1,3'-bipyridin]-6'-yl)amino)cyclopentyl)cyanamide

At room temperature, 6'-(((1S,3S)-3-aminocyclopentyl)amino)-5'-fluoro-2H-[1,3'-bipyridin]-2-one (0.864 g, 3.0 mmol, 1.0 eq) was dissolved in tetrahydrofuran (10 mL). Potassium acetate (0.882 g, 9.0 mmol, 3.0 eq) and cyanogen bromide (0.738 g, 9 mmol, 3.0 eq) were added. The mixture was stirred vigorously overnight.

After completion of the reaction, ethyl acetate (50 mL) was added to dilute the reaction mixture. Insoluble impurities were filtered off. The resulting organic phase was concentrated under reduced pressure to dryness to obtain (N-((1S,3S)-3-((5'-fluoro-2-oxo-2H-[1,3'-bipyridin]-6'-yl)amino)cyclopentyl)cyanamide (1.25 g) as a brown oil, which was used directly in the next step. LC-MS: [M+H]+ = 314.

Step 2: Synthesis of (E)-1-((1S,3S)-3-((5'-fluoro-2-oxo-2H-[1,3'-bipyridin]-6'-yl)amino)cyclopentyl)-2-hydroxyguani dine

At room temperature, (N-((1S,3S)-3-((5'-fluoro-2-oxo-2H-[1,3'-bipyridin]-6'-yl)amino)cyclopentyl)cyanamide (1.25 g, 4 mmol, 1.0 eq) was dissolved in ethanol (10 mL). Hydroxylamine hydrochloride (3.39 g, 32 mmol, 8.0 eq) and triethylamine (3.23 g, 32 mmol, 8.0 eq) were added. After stirring evenly, the mixture was transferred to an oil bath and heated to 50 °C and reacted overnight.

After completion of the reaction, the reaction mixture was concentrated under reduced pressure. The crude product was purified by C18 column chromatography (330 g C18 silica gel, eluent: acetonitrile/water) to obtain (E)-1-((1S,3S)-3-((5'-fluoro-2-oxo-2H-[1,3'-bipyridin]-6'-yl)amino)cyclopentyl)-2-hydroxyguani dine as a light yellow solid (373 mg, total yield for two steps: 36%). LC-MS: $[M+H]^+$ = 347.

Step 3: Synthesis of 5'-fluoro-6'-(((1S,3S)-3-((5-methyl-1,2,4-oxadiazol-3-yl)amino)cyclopentyl)amino)-2H-[1,3'-bip yridin]-2-one

At room temperature, (E)-1-((1S,3S)-3-((5'-fluoro-2-oxo-2H-[1,3'-bipyridin]-6'-yl)amino)cyclopentyl)-2-hydroxyguani dine (373 mg, 1.07 mmol, 1.0 eq) was added to trimethyl orthoacetate (3 mL). Acetic acid (130 mg, 1.07 mmol, 1.0 eq) was added. After stirring evenly, the mixture was transferred to an oil bath and heated to 60 °C. The reaction was stirred for 3 hours.

After completion of the reaction, the reaction mixture was concentrated under reduced pressure. The crude product was purified by C18 column chromatography (120 g C18 silica gel, eluent: acetonitrile/water) to obtain 5'-fluoro-6'-(((1S,3S)-3-((5-methyl-1,2,4-oxadiazol-3-yl)amino)cyclopentyl)amino)-2H-[1,3'-bip yridin]-2-one (15 mg, yield: 3.8%) as a white solid. LC-MS: $[M+H]^+$ = 371.

NMR data: H NMR (400 MHz, DMSO-$d_6$) δ 7.84 (d, J = 2.2 Hz, 1H), 7.68 - 7.61 (m, 1H), 7.58 - 7.45 (m, 2H), 6.97 - 6.87 (m, 1H), 6.80 (d, J = 7.0 Hz, 1H), 6.46 (dt, J = 9.1, 1.1 Hz, 1H), 6.28 (td, J = 6.7, 1.4 Hz, 1H), 4.49 (h, J = 6.9 Hz, 1H), 3.90 (q, J = 6.5 Hz, 1H), 2.38 (s, 3H), 2.09 (tt, J = 11.5, 5.9 Hz, 2H), 1.99 - 1.86 (m, 2H), 1.55 (tt, J = 16.0, 7.0 Hz, 2H).

Example H3

Synthesis of 1-(5-(((1S,3S5)-3-((5-cyclopropyl-1-methyl-1H-1,2,4-triazol-3-yl)amino)cyclopentyl)amino)pyra zin-2-yl) pyridin-2(1H)-one

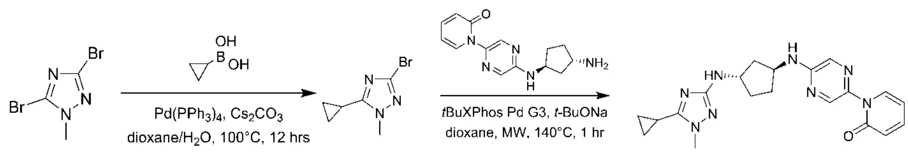


Step 1: Synthesis of 3-bromo-5-cyclopropyl-1-methyl-1H-1,2,4-triazole

3,5-Dibromo-1-methyl-1H-1,2,4-triazole (2.00 g, 8.30 mmol, 1.0 eq), cyclopropylboronic acid (713 mg, 8.30 mmol, 1.0 eq), and cesium carbonate (8.12 g, 24.9 mmol, 3.0 eq) were dissolved in 1,4-dioxane (20 mL) and water (5 mL). Then $Pd(PPh_3)_4$ (959 mg, 830 μmol, 0.1 eq) was added. After addition, the mixture was stirred at 100 °C for 12 hours. LCMS showed the MS value of the product (RT = 0.374 min). The reaction mixture was cooled to room temperature. Water (50 mL) was added to dilute the mixture. The mixture was extracted with ethyl acetate (20 mL * 2). The organic phases were combined, washed with saturated brine (100 mL * 2), dried over anhydrous sodium sulfate, filtered, and concentrated to obtain the crude product. The crude product was purified by reverse-phase chromatography (0.1% FA condition) to obtain 3-bromo-5-cyclopropyl-1-methyl-1H-1,2,4-triazole (700 mg, 3.46 mmol, 41.7% yield). LCMS: (ESI) m/z = 202.0 $[M+1]^+$.

Step 2: Synthesis of 1-(5-(((1S,3S)-3-((5-cyclopropyl-1-methyl-1H-1,2,4-triazol-3-yl)amino)cyclopentyl)amino)pyra zin-2-yl)pyridin-2(1H)-one

3-Bromo-5-cyclopropyl-1-methyl-1H-1,2,4-triazole (100 mg, 495 μmol, 1.0 eq), 1-(5-(((1S,3S)-3-aminocyclopentyl)amino)pyrazin-2-yl)pyridin-2(1H)-one (134 mg, 495 μmol, 1.0 eq), sodium tert-butoxide (119 mg, 1.24 mmol, 2.5 eq), and tBuXPhos Pd G3 (39.3 mg, 49.5 μmol, 0.10 eq) were dissolved in 1,4-dioxane (5 mL). Then the mixture was stirred at 140 °C under microwave irradiation for 1 hour. LCMS showed the MS value of the product (RT = 0.381 min). The reaction mixture was filtered. The filtrate was concentrated to obtain the crude product. The crude product was first purified by reverse-phase preparative chromatography (acidic condition) (column: Phenomenex luna C18 150*40mm*15μm; mobile phase: [water(FA)-ACN]; gradient: 5% to 35% B over 15 min), then further purified by reverse-phase preparative chromatography (basic condition) (column: Waters Xbridge 150*25mm*5μm; mobile phase: [water (ammonia hydroxide v/v)-ACN]; gradient: 0% to 30% B over 10 min) to obtain 1-(5-(((1S,3S)-3-((5-cyclopropyl-1-methyl-1H-1,2,4-triazol-3-yl) amino)cyclopentyl)amino)pyra zin-2-yl)pyridin-2(1H)-one (three parallel batches were processed in microwave, total: 174 mg, 438 μmol, 29.5% yield, 98.9% purity). LCMS: (ESI) m/z = 393.2 $[M+1]^+$.

H NMR: (400 MHz, DMSO-d6): δ 8.23 (d, J = 1.3 Hz, 1H), 7.83 (d, J = 1.3 Hz, 1H), 7.70 (dd, J = 1.6, 6.9 Hz, 1H), 7.49 (ddd, J = 2.1, 6.7, 9.1 Hz, 1H), 7.42 (d, J = 6.9 Hz, 1H), 6.46 (d, J = 9.0 Hz, 1H), 6.31 (dt, J = 1.3, 6.8 Hz, 1H), 5.70 (d, J = 7.0 Hz, 1H), 4.26 (sxt, J = 6.5 Hz, 1H), 3.91 (sxt, J = 6.6 Hz, 1H), 3.64 (s, 3H), 2.18 - 2.00 (m, 2H), 2.00 - 1.93 (m, 1H), 1.93 - 1.73 (m, 2H), 1.52 - 1.39 (m, 2H), 0.98 - 0.91 (m, 2H), 0.83 - 0.76 (m, 2H).

Example H4

According to the preparation methods of the aforementioned Examples, Compound H4 was prepared:

| | |
|---|---|
| 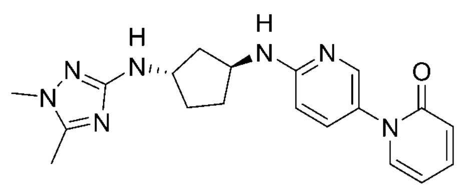 | $^1$H NMR (400 MHz, DMSO-$d_6$) δ 7.90 (d, *J* = 2.6 Hz, 1H), 7.59 (dd, J = 6.9, 2.1 Hz, 1H), 7.53 - 7.33 (m, 2H), 6.85 (d, *J* = 6.9 Hz, 1H), 6.47 (dd, *J* = 27.4, 9.0 Hz, 2H), 6.27 (td, *J* = 6.7, 1.3 Hz, 1H), 5.60 (d, *J* = 7.2 Hz, 1H), 4.27 (p, *J* = 6.6 Hz, 1H), 3.93 (h, *J* = 6.7 Hz, 1H), 3.52 (s, 3H), 2.30 - 1.94 (m, 5H), 1.92 - 1.72 (m, 2H), 1.57 - 1.33 (m, 2H). |

Examples H5 to H14

According to the preparation methods of the aforementioned Examples, Compounds H5 to H14 were prepared:

| No. | Compound Structure | No. | Compound Structure |
|---|---|---|---|
| H5 | 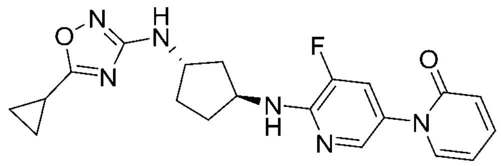 $^1$H NMR (400 MHz, DMSO-$d_6$) δ = 7.93 - 7.79 (m, 1H), 7.71 - 7.61 (m, 1H), 7.58 - 7.42 (m, 2H), 6.98 - 6.84 (m, 1H), 6.84 - 6.70 (m, 1H), 6.54 - 6.40 (m, 1H), 6.36 - 6.21 (m, 1H), 4.61 - 4.37 (m, 1H), 3.98 - 3.78 (m, 1H), 2.18 - 2.02 (m, 3H), 1.99 - 1.84 (m, 2H), 1.63 - 1.45 (m, 2H), 1.18 - 1.08 (m, 2H), 1.03 - 0.93 (m, 2H). | H6 | 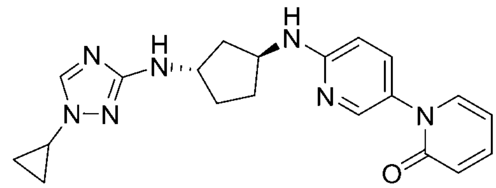 $^1$H NMR: (400 MHz, DMSO-*d*6): δ 8.05 - 8.01 (m, 1H), 7.92 (d, *J* = 2.6 Hz, 1H), 7.60 (dd, *J* = 1.7, 6.8 Hz, 1H), 7.48 (ddd, *J* = 2.1, 6.7, 9.1 Hz, 1H), 7.39 (dd, *J* = 2.6, 8.9 Hz, 1H), 6.88 (d, *J* = 6.9 Hz, 1H), 6.52 (d, *J* = 8.9 Hz, 1H), 6.44 (d, *J* = 9.1 Hz, 1H), 6.27 (dt, *J* = 1.3, 6.7 Hz, 1H), 5.87 (d, *J* = 7.1 Hz, 1H), 4.28 (sxt, *J* = 6.6 Hz, 1H), 4.05 - 3.91 (m, 1H), 3.48 (tt, *J* = 3.8, 7.3 Hz, 1H), 2.18 - 2.02 (m, 2H), 1.95 - 1.75 (m, 2H), 1.56 - 1.37 (m, 2H), 1.03 - 0.86 (m, 4H). |
| H7 | 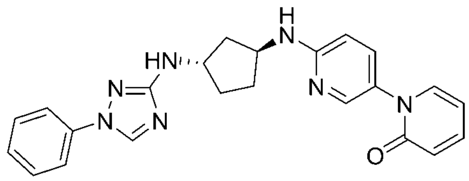 | H8 | 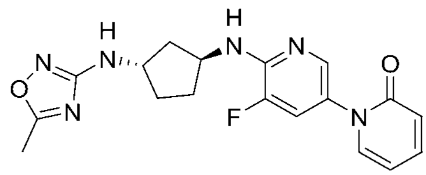 |
| | $^1$H NMR: (400 MHz, DMSO-*d*6) δ 8.85 (s, 1H), 7.93 (d, *J* = 2.6 Hz, 1H), 7.74 (d, *J* = 7.9 Hz, 2H), 7.61 (dd, *J* = 1.9, 6.8 Hz, 1H), 7.52 - 7.45 (m, 3H), 7.40 (dd, *J* = 2.6, 8.9 Hz, 1H), 7.32 - 7.25 (m, 1H), 6.91 (d, *J* = 6.9 Hz, 1H), 6.53 (d, *J* = 9.0 Hz, 1H), 6.45 (d, *J* = 9.1 Hz, 1H), 6.35 (d, *J* = 7.0 Hz, 1H), 6.27 (dt, *J* = 1.1, 6.7 Hz, 1H), 4.40 - 4.25 (m, 1H), 4.19 - 4.01 (m, 1H), 2.24 - 2.09 (m, 2H), 2.05 - 1.95 (m, 1H), 1.92 - 1.80 (m, 1H), 1.67 - 1.42 (m, 2H). | | $^1$H NMR (400 MHz, DMSO-$d_6$) δ 7.84 (d, *J* = 2.2 Hz, 1H), 7.68 - 7.61 (m, 1H), 7.58 - 7.45 (m, 2H), 6.97 - 6.87 (m, 1H), 6.80 (d, *J* = 7.0 Hz, 1H), 6.46 (dt, *J* = 9.1, 1.1 Hz, 1H), 6.28 (td, *J* = 6.7, 1.4 Hz, 1H), 4.49 (h, *J* = 6.9 Hz, 1H), 3.90 (q, *J* = 6.5 Hz, 1H), 2.38 (s, 3H), 2.09 (tt, *J* = 11.5, 5.9 Hz, 2H), 1.99 - 1.86 (m, 2H), 1.55 (tt, *J* = 16.0, 7.0 Hz, 2H). |
| H9 | 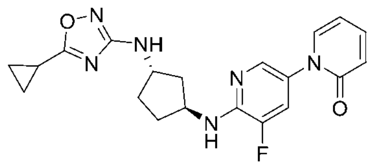 $^1$H NMR: (400 MHz, DMSO-d6) δ 7.93 - 7.79 (m, 1H), 7.71 - 7.61 (m, 1H), 7.58 - 7.42 (m, 2H), 6.98 - 6.84 (m, 1H), 6.84 - 6.70 (m, 1H), 6.54 - 6.40 (m, 1H), 6.36 - 6.21 (m, 1H), 4.61 - 4.37 (m, 1H), 3.98 - 3.78 (m, 1H), 2.18 - 2.02 (m, 3H), 1.99 - 1.84 (m, 2H), 1.63 - 1.45 (m, 2H), 1.18 - 1.08 (m, 2H), 1.03 - 0.93 (m, 2H). | H10 | 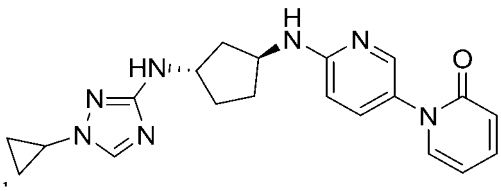 $^1$H NMR (400 MHz, DMSO-*d*6) δ 8.05 - 8.00 (m, 1H), 7.92 (d, *J* = 2.6 Hz, 1H), 7.60 (dd, J = 1.7, 6.8 Hz, 1H), 7.48 (ddd, *J* = 2.1, 6.7, 9.1 Hz, 1H), 7.39 (dd, *J* = 2.6, 8.9 Hz, 1H), 6.88 (d, *J* = 6.9 Hz, 1H), 6.52 (d, *J* = 8.9 Hz, 1H), 6.44 (d, *J* = 9.1 Hz, 1H), 6.27 (dt, *J* = 1.3, 6.7 Hz, 1H), 5.87 (d, *J* = 7.1 Hz, 1H), 4.28 (sxt, J = 6.6 Hz, 1H), 3.97 (sxt, J = 6.6 Hz, 1H), 3.48 (tt, *J* = 3.8, 7.3 Hz, 1H), 2.20 - 2.01 (m, 2H), 1.94 - 1.73 (m, 2H), 1.54 - 1.36 (m, 2H), 1.04 - 0.84 (m, 4H) |

(continued)

| No. | Compound Structure | No. | Compound Structure |
|---|---|---|---|
| H11 | 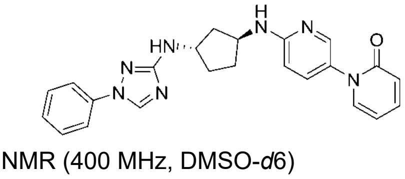 $^1$H NMR (400 MHz, DMSO-*d*6) δ 8.87 - 8.82 (m, 1H), 7.93 (d, *J* = 2.6 Hz, 1H), 7.74 (d, *J* = 7.9 Hz, 2H), 7.61 (dd, *J* = 1.9, 6.8 Hz, 1H), 7.53 - 7.44 (m, 3H), 7.40 (dd, *J* = 2.6, 8.9 Hz, 1H), 7.33 - 7.23 (m, 1H), 6.91 (d, *J* = 6.9 Hz, 1H), 6.53 (d, *J* = 9.0 Hz, 1H), 6.45 (d, *J* = 9.1 Hz, 1H), 6.35 (d, *J* = 7.0 Hz, 1H), 6.27 (dt, *J* = 1.1, 6.7 Hz, 1H), 4.37 - 4.25 (m, 1H), 4.16 - 4.03 (m, 1H), 2.22 - 2.09 (m, 2H), 2.06 - 1.95 (m, 1H), 1.93 - 1.83 (m, 1H), 1.66 - 1.41 (m, 2H) | H12 | 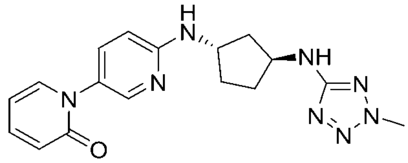 $^1$H NMR (400 MHz, DMSO-d6) δ7.91 (d, *J* = 2.4 Hz, 1H), 7.63 - 7.56 (m, 1H), 7.52 - 7.44 (m, 1H), 7.39 (dd, *J* = 2.6, 8.9 Hz, 1H), 6.90 (d, *J* = 6.9 Hz, 1H), 6.66 (d, *J* = 6.9 Hz, 1H), 6.51 (d, *J* = 8.9 Hz, 1H), 6.44 (d, *J* = 9.1 Hz, 1H), 6.27 (t, *J* = 6.7 Hz, 1H), 4.37 - 4.24 (m, 1H), 4.10 (s, 3H), 4.01 (sxt, *J* = 6.6 Hz, 1H), 2.20 - 2.04 (m, 2H), 1.88 (ddt, *J* = 6.7, 13.2, 19.4 Hz, 2H), 1.60 - 1.40 (m, 2H). |
| H13 | 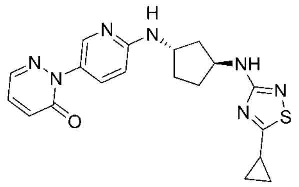 | H14 | 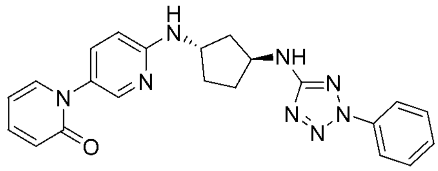 $^1$H NMR, (400 MHz, DMSO-d6) |
| | $^1$H NMR (DMSO-$d_6$, 400 MHz) 8.09 (d, J = 2.5 Hz, 1H), 8.01 (dd, J = 1.6, 3.8 Hz, 1H), 7.50 (dd, J = 2.7, 8.9 Hz, 1H), 7.45 (dd, J = 3.8, 9.4 Hz, 1H), 7.35 (d, J = 6.9 Hz, 1H), 7.02 (dd, J = 1.5, 9.5 Hz, 1H), 6.90 (d, J = 6.9 Hz, 1H), 6.51 (d, J = 9.0 Hz, 1H), 4.33 - 4.22 (m, 1H), 4.14 (qd, J = 6.4, 13.1 Hz, 1H), 2.46 - 2.38 (m, 1H), 2.15 - 2.05 (m, 2H), 1.96 - 1.78 (m, 2H), 1.57 - 1.42 (m, 2H), 1.23 - 1.14 (m, 2H), 1.05 - 0.95 (m, 2H) | | δ8.01 - 7.88 (m, 3H), 7.65 - 7.55 (m, 3H), 7.54 - 7.44 (m, 2H), 7.40 (dd, *J* = 2.6, 8.9 Hz, 1H), 7.14 (d, *J* = 6.8 Hz, 1H), 6.94 (d, *J* = 6.9 Hz, 1H), 6.53 (d, *J* = 8.9 Hz, 1H), 6.44 (d, *J* = 9.3 Hz, 1H), 6.27 (t, *J* = 6.8 Hz, 1H), 4.43 - 4.29 (m, 1H), 4.23 - 4.08 (m, 1H), 2.25 - 2.11 (m, 2H), 2.07 - 1.97 (m, 1H), 1.96 - 1.86 (m, 1H), 1.67 - 1.46 (m, 2H). |

Example J1

Synthesis of 2-(6-(((1S,3S)-3-((5-(difluoromethoxy)pyrimidin-2-yl)amino)cyclohexyl)amino)pyridin-3-yl)pyr idazin-3(2H)-one

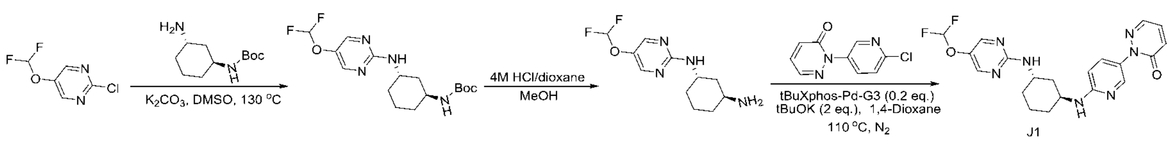


Step A: Synthesis of tert-butyl ((1S,3S)-3-((5-(difluoromethoxy)pyrimidin-2-yl)amino)cyclohexyl)carbamate

At room temperature under a nitrogen atmosphere, 2-chloro-5-(difluoromethoxy)pyrimidine (0.6 g, 3.33 mmol, 1.2 eq), tert-butyl ((1S,3S)-3-aminocyclohexyl)carbamate (0.60 g, 2.78 mmol, 1.0 eq), and potassium carbonate (1.53 g, 11.12 mmol, 4.0 eq) were added to dimethyl sulfoxide (10 mL). After stirring evenly, the mixture was transferred to an oil bath and heated to 130 °C. The reaction was stirred overnight.

After completion of the reaction, cold water (35 mL) was added to quench the reaction. The mixture was extracted with ethyl acetate (75 mL × 3). The organic phases were combined, dried over anhydrous sodium sulfate, filtered, and

concentrated. The resulting residue was purified by silica gel column chromatography (eluent: ethyl acetate/n-hexane = 5/10) to obtain 0.96 g of tert-butyl ((1S,3S)-3-((5-(difluoromethoxy)pyrimidin-2-yl)amino)cyclohexyl)carbamate as a pale yellow solid (yield: 96%). LC-MS: [M+H]+ = 359.

Step B: Synthesis of (1S,3S)-$N^1$-(5-(difluoromethoxy)pyrimidin-2-yl)cyclohexane-1,3-diamine

At room temperature, tert-butyl ((1S,3S)-3-((5-(difluoromethoxy)pyrimidin-2-yl)amino)cyclohexyl)carbamate (0.358 g, 1.0 mmol, 1.0 eq) was dissolved in methanol (4 mL). Then a 4 M HCl/1,4-dioxane solution (2.5 mL, 10 eq) was added dropwise. The reaction was stirred vigorously for 4 hours.

After completion of the reaction, the organic solvent was evaporated under reduced pressure to obtain (1S,3S)-$N^1$-(5-(difluoromethoxy)pyrimidin-2-yl)cyclohexane-1,3-diamine as a brown foamy solid powder. It was used directly in the next step without further purification. LC-MS: [M+H]+ = 259.

Step C: Synthesis of 2-(6-(((1S,3S)-3-((5-(difluoromethoxy)pyrimidin-2-yl)amino)cyclohexyl)amino)pyridin-3-yl)pyr idazin-3(2H)-one

At room temperature under a nitrogen atmosphere, (1S,3S)-$N^1$-(5-(difluoromethoxy)pyrimidin-2-yl)cyclohexane-1,3-diamine (0.258 g, 1 mmol, 1.0 eq), 2-(6-chloropyridin-3-yl)pyridazin-3(2H)-one (0.25 g, 1.2 mmol, 1.0 eq), methanesulfonato(2-di-tert-butylphosphino-2',4',6'-triisopropyl-1,1'-biphenyl)(2'-amino-1,1'-bi phenyl-2-yl)palladium(II) (0.16 g, 0.2 mmol, 0.2 eq), and potassium tert-butoxide (0.224 g, 2.0 mmol, 2.0 eq) were dissolved in 1,4-dioxane (4 mL). The mixture was heated to 110 °C and reacted overnight.

After completion of the reaction, cold water (25 mL) was added to quench the reaction. The mixture was extracted with ethyl acetate (50 mL × 3). The organic phases were combined, dried over anhydrous sodium sulfate, filtered, and concentrated. The resulting residue was purified by silica gel column chromatography (eluent: ethyl acetate) to obtain 0.09 g of 2-(6-(((1S,3S)-3-((5-(difluoromethoxy)pyrimidin-2-yl)amino)cyclohexyl)amino)pyridin-3-yl)pyr idazin-3(2H)-one as a yellow solid (yield: 21%).

LC-MS: [M+H]+ = 430.

NMR data: H NMR (400 MHz, DMSO-$d_6$) δ 8.22 (s, 2H), 8.07 (d, J = 2.6 Hz, 1H), 8.01 (dd, J = 3.9, 1.6 Hz, 1H), 7.47 (ddd, J = 13.3, 9.2, 3.3 Hz, 2H), 7.34 (d, J = 7.8 Hz, 1H), 7.21 - 6.83 (m, 3H), 6.63 (d, J = 9.0 Hz, 1H), 4.23 (s, 1H), 4.09 (d, J = 6.1 Hz, 1H), 1.77 (d, J = 20.9 Hz, 3H), 1.65 (s, 3H), 1.52 (s, 1H), 1.37 (s, 1H).

Example J2

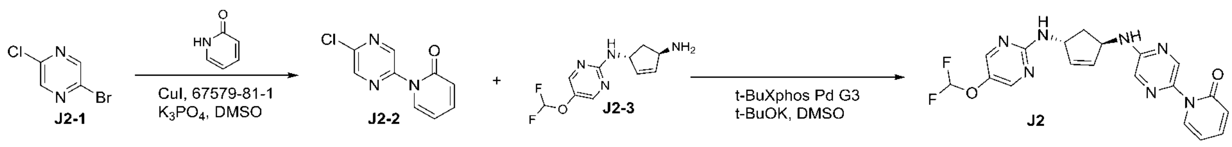


Step A: Synthesis of Compound J2-2

Compound J2-1 (1.92 g, 10 mmol) and pyridin-2(1H)-one (1.14 g, 12 mmol) were dissolved in dimethyl sulfoxide (20 mL). Potassium phosphate (4.2 g, 20 mmol), copper(I) iodide (0.19 g, 1 mmol), and trans-N,N'-dimethylcyclohexane-1,2-diamine (0.28 g, 2 mmol) were added. Under a nitrogen atmosphere, the mixture was heated to 110 °C and reacted for 16 hours. The reaction mixture was poured into saturated ammonium chloride aqueous solution and extracted with ethyl acetate (50 mL × 3). The organic phases were combined, dried and concentrated. The resulting material was purified by Flash silica gel column chromatography (0 to 50% ethyl acetate/petroleum ether) to obtain 500 mg of Compound J2-2 as a white solid (yield: 24%). LCMS: [M+H]+ = 208.

Step B: Synthesis of Target Compound

Compound J2-2 (100 mg, 0.48 mmol) and Compound J2-3 (130 mg, 0.48 mmol) were dissolved in dimethyl sulfoxide (2 mL). Potassium tert-butoxide (108 mg, 0.96 mmol) and (2-dicyclohexylphosphino-2',4',6'-triisopropyl-1,1'-biphenyl)[2-(2-aminoethyl)phenyl]palladium( II) methanesulfonate (76 mg, 0.1 mmol) were added. Under a nitrogen atmosphere, the mixture was heated to 110 °C and reacted for 16 hours. The reaction mixture was poured into saturated ammonium chloride aqueous solution and extracted with ethyl acetate (10 mL × 3). The organic phases were combined,

dried and concentrated. The resulting material was purified by Flash silica gel column chromatography (0 to 10% methanol/dichloromethane) to obtain 10 mg of the target compound as a pale yellow solid (yield: 5%).

LCMS: [M+H]+ = 414. H NMR (400 MHz, DMSO-d6) δ 8.33 (d, J = 2.2 Hz, 3H), 7.92 (d, J = 1.4 Hz, 1H), 7.78 (dd, J = 6.9, 2.1 Hz, 1H), 7.65 (t, J = 7.3 Hz, 2H), 7.57 (ddd, J = 9.0, 6.6, 2.1 Hz, 1H), 7.12 (s, 1H), 6.54 (d, J = 9.2 Hz, 1H), 6.39 (td, J = 6.6, 1.3 Hz, 1H), 6.07 (s, 2H), 5.17 (q, J = 6.7 Hz, 2H), 2.27 - 2.14 (m, 2H).

Example J3

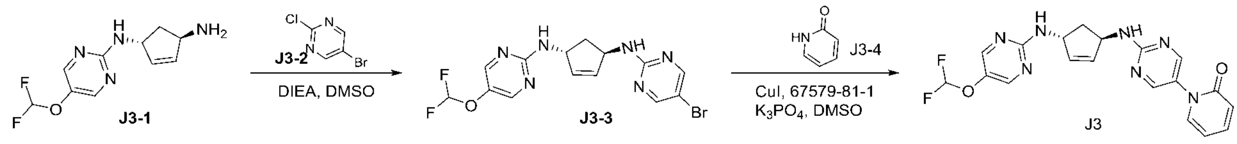


Step A: Synthesis of Compound J3-3

Compound J3-1 (200 mg, 0.83 mmol) was dissolved in dimethyl sulfoxide (2 mL). Compound J3-2 (190 mg, 0.99 mmol) and N,N-diisopropylethylamine (214 mg, 1.66 mmol) were added. The mixture was reacted at 80 °C for 2 hours. The reaction mixture was poured into saturated ammonium chloride aqueous solution and extracted with ethyl acetate (10 mL × 3). The organic phases were combined, dried and concentrated. The resulting material was purified by Flash column chromatography (0 to 5% methanol/dichloromethane) to obtain 130 mg of Compound J3-3as a yellow oil (yield: 39.4%). LCMS: [M+H]+ = 399/401.

Step B: Synthesis of Target Compound

Compound J3-3 (130 mg, 0.33 mmol) and Compound J3-4 (37 mg, 0.39 mmol) were dissolved in dimethyl sulfoxide (1 mL). Potassium phosphate (140 mg, 0.66 mmol), copper(I) iodide (6 mg, 0.03 mmol), and trans-N,N'-dimethylcyclohexane-1,2-diamine (9 mg, 0.06 mmol) were added. Under a nitrogen atmosphere, the mixture was heated to 110 °C and reacted for 16 hours. The reaction mixture was poured into saturated ammonium chloride aqueous solution and extracted with ethyl acetate (10 mL × 3). The organic phases were combined, dried and concentrated. The resulting material was purified by Flash column chromatography (0 to 10% methanol/dichloromethane) to obtain 5 mg of the target compound as a white solid (yield: 3.7%).

LCMS: [M+H]+ = 414. $^1$H NMR (400 MHz, DMSO-$d_6$) δ 8.34 (s, 2H), 8.26 (s, 2H), 7.73 (d, J = 7.6 Hz, 1H), 7.68 (dd, J = 6.8, 2.0 Hz, 1H), 7.56 (d, J = 7.6 Hz, 1H), 7.51 (ddd, J = 8.8, 6.6, 2.0 Hz, 1H), 7.06 (s, 1H), 6.51 - 6.44 (m, 1H), 6.32 (td, J = 6.6, 1.2 Hz, 1H), 5.96 (s, 2H), 5.14 (q, J = 6.4, 5.8 Hz, 1H), 5.10 - 5.03 (m, 1H), 2.14 (t, J = 6.0 Hz, 2H).

Example J4

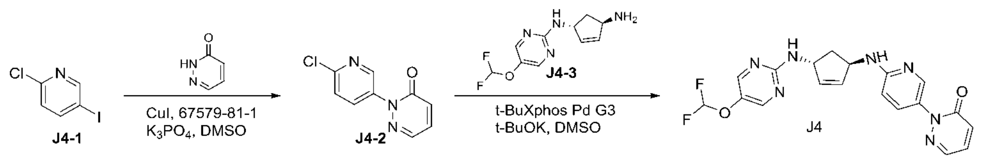


Step A: Synthesis of Compound J4-2

Compound J4-1 (3.6 g, 15 mmol) and pyridazin-3(2H)-one (1.7 g, 18 mmol) were dissolved in dimethyl sulfoxide (40 mL). Potassium phosphate (6.4 g, 30 mmol), copper(I) iodide (0.3 g, 1.5 mmol), and trans-N,N'-dimethylcyclohexane-1,2-diamine (0.43 g, 3 mmol) were added. Under a nitrogen atmosphere, the mixture was heated to 110 °C and reacted for 16 hours. The reaction mixture was poured into saturated ammonium chloride aqueous solution (100 mL) and extracted with ethyl acetate (50 mL × 3). The organic phases were combined, dried and concentrated. The resulting material was purified by Flash silica gel column chromatography (0 to 50% ethyl acetate/petroleum ether) to obtain 500 mg of Compound J4-2 as a white solid (yield: 16%). LCMS: [M+H]+ = 208.

Step B: Synthesis of Target Compound

Compound J4-2 (100 mg, 0.48 mmol) and Compound J4-3 (130 mg, 0.48 mmol) were dissolved in dimethyl sulfoxide (2 mL). Potassium tert-butoxide (108 mg, 0.96 mmol) and (2-dicyclohexylphosphino-2',4',6'-triisopropyl-1,1'-biphenyl)[2-(2-aminoethyl)phenyl]palladium( II) methanesulfonate (76 mg, 0.1 mmol) were added. Under a nitrogen atmosphere, the mixture was heated to 110 °C and reacted for 16 hours. The reaction mixture was poured into saturated ammonium chloride aqueous solution and extracted with ethyl acetate (10 mL × 3). The organic phases were combined, dried and concentrated. The resulting material was purified by Flash silica gel column chromatography (0 to 10% methanol/dichloromethane) to obtain 9.5 mg of the target compound as a yellow solid (yield: 5%).

LCMS: [M+H]+ = 414. H NMR (400 MHz, DMSO-$d_6$) δ 8.32 (s, 2H), 8.18 (d, J = 2.6 Hz, 1H), 8.08 (dd, J = 3.9, 1.6 Hz, 1H), 7.68 - 7.56 (m, 2H), 7.53 (dd, J = 9.5, 3.8 Hz, 1H), 7.18 - 7.01 (m, 2H), 6.60 (d, J = 8.9 Hz, 1H), 6.10 - 5.99 (m, 2H), 5.26 - 5.07 (m, 2H), 2.22 - 2.08 (m, 2H).

Examples J5 to J9

According to the aforementioned Compound preparation methods, Compounds J5 to J9 were prepared:

| | | | |
|---|---|---|---|
| J5 | 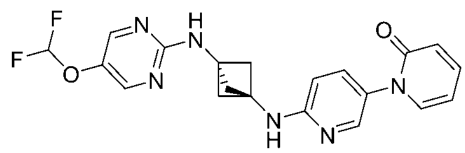 NMR: $^1$H NMR (400 MHz, DMSO-$d_6$) δ = 8.30 (s, 2H), 8.11 (s, 1H), 7.99 (d, *J* = 2.6 Hz, 1H), 7.62 (dd, *J* = 1.8, 6.8 Hz, 1H), 7.55 (s, 1H), 7.51 - 7.43 (m, 2H), 7.06 (t, *J* = 73.9 Hz, 1H), 6.59 (d, *J* = 8.9 Hz, 1H), 6.45 (d, *J* = 9.1 Hz, 1H), 6.28 (dt, *J* = 1.3, 6.7 Hz, 1H), 2.36 (s, 6H). | J6 | 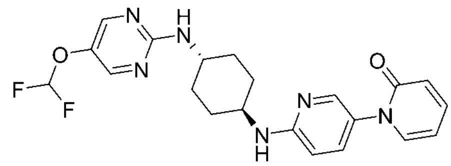 NMR: $^1$H NMR (400 MHz, DMSO-$d_6$) δ 8.24 (s, 2H), 7.92 (s, 1H), 7.60 (dd, *J* = 6.8, 2.1 Hz, 1H), 7.48 (ddd, *J* = 9.0, 6.6, 2.1 Hz, 1H), 7.39 (dd, *J* = 8.9, 2.6 Hz, 1H), 7.32 (d, *J* = 7.9 Hz, 1H), 7.04 (t, *J* = 74.0 Hz, 1H), 6.79 (d, *J* = 7.5 Hz, 1H), 6.52 (d, *J* = 8.9 Hz, 1H), 6.45 (d, *J* = 9.2 Hz, 1H), 6.34 - 6.17 (m, 1H), 3.68 (tt, *J* = 7.1, 3.4 Hz, 2H), 2.12 - 1.89 (m, 4H), 1.48 - 1.21 (m, 4H). |
| J7 | 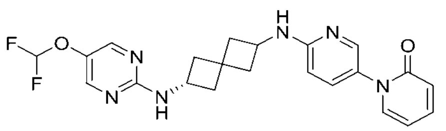 NMR: $^1$H NMR (400 MHz, DMSO-$d_6$) δ 8.24 (s, 2H), 7.93 (d, *J* = 2.6 Hz, 1H), 7.71 - 7.55 (m, 2H), 7.49 (ddd, *J* = 8.9, 6.6, 2.1 Hz, 1H), 7.42 (dd, *J* = 8.8, 2.7 Hz, 1H), 7.26 - 6.81 (m, 2H), 6.57 - 6.37 (m, 2H), 6.29 (td, *J* = 6.7, 1.4 Hz, 1H), 4.28 - 4.10 (m, 2H), 2.51 - 2.41 (m, 2H), 2.31 (dddd, *J* = 22.7, 12.0, 7.3, 5.0 Hz, 2H), 1.98 (dddd, *J* = 32.9, 19.7, 11.0, 8.5 Hz, 4H). | J8 | 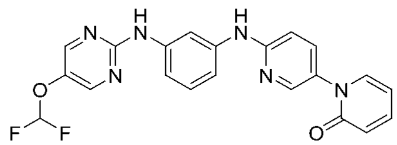 NMR: $^1$H NMR (400 MHz, DMSO-$d_6$) δ 9.81 - 9.76 (s, 1H), 9.30 - 9.25 (s, 1H), 8.50 - 8.45 (s, 2H), 8.18 - 8.12 (d, *J* = 2.8 Hz, 1H), 8.07 - 8.02 (t, *J* = 2.1 Hz, 1H), 7.72 - 7.61 (m, 2H), 7.57 - 7.49 (ddd, *J* = 9.0, 6.6, 2.1 Hz, 1H), 7.42 - 7.38 (dt, *J* = 8.0, 1.6 Hz, 1H), 7.35 - 7.14 (m, 3H), 7.00 - 6.93 (m, 1H), 6.53 - 6.48 (d, *J* = 9.2 Hz, 1H), 6.38 - 6.30 (td, *J* = 6.7, 1.4 Hz, 1H). |
| J9 | 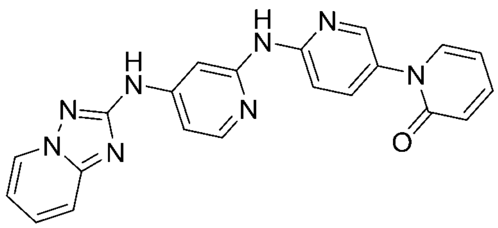 | NMR: $^1$H NMR (400 MHz, DMSO-$d_6$) δ 10.12 (s, 1H), 9.80 (s, 1H), 8.82 (d, *J* = 6.6 Hz, 1H), 8.28 (d, *J* = 2.6 Hz, 1H), 8.13 (d, *J* = 1.9 Hz, 1H), 8.07 (d, *J* = 5.7 Hz, 1H), 7.82 (d, *J* = 8.9 Hz, 1H), 7.77 - 7.69 (m, 2H), 7.63 (d, *J* = 6.8 Hz, 2H), 7.54 (ddd, *J* = 8.9, 6.6, 2.0 Hz, 1H), 7.30 (dd, *J* = 5.7, 2.0 Hz, 1H), 7.10 (td, *J* = 6.5, 2.0 Hz, 1H), 6.52 (d, *J* = 9.2 Hz, 1H), 6.36 (t, *J* = 6.6 Hz, 1H). | |

Example A74: Related Activity Test

Test Method:

Surface plasmon resonance data were collected on a Biacore™ 8K system (GE Healthcare) at 25 °C. At 25 °C, HBS-EP (10 mM HEPES, 0.15 M NaCl, 0.05% Tween-20, pH 7.4) was used as a running buffer, streptavidin was

immobilized on an SA (Cytiva) sensor chip using standard amine coupling chemistry. Briefly, the carboxymethyl dextran surface was activated by injecting a 1:1 ratio of 0.4 M 1-ethyl-3-(3-dimethylaminopropyl)carbodiimide hydrochloride (EDC)/0.1 M N-hydroxysuccinimide (NHS) for 12 min at a flow rate of 10 $\mu$L/min. To capture streptavidin, the protein was diluted to 0.5 mg/mL in 10 mM sodium acetate (pH 4.5) and 100 $\mu$L of the diluted protein was injected onto the activated chip surface for capture. Excess remaining active groups were blocked by injecting 1 M ethanolamine (pH 8.5) for 7 min. 5 $\mu$L protein was diluted to 10 $\mu$g/mL in NBS-N, 0.05% Tween, and 0.1 mM $CaCl_2$ at a flow rate of 5 $\mu$L/min for 60 s. The Aci-tagged PCSK9 protein was captured on the streptavidin surface. Typical surface densities obtained were 2900-3200 RU. SPR binding data were obtained using an appropriate dilution series of each compound at a flow rate of 30 $\mu$L/min, with an association time of 60 s per concentration point and a dissociation time of 3600 s. The running buffer used for compound binding studies was 10 mM HEPES, pH 7.4, 150 mM NaCl, 0.05% Tween-20, 2% DMSO. Data were corrected for DMSO excluding bulk effects. All data were double-referenced against blank injections and the reference surface using standard processing procedures. Data processing and kinetic fitting were performed using Scrubber software version 2.0c (BioLogic Software). Data were fitted using a simple 1:1 binding model to determine KD values.

Test Results:

$K_D$ values are shown in Table 1 below:

Table 1. PCSK9 Kinetics Data

| Examples | Kinetics $K_D$ (M) | Examples | Kinetics $K_D$ (M) | Examples | Kinetics $K_D$ (M) | Examples | Kinetics $K_D$ (M) |
|---|---|---|---|---|---|---|---|
| A1 | 8.78E-14 | A25 | 9.77E-10 | D8 | A | G1 | A |
| A2 | 1. 13E-09 | A28 | 2.11E-10 | D9 | A | G2 | A |
| A3 | 1.96E-09 | A29 | 4.98E-10 | D10 | A | G4 | A |
| A4 | 6.85E-09 | A31 | 1.48E-13 | D11 | A | G12 | A |
| A5 | 4.22E-11 | A34 | 1.02E-10 | D12 | A | G19 | A |
| A6A | 3.19E-12 | A39 | 7.07E-10 | E2 | A | J4 | A |
| A8 | 6.18E-11 | A42 | 1.82E-10 | E3 | A | | |
| A9 | 3.78E-11 | B1 | A | F1 | A | | |
| A10 | 3.62E-12 | B2 | A | F2 | A | | |
| AllA | 8.73E-11 | B3 | A | F3 | A | | |
| A12 | 1.26E-10 | B12 | A | F4 | A | | |
| A13 | 5.10E-13 | B13 | A | F5 | A | | |
| A14 | 9.01E-10 | C2 | A | F6 | A | | |
| A15 | 3.44E-10 | C3 | A | F7 | A | | |
| A17 | 3.34E-10 | D1 | A | F8/8A/8B | A | | |
| A18 | 4.14E-10 | D2 | A | F9 | A | | |
| A24 | 2.55E-10 | D7 | A | F19 | A | | |

A<1*E-8.

Example A75: Pharmacokinetic Experiment

1. Reagents and Instruments

Polyethylene glycol 400 (lot number R22040588, Shanghai Shaoyuan Reagent Co., Ltd.), DMSO (lot number 20200319, Guangdong Guanghua Sci-Tech Co., Ltd.), Sodium Chloride Physiological Solution (lot number 2011110727, Chenxin Pharmaceutical Co., Ltd.). LC-MS instrument (Thermo Fisher Ultimate 3000 UPLC, TSQ QUANTUM ULTRA triple quadrupole mass spectrometer, AB SCIEX 5500+ QTARP).

2. Experimental Animals

SD rats: Male, 180-250 g, purchased from Guangdong Vital River Laboratory Animal Technology Co., Ltd.

3. Formulation Preparation

The test compound powder was accurately weighed and completely dissolved in DMSO. PEG-400 was added, vortexed and sonicated to mix, then sodium chloride physiological solution was added, vortexed and sonicated to mix to form a 0.5 mg/mL solution (DMSO:PEG-400:NS = 5:60:35, V/V/V). For oral gavage administration, the dose was 10 mL/kg; for intravenous administration, the dose was 2 mL/kg.

4. Blood Sample Collection

After intravenous or oral gavage administration to rats, venous blood (200 μL) was collected at 5 min (not collected for oral gavage administration), 15 min, 30 min, 1 h, 2 h, 5 h, 7 h, and 24 h into EDTA-K2-anticoagulated EP tubes. The samples were centrifuged at 10000 rpm for 2 min, and the plasma was collected and stored at -80 °C until analysis.

5. Bioanalysis

A certain amount of the test compound was accurately weighed and dissolved in DMSO to 2 mg/mL as a stock solution. An appropriate volume of the compound stock solution was accurately transferred and diluted with acetonitrile to prepare a series of standard solutions. 4 μL of each standard solution was accurately transferred, and 36 μL of blank plasma was added, vortexed to mix, to prepare plasma samples equivalent to plasma concentrations of 1, 3, 5, 10, 30, 100, 300, 1000, 3000 ng/mL. Each concentration was analyzed in duplicate to establish a standard curve. 30 μL of plasma was taken, and 200 μL of an acetonitrile solution containing internal standard propranolol (5 ng/mL) was added. After vortex mixing, the samples were centrifuged at 4000 rpm for 10 min, and the supernatant was taken for LC-MS analysis. The LC-MS detection conditions were as follows:
Column: YMC-Triart C18, 50×2.1 mm, S-3μm, 12 nm.

Mobile phase A: water (0.1% formic acid), mobile phase B: acetonitrile, flow rate: 0.5 mL/min, gradient elution as shown in Table 2 below:

Table 2

| Time (min) | A(%) | B(%) |
|---|---|---|
| 0 | 80% | 20% |
| 1.2 | 20% | 80% |
| 2.6 | 20% | 80% |
| 2.61 | 80% | 20% |
| 3.0 | 80% | 20% |

6. Data Processing

After detecting plasma concentrations by LC-MS, pharmacokinetic parameters in rats after administration were calculated using a non-compartmental model with WinNonlin 6.1 software. The results are shown in Table 3 below.

Table 3: Pharmacokinetic Parameters of the Compound of the Present Disclosure in Rats (iv and PO Administration)

| Compound No. | Route of Administration and Dose (mg/kg) | t1/2 (h) | AUC0-24h (h*ng/mL) |
|---|---|---|---|
| A9 | PO, 5 | / | 29400 |
| A13 | PO, 5 | 6.02 | 42500 |
| A14 | PO, 5 | 4.42 | 31400 |
| A17 | PO, 5 | 2.97 | 43800 |
| A27 | PO, 5 | 3.89 | 32000 |
| A28 | PO, 5 | / | 70100 |

(continued)

| Compound No. | Route of Administration and Dose (mg/kg) | t1/2 (h) | AUC0-24h (h*ng/mL) |
|---|---|---|---|
| A35 | PO, 5 | 14.9 | 60100 |
| C9 | 5, PO | / | 40300 |
| C17 | 5, PO | / | 39800 |
| C22 | 5, PO | / | 37700 |
| C34 | 5, PO | / | 41400 |
| C35 | 5, PO | / | 39700 |
| C47 | 5, PO | / | 53000 |

The half-life and in vivo exposure of the compound of the present disclosure were improved compared to the control compound, and superior to Compound 458B and 464 of CN113574055A, as well as Compound C14.

Example A76: Effect on hERG Current in hERG-HEK293 Cells

Test Method: Human embryonic kidney cells stably expressing the hERG channel (hERG-HEK293 cells) were used for the experiment. An automated patch-clamp system was used to clamp hERG-HEK293 cells to establish whole-cell voltage-clamp mode, and corresponding voltages were applied to elicit hERG currents. Cells were perfused with extracellular solution containing 0.3% DMSO (negative control), 30 μM compound, or 1, 10, 100, and 1000 nM Cisapride (positive control). The hERG channel tail currents were recorded, and the peak tail current at each concentration was obtained. Using the peak tail current recorded under the negative control (0.3% DMSO) as 100%, the inhibition rates of hERG current by 30 μM compound and different concentrations of Cisapride were calculated. The concentration-response curve fitting and $IC_{50}$ calculation for Cisapride were performed using GraphPad Prism software. The results are shown in Table 4.

Table 4: Effect on hERG Current in hERG-HEK293 Cells

| Compound No. | 30 uM-hERG Inhibition |
|---|---|
| A22 | 19.56 |
| A27 | 40.72 |
| A30 | 33.75 |
| A35 | 33.81 |

| Compound No. | hERG Inhibition (%) | Compound No. | hERG Inhibition (%) | Compound No. | hERG Inhibition (%) |
|---|---|---|---|---|---|
| C7 | <50% | C30 | <50% | C58 | <50% |
| C8 | <50% | C32 | <50% | C61 | <50% |
| C12 | <50% | C34 | <50% | C64 | <50% |
| C13 | <50% | C35 | <50% | C65 | <50% |
| C15 | <50% | C36 | <50% | C66 | <50% |
| C17 | <50% | C43 | <50% | C67 | <50% |
| C19 | <50% | C47 | <50% | C69 | <50% |
| C21 | <50% | C48 | <50% | C71 | <50% |
| C22 | <50% | C53 | <50% | C84 | <50% |
| C26 | <50% | C56 | <50% | | |

The hERG risk of the compound of the present disclosure was improved compared to the control compound, and

superior to the control compound 458B of CN113574055A.

Example A77: Binding Affinity Test of Compound with PCSK9 Protein

The binding affinity of the compounds of the present disclosure to the PCSK9 protein was determined using a fluorescence polarization assay.

All compounds were dissolved in DMSO to prepare 10 mM stock solutions. Positive control compounds and test compounds were serially diluted from 10 mM with DMSO using 5-fold serial dilutions, resulting in 8 concentration gradients. First, a certain volume of fluorescent probe solution was prepared using assay buffer (20 mM HEPES, 150 mM NaCl, 1 mM $CaCl_2$, and 0.01% Tween-20) to achieve a concentration of 5 nM. Then, the series of DMSO solutions of the test compounds were diluted 50-fold with the fluorescent probe solution. Finally, a human recombinant PCSK9 protein (ACRO, Cat# PC9-H5223) solution at a concentration of 4.5 μg/mL was prepared using assay buffer. After preparation of the test solutions, 5 μL of PCSK9 protein was added to a black 384-well plate (PerkinElmer, Cat# 6008260), followed by the addition of 5 μL of different concentrations of compound (final DMSO concentration of 1%). Positive control groups (assay buffer + equal volume of target protein + proportional fluorescent probe molecule) and negative control groups (assay buffer + proportional fluorescent probe molecule) were set up simultaneously. The final concentration of the probe molecule in the system was 2.5 nM, and the final concentration of PCSK9 protein was 2.25 μg/mL. After shaking and incubating at room temperature for 15 minutes, the fluorescence polarization (mP) values were read using a microplate reader at an excitation wavelength of 485 nm and an emission wavelength of 520 nm. The inhibition rate of the drug = [1 - $(mP_{(drug\ screening\ group)} - mP_{(negative\ control\ group)})] \div [mP_{(positive\ control\ group)} - mP_{(negative\ control\ group)})] \times 100$. Using the logarithm of the compound concentration as the x-axis and the inhibition rate as the y-axis, a 4-parameter nonlinear regression curve was fitted to calculate the $IC_{50}$ value (Y=Bottom + (Top-Bottom)/(1+10^((Log$IC_{50}$-X)*HillSlope)), where: HillSlope represents the slope of the curve, and $IC_{50}$ represents the half-maximal inhibitory concentration.

Table 5. FP-$IC_{50}$ Data

| Example | FP-$IC_{50}$ (nm) | Example | FP-$IC_{50}$ (nm) | Example | FP-$IC_{50}$ (nm) |
|---|---|---|---|---|---|
| A17-D | B | B4 | B | B26* | B |
| A43 | B | B6 | B | B27 | B |
| A43A | B | B7 | B | B28 | B |
| A43B | B | B8 | B | B29 | B |
| A43A-D | B | B9* | B | B30 | B |
| A43B-D | B | B10 | B | B31* | B |
| A51 | B | B11 | B | B32* | B |
| A52 | B | B24 | B | B33* | B |
| C3 | B | C49 | B | C80 | B |
| C7 | B | C50 | B | C81 | B |
| C8 | B | C51 | B | C82 | B |
| C12A | B | C52 | B | C83 | B |
| C12B | B | C53 | B | C84 | B |
| C13A | B | C55 | B | C85 | B |
| C13B | B | C57 | B | C86 | B |
| C15 | B | C58 | B | C87 | B |
| C17 | B | C59 | B | C88 | B |
| C17A | B | C60 | B | C89 | B |
| C19 | B | C61 | B | C90 | B |
| C20 | B | C62 | B | C91* | B |
| C21 | B | C63 | B | C92* | B |
| C22 | B | C64 | B | C93* | B |

(continued)

| Example | FP-$IC_{50}$ (nm) | Example | FP-$IC_{50}$ (nm) | Example | FP-$IC_{50}$ (nm) |
|---|---|---|---|---|---|
| C24 | B | C65 | B | C94* | B |
| C25 | B | C66 | B | C95* | B |
| C26 | B | C67 | B | D5 | B |
| C31 | B | C68 | B | D6 | B |
| C32 | B | C69 | B | D7 | B |
| C33 | B | C70 | B | D15 | B |
| C34 | B | C71 | B | D20 | B |
| C35 | B | C72 | B | D22 | B |
| C44 | B | C77 | B | E16 | B |
| C47 | B | C78 | B | E18* | B |
| C48 | B | C79 | B | | |
| F10 | B | F24 | B | F35 | B* |
| F11 | B | F25 | B | F36 | B* |
| F12 | B | F26 | B | F37 | B* |
| F13 | B | F27 | B | H2 | B |
| F14 | B | F28 | B | H5 | B |
| F15 | B | F29 | B | H8 | B |
| F16 | B | F30 | B* | H9 | B |
| F17 | B | F31 | B* | H12 | B |
| F18 | B | F32 | B* | H14 | B |
| F22 | B | F33 | B* | | |
| F23 | B | F34 | B* | | |
| B < 300 nm. * Incubated for 18 h. | | | | | |

The above examples are preferred embodiments of the present disclosure, but the embodiments of the present disclosure are not limited by the above examples. Any other changes, modifications, substitutions, combinations, and simplifications made without departing from the spirit and principle of the present disclosure shall be considered as equivalent substitution methods and are included within the protection scope of the present disclosure.

## Claims

1. A compound represented by general formula (A), or an isomer thereof, or a racemate thereof, or a pharmaceutically acceptable salt thereof, wherein

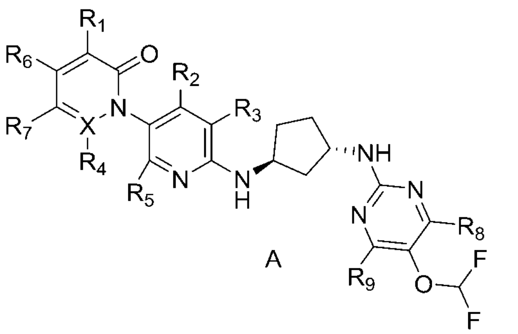

A

X is C or N, and when X is N, $R_4$ is absent;

$R_1$, $R_4$, $R_6$, and $R_7$ are selected from the group consisting of hydrogen, halogen, hydroxyl, substituted or unsubstituted alkyl, cyano, -C(O)-O-alkyl, phenylalkoxy, carboxyl, hydroxymethyl, cycloalkyl, amide, alkyl-substituted amide, Z-C(O)-, and heteroaryl, Z being selected from the group consisting of alkyl and heterocycloalkyl, and a substituent being hydroxyl;

$R_2$, $R_3$, and $R_5$ are each selected from the group consisting of hydrogen, alkyl, alkoxy, cyano, halogen, haloalkyl, amide, hydroxyl, hydroxyl-substituted alkyl, carboxyl, amino, amino-substituted alkyl, alkylcarbonyl, and $-(CH_2)_n-O-(CH_2)_n$-phenyl, and n = 1, 2, or 3;

$R_8$ and $R_9$ are each selected from the group consisting of hydrogen, alkyl, halogen, cycloalkyl, alkylthio, alkoxy, aryl, and heteroaryl; and

$R_1$, $R_2$, $R_3$, $R_4$, $R_5$, $R_6$, and $R_7$ are not all hydrogen simultaneously; or when $R_1$, $R_2$, $R_3$, $R_4$, $R_5$, $R_6$, and $R_7$ are all hydrogen simultaneously, then $R_8$ and $R_9$ are not both hydrogen simultaneously.

**2.** The compound, or the isomer thereof, or the racemate thereof, or the pharmaceutically acceptable salt thereof according to claim 1, wherein the compound, or the isomer thereof, or the racemate thereof, or the pharmaceutically acceptable salt thereof is a compound represented by general formula (A1), or an isomer thereof, or a racemate thereof, or a pharmaceutically acceptable salt thereof:

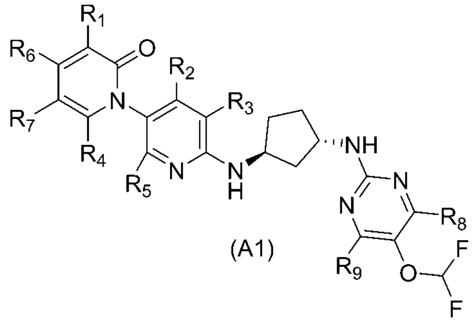

(A1)

$R_1$, $R_4$, $R_6$, and $R_7$ are selected from the group consisting of hydrogen, halogen, hydroxyl, substituted or unsubstituted alkyl, cyano, -C(O)-O-alkyl, phenylalkoxy, carboxyl, hydroxymethyl, cycloalkyl, amide, alkyl-substituted amide, Z-C(O)-, and heteroaryl, Z being selected from the group consisting of alkyl and heterocycloalkyl, and a substituent being hydroxyl;

$R_2$, $R_3$, and $R_5$ are each selected from the group consisting of hydrogen, alkyl, alkoxy, cyano, halogen, haloalkyl, amide, hydroxyl, hydroxyl-substituted alkyl, carboxyl, amino, amino-substituted alkyl, alkylcarbonyl, and $-(CH_2)_n-O-(CH_2)_n$-phenyl, and n = 1, 2, or 3;

$R_8$ and $R_9$ are each selected from the group consisting of hydrogen, alkyl, halogen, cycloalkyl, alkylthio, alkoxy, aryl, and heteroaryl; and

$R_1$, $R_2$, $R_3$, $R_4$, $R_5$, $R_6$, and $R_7$ are not all hydrogen simultaneously; or when $R_1$, $R_2$, $R_3$, $R_4$, $R_5$, $R_6$, and $R_7$ are all hydrogen simultaneously, then $R_8$ and $R_9$ are not both hydrogen simultaneously.

**3.** The compound, or the isomer thereof, or the racemate thereof, or the pharmaceutically acceptable salt thereof according to claim 1 or 2, wherein $R_1$, $R_2$, $R_4$, $R_5$, $R_6$, and $R_7$ are all hydrogen simultaneously, and $R_3$ is selected from the group consisting of alkyl, alkoxy, cyano, halogen, haloalkyl, amide, hydroxyl, hydroxyl-substituted alkyl, carboxyl, amino, amino-substituted alkyl, alkylcarbonyl, and $-(CH_2)_n-O-(CH_2)_n$-phenyl, and n = 1, 2, or 3; or
$R_1$, $R_3$, $R_4$, $R_5$, $R_6$, and $R_7$ are all hydrogen simultaneously, and $R_2$ is selected from the group consisting of alkyl, alkoxy, cyano, halogen, haloalkyl, amide, hydroxyl, hydroxyl-substituted alkyl, carboxyl, amino, amino-substituted alkyl, alkylcarbonyl, and $-(CH_2)_n-O-(CH_2)_n$-phenyl, and n = 1, 2, or 3.

**4.** The compound, or the isomer thereof, or the racemate thereof, or the pharmaceutically acceptable salt thereof according to claim 1 or 2, wherein

the alkyl is selected from the group consisting of methyl, ethyl, n-propyl, isopropyl, n-butyl, isobutyl, sec-butyl, tert-butyl, n-pentyl, sec-pentyl, 1-ethylpropyl, 2-methylbutyl, tert-pentyl, 1,2-dimethylpropyl, isopentyl, neopentyl, n-hexyl, isohexyl, sec-hexyl, tert-hexyl, neohexyl, 2-methylpentyl, 1,2-dimethylbutyl, and 1-ethylbutyl; the haloalkyl refers to alkyl in which one or more hydrogens are substituted by halogen; the hydroxyl-substituted alkyl refers to alkyl in which one or more hydrogens are substituted by hydroxyl; the halogen is selected from the group

consisting of fluorine, chlorine, bromine, and iodine; and
the cycloalkyl is selected from the group consisting of cyclopropyl, cyclobutyl, cyclopentyl, and cyclohexyl; the heterocycloalkyl refers to cycloalkyl in which one or more carbon atoms are replaced by heteroatoms, the heteroatoms being selected from the group consisting of N, O, and S; the alkoxy is selected from the group consisting of methoxy, ethoxy, n-propoxy, and isopropoxy.

**5.** The compound, or the isomer thereof, or the racemate thereof, or the pharmaceutically acceptable salt thereof according to claim 1 or 2, wherein $R_2$ or $R_3$ is selected from the group consisting of F, Cl, Br, methyl, hydroxyl, hydroxymethyl, trifluoromethyl, $-CF_2H$, $-C(O)NH_2$, $-NH_2$, cyano, -COOH, methoxy, $-CH_2-O-CH_2$-phenyl,

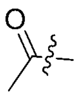 , 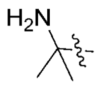 , and 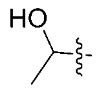 ;

and/or

$R_1$ is selected from the group consisting of methyl, hydroxyl, hydroxymethyl, cyano, F, Cl, Br, $-O-CH_2$-phenyl, -COOH, $-COOCH_2CH_3$,

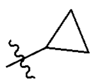 , 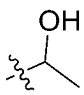 ,

amide, formamide, $CH_3-C(O)-$,

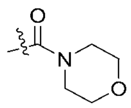 , 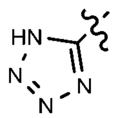 , and 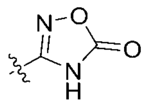 ;

and/or
$R_6$ and $R_7$ are independently H or -COOH; and/or $R_4$, $R_6$, and $R_7$ are independently H; and/or $R_8$ and $R_9$ are independently selected from the group consisting of hydrogen, chlorine, methyl, methoxy, methylthio, ethyl, isopropyl, phenyl, cyclopropyl, and

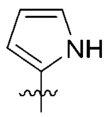 .

**6.** The compound, or the isomer thereof, or the racemate thereof, or the pharmaceutically acceptable salt thereof according to claim 1 or 2, wherein the compound, or the isomer thereof, or the racemate thereof, or the pharmaceutically acceptable salt thereof is selected from the group consisting of:

| No. | Compound Structure | No. | Compound Structure | No. | Compound Structure |
|---|---|---|---|---|---|
| A1 | 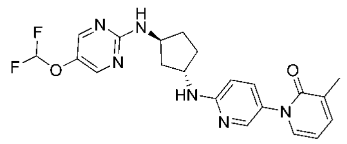 | A5 | 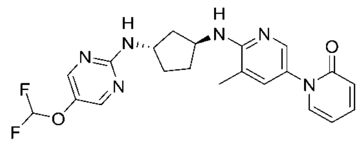 | A6 | 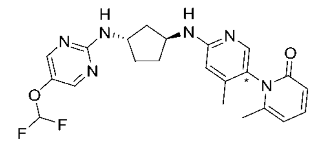 |
| A7 | 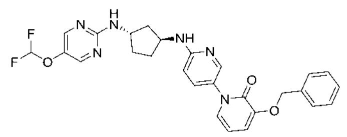 | A8 | 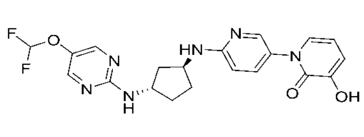 | A9 | 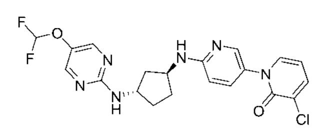 |
| A10 | 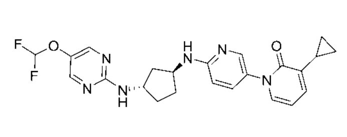 | A11 | 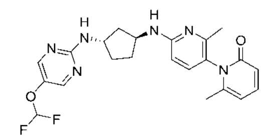 | A12 | 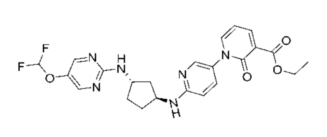 |
| A13 | 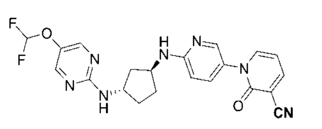 | A14 | 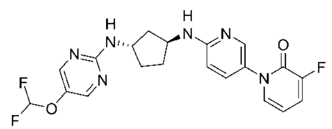 | A15 | 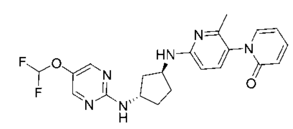 |
| A16 | 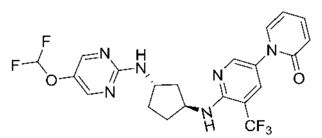 | A17 | 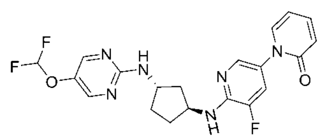 | A18 | 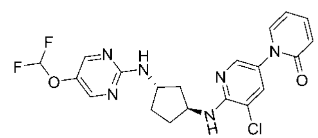 |
| A19 | 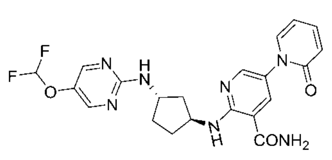 | A20 | 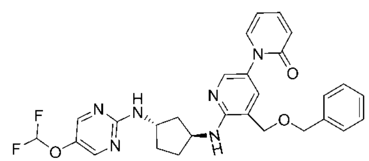 | A21 | 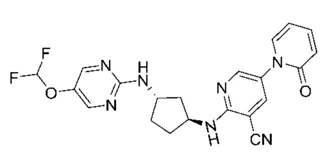 |
| A22 | 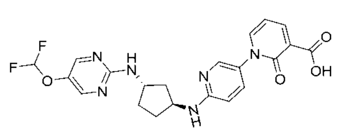 | A23 | 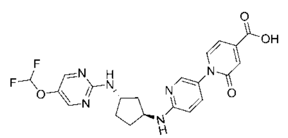 | A24 | 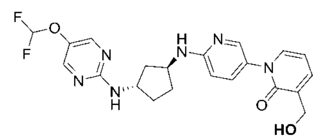 |

(continued)

| No. | Compound Structure | No. | Compound Structure | No. | Compound Structure |
|---|---|---|---|---|---|
| A25 | 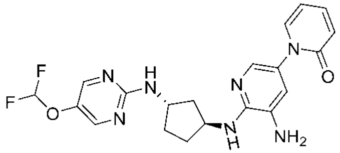 | A26 | 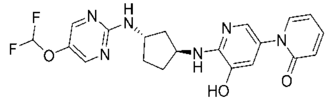 | A27 | 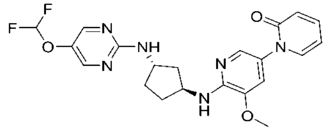 |
| A28 | 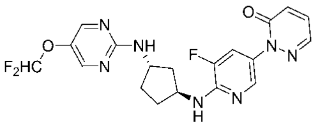 | A29 | 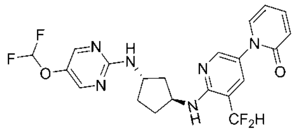 | A30 | 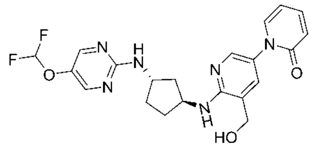 |
| A31 | 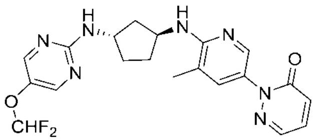 | A32 | 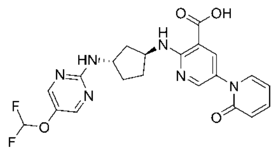 | A33 | 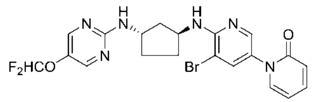 |
| A35 | 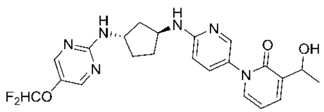 | A36 | 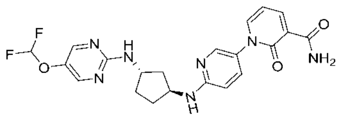 | A37 | 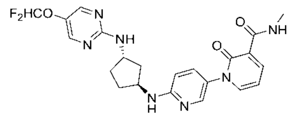 |
| A38 | 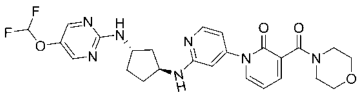 | A39 | 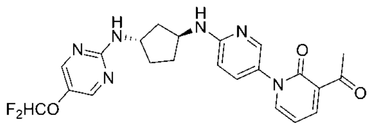 | A40 | 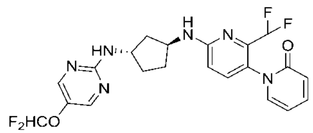 |
| A41 | 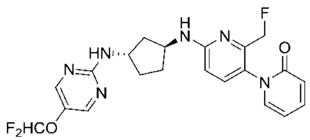 | A42 | 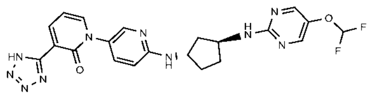 | A43 | 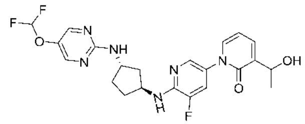 |

(continued)

| No. | Compound Structure | No. | Compound Structure | No. | Compound Structure |
|---|---|---|---|---|---|
| A44 | 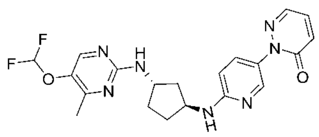 | A45 | 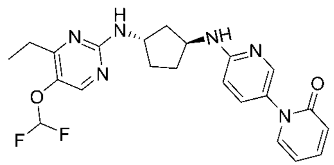 | A46 | 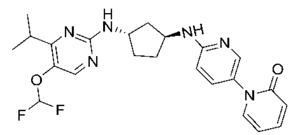 |
| A47 | 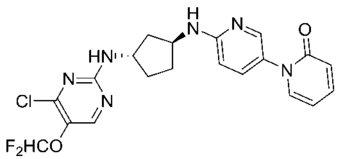 | A48 | 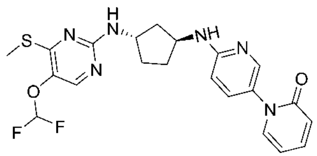 | A49 | 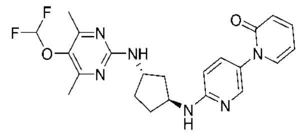 |
| A50 | 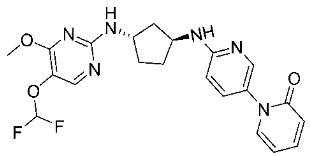 | A51 | 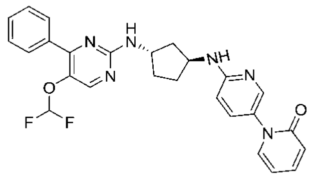 | A52 | 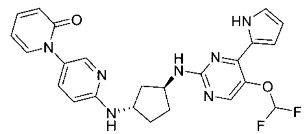 |
| A53 | 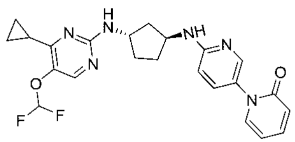 | A54 | 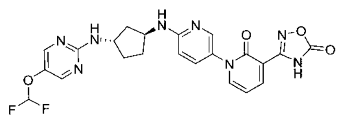 | A55 | 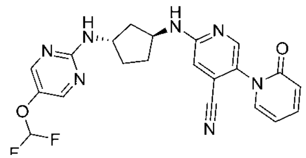 |
| A56 | 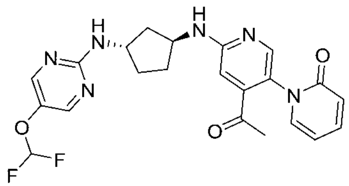 | A57 | 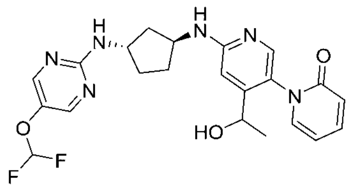 | A58 | 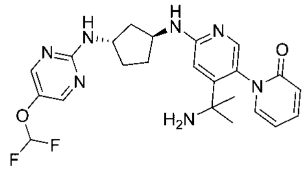 |

(continued)

| No. | Compound Structure | No. | Compound Structure | No. | Compound Structure |
|---|---|---|---|---|---|
| A59 | 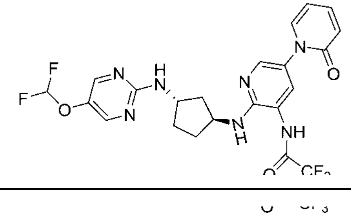 | A60 | 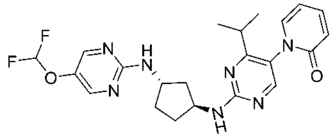 | A61 | 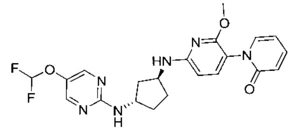 |
| A62 | 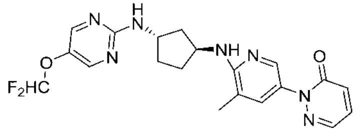 | A63 | 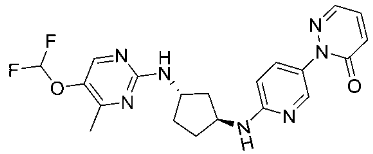 | A64 | 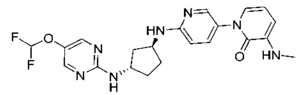 |
| A65 | 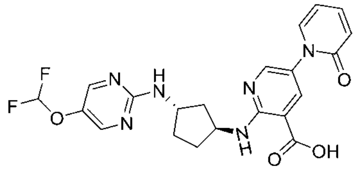 | A66 | 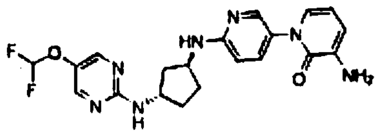 | A67 | 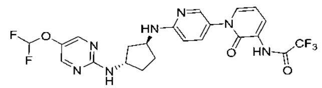 |
| A68 | 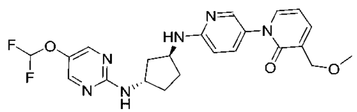 | A69 | 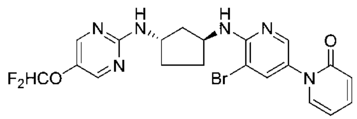 | A70 | 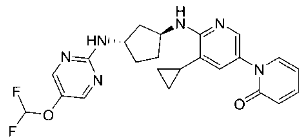 |
| A71 | 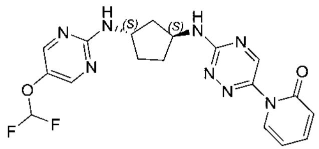 | A72 | 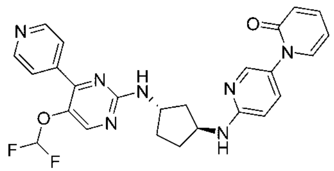 | A73 | 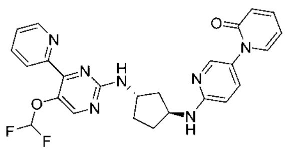 |

**7.** A compound represented by general formula (B), or an isomer thereof, or a racemate thereof, or a pharmaceutically acceptable salt thereof, wherein

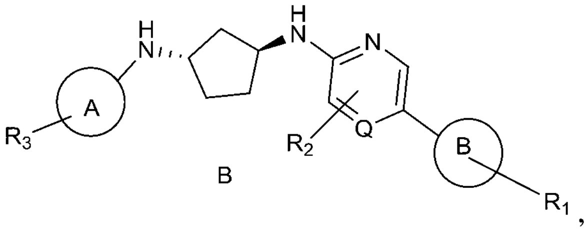

B

A is selected from the group consisting of

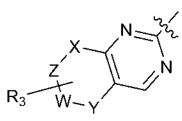, 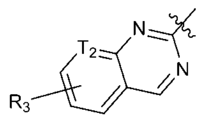, 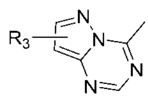,

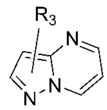, and 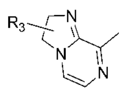;

B is selected from the group consisting of

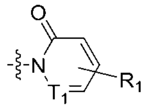, 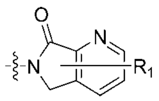, 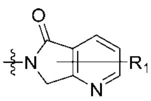,

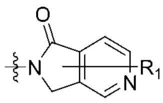, 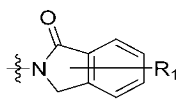, 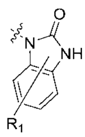, 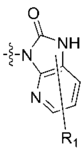, 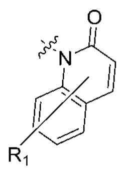, and 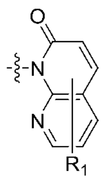;

Q is selected from the group consisting of N and $CR_4$, $R_4$ being selected from the group consisting of H and halogen;
$T_1$ and $T_2$ are selected from the group consisting of N and CH;
X and Y are independently selected from the group consisting of C(O), O, S, NH, and $CH_2$,
Z is absent, or Z is selected from the group consisting of C(O), O, S, NH, and $CH_2$,
W is selected from the group consisting of C(O), O, S, NH, and $CH_2$,
$R_1$ is hydrogen, or $R_1$ represents that the hydrogen on a ring is further substituted by alkyl, haloalkyl, carboxyl, alkoxy, haloalkoxy, cyano, halogen, or

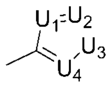;

$U_1$, $U_2$, and $U_4$ are independently selected from the group consisting of CH and N; $U_3$ is selected from the group consisting of $CH_2$ and NH; and a number of $R_1$ is one or more;
$R_2$ is selected from the group consisting of H, alkyl, and halogen; and a number of $R_2$ is one or more; and
$R_3$ is hydrogen, or $R_3$ represents that the hydrogen on a ring is further substituted by alkyl, halogen, oxo, substituted or unsubstituted phenyl, a substituent of the substituted phenyl being selected from the group consisting of alkyl and halogen; and a number of $R_3$ is one or more.

**8.** The compound, or the isomer thereof, or the racemate thereof, or the pharmaceutically acceptable salt thereof according to claim 7, wherein

the alkyl is selected from the group consisting of methyl, ethyl, n-propyl, isopropyl, n-butyl, isobutyl, sec-butyl, tert-butyl, n-pentyl, sec-pentyl, 1-ethylpropyl, 2-methylbutyl, tert-pentyl, 1,2-dimethylpropyl, isopentyl, neopentyl, n-hexyl, isohexyl, sec-hexyl, tert-hexyl, neohexyl, 2-methylpentyl, 1,2-dimethylbutyl, and 1-ethylbutyl;
the alkoxy is $C_{1-6}$ alkoxy, the $C_{1-6}$ alkoxy is selected from the group consisting of methoxy, ethoxy, n-propoxy, isopropoxy, n-butoxy, isobutoxy, sec-butoxy, tert-butoxy, n-pentoxy, sec-pentoxy, 1-ethylpropoxy, 2-methylbutoxy, tert-pentoxy, 1,2-dimethylpropoxy, isopentoxy, neopentoxy, n-hexoxy, isohexoxy, sec-hexoxy, tert-hexoxy, neohexoxy, 2-methylpentoxy, 1,2-dimethylbutoxy, and 1-ethylbutoxy; and the halogen is selected from the group consisting of fluorine, chlorine, bromine, and iodine.

**9.** The compound, or the isomer thereof, or the racemate thereof, or the pharmaceutically acceptable salt thereof according to claim 7, wherein Q is N; and/or Q is CH, and $R_2$ is F; and/or $T_1$ is N.

**10.** The compound, or the isomer thereof, or the racemate thereof, or the pharmaceutically acceptable salt thereof according to claim 7, wherein a moiety

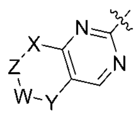

in general formula (B) is selected from the group consisting of:

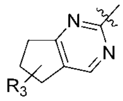, 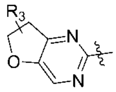, 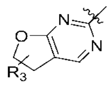,

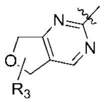, 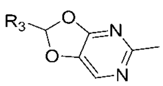, 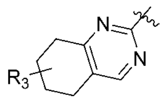, 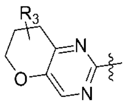, 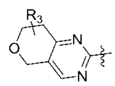, 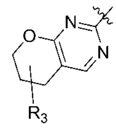, 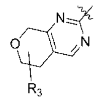, 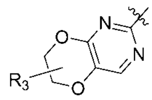,

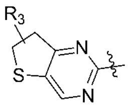, 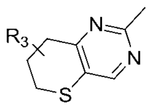, 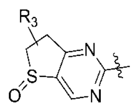, 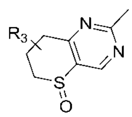, 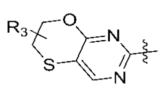, 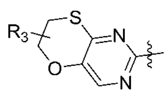, 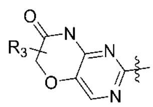,

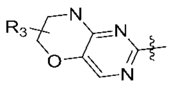, 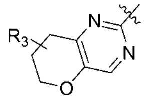, 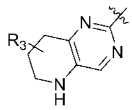, and 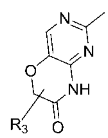,

and 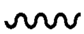 represents a connection site;
a moiety

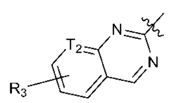

in general formula (B) is selected from the group consisting of:

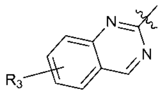 and 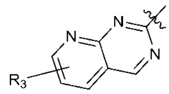,

and 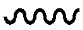 represents a connection site.

**11.** The compound, or the isomer thereof, or the racemate thereof, or the pharmaceutically acceptable salt thereof according to claim 7, wherein $R_3$ is hydrogen, methyl, F, phenyl, or

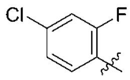,

and 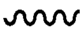 represents a connection site; and/or
A is selected from the group consisting of

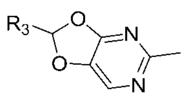 and 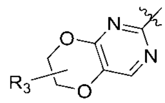;

and/or B is selected from the group consisting of

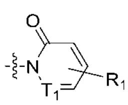, 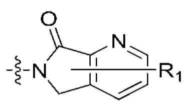, 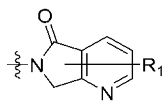, 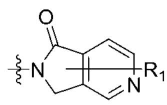,

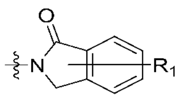, 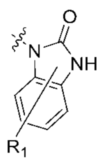, 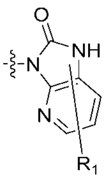, 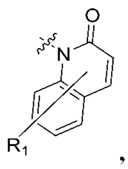, and 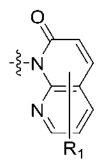; and/or 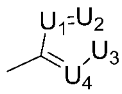

is

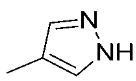.

**12.** The compound, or the isomer thereof, or the racemate thereof, or the pharmaceutically acceptable salt thereof according to claim 7, wherein the compound, or the isomer thereof, or the racemate thereof, or the pharmaceutically acceptable salt thereof is selected from the group consisting of:

| No. | Compound | No. | Compound | No. | Compound |
|---|---|---|---|---|---|
| B1 | 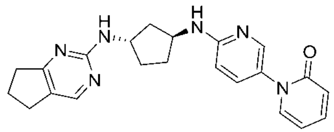 | B2 | 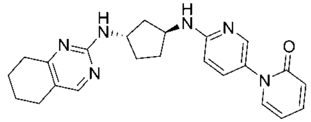 | B3 | 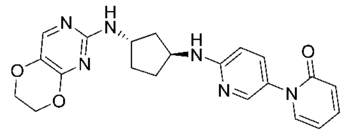 |
| B4 | 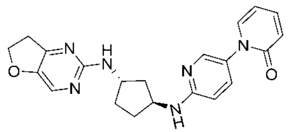 | B5 | 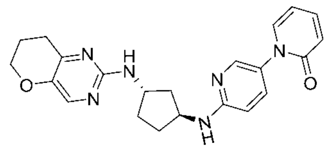 | B6 | 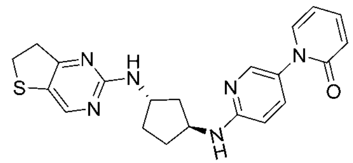 |
| B7 | 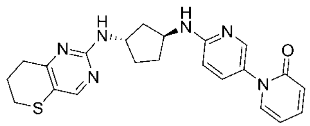 | B8 | 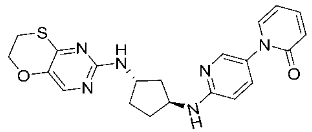 | B9 | 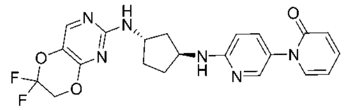 |
| B10 | 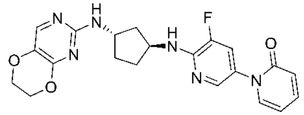 | B11 | 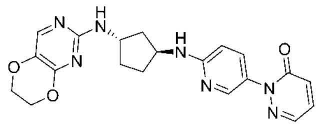 | B12 | 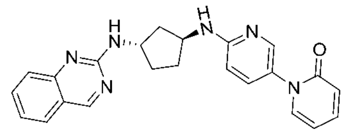 |
| B13 | 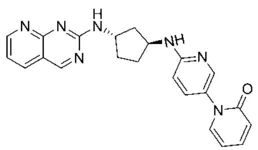 | B14 | 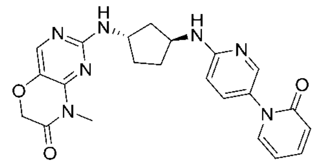 | B15 | 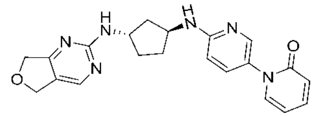 |
| B16 | 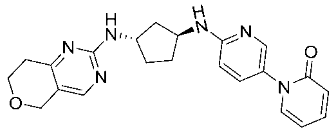 | B17 | 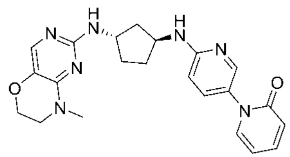 | B18 | 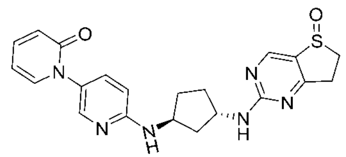 |

(continued)

| No. | Compound | No. | Compound | No. | Compound |
|---|---|---|---|---|---|
| B19 | 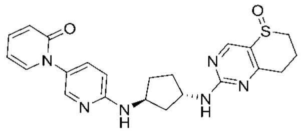 | B20 | 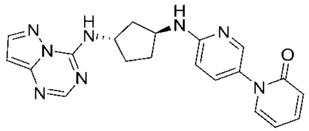 | B21 | 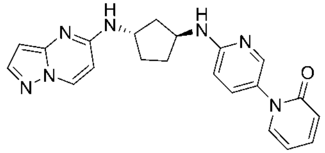 |
| B22 | 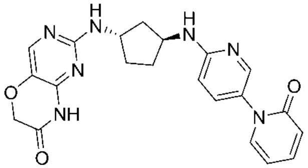 | B23 | 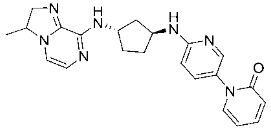 | B24 | 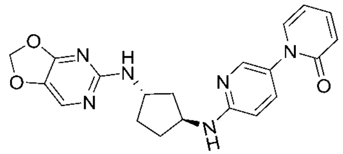 |
| B25 | 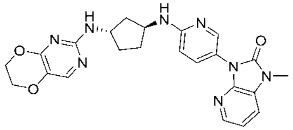 | B26 | 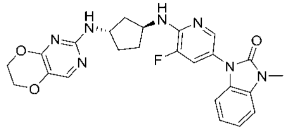 | B27 | 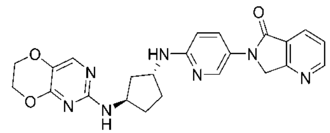 |
| B28 | 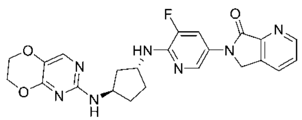 | B29 | 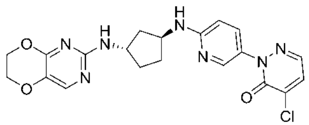 | B30 | 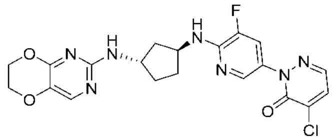 |
| B31 | 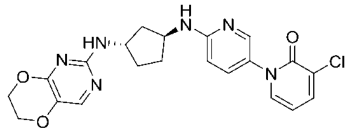 | B32 | 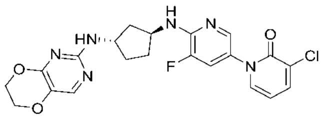 | B33 | 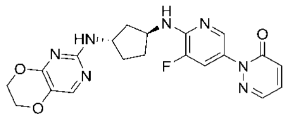 |
| B34 | 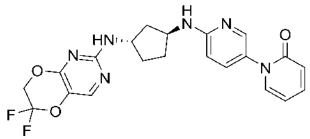 | | | | |

**13.** A compound represented by general formula (C), or an isomer thereof, or a racemate thereof, or a pharmaceutically acceptable salt thereof, wherein

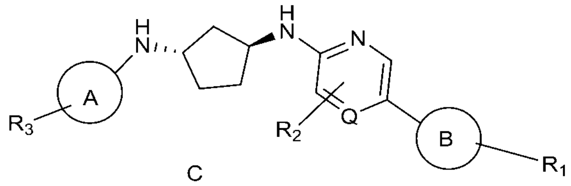

C ,

ring A is selected from the group consisting of

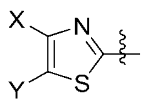 , 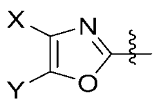 , 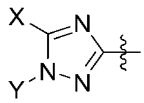 ,

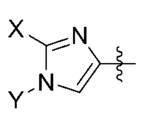 , 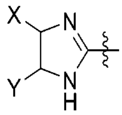 , and 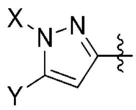 ,

and X and Y together form a 5- to 7-membered saturated or unsaturated ring, the 5- to 7-membered saturated or unsaturated ring comprises 0, 1, or 2 heteroatoms, the heteroatoms being selected from the group consisting of O, N, and S; B is selected from the group consisting of

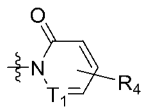 , 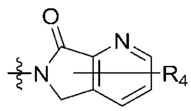 , 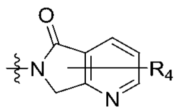 , 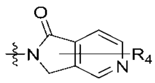 ,

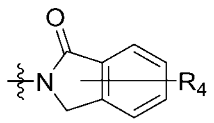 , 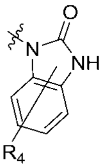 , 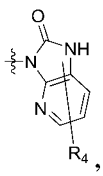 , 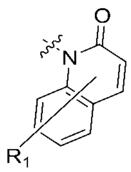 , and 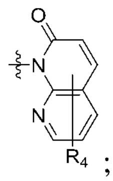 ;

Q is selected from the group consisting of N and $CR_{Q1}$, $R_{Q1}$ being selected from the group consisting of H and halogen;

$T_1$ is selected from the group consisting of N and CH;

$R_2$ is selected from the group consisting of H, alkyl, and halogen, and a number of $R_2$ is one or more;

$R_3$ is hydrogen, or $R_3$ represents that the hydrogen on ring A is further substituted by oxo, alkyl, halogen, alkoxy, alkylthio, haloalkyl, haloalkoxy, cycloalkyl, cycloalkylalkyl, or alkynyl; and a number of $R_3$ is one or more, or adjacent $R_3$ together form an alkoxy group

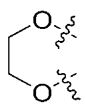 ;

$R_4$ is selected from the group consisting of hydrogen, halogen, hydroxyl, alkoxy, haloalkoxy, substituted or unsubstituted alkyl, cyano, -C(O)-O-alkyl, phenylalkoxy, carboxyl, hydroxymethyl, and cycloalkyl, a substituent being selected from the group consisting of hydroxyl, amide, halogen, and

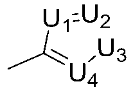;

$U_1$, $U_2$, and $U_4$ are independently selected from the group consisting of CH and N; $U_3$ is selected from the group consisting of $CH_2$ and NH; and a number of $R_4$ is one or more; and
when ring A is

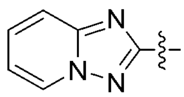,

ring B is not

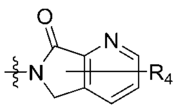.

**14.** The compound, or the isomer thereof, or the racemate thereof, or the pharmaceutically acceptable salt thereof according to claim 13, wherein

the alkyl is selected from the group consisting of methyl, ethyl, n-propyl, isopropyl, n-butyl, isobutyl, sec-butyl, tert-butyl, n-pentyl, sec-pentyl, 1-ethylpropyl, 2-methylbutyl, tert-pentyl, 1,2-dimethylpropyl, isopentyl, neopentyl, n-hexyl, isohexyl, sec-hexyl, tert-hexyl, neohexyl, 2-methylpentyl, 1,2-dimethylbutyl, and 1-ethylbutyl;
the alkoxy is selected from the group consisting of methoxy, ethoxy, n-propoxy, isopropoxy, n-butoxy, isobutoxy, sec-butoxy, and tert-butoxy;
the cycloalkyl is selected from the group consisting of cyclopropyl, cyclobutyl, cyclopentyl, and cyclohexyl; and the halogen is selected from the group consisting of fluorine, chlorine, bromine, and iodine.

**15.** The compound, or the isomer thereof, or the racemate thereof, or the pharmaceutically acceptable salt thereof according to claim 13, wherein Q is N; and/or Q is CH, and $R_2$ is F; and/or $T_1$ is N; and/or

$R_3$ is selected from the group consisting of hydrogen, methyl, methoxy, cyclopropyl, cyclopropylmethyl, fluorine, chlorine, oxo, and $CHF_2$-O-; and $R_4$ is selected from the group consisting of hydrogen, methyl, hydroxyl, hydroxymethyl, cyano, F, Cl, Br, -O-$CH_2$-phenyl, -COOH, -$COOCH_2CH_3$,

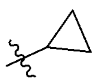, 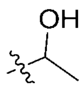,

amide, formamide, $CH_3$-C(O)-, ethynyl, trifluoromethyl, difluoromethoxy, and methoxy.

**16.** The compound, or the isomer thereof, or the racemate thereof, or the pharmaceutically acceptable salt thereof according to claim 13, wherein ring A is selected from the group consisting of

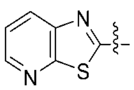, 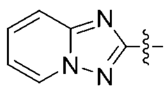, 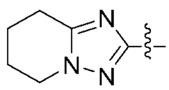, 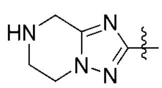, 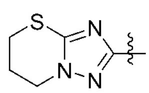,

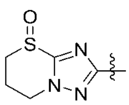, 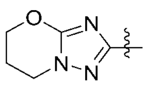, 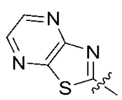, 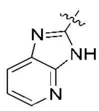, 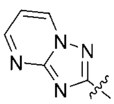, 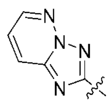, 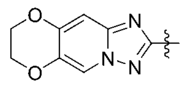, 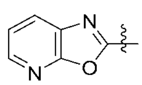,

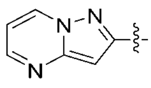, and 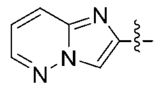;

further, $R_3$-substituted ring A is selected from the group consisting of:

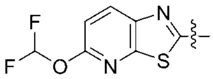,

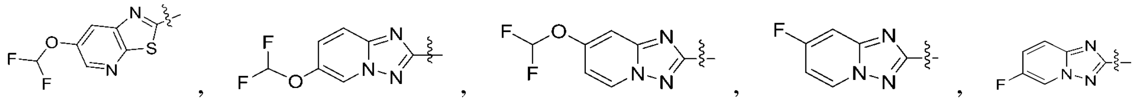

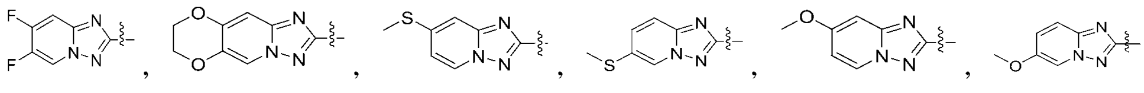

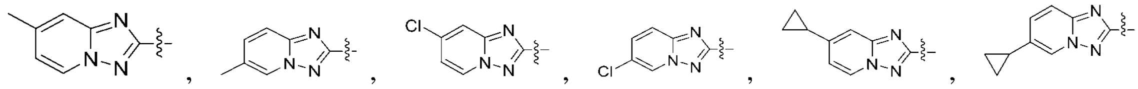

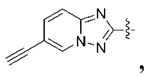, 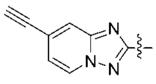, and 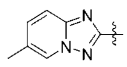;

further, $R_4$-substituted ring B is selected from the group consisting of:

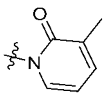, 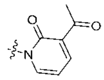,

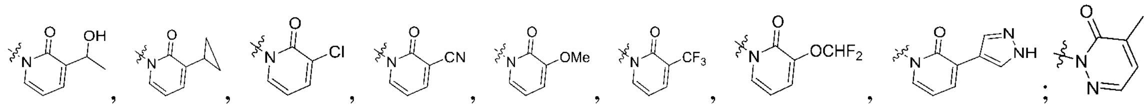

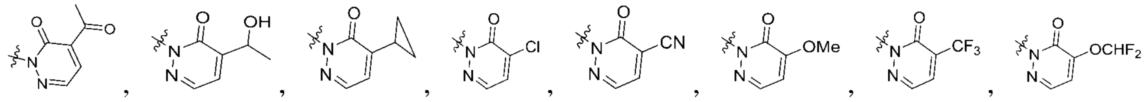

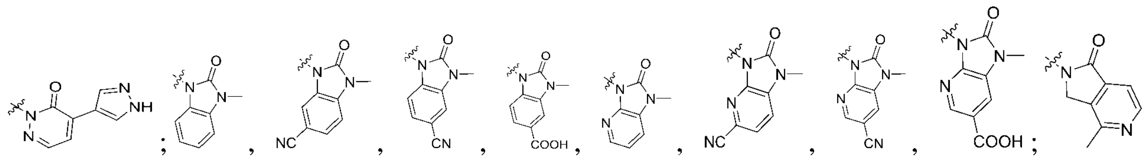

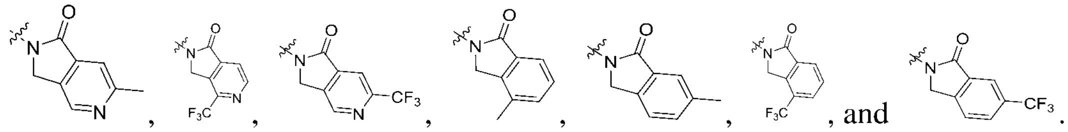

**17.** The compound, or the isomer thereof, or the racemate thereof, or the pharmaceutically acceptable salt thereof according to claim 13, wherein the compound, or the isomer thereof, or the racemate thereof, or the pharmaceutically acceptable salt thereof is selected from the group consisting of:

| No. | Compound | No. | Compound | No. | Compound |
|---|---|---|---|---|---|
| C1 | 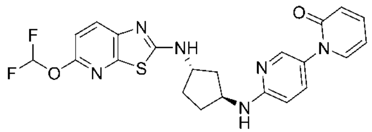 | C2 | 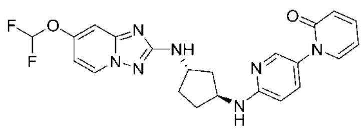 | C3 | 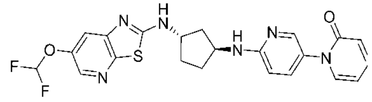 |
| C4 | 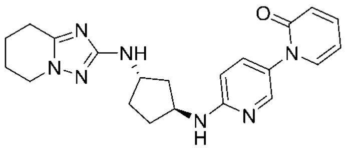 | C5 | 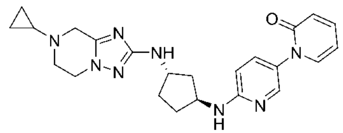 | C6 | 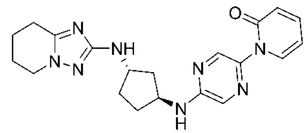 |
| C7 | 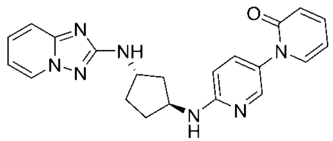 | C8 | 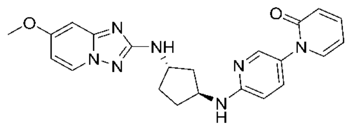 | C9 | 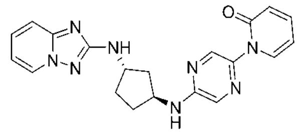 |
| C10 | 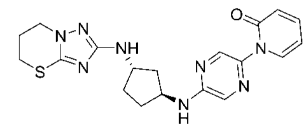 | C11 | 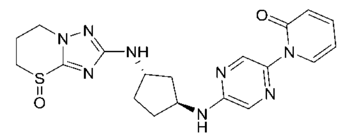 | C12 | 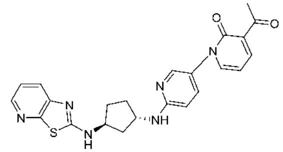 |
| C13 | 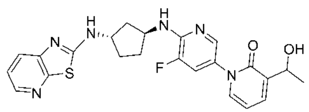 | C15 | 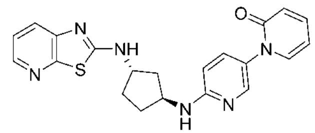 | C16 | 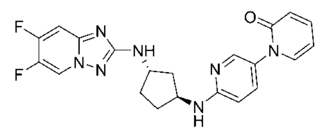 |
| C17 | 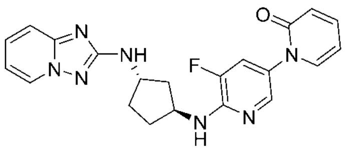 | C18 | 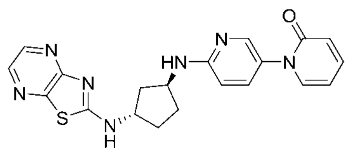 | C19 | 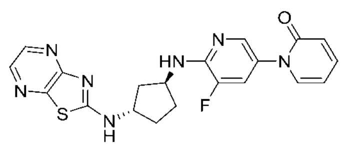 |
| C20 | 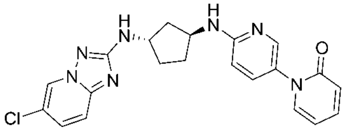 | C21 | 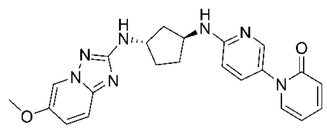 | C22 | 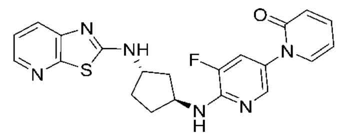 |

(continued)

| No. | Compound | No. | Compound | No. | Compound |
|---|---|---|---|---|---|
| C23 | 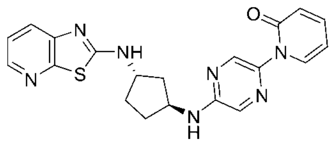 | C24 | 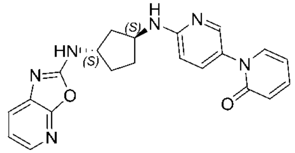 | C25 | 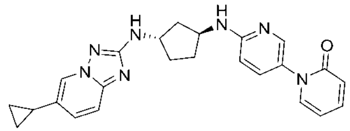 |
| C26 | 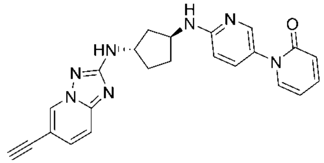 | C27 | 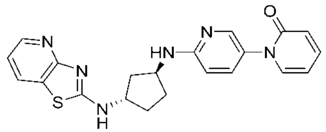 | C28 | 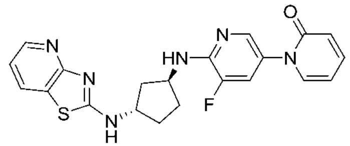 |
| C29 | 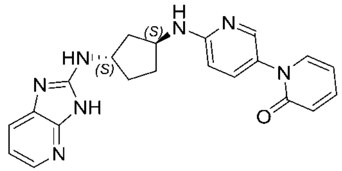 | C30 | 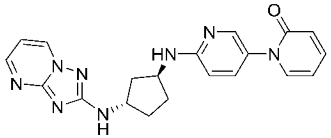 | C31 | 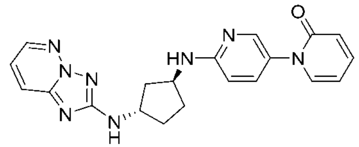 |
| C32 | 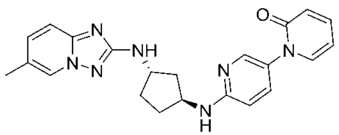 | C33 | 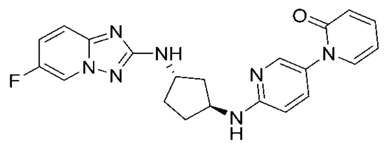 | C34 | 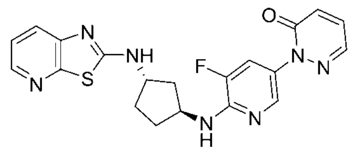 |
| C35 | 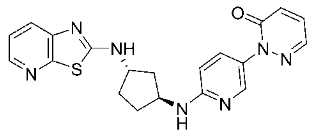 | C36 | 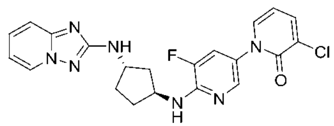 | C37 | 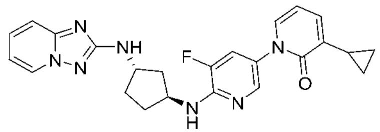 |
| C38 | 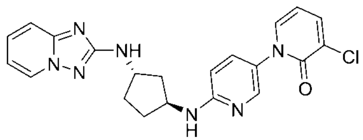 | C39 | 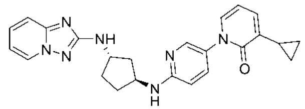 | C40 | 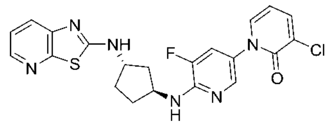 |

(continued)

| No. | Compound | No. | Compound | No. | Compound |
|---|---|---|---|---|---|
| C41 | 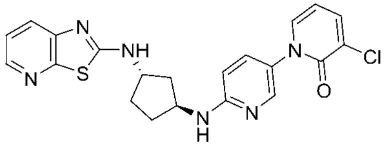 | C42 | 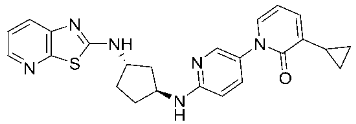 | C43 | 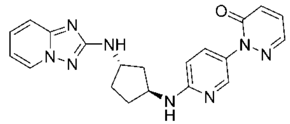 |
| C44 | 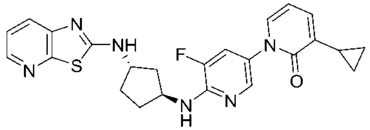 | C45 | 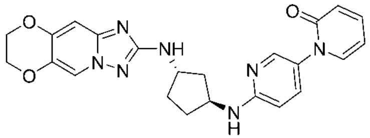 | C46 | 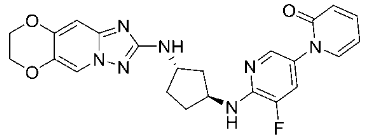 |
| C47 | 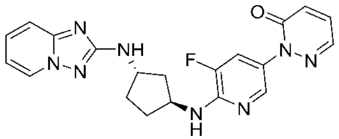 | C48 | 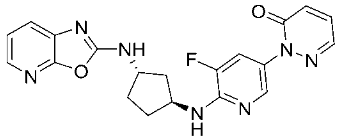 | C49 | 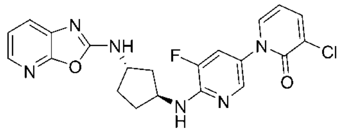 |
| C50 | 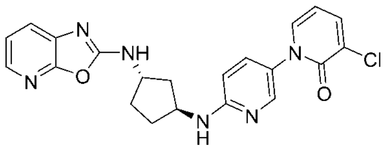 | C51 | 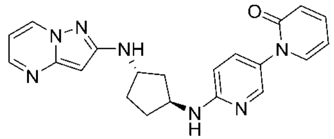 | C52 | 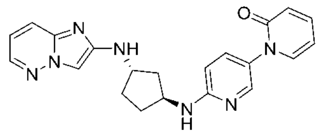 |
| C53 | 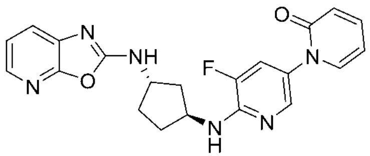 | C54 | 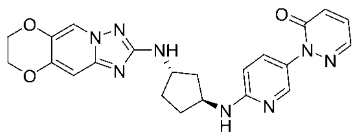 | C55 | 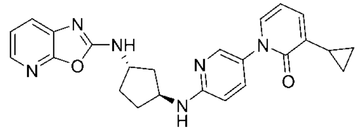 |
| C56 | 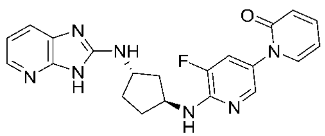 | C57 | 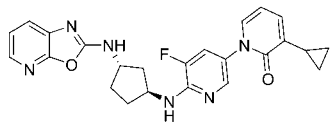 | C58 | 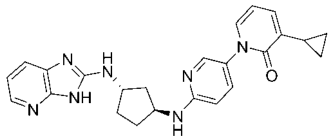 |

(continued)

| No. | Compound | No. | Compound | No. | Compound |
|---|---|---|---|---|---|
| C59 | 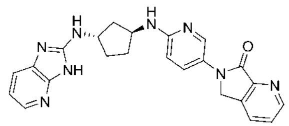 | C60 | 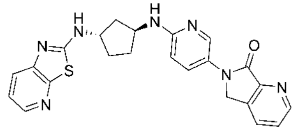 | C61 | 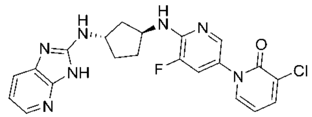 |
| C62 | 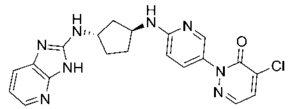 | C63 | 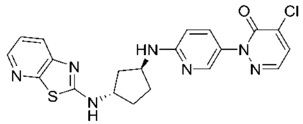 | C64 | 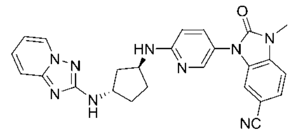 |
| C65 | 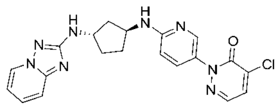 | C66 | 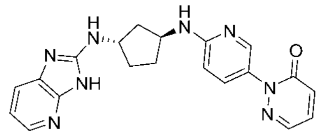 | C67 | 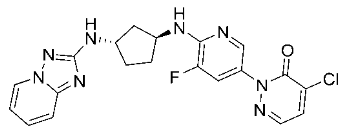 |
| C68 | 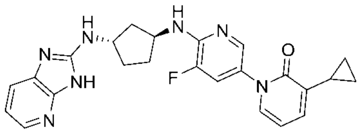 | C69 | 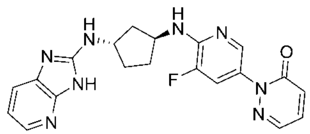 | C70 | 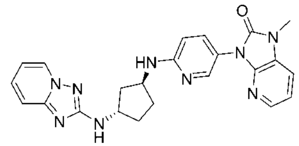 |
| C71 | 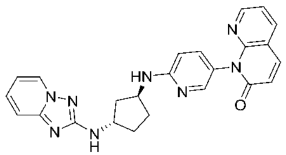 | C72 | 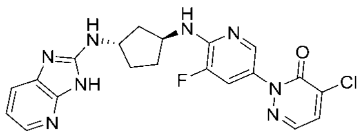 | C73 | 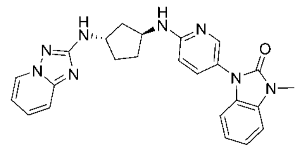 |
| C74 | 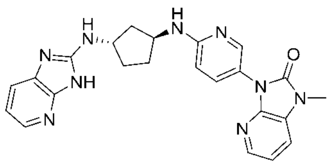 | C75 | 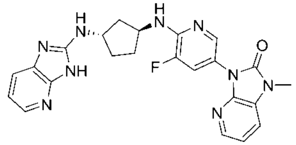 | C76 | 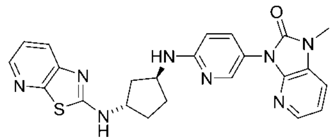 |

(continued)

| No. | Compound | No. | Compound | No. | Compound |
|---|---|---|---|---|---|
| C77 | 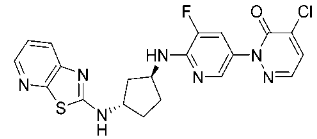 | C78 | 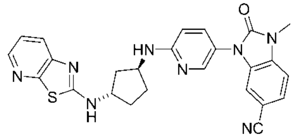 | C79 | 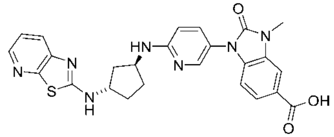 |
| C80 | 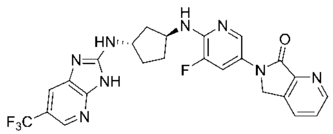 | C81 | 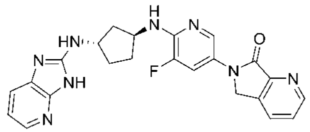 | C82 | 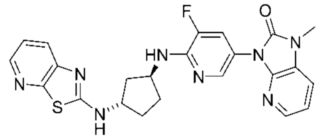 |
| C83 | 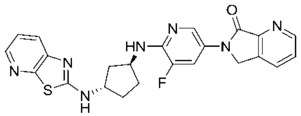 | C84 | 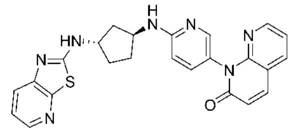 | C85 | 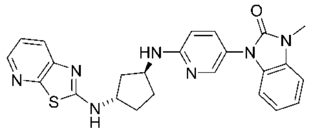 |
| C86 | 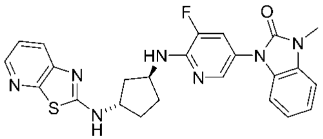 | C87 | 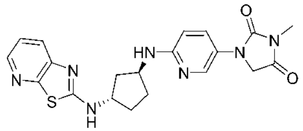 | C88 | 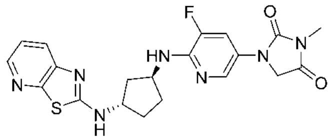 |
| C89 | 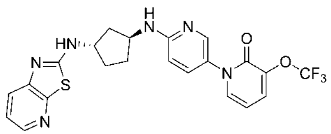 | C90 | 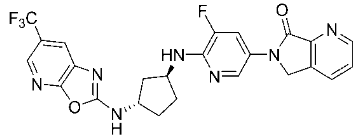 | C91 | 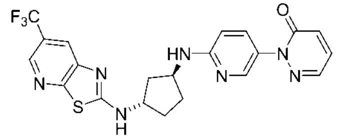 |
| C92 | 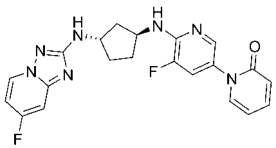 | C93 | 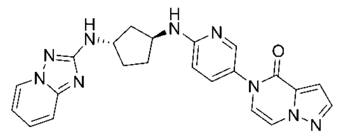 | C94 | 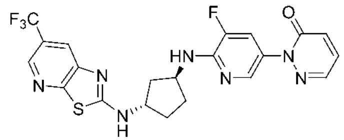 |
| C95 | 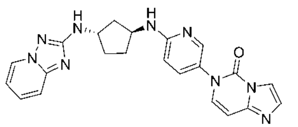 | | | | |

**18.** A compound represented by general formula (D), or an isomer thereof, or a racemate thereof, or a pharmaceutically acceptable salt thereof, wherein

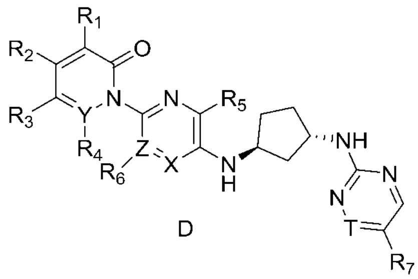

D

$R_1$, $R_2$, $R_3$, and $R_4$ are each independently selected from the group consisting of hydrogen, halogen, alkyl, cyano, haloalkyl, and cycloalkyl;
X is selected from the group consisting of CH and N, Z is selected from the group consisting of CH and N, and when Z is N, $R_6$ is absent;
Y is selected from the group consisting of CH and N, and when Y is N, $R_4$ is absent;
T is selected from the group consisting of CH, N and $CT_1$, and $T_1$ is selected from the group consisting of CN, alkyl, and haloalkyl;
$R_5$ and $R_6$ are each independently selected from the group consisting of hydrogen, halogen, and alkyl, and when X is CH, $R_5$ and $R_6$ are not both hydrogen simultaneously, or when X is CH, $R_1$, $R_2$, $R_3$, $R_4$, $R_5$, and $R_6$ are all hydrogen simultaneously; and
$R_7$ is selected from the group consisting of hydrogen, alkyl, alkoxy, cyano, alkynyl, substituted or unsubstituted cycloalkyl, alkylsulfonyl, alkylphosphonyl, substituted or unsubstituted $-N(C(O))_nX_1X_2$, substituted or unsubstituted -O-cycloalkyl, substituted or unsubstituted -O-heterocycloalkyl, boronic acid group, and halogen, and $X_1$ and $X_2$ are independently selected from the group consisting of alkyl, or $X_1$ and $X_2$ together with the N atom to which they are attached form a heterocycloalkyl, a substituent being selected from the group consisting of hydrogen, halogen, and alkyl, and n = 0 or 1; and when $R_7$ is selected from the group consisting of alkyl, alkoxy, and halogen, $R_5$ and $R_6$ are not both hydrogen simultaneously, or X is N.

**19.** The compound, or the isomer thereof, or the racemate thereof, or the pharmaceutically acceptable salt thereof according to claim 18, wherein

the alkyl is selected from the group consisting of methyl, ethyl, n-propyl, isopropyl, n-butyl, isobutyl, sec-butyl, tert-butyl, n-pentyl, sec-pentyl, 1-ethylpropyl, 2-methylbutyl, tert-pentyl, 1,2-dimethylpropyl, isopentyl, neopentyl, n-hexyl, isohexyl, sec-hexyl, tert-hexyl, neohexyl, 2-methylpentyl, 1,2-dimethylbutyl, and 1-ethylbutyl;
the halogen is selected from the group consisting of fluorine, chlorine, bromine, and iodine;
the cycloalkyl is selected from the group consisting of cyclopropyl, cyclobutyl, cyclopentyl, and cyclohexyl;
the heterocycloalkyl is cycloalkyl in which one or more carbon atoms are replaced by heteroatoms, the heteroatoms being selected from the group consisting of O, N, and S;
the alkynyl is $C_{2-4}$ alkynyl, such as ethynyl, propynyl, and butynyl.

**20.** The compound, or the isomer thereof, or the racemate thereof, or the pharmaceutically acceptable salt thereof according to claim 18, wherein the haloalkyl is $-CF_3$.

**21.** The compound, or the isomer thereof, or the racemate thereof, or the pharmaceutically acceptable salt thereof according to claim 18, wherein the $R_5$ and $R_6$ are each independently selected from the group consisting of hydrogen, fluorine, and methyl; $R_7$ is selected from the group consisting of hydrogen, methyl, methoxy, ethoxy, fluorine, ethynyl, chlorine,

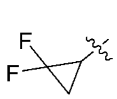, 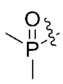, 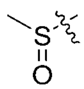, 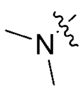, 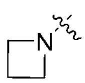, 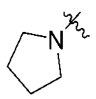, 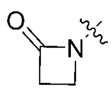, 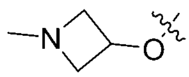, 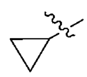,

and

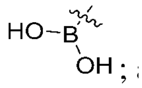

and/or

the $R_1$ is selected from the group consisting of hydrogen, fluorine, chlorine, cyclopropyl, cyano, methyl, and $-CF_3$; $R_2$, $R_3$, and $R_4$ are each independently hydrogen; and/or
the X is N.

**22.** The compound, or the isomer thereof, or the racemate thereof, or the pharmaceutically acceptable salt thereof according to claim 18, wherein the compound, or the isomer thereof, or the racemate thereof, or the pharmaceutically acceptable salt thereof is selected from the group consisting of:

| No. | Compound Structure | No. | Compound Structure | No. | Compound Structure |
|---|---|---|---|---|---|
| D1 | 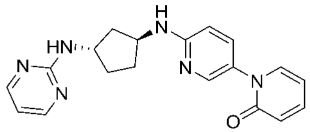 | D2 | 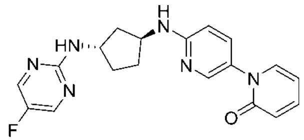 | D3 | 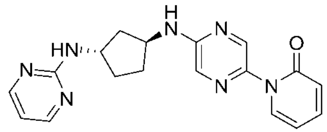 |
| D4 | 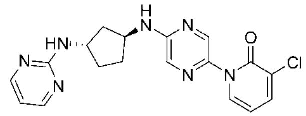 | D5 | 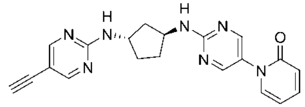 | D6 | 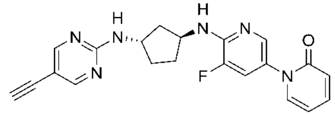 |
| D7 | 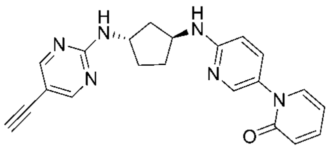 | D8 | 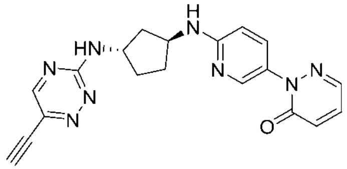 | D9 | 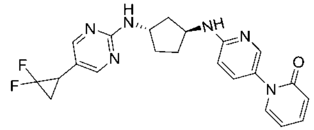 |
| D10 | 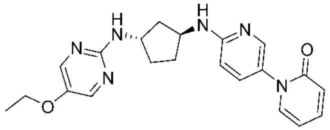 | D11 | 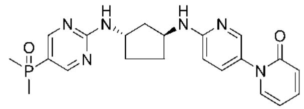 | D12 | 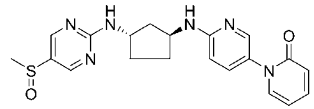 |
| D13 | 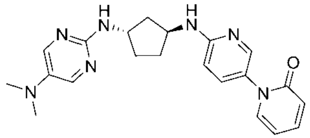 | 14 | 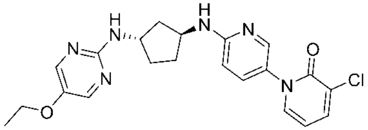 | D15 | 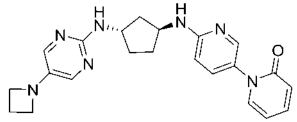 |
| D16 | 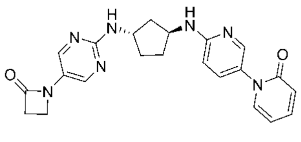 | D17 | 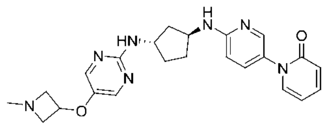 | D18 | 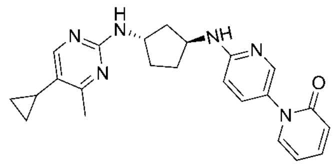 |

(continued)

| No. | Compound Structure | No. | Compound Structure | No. | Compound Structure |
|---|---|---|---|---|---|
| D19 | 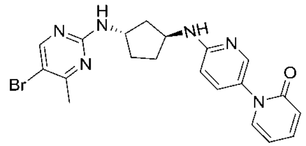 | D20 | 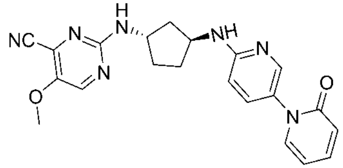 | D21 | 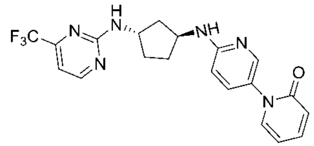 |
| D22 | 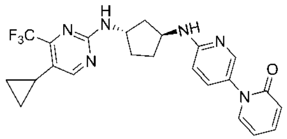 | D23 | 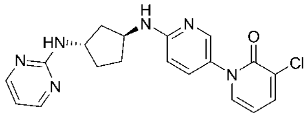 | D24 | 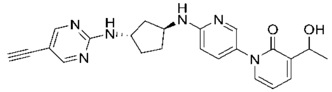 |
| D25 | 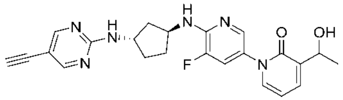 | D26 | 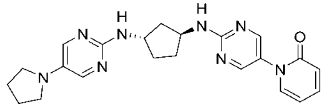 | D27 | 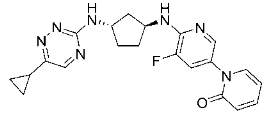 |
| D28 | 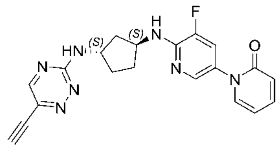 | D29 | 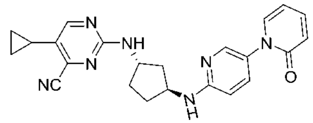 | | |

**23.** A compound represented by general formula (E), or an isomer thereof, or a racemate thereof, or a pharmaceutically acceptable salt thereof, wherein

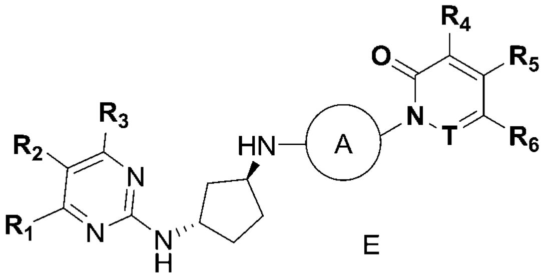

E

ring A is selected from the group consisting of benzofuran, benzothiazole, 1H-indole, indole, quinoline, and a structure in which one or more -C= or -CH- in a ring of benzofuran, benzothiazole, 1H-indole, indole, or quinoline is replaced by N atom;
T is selected from the group consisting of N and $CR_7$; and
$R_1$, $R_2$, $R_3$, $R_4$, $R_5$, $R_6$, and $R_7$ are independently selected from the group consisting of hydrogen, halogen, substituted or unsubstituted alkyl, cycloalkyl, and alkoxy, and a substitution being selected from the group consisting of halogen and alkyl.

**24.** The compound, or the isomer thereof, or the racemate thereof, or the pharmaceutically acceptable salt thereof according to claim 23, wherein

the alkyl is selected from the group consisting of methyl, ethyl, n-propyl, isopropyl, n-butyl, isobutyl, sec-butyl, tert-butyl, n-pentyl, sec-pentyl, 1-ethylpropyl, 2-methylbutyl, tert-pentyl, 1,2-dimethylpropyl, isopentyl, neopentyl, n-hexyl, isohexyl, sec-hexyl, tert-hexyl, neohexyl, 2-methylpentyl, 1,2-dimethylbutyl, and 1-ethylbutyl;
the halogen is selected from the group consisting of fluorine, chlorine, bromine, and iodine;
the alkoxy is selected from the group consisting of methoxy, ethoxy, n-propoxy, isopropoxy, n-butoxy, isobutoxy, sec-butoxy, tert-butoxy, n-pentoxy, sec-pentoxy, 1-ethylpropoxy, 2-methylbutoxy, tert-pentoxy, 1,2-dimethylpropoxy, isopentoxy, neopentoxy, n-hexoxy, isohexoxy, sec-hexoxy, tert-hexoxy, neohexoxy, 2-methylpentoxy, 1,2-dimethylbutoxy, 1-ethylbutoxy, n-heptoxy, and isoheptoxy; and
the cycloalkyl is selected from the group consisting of cyclopropyl, cyclobutyl, cyclopentyl, and cyclohexyl; the heterocycloalkyl refers to cycloalkyl in which at least one carbon atom is replaced by a heteroatom, the heteroatom being selected from the group consisting of nitrogen, oxygen, and sulfur.

**25.** The compound, or the isomer thereof, or the racemate thereof, or the pharmaceutically acceptable salt thereof according to claim 23, wherein T is of N; and/or $R_1$ and $R_3$ are hydrogen, and $R_2$ is $-OCHF_2$.

**26.** The compound, or the isomer thereof, or the racemate thereof, or the pharmaceutically acceptable salt thereof according to claim 23, wherein ring A is selected from the group consisting of:

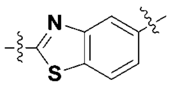, 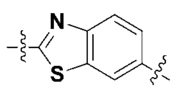, 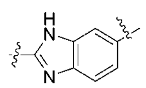, 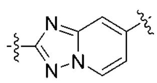, 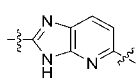,

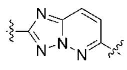, 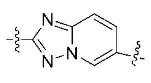, 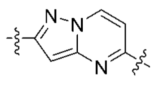, 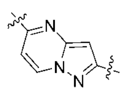, 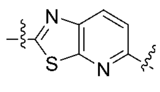, 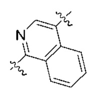, 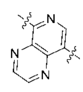, 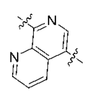,

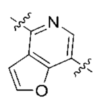, 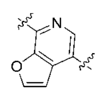, 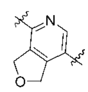, and 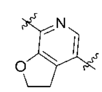.

**27.** The compound, or the isomer thereof, or the racemate thereof, or the pharmaceutically acceptable salt thereof according to claim 23, wherein the compound, or the isomer thereof, or the racemate thereof, or the pharmaceutically

acceptable salt thereof is selected from the group consisting of:

| No. | Compound | No. | Compound | No. | Compound |
|---|---|---|---|---|---|
| E1 | 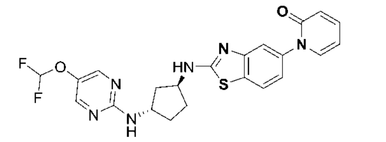 | E2 | 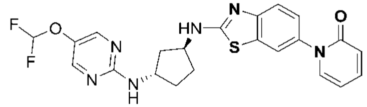 | E3 | 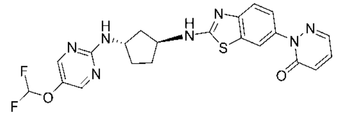 |
| E4 | 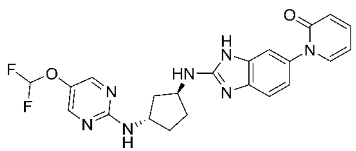 | E5 | 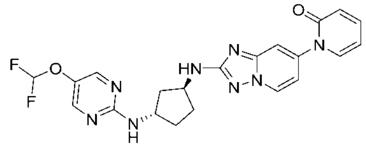 | E6 | 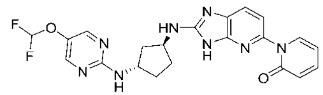 |
| E7 | 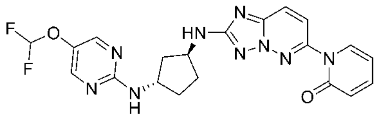 | E8 | 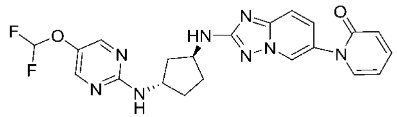 | E9 | 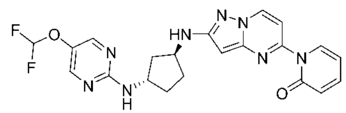 |
| E10 | 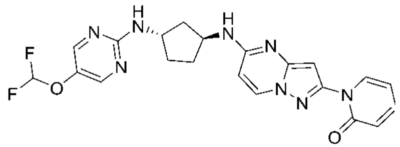 | E11 | 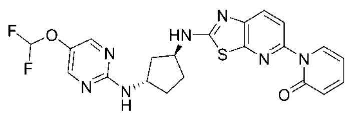 | E12 | 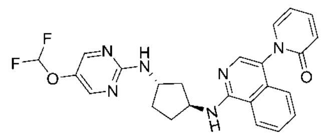 |
| E13 | 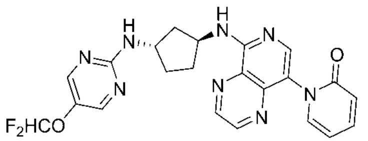 | E14 | 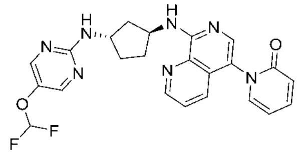 | E15 | 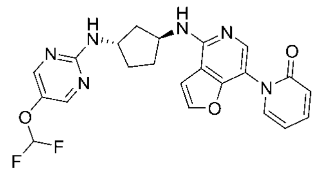 |
| E16 | 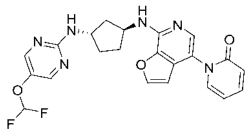 | E17 | 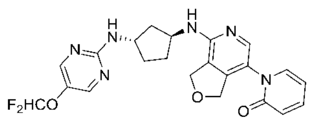 | E18 | 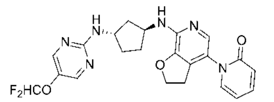 |

**28.** A compound represented by general formula (F), or an isomer thereof, or a racemate thereof, or a pharmaceutically acceptable salt thereof, wherein

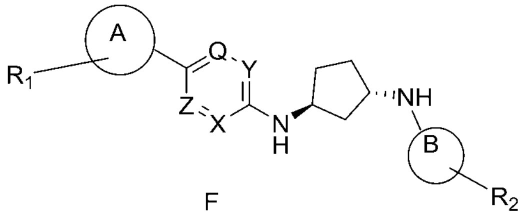

F

ring A is selected from the group consisting of:

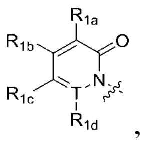 , 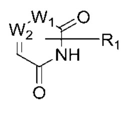 ,

and $R_1$-substituted or unsubstituted

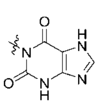 , 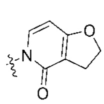 , 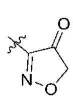 , 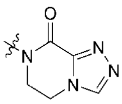 , 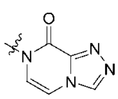 , 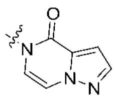 , 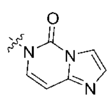 , 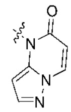 , 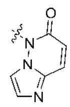 ,

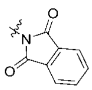 , 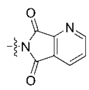 , 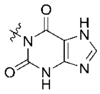 , 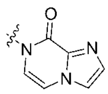 , 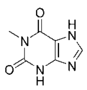 , 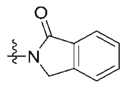 , 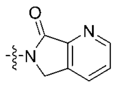 , 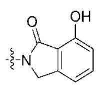 , and 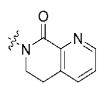 ;

ring B is selected from the group consisting of

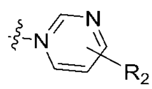 , 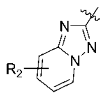 , and 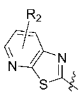 ;

$R_1$, $R_{1a}$, $R_{1b}$, $R_{1c}$, $R_{1d}$ are each independently selected from the group consisting of hydrogen, oxo, hydroxyl, alkynyl, alkylalkynyl, alkylalkynylalkyl, alkyl, halogen, alkoxy, alkylthio, haloalkyl, haloalkoxy, cycloalkyl, cycloalkylalkyl, cyano, and

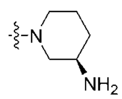 ,

or two adjacent substituents together form a 5- to 6-membered saturated or unsaturated heterocycle;
a number of $R_2$ is one or more, each independently selected from the group consisting of hydrogen, halogen, hydroxyl, alkoxy, haloalkoxy, substituted or unsubstituted alkyl, cyano, -C(O)-O-alkyl, phenylalkoxy, carboxyl, hydroxymethyl, and cycloalkyl, a substituent being selected from the group consisting of hydroxyl, amide, and halogen;
Q, X, Y, Z are each independently selected from the group consisting of $CR_3$ and N, $R_3$ being independently selected from the group consisting of hydrogen, oxo, alkyl, halogen, alkoxy, alkylthio, haloalkyl, haloalkoxy, cycloalkyl, cycloalkylalkyl, and cyano, and when A is

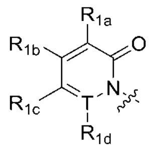,

at least two of Q, X, Y, Z are N;
T is each independently selected from the group consisting of CH and N; and
$W_1$ is selected from the group consisting of $CH_2$ and NH, and $W_2$ is each independently selected from the group consisting of CH and N.

**29.** The compound, or the isomer thereof, or the racemate thereof, or the pharmaceutically acceptable salt thereof according to claim 28, wherein

the alkyl is selected from the group consisting of methyl, ethyl, n-propyl, isopropyl, n-butyl, isobutyl, sec-butyl, tert-butyl, n-pentyl, sec-pentyl, 1-ethylpropyl, 2-methylbutyl, tert-pentyl, 1,2-dimethylpropyl, isopentyl, neopentyl, n-hexyl, isohexyl, sec-hexyl, tert-hexyl, neohexyl, 2-methylpentyl, 1,2-dimethylbutyl, and 1-ethylbutyl;
the halogen is selected from the group consisting of fluorine, chlorine, bromine, and iodine;
the alkoxy is selected from the group consisting of methoxy, ethoxy, n-propoxy, isopropoxy, n-butoxy, isobutoxy, sec-butoxy, and tert-butoxy; the alkylthio refers to alkoxy in which the oxygen atom is replaced by a sulfur atom; and the cycloalkyl is selected from the group consisting of cyclopropyl, cyclobutyl, cyclopentyl, and cyclohexyl.

**30.** The compound, or the isomer thereof, or the racemate thereof, or the pharmaceutically acceptable salt thereof according to claim 28, wherein Q is N; and/or $R_2$ is $-O-CHF_2$.

**31.** The compound, or the isomer thereof, or the racemate thereof, or the pharmaceutically acceptable salt thereof according to claim 28, wherein

ring A is selected from the group consisting of

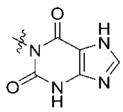, 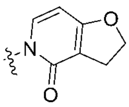, 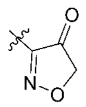, 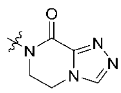,

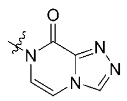, 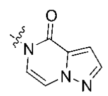, 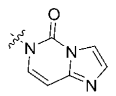, 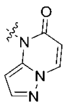, 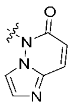, 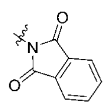, 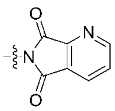, 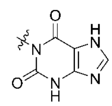, 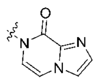,

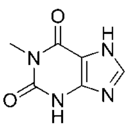, 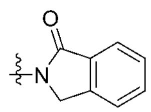, 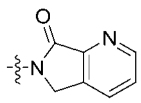, 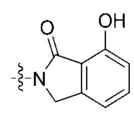, and 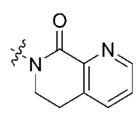;

substituted ring A is selected from the group consisting of:

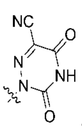, 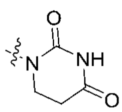, 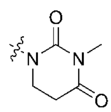,

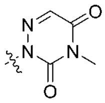, 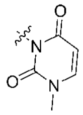, 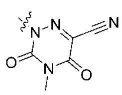, 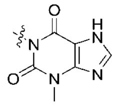, 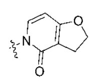, 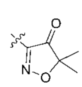, 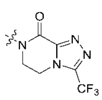, 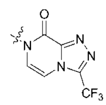, 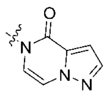,

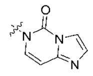, 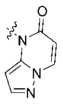, 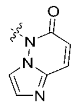, 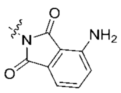, 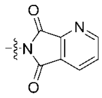, 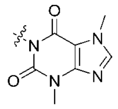, 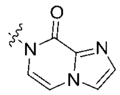, 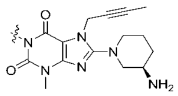,

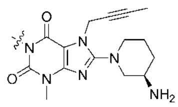, 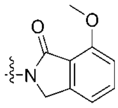, 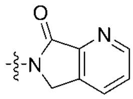, 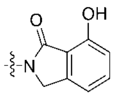, and 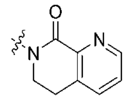;

or ring A is selected from the group consisting of

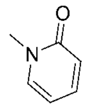 and 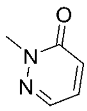,

and substituted ring A is selected from the group consisting of

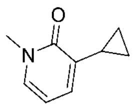 and 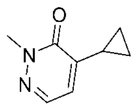.

**32.** The compound, or the isomer thereof, or the racemate thereof, or the pharmaceutically acceptable salt thereof according to claim 28, wherein the compound, or the isomer thereof, or the racemate thereof, or the pharmaceutically acceptable salt thereof is selected from the group consisting of:

| No. | Compound Structure | No. | Compound Structure | No. | Compound Structure |
|---|---|---|---|---|---|
| F1 | 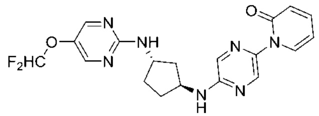 | F2 | 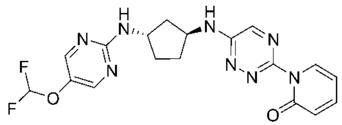 | F3 | 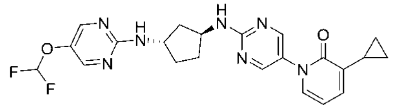 |
| F4 | 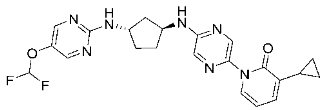 | F5 | 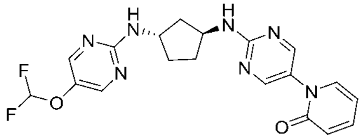 | F6 | 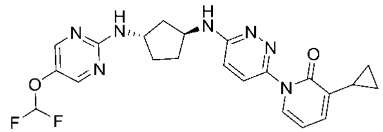 |
| F7 | 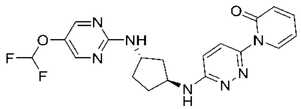 | F8 | 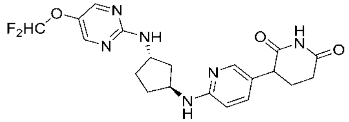 | F9 | 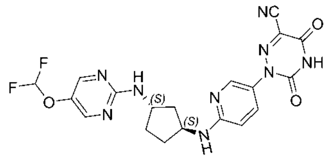 |
| F10 | 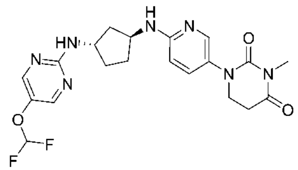 | F11 | 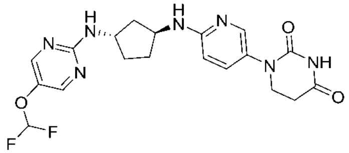 | F12 | 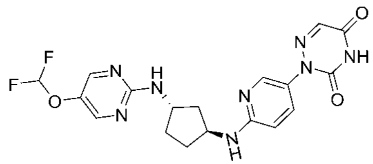 |
| F13 | 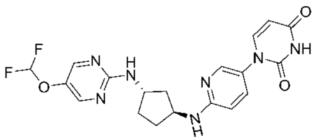 | F14 | 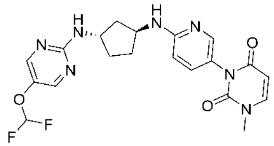 | F15 | 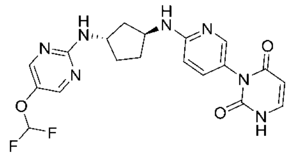 |
| F16 | 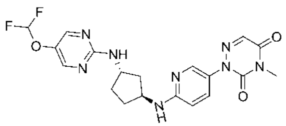 | F17 | 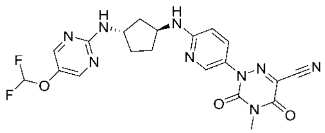 | F18 | 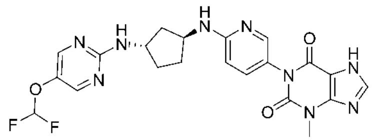 |

(continued)

| No. | Compound Structure | No. | Compound Structure | No. | Compound Structure |
|---|---|---|---|---|---|
| F19 | 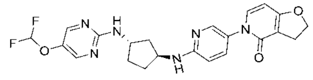 | F20 | 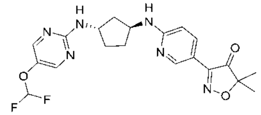 | F21 | 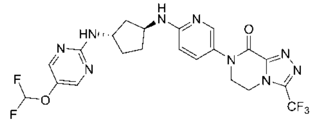 |
| F22 | 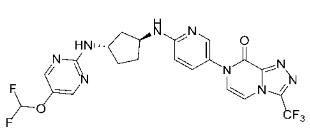 | F23 | 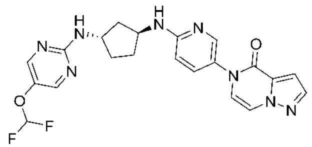 | F24 | 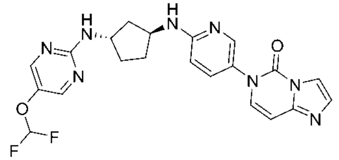 |
| F25 | 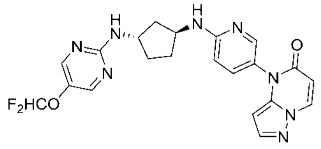 | F26 | 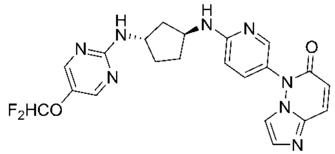 | F27 | 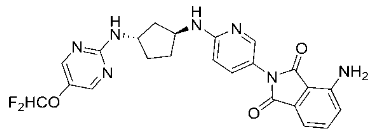 |
| F28 | 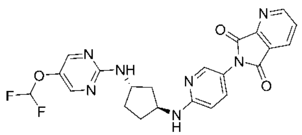 | F29 | 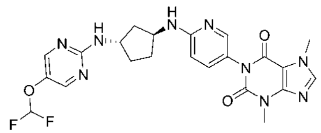 | F30 | 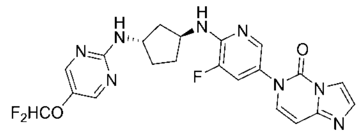 |
| F31 | 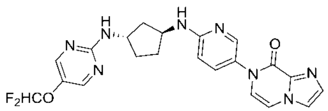 | F32 | 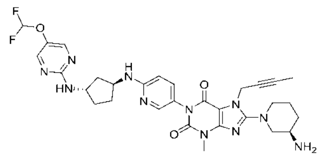 | F33 | 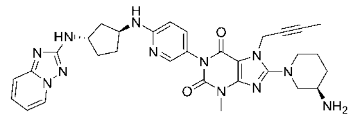 |
| F34 | 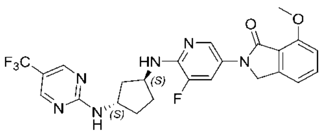 | F35 | 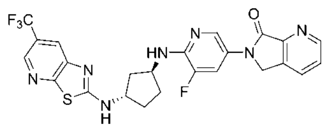 | F36 | 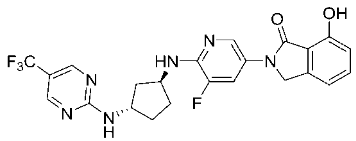 |

(continued)

| No. | Compound Structure | No. | Compound Structure | No. | Compound Structure |
|---|---|---|---|---|---|
| F37 | 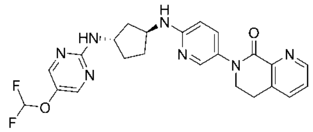 | | | | |

**33.** A compound represented by general formula (G), or an isomer thereof, or a racemate thereof, or a pharmaceutically acceptable salt thereof, wherein

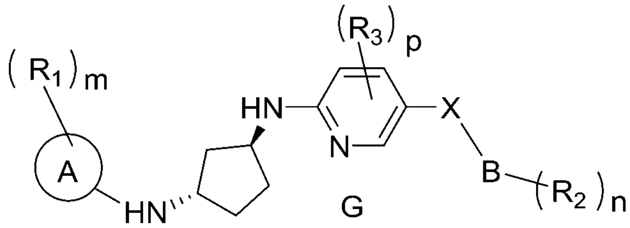,

X is selected from the group consisting of a substituted or unsubstituted alkyl, amino, O, and S, and a substituent being selected from the group consisting of oxo, alkyl, halogen, alkoxy, hydroxyl, and alkoxycarbonyl;
ring A is $R_1$-substituted or unsubstituted 5- to 14-membered saturated or unsaturated heterocycle, and the heterocycle can be monocyclic, bicyclic, or tricyclic;
$R_1$ is independently selected from the group consisting of hydrogen, oxo, alkyl, halogen, alkoxy, alkylthio, haloalkyl, haloalkoxy, cycloalkyl, cycloalkylalkyl, and cyano, and m = 0, 1, 2, or 3;
B is ring B, which is selected from the group consisting of $R_2$-substituted or unsubstituted 5- to 7-membered saturated or unsaturated heterocycle, oxo-substituted alkyl, and -C(O)-pyridine;
$R_2$ is independently selected from the group consisting of hydrogen, oxo, thio, alkyl, halogen, alkoxy, alkylthio, haloalkyl, haloalkoxy, cycloalkyl, cycloalkylalkyl, cyano, and carboxyl, and n = 0, 1, 2, or 3;
$R_3$ are each selected from the group consisting of hydrogen, alkyl, alkoxy, cyano, halogen, haloalkyl, amide, hydroxyl, hydroxyl-substituted alkyl, carboxyl, and amino, and p = 0, 1, 2, or 3.

**34.** The compound, or the isomer thereof, or the racemate thereof, or the pharmaceutically acceptable salt thereof according to claim 33, wherein

the alkyl is selected from the group consisting of methyl, ethyl, n-propyl, isopropyl, n-butyl, isobutyl, sec-butyl, tert-butyl, n-pentyl, sec-pentyl, 1-ethylpropyl, 2-methylbutyl, tert-pentyl, 1,2-dimethylpropyl, isopentyl, neopentyl, n-hexyl, isohexyl, sec-hexyl, tert-hexyl, neohexyl, 2-methylpentyl, 1,2-dimethylbutyl, and 1-ethylbutyl;
the alkoxy is selected from the group consisting of methoxy, ethoxy, n-propoxy, isopropoxy, n-butoxy, isobutoxy, sec-butoxy, and tert-butoxy;
the alkylthio is selected from the group consisting of methylthio, ethylthio, propylthio, isopropylthio, n-butylthio, isobutylthio, sec-butylthio, and tert-butylthio;
the cycloalkyl is selected from the group consisting of cyclopropyl, cyclobutyl, cyclopentyl, and cyclohexyl;
the halogen is selected from the group consisting of fluorine, chlorine, bromine, and iodine.

**35.** The compound, or the isomer thereof, or the racemate thereof, or the pharmaceutically acceptable salt thereof according to claim 33, wherein

ring A is selected from the group consisting of

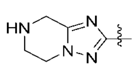, 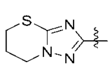, 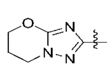, 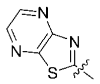, 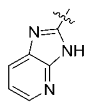, 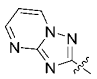, 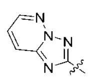, 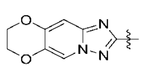, 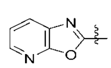,

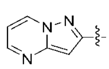, 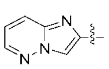, 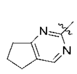, 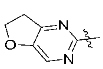, 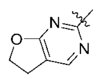, 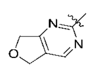, 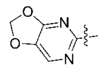, 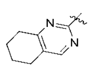, 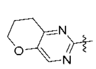, 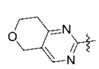,

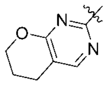, 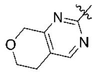, 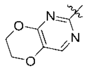, 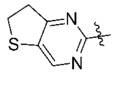, 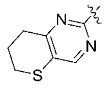, 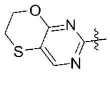, 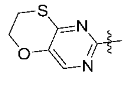, 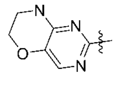, 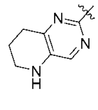,

and

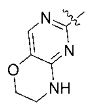 ;

and/or

ring B is selected from the group consisting of:

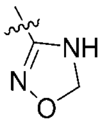 , 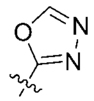 , 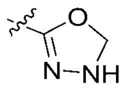 , 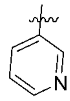 , 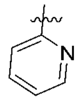 ,

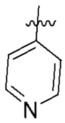 , 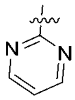 , 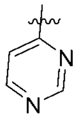 , 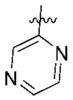 , 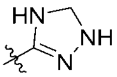 , 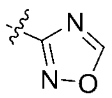 , 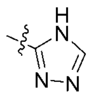 , 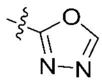 , 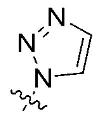 , and 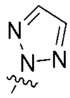 .

**36.** The compound, or the isomer thereof, or the racemate thereof, or the pharmaceutically acceptable salt thereof according to claim 33, wherein $R_1$ is selected from the group consisting of hydrogen, methyl, cyano, methoxy, cyclopropyl, cyclopropylmethyl, fluorine, chlorine, and $CHF_2$-O-; and/or $R_2$ is selected from the group consisting of hydrogen, methyl, oxo, and thio; and/or $R_3$ is selected from the group consisting of hydrogen, methyl, cyano, methoxy, cyclopropyl, cyclopropylmethyl, fluorine, and chlorine; and/or X is selected from the group consisting of $-CH_2-$, -C(O)-, -CH(OH)-, $-NH_2-$, $-NH(CH_3)--$,

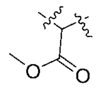 ,

O, and S.

**37.** The compound, or the isomer thereof, or the racemate thereof, or the pharmaceutically acceptable salt thereof according to claim 33, wherein the compound, or the isomer thereof, or the racemate thereof, or the pharmaceutically acceptable salt thereof is selected from the group consisting of:

| No. | Compound | No. | Compound | No. | Compound |
|---|---|---|---|---|---|
| G1 | 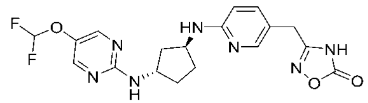 | G2 | 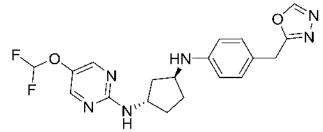 | G3 | 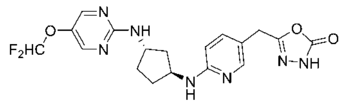 |
| G4 | 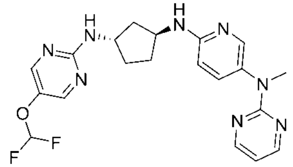 | G5 | 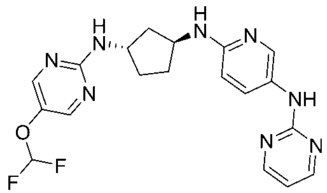 | G6 | 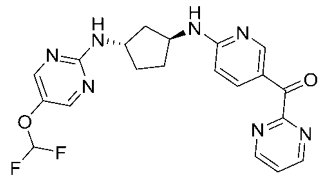 |
| G7 | 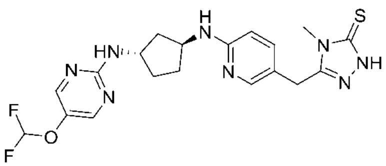 | G8 | 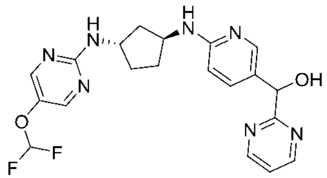 | G9 | 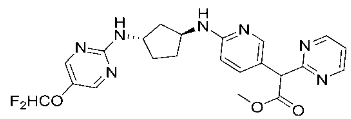 |
| G10 | 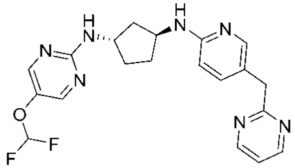 | G11 | 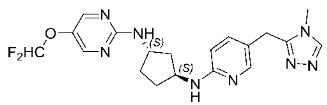 | G12 | 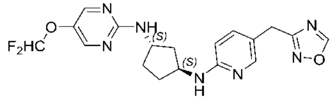 |
| G13 | 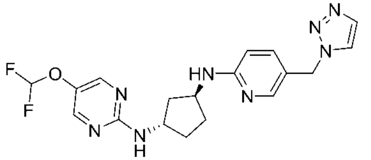 | G14 | 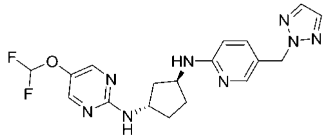 | G15 | 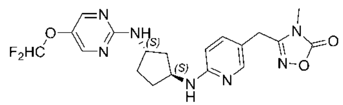 |
| G16 | 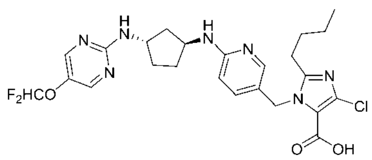 | G17 | 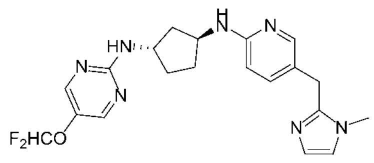 | G18 | 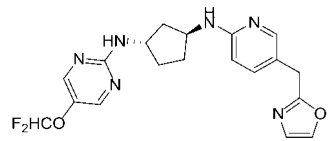 |

(continued)

| No. | Compound | No. | Compound | No. | Compound |
|---|---|---|---|---|---|
| G19 | 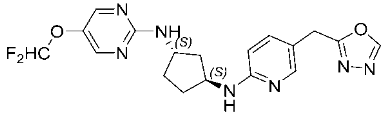 | G20 | 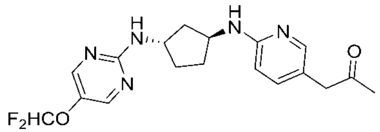 | G21 | 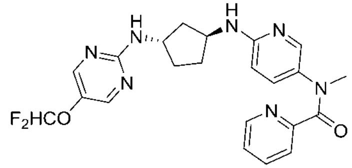 |

**38.** A compound represented by general formula (H), or an isomer thereof, or a racemate thereof, or a pharmaceutically acceptable salt thereof, wherein

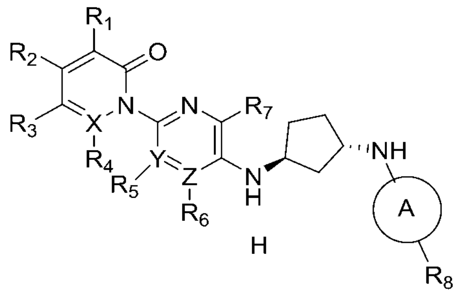

H

$R_1$, $R_2$, $R_3$, and $R_4$ are each independently selected from the group consisting of hydrogen, halogen, alkyl, cyano, haloalkyl, and cycloalkyl;
X is selected from the group consisting of C and N, and when X is N, $R_5$ is absent;
Y is selected from the group consisting of C and N, and when Y is N, $R_6$ is absent;
Z is selected from the group consisting of C and N, and when Z is N, $R_7$ is absent;
$R_5$, $R_6$, and $R_7$ are each independently selected from the group consisting of hydrogen, halogen, and alkyl;
a number of $R_8$ is one or more, which is independently selected from the group consisting of hydrogen, halogen, phenyl, substituted or unsubstituted alkyl, substituted or unsubstituted cycloalkylalkyl, and substituted or unsubstituted cycloalkyl, a substituent being selected from the group consisting of alkyl and halogen; and
ring A is selected from the group consisting of

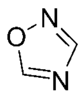 , 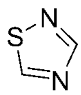 , 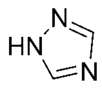 , 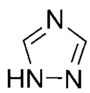 ,

and

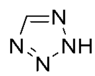 ,

and when A is

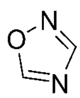 ,

and X, Y, and Z are all C, $R_1$, $R_2$, $R_3$, $R_4$, $R_5$, $R_6$, and $R_7$ are not all hydrogen simultaneously.

**39.** The compound, or the isomer thereof, or the racemate thereof, or the pharmaceutically acceptable salt thereof according to claim 38, wherein

the alkyl is selected from the group consisting of methyl, ethyl, n-propyl, isopropyl, n-butyl, isobutyl, sec-butyl, tert-butyl, n-pentyl, sec-pentyl, 1-ethylpropyl, 2-methylbutyl, tert-pentyl, 1,2-dimethylpropyl, isopentyl, neopentyl, n-hexyl, isohexyl, sec-hexyl, tert-hexyl, neohexyl, 2-methylpentyl, 1,2-dimethylbutyl, and 1-ethylbutyl;
the halogen is selected from the group consisting of fluorine, chlorine, bromine, and iodine;
the cycloalkyl is selected from the group consisting of cyclopropyl, cyclobutyl, cyclopentyl, and cyclohexyl.

**40.** The compound, or the isomer thereof, or the racemate thereof, or the pharmaceutically acceptable salt thereof according to claim 38, wherein $R_1$ is selected from the group consisting of halogen and alkyl; and/or Y is N, or Y is C, and $R_5$ is selected from the group consisting of halogen and alkyl; and/or $R_8$ is selected from the group consisting of methyl, cyclopropyl, cyclobutyl,

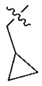, 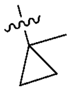, 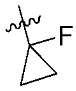, 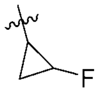, and 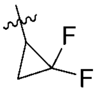;

and/or Z is of N.

**41.** The compound, or the isomer thereof, or the racemate thereof, or the pharmaceutically acceptable salt thereof according to claim 38, wherein the

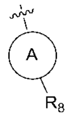

is selected from the group consisting of

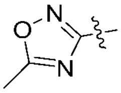, 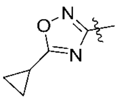, 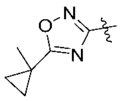, 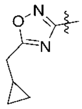, 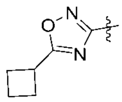, 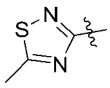,

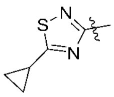, 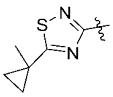, 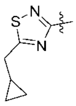, 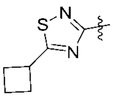; 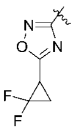, 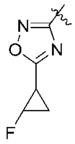, 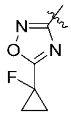, 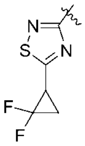, 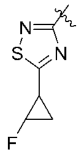, 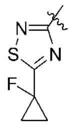, 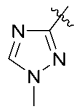,

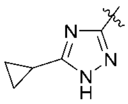, 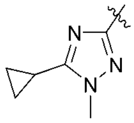, 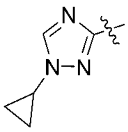, and 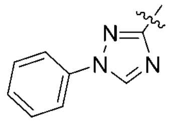.

**42.** The compound, or the isomer thereof, or the racemate thereof, or the pharmaceutically acceptable salt thereof according to claim 38, wherein the compound, or the isomer thereof, or the racemate thereof, or the pharmaceutically acceptable salt thereof is selected from the group consisting of:

| No. | Compound Structure | No. | Compound Structure | No. | Compound Structure |
|---|---|---|---|---|---|
| H1 | 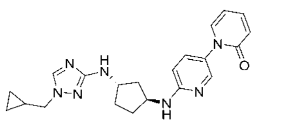 | H2 | 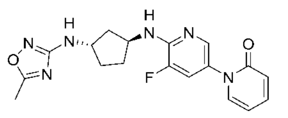 | H3 | 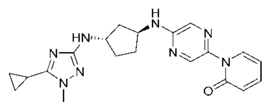 |
| H4 | 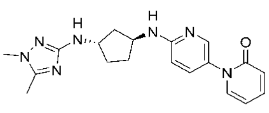 | H5 | 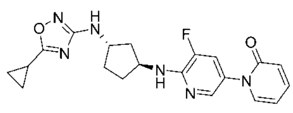 | H6 | 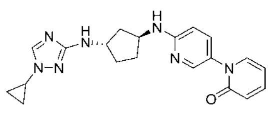 |
| H7 | 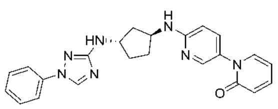 | H8 | 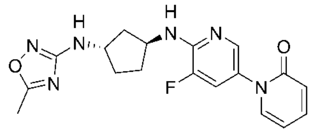 | H9 | 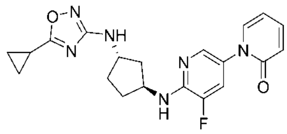 |
| H10 | 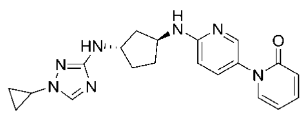 | H11 | 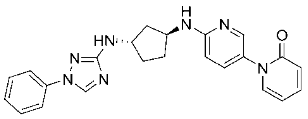 | H12 | 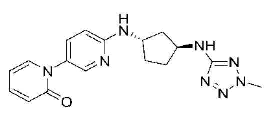 |

(continued)

| No. | Compound Structure | No. | Compound Structure | No. | Compound Structure |
|---|---|---|---|---|---|
| H13 | 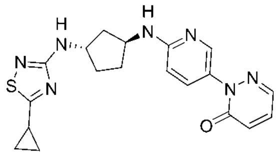 | H14 | 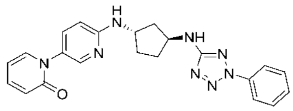 | | |

**43.** A compound represented by general formula (J), or an isomer thereof, or a racemate thereof, or a pharmaceutically acceptable salt thereof, wherein

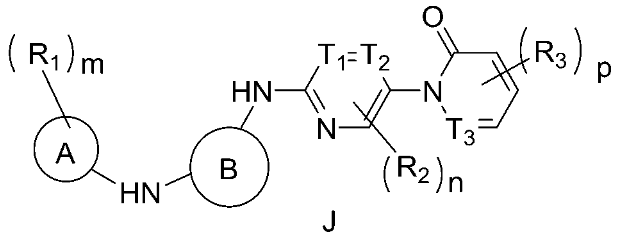

J

,

ring A is $R_1$-substituted or unsubstituted 5- to 14-membered saturated or unsaturated heterocycle, and the heterocycle can be monocyclic, bicyclic, or tricyclic;
$R_1$ is independently selected from the group consisting of hydrogen, oxo, alkyl, halogen, alkoxy, alkylthio, haloalkyl, haloalkoxy, cycloalkyl, cycloalkylalkyl, and cyano, and m = 0, 1, 2, or 3;
ring B is selected from the group consisting of a 5- to 8-membered saturated or unsaturated monocyclic and bicyclic ring, and is not

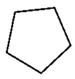

;

$R_2$ is independently selected from the group consisting of hydrogen, oxo, thio, alkyl, halogen, alkoxy, alkylthio, haloalkyl, haloalkoxy, cycloalkyl, cycloalkylalkyl, and cyano, and n = 0, 1, 2, or 3;
$R_3$ is independently selected from the group consisting of hydrogen, halogen, hydroxyl, substituted or unsubstituted alkyl, cyano, -C(O)-O-alkyl, phenylalkoxy, carboxyl, hydroxymethyl, cycloalkyl, alkyl, amide, alkyl-substituted amide, Z-C(O)-, aryl, and heteroaryl, Z being selected from the group consisting of alkyl and heterocycloalkyl, a substituent is hydroxyl, and p = 0, 1, 2, or 3; and
$T_1$, $T_2$, and $T_3$ are independently selected from the group consisting of CH and N.

**44.** The compound, or the isomer thereof, or the racemate thereof, or the pharmaceutically acceptable salt thereof according to claim 43, wherein

the alkyl is selected from the group consisting of methyl, ethyl, n-propyl, isopropyl, n-butyl, isobutyl, sec-butyl, tert-butyl, n-pentyl, sec-pentyl, 1-ethylpropyl, 2-methylbutyl, tert-pentyl, 1,2-dimethylpropyl, isopentyl, neopentyl, n-hexyl, isohexyl, sec-hexyl, tert-hexyl, neohexyl, 2-methylpentyl, 1,2-dimethylbutyl, and 1-ethylbutyl;
the alkoxy is selected from the group consisting of methoxy, ethoxy, n-propoxy, isopropoxy, n-butoxy, isobutoxy, sec-butoxy, and tert-butoxy;
the alkylthio is selected from the group consisting of methylthio, ethylthio, propylthio, isopropylthio, n-butylthio, isobutylthio, sec-butylthio, and tert-butylthio;
the cycloalkyl is selected from the group consisting of cyclopropyl, cyclobutyl, cyclopentyl, and cyclohexyl; and the halogen is selected from the group consisting of fluorine, chlorine, bromine, and iodine.

**45.** The compound, or the isomer thereof, or the racemate thereof, or the pharmaceutically acceptable salt thereof according to claim 43, wherein
ring A is selected from the group consisting of

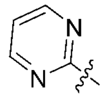, 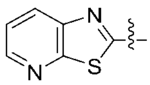, 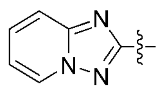,

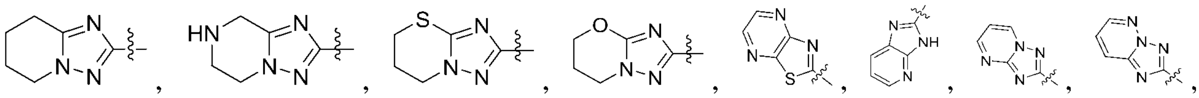,

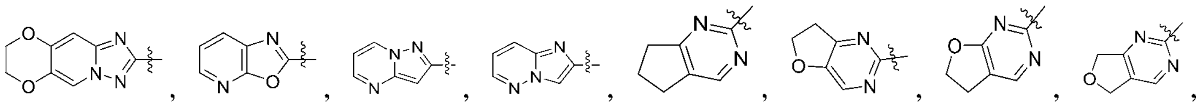,

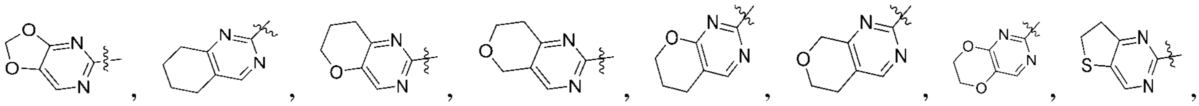,

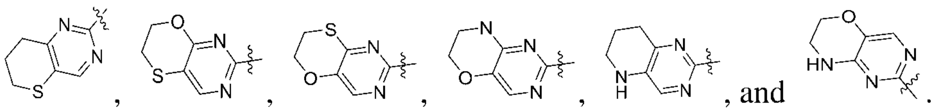.

**46.** The compound, or the isomer thereof, or the racemate thereof, or the pharmaceutically acceptable salt thereof according to claim 43, wherein
ring B is selected from

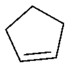, 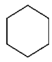, 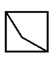, 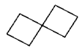, 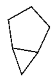, 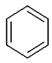, and 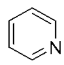;

and/or $R_1$ is selected from the group consisting of hydrogen, methyl, cyano, methoxy, cyclopropyl, cyclopropylmethyl, fluorine, chlorine, and $CHF_2$-O-; and/or $R_2$ is selected from the group consisting of hydrogen, methyl, cyano, methoxy, cyclopropyl, cyclopropylmethyl, fluorine, and chlorine; and/or ring B is connected in the following way:

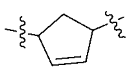, 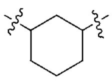, 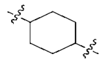,

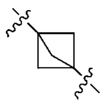, 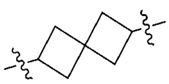, 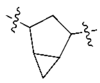, 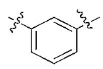, or 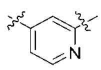.

**47.** The compound, or the isomer thereof, or the racemate thereof, or the pharmaceutically acceptable salt thereof according to claim 43, wherein the compound, or the isomer thereof, or the racemate thereof, or the pharmaceutically acceptable salt thereof is selected from the group consisting of:

| No. | Compound | No. | Compound | No. | Compound |
|---|---|---|---|---|---|
| J1 | 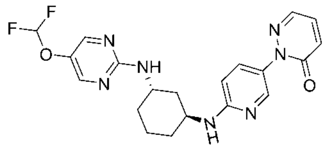 | J2 | 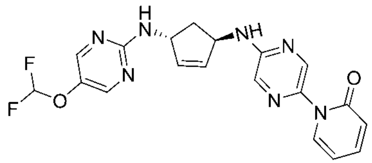 | J3 | 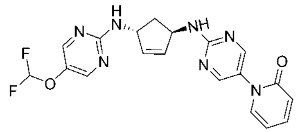 |
| J4 | 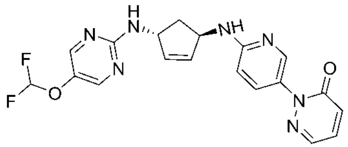 | J5 | 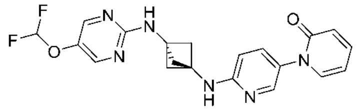 | J6 | 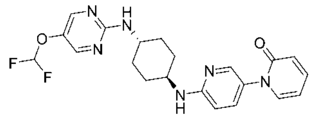 |
| J7 | 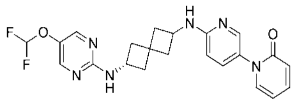 | J8 | 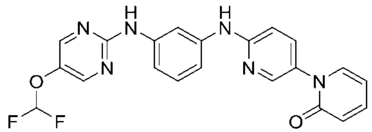 | J9 | 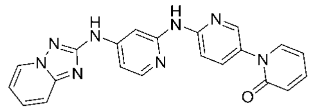 |

**48.** A pharmaceutical composition, comprising a therapeutically effective amount of the compound, or the isomer thereof, or the racemate thereof, or the pharmaceutically acceptable salt thereof according to any one of claims 1 to 47, and a pharmaceutically acceptable carrier.

**49.** Use of the compound, or the isomer thereof, or the racemate thereof, or the pharmaceutically acceptable salt thereof according to any one of claims 1 to 47 in the manufacture of a medicament for treating a PCSK9 inhibitor-related disease; preferably, the PCSK9 inhibitor-related disease comprises hypercholesterolemia.

**INTERNATIONAL SEARCH REPORT**

International application No.
**PCT/CN2024/120513**

**A. CLASSIFICATION OF SUBJECT MATTER**

C07D401/14(2006.01)i; C07D403/14(2006.01)i; A61K31/506(2006.01)i; A61P9/00(2006.01)i

According to International Patent Classification (IPC) or to both national classification and IPC

**B. FIELDS SEARCHED**

Minimum documentation searched (classification system followed by classification symbols)

IPC: C07D, A61K, A61P

Documentation searched other than minimum documentation to the extent that such documents are included in the fields searched

Electronic data base consulted during the international search (name of data base and, where practicable, search terms used)

CNABS, CNTXT, VEN, ENTXTC, CNKI, STN (CAPLUS, MARPAT, REGISTRY): PCSK9, 吡啶酮, pyridinone, 检索结构式, search for structural formula

**C. DOCUMENTS CONSIDERED TO BE RELEVANT**

| Category* | Citation of document, with indication, where appropriate, of the relevant passages | Relevant to claim No. |
|---|---|---|
| X | CN 113574055 A (ASTRAZENECA AB) 29 October 2021 (2021-10-29)<br>abstract, claims 1-71, and description, paragraphs [0384]-[1363] | 1-32, 43-49 |
| PX | WO 2024078620 A1 (SHANGHAI HANSOH BIOMEDICAL CO., LTD. et al.) 18 April 2024 (2024-04-18)<br>claims 1-18, and description, pages 38-92 | 1-22, 28-42, 48-49 |
| PX | WO 2024062090 A1 (ASTRAZENECA AB) 28 March 2024 (2024-03-28)<br>claims 1-22, and description, page 195 | 18-22, 48-49 |

☐ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **02 December 2024** | **02 January 2025** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | <br>Telephone No. |

Form PCT/ISA/210 (second sheet) (July 2022)

**INTERNATIONAL SEARCH REPORT**

International application No.
**PCT/CN2024/120513**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **49**
because they relate to subject matter not required to be searched by this Authority, namely:

Claim 49 sets forth a medicinal use of a compound, which relates to a method for treatment of diseases. The present search report is provided on the basis of a pharmaceutical use.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.
**PCT/CN2024/120513**

| Patent document cited in search report | Publication date (day/month/year) | Patent family member(s) | Publication date (day/month/year) |
|---|---|---|---|
| CN 113574055 A | 29 October 2021 | PE 20220138 A1 | 27 January 2022 |
| | | CL 2021001859 A1 | 14 January 2022 |
| | | CA 3125765 A1 | 23 July 2020 |
| | | DOP 2021000150 A | 31 August 2021 |
| | | US 2022220122 A1 | 14 July 2022 |
| | | ECSP 21060240 A | 30 September 2021 |
| | | EP 3911648 A2 | 24 November 2021 |
| | | EP 3911648 A4 | 26 October 2022 |
| | | EP 3911648 B1 | 23 October 2024 |
| | | SG 11202107614 PA | 30 August 2021 |
| | | CO 2021010312 A2 | 20 September 2021 |
| | | JOP 20210193 A1 | 30 January 2023 |
| | | US 2020291041 A1 | 17 September 2020 |
| | | US 11248001 B2 | 15 February 2022 |
| | | MX 2021008533 A | 19 August 2021 |
| | | JP 2022518015 A | 11 March 2022 |
| | | JP 7128969 B2 | 31 August 2022 |
| | | AU 2020209215 A1 | 26 August 2021 |
| | | AU 2020209215 B2 | 02 February 2023 |
| | | KR 20210116548 A | 27 September 2021 |
| | | MA 54261 A | 27 April 2022 |
| | | BR 112021013807 A2 | 30 November 2021 |
| | | TW 202043213 A | 01 December 2020 |
| | | TWI 852981 B | 21 August 2024 |
| | | IL 284640 A | 31 August 2021 |
| | | JP 2022116083 A | 09 August 2022 |
| | | EA 202191892 A1 | 24 February 2022 |
| | | CR 20210441 A | 11 March 2022 |
| | | WO 2020150473 A2 | 23 July 2020 |
| | | WO 2020150473 A3 | 04 February 2021 |
| WO 2024078620 A1 | 18 April 2024 | None | |
| WO 2024062090 A1 | 28 March 2024 | None | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 113574055 A


### Non-patent literature cited in the description


- Remington: The Science and Practice of Pharmacy. Lippincott, Williams & Wilkins, 2005